# EUROPEAN PATENT APPLICATION

(11) **EP 4 707 398 A2**
(43) Date of publication of application: **11.03.2026**
(21) Application number: 25208651.7
(22) Date of filing: 05.02.2021
(51) Int. Cl.: C12N 15/86

(54) **GENE EDITING METHODS FOR TREATING SPINAL MUSCULAR ATROPHY**

(30) Priority: 05.02.2020 US 202062970670 P
(62) Divisional of application: 21709823.5
(71) Applicant: The Broad Institute, Inc., Cambridge, MA 02142 (US); President and Fellows of Harvard College, Cambridge, MA 02138 (US)
(72) Inventor: LIU, David R., Cambridge, MA 02142 (US); ARBAB, Mandana, Cambridge, MA 02142 (US)
(74) Representative: Atkins, James Gordon John

(57) **Abstract**

The disclosure provides methods, base editors, vectors encoding base editors and cognate gRNAs, and compositions and kits comprise said components, for installing nucleobase edits to the *SMN2* locus to increase the activity and/or amount and/or stability of SMN2 protein in a cell, thereby treating Spinal Muscular Atrophy. In certain aspect, the disclosure provides compositions and methods to edit C840T of exon 7 of the *SMN2* gene, or installing another one or more nucleobase edits which have the effect of removing or inactivating a degron, such as the C-terminal portion of the region encoded by exon 6 or the 4-amino acid region encoded by exon 8 (i.e., the EMLA (SEQ ID NO: 466) -tail) so as to remove or limit their degron activity to reduce, mitigate, or eliminate the intracellular degradation of the SMN2 protein.

## Description

### RELATED APPLICATIONS

This application claims priority under 35 U.S.C. § 119(e) to U.S. Provisional Application, U.S.S.N. 62/970,670, filed February 5, 2020, which is incorporated herein by reference.

### BACKGROUND OF THE INVENTION

Spinal muscular atrophy (SMA) is a rare progressive motor neuron degeneration disorder that arises at various ages, including in infants. The disease is debilitating and is the leading heredity childhood disorder that results in fatality.^{1, 2} A rare condition, SMA occurs in approximately 1 in 11,000 births and manifests as a result of insufficient survival motor neuron (SMN) protein in spinal mdpcas9otor neurons encoded by the *SMN1* gene, leading to atrophy of skeletal muscle, paralysis of the patient, and eventual death. Symptoms include muscle weakness, poor muscle tone, weak cry, limpness or a tendency to flop, difficulty sucking or swallowing, accumulation of secretions in the lungs or throat, feeding difficulties, and increased susceptibility to respiratory tract infections. The legs tend to be weaker than the arms and developmental milestones often cannot be reached. In general, the earlier the symptoms appear, the shorter the individual's lifespan.

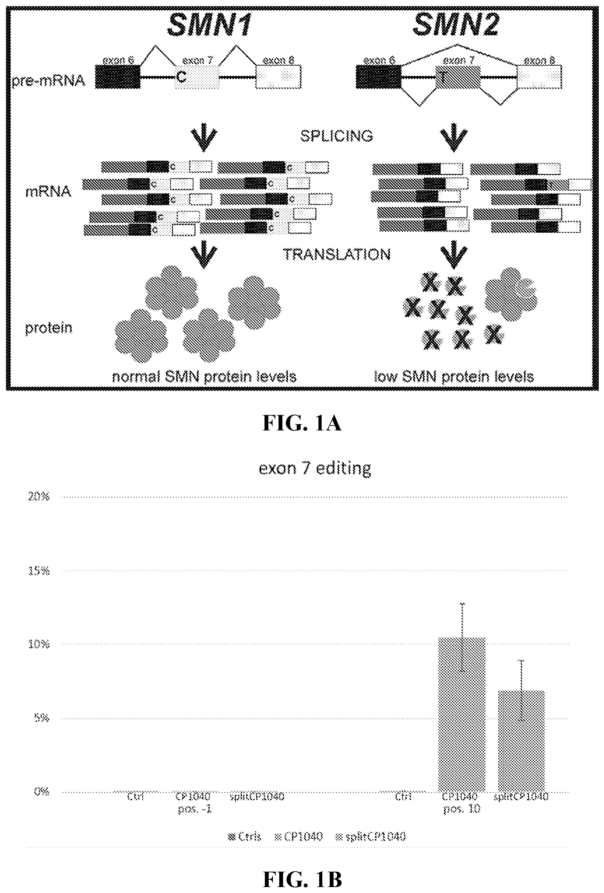

The low levels of SMN protein typically result from the homozygous deletion of both copies of the telomere-located *SMN1* gene.¹ However, most individuals have a second, virtually duplicate gene to *SMN1* known as the survival motor neuron 2 (*SMN2*) gene, which can compensate to varying degrees for the body's loss of the *SMN1* gene. The *SMN2* gene differs from the *SMN1* gene by five nucleotides, which importantly includes a C-to-T nucleotide change in exon 7 (also referred to as "C840T" or C6U" in the resulting mRNA). C840T in the *SMN2* gene results in the "skipping" or "exclusion" of exon 7 in the final mRNA transcript, which in turn, encodes a truncated but partially-functional SMN protein. See FIG. 1A. This exon skipping event due to C840T occurs about 90% of the time. Thus, the majority of SMN protein produced by the *SMN2* gene is truncated and only partially functional. Moreover, the truncate protein is more susceptible to degradation due to independent signaling of the proteosome complex by the exon 6-encoded portion and the terminal exon 8-encoded EMLA (SEQ ID NO: 466) sequence^{8.9}

The copy number of the *SMN2* gene can also vary among SMA individuals and can affect the severity of the disorder. In general, a higher *SMN2* gene copy number correlates

with less severe disease as the level of functional SMN2 protein is increased with gene copy number. As such, *SMN2* copy numbers are a strong determinant of patients' symptomatic outcomes.⁵⁻⁷ There are four recognized categories of SMA which differ by degree of severity, as well as other aspects.

Type 0 SMA is the rarest and most severe form of the condition and is apparent before birth. Affected infants are often born with joint deformities and have extremely weak muscle tone at birth. Type 0 SMA infants have weak respiratory muscles and often do not survive past infancy due to respiratory failure.

Type 1 SMA, which is also referred to as Werdnig-Hoffmann disease, is the most common form of the condition. It is a severe form of the disorder with muscle weakness evident at birth or within the first few months of life. Most children with type 1 SMA cannot control their head movements and cannot sit without assistance. These types of children may also experience difficulty swallowing, which can lead to poor feeding and growth. Also, due to weakened respiratory muscles and a bell-shaped chest condition, type 1 SMA children can also have severe breathing problems. Most children with type I SMA do not survive past early childhood due to respiratory failure.

Type 2 SMA, which is also referred to as Dubowitz disease, is characterized by muscle weakness that develops in children between ages 6 and 12 months. Children with this type can sit without support, although they may need help getting to a seated position. During later childhood, the muscle weakness worsens to the point where the individual may need support to sit. Individuals with type 2 SMA cannot stand or walk unaided. They often have involuntary tremors, scoliosis, and respiratory muscle weakness that can be life-threatening. The life span of individuals with spinal muscular atrophy type 2 varies and can be as long as their 20s or 30s.

Type 3 SMA, which is also called Kugelberg-Welander disease, typically has an onset that begins after early childhood. Individuals with this condition can stand and walk unaided, but over time, walking and climbing stairs may become increasingly difficult. Many affected individuals require wheelchair assistance later in life. Individuals with type 3 SMA typically have a normal life expectancy.

Type 4 SMA is the rarest of the SMA conditions and often does not begin until early adulthood. Affected individuals usually experience mild to moderate muscle weakness, tremors, and mild breathing problems. People with spinal SMA type 4 have a normal life expectancy.

The genetics of SMA are well understood, and approaches involving gene therapies have been pursued. Antisense oligonucleotides (ASOs) (*e.g*., nusinersen, approved in the U.S. as SPINRAZA^{®} in 2016) encoding short sequences that target *SMN2* exon 7 splicing regulatory sites that aid in the recruitment of the splicing machinery to the acceptor splice site to create corrected SMN mRNA transcripts that no longer translate into truncated, unstable proteins.^{10, 11} While this method has proven effective in upregulating SMN even *in vivo* in mouse models of SMA, the post-transcriptional modification of ASOs is only transient and thus, not curative. On the other hand, genetic modification that corrects SMN protein stability would lead to a lasting cure for SMA. However, no such therapies are available to date.

### SUMMARY OF THE INVENTION

The present disclosure relates in part to the inventors' discovery of base editing strategies that may be used to effectively target the *SMN2* genomic locus to install edits that affect SMN protein production and stability, thereby providing a new platform for treating SMA that goes beyond the currently available treatments, such as, antisense oligonucleotide treatments (e.g., nusinersen) which are transient in nature. The methods and compositions disclosed herein provide a treatment for SMA. Accordingly, the disclosure provides methods, base editors, gRNAs, complexes of base editors and gRNAs, nucleic acids and vectors encoding base editors and cognate gRNAs (*e.g*., gRNAs that the base editors provided herein bind), compositions and kits comprising said components, and systems for delivering said components. The aspects provided in this disclosure are useful for editing one or more genomic sequences associated with SMA, such as, but not limited to, editing C840T of exon 7 of the *SMN2* gene. They are also useful for installing one or more nucleobase edits which have the effect of removing or inactivating a degron, such as the C-terminal portion of the region encoded by exon 6 or the 4-amino acid region encoded by exon 8 (i.e., the EMLA (SEQ ID NO: 466) -tail) so as to remove or limit their degron activity to reduce, mitigate, or eliminate the degradation of the SMN2 protein.

Accordingly, aspects of the disclosure provide napDNAbp domains, deaminase domains, base editors comprising said napDNAbp domains and deaminase domains, guide RNAs for targeting the *SMN2* locus, nucleic acids and vectors encoding napDNAbp domains, deaminase domains, guide RNAs, and/or base editor fusions comprising said napDNAbp and deaminase domains, kits, and methods for modifying a polynucleotide (*e.g*., DNA) using a deaminase (e.g., an adenosine or cytidine deaminase) and a nucleic acid programmable DNA binding protein (*e.g*., Cas9) to correct the SNP and/or to increase the stability of the SMN

protein products. Such deaminases are described in International Application Nos.: PCT/US2017/045381 (WO 2018/027078), filed August 3, 2017; PCT/US2018/056146 (WO 2019/079347), filed October 16, 2018; PCT/US2019/033848 (WO 2019/226953), filed May 23, 2019; PCT/US2019/018285 (WO 2019/161251), filed February 15, 2019; PCT/US2019/019794 (WO 2019/168953), filed February 27, 2019; the entire contents of each of which are incorporated herein by reference.

In some embodiments, fusion proteins provided herein (*e.g*., adenosine or cytidine base editors) are used to edit C840T in the *SMN2* gene. For example, the disclosure provides methods and compositions for editing C840T in exon 7 of an *SMN2* gene, e.g., compositions including those gRNA sequences disclosed herein, including in the Examples. In other embodiments, fusion proteins provided herein (*e.g*., adenosine or cytidine base editors) are used to induce a frameshift mutation in exon 8 to prevent generation of the EMLA (SEQ ID NO: 466) tail, which signals protein degradation. In still other embodiments, fusion proteins provided herein (*e.g*., adenosine or cytidine base editors) are used to destroy a fifth codon stop sequence in exon 8, resulting in the addition of five amino acids onto the C-terminus of the translated protein. In another embodiment, fusion proteins provided herein (*e.g*., adenosine or cytidine base editors) are used to modify a specific amino acid of SMN2-derived proteins. For example, the fusion protein may be used to modify the S270 amino acid of *SMN2's* exon 6, a modification that has been shown to reduce the degradation rate of the resulting protein. The S270 modification is exemplified in Example 1 herein. Without wishing to be bound by any particular theory, a modified SMN2 protein comprising the amino acid sequence encoded by exon 7 is not sensitive to cellular degradation, unlike the wild type, truncated SMN2 product formed from the wild type *SMN2* gene as a result of exon 7-skipping. The overall activity and/or production of the modified SMN2 protein (i.e., now including the amino acid region encoded by exon 7) are increased, thereby treating SMA. Further, the increased production and/or activity of the modified SMN2 protein relates to the elimination or reduction in protein degradation associated with the truncated SMN2 wild type protein.

In some embodiments, the one or more nucleobase edits installed in the *SMN2* gene locus result in a modified *SMN2* gene product having increased stability, activity, and/or production relative to the wild type, truncated SMN2 protein of SEQ ID NO: 4. In various embodiments, the genome editing strategies disclosed herein can be implemented to target the editing of specific nucleobase positions in the *SMN2* gene locus, which when edited, impart the modified *SMN2* gene product with increased stability, activity, and/or production. In some embodiments, *SMN2* is a centromeric survival of motor neuron 2 from *Homo sapiens.* For example, Gene ID: 6607, which may also be referred to as SMNC, BCD541, GEMIN1, TDRD16B, or C-BCD541. *See,* for example, NG_008728.1 RefSeqGene.

For example, in one embodiment, the genome editing strategies disclosed herein target position 6 of exon 7 of the *SMN2* gene locus, which can be an inactive splice acceptor site due to the presence of a T in place of a C in exon 7 at that position. This nucleobase position is often referred to as C840T, i.e., which bears a T at position 6 of exon 7. By contrast, the corresponding position in the *SMN1* gene presents a C at position 6 of exon 7, which defines an active splice site. Thus, in one embodiment, the genome editing methods and compositions may be used to introduce a T-to-C edit at position 6 of exon 7 of the *SMN2* gene using an adenosine base editor, i.e., editing C840T to a C at position 6 of exon 7 and restoring proper splicing of exon 7, thereby encoding a modified SMN2 protein that includes the amino acid sequence encoded by exon 7.

In another embodiment, the genome editing strategies disclosed herein target the removal of one or more degrons present in the encoded SMN2 protein, thereby reducing or eliminating the degron-dependent degradation of the SMN2 protein. For example, the disclosure contemplates the use of genome editing to eliminate the 4-amino acid sequence tail of NH₃-EMLA (SEQ ID NO: 466) -COOH encoded by exon 8 of the *SMN2* gene-which functions as a degron-thereby minimizing or eliminating the degradation associated with said degron. In certain aspects, the EMLA (SEQ ID NO: 466) sequence can be removed by using genome editing to remove the exon 8 splice site, such that exon 8 is eliminated from the final messenger RNA, and thus, not translated into the resulting SMN2 protein.

In yet another embodiment as described in Example 2, the genome editing strategies disclosed herein target the modification of one or more degrons present in the encoded SMN2 protein, thereby reducing or eliminating the degradation associated with said one or more degrons. For example, the disclosure contemplates the use of genome editing to eliminate a stop codon corresponding to the C-terminal end of the region encoded by exon 8 (i.e., which codes for the 4-amino acid sequence tail of EMLA (SEQ ID NO: 466) ) such that the 4-amino acid EMLA (SEQ ID NO: 466) tail is lengthened to include additional amino acids, *e.g.,* 2-10 additional amino acid (for example, 5 additional amino acids), which prevents or limits the degradation associated with said EMLA (SEQ ID NO: 466) tail.

In another embodiment, the disclosure provides a genome editing strategy comprising modification of the ISS-N1 splicing silencer in exon 7. Modification of the ISS-N1 splicing silencer (e.g., using nucleases or base editors) prevents skipping of exon 7 in SMN transcripts.

Aspects of the disclosure provide methods and compositions for deaminating an adenine nucleobase (A) in an *SMN2* gene using an adenine base editor (ABE) bound to a guide RNA (gRNA), wherein the gRNA comprises a guide sequence that is complementary to a target nucleic acid sequence in the *SNM2* gene.

Other aspects of the disclosure provide methods and compositions for deaminating an cytosine nucleobase (C) in an *SMN2* gene using a cytidine base editor (CBE) bound to a guide RNA (gRNA), wherein the gRNA comprises a guide sequence that is complementary to a target nucleic acid sequence in the *SNM2* gene.

In some embodiments, the base editor is delivered *in vivo* to a subject. In some embodiments, the base editor is delivered in two parts, for example, by using a split-intein strategy. In some embodiments, editing of C840T in exon 7 results in an increase, *e.g*., at least 5%, 10%, 20%, 30%, 40%, 50%, 60%, 70%, 80%, 90%, 95%, 96%, 97%, 98%, 99%, 100%, or more increase, in the level of SMN protein in a subject, in an organ or tissue of a subject (*e.g*., brain, heart, lungs, liver, intestine, and/or pancreas), or in a cell of a subject (*e.g*., neuron, such as a motor neuron). In certain embodiments, the base editor is delivered by intracerebroventricular injection.

In some embodiments, the disclosure provides compositions comprising a base editor (*e.g*., adenine base editor) and a guide RNA (gRNA). In some embodiments, the gRNA directs the base editor in proximity to a point mutation in the *SMN2* gene, for example, a point mutation in exon 7. In some embodiments, the gRNA directs the base editor within 1, 2, 3, 4, 5, 6, 7, 8, 9, 10, 11, 12, 13, 14, 15, 16, 17, 18, 19, or 20 base pairs of a point mutation within the *SMN2* gene. In some embodiments, the gRNA comprises the protospacer sequence 5'-ATTTTGTCTAAAACCctgta-3' (SEQ ID NO: 364), where the nucleotide target is indicated in bold. It should be appreciated that the Ts indicated in the gRNA sequence are uracil (Us) in the RNA sequence. Accordingly, in some embodiments, the gRNA comprises the sequence 5'-AUUUUGUCUAAAACCcugua-3' (SEQ ID NO: 365). In some embodiments, the guide sequence of the gRNA comprises a nucleic acid sequence selected from the group consisting of 5'-TTTGTCTAAAACCCTGTAAG-3' (SEQ ID NO: 366), 5'-TTTTGTCT**A**AAACCCTGTAA-3' (SEQ ID NO: 368), 5'-TGATTTTGTCT**A**AAACCC-3' (SEQ ID NO: 370), 5'-GATTTTGTCT**A**AAACCCT-3' (SEQ ID NO: 372), 5'-ATTTTGTCT**A**AAACCCTG-3' (SEQ ID NO: 374), 5'-GTCT**A**AAACCCTGTAAGG-3' (SEQ ID NO: 376), and 5'-TCT**A**AAACCCTGTAAGGA-3' (SEQ ID NO: 378). As noted previously, the gRNA sequence may comprise uracil (U) instead of thymine (T). Therefore, in some embodiments, the guide sequence of the gRNA comprises a nucleic acid sequence selected from the group consisting of 5'-UUUGUCU**A**AAACCCUGUAAG-3' (SEQ ID NO: 367), 5'-UUUUGUCU**A**AAACCCUGUAA-3' (SEQ ID NO: 369), 5'-UGAUUUUGUCU**A**AAACCC-3' (SEQ ID NO: 371), 5'-GAUUUUGUCU**A**AAACCCU-3' (SEQ ID NO: 373), 5'-AUUUUGUCU**A**AAACCCUG-3' (SEQ ID NO: 375), 5'-GUCU**A**AAACCCUGUAAGG-3' (SEQ ID NO: 377), and 5'-UCU**A**AAACCCUGUAAGGA-3' (SEQ ID NO: 379).

In some embodiments, the disclosure provides compositions comprising a fusion protein (*e.g*., adenosine base editor or cytidine base editor) and a guide RNA (gRNA). In some embodiments, the gRNA directs the fusion protein in proximity to a stop sequence in the *SMN2* gene, for example, in exon 8. In some embodiments, the gRNA directs the fusion protein within 1, 2, 3, 4, 5, 6, 7, 8, 9, 10, 11, 12, 13, 14, 15, 16, 17, 18, 19, or 20 base pairs of stop codon within the *SMN2* gene. In some embodiments, the gRNA comprises a nucleic acid sequence selected from the group consisting of: 5'-TTTGCAGGAAATGCTGGCA**T**-3' (SEQ ID NO: 381), 5'-TTCTCATTTGCAGGAAATGC-3' (SEQ ID NO: 383), 5'-CATTTAGTGCTGCT**CTA**TGC-3' (SEQ ID NO: 385), 5'-CAGGAAATGCTGGCA**TAG**AG-3' (SEQ ID NO: 387), 5'-TTGCAGGAAATGCTGGCA**TA**-3' (SEQ ID NO: 389), 5'-ATTTGCAGGAAATGCTGGCA-3' (SEQ ID NO: 391), and 5'-TGGCA**TAG**AGCAGCACTAAA-3' (SEQ ID NO: 393), where the nucleotide target is indicated in bold. It should be appreciated that the thymidines (Ts) indicated in the gRNA sequences above are uracils (Us) in the RNA sequence. Accordingly, in some embodiments, the gRNA comprises a sequence selected from the group consisting of: 5'-UUUGCAGGAAAUGCUGGCAU-3' (SEQ ID NO: 382), 5'-UUCUCAUUUGCAGGAAAUGC-3' (SEQ ID NO: 384), 5'-CAUUUAGUGCUGCU**CUA**UGC-3' (SEQ ID NO: 386), 5'-CAGGAAAUGCUGGCA**UAG**AG-3' (SEQ ID NO: 388), 5'-UUGCAGGAAAUGCUGGCA**UA**-3' (SEQ ID NO: 390), 5'-AUUUGCAGGAAAUGCUGGCA-3' (SEQ ID NO: 392), and 5'-UGGCA**UAG**AGCAGCACUAAA-3' (SEQ ID NO: 394).

In some embodiments, the disclosure provides compositions comprising a fusion protein (*e.g*., adenine base editor or cytidine base editor) and a guide RNA (gRNA). In some embodiments, the gRNA directs the fusion protein in proximity to a specific codon sequence in the *SMN2* gene, for example, in exon 6. In one embodiment, the gRNA directs the fusion protein in proximity to the S270 amino acid of *SMN2.* In some embodiments, the gRNA directs the fusion protein within 1, 2, 3, 4, 5, 6, 7, 8, 9, 10, 11, 12, 13, 14, 15, 16, 17, 18, 19, or 20 base pairs of a specific codon within the *SMN2* gene. In some embodiments, the gRNA comprises the nucleic acid sequence 5'-TGGCA**TAG**AGCAGCACTAAA-3' (SEQ ID NO: 393), where the nucleotide target is indicated in bold. It should be appreciated that the thymidine (Ts) indicated in the gRNA sequences above are uracil (Us) in the RNA sequence. Accordingly, in some embodiments, the gRNA comprises the sequence: 5'-UGGCA**UAG**AGCAGCACUAAA-3' (SEQ ID NO: 394).

In some embodiments, the base editor nicks the target sequence that is complementary to the guide sequence. In some embodiments, the base editor is a circular permutant (CP) adenine base editor, such as the CP₁₀₄₁ base editor. In some embodiments, the base editor may be formulated as a split-intein base editor. The base editor may be, for example, wild-type SpCas9, SaCas9-KKH, Cas9-VQR, Cas9-VRQR, Cas9-VRER, Cas9-NG, CP1028, CP1041, CP1041-NG, Cpfl, iSpyMac, SpCas9-NRRH, or SpCas9-NRCH.

Compositions, kits, methods, systems, proteins, and nucleic acids, provided herein, in some embodiments, are used to treat a subject having or a subject suspected of having SMA.

Thus, in some aspects, the base editor and guide RNA complexes described herein may be useful for treating a disease or a disorder caused by a C to T mutation in a *SMN2* gene. In some embodiments, deaminating the adenosine nucleobase in the *SMN2* gene results in a T-A base pair in the *SMN2* gene being mutated to a C-G base pair in the *SMN2* gene. In some embodiments, deaminating the adenosine nucleobase in the *SMN2* gene leads to an increase in SMN protein levels (*e.g.,* by preventing skipping of exon 7, reducing protein degradation). In some embodiments, deaminating the adenosine nucleobase in the *SMN2* gene results in correcting a sequence associated with SMA. In some embodiments, deaminating the adenosine nucleobase in the *SMN2* gene ameliorates one or more symptoms of SMA.

It should be appreciated that the foregoing concepts, and additional concepts discussed below, may be arranged in any suitable combination, as the present disclosure is not limited in this respect. Further, other advantages and novel features of the present disclosure will become apparent from the following detailed description of various non-limiting embodiments when considered in conjunction with the accompanying Figures.

### BRIEF DESCRIPTION OF THE DRAWINGS

The following Figures form part of the present specification and are included to further demonstrate certain aspects of the present disclosure, which can be better understood by reference to one or more of these drawings in combination with the detailed description of specific embodiments presented herein.
FIG. 1A is a schematic comparing the *SMN1* and *SMN2* genes and in particular shows the variable splicing pattern associated with the C-to-T mutation in exon 7.
FIG. 1B is a graph demonstrating A-to-G conversions of adenines at position 1 and position 10 of the CP₁₀₄₁ and split-intein CP₁₀₄₁ ("splitCP1041") at the *SMN2* exon 7 locus. Triplicate transfection experiments in mouse embryonic stem cells (mESCs) derived from the SMA mouse model (Jackson Laboratories, 005025) at the human *SMN2* transgene were undertaken.
FIG. 2A is a graph showing inDelphi predictions of favorable ("good phenotype") and unfavorable ("bad genotype") repair outcomes following cutting with an sgRNA described herein (5' - TTTGCAGGAAATGCTGGCAT- 3' (SEQ ID NO: 381)).
FIG. 2B is a graph showing inDelphi predictions of favorable ("good phenotype") and unfavorable ("bad genotype") repair outcomes following cutting with an sgRNA described herein (5' - TTCTCATTTGCAGGAAATGC- 3' (SEQ ID NO: 383)).
FIG. 2C provides editing outcomes at *SMN2* exon 8 of the human *SMN2* transgene in SMA mESCs following transfection with 5' - TTTGCAGGAAATGCTGGCAT- 3' (SEQ ID NO: 381) (from FIG. 2A) and Cas9-NG or 5' - TTCTCATTTGCAGGAAATGC- 3' (SEQ ID NO: 383) (from FIG. 2B) and wild-type SpCas9.

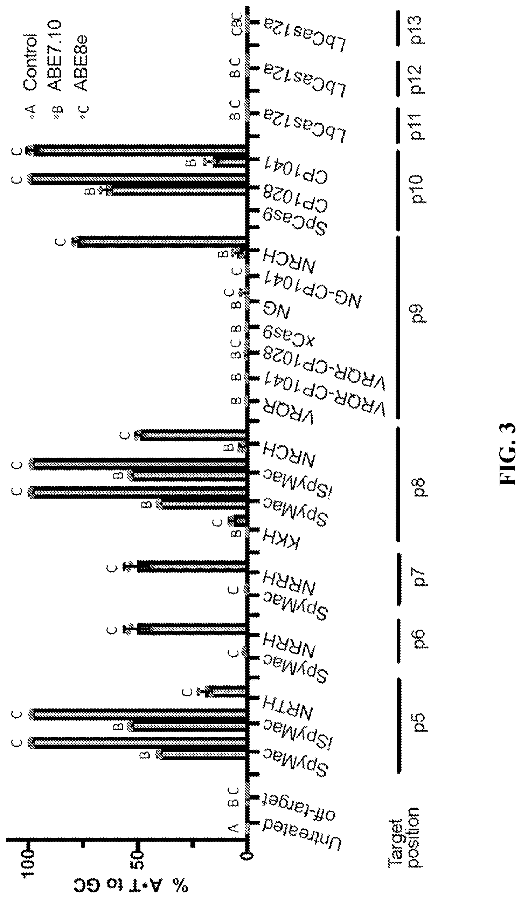

FIG. 3 shows adenine base editing efficiency at the SMN2 C6T in SMA mESCs with ABE7.10 and ABE8e editors using the Cas-protein components indicated on the x-axis. Protospacer position of C6T for each condition is indicated. See Example 3.
FIG. 4 shows gel electrophoresis image of SMN cDNA PCR amplification spanning exon 6 to exon 8, depicting bands that include or that have skipped exon 7 in pre-mRNA splicing in SMA mESCs treated with the indicated ABE8-fusion base editors. See Example 3.

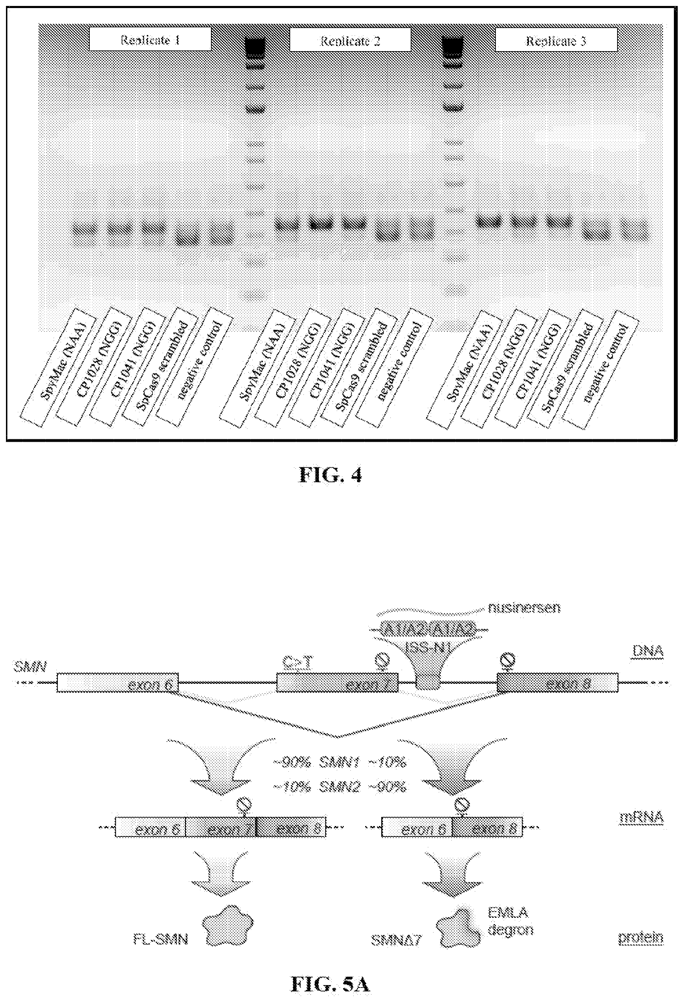

FIG. 5A is a schematic representation of SMN exons 6 to 8, and SMN mRNA and protein products. The C>T transition is a master splicing regulator that determines whether the majority of products include (C, SMN1) or skip (T, SMN2) the terminal coding exon 7. Full-length SMN transcripts yield stable FL-SMN protein. Skipped transcripts encode truncated SMNΔ7 proteins that contain a short-peptide chain translated from the 3'-UTR in exon 8 (EMLA (SEQ ID NO: 466) ) that signals for protein degradation. The intron 7 splicing silencer ISS-N1, containing two hnRNP A1/A2 domains, is targeted by nusinersen to restore exon 7 inclusion in the majority of SMN2 transcripts.
FIG. 5B is an illustration of two Cas nuclease strategies to rescue SMN pathology. 1) ISS-N1 editing strategy targeting the 3' hnRNPA1/A2 domain. Deletions may cause loss of function of the splicing silencer, enhancing inclusion of exon 7 in SMN2 transcripts and production of FL-SMN. 2) Exon 8 editing strategy showing Cas nuclease targeting of the 5' sequence that encodes the degradation-enhancing EMLA (SEQ ID NO: 466) peptide and stop codons in the majority of SMN2 transcripts. Deletions disrupting the first five codons may result in more stable, modified SMNΔ7 (SMNΔ7mod) proteins.
FIG. 5C is a table of potential sgRNA and nuclease editor combinations targeting ISS-N1 detailing the cutsite of the DSB, predicted percentage of 'good' outcomes among all edits, and relative Cas-protein efficiency at the respective PAM.
FIG. 5D shows genome editing efficiency at ISS-N1 with the indicated sgRNA and Cas nuclease combinations in SMA mESCs.
FIG. 5E shows genome editing efficiency at SMN2 exon 8 with the indicated sgRNA and Cas nuclease combinations, including the observed percentage of 'good' outcomes among all edits.
FIG. 5F is a plot depicting SMN protein quantitation relative to Histone H3, following SMN2 exon 8 with the indicated sgRNA and Cas nuclease combinations.
FIG. 6A is an illustration of an adenine base editing strategy to edit C6T in SMN2, restoring exon 7 splicing inclusion in the majority of transcripts and resulting in upregulation of FL-SMN.
FIG. 6B is a schematic of a high-throughput genome integrated library of sgRNA: target pairs to enable comprehensive characterization of ABE8e editing outcomes. A library of highly diverse sequences was stably integrated into mESCs using Tol2-transposase followed by hygromycin antibiotic selection. Library cells were targeted with ABE8e and cells were stably selected using blasticidin. Library cassettes were then amplified and analyzed by high-throughput sequencing.
FIG. 6C shows the ABE8e activity profile. Values reflect percent editing efficiency by indicated protospacer position of the outcome specified at the bottom of each column, relative to maximum editing. The middle column indicates canonical A to G base editing activity. The two leftmost columns indicate other adenine mutation activity. The two rightmost columns indicate other rare mutations. Positions with values ≥ 30% of maximum are boxed, indicating the ABE8e editing window.
FIG. 6D shows the sequence motif of canonical A to G ABE8e base editing activity from logistic regression modeling. The sign of each learned weight indicates a contribution above (positive sign) or below (negative sign) the mean activity. Logo opacity is proportional to the Pearson's R or AUC on held-out sequence contexts.
FIG. 6E shows adenine base editing efficiency at the on-target SMN2 locus and top off-target loci in HEK293T cells by ABE8e-SpyMac paired with sgRNA52 (collectively: ABE8e) transfection. Bars represent editing of the highest edited nucleotide position at each locus, as indicated.
FIG. 6F is a plot depicting relative quantity of FL-SMN over total SMN transcripts of SMA mESCs following ABE editing, as indicated. Values calculated by automated electrophoresis of RT-PCR products.
FIG. 6G is a plot depicting SMN protein quantitation relative to Histone H3.
FIG. 6H is a schematic of split-intein ABE8e-SpyMac and sgRNA52 cassettes as encoded into the dual-AAV vectors, constituting AAV-ABE.
FIG. 6I is a plot depicting percent SMN2 C6T genome editing, and relative quantity of FL-SMN over total SMN transcripts of SMA mESCs following transfection with AAV-ABE plasmids.
FIG. 7A is a Kaplan-Meier survival plot of SMA mutant mice with ("A"), and without ("B") ICV injection of AAV-ABE on P0-P1.
FIG. 7B shows bodyweight measurements for the indicated SMA mouse cohorts.
FIG. 7C is a plot depicting percent SMN2 C6T genome editing, and relative quantity of FL-SMN over total SMN transcripts of SMA mESCs following transfection with nusinersen (ASO) and the optimized ABE strategy individually, and in combination.
FIG. 7D is a Kaplan-Meier survival plot of SMA mutant mice with, and without ICV injection of AAV-ABE on P0-P1 and a single injection of 1ug ASO on P0.
FIG. 7E shows bodyweight measurements for the indicated SMA mouse cohorts.
FIG. 7F is a plot depicting percent SMN2 C6T genome editing in the CNS of treated SMA mice. Bars represent bulk, and GFP positive flow sorted nuclei.
FIG. 8A is an automated electrophoresis image of SMN2 RT-PCR products of transfected SMA mESCs, as indicated, beside a DNA ladder.
FIG. 8B is a plot depicting relative quantity of FL-SMN over total SMN transcripts of SMA mESCs following Cas nuclease editing.
FIGs. 8C-8D show BE-Hive modeling predictions of ABE7.10-CP1041 and ABE7.10-SpCas9 base edited outcomes for SMN2 C6T. Percentages indicate the predicted fraction of a given genotype among edited alleles.
FIG. 8E shows BE-Hive modeling predictions of ABE7.10-CP1041 and ABE7.10-SpCas9 base editing efficiency at the SMN2 C6T locus.
FIG. 8F shows CIRCLE-Seq off-target editing outcomes in the human genome of SpyMac nuclease paired with sgRNA52. Mismatches at off-target loci are indicated below the corresponding protospacer position.
FIG. 8G shows nuclease editing efficiency at the on-target SMN2 locus and top off-target loci in HEK293T cells by SpyMac nuclease paired with sgRNA52 (collectively: Nuclease).
FIG. 8H shows a time course of exon 7 splicing inclusion in SMN transcripts following nusinersen transfection in SMA mESCs for. Values calculated by automated electrophoresis of RT-PCR products.

### DEFINITIONS

As used herein and in the claims, the singular forms "a," "an," and "the" include the singular and the plural reference unless the context clearly indicates otherwise. Thus, for example, a reference to "an agent" includes a single agent and a plurality of such agents.

### AAV

An "adeno-associated virus" or "AAV" is a virus which infects humans and some other primate species. The wild-type AAV genome is a single-stranded deoxyribonucleic acid (ssDNA), either positive- or negative-sensed. The genome comprises two inverted terminal repeats (ITRs), one at each end of the DNA strand, and two open reading frames (ORFs): rep and cap between the ITRs. The rep ORF comprises four overlapping genes encoding Rep proteins required for the AAV life cycle. The cap ORF comprises overlapping genes encoding capsid proteins: VP1, VP2 and VP3, which interact together to form the viral capsid. VP1, VP2 and VP3 are translated from one mRNA transcript, which can be spliced in two different manners: either a longer or shorter intron can be excised resulting in the formation of two isoforms of mRNAs: a ~2.3 kb- and a ~2.6 kb-long mRNA isoform. The capsid forms a supramolecular assembly of approximately 60 individual capsid protein subunits into a non-enveloped, T-1 icosahedral lattice capable of protecting the AAV genome. The mature capsid is composed of VP1, VP2, and VP3 (molecular masses of approximately 87, 73, and 62 kDa respectively) in a ratio of about 1:1:10.

rAAV particles may comprise a nucleic acid vector (*e.g*., a recombinant genome), which may comprise at a minimum: (a) one or more heterologous nucleic acid regions comprising a sequence encoding a protein or polypeptide of interest (*e.g*., a split Cas9 or split nucleobase) or an RNA of interest (*e.g.,* a gRNA), or one or more nucleic acid regions comprising a sequence encoding a Rep protein; and (b) one or more regions comprising inverted terminal repeat (ITR) sequences (*e.g*., wild-type ITR sequences or engineered ITR sequences) flanking the one or more nucleic acid regions (*e.g*., heterologous nucleic acid regions). In some embodiments, the nucleic acid vector is between 4 kb and 5 kb in size (*e.g.,* 4.2 to 4.7 kb in size). In some embodiments, the nucleic acid vector further comprises a region encoding a Rep protein. In some embodiments, the nucleic acid vector is circular. In some embodiments, the nucleic acid vector is single-stranded. In some embodiments, the nucleic acid vector is double-stranded. In some embodiments, a double-stranded nucleic acid vector may be, for example, a self-complimentary vector that contains a region of the nucleic acid vector that is complementary to another region of the nucleic acid vector, initiating the formation of the double-strandedness of the nucleic acid vector.

### Adenosine deaminase (or adenine deaminase)

As used herein, the term "adenosine deaminase" or "adenosine deaminase domain" refers to a protein or enzyme that catalyzes a deamination reaction of an adenosine (or adenine). The terms "adenosine" and "adenine" are used interchangeably for purposes of the present disclosure. For example, for purposes of the disclosure, reference to an "adenine base editor" (ABE) refers to the same entity as an "adenosine base editor" (ABE). Similarly, for purposes of the disclosure, reference to an "adenine deaminase" refers to the same entity as an "adenosine deaminase." However, the person having ordinary skill in the art will appreciate that "adenine" refers to the purine base whereas "adenosine" refers to the larger nucleoside molecule that includes the purine base (adenine) and sugar moiety (e.g., either ribose or deoxyribose). In certain embodiments, the disclosure provides base editor fusion proteins comprising one or more adenosine deaminase domains. For instance, an adenosine deaminase domain may comprise a heterodimer of a first adenosine deaminase and a second deaminase domain, connected by a linker. Adenosine deaminases (e.g., engineered adenosine deaminases or evolved adenosine deaminases) provided herein may be enzymes that convert adenine (A) to inosine (I) in DNA or RNA. Such adenosine deaminase can lead to an A:T to G:C base pair conversion. In some embodiments, the deaminase is a variant of a naturally-occurring deaminase from an organism. In some embodiments, the deaminase does not occur in nature. For example, in some embodiments, the deaminase is at least 50%, at least 55%, at least 60%, at least 65%, at least 70%, at least 75% at least 80%, at least 85%, at least 90%, at least 95%, at least 96%, at least 97%, at least 98%, at least 99%, or at least 99.5% identical to a naturally-occurring deaminase.

In some embodiments, the adenosine deaminase is derived from a bacterium, such as, *E.coli, S. aureus, S. typhi, S. putrefaciens, H. influenzae,* or C. *crescentus.* In some embodiments, the adenosine deaminase is a TadA deaminase. In some embodiments, the TadA deaminase is an *E. coli* TadA deaminase (ecTadA). In some embodiments, the TadA deaminase is a truncated *E. coli* TadA deaminase. For example, the truncated ecTadA may be missing one or more N-terminal amino acids relative to a full-length ecTadA. In some embodiments, the truncated ecTadA may be missing 1, 2, 3, 4, 5 ,6, 7, 8, 9, 10, 11, 12, 13, 14, 15, 6, 17, 18, 19, or 20 N-terminal amino acid residues relative to the full length ecTadA. In some embodiments, the truncated ecTadA may be missing 1, 2, 3, 4, 5 ,6, 7, 8, 9, 10, 11, 12, 13, 14, 15, 6, 17, 18, 19, or 20 C-terminal amino acid residues relative to the full length ecTadA. In some embodiments, the ecTadA deaminase does not comprise an N-terminal methionine. Reference is made to U.S. Patent Publication No. 2018/0073012, published March 15, 2018, which is incorporated herein by reference.

### Antisense strand

In genetics, the "antisense" strand of a segment within double-stranded DNA is the template strand, and which is considered to run in the 3' to 5' orientation. By contrast, the "sense" strand is the segment within double-stranded DNA that runs from 5' to 3', and which is complementary to the antisense strand of DNA, or template strand, which runs from 3' to 5'. In the case of a DNA segment that encodes a protein, the sense strand is the strand of DNA that has the same sequence as the mRNA, which takes the antisense strand as its template during transcription, and eventually undergoes (typically, not always) translation into a protein. The antisense strand is thus responsible for the RNA that is later translated to protein, while the sense strand possesses a nearly identical makeup to that of the mRNA. Note that for each segment of dsDNA, there will possibly be two sets of sense and antisense, depending on which direction one reads (since sense and antisense is relative to perspective). It is ultimately the gene product, or mRNA, that dictates which strand of one segment of dsDNA is referred to as sense or antisense.

### Base editing

"Base editing" refers to genome editing technology that involves the conversion of a specific nucleic acid base into another at a targeted genomic locus. In certain embodiments, this can be achieved without requiring double-stranded DNA breaks (DSB), or single stranded breaks (*i.e.,* nicking). To date, other genome editing techniques, including CRISPR-based systems, begin with the introduction of a DSB at a locus of interest. Subsequently, cellular DNA repair enzymes mend the break, commonly resulting in random insertions or deletions (indels) of bases at the site of the DSB. However, when the introduction or correction of a point mutation at a target locus is desired rather than stochastic disruption of the entire gene, these genome editing techniques are unsuitable, as correction rates are low (*e.g*. typically 0.1% to 5%), with the major genome editing products being indels. In order to increase the efficiency of gene correction without simultaneously introducing random indels, the present inventors previously modified the CRISPR/Cas9 system to directly convert one DNA base into another without DSB formation. See, Komor, A.C., et al., Programmable editing of a target base in genomic DNA without double-stranded DNA cleavage. Nature 533, 420-424 (2016), the entire contents of which is incorporated by reference herein.

### Base editor

The term "base editor (BE)" as used herein, refers to an agent comprising a polypeptide that is capable of making a modification to a base (*e.g.,* A, T, C, G, or U) within a nucleic acid sequence (*e.g*., DNA or RNA) that converts one base to another (e.g., A to G, A to C, A to T, C to T, C to G, C to A, G to A, G to C, G to T, T to A, T to C, T to G). In some embodiments, the base editor is capable of deaminating a base within a nucleic acid such as a base within a DNA molecule. In the case of an adenine base editor, the base editor is capable of deaminating an adenine (A) in DNA. Such base editors may include a nucleic acid programmable DNA binding protein (napDNAbp) fused to an adenosine deaminase. Some base editors include CRISPR-mediated fusion proteins that are utilized in the base editing methods described herein. In some embodiments, the base editor comprises a nuclease-inactive Cas9 (dCas9) fused to a deaminase which binds a nucleic acid in a guide RNA-programmed manner via the formation of an R-loop, but does not cleave the nucleic acid. For example, the dCas9 domain of the fusion protein may include a D10A and a H840A mutation (which renders Cas9 capable of cleaving only one strand of a nucleic acid duplex), as described in PCT/US2016/058344, which published as WO 2017/070632 on April 27, 2017, and is incorporated herein by reference in its entirety. The DNA cleavage domain of *S*. *pyogenes* Cas9 includes two subdomains, the HNH nuclease subdomain and the RuvC1 subdomain. The HNH subdomain cleaves the strand complementary to the gRNA (the "targeted strand", or the strand in which editing or deamination occurs), whereas the RuvC1 subdomain cleaves the non-complementary strand containing the PAM sequence (the "non-edited strand"). The RuvC1 mutant D10A generates a nick in the targeted strand, while the HNH mutant H840A generates a nick on the non-edited strand (see Jinek et al., Science, 337:816-821(2012); Qi et al., Cell. 28;152(5):1173-83 (2013)).

In some embodiments, a nucleobase editor is a macromolecule or macromolecular complex that results primarily (*e.g*., more than 80%, more than 85%, more than 90%, more than 95%, more than 99%, more than 99.9%, or 100%) in the conversion of a nucleobase in a polynucleic acid sequence into another nucleobase (i.e., a transition or transversion) using a combination of 1) a nucleotide-, nucleoside-, or nucleobase-modifying enzyme; and 2) a nucleic acid binding protein that can be programmed to bind to a specific nucleic acid sequence.

In some embodiments, the nucleobase editor comprises a DNA binding domain (*e.g*., a programmable DNA binding domain such as a dCas9 or nCas9) that directs it to a target sequence. In some embodiments, the nucleobase editor comprises a nucleobase modifying enzyme fused to a programmable DNA binding domain (*e.g.,* a dCas9 or nCas9). A "nucleobase modifying enzyme" is an enzyme that can modify a nucleobase and convert one nucleobase to another (*e.g*., a deaminase such as a cytidine deaminase or a adenosine deaminase). In some embodiments, the nucleobase editor may target cytosine (C) bases in a nucleic acid sequence and convert the C to thymine (T) base. In some embodiments, the C to T editing is carried out by a deaminase, e.g., a cytidine deaminase. Base editors that can carry out other types of base conversions (*e.g*., adenosine (A) to guanine (G), C to G) are also contemplated.

Nucleobase editors that convert a C to T, in some embodiments, comprise a cytidine deaminase. A "cytidine deaminase" refers to an enzyme that catalyzes the chemical reaction "cytosine + H₂O → uracil + NH₃" or "5-methyl-cytosine + H₂O → thymine + NH₃." As it may be apparent from the reaction formula, such chemical reactions result in a C to U/T nucleobase change. In the context of a gene, such a nucleotide change, or mutation, may in turn lead to an amino acid change in the protein, which may affect the protein's function, *e.g*., loss-of-function or gain-of-function. In some embodiments, the C to T nucleobase editor comprises a dCas9 or nCas9 fused to a cytidine deaminase. In some embodiments, the cytidine deaminase domain is fused to the N-terminus of the dCas9 or nCas9. In some embodiments, the nucleobase editor further comprises a domain that inhibits uracil glycosylase, and/or a nuclear localization signal. Such nucleobase editors have been described in the art, *e.g.,* in Rees & Liu, Nat Rev Genet. 2018;19(12):770-788 and Koblan et al., Nat Biotechnol. 2018;36(9):843-846; as well as.U.S. Patent Publication No. 2018/0073012, published March 15, 2018, which issued as U.S. Patent No. 10,113,163; on October 30, 2018; U.S. Patent Publication No. 2017/0121693, published May 4, 2017, which issued as U.S. Patent No. 10,167,457 on January 1, 2019; International Publication No. WO 2017/070633, published April 27, 2017; U.S. Patent Publication No. 2015/0166980, published June 18, 2015; U.S. Patent No. 9,840,699, issued December 12, 2017; U.S. Patent No. 10,077,453, issued September 18, 2018; International Publication No. WO 2019/023680, published January 31, 2019; International Publication No. WO 2018/0176009, published September 27, 2018, International Application No PCT/US2019/033848, filed May 23, 2019, International Application No. PCT/US2019/47996, filed August 23, 2019; International Application No. PCT/US2019/049793, filed September 5, 2019; U.S. Provisional Application No. 62/835,490, filed April 17, 2019; International Application No. PCT/US2019/61685, filed November 15, 2019; International Application No. PCT/US2019/57956, filed October 24, 2019; U.S. Provisional Application No. 62/858,958, filed June 7, 2019; International Publication No. PCT/US2019/58678, filed October 29, 2019, the contents of each of which are incorporated herein by reference in their entireties.

In some embodiments, a nucleobase editor converts an A to G. In some embodiments, the nucleobase editor comprises an adenosine deaminase. An "adenosine deaminase" is an enzyme involved in purine metabolism. It is needed for the breakdown of adenosine from food and for the turnover of nucleic acids in tissues. Its primary function in humans is the development and maintenance of the immune system. An adenosine deaminase catalyzes hydrolytic deamination of adenosine (forming inosine, which base pairs as G) in the context of DNA. There are no known adenosine deaminases that act on DNA. Instead, known adenosine deaminase enzymes only act on RNA (tRNA or mRNA). Evolved deoxyadenosine deaminase enzymes that accept DNA substrates and deaminate dA to deoxyinosine have been described, e.g., in PCT Application PCT/US2017/045381, filed August 3, 2017, which published as WO 2018/027078, and PCT Application No. PCT/US2019/033848, which published as WO 2019/226953, each of which is herein incorporated by reference by reference.

Exemplary adenine base editors (ABEs) (or "adenosine base editors") and cytidine base editors (CBEs) (or "cytosine base editors") are also described in Rees & Liu, Base editing: precision chemistry on the genome and transcriptome of living cells, Nat. Rev. Genet. 2018;19(12):770-788; as well as U.S. Patent Publication No. 2018/0073012, published March 15, 2018, which issued as U.S. Patent No. 10,113,163, on October 30, 2018; U.S. Patent Publication No. 2017/0121693, published May 4, 2017, which issued as U.S. Patent No. 10,167,457 on January 1, 2019; International Publication No. WO 2017/070633, published April 27, 2017; U.S. Patent Publication No. 2015/0166980, published June 18, 2015; U.S. Patent No. 9,840,699, issued December 12, 2017; and U.S. Patent No. 10,077,453, issued September 18, 2018, the contents of each of which are incorporated herein by reference in their entireties.

### Cas9

The term "Cas9" or "Cas9 nuclease" refers to an RNA-guided nuclease comprising a Cas9 domain, or a fragment thereof (*e.g.,* a protein comprising an active or inactive DNA cleavage domain of Cas9, and/or the gRNA binding domain of Cas9). A "Cas9 domain" as used herein, is a protein fragment comprising an active or inactive cleavage domain of Cas9 and/or the gRNA binding domain of Cas9. A "Cas9 protein" is a full length Cas9 protein. A Cas9 nuclease is also referred to sometimes as a casn1 nuclease or a CRISPR (Clustered Regularly Interspaced Short Palindromic Repeat)-associated nuclease. CRISPR is an adaptive immune system that provides protection against mobile genetic elements (viruses, transposable elements, and conjugative plasmids). CRISPR clusters contain spacers, sequences complementary to antecedent mobile elements, and target invading nucleic acids. CRISPR clusters are transcribed and processed into CRISPR RNA (crRNA). In type II CRISPR systems correct processing of pre-crRNA requires a trans-encoded small RNA (tracrRNA), endogenous ribonuclease 3 (rnc) and a Cas9 domain. The tracrRNA serves as a guide for ribonuclease 3-aided processing of pre-crRNA. Subsequently, Cas9/crRNA/tracrRNA endonucleolytically cleaves a linear or circular dsDNA target complementary to the spacer. The target strand not complementary to crRNA is first cut endonucleolytically, then trimmed 3'-5' exonucleolytically. In nature, DNA-binding and cleavage typically requires protein and both RNAs. However, single guide RNAs ("sgRNA", or simply "gNRA") can be engineered so as to incorporate aspects of both the crRNA and tracrRNA into a single RNA species. See, *e.g.,* Jinek M., Chylinski K., Fonfara I., Hauer M., Doudna J.A., Charpentier E. Science 337:816-821(2012), the entire contents of which are hereby incorporated by reference. Cas9 recognizes a short motif in the CRISPR repeat sequences (the PAM or protospacer adjacent motif) to help distinguish self versus non-self. Cas9 nuclease sequences and structures are well known to those of skill in the art (see, *e.g.,* "Complete genome sequence of an M1 strain of Streptococcus pyogenes." Ferretti et al., J.J., McShan W.M., Ajdic D.J., Savic D.J., Savic G., Lyon K., Primeaux C., Sezate S., Suvorov A.N., Kenton S., Lai H.S., Lin S.P., Qian Y., Jia H.G., Najar F.Z., Ren Q., Zhu H., Song L., White J., Yuan X., Clifton S.W., Roe B.A., McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma C.M., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012), the entire contents of each of which are incorporated herein by reference). Cas9 orthologs have been described in various species, including, but not limited to, *S. pyogenes* and *S. thermophilus.* Additional suitable Cas9 nucleases and sequences will be apparent to those of skill in the art based on this disclosure, and such Cas9 nucleases and sequences include Cas9 sequences from the organisms and loci disclosed in Chylinski, Rhun, and Charpentier, "The tracrRNA and Cas9 families of type II CRISPR-Cas immunity systems" (2013) RNA Biology 10:5, 726-737; the entire contents of which are incorporated herein by reference. In some embodiments, a Cas9 nuclease comprises one or more mutations that partially impair or inactivate the DNA cleavage domain.

A nuclease-inactivated Cas9 domain may interchangeably be referred to as a "dCas9" protein (for nuclease-"dead" Cas9). Methods for generating a Cas9 domain (or a fragment thereof) having an inactive DNA cleavage domain are known (see, *e.g.,* Jinek et al., Science. 337:816-821(2012); Qi et al., "Repurposing CRISPR as an RNA-Guided Platform for Sequence-Specific Control of Gene Expression" (2013) Cell. 28;152(5):1173-83, the entire contents of each of which are incorporated herein by reference). For example, the DNA cleavage domain of Cas9 is known to include two subdomains, the HNH nuclease subdomain and the RuvC1 subdomain. The HNH subdomain cleaves the strand complementary to the gRNA, whereas the RuvC1 subdomain cleaves the non-complementary strand. Mutations within these subdomains can silence the nuclease activity of Cas9. For example, the mutations D10A and H840A completely inactivate the nuclease activity of *S. pyogenes* Cas9 (Jinek et al., Science. 337:816-821(2012); Qi et al., Cell. 28;152(5):1173-83 (2013)). In some embodiments, proteins comprising fragments of Cas9 are provided. For example, in some embodiments, a protein comprises one of two Cas9 domains: (1) the gRNA binding domain of Cas9; or (2) the DNA cleavage domain of Cas9. In some embodiments, proteins comprising Cas9 or fragments thereof are referred to as "Cas9 variants." A Cas9 variant shares homology to Cas9, or a fragment thereof. For example, a Cas9 variant is at least about 70% identical, at least about 80% identical, at least about 90% identical, at least about 95% identical, at least about 96% identical, at least about 97% identical, at least about 98% identical, at least about 99% identical, at least about 99.5% identical, at least about 99.8% identical, or at least about 99.9% identical to wild type Cas9 (e.g., SpCas9 of SEQ ID NO: 5). In some embodiments, the Cas9 variant may have 1, 2, 3, 4, 5, 6, 7, 8, 9, 10, 11, 12, 13, 14, 15, 16, 17, 18, 19, 20, 21, 22, 21, 24, 25, 26, 27, 28, 29, 30, 31, 32, 33, 34, 35, 36, 37, 38, 39, 40, 41, 42, 43, 44, 45, 46, 47, 48, 49, 50, or more amino acid changes compared to wild type Cas9 (e.g., SpCas9 of SEQ ID NO: 5). In some embodiments, the Cas9 variant comprises a fragment of Cas9 (*e.g.,* a gRNA binding domain or a DNA-cleavage domain), such that the fragment is at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to the corresponding fragment of wild type Cas9 (e.g., SpCas9 of SEQ ID NO: 5). In some embodiments, the fragment is at least 30%, at least 35%, at least 40%, at least 45%, at least 50%, at least 55%, at least 60%, at least 65%, at least 70%, at least 75%, at least 80%, at least 85%, at least 90%, at least 95% identical, at least 96%, at least 97%, at least 98%, at least 99%, or at least 99.5% of the amino acid length of a corresponding wild type Cas9 (e.g., SpCas9 of SEQ ID NO: 5).

As used herein, the term "nCas9" or "Cas9 nickase" refers to a Cas9 or a variant thereof, which cleaves or nicks only one of the strands of a target cut site thereby introducing a nick in a double strand DNA molecule rather than creating a double strand break. This can be achieved by introducing appropriate mutations in a wild-type Cas9 which inactivates one of the two endonuclease activities of the Cas9. Any suitable mutation which inactivates one Cas9 endonuclease activity but leaves the other intact is contemplated, such as one of D10A or H840A mutations in the wild-type *S. pyogenes* Cas9 amino acid sequence, or a D10A mutation in the wild-type *S. aureus* Cas9 amino acid sequence, may be used to form the nCas9.

### cDNA

The term "cDNA" refers to a strand of DNA copied from an RNA template. cDNA is complementary to the RNA template.

### Circular permutant

As used herein, the term "circular permutant" refers to a protein or polypeptide (e.g., a Cas9) comprising a circular permutation, which is change in the protein's structural configuration involving a change in order of amino acids appearing in the protein's amino acid sequence. In other words, circular permutants are proteins that have altered N- and C-termini as compared to a wild-type counterpart, *e.g*., the wild-type C-terminal half of a protein becomes the new N-terminal half. Circular permutation (or CP) is essentially the topological rearrangement of a protein's primary sequence, connecting its N- and C-terminus, often with a peptide linker, while concurrently splitting its sequence at a different position to create new, adjacent N- and C-termini. The result is a protein structure with different connectivity, but which often can have the same overall similar three-dimensional (3D) shape, and possibly include improved or altered characteristics, including, reduced proteolytic susceptibility, improved catalytic activity, altered substrate or ligand binding, and/or improved thermostability. Circular permutant proteins can occur in nature (*e.g*., concanavalin A and lectin). In addition, circular permutation can occur as a result of posttranslational modifications or may be engineered using recombinant techniques (*e.g*., see, Oakes et al., "Protein Engineering of Cas9 for enhanced function," Methods Enzymol, 2014, 546: 491-511 and Oakes et al., "CRISPR-Cas9 Circular Permutants as Programmable Scaffolds for Genome Modification," Cell, January 10, 2019, 176: 254-267, each of are incorporated herein by reference).

### Circularly permuted napDNAbp

The term "circularly permuted napDNAbp" refers to any napDNAbp protein, or variant thereof (*e.g*., SpCas9), that occurs as or engineered as a circular permutant, whereby its N- and C-termini have been topically rearranged. Such circularly permuted proteins ("CP-napDNAbp", such as "CP-Cas9" in the case of Cas9), or variants thereof, retain the ability to bind DNA when complexed with a guide RNA (gRNA). See, Oakes et al., "Protein Engineering of Cas9 for enhanced function," Methods Enzymol, 2014, 546: 491-511 and Oakes et al., "CRISPR-Cas9 Circular Permutants as Programmable Scaffolds for Genome Modification," Cell, January 10, 2019, 176: 254-267, each of are incorporated herein by reference. The instant disclosure contemplates any previously known CP-Cas9 or use a new CP-Cas9 so long as the resulting circularly permuted protein retains the ability to bind DNA when complexed with a guide RNA (gRNA). Exemplary CP-Cas9 proteins are SEQ ID NOs: 64-68.

### Cytidine deaminase (or cytosine deaminase)

As used herein, the term "cytidine deaminase" or "cytidine deaminase domain" refers to a protein or enzyme that catalyzes a deamination reaction of a cytidine or cytosine. The terms "cytidine" and "cytosine" are used interchangeably for purposes of the present disclosure. For example, for purposes of the disclosure, reference to an "cytidine base editor" (CBE) refers to the same entity as an "cytosine base editor" (CBE). Similarly, for purposes of the disclosure, reference to an "cytidine deaminase" refers to the same entity as an "cytosine deaminase." However, the person having ordinary skill in the art will appreciate that "cytosine" refers to the pyrimidine base whereas "cytidine" refers to the larger nucleoside molecule that includes the pyrimidine base (cytosine) and sugar moiety (e.g., either ribose or deoxyribose). A cytidine deaminase is encoded by the CDA gene and is an enzyme that catalyzes the removal of an amine group from cytidine (i.e., the base cytosine when attached to a ribose ring, i.e., the nucleoside referred to as cytidine) to uridine (C to U) and deoxycytidine to deoxyuridine (C to U). A non-limiting example of a cytidine deaminase is APOBEC1 ("apolipoprotein B mRNA editing enzyme, catalytic polypeptide 1"). Another example is AID ("activation-induced cytidine deaminase"). Under standard Watson-Crick hydrogen bond pairing, a cytosine base hydrogen bonds to a guanine base. When cytidine is converted to uridine (or deoxycytidine is converted to deoxyuridine), the uridine (or the uracil base of uridine) undergoes hydrogen bond pairing with the base adenine. Thus, a conversion of "C" to uridine ("U") by cytidine deaminase will cause the insertion of "A" instead of a "G" during cellular repair and/or replication processes. Since the adenine "A" pairs with thymine "T", the cytidine deaminase in coordination with DNA replication causes the conversion of an C·G pairing to a T·A pairing in the double-stranded DNA molecule.

### CRISPR

CRISPR is a family of DNA sequences (*i.e.,* CRISPR clusters) in bacteria and archaea that represent snippets of prior infections by a virus that have invaded the prokaryote. The snippets of DNA are used by the prokaryotic cell to detect and destroy DNA from subsequent attacks by similar viruses and effectively compose, along with an array of CRISPR-associated proteins (including Cas9 and homologs thereof) and CRISPR-associated RNA, a prokaryotic immune defense system. In nature, CRISPR clusters are transcribed and processed into CRISPR RNA (crRNA). In certain types of CRISPR systems (*e.g*., type II CRISPR systems), correct processing of pre-crRNA requires a trans-encoded small RNA (tracrRNA), endogenous ribonuclease 3 (rnc) and a Cas9 protein. The tracrRNA serves as a guide for ribonuclease 3-aided processing of pre-crRNA. Subsequently, Cas9/crRNA/tracrRNA endonucleolytically cleaves linear or circular dsDNA target complementary to the RNA. Specifically, the target strand not complementary to crRNA is first cut endonucleolytically, then trimmed 3'-5' exonucleolytically. In nature, DNA-binding and cleavage typically requires protein and both RNAs. However, single guide RNAs ("sgRNA", or simply "gRNA") can be engineered so as to incorporate aspects of both the crRNA and tracrRNA into a single RNA species - the guide RNA. See, *e.g*., Jinek M., Chylinski K., Fonfara I., Hauer M., Doudna J.A., Charpentier E. Science 337:816-821(2012), the entire contents of which is hereby incorporated by reference. Cas9 recognizes a short motif in the CRISPR repeat sequences (the PAM or protospacer adjacent motif) to help distinguish self versus non-self. CRISPR biology, as well as Cas9 nuclease sequences and structures are well known to those of skill in the art (see, *e.g.,* "Complete genome sequence of an M1 strain of Streptococcus pyogenes." Ferretti et al., J.J., McShan W.M., Ajdic D.J., Savic D.J., Savic G., Lyon K., Primeaux C., Sezate S., Suvorov A.N., Kenton S., Lai H.S., Lin S.P., Qian Y., Jia H.G., Najar F.Z., Ren Q., Zhu H., Song L., White J., Yuan X., Clifton S.W., Roe B.A., McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma C.M., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012), the entire contents of each of which are incorporated herein by reference). Cas9 orthologs have been described in various species, including, but not limited to, *S. pyogenes* and *S. thermophilus.* Additional suitable Cas9 nucleases and sequences will be apparent to those of skill in the art based on this disclosure, and such Cas9 nucleases and sequences include Cas9 sequences from the organisms and loci disclosed in Chylinski, Rhun, and Charpentier, "The tracrRNA and Cas9 families of type II CRISPR-Cas immunity systems" (2013) RNA Biology 10:5, 726-737; the entire contents of which are incorporated herein by reference.

In certain types of CRISPR systems (e.g., type II CRISPR systems), correct processing of pre-crRNA requires a trans-encoded small RNA (tracrRNA), endogenous ribonuclease 3 (rnc), and a Cas9 protein. The tracrRNA serves as a guide for ribonuclease 3-aided processing of pre-crRNA. Subsequently, Cas9/crRNA/tracrRNA endonucleolytically cleaves a linear or circular nucleic acid target complementary to the RNA. Specifically, the target strand not complementary to crRNA is first cut endonucleolytically, then trimmed 3'-5' exonucleolytically. In nature, DNA-binding and cleavage typically requires protein and both RNAs. However, single guide RNAs ("sgRNA", or simply "gRNA") can be engineered to incorporate embodiments of both the crRNA and tracrRNA into a single RNA species-the guide RNA.

In general, a "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. The tracrRNA of the system is complementary (fully or partially) to the tracr mate sequence present on the guide RNA.

### Deaminase

The term "deaminase" or "deaminase domain" refers to a protein or enzyme that catalyzes a deamination reaction. In some embodiments, the deaminase is an adenosine (or adenine) deaminase, which catalyzes the hydrolytic deamination of adenine or adenosine. In some embodiments, the adenosine deaminase catalyzes the hydrolytic deamination of adenine or adenosine in deoxyribonucleic acid (DNA) to inosine. In other embodiments, the deminase is a cytidine (or cytosine) deaminase, which catalyzes the hydrolytic deamination of cytidine or cytosine.

The deaminases provided herein may be from any organism, such as a bacterium. In some embodiments, the deaminase or deaminase domain is a variant of a naturally-occurring deaminase from an organism. In some embodiments, the deaminase or deaminase domain does not occur in nature. For example, in some embodiments, the deaminase or deaminase domain is at least 50%, at least 55%, at least 60%, at least 65%, at least 70%, at least 75% at least 80%, at least 85%, at least 90%, at least 95%, at least 96%, at least 97%, at least 98%, at least 99%, or at least 99.5% identical to a naturally-occurring deaminase.

### Degron

The term "degron" or "degron domain" refers to a portion of a polypeptide that influence, controls, directs, or otherwise regulates the rate of degradation of the polypeptide. Degrons can be highly variable and can include short amino acid sequences, structural motifs, and/or exposed amino acids. Also, degrons may be positioned at any location within a polypeptide (*e.g*., at the N-terminus, the C-terminus, or at an internal position within the primary structure). The particular mechanism of degradation of a polypeptide which is regulated by the degron is not limited and can include ubiquitin-dependent degradation (*i.e.,* degradation that involves proteasomal-based degradation) or ubiquitin-independent degradation. For example, the 4-amino acid sequence tail of NH₃-EMLA (SEQ ID NO: 466) -COOH encoded by exon 8 of the *SMN2* gene functions as a degron, triggering degradation of SMN2.

### DNA Binding Protein

As used herein, the term "DNA binding protein" or "DNA binding protein domain" refers to any protein that localizes to and binds a specific target DNA nucleotide sequence (*e.g.* a gene locus of a genome). This term embraces RNA-programmable proteins, which associate (*e.g.* form a complex) with one or more nucleic acid molecules (*i.e.,* which includes, for example, guide RNA in the case of Cas systems) that direct or otherwise program the protein to localize to a specific target nucleotide sequence (*e.g*., DNA sequence) that is complementary to the one or more nucleic acid molecules (or a portion or region thereof) associated with the protein. Exemplary RNA-programmable proteins are CRISPR-Cas9 proteins, as well as Cas9 equivalents, homologs, orthologs, or paralogs, whether naturally occurring or non-naturally occurring (*e.g*. engineered or modified), and may include a Cas9 equivalent from any type of CRISPR system (*e.g*. type II, V, VI), including Cpfl (a type-V CRISPR-Cas systems), C2c1 (a type V CRISPR-Cas system), C2c2 (a type VI CRISPR-Cas system), C2c3 (a type V CRISPR-Cas system), dCas9, GeoCas9, CjCas9, Cas12a, Cas12b, Cas12c, Cas12d, Cas12g, Cas12h, Cas12i, Cas13d, Cas14, Argonaute, and nCas9. Further Cas-equivalents are described in Makarova et al., "C2c2 is a single-component programmable RNA-guided RNA-targeting CRISPR effector," Science 2016; 353(6299), the contents of which are incorporated herein by reference.

### DNA Editing Efficiency

The term "DNA editing efficiency," as used herein, refers to the number or proportion of intended base pairs that are edited. For example, if a base editor edits 10% of the base pairs that it is intended to target (*e.g*., within a cell or within a population of cells), then the base editor can be described as being 10% efficient. Some aspects of editing efficiency embrace the modification (*e.g*. deamination) of a specific nucleotide within DNA, without generating a large number or percentage of insertions or deletions (*i.e.*, indels). It is generally accepted that editing while generating less than 5% indels (as measured over total target nucleotide substrates) is high editing efficiency. The generation of more than 20% indels is generally accepted as poor or low editing efficiency. Indel formation may be measured by techniques known in the art, including high-throughput screening of sequencing reads.

### Downstream

As used herein, the terms "upstream" and "downstream" are terms of relativety that define the linear position of at least two elements located in a nucleic acid molecule (whether single or double-stranded) that is orientated in a 5'-to-3' direction. In particular, a first element is upstream of a second element in a nucleic acid molecule where the first element is positioned somewhere that is 5 ' to the second element. For example, a SNP is upstream of a Cas9-induced nick site if the SNP is on the 5 ' side of the nick site. Conversely, a first element is downstream of a second element in a nucleic acid molecule where the first element is positioned somewhere that is 3' to the second element. For example, a SNP is downstream of a Cas9-induced nick site if the SNP is on the 3' side of the nick site. The nucleic acid molecule can be a DNA (double or single stranded). RNA (double or single stranded), or a hybrid of DNA and RNA. The analysis is the same for single strand nucleic acid molecule and a double strand molecule since the terms upstream and downstream are in reference to only a single strand of a nucleic acid molecule, except that one needs to select which strand of the double stranded molecule is being considered. Often, the strand of a double stranded DNA which can be used to determine the positional relativity of at least two elements is the "sense" or "coding" strand. In genetics, a "sense" strand is the segment within double-stranded DNA that runs from 5' to 3', and which is complementary to the antisense strand of DNA, or template strand, which runs from 3' to 5'. Thus, as an example, a SNP nucleobase is "downstream" of a promoter sequence in a genomic DNA (which is double-stranded) if the SNP nucleobase is on the 3' side of the promoter on the sense or coding strand.

### Effective Amount

The term "effective amount," as used herein, refers to an amount of a biologically active agent that is sufficient to elicit a desired biological response. For example, in some embodiments, an effective amount of a base editor may refer to the amount of the editor that is sufficient to edit a target site nucleotide sequence, *e.g.,* a genome. In some embodiments, an effective amount of a base editor provided herein, *e.g.,* of a fusion protein comprising a nickase Cas9 domain and a guide RNA may refer to the amount of the fusion protein that is sufficient to induce editing of a target site specifically bound and edited by the fusion protein. As will be appreciated by the skilled artisan, the effective amount of an agent, *e.g.,* a fusion protein, a nuclease, a hybrid protein, a protein dimer, a complex of a protein (or protein dimer) and a polynucleotide, or a polynucleotide, may vary depending on various factors as, for example, on the desired biological response, e.g., on the specific allele, genome, or target site to be edited, on the cell or tissue being targeted, and on the agent being used.

### Functional Equivalent

The term "functional equivalent" refers to a second biomolecule that is equivalent in function, but not necessarily equivalent in structure to a first biomolecule. For example, a "Cas9 equivalent" refers to a protein that has the same or substantially the same functions as Cas9, but not necessarily the same amino acid sequence. In the context of the disclosure, the specification refers throughout to "a protein X, or a functional equivalent thereof." In this context, a "functional equivalent" of protein X embraces any homolog, paralog, fragment, naturally occurring, engineered, circular permutant, mutated, or synthetic version of protein X which bears an equivalent function.

### Fusion Protein

The term "fusion protein" as used herein refers to a hybrid polypeptide which comprises protein domains from at least two different proteins. One protein may be located at the amino-terminal (N-terminal) portion of the fusion protein or at the carboxy-terminal (C-terminal) protein thus forming an "amino-terminal fusion protein" or a "carboxy-terminal fusion protein," respectively. A protein may comprise different domains, for example, a nucleic acid binding domain (*e.g*., the gRNA binding domain of Cas9 that directs the binding of the protein to a target site) and a nucleic acid cleavage domain or a catalytic domain of a nucleic-acid editing protein. Another example includes a Cas9 or equivalent thereof fused to an adenosine deaminae. Any of the proteins provided herein may be produced by any method known in the art. For example, the proteins provided herein may be produced via recombinant protein expression and purification, which is especially suited for fusion proteins comprising a peptide linker. Methods for recombinant protein expression and purification are well known, and include those described by Green and Sambrook, Molecular Cloning: A Laboratory Manual (4th ed., Cold Spring Harbor Laboratory Press, Cold Spring Harbor, N.Y. (2012)), the entire contents of which are incorporated herein by reference.

### Guide Nucleic Acid

The term "guide nucleic acid" or "napDNAbp-programming nucleic acid molecule" or equivalently "guide sequence" refers the one or more nucleic acid molecules which associate with and direct or otherwise program a napDNAbp protein to localize to a specific target nucleotide sequence (*e.g.,* a gene locus of a genome) that is complementary to the one or more nucleic acid molecules (or a portion or region thereof) associated with the protein, thereby causing the napDNAbp protein to bind to the nucleotide sequence at the specific target site. A non-limiting example is a guide RNA of a Cas protein of a CRISPR-Cas genome editing system.

Guide RNA is a particular type of guide nucleic acid which is mostly commonly associated with a Cas protein of a CRISPR-Cas9 and which associates with Cas9, directing the Cas9 protein to a specific sequence in a DNA molecule that includes complementarity to protospace sequence of the guide RNA. As used herein, a "guide RNA" refers to a synthetic fusion of the endogenous bacterial crRNA and tracrRNA that provides both targeting specificity and scaffolding and/or binding ability for Cas9 nuclease to a target DNA. This synthetic fusion does not exist in nature and is also commonly referred to as an sgRNA. However, this term also embraces the equivalent guide nucleic acid molecules that associate with Cas9 equivalents, homologs, orthologs, or paralogs, whether naturally occurring or non-naturally occurring (*e.g*., engineered or recombinant), and which otherwise program the Cas9 equivalent to localize to a specific target nucleotide sequence. The Cas9 equivalents may include other napDNAbp from any type of CRISPR system (*e.g*., type II, V, VI), including Cpfl (a type-V CRISPR-Cas systems), C2c1 (a type V CRISPR-Cas system), C2c2 (a type VI CRISPR-Cas system) and C2c3 (a type V CRISPR-Cas system). Further Cas-equivalents are described in Makarova et al., "C2c2 is a single-component programmable RNA-guided RNA-targeting CRISPR effector," Science 2016; 353(6299), the contents of which are incorporated herein by reference. Exemplary sequences are and structures of guide RNAs are provided herein. In addition, methods for designing appropriate guide RNA sequences are provided herein.

### Guide RNA ("gRNA")

As used herein, the term "guide RNA" is a particular type of guide nucleic acid which is mostly commonly associated with a Cas protein of a CRISPR-Cas9 and which associates with Cas9, directing the Cas9 protein to a specific sequence in a DNA molecule that includes complementarity to protospacer sequence of the guide RNA. However, this term also embraces the equivalent guide nucleic acid molecules that associate with Cas9 equivalents, homologs, orthologs, or paralogs, whether naturally occurring or non-naturally occurring (e.g., engineered or recombinant), and which otherwise program the Cas9 equivalent to localize to a specific target nucleotide sequence. The Cas9 equivalents may include other napDNAbp from any type of CRISPR system (e.g., type II, V, VI), including Cpfl (a type-V CRISPR-Cas systems), C2c1 (a type V CRISPR-Cas system), C2c2 (a type VI CRISPR-Cas system) and C2c3 (a type V CRISPR-Cas system). Further Cas-equivalents are described in Makarova et al., "C2c2 is a single-component programmable RNA-guided RNA-targeting CRISPR effector," Science 2016; 353(6299), the contents of which are incorporated herein by reference. Exemplary sequences are and structures of guide RNAs are provided herein.

Guide RNAs may comprise various structural elements that include, but are not limited to (a) a spacer sequence - the sequence in the guide RNA (having ~20 nts in length) which binds to a complementary strand of the target DNA (and has the same sequence as the protospacer of the DNA) and (b) a gRNA core (or gRNA scaffold or backbone sequence) - refers to the sequence within the gRNA that is responsible for Cas9 binding, it does not include the ~20 bp spacer sequence that is used to guide Cas9 to target DNA.

### Guide RNA Target Sequence

As used herein, the "guide RNA target sequence" refers to the ~20 nucleotides that are complementary to the protospacer sequence in the PAM strand. The target sequence is the sequence that anneals to or is targeted by the spacer sequence of the guide RNA. The spacer sequence of the guide RNA and the protospacer have the same sequence (except the spacer sequence is RNA and the protospacer is DNA).

### Guide RNA Scaffold Sequence

As used herein, the "guide RNA scaffold sequence" refers to the sequence within the gRNA that is responsible for Cas9 binding, it does not include the 20 bp spacer/targeting sequence that is used to guide Cas9 to target DNA.

### Host Cell

The term "host cell," as used herein, refers to a cell that can host, replicate, and transfer a phage vector useful for a continuous evolution process as provided herein. In embodiments where the vector is a viral vector, a suitable host cell is a cell that may be infected by the viral vector, can replicate it, and can package it into viral particles that can infect fresh host cells. A cell can host a viral vector if it supports expression of genes of viral vector, replication of the viral genome, and/or the generation of viral particles. One criterion to determine whether a cell is a suitable host cell for a given viral vector is to determine whether the cell can support the viral life cycle of a wild-type viral genome that the viral vector is derived from. For example, if the viral vector is a modified M13 phage genome, as provided in some embodiments described herein, then a suitable host cell would be any cell that can support the wild-type M13 phage life cycle. Suitable host cells for viral vectors useful in continuous evolution processes are well known to those of skill in the art, and the disclosure is not limited in this respect. In some embodiments, the viral vector is a phage and the host cell is a bacterial cell. In some embodiments, the host cell is an *E. coli* cell. Suitable *E. coli* host strains will be apparent to those of skill in the art, and include, but are not limited to, New England Biolabs (NEB) Turbo, Top10F', DH12S, ER2738, ER2267, and XL1-Blue MRF'. These strain names are art recognized and the genotype of these strains has been well characterized. It should be understood that the above strains are exemplary only and that the invention is not limited in this respect. The term "fresh," as used herein interchangeably with the terms "non-infected" or "uninfected" in the context of host cells, refers to a host cell that has not been infected by a viral vector comprising a gene of interest as used in a continuous evolution process provided herein. A fresh host cell can, however, have been infected by a viral vector unrelated to the vector to be evolved or by a vector of the same or a similar type but not carrying the gene of interest.

In some embodiments, the host cell is a prokaryotic cell, for example, a bacterial cell. In some embodiments, the host cell is an *E. coli* cell. In some embodiments, the host cell is a eukaryotic cell, for example, a yeast cell, an insect cell, or a mammalian cell. The type of host cell, will, of course, depend on the viral vector employed, and suitable host cell/viral vector combinations will be readily apparent to those of skill in the art.

### Inteins and split-inteins

As used herein, the term "intein" refers to auto-processing polypeptide domains found in organisms from all domains of life. An intein (intervening *protein*) carries out a unique auto-processing event known as protein splicing in which it excises itself out from a larger precursor polypeptide through the cleavage of two peptide bonds and, in the process, ligates the flanking extein (external protein) sequences through the formation of a new peptide bond. This rearrangement occurs post-translationally (or possibly co-translationally), as intein genes are found embedded in frame within other protein-coding genes. Furthermore, intein-mediated protein splicing is spontaneous; it requires no external factor or energy source, only the folding of the intein domain. This process is also known as *cis*-protein splicing, as opposed to the natural process of *trans*-protein splicing with "split inteins."

Split inteins are a sub-category of inteins. Unlike the more common contiguous inteins, split inteins are transcribed and translated as two separate polypeptides, the N-intein and C-intein, each fused to one extein. Upon translation, the intein fragments spontaneously and non-covalently assemble into the canonical intein structure to carry out protein splicing *in trans.*

Inteins and split inteins are the protein equivalent of the self-splicing RNA introns (see Perler et al., Nucleic Acids Res. 22:1125-1127 (1994)), which catalyze their own excision from a precursor protein with the concomitant fusion of the flanking protein sequences, known as exteins (reviewed in Perler et al., Curr. Opin. Chem. Biol. 1:292-299 (1997); Perler, F. B. Cell 92(1):1-4 (1998); Xu et al., EMBO J. 15(19):5146-5153 (1996)).

As used herein, the term "protein splicing" refers to a process in which an interior region of a precursor protein (an intein) is excised and the flanking regions of the protein (exteins) are ligated to form the mature protein. This natural process has been observed in numerous proteins from both prokaryotes and eukaryotes (Perler, F. B., Xu, M. Q., Paulus, H. Current Opinion in Chemical Biology 1997, 1, 292-299; Perler, F. B. Nucleic Acids Research 1999, 27, 346-347). The intein unit contains the necessary components needed to catalyze protein splicing and often contains an endonuclease domain that participates in intein mobility (Perler, F. B., Davis, E. O., Dean, G. E., Gimble, F. S., Jack, W. E., Neff, N., Noren, C. J., Thomer, J., Belfort, M. Nucleic Acids Research 1994, 22, 1127-1127). The resulting proteins are linked, however, not expressed as separate proteins. Protein splicing may also be conducted in *trans* with split inteins expressed on separate polypeptides spontaneously combine to form a single intein which then undergoes the protein splicing process to join to separate proteins.

The elucidation of the mechanism of protein splicing has led to a number of intein-based applications (Comb, et al., U.S. Pat. No. 5,496,714; Comb, et al., U.S. Pat. No. 5,834,247; Camarero and Muir, J. Amer. Chem. Soc., 121:5597-5598 (1999); Chong, et al., Gene, 192:271-281 (1997), Chong, et al., Nucleic Acids Res., 26:5109-5115 (1998); Chong, et al., J. Biol. Chem., 273:10567-10577 (1998); Cotton, et al. J. Am. Chem. Soc., 121:1100-1101 (1999); Evans, et al., J. Biol. Chem., 274:18359-18363 (1999); Evans, et al., J. Biol. Chem., 274:3923-3926 (1999); Evans, et al., Protein Sci., 7:2256-2264 (1998); Evans, et al., J. Biol. Chem., 275:9091-9094 (2000); Iwai and Pluckthun, FEBS Lett. 459:166-172 (1999); Mathys, et al., Gene, 231:1-13 (1999); Mills, et al., Proc. Natl. Acad. Sci. USA 95:3543-3548 (1998); Muir, et al., Proc. Natl. Acad. Sci. USA 95:6705-6710 (1998); Otomo, et al., Biochemistry 38:16040-16044 (1999); Otomo, et al., J. Biolmol. NMR 14:105-114 (1999); Scott, et al., Proc. Natl. Acad. Sci. USA 96:13638-13643 (1999); Severinov and Muir, J. Biol. Chem., 273:16205-16209 (1998); Shingledecker, et al., Gene, 207:187-195 (1998); Southworth, et al., EMBO J. 17:918-926 (1998); Southworth, et al., Biotechniques, 27:110-120 (1999); Wood, et al., Nat. Biotechnol., 17:889-892 (1999); Wu, et al., Proc. Natl. Acad. Sci. USA 95:9226-9231 (1998a); Wu, et al., Biochim Biophys Acta 1387:422-432 (1998b); Xu, et al., Proc. Natl. Acad. Sci. USA 96:388-393 (1999); Yamazaki, et al., J. Am. Chem. Soc., 120:5591-5592 (1998)). Each reference is incorporated herein by reference.

### Ligand-dependent intein

The term "ligand-dependent intein," as used herein refers to an intein that comprises a ligand-binding domain. Typically, the ligand-binding domain is inserted into the amino acid sequence of the intein, resulting in a structure intein (N) - ligand-binding domain - intein (C). Typically, ligand-dependent inteins exhibit no or only minimal protein splicing activity in the absence of an appropriate ligand, and a marked increase of protein splicing activity in the presence of the ligand. In some embodiments, the ligand-dependent intein does not exhibit observable splicing activity in the absence of ligand but does exhibit splicing activity in the presence of the ligand. In some embodiments, the ligand-dependent intein exhibits an observable protein splicing activity in the absence of the ligand, and a protein splicing activity in the presence of an appropriate ligand that is at least 5 times, at least 10 times, at least 50 times, at least 100 times, at least 150 times, at least 200 times, at least 250 times, at least 500 times, at least 1000 times, at least 1500 times, at least 2000 times, at least 2500 times, at least 5000 times, at least 10000 times, at least 20000 times, at least 25000 times, at least 50000 times, at least 100000 times, at least 500000 times, or at least 1000000 times greater than the activity observed in the absence of the ligand. In some embodiments, the increase in activity is dose dependent over at least 1 order of magnitude, at least 2 orders of magnitude, at least 3 orders of magnitude, at least 4 orders of magnitude, or at least 5 orders of magnitude, allowing for fine-tuning of intein activity by adjusting the concentration of the ligand. Suitable ligand-dependent inteins are known in the art, and in include those provided below and those described in published U.S. Patent Application U.S. 2014/0065711 A1; Mootz et al., "Protein splicing triggered by a small molecule." J. Am. Chem. Soc. 2002; 124, 9044-9045; Mootz et al., "Conditional protein splicing: a new tool to control protein structure and function in vitro and in vivo." J. Am. Chem. Soc. 2003; 125, 10561-10569; Buskirk et al., Proc. Natl. Acad. Sci. USA. 2004; 101, 10505-10510); Skretas & Wood, "Regulation of protein activity with small-molecule-controlled inteins." Protein Sci. 2005; 14, 523-532; Schwartz, et al., "Post-translational enzyme activation in an animal via optimized conditional protein splicing." Nat. Chem. Biol. 2007; 3, 50-54; Peck et al., Chem. Biol. 2011; 18 (5), 619-630; the entire contents of each are hereby incorporated by reference. Exemplary sequences are as follows:

| NAME | SEQUENCE OF LIGAND-DEPENDENT INTEIN |
|---|---|
| 2-4 INTEIN: | CLAEGTRIFDPVTGTTHRIEDVVDGRKPIHVVAAAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 164) |
| 3-2 INTEIN | CLAEGTRIFDPVTGTTHRIEDVVDGRKPIHVVAVAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII |
| | LLNSGVYTFLSSTLKSLEEKDHIHRALDKITDTLIHLMAKAGLTLQQQHQR LAQLLLILSHIRHMSNKGMEHLYSMKYTNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 165) |
| 30R3-1 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPIPYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC (SEQ ID NO: 166) |
| 30R3-2 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 167) |
| 30R3-3 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPIPYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 168) |
| 37R3-1 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPILYSEYNPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII |
| | LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC ((SEQ ID NO: 169) |
| 37R3-2 INTEIN | CLAEGTRIFDPVTGTTHRIEDVVDGRKPIHVVAAAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC (SEQ ID NO: 170) |
| 37R3-3 INTEIN | CLAEGTRIFDPVTGTTHRIEDVVDGRKPIHVVAVAKDGTLLARPVVSWFD QGTRDVIGLRIAGGATVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 171) |

### Linker

The term "linker," as used herein, refers to a chemical group or a molecule linking two molecules or domains, *e.g*. dCas9 and a deaminase. Typically, the linker is positioned between, or flanked by, two groups, molecules, or other domains and connected to each one via a covalent bond, thus connecting the two. In some embodiments, the linker is an amino acid or a plurality of amino acids (*e.g.* a peptide or protein). In some embodiments, the linker is an organic molecule, group, polymer, or chemical domain. Chemical groups include, but are not limited to, disulfide, hydrazone, and azide domains. In some embodiments, the linker is 5-100 amino acids in length, for example, 5, 6, 7, 8, 9, 10, 11, 12, 13, 14, 15, 16, 17, 18, 19, 20, 21, 22, 23, 24, 25, 26, 27, 28, 29, 30, 30-35, 35-40, 40-45, 45-50, 50-60, 60-70, 70-80, 80-90, 90-100, 100-150, or 150-200 amino acids in length. Longer or shorter linkers are also contemplated. In some embodiments, the linker is an XTEN linker. In some embodiments, the linker is a 32-amino acid linker. In other embodiments, the linker is a 30-, 31-, 33- or 34-amino acid linker.

### Mutation

The term "mutation," as used herein, refers to a substitution of a residue within a sequence, *e.g*. a nucleic acid or amino acid sequence, with another residue; a deletion or insertion of one or more residues within a sequence; or a substitution of a residue within a sequence of a genome in a subject to be corrected. Mutations are typically described herein by identifying the original residue followed by the position of the residue within the sequence and by the identity of the newly substituted residue. Various methods for making the amino acid substitutions (mutations) provided herein are well known in the art, and are provided by, for example, Green and Sambrook, Molecular Cloning: A Laboratory Manual (4th ed., Cold Spring Harbor Laboratory Press, Cold Spring Harbor, N.Y. (2012)). Mutations can include a variety of categories, such as single base polymorphisms, microduplication regions, indel, and inversions, and is not meant to be limiting in any way. Mutations can include "loss-of-function" mutations which are mutations that reduce or abolish a protein activity. Most loss-of-function mutations are recessive, because in a heterozygote the second chromosome copy carries an unmutated version of the gene coding for a fully functional protein whose presence compensates for the effect of the mutation. There are some exceptions where a loss-of-function mutation is dominant, one example being haploinsufficiency, where the organism is unable to tolerate the approximately 50% reduction in protein activity suffered by the heterozygote. This is the explanation for a few genetic diseases in humans, including Marfan syndrome, which results from a mutation in the gene for the connective tissue protein called fibrillin. Mutations also embrace "gain-of-function" mutations, which is one which confers an abnormal activity on a protein or cell that is otherwise not present in a normal condition. Many gain-of-function mutations are in regulatory sequences rather than in coding regions, and can therefore have a number of consequences. For example, a mutation might lead to one or more genes being expressed in the wrong tissues, these tissues gaining functions that they normally lack. Alternatively the mutation could lead to overexpression of one or more genes involved in control of the cell cycle, thus leading to uncontrolled cell division and hence to cancer. Because of their nature, gain-of-function mutations are usually dominant.

### napDNAbp

The term "napDNAb" which stand for "nucleic acid programmable DNA binding protein" refers to any protein that may associate (*e.g*., form a complex) with one or more nucleic acid molecules (*i.e.,* which may broadly be referred to as a "napDNAbp-programming nucleic acid molecule" and includes, for example, guide RNA in the case of Cas systems) which direct or otherwise program the protein to localize to a specific target nucleotide sequence (*e.g*., a gene locus of a genome) that is complementary to the one or more nucleic acid molecules (or a portion or region thereof) associated with the protein, thereby causing the protein to bind to the nucleotide sequence at the specific target site. This term napDNAbp embraces CRISPR-Cas9 proteins, as well as Cas9 equivalents, homologs, orthologs, or paralogs, whether naturally occurring or non-naturally occurring (*e.g*., engineered or modified), and may include a Cas9 equivalent from any type of CRISPR system (*e.g*., type II, V, VI), including Cpfl (a type-V CRISPR-Cas systems), C2c1 (a type V CRISPR-Cas system), C2c2 (a type VI CRISPR-Cas system), C2c3 (a type V CRISPR-Cas system), dCas9, GeoCas9, CjCas9, Cas12a, Cas12b, Cas12c, Cas12d, Cas12g, Cas12h, Cas12i, Cas13d, Cas14, Argonaute, and nCas9. Further Cas-equivalents are described in Makarova et al., "C2c2 is a single-component programmable RNA-guided RNA-targeting CRISPR effector," Science 2016; 353 (6299), the contents of which are incorporated herein by reference. However, the nucleic acid programmable DNA binding protein (napDNAbp) that may be used in connection with this invention are not limited to CRISPR-Cas systems. The invention embraces any such programmable protein, such as the Argonaute protein from *Natronobacterium gregoryi* (NgAgo) which may also be used for DNA-guided genome editing. NgAgo-guide DNA system does not require a PAM sequence or guide RNA molecules, which means genome editing can be performed simply by the expression of generic NgAgo protein and introduction of synthetic oligonucleotides on any genomic sequence. See Gao et al., DNA-guided genome editing using the Natronobacterium gregoryi Argonaute. Nature Biotechnology 2016; 34(7):768-73, which is incorporated herein by reference.

In some embodiments, the napDNAbp is a RNA-programmable nuclease, when in a complex with an RNA, may be referred to as a nuclease:RNA complex. Typically, the bound RNA(s) is referred to as a guide RNA (gRNA). gRNAs can exist as a complex of two or more RNAs, or as a single RNA molecule. gRNAs that exist as a single RNA molecule may be referred to as single-guide RNAs (sgRNAs), though "gRNA" is used interchangeably to refer to guide RNAs that exist as either single molecules or as a complex of two or more molecules. Typically, gRNAs that exist as single RNA species comprise two domains: (1) a domain that shares homology to a target nucleic acid (*e.g.,* and directs binding of a Cas9 (or equivalent) complex to the target); and (2) a domain that binds a Cas9 protein. In some embodiments, domain (2) corresponds to a sequence known as a tracrRNA, and comprises a stem-loop structure. For example, in some embodiments, domain (2) is homologous to a tracrRNA as depicted in Figure 1E of Jinek et al., Science 337:816-821(2012), the entire contents of which is incorporated herein by reference. Other examples of gRNAs (*e.g*., those including domain 2) can be found in U.S. Patent No. 9,340,799, entitled "mRNA-Sensing Switchable gRNAs," and International Patent Application No. PCT/US2014/054247, filed September 6, 2013, published as WO 2015/035136 and entitled "Delivery System For Functional Nucleases," the entire contents of each are herein incorporated by reference. In some embodiments, a gRNA comprises two or more of domains (1) and (2), and may be referred to as an "extended gRNA." For example, an extended gRNA will, *e.g.,* bind two or more Cas9 proteins and bind a target nucleic acid at two or more distinct regions, as described herein. The gRNA comprises a nucleotide sequence that complements a target site, which mediates binding of the nuclease/RNA complex to said target site, providing the sequence specificity of the nuclease:RNA complex. In some embodiments, the RNA-programmable nuclease is the (CRISPR-associated system) Cas9 endonuclease, for example Cas9 (Csn1) from Streptococcus pyogenes (see, *e.g*., "Complete genome sequence of an M1 strain of Streptococcus pyogenes." Ferretti J.J. et al.., Proc. Natl. Acad. Sci. U.S.A. 98:4658-4663(2001); "CRISPR RNA maturation by trans-encoded small RNA and host factor RNase III." Deltcheva E. et al., Nature 471:602-607(2011); and "A programmable dual-RNA-guided DNA endonuclease in adaptive bacterial immunity." Jinek M. et al., Science 337:816-821(2012), the entire contents of each of which are incorporated herein by reference.

The napDNAbp nucleases (*e.g*., Cas9) use RNA:DNA hybridization to target DNA cleavage sites, these proteins are able to be targeted, in principle, to any sequence specified by the guide RNA. Methods of using napDNAbp nucleases, such as Cas9, for site-specific cleavage (*e.g.,* to modify a genome) are known in the art (see *e.g.,* Cong, L. et al. Multiplex genome engineering using CRISPR/Cas systems. Science 339, 819-823 (2013); Mali, P. et al. RNA-guided human genome engineering via Cas9. Science 339, 823-826 (2013); Hwang, W.Y. et al. Efficient genome editing in zebrafish using a CRISPR-Cas system. Nature Biotechnology 31, 227-229 (2013); Jinek, M. et al. RNA-programmed genome editing in human cells. eLife 2, e00471 (2013); Dicarlo, J.E. et al., Genome engineering in Saccharomyces cerevisiae using CRISPR-Cas systems. Nucleic Acid Res. (2013); Jiang, W. et al. RNA-guided editing of bacterial genomes using CRISPR-Cas systems. Nature Biotechnology 31, 233-239 (2013); the entire contents of each of which are incorporated herein by reference).

### Nickase

The term "nickase" refers to a napDNAbp having only a single nuclease activity (e.g., one of the two nuclease domain is inactivated) that cuts only one strand of a target DNA, rather than both strands. Thus, a nickase type napDNAbp does not leave a double-strand break.

### Nuclear localization signal

A nuclear localization signal or sequence (NLS) is an amino acid sequence that tags, designates, or otherwise marks a protein for import into the cell nucleus by nuclear transport. Typically, this signal consists of one or more short sequences of positively charged lysines or arginines exposed on the protein surface. Different nuclear localized proteins may share the same NLS. An NLS has the opposite function of a nuclear export signal (NES), which targets proteins out of the nucleus. Thus, a single nuclear localization signal can direct the entity with which it is associated to the nucleus of a cell. Such sequences may be of any size and composition, for example more than 25, 25, 15, 12, 10, 8, 7, 6, 5, or 4 amino acids, but will preferably comprise at least a four to eight amino acid sequence known to function as a nuclear localization signal (NLS).

### Nucleic acid molecule

The term "nucleic acid molecule" as used herein, refers to RNA as well as single and/or double-stranded DNA. Nucleic acid molecules may be naturally occurring, for example, in the context of a genome, a transcript, an mRNA, tRNA, rRNA, siRNA, snRNA, a plasmid, cosmid, chromosome, chromatid, or other naturally occurring nucleic acid molecule. On the other hand, a nucleic acid molecule may be a non-naturally occurring molecule, *e.g.* a recombinant DNA or RNA, an artificial chromosome, an engineered genome, or fragment thereof, or a synthetic DNA, RNA, DNA/RNA hybrid, or including non-naturally occurring nucleotides or nucleosides. Furthermore, the terms "nucleic acid," "DNA," "RNA," and/or similar terms include nucleic acid analogs, e.g. analogs having other than a phosphodiester backbone. Nucleic acids may be purified from natural sources, produced using recombinant expression systems and optionally purified, chemically synthesized, *etc.* Where appropriate, *e.g.* in the case of chemically synthesized molecules, nucleic acids may comprise nucleoside analogs such as analogs having chemically modified bases or sugars, and backbone modifications. A nucleic acid sequence is presented in the 5' to 3' direction unless otherwise indicated. In some embodiments, a nucleic acid is or comprises natural nucleosides (*e.g*. adenosine, thymidine, guanosine, cytidine, uridine, deoxyadenosine, deoxythymidine, deoxyguanosine, and deoxycytidine); nucleoside analogs (*e.g.* 2-aminoadenosine, 2-thiothymidine, inosine, pyrrolo-pyrimidine, 3-methyl adenosine, 5-methylcytidine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, inosinedenosine, 8-oxoguanosine, O(6)-methylguanine, and 2-thiocytidine); chemically modified bases; biologically modified bases (*e.g*. methylated bases); intercalated bases; modified sugars (*e.g*. 2'-fluororibose, ribose, 2'-deoxyribose, arabinose, and hexose); and/or modified phosphate groups (*e.g*. phosphorothioates and 5'-N-phosphoramidite linkages).

### PACE

The term "phage-assisted continuous evolution (PACE)," as used herein, refers to continuous evolution that employs phage as viral vectors. The general concept of PACE technology has been described, for example, in International PCT Application, PCT/US2009/056194, filed September 8, 2009, published as WO 2010/028347 on March 11, 2010; International PCT Application, PCT/US2011/066747, filed December 22, 2011, published as WO 2012/088381 on June 28, 2012; U.S. Application, U.S. Patent No. 9,023,594, issued May 5, 2015, International PCT Application, PCT/US2015/012022, filed January 20, 2015, published as WO 2015/134121 on September 11, 2015, and International PCT Application, PCT/US2016/027795, filed April 15, 2016, published as WO 2016/168631 on October 20, 2016, the entire contents of each of which are incorporated herein by reference.

### Promoter

The term "promoter" is art-recognized and refers to a nucleic acid molecule with a sequence recognized by the cellular transcription machinery and able to initiate transcription of a downstream gene. A promoter may be constitutively active, meaning that the promoter is always active in a given cellular context, or conditionally active, meaning that the promoter is only active in the presence of a specific condition. For example, a conditional promoter may only be active in the presence of a specific protein that connects a protein associated with a regulatory element in the promoter to the basic transcriptional machinery, or only in the absence of an inhibitory molecule. A subclass of conditionally active promoters is inducible promoters that require the presence of a small molecule "inducer" for activity. Examples of inducible promoters include, but are not limited to, arabinose-inducible promoters, Tet-on promoters, and tamoxifen-inducible promoters. A variety of constitutive, conditional, and inducible promoters are well known to the skilled artisan, and the skilled artisan will be able to ascertain a variety of such promoters useful in carrying out the instant invention, which is not limited in this respect. In various embodiments, the disclosure provides vectors with appropriate promoters for driving expression of the nucleic acid sequences encoding the fusion proteins (or one or more individual components thereof).

### Protein, Peptide, and Polypeptide

The terms "protein," "peptide," and "polypeptide" are used interchangeably herein, and refer to a polymer of amino acid residues linked together by peptide (amide) bonds. The terms refer to a protein, peptide, or polypeptide of any size, structure, or function. Typically, a protein, peptide, or polypeptide will be at least three amino acids long. A protein, peptide, or polypeptide may refer to an individual protein or a collection of proteins. One or more of the amino acids in a protein, peptide, or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification, etc. A protein, peptide, or polypeptide may also be a single molecule or may be a multi-molecular complex. A protein, peptide, or polypeptide may be just a fragment of a naturally occurring protein or peptide. A protein, peptide, or polypeptide may be naturally occurring, recombinant, or synthetic, or any combination thereof. The term "fusion protein" as used herein refers to a hybrid polypeptide which comprises protein domains from at least two different proteins. One protein may be located at the amino-terminal (N-terminal) portion of the fusion protein or at the carboxy-terminal (C-terminal) protein thus forming an "amino-terminal fusion protein" or a "carboxy-terminal fusion protein," respectively. A protein may comprise different domains, for example, a nucleic acid binding domain (*e.g*., the gRNA binding domain of Cas9 that directs the binding of the protein to a target site) and a nucleic acid cleavage domain or a catalytic domain of a recombinase. In some embodiments, a protein comprises a proteinaceous part, *e.g.,* an amino acid sequence constituting a nucleic acid binding domain, and an organic compound, *e.g*., a compound that can act as a nucleic acid cleavage agent. In some embodiments, a protein is in a complex with, or is in association with, a nucleic acid, *e.g.,* RNA. Any of the proteins provided herein may be produced by any method known in the art. For example, the proteins provided herein may be produced via recombinant protein expression and purification, which is especially suited for fusion proteins comprising a peptide linker. Methods for recombinant protein expression and purification are well known, and include those described by Green and Sambrook, Molecular Cloning: A Laboratory Manual (4th ed., Cold Spring Harbor Laboratory Press, Cold Spring Harbor, N.Y. (2012)), the entire contents of which are incorporated herein by reference. It should be appreciated that the disclosure provides any of the polypeptide sequences provided herein without an N-terminal methionine (M) residue.

### Protospacer

As used herein, the term "protospacer" refers to the sequence (~20 bp) in DNA adjacent to the PAM (protospacer adjacent motif) sequence. The protospacer shares the same sequence as the spacer sequence of the guide RNA. The guide RNA anneals to the complement of the protospacer sequence on the target DNA (specifically, one strand thereof, i.e., the "target strand" versus the "non-target strand" of the target DNA sequence). In order for Cas9 to function it also requires a specific protospacer adjacent motif (PAM) that varies depending on the bacterial species of the Cas9 gene. The most commonly used Cas9 nuclease, derived from *S. pyogenes,* recognizes a PAM sequence of NGG that is found directly downstream of the target sequence in the genomic DNA, on the non-target strand.

The skilled person will appreciate that the literature in the state of the art sometimes refers to the "protospacer" as the ~20-nt target-specific guide sequence on the guide RNA itself, rather than referring to it as a "spacer." Thus, in some cases, the term "protospacer" as used herein may be used interchangeably with the term "spacer." The context of the description surrounding the appearance of either "protospacer" or "spacer" will help inform the reader as to whether the term is in reference to the gRNA or the DNA target.

### Protospacer adjacent motif (PAM)

As used herein, the term "protospacer adjacent sequence" or "PAM" refers to an approximately 2-6 base pair DNA sequence that is an important targeting component of a Cas9 nuclease. Typically, the PAM sequence is on either strand, and is downstream in the 5' to 3' direction of the Cas9 cut site. The canonical PAM sequence (i.e., the PAM sequence that is associated with the Cas9 nuclease of Streptococcus pyogenes or SpCas9) is 5'-NGG-3' wherein "N" is any nucleobase followed by two guanine ("G") nucleobases. Different PAM sequences can be associated with different Cas9 nucleases or equivalent proteins from different organisms. In addition, any given Cas9 nuclease, e.g., SpCas9, may be modified to alter the PAM specificity of the nuclease such that the nuclease recognizes alternative PAM sequence.

For example, with reference to the canonical SpCas9 amino acid sequence is SEQ ID NO: 2, the PAM sequence can be modified by introducing one or more mutations, including (a) D1135V, R1335Q, and T1337R "the VQR variant", which alters the PAM specificity to NGAN or NGNG, (b) D1135E, R1335Q, and T1337R "the EQR variant", which alters the PAM specificity to NGAG, and (c) D1135V, G1218R, R1335E, and T1337R "the VRER variant", which alters the PAM specificity to NGCG. In addition, the D1135E variant of canonical SpCas9 still recognizes NGG, but it is more selective compared to the wild type SpCas9 protein.

It will also be appreciated that Cas9 enzymes from different bacterial species (i.e., Cas9 orthologs) can have varying PAM specificities. For example, Cas9 from *Staphylococcus aureus* (SaCas9) recognizes NGRRT or NGRRN. In addition, Cas9 from *Neisseria meningitis* (NmCas) recognizes NNNNGATT. In another example, Cas9 from *Streptococcus thermophilis* (StCas9) recognizes NNAGAAW. In still another example, Cas9 from *Treponema denticola* (TdCas) recognizes NAAAAC. These are examples and are not meant to be limiting. It will be further appreciated that non-SpCas9s bind a variety of PAM sequences, which makes them useful when no suitable SpCas9 PAM sequence is present at the desired target cut site. Furthermore, non-SpCas9s may have other characteristics that make them more useful than SpCas9. For example, Cas9 from *Staphylococcus aureus* (SaCas9) is about 1 kilobase smaller than SpCas9, so it can be packaged into adeno-associated virus (AAV). Further reference may be made to Shah et al., "Protospacer recognition motifs: mixed identities and functional diversity," RNA Biology, 10(5): 891-899 (which is incorporated herein by reference).

### RNA-protein recruitment system

In various embodiments, two separate protein domains (e.g., a Cas9 domain and a cytidine deaminase domain) may be colocalized to one another to form a functional complex (akin to the function of a fusion protein comprising the two separate protein domains) by using an "RNA-protein recruitment system," such as the "MS2 tagging technique." Such systems generally tag one protein domain with an "RNA-protein interaction domain" (aka "RNA-protein recruitment domain") and the other with an "RNA-binding protein" that specifically recognizes and binds to the RNA-protein interaction domain, e.g., a specific hairpin structure. These types of systems can be leveraged to colocalize the domains of a base editor, as well as to recruitment additional functionalities to a base editor, such as a UGI domain. In one example, the MS2 tagging technique is based on the natural interaction of the MS2 bacteriophage coat protein ("MCP" or "MS2cp") with a stem-loop or hairpin structure present in the genome of the phage, i.e., the "MS2 hairpin." In the case of the MS2 hairpin, it is recognized and bound by the MS2 bacteriophage coat protein (MCP). Thus, in one exemplarly scenario a deaminase-MS2 fusion can recruit a Cas9-MCP fusion.

A review of other modular RNA-protein interaction domains are described in the art, for example, in Johansson et al., "RNA recognition by the MS2 phage coat protein," Sem Virol., 1997, Vol. 8(3): 176-185; Delebecque et al., "Organization of intracellular reactions with rationally designed RNA assemblies," Science, 2011, Vol. 333: 470-474; Mali et al., "Cas9 transcriptional activators for target specificity screening and paired nickases for cooperative genome engineering," Nat. Biotechnol., 2013, Vol.31: 833-838; and Zalatan et al., "Engineering complex synthetic transcriptional programs with CRISPR RNA scaffolds," Cell, 2015, Vol.160: 339-350, each of which are incorporated herein by reference in their entireties. Other systems include the PP7 hairpin, which specifically recruits the PCP protein, and the "com" hairpin, which specifically recruits the Com protein. See Zalatan et al.

The nucleotide sequence of the MS2 hairpin (or equivalently referred to as the "MS2 aptamer") is: GCCAACATGAGGATCACCCATGTCTGCAGGGCC (SEQ ID NO: 172).

The amino acid sequence of the MCP or MS2cp is:

GSASNFTQFVLVDNGGTGDVTVAPSNFANGVAEWISSNSRSQAYKVTCSVRQSSAQ
NRKYTIKVEVPKVATQTVGGEELPVAGWRSYLNMELTIPIFATNSDCELIVKAMQGL
LKDGNPIPSAIAANSGIY (SEQ ID NO: 173).

### Sense strand

In genetics, a "sense" strand is the segment within double-stranded DNA that runs from 5' to 3', and which is complementary to the antisense strand of DNA, or template strand, which runs from 3' to 5'. In the case of a DNA segment that encodes a protein, the sense strand is the strand of DNA that has the same sequence as the mRNA, which takes the antisense strand as its template during transcription, and eventually undergoes (typically, not always) translation into a protein. The antisense strand is thus responsible for the RNA that is later translated to protein, while the sense strand possesses a nearly identical makeup to that of the mRNA. Note that for each segment of dsDNA, there will possibly be two sets of sense and antisense, depending on which direction one reads (since sense and antisense is relative to perspective). It is ultimately the gene product, or mRNA, that dictates which strand of one segment of dsDNA is referred to as sense or antisense.

In the context of a PEgRNA, the first step is the synthesis of a single-strand complementary DNA (i.e., the 3' ssDNA flap, which becomes incorporated) oriented in the 5' to 3' direction which is templated off of the PEgRNA extension arm. Whether the 3' ssDNA flap should be regarded as a sense or antisense strand depends on the direction of transcription since it well accepted that both strands of DNA may serve as a template for transcription (but not at the same time). Thus, in some embodiments, the 3' ssDNA flap (which overall runs in the the 5' to 3' direction) will serve as the sense strand because it is the coding strand. In other embodiments, the 3' ssDNA flap (which overall runs in the the 5' to 3' direction) will serve as the antisense strand and thus, the template for transcription.

### Spacer sequence

As used herein, the term "spacer sequence" in connection with a guide RNA refers to the portion of the guide RNA of about 20 nucleotides which contains a nucleotide sequence that is complementary to the protospacer sequence in the target DNA sequence. The spacer sequence anneals to the protospacer sequence to form a ssRNA/ssDNA hybrid structure at the target site and a corresponding R loop ssDNA structure of the endogenous DNA strand that is complementary to the protospacer sequence.

### Subject

The term "subject," as used herein, refers to an individual organism, for example, an individual mammal. In some embodiments, the subject is a human. In some embodiments, the subject is a non-human mammal. In some embodiments, the subject is a non-human primate. In some embodiments, the subject is a rodent. In some embodiments, the subject is a sheep, a goat, a cattle, a cat, or a dog. In some embodiments, the subject is a vertebrate, an amphibian, a reptile, a fish, an insect, a fly, or a nematode. In some embodiments, the subject is a research animal. In some embodiments, the subject is genetically engineered, *e.g*., a genetically engineered non-human subject. The subject may be of either sex and at any stage of development.

### Target site

The term "target site" refers to a sequence within a nucleic acid molecule that is edited by a fusion protein (*e.g.* a dCas9-deaminase fusion protein provided herein). The target site further refers to the sequence within a nucleic acid molecule to which a complex of the fusion protein and gRNA binds.

### Transcription terminator

A "transcriptional terminator" is a nucleic acid sequence that causes transcription to stop. A transcriptional terminator may be unidirectional or bidirectional. It is comprised of a DNA sequence involved in specific termination of an RNA transcript by an RNA polymerase. A transcriptional terminator sequence prevents transcriptional activation of downstream nucleic acid sequences by upstream promoters. A transcriptional terminator may be necessary *in vivo* to achieve desirable expression levels or to avoid transcription of certain sequences. A transcriptional terminator is considered to be "operably linked to" a nucleotide sequence when it is able to terminate the transcription of the sequence it is linked to.

The most commonly used type of terminator is a forward terminator. When placed downstream of a nucleic acid sequence that is usually transcribed, a forward transcriptional terminator will cause transcription to abort. In some embodiments, bidirectional transcriptional terminators are provided, which usually cause transcription to terminate on both the forward and reverse strand. In some embodiments, reverse transcriptional terminators are provided, which usually terminate transcription on the reverse strand only.

In prokaryotic systems, terminators usually fall into two categories (1) rho-independent terminators and (2) rho-dependent terminators. Rho-independent terminators are generally composed of palindromic sequence that forms a stem loop rich in G-C base pairs followed by several T bases. Without wishing to be bound by theory, the conventional model of transcriptional termination is that the stem loop causes RNA polymerase to pause, and transcription of the poly-A tail causes the RNA:DNA duplex to unwind and dissociate from RNA polymerase.

In eukaryotic systems, the terminator region may comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in some embodiments involving eukaryotes, a terminator may comprise a signal for the cleavage of the RNA. In some embodiments, the terminator signal promotes polyadenylation of the message. The terminator and/or polyadenylation site elements may serve to enhance output nucleic acid levels and/or to minimize read through between nucleic acids.

Terminators for use in accordance with the present disclosure include any terminator of transcription described herein or known to one of ordinary skill in the art. Examples of terminators include, without limitation, the termination sequences of genes such as, for example, the bovine growth hormone terminator, and viral termination sequences such as, for example, the SV40 terminator, spy, yejM, secG-leuU, thrLABC, rrnB T1, hisLGDCBHAFI, metZWV, rrnC, xapR, aspA and arcA terminator. In some embodiments, the termination signal may be a sequence that cannot be transcribed or translated, such as those resulting from a sequence truncation.

### Transition

As used herein, "transitions" refer to the interchange of purine nucleobases (A ↔ G) or the interchange of pyrimidine nucleobases (C ↔ T). This class of interchanges involves nucleobases of similar shape. The compositions and methods disclosed herein are capable of inducing one or more transitions in a target DNA molecule. The compositions and methods disclosed herein are also capable of inducing both transitions and transversion in the same target DNA molecule. These changes involve A ↔ G, G ↔ A, C ↔ T, or T ↔ C. In the context of a double-strand DNA with Watson-Crick paired nucleobases, transversions refer to the following base pair exchanges: A:T ↔ G:C, G:G ↔ A:T, C:G ↔ T:A, or T:A↔ C:G. The compositions and methods disclosed herein are capable of inducing one or more transitions in a target DNA molecule. The compositions and methods disclosed herein are also capable of inducing both transitions and transversion in the same target DNA molecule, as well as other nucleotide changes, including deletions and insertions.

### Transversion

As used herein, "transversions" refer to the interchange of purine nucleobases for pyrimidine nucleobases, or in the reverse and thus, involve the interchange of nucleobases with dissimilar shape. These changes involve T ↔ A, T↔ G, C ↔ G, C ↔ A, A ↔ T, A ↔ C, G ↔ C, and G ↔ T. In the context of a double-strand DNA with Watson-Crick paired nucleobases, transversions refer to the following base pair exchanges: T:A ↔ A:T, T:A ↔ G:C, C:G ↔ G:C, C:G ↔ A:T, A:T ↔ T:A, A:T ↔ C:G, G:C ↔ C:G, and G:C ↔ T:A. The compositions and methods disclosed herein are capable of inducing one or more transversions in a target DNA molecule. The compositions and methods disclosed herein are also capable of inducing both transitions and transversion in the same target DNA molecule, as well as other nucleotide changes, including deletions and insertions.

### Treatment

The terms "treatment," "treat," and "treating," refer to a clinical intervention aimed to reverse, alleviate, delay the onset of, or inhibit the progress of a disease or disorder, or one or more symptoms thereof, as described herein. As used herein, the terms "treatment," "treat," and "treating" refer to a clinical intervention aimed to reverse, alleviate, delay the onset of, or inhibit the progress of a disease or disorder, or one or more symptoms thereof, as described herein. In some embodiments, treatment may be administered after one or more symptoms have developed and/or after a disease has been diagnosed. In other embodiments, treatment may be administered in the absence of symptoms, e.g., to prevent or delay onset of a symptom or inhibit onset or progression of a disease. For example, treatment may be administered to a susceptible individual prior to the onset of symptoms (e.g., in light of a history of symptoms and/or in light of genetic or other susceptibility factors). Treatment may also be continued after symptoms have resolved, for example, to prevent or delay their recurrence.

### Upstream

As used herein, the terms "upstream" and "downstream" are terms of relativety that define the linear position of at least two elements located in a nucleic acid molecule (whether single or double-stranded) that is orientated in a 5'-to-3' direction. In particular, a first element is upstream of a second element in a nucleic acid molecule where the first element is positioned somewhere that is 5' to the second element. For example, a SNP is upstream of a Cas9-induced nick site if the SNP is on the 5' side of the nick site. Conversely, a first element is downstream of a second element in a nucleic acid molecule where the first element is positioned somewhere that is 3' to the second element. For example, a SNP is downstream of a Cas9-induced nick site if the SNP is on the 3' side of the nick site. The nucleic acid molecule can be a DNA (double or single stranded). RNA (double or single stranded), or a hybrid of DNA and RNA. The analysis is the same for single strand nucleic acid molecule and a double strand molecule since the terms upstream and downstream are in reference to only a single strand of a nucleic acid molecule, except that one needs to select which strand of the double stranded molecule is being considered. Often, the strand of a double stranded DNA which can be used to determine the positional relativity of at least two elements is the "sense" or "coding" strand. In genetics, a "sense" strand is the segment within double-stranded DNA that runs from 5' to 3', and which is complementary to the antisense strand of DNA, or template strand, which runs from 3' to 5'. Thus, as an example, a SNP nucleobase is "downstream" of a promoter sequence in a genomic DNA (which is double-stranded) if the SNP nucleobase is on the 3' side of the promoter on the sense or coding strand.

### Uracil glycosylase inhibitor

The term "uracil glycosylase inhibitor" or "UGI," as used herein, refers to a protein that is capable of inhibiting a uracil-DNA glycosylase base-excision repair enzyme. In some embodiments, a UGI domain comprises a wild-type UGI or a UGI as set forth in SEQ ID NO: 163. In some embodiments, the UGI proteins provided herein include fragments of UGI and proteins homologous to a UGI or a UGI fragment. For example, in some embodiments, a UGI domain comprises a fragment of the amino acid sequence set forth in SEQ ID NO: 163. In some embodiments, a UGI fragment comprises an amino acid sequence that comprises at least 60%, at least 65%, at least 70%, at least 75%, at least 80%, at least 85%, at least 90%, at least 95%, at least 96%, at least 97%, at least 98%, at least 99%, or at least 99.5% of the amino acid sequence as set forth in SEQ ID NO: 163. In some embodiments, a UGI comprises an amino acid sequence homologous to the amino acid sequence set forth in SEQ ID NO: 163, or an amino acid sequence homologous to a fragment of the amino acid sequence set forth in SEQ ID NO: 163. In some embodiments, proteins comprising UGI or fragments of UGI or homologs of UGI or UGI fragments are referred to as "UGI variants." A UGI variant shares homology to UGI, or a fragment thereof. For example a UGI variant is at least 70% identical, at least 75% identical, at least 80% identical, at least 85% identical, at least 90% identical, at least 95% identical, at least 96% identical, at least 97% identical, at least 98% identical, at least 99% identical, at least 99.5% identical, or at least 99.9% identical to a wild type UGI or a UGI as set forth in SEQ ID NO: 163. In some embodiments, the UGI variant comprises a fragment of UGI, such that the fragment is at least 70% identical, at least 80% identical, at least 90% identical, at least 95% identical, at least 96% identical, at least 97% identical, at least 98% identical, at least 99% identical, at least 99.5% identical, or at least 99.9% to the corresponding fragment of wild-type UGI or a UGI as set forth in SEQ ID NO: 163. In some embodiments, the UGI comprises the following amino acid sequence: MTNLSDIIEKETGKQLVIQESILMLPEEVEEVIGNKPESDILVHTAYDESTDENVMLLT SDAPEYKPWALVIQDSNGENKIKML (SEQ ID NO: 163) (P14739|UNGI_BPPB2 Uracil-DNA glycosylase inhibitor).

### Variant

As used herein, the term "variant" refers to a protein having characteristics that deviate from what occurs in nature that retains at least one functional *i.e*. binding, interaction, or enzymatic ability and/or therapeutic property thereof. A "variant" is at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to the wild type protein. For instance, a variant of Cas9 may comprise a Cas9 that has one or more changes in amino acid residues as compared to a wild type Cas9 amino acid sequence. As another example, a variant of a deaminase may comprise a deaminase that has one or more changes in amino acid residues as compared to a wild type deaminase amino acid sequence, *e.g*. following ancestral sequence reconstruction of the deaminase. These changes include chemical modifications, including substitutions of different amino acid residues truncations, covalent additions (*e.g*. of a tag), and any other mutations. The term also encompasses circular permutants, mutants, truncations, or domains of a reference sequence, and which display the same or substantially the same functional activity or activities as the reference sequence. This term also embraces fragments of a wild type protein.

The level or degree of which the property is retained may be reduced relative to the wild type protein but is typically the same or similar in kind. Generally, variants are overall very similar, and in many regions, identical to the amino acid sequence of the protein described herein. A skilled artisan will appreciate how to make and use variants that maintain all, or at least some, of a functional ability or property.

The variant proteins may comprise, or alternatively consist of, an amino acid sequence which is at least 80%, 85%, 90%, 95%, 96%, 97%, 98%, 99%, or 100%, identical to, for example, the amino acid sequence of a wild-type protein, or any protein provided herein (*e.g*. SMN protein).

By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino- or carboxy-terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 95%, 96%, 97%, 98%, or 99% identical to, for instance, the amino acid sequence of a protein such as a SMN protein, can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245 (1990)). In a sequence alignment the query and subject sequences are either both nucleotide sequences or both amino acid sequences. The result of said global sequence alignment is expressed as percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

### Vector

The term "vector," as used herein, refers to a nucleic acid that can be modified to encode a gene of interest and that is able to enter into a host cell, mutate and replicate within the host cell, and then transfer a replicated form of the vector into another host cell. Exemplary suitable vectors include viral vectors, such as retroviral vectors or bacteriophages and filamentous phage, and conjugative plasmids. Additional suitable vectors will be apparent to those of skill in the art based on the instant disclosure.

### Wild Type

As used herein the term "wild type" is a term of the art understood by skilled persons and means the typical form of an organism, strain, gene or characteristic as it occurs in nature as distinguished from mutant or variant forms.

These and other exemplary substituents are described in more detail in the Detailed Description, Examples, and claims.

### DETAILED DESCRIPTION OF CERTAIN EMBODIMENTS

The present disclosure provides compositions, fusion proteins, napDNAbps, deaminases (e.g., cytidine or adenine deaminases), guide RNAs, base editors (e.g., CBEs and/or ABEs), nucleic acid molecules, vectors, kits, and methods for modifying a polynucleotide using base editing strategies that comprise the use of a nucleic acid programmable DNA binding protein ("napDNAbp"), a deaminase (e.g., a cytosine or adenine deaminase) and a suitable guide RNA to modify the *SMN2* gene such that a stable and functional SMN2 protein is expressed in spinal motor neurons, thereby treating spinal muscular atrophy (SMA). The disclosure relates in part to the inventors' discovery of base editing strategies that may be used to effectively target the *SMN2* genomic locus for to install edits that affect SMN protein production and stability, thereby providing a new platform for treating SMA that goes beyond the limitations recognized in the art, such as, antisense oligonucleotide treatments (e.g., nusinersen) which are transient in nature. The methods and composition disclosed herein provide a curative treatment for SMA. Accordingly, the disclosure provides methods, base editors, vectors encoding base editor and cognate gRNAs, and compositions and kits comprise said components, for edits to correct one or more mutations associated with SMA, such as, but not limited to editing C840T of exon 7 of the *SMN2* gene, or installing another one or more nucleobase edits which have the effect of removing or inactivating a degron, such as the C-terminal portion of the region encoded by exon 6 or the 4-amino acid region encoded by exon 8 (i.e., the EMLA (SEQ ID NO: 466) - tail) so as to remove or limit their degron activity to reduce, mitigate, or eliminate the intracellular degradation of the SMN2 protein.

Spinal Muscular Atrophy (SMA) is a progressive motor neuron degeneration disorder that occurs as a result of insufficient survival motor neuron (SMN) protein in spinal motor neurons which leads to atrophy of skeletal muscle and paralysis of the patient. The disease typically involves the status of two SMN-encoding genes, namely, the telomeric *SMN1* gene and the almost identical centromeric copy, the *SMN2* gene. In SMA, the *SMN1* gene is not present due to homozygous deletion (see Cho et al., "A degron created by SMN2 exon 7 skipping is a principle contributor to spinal muscular atrophy severity," Genes & Development, vol. 24: pp. 438-442, which is incorporated herein by reference). Thus, SMA patients do not typically express SMN1 protein. The centromeric *SMN2* gene partially rescues the deleted *SMN1* gene but the level of rescue is insufficient because of the presence of a single nucleotide mutation in the splice acceptor site at position 6 within exon 7 that results in the frequent skipping (i.e., ~ 80%) of exon 7 during *SMN2* post-transcriptional processing (a C-to-T substitution at position 6 of exon 7). Thus, while a small amount of full-size SMN protein is produced from the *SMN2* gene, the majority of the *SMN2* gene product is truncated in the region corresponding to exon 7. In addition, the defective *SMN2* gene product also acquires four amino acids, EMLA (SEQ ID NO: 466) , encoded by exon 8 as a new C-terminus of the protein ("the EMLA (SEQ ID NO: 466) tail"). This defective gene product is often referred to as the SMNδ7 product.

While the SMNδ7 product bears the same function as SMN1, although somewhat diminished, it is rapidly degraded due to the appearance of at least two degron signals formed as a result of the exon-7 skipping event. Specifically, it has been reported that the truncated SMNδ7 product signals its own cellular degradation by the proteasome complex due to the presence of (1) the C-terminal portion of the region encoded by exon 6 and (2) the 4-amino acid region encoded by exon 8 (i.e., the EMLA (SEQ ID NO: 466) -tail), both of which function as degron signals in the absence of the exon 7-encoded region^{8, 9}.

This disclosure describes the design and use of various exemplary base editors and associated guide RNAs that are capable of installing precise nucleobase changes in the *SMN2* genomic locus, thereby resulting in the production of a modified SMN2 protein that avoids or limits its proteasome-dependent degradation and which retains SMN1-compensatory function. For example, the base editors described herein may be used to eliminate and/or modify one or more degrons in the naturally occurring truncated SMNδ7 product to produce a modified SMN2 product having greater stability. For example, such BE-induced modifications can include, but are not limited to, (1) the removal and/or modification of the C-terminal portion of the region encoded by exon 6 and (2) removal and/or modification of the 4-amino acid region encoded by exon 8 (i.e., the EMLA (SEQ ID NO: 466) -tail) so as to remove or limit their degron activity.

For example, in one embodiment, the genome editing strategies disclosed herein target position 6 of exon 7 of the *SMN2* gene locus, which is an inactive splice acceptor site due to the presence of a T in place of a C in exon 7 at that position. This nucleobase position is often referred to as C840T, which is in relation to the counterpart position in the *SMN1* gene which presents a C at that position of exon 7, defining an active splice site. Thus, in one embodiment, the genome editing methods and compositions may be used to introduce a T-to-C edit at position 6 of exon 7 of the *SMN2* gene, i.e., editing C840T to a C at position 6 of exon 7 and restoring splicing of exon 7, thereby encoding a modified SMN2 protein that includes the amino acid sequence encoded by exon 7. Without wishing to be bound by theory, a modified SMN2 protein comprising the amino acid sequence encoded by exon 7 is not sensitive to cellular degradation, unlike the wild type, truncated SMN2 product formed from the wild type *SMN2* gene as a result of exon 7-skipping. The overall activity and/or production of the modified SMN2 protein (i.e., now including the amino acid region encoded by exon 7) are increased, thereby treating SMA. Without being bound by any particular theory, the increased production and/or activity of the modified SMN2 protein relates to the elimination or reduction in protein degradation associated with the truncated SMN2 wild type protein.

In another embodiment, the genome editing strategies disclosed herein target the removal of one or more degrons present in the encoded SMN2 protein, thereby reducing or eliminating the degron-dependent degradation of the SMN2 protein. For example, the disclosure contemplates the use of genome editing to eliminate the 4-amino acid sequence tail of EMLA (SEQ ID NO: 466) encoded by exon 8 of the *SMN2* gene-which functions as a degron-thereby minimizing or eliminating the degradation associated with said degron. In certain aspects, the EMLA (SEQ ID NO: 466) sequence can be removed by using genome editing to remove the exon 8 splice site, such that exon 8 is eliminated from the final messenger RNA, and thus, not encoded into the resulting SMN2 protein.

In yet another embodiment, the genome editing strategies disclosed herein target the modification of one or more degrons present in the encoded SMN2 protein, thereby reducing or eliminating the degradation associated with said one or more degrons. For example, the disclosure contemplates the use of genome editing to eliminate a stop codon corresponding to the C-terminal end of the region encoded by exon 8 (i.e., which codes for the 4-amino acid sequence tail of EMLA (SEQ ID NO: 466) ) such that the 4-amino acid EMLA (SEQ ID NO: 466) tail is lengthened to include additional amino acids, e.g., 5 additional amino acids, which prevents or limits the degradation associated with said EMLA (SEQ ID NO: 466) tail.

The present disclosure relates in part to the discovery of a variety of base editing strategies to target *SMN2* genomic locus for point mutations that affect SMN protein production and stability, which has implications for the treatment of SMA. The disclosure provides methods of correcting the single nucleotide polymorphism (SNP) associated with SMA, as well as methods of increasing the stability and/or decreasing degradation of SMN protein products. For example, in some methods Cas9-nuclease is used to perturb or delete regions of the *SMN2* gene to increase protein stability.

The base editors embrace any type of base editor, and in particular, are exemplified herein using cytidine deaminase base editors (i.e., capable of installing a C-to-T edits, a type of transition editor) and adenine base editors (i.e., capable of installing A-to-G edits, a type of transition editor) to account for a variety of genetic strategies that result in the production of a modified SMN2 protein that is both stable and functional, and which is capable of rescuing the loss of SMN1 function. Such genetic changes are permanent since they are at the level of genomic change, as opposed to a more transient effect of the use of antisense oligonucleotides (ASO) (e.g., nusinersen, approved in the U.S. as SPINRAZA^{®} in 2016), which are only capable of transiently repairing exon 7 splicing in an *SMN2* mRNA transcript.

Aspects of the disclosure provide methods and compositions for deaminating an adenine nucleobase (A) in an *SMN2* gene using an adenine base editor (ABE) bound to a guide RNA (gRNA), wherein the gRNA comprises a guide sequence that is complementary to a target nucleic acid sequence in the *SNM2* gene.

Other aspects of the disclosure provide methods and compositions for deaminating a cytosine nucleobase (C) in an *SMN2* gene using a cytidine base editor (CBE) bound to a guide RNA (gRNA), wherein the gRNA comprises a guide sequence that is complementary to a target nucleic acid sequence in the *SNM2* gene.

In other aspect, the disclosure relates to the delivery of base editors to cells for modifying the *SMN2* gene. Such based editors can be delivered *in vivo* to a subject. In some embodiments, the fusion protein is delivered in two parts, for example by using a split-intein strategy. In some embodiments, editing of C840T in exon 7 results in an increase, *e.g*., at least 5%, 10%, 20%, 30%, 40%, 50%, 60%, 70%, 80%, 90%, 95%, 96%, 97%, 98%, 99%, 100% or more increase in a level of SMN protein in a subject, in an organ of a subject (e.g., brain, heart, lungs, liver, intestine, and/or pancreas), or in a cell of a subject (*e.g*., neuron, such as a motor neuron).

In still other aspects, the disclosure relates to guide RNAs (gRNA) that direct the base editors to a target *SMN2* site. In some embodiments, the gRNA directs the fusion protein in proximity to a point mutation in the *SMN2* gene, for example, a point mutation in exon 7. In some embodiments, the gRNA directs the fusion protein within 1, 2, 3, 4, 5, 6, 7, 8, 9, 10, 11, 12, 13, 14, 15, 16, 17, 18, 19, or 20 base pairs of a point mutation within the *SMN2* gene. In some embodiments, the gRNA comprises the sequence 5'-ATTTTGTCT**A**AAACCctgta-3' (SEQ ID NO: 364), where the nucleotide target is indicated in bold. It should be appreciated that the Ts indicated in the gRNA sequence are uracils (Us) in the RNA sequence.
Accordingly, in some embodiments, the gRNA comprises the sequence 5'-AUUUUGUCU**A**AAACCcugua-3' (SEQ ID NO: 365). In some embodiments, the guide sequence of the gRNA comprises a nucleic acid sequence selected from the group consisting of 5'-TTTGTCT**A**AAACCCTGTAAG-3' (SEQ ID NO: 366), 5'-TTTTGTCT**A**AAACCCTGTAA-3' (SEQ ID NO: 368), 5'-TGATTTTGTCT**A**AAACCC-3' (SEQ ID NO: 370), 5'-GATTTTGTCT**A**AAACCCT-3' (SEQ ID NO: 372), 5'-ATTTTGTCT**A**AAACCCTG-3' (SEQ ID NO: 374), 5'-GTCT**A**AAACCCTGTAAGG-3' (SEQ ID NO: 376), and 5'-TCT**A**AAACCCTGTAAGGA-3' (SEQ ID NO: 378). As noted previously, the gRNA sequence may comprise uracil (U) instead of thymine (T). Therefore, in some embodiments, the guide sequence of the gRNA comprises a nucleic acid sequence selected from the group consisting of 5'-UUUGUCU**A**AAACCCUGUAAG-3' (SEQ ID NO: 367), 5'-UUUUGUCU**A**AAACCCUGUAA-3' (SEQ ID NO: 369), 5'-UGAUUUUGUCU**A**AAACCC-3' (SEQ ID NO: 371), 5'-GAUUUUGUCU**A**AAACCCU-3' (SEQ ID NO: 373), 5'-AUUUUGUCU**A**AAACCCUG-3' (SEQ ID NO: 375), 5'-GUCU**A**AAACCCUGUAAGG-3' (SEQ ID NO: 377), and 5'-UCU**A**AAACCCUGUAAGGA-3' (SEQ ID NO: 379).

In other aspects, the disclosure provides compositions comprising a fusion protein (*e.g*., adenine base editor or cytidine base editor) and a guide RNA (gRNA). In some embodiments, the gRNA directs the fusion protein in proximity to a stop sequence in the *SMN2* gene, for example, in exon 8. In some embodiments, the gRNA directs the fusion protein within 1, 2, 3, 4, 5, 6, 7, 8, 9, 10, 11, 12, 13, 14, 15, 16, 17, 18, 19, or 20 base pairs of stop codon within the *SMN2* gene. In some embodiments, the gRNA comprises a nucleic acid sequence selected from the group consisting of: 5'-TTTGCAGGAAATGCTGGCAT-3' (SEQ ID NO: 381), 5'-TTCTCATTTGCAGGAAATGC-3' (SEQ ID NO: 383), 5'-CATTTAGTGCTGCT**CTA**TGC-3' (SEQ ID NO: 385), 5'-CAGGAAATGCTGGCA**TAG**AG-3' (SEQ ID NO: 387), 5'-TTGCAGGAAATGCTGGCA**TA**-3' (SEQ ID NO: 389), 5'-ATTTGCAGGAAATGCTGGCA-3' (SEQ ID NO: 391), and 5'-TGGCA**TAG**AGCAGCACTAAA-3' (SEQ ID NO: 393), where the nucleotide target is indicated in bold. It should be appreciated that the Ts indicated in the gRNA sequence are uracils (Us) in the RNA sequence. Accordingly, in some embodiments, the gRNA comprises a sequence selected from the group consisting of:
5'-UUUGCAGGAAAUGCUGGCAU-3' (SEQ ID NO: 324),
5'-UUCUCAUUUGCAGGAAAUGC-3' (SEQ ID NO: 325),
5'-CAUUUAGUGCUGCU**CUA**UGC-3' (SEQ ID NO: 326),
5'-CAGGAAAUGCUGGCA**UAG**AG-3' (SEQ ID NO: 327),
5'-UUGCAGGAAAUGCUGGCA**UA**-3' (SEQ ID NO: 328),
5'-AUUUGCAGGAAAUGCUGGCA-3' (SEQ ID NO: 329), and
5'-UGGCA**UAG**AGCAGCACUAAA-3' (SEQ ID NO: 394).

In some embodiments, the gRNA directs the fusion protein in proximity to a specific codon sequence in the *SMN2* gene, for example, in exon 6. In one embodiment, the gRNA directs the fusion protein in proximity to the S270 amino acid of *SMN2.* In some embodiments, the gRNA directs the fusion protein within 1, 2, 3, 4, 5, 6, 7, 8, 9, 10, 11, 12, 13, 14, 15, 16, 17, 18, 19, or 20 base pairs of a specific codon within the *SMN2* gene. In some embodiments, the gRNA comprises the nucleic acid sequence 5'-TGGCA**TAG**AGCAGCACTAAA-3' (SEQ ID NO: 353), where the nucleotide target is indicated in bold. It should be appreciated that the Ts indicated in the gRNA sequence are uracils (Us) in the RNA sequence. Accordingly, in some embodiments, the gRNA comprises the sequence: 5'-UGGCA**UAG**AGCAGCACUAAA-3' (SEQ ID NO: 394).

In some embodiments, the disclosure provides guide sequences capable of directing base editors (*e.g.,* adenosine base editors) to the fifth stop codon of exon 8 in an *SMN2* gene. In some embodiments, the disclosure provides guide sequences capable of directing base editors (*e.g*., adenosine base editors) to the AGT codon encoding S270 (in exon 6) of the *SMN2* gene. In some aspects the disclosure provides proteins that deaminate the nucleobase adenine, for example in an *SMN2* gene to treat SMA.

In some embodiments, the base editor nicks the target sequence that is complementary to the guide sequence. In some embodiments, the base editor is a circular permutant (CP) adenine base editor, such as the CP₁₀₄₁ base editor. In some embodiments, the base editor may be formulated as a split-intein base editor. The base editor may be, for example, wild-type SpCas9, SaCas9-KKH, Cas9-VQR, Cas9-VRQR, Cas9-VRER, Cas9-NG, CP1028, CP1041, CP1041-NG, Cpf1, iSpyMac, SpCas9-NRRH, or SpCas9-NRCH.

Methods and compositions provided herein, in some embodiments, are used to treat a subject having or a subject suspected of having SMA.

Thus, in some aspects, the base editor and guide RNA complexes described herein may be useful for treating a disease or a disorder caused by a C to T mutation in a *SMN2* gene. In some embodiments, deaminating the adenosine nucleobase in the *SMN2* gene results in a T-A base pair in the *SMN2* gene being mutated to a C-G base pair in the *SMN2* gene. In some embodiments, deaminating the adenosine nucleobase in the *SMN2* gene leads to an increase in SMN protein. In some embodiments, deaminating the adenosine nucleobase in the *SMN2* gene results in correcting a sequence associated with SMA. In some embodiments, deaminating the adenosine nucleobase in the *SMN2* gene ameliorates one or more symptoms of SMA.

In some embodiments, the *SMN2* is centromeric survival of motor neuron 2 from *Homo sapiens.* For example, Gene ID: 6607, which may also be referred to as SMNC, BCD541, GEMIN1, TDRD16B, or C-BCD541. *See,* for example, NG_008728.1 RefSeqGene.

### I. SMN sequences

In various aspects, the disclosure references the *SMN1* gene and the *SMN2* genes, with the *SMN2* gene being targeted for editing by the base editor constructs and compositions described herein. The full-length human SMN1 and SMN2 proteins and their nucleotide sequences are provided below, in Table 1.

**Table 1: The nucleotide and amino acid sequences of SMN1 and SMN2 in humans.**

| Description | Sequence | SEQ ID NO: |
|---|---|---|
| SMN1 survival of motor neuron 1, telomeric [ Homo sapiens (human) ] | CCACAAATGTGGGAGGGCGATAACCACTCGTAGAAAGC GTGAGAAGTTACTACAAGCGGTCCTCCCGGCCACCGTA CTGTTCCGCTCCCAGAAGCCCCGGGCGGCGGAAGTCGT CACTCTTAAGAAGGGACGGGGCCCCACGCTGCGCACCC GCGGGTTTGCTATGGCGATGAGCAGCGGCGGCAGTGGT GGCGGCGTCCCGGAGCAGGAGGATTCCGTGCTGTTCCG GCGCGGCACAGGCCAGAGCGATGATTCTGACATTTGGG ATGATACAGCACTGATAAAAGCATATGATAAAGCTGTG GCTTCATTTAAGCATGCTCTAAAGAATGGTGACATTTGT GAAACTTCGGGTAAACCAAAAACCACACCTAAAAGAA AACCTGCTAAGAAGAATAAAAGCCAAAAGAAGAATAC TGCAGCTTCCTTACAACAGTGGAAAGTTGGGGACAAAT GTTCTGCCATTTGGTCAGAAGACGGTTGCATTTACCCAG CTACCATTGCTTCAATTGATTTTAAGAGAGAAACCTGTG TTGTGGTTTACACTGGATATGGAAATAGAGAGGAGCAA AATCTGTCCGATCTACTTTCCCCAATCTGTGAAGTAGCT AATAATATAGAACAAAATGCTCAAGAGAATGAAAATG AAAGCCAAGTTTCAACAGATGAAAGTGAGAACTCCAGG TCTCCTGGAAATAAATCAGATAACATCAAGCCCAAATC TGCTCCATGGAACTCTTTTCTCCCTCCACCACCCCCCAT GCCAGGGCCAAGACTGGGACCAGGAAAGCCAGGTCTA AAATTCAATGGCCCACCACCGCCACCGCCACCACCACC ACCCCACTTACTATCATGCTGGCTGCCTCCATTTCCTTC TGGACCACCAATAATTCCCCCACCACCTCCCATATGTCC AGATTCTCTTGATGATGCTGATGCTTTGGGAAGTATGTT AATTTCATGGTACATGAGTGGCTATCATACTGGCTATTA TATGGAAATGCTGGCATAGAGCAGCACTAAATGACACC ACTAAAGAAACGATCAGACAGATCTGGAATGTGAAGC GTTATAGAAGATAACTGGCCTCATTTCTTCAAAATATCA AGTGTTGGGAAAGAAAAAAGGAAGTGGAATGGGTAAC TCTTCTTGATTAAAAGTTATGTAATAACCAAATGCAATG TGAAATATTTTACTGGACTCTATTTTGAAAAACCATCTG TAAAAGACTGGGGTGGGGGTGGGAGGCCAGCACGGTG | 1 |
| | GTGAGGCAGTTGAGAAAATTTGAATGTGGATTAGATTT TGAATGATATTGGATAATTATTGGTAATTTTATGAGCTG TGAGAAGGGTGTTGTAGTTTATAAAAGACTGTCTTAATT TGCATACTTAAGCATTTAGGAATGAAGTGTTAGAGTGT CTTAAAATGTTTCAAATGGTTTAACAAAATGTATGTGA GGCGTATGTGGCAAAATGTTACAGAATCTAACTGGTGG ACATGGCTGTTCATTGTACTGTTTTTTTCTATCTTCTATA TGTTTAAAAGTATATAATAAAAATATTTAATTTTTTTTT AAA | |
| SMN1 survival of motor neuron 1, telomeric [ Homo sapiens (human) ] | MAMSSGGSGGGVPEQEDSVLFRRGTGQSDDSDIWDDTAL IKAYDKAVASFKHALKNGDICETSGKPKTTPKRKPAKKN KSQKKNTAASLQQWKVGDKCSAIWSEDGCIYPATIASIDF KRETCVVVYTGYGNREEQNLSDLLSPICEVANNIEQNAQE NENESQVSTDESENSRSPGNKSDNIKPKSAPWNSFLPPPPP MPGPRLGPGKPGLKFNGPPPPPPPPPPHLLSCWLPPFPSGPP IIPPPPPICPDSLDDADALGSMLISWYMSGYHTGYYMGFRQ NQKEGRCSHSLN | 2 |
| SMN2 survival of motor neuron 1, telomeric [ Homo sapiens (human) ] | GCACCCGCGGGTTTGCTATGGCGATGAGCAGCGGCGGC AGTGGTGGCGGCGTCCCGGAGCAGGAGGATTCCGTGCT GTTCCGGCGCGGCACAGGCCAGAGCGATGATTCTGACA TTTGGGATGATACAGCACTGATAAAAGCATATGATAAA GCTGTGGCTTCATTTAAGCATGCTCTAAAGAATGGTGA CATTTGTGAAACTTCGGGTA AACCAAAAACCACACCTAAAAGAAAACCTGCTAAGAA GAATAAAAGCCAAAAGAAGAATACTGCAGCTTCCTTAC AACAGTGGAAAGTTGGGGACAAATGTTCTGCCATTTGG TCAGAAGACGGTTGCATTTACCCAGCTACCATTGCTTCA ATTGATTTTAAGAGAGAAACCTGTGTTGTGGTTTACACT GGATATGGAAATAGAGAGGAGCAAAATCTGTCCGATCT ACTTTCCCCAATCTGTGAAGTAGCTAATAATATAGAAC AAAATGCTCAAGAGAATGAAAATGAAAGCCAAGTTTCA ACAGATGAAAGTGAGAACTCCAGGTCTCCTGGAAATAA ATCAGATAACATCAAGCCCAAATCTGCTCCATGGAACT CTTTTCTCCCTCCACCACCCCCCATGCCAGGGCCAAGAC TGGGACCAGGAAAGCCAGGTCTAAAATTCAATGGCCCA CCACCGCCACCGCCACCACCACCACCCCACTTACTATC | 3 |
| | ATGCTGGCTGCCTCCATTTCCTTCTGGACCACCAATAAT TCCCCCACCACCTCCCATATGTCCAGATTCTCTTGATGA TGCTGATGCTTTGGGAAGTATGTTAATTTCATGGTACAT GAGTGGCTATCATACTGGCTATTATATGGAAATGCTGG CATAGAGCAGCACTAAATGACACCACTAAAGAAACGAT CAGACAGATCTGGAATGTGAAGCGTTATAGAAGATAAC TGGCCTCATTTCTTCAAAATATCAAGTGTTGGGAAAGA AAAAAGGAAGTGGAATGGGTAACTCTTCTTGATTAAAA GTTATGTAATAACCAAATGCAATGTGAAATATTTTACTG GACTCTATTTTGAAAAACCATCTGTAAAAGACTGAGGT GGGGGTGGGAGGCCAGCACGGTGGTGAGGCAGTTGAG AAAATTTGAATGTGGATTAGATTTTGAATGATATTGGAT AATTATTGGTAATTTTATGAGCTGTGAGAAGGGTGTTGT AGTTTATAAAAGACTGTCTTAATTTGCATACTTAAGCAT TTAGGAATGAAGTGTTAGAGTGTCTTAAAATGTTTCAA ATGGTTTAACAAAATGTATGTGAGGCGTATGTGGCAAA ATGTTACAGAATCTAACTGGTGGACATGGCTGTTCATTG TACTGTTTTTTTCTATCTTCTATATGTTTAAAAGTATATA ATAAAAATATTTAATTTTTTTTTAAATTA | |
| SMN2 survival of motor neuron 1, telomeric [ Homo sapiens (human) ] | MAMSSGGSGGGVPEQEDSVLFRRGTGQSDDSDIWDDTAL IKAYDKAVASFKHALKNGDICETSGKPKTTPKRKPAKKN KSQKKNTAASLQQWKVGDKCSAIWSEDGCIYPATIASIDF KRETCVVVYTGYGNREEQNLSDLLSPICEVANNIEQNAQE NENESQVSTDESENSRSPGNKSDNIKPKSAPWNSFLPPPPP MPGPRLGPGKPGLKFNGPPPPPPPPPPHLLSCWLPPFPSGPP IIPPPPPICPDSLDDADALGSMLISWYMSGYHTGYYMEM LA | 4 |

### II. napDNAbp (Cas9 Domains)

In one aspect, the methods and base editor compositions described herein involve a nucleic acid programmable DNA binding protein (napDNAbp). Each napDNAbp is associated with at least one guide nucleic acid (e.g., guide RNA), which localizes the napDNAbp to a DNA sequence that comprises a DNA strand (i.e., a target strand) that is complementary to the guide nucleic acid, or a portion thereof (e.g., the spacer of a guide RNA that anneals to the protospacer of the DNA target). In other words, the guide nucleic-acid "programs" the napDNAbp (e.g., Cas9 or equivalent) to localize and bind to a complementary sequence of the protospacer in the DNA. In various embodiments, the napDNAbp can be fused to a herein disclosed adenosine deaminase or cytidine deaminase. In some embodiments, a napDNAbp (*e.g*., Cas9) can be used in the methods described herein to induce formation of an indel in SMN2, preventing exon skipping.

Any suitable napDNAbp may be used in the methods and base editor compositions described herein. In various embodiments, the napDNAbp may be any Class 2 CRISPR-Cas system, including any type II, type V, or type VI CRISPR-Cas enzyme. Given the rapid development of CRISPR-Cas as a tool for genome editing, there have been constant developments in the nomenclature used to describe and/or identify CRISPR-Cas enzymes, such as Cas9 and Cas9 orthologs. This application references CRISPR-Cas enzymes with nomenclature that may be old and/or new. The skilled person will be able to identify the specific CRISPR-Cas enzyme being referenced in this Application based on the nomenclature that is used, whether it is old (i.e., "legacy") or new nomenclature. CRISPR-Cas nomenclature is extensively discussed in Makarova et al., "Classification and Nomenclature of CRISPR-Cas Systems: Where from Here?," The CRISPR Journal, Vol. 1. No. 5, 2018, the entire contents of which are incorporated herein by reference. The particular CRISPR-Cas nomenclature used in any given instance in this Application is not limiting in any way and the skilled person will be able to identify which CRISPR-Cas enzyme is being referenced.

For example, the following type II, type V, and type VI Class 2 CRISPR-Cas enzymes have the following art-recognized old (i.e., legacy) and new names. Each of these enzymes, and/or variants thereof, may be used with the methods and base editor compositions described herein:

| **Legacy nomenclature** | **Current nomenclature*** |
|---|---|
| *type II CRISPR-Cas enzymes* | |
| Cas9 | same |

| *type V CRISPR-Cas enzymes* | |
|---|---|
| Cpf1 | Cas12a |
| CasX | Cas12e |
| C2c1 | Cas12b1 |
| Cas12b2 | same |
| C2c3 | Cas12c |
| CasY | Cas12d |
| C2c4 | same |
| C2c8 | same |
| C2c5 | same |
| C2c10 | same |
| C2c9 | same |

| *type VI CRISPR-Cas enzymes* | |
|---|---|
| C2c2 | Cas13a |

| Cas13d | same |
|---|---|
| C2c7 | Cas13c |
| C2c6 | Cas13b |

| | |
|---|---|
| * See Makarova et al., The CRISPR Journal, Vol. 1, No. 5, 2018 | |

Without being bound by any particular theory, the binding mechanism of certain napDNAbps contemplated herein includes the step of forming an R-loop whereby the napDNAbp induces the unwinding of a double-strand DNA target, thereby separating the strands in the region bound by the napDNAbp. The guide RNA spacer then hybridizes to the target strand at the protospacer sequence. This displaces a "non-target strand" that is complementary to the target strand, which forms the single strand region of the R-loop. In some embodiments, the napDNAbp includes one or more nuclease activities, which then cut the DNA leaving various types of lesions. For example, the napDNAbp may comprises a nuclease activity that cuts the non-target strand at a first location, and/ or cuts the target strand at a second location. Depending on the nuclease activity, the target DNA can be cut to form a "double-stranded break" whereby both strands are cut. In other embodiments, the target DNA can be cut at only a single site, i.e., the DNA is "nicked" on one strand. Exemplary napDNAbp with different nuclease activities include "Cas9 nickase" ("nCas9") and a deactivated Cas9 having no nuclease activities ("dead Cas9" or "dCas9").

The below description of various napDNAbps which can be used in connection with the presently disclose base editors is not meant to be limiting in any way. The base editors may comprise the canonical SpCas9, or any ortholog Cas9 protein, or any variant Cas9 protein-including any naturally occurring variant, mutant, or otherwise engineered version of Cas9-that is known or which can be made or evolved through a directed evolutionary or otherwise mutagenic process. In various embodiments, the Cas9 or Cas9 variants have a nickase activity, i.e., only cleave one strand of the target DNA sequence. In other embodiments, the Cas9 or Cas9 variants have inactive nucleases, i.e., are "dead" Cas9 proteins. Other variant Cas9 proteins that may be used are those having a smaller molecular weight than the canonical SpCas9 (e.g., for easier delivery) or having modified or rearranged primary amino acid structure (e.g., the circular permutant formats).

The base editors described herein may also comprise Cas9 equivalents, including Cas12a (Cpf1) and Cas12b1 proteins which are the result of convergent evolution. The napDNAbps used herein (e.g., SpCas9, Cas9 variant, or Cas9 equivalents) may also may also contain various modifications that alter/enhance their PAM specifities. Lastly, the application contemplates any Cas9, Cas9 variant, or Cas9 equivalent which has at least 70%, at least 75%, at least 80%, at least 85%, at least 90%, at least 91%, at least 92%, at least 93%, at least 94%, at least 95%, at least 96%, at least 97%, at least 98%, at least 99%, or at least 99.9% sequence identity to a reference Cas9 sequence, such as a reference SpCas9 canonical sequence or a reference Cas9 equivalent (e.g., Cas12a (Cpf1)).

The napDNAbp can be a CRISPR (clustered regularly interspaced short palindromic repeat)-associated nuclease. As outlined above, CRISPR is an adaptive immune system that provides protection against mobile genetic elements (viruses, transposable elements and conjugative plasmids). CRISPR clusters contain spacers, sequences complementary to antecedent mobile elements, and target invading nucleic acids. CRISPR clusters are transcribed and processed into CRISPR RNA (crRNA). In type II CRISPR systems correct processing of pre-crRNA requires a trans-encoded small RNA (tracrRNA), endogenous ribonuclease 3 (rnc) and a Cas9 protein. The tracrRNA serves as a guide for ribonuclease 3-aided processing of pre-crRNA. Subsequently, Cas9/crRNA/tracrRNA endonucleolytically cleaves linear or circular dsDNA target complementary to the spacer. The target strand not complementary to crRNA is first cut endonucleolytically, then trimmed 3'-5' exonucleolytically. In nature, DNA-binding and cleavage typically requires protein and both RNAs. However, single guide RNAs ("sgRNA", or simply "gRNA") can be engineered so as to incorporate aspects of both the crRNA and tracrRNA into a single RNA species. See, e.g., Jinek M. et al., Science 337:816-821(2012), the entire contents of which is hereby incorporated by reference.

In some embodiments, the napDNAbp directs cleavage of one or both strands at the location of a target sequence, such as within the target sequence and/or within the complement of the target sequence. In some embodiments, the napDNAbp directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a napDNAbp that is mutated to with respect to a corresponding wild-type enzyme such that the mutated napDNAbp lacks the ability to cleave one or both strands of a target polynucleotide containing a target sequence. For example, an aspartate-to-alanine substitution (D10A) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A in reference to the canonical SpCas9 sequence, or to equivalent amino acid positions in other Cas9 variants or Cas9 equivalents.

As used herein, the term "Cas protein" refers to a full-length Cas protein obtained from nature, a recombinant Cas protein having a sequences that differs from a naturally occurring Cas protein, or any fragment of a Cas protein that nevertheless retains all or a significant amount of the requisite basic functions needed for the disclosed methods, i.e., (i) possession of nucleic-acid programmable binding of the Cas protein to a target DNA, and (ii) ability to nick the target DNA sequence on one strand. The Cas proteins contemplated herein embrace CRISPR Cas 9 proteins, as well as Cas9 equivalents, variants (e.g., Cas9 nickase (nCas9) or nuclease inactive Cas9 (dCas9)) homologs, orthologs, or paralogs, whether naturally occurring or non-naturally occurring (e.g., engineered or recombinant), and may include a Cas9 equivalent from any Class 2 CRISPR system (e.g., type II, V, VI), including Cas12a (Cpf1), Cas12e (CasX), Cas12b1 (C2c1), Cas12b2, Cas12c (C2c3), C2c4, C2c8, C2c5, C2c10, C2c9 Cas13a (C2c2), Cas13d, Cas13c (C2c7), Cas13b (C2c6), and Cas13b. Further Cas-equivalents are described in Makarova et al., "C2c2 is a single-component programmable RNA-guided RNA-targeting CRISPR effector," Science 2016; 353(6299) and Makarove et al., "Classification and Nomenclature of CRISPR-Cas Systems: Where from Here?," The CRISPR Journal, Vol. 1 No. 5, 2018, the contents of which are incorporated herein by reference.

The terms "Cas9" or "Cas9 nuclease" or "Cas9 moiety" or "Cas9 domain" embrace any naturally occurring Cas9 from any organism, any naturally-occurring Cas9 equivalent or functional fragment thereof, any Cas9 homolog, ortholog, or paralog from any organism, and any mutant or variant of a Cas9, naturally-occurring or engineered. The term Cas9 is not meant to be particularly limiting and may be referred to as a "Cas9 or equivalent." Exemplary Cas9 proteins are further described herein and/or are described in the art and are incorporated herein by reference. The present disclosure is unlimited with regard to the particular Cas9 that is employed in the base editor (PE) of the invention.

As noted herein, Cas9 nuclease sequences and structures are well known to those of skill in the art (see, e.g., "Complete genome sequence of an M1 strain of Streptococcus pyogenes." Ferretti et al., J.J., McShan W.M., Ajdic D.J., Savic D.J., Savic G., Lyon K., Primeaux C., Sezate S., Suvorov A.N., Kenton S., Lai H.S., Lin S.P., Qian Y., Jia H.G., Najar F.Z., Ren Q., Zhu H., Song L., White J., Yuan X., Clifton S.W., Roe B.A., McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma C.M., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012), the entire contents of each of which are incorporated herein by reference).

Examples of Cas9 and Cas9 equivalents are provided as follows; however, these specific examples are not meant to be limiting. The base editor fusions of the present disclosure may use any suitable napDNAbp, including any suitable Cas9 or Cas9 equivalent.

### (1) Wild type canonical SpCas9

In one embodiment, the base editor constructs described herein may comprise the "canonical SpCas9" nuclease from *S. pyogenes,* which has been widely used as a tool for genome engineering and is categorized as the type II subgroup of enzyme of the Class 2 CRISPR-Cas systems. This Cas9 protein is a large, multi-domain protein containing two distinct nuclease domains. Point mutations can be introduced into Cas9 to abolish one or both nuclease activities, resulting in a nickase Cas9 (nCas9) or dead Cas9 (dCas9), respectively, that still retains its ability to bind DNA in a sgRNA-programmed manner. In principle, when fused to another protein or domain, Cas9 or a variant thereof (e.g., nCas9) can target that protein to virtually any DNA sequence simply by co-expression with an appropriate sgRNA. As used herein, the canonical SpCas9 protein refers to the wild type protein from *Streptococcus pyogenes* having the following amino acid sequence:

| Description | Sequence | SEQ ID NO: |
|---|---|---|
| SpCas9 *Streptococcu s pyogenes* M1 SwissProt Accession No. Q99ZW2 Wild type | | 5 |
| | | |
| SpCas9 Reverse translation of SwissProt Accession No. Q99ZW2 *Streptococcu s pyogenes* | ATGGATAAAAAATATAGCATTGGCCTGGATATTGGCACC AACAGCGTGGGCTGGGCGGTGATTACCGATGAATATAAA GTGCCGAGCAAAAAATTTAAAGTGCTGGGCAACACCGAT CGCCATAGCATTAAAAAAAACCTGATTGGCGCGCTGCTG TTTGATAGCGGCGAAACCGCGGAAGCGACCCGCCTGAAA CGCACCGCGCGCCGCCGCTATACCCGCCGCAAAAACCGC ATTTGCTATCTGCAGGAAATTTTTAGCAACGAAATGGCGA AAGTGGATGATAGCTTTTTTCATCGCCTGGAAGAAAGCTT TCTGGTGGAAGAAGATAAAAAACATGAACGCCATCCGAT TTTTGGCAACATTGTGGATGAAGTGGCGTATCATGAAAA ATATCCGACCATTTATCATCTGCGCAAAAAACTGGTGGAT AGCACCGATAAAGCGGATCTGCGCCTGATTTATCTGGCG CTGGCGCATATGATTAAATTTCGCGGCCATTTTCTGATTG AAGGCGATCTGAACCCGGATAACAGCGATGTGGATAAAC TGTTTATTCAGCTGGTGCAGACCTATAACCAGCTGTTTGA AGAAAACCCGATTAACGCGAGCGGCGTGGATGCGAAAGC GATTCTGAGCGCGCGCCTGAGCAAAAGCCGCCGCCTGGA AAACCTGATTGCGCAGCTGCCGGGCGAAAAAAAAAACGG CCTGTTTGGCAACCTGATTGCGCTGAGCCTGGGCCTGACC | 6 |
| | CCGAACTTTAAAAGCAACTTTGATCTGGCGGAAGATGCG AAACTGCAGCTGAGCAAAGATACCTATGATGATGATCTG GATAACCTGCTGGCGCAGATTGGCGATCAGTATGCGGAT CTGTTTCTGGCGGCGAAAAACCTGAGCGATGCGATTCTGC TGAGCGATATTCTGCGCGTGAACACCGAAATTACCAAAG CGCCGCTGAGCGCGAGCATGATTAAACGCTATGATGAAC ATCATCAGGATCTGACCCTGCTGAAAGCGCTGGTGCGCC AGCAGCTGCCGGAAAAATATAAAGAAATTTTTTTGATC AGAGCAAAAACGGCTATGCGGGCTATATTGATGGCGGCG CGAGCCAGGAAGAATTTTATAAATTTATTAAACCGATTCT GGAAAAAATGGATGGCACCGAAGAACTGCTGGTGAAACT GAACCGCGAAGATCTGCTGCGCAAACAGCGCACCTTTGA TAACGGCAGCATTCCGCATCAGATTCATCTGGGCGAACT GCATGCGATTCTGCGCCGCCAGGAAGATTTTTATCCGTTT CTGAAAGATAACCGCGAAAAAATTGAAAAAATTCTGACC TTTCGCATTCCGTATTATGTGGGCCCGCTGGCGCGCGGCA ACAGCCGCTTTGCGTGGATGACCCGCAAAAGCGAAGAAA CCATTACCCCGTGGAACTTTGAAGAAGTGGTGGATAAAG GCGCGAGCGCGCAGAGCTTTATTGAACGCATGACCAACT TTGATAAAAACCTGCCGAACGAAAAAGTGCTGCCGAAAC ATAGCCTGCTGTATGAATATTTTACCGTGTATAACGAACT GACCAAAGTGAAATATGTGACCGAAGGCATGCGCAAACC GGCGTTTCTGAGCGGCGAACAGAAAAAAGCGATTGTGGA TCTGCTGTTTAAAACCAACCGCAAAGTGACCGTGAAACA GCTGAAAGAAGATTATTTTAAAAAAATTGAATGCTTTGAT AGCGTGGAAATTAGCGGCGTGGAAGATCGCTTTAACGCG AGCCTGGGCACCTATCATGATCTGCTGAAAATTATTAAAG ATAAAGATTTTCTGGATAACGAAGAAAACGAAGATATTC TGGAAGATATTGTGCTGACCCTGACCCTGTTTGAAGATCG CGAAATGATTGAAGAACGCCTGAAAACCTATGCGCATCT GTTTGATGATAAAGTGATGAAACAGCTGAAACGCCGCCG CTATACCGGCTGGGGCCGCCTGAGCCGCAAACTGATTAA CGGCATTCGCGATAAACAGAGCGGCAAAACCATTCTGGA TTTTCTGAAAAGCGATGGCTTTGCGAACCGCAACTTTATG CAGCTGATTCATGATGATAGCCTGACCTTTAAAGAAGAT ATTCAGAAAGCGCAGGTGAGCGGCCAGGGCGATAGCCTG | |
| | CATGAACATATTGCGAACCTGGCGGGCAGCCCGGCGATT AAAAAAGGCATTCTGCAGACCGTGAAAGTGGTGGATGAA CTGGTGAAAGTGATGGGCCGCCATAAACCGGAAAACATT GTGATTGAAATGGCGCGCGAAAACCAGACCACCCAGAAA GGCCAGAAAAACAGCCGCGAACGCATGAAACGCATTGA AGAAGGCATTAAAGAACTGGGCAGCCAGATTCTGAAAGA ACATCCGGTGGAAAACACCCAGCTGCAGAACGAAAAACT GTATCTGTATTATCTGCAGAACGGCCGCGATATGTATGTG GATCAGGAACTGGATATTAACCGCCTGAGCGATTATGAT GTGGATCATATTGTGCCGCAGAGCTTTCTGAAAGATGATA GCATTGATAACAAAGTGCTGACCCGCAGCGATAAAAACC GCGGCAAAAGCGATAACGTGCCGAGCGAAGAAGTGGTG AAAAAAATGAAAAACTATTGGCGCCAGCTGCTGAACGCG AAACTGATTACCCAGCGCAAATTTGATAACCTGACCAAA GCGGAACGCGGCGGCCTGAGCGAACTGGATAAAGCGGG CTTTATTAAACGCCAGCTGGTGGAAACCCGCCAGATTACC AAACATGTGGCGCAGATTCTGGATAGCCGCATGAACACC AAATATGATGAAAACGATAAACTGATTCGCGAAGTGAAA GTGATTACCCTGAAAAGCAAACTGGTGAGCGATTTTCGC AAAGATTTTCAGTTTTATAAAGTGCGCGAAATTAACAACT ATCATCATGCGCATGATGCGTATCTGAACGCGGTGGTGG GCACCGCGCTGATTAAAAAATATCCGAAACTGGAAAGCG AATTTGTGTATGGCGATTATAAAGTGTATGATGTGCGCAA AATGATTGCGAAAAGCGAACAGGAAATTGGCAAAGCGA CCGCGAAATATTTTTTTTATAGCAACATTATGAACTTTTTT AAAACCGAAATTACCCTGGCGAACGGCGAAATTCGCAAA CGCCCGCTGATTGAAACCAACGGCGAAACCGGCGAAATT GTGTGGGATAAAGGCCGCGATTTTGCGACCGTGCGCAAA GTGCTGAGCATGCCGCAGGTGAACATTGTGAAAAAAACC GAAGTGCAGACCGGCGGCTTTAGCAAAGAAAGCATTCTG CCGAAACGCAACAGCGATAAACTGATTGCGCGCAAAAAA GATTGGGATCCGAAAAAATATGGCGGCTTTGATAGCCCG ACCGTGGCGTATAGCGTGCTGGTGGTGGCGAAAGTGGAA AAAGGCAAAAGCAAAAAACTGAAAAGCGTGAAAGAACT GCTGGGCATTACCATTATGGAACGCAGCAGCTTTGAAAA AAACCCGATTGATTTTCTGGAAGCGAAAGGCTATAAAGA | |
| | AGTGAAAAAAGATCTGATTATTAAACTGCCGAAATATAG CCTGTTTGAACTGGAAAACGGCCGCAAACGCATGCTGGC GAGCGCGGGCGAACTGCAGAAAGGCAACGAACTGGCGC TGCCGAGCAAATATGTGAACTTTCTGTATCTGGCGAGCCA TTATGAAAAACTGAAAGGCAGCCCGGAAGATAACGAACA GAAACAGCTGTTTGTGGAACAGCATAAACATTATCTGGA TGAAATTATTGAACAGATTAGCGAATTTAGCAAACGCGT GATTCTGGCGGATGCGAACCTGGATAAAGTGCTGAGCGC GTATAACAAACATCGCGATAAACCGATTCGCGAACAGGC GGAAAACATTATTCATCTGTTTACCCTGACCAACCTGGGC GCGCCGGCGGCGTTTAAATATTTTGATACCACCATTGATC GCAAACGCTATACCAGCACCAAAGAAGTGCTGGATGCGA CCCTGATTCATCAGAGCATTACCGGCCTGTATGAAACCCG CATTGATCTGAGCCAGCTGGGCGGCGAT | |

The base editors described herein may include canonical SpCas9, or any variant thereof having at least 80%, at least 85%, at least 90%, at least 95%, or at least 99% sequence identity with a wild type Cas9 sequence provided above. These variants may include SpCas9 variants containing one or more mutations, including any known mutation reported with the SwissProt Accession No. Q99ZW2 entry, which include:

| SpCas9 mutation (relative to the amino acid sequence of the canonical SpCas9 sequence, SEQ ID NO: 5) | Function/Characteristic (as reported) (see UniProtKB - Q99ZW2 (CAS9_STRPT1) entry - incorporated herein by reference) |
|---|---|
| D10A | Nickase mutant which cleaves the protospacer strand (but no cleavage of non-protospacer strand) |
| S15A | Decreased DNA cleavage activity |
| R66A | Decreased DNA cleavage activity |
| R70A | No DNA cleavage |
| R74A | Decreased DNA cleavage |
| R78A | Decreased DNA cleavage |
| 97-150 deletion | No nuclease activity |
| R165A | Decreased DNA cleavage |
| 175-307 deletion | About 50% decreased DNA cleavage |
| 312-409 deletion | No nuclease activity |
| E762A | Nickase |
| H840A | Nickase mutant which cleaves the non-protospacer strand but does not cleave the protospacer strand |
| N854A | Nickase |
| N863A | Nickase |
| H982A | Decreased DNA cleavage |
| D986A | Nickase |
| 1099-1368 deletion | No nuclease activity |
| R1333A | Reduced DNA binding |

Other wild type SpCas9 sequences that may be used in the present disclosure, include:

| Description | Sequence | SEQ ID NO: |
|---|---|---|
| SpCas9 *Streptococcus pyogenes* MGAS1882 wild type NC_017053.1 | ATGGATAAGAAATACTCAATAGGCTTAGATATCGGCACAAA TAGCGTCGGATGGGCGGTGATCACTGATGATTATAAGGTTC CGTCTAAAAAGTTCAAGGTTCTGGGAAATACAGACCGCCAC AGTATCAAAAAAAATCTTATAGGGGCTCTTTTATTTGGCAG TGGAGAGACAGCGGAAGCGACTCGTCTCAAACGGACAGCT CGTAGAAGGTATACACGTCGGAAGAATCGTATTTGTTATCT ACAGGAGATTTTTTCAAATGAGATGGCGAAAGTAGATGATA GTTTCTTTCATCGACTTGAAGAGTCTTTTTTGGTGGAAGAAG ACAAGAAGCATGAACGTCATCCTATTTTTGGAAATATAGTA GATGAAGTTGCTTATCATGAGAAATATCCAACTATCTATCA TCTGCGAAAAAAATTGGCAGATTCTACTGATAAAGCGGATT TGCGCTTAATCTATTTGGCCTTAGCGCATATGATTAAGTTTC GTGGTCATTTTTTGATTGAGGGAGATTTAAATCCTGATAATA GTGATGTGGACAAACTATTTATCCAGTTGGTACAAATCTAC AATCAATTATTTGAAGAAAACCCTATTAACGCAAGTAGAGT AGATGCTAAAGCGATTCTTTCTGCACGATTGAGTAAATCAA GACGATTAGAAAATCTCATTGCTCAGCTCCCCGGTGAGAAG AGAAATGGCTTGTTTGGGAATCTCATTGCTTTGTCATTGGGA TTGACCCCTAATTTTAAATCAAATTTTGATTTGGCAGAAGAT GCTAAATTACAGCTTTCAAAAGATACTTACGATGATGATTT AGATAATTTATTGGCGCAAATTGGAGATCAATATGCTGATT TGTTTTTGGCAGCTAAGAATTTATCAGATGCTATTTTACTTT CAGATATCCTAAGAGTAAATAGTGAAATAACTAAGGCTCCC CTATCAGCTTCAATGATTAAGCGCTACGATGAACATCATCA AGACTTGACTCTTTTAAAAGCTTTAGTTCGACAACAACTTCC AGAAAAGTATAAAGAAATCTTTTTTGATCAATCAAAAAACG | 7 |
| | GATATGCAGGTTATATTGATGGGGGAGCTAGCCAAGAAGA ATTTTATAAATTTATCAAACCAATTTTAGAAAAAATGGATG GTACTGAGGAATTATTGGTGAAACTAAATCGTGAAGATTTG CTGCGCAAGCAACGGACCTTTGACAACGGCTCTATTCCCCA TCAAATTCACTTGGGTGAGCTGCATGCTATTTTGAGAAGAC AAGAAGACTTTTATCCATTTTTAAAAGACAATCGTGAGAAG ATTGAAAAAATCTTGACTTTTCGAATTCCTTATTATGTTGGT CCATTGGCGCGTGGCAATAGTCGTTTTGCATGGATGACTCG GAAGTCTGAAGAAACAATTACCCCATGGAATTTTGAAGAAG TTGTCGATAAAGGTGCTTCAGCTCAATCATTTATTGAACGC ATGACAAACTTTGATAAAAATCTTCCAAATGAAAAAGTACT ACCAAAACATAGTTTGCTTTATGAGTATTTTACGGTTTATAA CGAATTGACAAAGGTCAAATATGTTACTGAGGGAATGCGA AAACCAGCATTTCTTTCAGGTGAACAGAAGAAAGCCATTGT TGATTTACTCTTCAAAACAAATCGAAAAGTAACCGTTAAGC AATTAAAAGAAGATTATTTCAAAAAAATAGAATGTTTTGAT AGTGTTGAAATTTCAGGAGTTGAAGATAGATTTAATGCTTC ATTAGGCGCCTACCATGATTTGCTAAAAATTATTAAAGATA AAGATTTTTTGGATAATGAAGAAAATGAAGATATCTTAGAG GATATTGTTTTAACATTGACCTTATTTGAAGATAGGGGGAT GATTGAGGAAAGACTTAAAACATATGCTCACCTCTTTGATG ATAAGGTGATGAAACAGCTTAAACGTCGCCGTTATACTGGT TGGGGACGTTTGTCTCGAAAATTGATTAATGGTATTAGGGA TAAGCAATCTGGCAAAACAATATTAGATTTTTTGAAATCAG ATGGTTTTGCCAATCGCAATTTTATGCAGCTGATCCATGATG ATAGTTTGACATTTAAAGAAGATATTCAAAAAGCACAGGTG TCTGGACAAGGCCATAGTTTACATGAACAGATTGCTAACTT AGCTGGCAGTCCTGCTATTAAAAAAGGTATTTTACAGACTG TAAAAATTGTTGATGAACTGGTCAAAGTAATGGGGCATAAG CCAGAAAATATCGTTATTGAAATGGCACGTGAAAATCAGAC AACTCAAAAGGGCCAGAAAAATTCGCGAGAGCGTATGAAA CGAATCGAAGAAGGTATCAAAGAATTAGGAAGTCAGATTC TTAAAGAGCATCCTGTTGAAAATACTCAATTGCAAAATGAA AAGCTCTATCTCTATTATCTACAAAATGGAAGAGACATGTA TGTGGACCAAGAATTAGATATTAATCGTTTAAGTGATTATG ATGTCGATCACATTGTTCCACAAAGTTTCATTAAAGACGAT | |
| | TCAATAGACAATAAGGTACTAACGCGTTCTGATAAAAATCG TGGTAAATCGGATAACGTTCCAAGTGAAGAAGTAGTCAAA AAGATGAAAAACTATTGGAGACAACTTCTAAACGCCAAGTT AATCACTCAACGTAAGTTTGATAATTTAACGAAAGCTGAAC GTGGAGGTTTGAGTGAACTTGATAAAGCTGGTTTTATCAAA CGCCAATTGGTTGAAACTCGCCAAATCACTAAGCATGTGGC ACAAATTTTGGATAGTCGCATGAATACTAAATACGATGAAA ATGATAAACTTATTCGAGAGGTTAAAGTGATTACCTTAAAA TCTAAATTAGTTTCTGACTTCCGAAAAGATTTCCAATTCTAT AAAGTACGTGAGATTAACAATTACCATCATGCCCATGATGC GTATCTAAATGCCGTCGTTGGAACTGCTTTGATTAAGAAAT ATCCAAAACTTGAATCGGAGTTTGTCTATGGTGATTATAAA GTTTATGATGTTCGTAAAATGATTGCTAAGTCTGAGCAAGA AATAGGCAAAGCAACCGCAAAATATTTCTTTTACTCTAATA TCATGAACTTCTTCAAAACAGAAATTACACTTGCAAATGGA GAGATTCGCAAACGCCCTCTAATCGAAACTAATGGGGAAA CTGGAGAAATTGTCTGGGATAAAGGGCGAGATTTTGCCACA GTGCGCAAAGTATTGTCCATGCCCCAAGTCAATATTGTCAA GAAAACAGAAGTACAGACAGGCGGATTCTCCAAGGAGTCA ATTTTACCAAAAAGAAATTCGGACAAGCTTATTGCTCGTAA AAAAGACTGGGATCCAAAAAAATATGGTGGTTTTGATAGTC CAACGGTAGCTTATTCAGTCCTAGTGGTTGCTAAGGTGGAA AAAGGGAAATCGAAGAAGTTAAAATCCGTTAAAGAGTTAC TAGGGATCACAATTATGGAAAGAAGTTCCTTTGAAAAAAAT CCGATTGACTTTTTAGAAGCTAAAGGATATAAGGAAGTTAA AAAAGACTTAATCATTAAACTACCTAAATATAGTCTTTTTG AGTTAGAAAACGGTCGTAAACGGATGCTGGCTAGTGCCGG AGAATTACAAAAAGGAAATGAGCTGGCTCTGCCAAGCAAA TATGTGAATTTTTTATATTTAGCTAGTCATTATGAAAAGTTG AAGGGTAGTCCAGAAGATAACGAACAAAAACAATTGTTTG TGGAGCAGCATAAGCATTATTTAGATGAGATTATTGAGCAA ATCAGTGAATTTTCTAAGCGTGTTATTTTAGCAGATGCCAAT TTAGATAAAGTTCTTAGTGCATATAACAAACATAGAGACAA ACCAATACGTGAACAAGCAGAAAATATTATTCATTTATTTA CGTTGACGAATCTTGGAGCTCCCGCTGCTTTTAAATATTTTG ATACAACAATTGATCGTAAACGATATACGTCTACAAAAGAA | |
| | GTTTTAGATGCCACTCTTATCCATCAATCCATCACTGGTCTT TATGAAACACGCATTGATTTGAGTCAGCTAGGAGGTGACTG A | |
| SpCas9 *Streptococcus pyogenes* MGAS1882 wild type NC_017053.1 | MDKKYSIGLDIGTNSVGWAVITDDYKVPSKKFKVLGNTDRHS IKKNLIGALLFGSGETAEATRLKRTARRRYTRRKNRICYLQEIF SNEMAKVDDSFFHRLEESFLVEEDKKHERHPIFGNIVDEVAYH EKYPTIYHLRKKLADSTDKADLRLIYLALAHMIKFRGHFLIEG DLNPDNSDVDKLFIQLVQIYNQLFEENPINASRVDAKAILSARL SKSRRLENLIAQLPGEKRNGLFGNLIALSLGLTPNFKSNFDLAE DAKLQLSKDTYDDDLDNLLAQIGDQYADLFLAAKNLSDAILL SDILRVNSEITKAPLSASMIKRYDEHHQDLTLLKALVRQQLPE KYKEIFFDQSKNGYAGYIDGGASQEEFYKFIKPILEKMDGTEEL LVKLNREDLLRKQRTFDNGSIPHQIHLGELHAILRRQEDFYPFL KDNREKIEKILTFRIPYYVGPLARGNSRFAWMTRKSEETITPW NFEEVVDKGASAQSFIERMTNFDKNLPNEKVLPKHSLLYEYFT VYNELTKVKYVTEGMRKPAFLSGEQKKAIVDLLFKTNRKVTV KQLKEDYFKKIECFDSVEISGVEDRFNASLGAYHDLLKIIKDKD FLDNEENEDILEDIVLTLTLFEDRGMIEERLKTYAHLFDDKVM KQLKRRRYTGWGRLSRKLINGIRDKQSGKTILDFLKSDGFANR NFMQLIHDDSLTFKEDIQKAQVSGQGHSLHEQIANLAGSPAIK KGILQTVKIVDELVKVMGHKPENIVIEMARENQTTQKGQKNS RERMKRIEEGIKELGSQILKEHPVENTQLQNEKLYLYYLQNGR DMYVDQELDINRLSDYDVDHIVPQSFIKDDSIDNKVLTRSDKN RGKSDNVPSEEVVKKMKNYWRQLLNAKLITQRKFDNLTKAE RGGLSELDKAGFIKRQLVETRQITKHVAQILDSRMNTKYDEND KLIREVKVITLKSKLVSDFRKDFQFYKVREINNYHHAHDAYLN AVVGTALIKKYPKLESEFVYGDYKVYDVRKMIAKSEQEIGKA TAKYFFYSNIMNFFKTEITLANGEIRKRPLIETNGETGEIVWDK GRDFATVRKVLSMPQVNIVKKTEVQTGGFSKESILPKRNSDKL IARKKDWDPKKYGGFDSPTVAYSVLVVAKVEKGKSKKLKSV KELLGITIMERSSFEKNPIDFLEAKGYKEVKKDLIIKLPKYSLFE LENGRKRMLASAGELQKGNELALPSKYVNFLYLASHYEKLKG SPEDNEQKQLFVEQHKHYLDEIIEQISEFSKRVILADANLDKVL SAYNKHRDKPIREQAENIIHLFTLTNLGAPAAFKYFDTTIDRKR YTSTKEVLDATLIHQSITGLYETRIDLSQLGGD | 8 |
| SpCas9 *Streptococcus pyogenes* wild type SWBC2D7WO 14 | ATGGATAAAAAGTATTCTATTGGTTTAGACATCGGCACTAA TTCCGTTGGATGGGCTGTCATAACCGATGAATACAAAGTAC CTTCAAAGAAATTTAAGGTGTTGGGGAACACAGACCGTCAT TCGATTAAAAAGAATCTTATCGGTGCCCTCCTATTCGATAGT GGCGAAACGGCAGAGGCGACTCGCCTGAAACGAACCGCTC GGAGAAGGTATACACGTCGCAAGAACCGAATATGTTACTTA CAAGAAATTTTTAGCAATGAGATGGCCAAAGTTGACGATTC TTTCTTTCACCGTTTGGAAGAGTCCTTCCTTGTCGAAGAGGA CAAGAAACATGAACGGCACCCCATCTTTGGAAACATAGTA GATGAGGTGGCATATCATGAAAAGTACCCAACGATTTATCA CCTCAGAAAAAAGCTAGTTGACTCAACTGATAAAGCGGAC CTGAGGTTAATCTACTTGGCTCTTGCCCATATGATAAAGTTC CGTGGGCACTTTCTCATTGAGGGTGATCTAAATCCGGACAA CTCGGATGTCGACAAACTGTTCATCCAGTTAGTACAAACCT ATAATCAGTTGTTTGAAGAGAACCCTATAAATGCAAGTGGC GTGGATGCGAAGGCTATTCTTAGCGCCCGCCTCTCTAAATC CCGACGGCTAGAAAACCTGATCGCACAATTACCCGGAGAG AAGAAAAATGGGTTGTTCGGTAACCTTATAGCGCTCTCACT AGGCCTGACACCAAATTTTAAGTCGAACTTCGACTTAGCTG AAGATGCCAAATTGCAGCTTAGTAAGGACACGTACGATGAC GATCTCGACAATCTACTGGCACAAATTGGAGATCAGTATGC GGACTTATTTTTGGCTGCCAAAAACCTTAGCGATGCAATCC TCCTATCTGACATACTGAGAGTTAATACTGAGATTACCAAG GCGCCGTTATCCGCTTCAATGATCAAAAGGTACGATGAACA TCACCAAGACTTGACACTTCTCAAGGCCCTAGTCCGTCAGC AACTGCCTGAGAAATATAAGGAAATATTCTTTGATCAGTCG AAAAACGGGTACGCAGGTTATATTGACGGCGGAGCGAGTC AAGAGGAATTCTACAAGTTTATCAAACCCATATTAGAGAAG ATGGATGGGACGGAAGAGTTGCTTGTAAAACTCAATCGCGA AGATCTACTGCGAAAGCAGCGGACTTTCGACAACGGTAGC ATTCCACATCAAATCCACTTAGGCGAATTGCATGCTATACTT AGAAGGCAGGAGGATTTTTATCCGTTCCTCAAAGACAATCG TGAAAAGATTGAGAAAATCCTAACCTTTCGCATACCTTACT ATGTGGGACCCCTGGCCCGAGGGAACTCTCGGTTCGCATGG ATGACAAGAAAGTCCGAAGAAACGATTACTCCATGGAATTT TGAGGAAGTTGTCGATAAAGGTGCGTCAGCTCAATCGTTCA | 9 |
| | TCGAGAGGATGACCAACTTTGACAAGAATTTACCGAACGAA AAAGTATTGCCTAAGCACAGTTTACTTTACGAGTATTTCAC AGTGTACAATGAACTCACGAAAGTTAAGTATGTCACTGAGG GCATGCGTAAACCCGCCTTTCTAAGCGGAGAACAGAAGAA AGCAATAGTAGATCTGTTATTCAAGACCAACCGCAAAGTGA CAGTTAAGCAATTGAAAGAGGACTACTTTAAGAAAATTGAA TGCTTCGATTCTGTCGAGATCTCCGGGGTAGAAGATCGATT TAATGCGTCACTTGGTACGTATCATGACCTCCTAAAGATAA TTAAAGATAAGGACTTCCTGGATAACGAAGAGAATGAAGA TATCTTAGAAGATATAGTGTTGACTCTTACCCTCTTTGAAGA TCGGGAAATGATTGAGGAAAGACTAAAAACATACGCTCAC CTGTTCGACGATAAGGTTATGAAACAGTTAAAGAGGCGTCG CTATACGGGCTGGGGACGATTGTCGCGGAAACTTATCAACG GGATAAGAGACAAGCAAAGTGGTAAAACTATTCTCGATTTT CTAAAGAGCGACGGCTTCGCCAATAGGAACTTTATGCAGCT GATCCATGATGACTCTTTAACCTTCAAAGAGGATATACAAA AGGCACAGGTTTCCGGACAAGGGGACTCATTGCACGAACA TATTGCGAATCTTGCTGGTTCGCCAGCCATCAAAAAGGGCA TACTCCAGACAGTCAAAGTAGTGGATGAGCTAGTTAAGGTC ATGGGACGTCACAAACCGGAAAACATTGTAATCGAGATGG CACGCGAAAATCAAACGACTCAGAAGGGGCAAAAAAACAG TCGAGAGCGGATGAAGAGAATAGAAGAGGGTATTAAAGAA CTGGGCAGCCAGATCTTAAAGGAGCATCCTGTGGAAAATAC CCAATTGCAGAACGAGAAACTTTACCTCTATTACCTACAAA ATGGAAGGGACATGTATGTTGATCAGGAACTGGACATAAA CCGTTTATCTGATTACGACGTCGATCACATTGTACCCCAATC CTTTTTGAAGGACGATTCAATCGACAATAAAGTGCTTACAC GCTCGGATAAGAACCGAGGGAAAAGTGACAATGTTCCAAG CGAGGAAGTCGTAAAGAAAATGAAGAACTATTGGCGGCAG CTCCTAAATGCGAAACTGATAACGCAAAGAAAGTTCGATAA CTTAACTAAAGCTGAGAGGGGTGGCTTGTCTGAACTTGACA AGGCCGGATTTATTAAACGTCAGCTCGTGGAAACCCGCCAA ATCACAAAGCATGTTGCACAGATACTAGATTCCCGAATGAA TACGAAATACGACGAGAACGATAAGCTGATTCGGGAAGTC AAAGTAATCACTTTAAAGTCAAAATTGGTGTCGGACTTCAG AAAGGATTTTCAATTCTATAAAGTTAGGGAGATAAATAACT | |
| | ACCACCATGCGCACGACGCTTATCTTAATGCCGTCGTAGGG ACCGCACTCATTAAGAAATACCCGAAGCTAGAAAGTGAGTT TGTGTATGGTGATTACAAAGTTTATGACGTCCGTAAGATGA TCGCGAAAAGCGAACAGGAGATAGGCAAGGCTACAGCCAA ATACTTCTTTTATTCTAACATTATGAATTTCTTTAAGACGGA AATCACTCTGGCAAACGGAGAGATACGCAAACGACCTTTA ATTGAAACCAATGGGGAGACAGGTGAAATCGTATGGGATA AGGGCCGGGACTTCGCGACGGTGAGAAAAGTTTTGTCCATG CCCCAAGTCAACATAGTAAAGAAAACTGAGGTGCAGACCG GAGGGTTTTCAAAGGAATCGATTCTTCCAAAAAGGAATAGT GATAAGCTCATCGCTCGTAAAAAGGACTGGGACCCGAAAA AGTACGGTGGCTTCGATAGCCCTACAGTTGCCTATTCTGTCC TAGTAGTGGCAAAAGTTGAGAAGGGAAAATCCAAGAAACT GAAGTCAGTCAAAGAATTATTGGGGATAACGATTATGGAGC GCTCGTCTTTTGAAAAGAACCCCATCGACTTCCTTGAGGCG AAAGGTTACAAGGAAGTAAAAAAGGATCTCATAATTAAAC TACCAAAGTATAGTCTGTTTGAGTTAGAAAATGGCCGAAAA CGGATGTTGGCTAGCGCCGGAGAGCTTCAAAAGGGGAACG AACTCGCACTACCGTCTAAATACGTGAATTTCCTGTATTTAG CGTCCCATTACGAGAAGTTGAAAGGTTCACCTGAAGATAAC GAACAGAAGCAACTTTTTGTTGAGCAGCACAAACATTATCT CGACGAAATCATAGAGCAAATTTCGGAATTCAGTAAGAGA GTCATCCTAGCTGATGCCAATCTGGACAAAGTATTAAGCGC ATACAACAAGCACAGGGATAAACCCATACGTGAGCAGGCG GAAAATATTATCCATTTGTTTACTCTTACCAACCTCGGCGCT CCAGCCGCATTCAAGTATTTTGACACAACGATAGATCGCAA ACGATACACTTCTACCAAGGAGGTGCTAGACGCGACACTGA TTCACCAATCCATCACGGGATTATATGAAACTCGGATAGAT TTGTCACAGCTTGGGGGTGACGGATCCCCCAAGAAGAAGA GGAAAGTCTCGAGCGACTACAAAGACCATGACGGTGATTAT AAAGATCATGACATCGATTACAAGGATGACGATGACAAGG CTGCAGGA | |
| SpCas9 *Streptococcus pyogenes* wild type | MDKKYSIGLDIGTNSVGWAVITDEYKVPSKKFKVLGNTDRHSI KKNLIGALLFDSGETAEATRLKRTARRRYTRRKNRICYLQEIFS NEMAKVDDSFFHRLEESFLVEEDKKHERHPIFGNIVDEVAYHE KYPTIYHLRKKLVDSTDKADLRLIYLALAHMIKFRGHFLIEGD | 10 |
| Encoded product of SWBC2D7WO 14 | LNPDNSDVDKLFIQLVQTYNQLFEENPINASGVDAKAILSARLS KSRRLENLIAQLPGEKKNGLFGNLIALSLGLTPNFKSNFDLAED AKLQLSKDTYDDDLDNLLAQIGDQYADLFLAAKNLSDAILLS DILRVNTEITKAPLSASMIKRYDEHHQDLTLLKALVRQQLPEK YKEIFFDQSKNGYAGYIDGGASQEEFYKFIKPILEKMDGTEELL VKLNREDLLRKQRTFDNGSIPHQIHLGELHAILRRQEDFYPFLK DNREKIEKILTFRIPYYVGPLARGNSRFAWMTRKSEETITPWNF EEVVDKGASAQSFIERMTNFDKNLPNEKVLPKHSLLYEYFTVY NELTKVKYVTEGMRKPAFLSGEQKKAIVDLLFKTNRKVTVKQ LKEDYFKKIECFDSVEISGVEDRFNASLGTYHDLLKIIKDKDFL DNEENEDILEDIVLTLTLFEDREMIEERLKTYAHLFDDKVMKQ LKRRRYTGWGRLSRKLINGIRDKQSGKTILDFLKSDGFANRNF MQLIHDDSLTFKEDIQKAQVSGQGDSLHEHIANLAGSPAIKKGI LQTVKVVDELVKVMGRHKPENIVIEMARENQTTQKGQKNSRE RMKRIEEGIKELGSQILKEHPVENTQLQNEKLYLYYLQNGRDM YVDQELDINRLSDYDVDHIVPQSFLKDDSIDNKVLTRSDKNRG KSDNVPSEEVVKKMKNYWRQLLNAKLITQRKFDNLTKAERG GLSELDKAGFIKRQLVETRQITKHVAQILDSRMNTKYDENDKL IREVKVITLKSKLVSDFRKDFQFYKVREINNYHHAHDAYLNAV VGTALIKKYPKLESEFVYGDYKVYDVRKMIAKSEQEIGKATA KYFFYSNIMNFFKTEITLANGEIRKRPLIETNGETGEIVWDKGR DFATVRKVLSMPQVNIVKKTEVQTGGFSKESILPKRNSDKLIA RKKDWDPKKYGGFDSPTVAYSVLVVAKVEKGKSKKLKSVKE LLGITIMERSSFEKNPIDFLEAKGYKEVKKDLIIKLPKYSLFELE NGRKRMLASAGELQKGNELALPSKYVNFLYLASHYEKLKGSP EDNEQKQLFVEQHKHYLDEIIEQISEFSKRVILADANLDKVLSA YNKHRDKPIREQAENIIHLFTLTNLGAPAAFKYFDTTIDRKRYT STKEVLDATLIHQSITGLYETRIDLSQLGGDGSPKKKRKVSSDY KDHDGDYKDHDIDYKDDDDKAAG | |
| SpCas9 *Streptococcus pyogenes* M1GAS wild type NC_002737.2 | ATGGATAAGAAATACTCAATAGGCTTAGATATCGGCACAAA TAGCGTCGGATGGGCGGTGATCACTGATGAATATAAGGTTC CGTCTAAAAAGTTCAAGGTTCTGGGAAATACAGACCGCCAC AGTATCAAAAAAAATCTTATAGGGGCTCTTTTATTTGACAG TGGAGAGACAGCGGAAGCGACTCGTCTCAAACGGACAGCT CGTAGAAGGTATACACGTCGGAAGAATCGTATTTGTTATCT ACAGGAGATTTTTTCAAATGAGATGGCGAAAGTAGATGATA | 11 |
| | GTTTCTTTCATCGACTTGAAGAGTCTTTTTTGGTGGAAGAAG ACAAGAAGCATGAACGTCATCCTATTTTTGGAAATATAGTA GATGAAGTTGCTTATCATGAGAAATATCCAACTATCTATCA TCTGCGAAAAAAATTGGTAGATTCTACTGATAAAGCGGATT TGCGCTTAATCTATTTGGCCTTAGCGCATATGATTAAGTTTC GTGGTCATTTTTTGATTGAGGGAGATTTAAATCCTGATAATA GTGATGTGGACAAACTATTTATCCAGTTGGTACAAACCTAC AATCAATTATTTGAAGAAAACCCTATTAACGCAAGTGGAGT AGATGCTAAAGCGATTCTTTCTGCACGATTGAGTAAATCAA GACGATTAGAAAATCTCATTGCTCAGCTCCCCGGTGAGAAG AAAAATGGCTTATTTGGGAATCTCATTGCTTTGTCATTGGGT TTGACCCCTAATTTTAAATCAAATTTTGATTTGGCAGAAGAT GCTAAATTACAGCTTTCAAAAGATACTTACGATGATGATTT AGATAATTTATTGGCGCAAATTGGAGATCAATATGCTGATT TGTTTTTGGCAGCTAAGAATTTATCAGATGCTATTTTACTTT CAGATATCCTAAGAGTAAATACTGAAATAACTAAGGCTCCC CTATCAGCTTCAATGATTAAACGCTACGATGAACATCATCA AGACTTGACTCTTTTAAAAGCTTTAGTTCGACAACAACTTCC AGAAAAGTATAAAGAAATCTTTTTTGATCAATCAAAAAACG GATATGCAGGTTATATTGATGGGGGAGCTAGCCAAGAAGA ATTTTATAAATTTATCAAACCAATTTTAGAAAAAATGGATG GTACTGAGGAATTATTGGTGAAACTAAATCGTGAAGATTTG CTGCGCAAGCAACGGACCTTTGACAACGGCTCTATTCCCCA TCAAATTCACTTGGGTGAGCTGCATGCTATTTTGAGAAGAC AAGAAGACTTTTATCCATTTTTAAAAGACAATCGTGAGAAG ATTGAAAAAATCTTGACTTTTCGAATTCCTTATTATGTTGGT CCATTGGCGCGTGGCAATAGTCGTTTTGCATGGATGACTCG GAAGTCTGAAGAAACAATTACCCCATGGAATTTTGAAGAAG TTGTCGATAAAGGTGCTTCAGCTCAATCATTTATTGAACGC ATGACAAACTTTGATAAAAATCTTCCAAATGAAAAAGTACT ACCAAAACATAGTTTGCTTTATGAGTATTTTACGGTTTATAA CGAATTGACAAAGGTCAAATATGTTACTGAAGGAATGCGA AAACCAGCATTTCTTTCAGGTGAACAGAAGAAAGCCATTGT TGATTTACTCTTCAAAACAAATCGAAAAGTAACCGTTAAGC AATTAAAAGAAGATTATTTCAAAAAAATAGAATGTTTTGAT AGTGTTGAAATTTCAGGAGTTGAAGATAGATTTAATGCTTC | |
| | ATTAGGTACCTACCATGATTTGCTAAAAATTATTAAAGATA AAGATTTTTTGGATAATGAAGAAAATGAAGATATCTTAGAG GATATTGTTTTAACATTGACCTTATTTGAAGATAGGGAGAT GATTGAGGAAAGACTTAAAACATATGCTCACCTCTTTGATG ATAAGGTGATGAAACAGCTTAAACGTCGCCGTTATACTGGT TGGGGACGTTTGTCTCGAAAATTGATTAATGGTATTAGGGA TAAGCAATCTGGCAAAACAATATTAGATTTTTTGAAATCAG ATGGTTTTGCCAATCGCAATTTTATGCAGCTGATCCATGATG ATAGTTTGACATTTAAAGAAGACATTCAAAAAGCACAAGTG TCTGGACAAGGCGATAGTTTACATGAACATATTGCAAATTT AGCTGGTAGCCCTGCTATTAAAAAAGGTATTTTACAGACTG TAAAAGTTGTTGATGAATTGGTCAAAGTAATGGGGCGGCAT AAGCCAGAAAATATCGTTATTGAAATGGCACGTGAAAATCA GACAACTCAAAAGGGCCAGAAAAATTCGCGAGAGCGTATG AAACGAATCGAAGAAGGTATCAAAGAATTAGGAAGTCAGA TTCTTAAAGAGCATCCTGTTGAAAATACTCAATTGCAAAAT GAAAAGCTCTATCTCTATTATCTCCAAAATGGAAGAGACAT GTATGTGGACCAAGAATTAGATATTAATCGTTTAAGTGATT ATGATGTCGATCACATTGTTCCACAAAGTTTCCTTAAAGAC GATTCAATAGACAATAAGGTCTTAACGCGTTCTGATAAAAA TCGTGGTAAATCGGATAACGTTCCAAGTGAAGAAGTAGTCA AAAAGATGAAAAACTATTGGAGACAACTTCTAAACGCCAA GTTAATCACTCAACGTAAGTTTGATAATTTAACGAAAGCTG AACGTGGAGGTTTGAGTGAACTTGATAAAGCTGGTTTTATC AAACGCCAATTGGTTGAAACTCGCCAAATCACTAAGCATGT GGCACAAATTTTGGATAGTCGCATGAATACTAAATACGATG AAAATGATAAACTTATTCGAGAGGTTAAAGTGATTACCTTA AAATCTAAATTAGTTTCTGACTTCCGAAAAGATTTCCAATTC TATAAAGTACGTGAGATTAACAATTACCATCATGCCCATGA TGCGTATCTAAATGCCGTCGTTGGAACTGCTTTGATTAAGA AATATCCAAAACTTGAATCGGAGTTTGTCTATGGTGATTAT AAAGTTTATGATGTTCGTAAAATGATTGCTAAGTCTGAGCA AGAAATAGGCAAAGCAACCGCAAAATATTTCTTTTACTCTA ATATCATGAACTTCTTCAAAACAGAAATTACACTTGCAAAT GGAGAGATTCGCAAACGCCCTCTAATCGAAACTAATGGGG AAACTGGAGAAATTGTCTGGGATAAAGGGCGAGATTTTGCC | |
| | ACAGTGCGCAAAGTATTGTCCATGCCCCAAGTCAATATTGT CAAGAAAACAGAAGTACAGACAGGCGGATTCTCCAAGGAG TCAATTTTACCAAAAAGAAATTCGGACAAGCTTATTGCTCG TAAAAAAGACTGGGATCCAAAAAAATATGGTGGTTTTGATA GTCCAACGGTAGCTTATTCAGTCCTAGTGGTTGCTAAGGTG GAAAAAGGGAAATCGAAGAAGTTAAAATCCGTTAAAGAGT TACTAGGGATCACAATTATGGAAAGAAGTTCCTTTGAAAAA AATCCGATTGACTTTTTAGAAGCTAAAGGATATAAGGAAGT TAAAAAAGACTTAATCATTAAACTACCTAAATATAGTCTTT TTGAGTTAGAAAACGGTCGTAAACGGATGCTGGCTAGTGCC GGAGAATTACAAAAAGGAAATGAGCTGGCTCTGCCAAGCA AATATGTGAATTTTTTATATTTAGCTAGTCATTATGAAAAGT TGAAGGGTAGTCCAGAAGATAACGAACAAAAACAATTGTT TGTGGAGCAGCATAAGCATTATTTAGATGAGATTATTGAGC AAATCAGTGAATTTTCTAAGCGTGTTATTTTAGCAGATGCC AATTTAGATAAAGTTCTTAGTGCATATAACAAACATAGAGA CAAACCAATACGTGAACAAGCAGAAAATATTATTCATTTAT TTACGTTGACGAATCTTGGAGCTCCCGCTGCTTTTAAATATT TTGATACAACAATTGATCGTAAACGATATACGTCTACAAAA GAAGTTTTAGATGCCACTCTTATCCATCAATCCATCACTGGT CTTTATGAAACACGCATTGATTTGAGTCAGCTAGGAGGTGA CTGA | |
| SpCas9 *Streptococcus pyogenes* M1GAS wild type Encoded product of NC_002737.2 (100% identical to the canonical Q99ZW2 wild type) | | 12 |

The base editors described herein may include any of the above SpCas9 sequences, or any variant thereof having at least 80%, at least 85%, at least 90%, at least 95%, or at least 99% sequence identity thereto.

### (2) Wild type Cas9 orthologs

In other embodiments, the Cas9 protein can be a wild type Cas9 ortholog from another bacterial species different from the canonical Cas9 from *S. pyogenes.* For example, the following Cas9 orthologs can be used in connection with the base editor constructs described in this specification. In addition, any variant Cas9 orthologs having at least 80%, at least 85%, at least 90%, at least 95%, or at least 99% sequence identity to any of the below orthologs may also be used with the present base editors.

| Description | Sequence |
|---|---|
| LfCas9 *Lactobacillus fermentum* wild type | MKEYHIGLDIGTSSIGWAVTDSQFKLMRIKGKTAIGVRLFEEGKTAAERRTFRT TRRRLKRRKWRLHYLDEIFAPHLQEVDENFLRRLKQSNIHPEDPTKNQAFIGKL LFPDLLKKNERGYPTLIKMRDELPVEQRAHYPVMNIYKLREAMINEDRQFDLRE VYLAVHHIVKYRGHFLNNASVDKFKVGRIDFDKSFNVLNEAYEELQNGEGSFTI EPSKVEKIGQLLLDTKMRKLDRQKAVAKLLEVKVADKEETKRNKQIATAMSK |
| *GenBank: SNX31424.1* 1 | LVLGYKADFATVAMANGNEWKIDLSSETSEDEIEKFREELSDAQNDILTEITSLF SQIMLNEIVPNGMSISESMMDRYWTHERQLAEVKEYLATQPASARKEFDQVYN KYIGQAPKERGFDLEKGLKKILSKKENWKEIDELLKAGDFLPKQRTSANGVIPH QMHQQELDRIIEKQAKYYPWLATENPATGERDRHQAKYELDQLVSFRIPYYVG PLVTPEVQKATSGAKFAWAKRKEDGEITPWNLWDKIDRAESAEAFIKRMTVKD TYLLNEDVLPANSLLYQKYNVLNELNNVRVNGRRLSVGIKQDIYTELFKKKKT VKASDVASLVMAKTRGVNKPSVEGLSDPKKFNSNLATYLDLKSIVGDKVDDN RYQTDLENIIEWRSVFEDGEIFADKLTEVEWLTDEQRSALVKKRYKGWGRLSK KLLTGIVDENGQRIIDLMWNTDQNFKEIVDQPVFKEQIDQLNQKAITNDGMTLR ERVESVLDDAYTSPQNKKAIWQVVRVVEDIVKAVGNAPKSISIEFARNEGNKGE ITRSRRTQLQKLFEDQAHELVKDTSLTEELEKAPDLSDRYYFYFTQGGKDMYT GDPINFDEISTKYDIDHILPQSFVKDNSLDNRVLTSRKENNKKSDQVPAKLYAA KMKPYWNQLLKQGLITQRKFENLTKDVDQNIKYRSLGFVKRQLVETRQVIKLT ANILGSMYQEAGTEIIETRAGLTKQLREEFDLPKVREVNDYHHAVDAYLTTFAG QYLNRRYPKLRSFFVYGEYMKFKHGSDLKLRNFNFFHELMEGDKSQGKVVDQ QTGELITTRDEVAKSFDRLLNMKYMLVSKEVHDRSDQLYGATIVTAKESGKLT SPIEIKKNRLVDLYGAYTNGTSAFMTIIKFTGNKPKYKVIGIPTTSAASLKRAGKP GSESYNQELHRIIKSNPKVKKGFEIVVPHVSYGQLIVDGDCKFTLASPTVQHPAT QLVLSKKSLETISSGYKILKDKPAIANERLIRVFDEVVGQMNRYFTIFDQRSNRQ KVADARDKFLSLPTESKYEGAKKVQVGKTEVITNLLMGLHANATQGDLKVLG LATFGFFQSTTGLSLSEDTMIVYQSPTGLFERRICLKDI(SEQ ID NO: 13) |
| SaCas9 *Staphylococc us aureus* wild type GenBank: AYD60528.1 | (SEQ ID NO: 14) |
| SaCas9 *Staphylococc us aureus* | MGKRNYILGLDIGITSVGYGIIDYETRDVIDAGVRLFKEANVENNEGRRSKRGA RRLKRRRRHRIQRVKKLLFDYNLLTDHSELSGINPYEARVKGLSQKLSEEEFSA ALLHLAKRRGVHNVNEVEEDTGNELSTKEQISRNSKALEEKYVAELQLERLKK DGEVRGSINRFKTSDYVKEAKQLLKVQKAYHQLDQSFIDTYIDLLETRRTYYEG PGEGSPFGWKDIKEWYEMLMGHCTYFPEELRSVKYAYNADLYNALNDLNNLV ITRDENEKLEYYEKFQIIENVFKQKKKPTLKQIAKEILVNEEDIKGYRVTSTGKPE FTNLKVYHDIKDITARKEIIENAELLDQIAKILTIYQSSEDIQEELTNLNSELTQEEI EQISNLKGYTGTHNLSLKAINLILDELWHTNDNQIAIFNRLKLVPKKVDLSQQKE IPTTLVDDFILSPVVKRSFIQSIKVINAIIKKYGLPNDIIIELAREKNSKDAQKMINE MQKRNRQTNERIEEIIRTTGKENAKYLIEKIKLHDMQEGKCLYSLEAIPLEDLLN NPFNYEVDHIIPRSVSFDNSFNNKVLVKQEENSKKGNRTPFQYLSSSDSKISYETF KKHILNLAKGKGRISKTKKEYLLEERDINRFSVQKDFINRNLVDTRYATRGLMN LLRSYFRVNNLDVKVKSINGGFTSFLRRKWKFKKERNKGYKHHAEDALIIANA DFIFKEWKKLDKAKKVMENQMFEEKQAESMPEIETEQEYKEIFITPHQIKHIKDF KDYKYSHRVDKKPNRKLINDTLYSTRKDDKGNTLIVNNLNGLYDKDNDKLKK LINKSPEKLLMYHHDPQTYQKLKLIMEQYGDEKNPLYKYYEETGNYLTKYSKK DNGPVIKKIKYYGNKLNAHLDITDDYPNSRNKVVKLSLKPYRFDVYLDNGVYK FVTVKNLDVIKKENYYEVNSKCYEEAKKLKKISNQAEFIASFYKNDLIKINGEL YRVIGVNNDLLNRIEVNMIDITYREYLENMNDKRPPHIIKTIASKTQSIKKYSTDI LGNLYEVKSKKHPQIIKK (SEQ ID NO: 15) |
| StCas9 *Streptococcu* s *thermophilus* UniProtKB/S wiss-Prot: G3ECR1.2 Wild type | MLFNKCIIISINLDFSNKEKCMTKPYSIGLDIGTNSVGWAVITDNYKVPSKKMKV LGNTSKKYIKKNLLGVLLFDSGITAEGRRLKRTARRRYTRRRNRILYLQEIFSTE MATLDDAFFQRLDDSFLVPDDKRDSKYPIFGNLVEEKVYHDEFPTIYHLRKYLA DSTKKADLRLVYLALAHMIKYRGHFLIEGEFNSKNNDIQKNFQDFLDTYNAIFE SDLSLENSKQLEEIVKDKISKLEKKDRILKLFPGEKNSGIFSEFLKLIVGNQADFR KCFNLDEKASLHFSKESYDEDLETLLGYIGDDYSDVFLKAKKLYDAILLSGFLT VTDNETEAPLSSAMIKRYNEHKEDLALLKEYIRNISLKTYNEVFKDDTKNGYAG YIDGKTNQEDFYVVYLKNLLAEFEGADYFLEKIDREDFLRKQRTFDNGSIPYQIHL QEMRAILDKQAKFYPFLAKNKERIEKILTFRIPYYVGPLARGNSDFAWSIRKRNE KITPWNFEDVIDKESSAEAFINRMTSFDLYLPEEKVLPKHSLLYETFNVYNELTK VRFIAESMRDYQFLDSKQKKDIVRLYFKDKRKVTDKDIIEYLHAIYGYDGIELK GIEKQFNSSLSTYHDLLNIINDKEFLDDSSNEAIIEEIIHTLTIFEDREMIKQRLSKF ENIFDKSVLKKLSRRHYTGWGKLSAKLINGIRDEKSGNTILDYLIDDGISNRNFM QLIHDDALSFKKKIQKAQIIGDEDKGNIKEVVKSLPGSPAIKKGILQSIKIVDELV KVMGGRKPESIVVEMARENQYTNQGKSNSQQRLKRLEKSLKELGSKILKENIPA KLSKIDNNALQNDRLYLYYLQNGKDMYTGDDLDIDRLSNYDIDHIIPQAFLKDN SIDNKVLVSSASNRGKSDDFPSLEVVKKRKTFWYQLLKSKLISQRKFDNLTKAE RGGLLPEDKAGFIQRQLVETRQITKHVARLLDEKFNNKKDENNRAVRTVKIITL KSTLVSQFRKDFELYKVREINDFHHAHDAYLNAVIASALLKKYPKLEPEFVYGD YPKYNSFRERKSATEKVYFYSNIMNIFKKSISLADGRVIERPLIEVNEETGESVW NKESDLATVRRVLSYPQVNVVKKVEEQNHGLDRGKPKGLFNANLSSKPKPNSN ENLVGAKEYLDPKKYGGYAGISNSFAVLVKGTIEKGAKKKITNVLEFQGISILD RINYRKDKLNFLLEKGYKDIELIIELPKYSLFELSDGSRRMLASILSTNNKRGEIH KGNQIFLSQKFVKLLYHAKRISNTINENHRKYVENHKKEFEELFYYILEFNENYV GAKKNGKLLNSAFQSWQNHSIDELCSSFIGPTGSERKGLFELTSRGSAADFEFLG VKIPRYRDYTPSSLLKDATLIHQSVTGLYETRIDLAKLGEG(SEQ ID NO: 16) |
| LcCas9 *Lactobacillus crispatus* NCBI Reference Sequence: WP_1334780 44.1 | MKIKNYNLALTPSTSAVGHVEVDDDLNILEPVHHQKAIGVAKFGEGETAEARR LARSARRTTKRRANRINHYFNEIMKPEIDKVDPLMFDRIKQAGLSPLDERKEFRT VIFDRPNIASYYHNQFPTIWHLQKYLMITDEKADIRLIYWALHSLLKHRGHFFNT TPMSQFKPGKLNLKDDMLALDDYNDLEGLSFAVANSPEIEKVIKDRSMHKKEK IAELKKLIVNDVPDKDLAKRNNKIITQIVNAIMGNSFHLNFIFDMDLDKLTSKA WSFKLDDPELDTKFDAISGSMTDNQIGIFETLQKIYSAISLLDILNGSSNVVDAKN ALYDKHKRDLNLYFKFLNTLPDEIAKTLKAGYTLYIGNRKKDLLAARKLLKVN |
| Wild type | VAKNFSQDDFYKLINKELKSIDKQGLQTRFSEKVGELVAQNNFLPVQRSSDNVF IPYQLNAITFNKILENQGKYYDFLVKPNPAKKDRKNAPYELSQLMQFTIPYYVG PLVTPEEQVKSGIPKTSRFAWMVRKDNGAITPWNFYDKVDIEATADKFIKRSIA KDSYLLSELVLPKHSLLYEKYEVFNELSNVSLDGKKLSGGVKQILFNEVFKKTN KVNTSRILKALAKHNIPGSKITGLSNPEEFTSSLQTYNAWKKYFPNQIDNFAYQQ DLEKMIEWSTVFEDHKILAKKLDEIEWLDDDQKKFVANTRLRGWGRLSKRLLT GLKDNYGKSIMQRLETTKANFQQIVYKPEFREQIDKISQAAAKNQSLEDILANS YTSPSNRKAIRKTMSVVDEYIKLNHGKEPDKIFLMFQRSEQEKGKQTEARSKQL NRILSQLKADKSANKLFSKQLADEFSNAIKKSKYKLNDKQYFYFQQLGRDALT GEVIDYDELYKYTVLHIIPRSKLTDDSQNNKVLTKYKIVDGSVALKFGNSYSDA LGMPIKAFWTELNRLKLIPKGKLLNLTTDFSTLNKYQRDGYIARQLVETQQIVK LLATIMQSRFKHTKIIEVRNSQVANIRYQFDYFRIKNLNEYYRGFDAYLAAVVG TYLYKVYPKARRLFVYGQYLKPKKTNQENQDMHLDSEKKSQGFNFLWNLLYG KQDQIFVNGTDVIAFNRKDLITKMNTVYNYKSQKISLAIDYHNGAMFKATLFPR NDRDTAKTRKLIPKKKDYDTDIYGGYTSNVDGYMLLAEIIKRDGNKQYGFYGV PSRLVSELDTLKKTRYTEYEEKLKEIIKPELGVDLKKIKKIKILKNKVPFNQVIID KGSKFFITSTSYRWNYRQLILSAESQQTLMDLVVDPDFSNHKARKDARKNADE RLIKVYEEILYQVKNYMPMFVELHRCYEKLVDAQKTFKSLKISDKAMVLNQILI LLHSNATSPVLEKLGYHTRFTLGKKHNLISENAVLVTQSITGLKENHVSIKQML (SEQ ID NO: 17) |
| PdCas9 *Pedicoccus damnosus* NCBI Reference Sequence: WP_0629132 73.1 Wild type | MTNEKYSIGLDIGTSSIGFAVVNDNNRVIRVKGKNAIGVRLFDEGKAAADRRSF RTTRRSFRTTRRRLSRRRWRLKLLREIFDAYITPVDEAFFIRLKESNLSPKDSKKQ YSGDILFNDRSDKDFYEKYPTIYHLRNALMTEHRKFDVREIYLAIHHIMKFRGH FLNATPANNFKVGRLNLEEKFEELNDIYQRVFPDESIEFRTDNLEQIKEVLLDNK RSRADRQRTLVSDIYQSSEDKDIEKRNKAVATEILKASLGNKAKLNVITNVEVD KEAAKEWSITFDSESIDDDLAKIEGQMTDDGHEIIEVLRSLYSGITLSAIVPENHT LSQSMVAKYDLHKDHLKLFKKLINGMTDTKKAKNLRAAYDGYIDGVKGKVLP QEDFYKQVQVNLDDSAEANEIQTYIDQDIFMPKQRTKANGSIPHQLQQQELDQI IENQKAYYPWLAELNPNPDKKRQQLAKYKLDELVTFRVPYYVGPMITAKDQK NQSGAEFAWMIRKEPGNITPWNFDQKVDRMATANQFIKRMTTTDTYLLGEDV LPAQSLLYQKFEVLNELNKIRIDHKPISIEQKQQIFNDLFKQFKNVTIKHLQDYLV SQGQYSKRPLIEGLADEKRFNSSLSTYSDLCGIFGAKLVEENDRQEDLEKIIEWS TIFEDKKIYRAKLNDLTWLTDDQKEKLATKRYQGWGRLSRKLLVGLKNSEHR NIMDILWITNENFMQIQAEPDFAKLVTDANKGMLEKTDSQDVINDLYTSPQNK KAIRQILLVVHDIQNAMHGQAPAKIHVEFARGEERNPRRSVQRQRQVEAAYEK VSNELVSAKVRQEFKEAINNKRDFKDRLFLYFMQGGIDIYTGKQLNIDQLSSYQI DHILPQAFVKDDSLTNRVLTNENQVKADSVPIDIFGKKMLSVWGRMKDQGLIS KGKYRNLTMNPENISAHTENGFINRQLVETRQVIKLAVNILADEYGDSTQIISVK ADLSHQMREDFELLKNRDVNDYHHAFDAYLAAFIGNYLLKRYPKLESYFVYG DFKKFTQKETKMRRFNFIYDLKHCDQVVNKETGEILWTKDEDIKYIRHLFAYK KILVSHEVREKRGALYNQTIYKAKDDKGSGQESKKLIRIKDDKETKIYGGYSGK SLAYMTIVQITKKNKVSYRVIGIPTLALARLNKLENDSTENNGELYKIIKPQFTH YKVDKKNGEIIETTDDFKIVVSKVRFQQLIDDAGQFFMLASDTYKNNAQQLVIS NNALKAINNTNITDCPRDDLERLDNLRLDSAFDEIVKKMDKYFSAYDANNFRE KIRNSNLIFYQLPVEDQWENNKITELGKRTVLTRILQGLHANATTTDMSIFKIKT PFGQLRQRSGISLSENAQLIYQSPTGLFERRVQLNKIK (SEQ ID NO: 18) |
| FnCas9 *Fusobateriu m nucleatum* NCBI Reference Sequence: WP_0607989 84.1 | MKKQKFSDYYLGFDIGTNSVGWCVTDLDYNVLRFNKKDMWGSRLFEEAKTA AERRVQRNSRRRLKRRKWRLNLLEEIFSNEILKIDSNFFRRLKESSLWLEDKSSK EKFTLFNDDNYKDYDFYKQYPTIFHLRNELIKNPEKKDIRLVYLAIHSIFKSRGH FLFEGQNLKEIKNFETLYNNLIAFLEDNGINKIIDKNNIEKLEKIVCDSKKGLKDK EKEFKEIFNSDKQLVAIFKLSVGSSVSLNDLFDTDEYKKGEVEKEKISFREQIYED DKPIYYSILGEKIELLDIAKTFYDFMVLNNILADSQYISEAKVKLYEEHKKDLKN LKYIIRKYNKGNYDKLFKDKNENNYSAYIGLNKEKSKKEVIEKSRLKIDDLIKNI KGYLPKVEEIEEKDKAIFNKILNKIELKTILPKQRISDNGTLPYQIHEAELEKILEN QSKYYDFLNYEENGIITKDKLLMTFKFRIPYYVGPLNSYHKDKGGNSWIVRKEE GKILPWNFEQKVDIEKSAEEFIKRMTNKCTYLNGEDVIPKDTFLYSEYVILNELN KVQVNDEFLNEENKRKIIDELFKENKKVSEKKFKEYLLVKQIVDGTIELKGVKD SFNSNYISYIRFKDIFGEKLNLDIYKEISEKSILWKCLYGDDKKIFEKKIKNEYGDI LTKDEIKKINTFKFNNWGRLSEKLLTGIEFINLETGECYSSVMDALRRTNYNLM ELLSSKFTLQESINNENKEMNEASYRDLIEESYVSPSLKRAIFQTLKIYEEIRKITG RVPKKVFIEMARGGDESMKNKKIPARQEQLKKLYDSCGNDIANFSIDIKEMKNS LISYDNNSLRQKKLYLYYLQFGKCMYTGREIDLDRLLQNNDTYDIDHIYPRSKV IKDDSFDNLVLVLKNENAEKSNEYPVKKEIQEKMKSFWRFLKEKNFISDEKYKR LTGKDDFELRGFMARQLVNVRQTTKEVGKILQQIEPEIKIVYSKAEIASSFREMF DFIKVRELNDTHHAKDAYLNIVAGNVYNTKFTEKPYRYLQEIKENYDVKKIYN YDIKNAWDKENSLEIVKKNMEKNTVNITRFIKEKKGQLFDLNPIKKGETSNEIISI KPKVYNGKDDKLNEKYGYYKSLNPAYFLYVEHKEKNKRIKSFERVNLVDVNN IKDEKSLVKYLIENKKLVEPRVIKKVYKRQVILINDYPYSIVTLDSNKLMDFENL KPLFLENKYEKILKNVIKFLEDNQGKSEENYKFIYLKKKDRYEKNETLESVKDR YNLEFNEMYDKFLEKLDSKDYKNYMNNKKYQELLDVKEKFIKLNLFDKAFTL KSFLDLFNRKTMADFSKVGLTKYLGKIQKISSNVLSKNELYLLEESVTGLFVKKI KL (SEQ ID NO: 19) |
| EcCas9 *Enterococcus cecorum* NCBI Reference Sequence: WP_0473385 01.1 Wild type | MNKYYLGLDMGSASVGWAVTDENYHLVRRKGKDLWGVRTFDVAQTAKERRI TRGNRRRQDRRKQRIQILQELLGEEVLKTDPGFFHRMKESRYVVEDKRTLDGK QVELPYALFVDKDYTDKEYYKQFPTINHLIVYLMTTSDTPDIRLVYLALHYYM KNRGNFLHSGDINNVKDINDILEQLDNVLETFLDGWNLKLKSYVEDIKNIYNRD LGRGERKKAFVNTLGAKTKAEKAFCSLISGGSTNLAELFDDSSLKEIETPKIEFA SSSLEDKIDGIQEALEDRFAVIEAAKRLYDWKTLTDILGDSSSLAEARVNSYQM HHEQLLELKSLVKEYLDRKVFQEVFVSLNVANNYPAYIGHTKINGKKKELEVK RTKRNDFYSYVKKQVIEPIKKKVSDEAVLTKLSEIESLIEVDKYLPLQVNSDNGV IPYQVKLNELTRIFDNLENRIPVLRENRDKIIKTFKFRIPYYVGSLNGVVKNGKCT NWMVRKEEGKIYPWNFEDKVDLEASAEQFIRRMTNKCTYLVNEDVLPKYSLL YSKYLVLSELNNLRIDGRPLDVKIKQDIYENVFKKNRKVTLKKIKKYLLKEGIIT DDDELSGLADDVKSSLTAYRDFKEKLGHLDLSEAQMENIILNITLFGDDKKLLK KRLAALYPFIDDKSLNRIATLNYRDWGRLSERFLSGITSVDQETGELRTIIQCMY ETQANLMQLLAEPYHFVEAIEKENPKVDLESISYRIVNDLYVSPAVKRQIWQTL LVIKDIKQVMKHDPERIFIEMAREKQESKKTKSRKQVLSEVYKKAKEYEHLFEK LNSLTEEQLRSKKIYLYFTQLGKCMYSGEPIDFENLVSANSNYDIDHIYPQSKTID DSFNNIVLVKKSLNAYKSNHYPIDKNIRDNEKVKTLWNTLVSKGLITKEKYERL IRSTPFSDEELAGFIARQLVETRQSTKAVAEILSNWFPESEIVYSKAKNVSNFRQD FEILKVRELNDCHHAHDAYLNIVVGNAYHTKFTNSPYRFIKNKANQEYNLRKL LQKVNKIESNGVVAWVGQSENNPGTIATVKKVIRRNTVLISRMVKEVDGQLFD LTLMKKGKGQVPIKSSDERLTDISKYGGYNKATGAYFTFVKSKKRGKVVRSFE YVPLHLSKQFENNNELLKEYIEKDRGLTDVEILIPKVLINSLFRYNGSLVRITGRG DTRLLLVHEQPLYVSNSFVQQLKSVSSYKLKKSENDNAKLTKTATEKLSNIDEL YDGLLRKLDLPIYSYWFSSIKEYLVESRTKYIKLSIEEKALVIFEILHLFQSDAQVP NLKILGLSTKPSRIRIQKNLKDTDKMSIIHQSPSGIFEHEIELTSL (SEQ ID NO: 20) |
| AhCas9 *Anaerostipes hadrus* NCBI Reference | MQNGFLGITVSSEQVGWAVTNPKYELERASRKDLWGVRLFDKAETAEDRRMF RTNRRLNQRKKNRIHYLRDIFHEEVNQKDPNFFQQLDESNFCEDDRTVEFNFDT NLYKNQFPTVYHLRKYLMETKDKPDIRLVYLAFSKFMKNRGHFLYKGNLGEV MDFENSMKGFCESLEKFNIDFPTLSDEQVKEVRDILCDHKIAKTVKKKNIITITK |
| Sequence: WP_0449242 78.1 Wild type | VKSKTAKAWIGLFCGCSVPVKVLFQDIDEEIVTDPEKISFEDASYDDYIANIEKG VGIYYEAIVSAKMLFDWSILNEILGDHQLLSDAMIAEYNKHHDDLKRLQKIIKG TGSRELYQDIFINDVSGNYVCYVGHAKTMSSADQKQFYTFLKNRLKNVNGISSE DAEWIDTEIKNGTLLPKQTKRDNSVIPHQLQLREFELILDNMQEMYPFLKENRE KLLKIFNFVIPYYVGPLKGVVRKGESTNWMVPKKDGVIHPWNFDEMVDKEAS AECFISRMTGNCSYLFNEKVLPKNSLLYETFEVLNELNPLKINGEPISVELKQRIY EQLFLTGKKVTKKSLTKYLIKNGYDKDIELSGIDNEFHSNLKSHIDFEDYDNLSD EEVEQIILRITVFEDKQLLKDYLNREFVKLSEDERKQICSLSYKGWGNLSEMLLN GITVTDSNGVEVSVMDMLWNTNLNLMQILSKKYGYKAEIEHYNKEHEKTIYNR EDLMDYLNIPPAQRRKVNQLITIVKSLKKTYGVPNKIFFKISREHQDDPKRTSSR KEQLKYLYKSLKSEDEKHLMKELDELNDHELSNDKVYLYFLQKGRCIYSGKKL NLSRLRKSNYQNDIDYIYPLSAVNDRSMNNKVLTGIQENRADKYTYFPVDSEIQ KKMKGFWMELVLQGFMTKEKYFRLSRENDFSKSELVSFIEREISDNQQSGRMIA SVLQYYFPESKIVFVKEKLISSFKRDFHLISSYGHNHLQAAKDAYITIVVGNVYH TKFTMDPAIYFKNHKRKDYDLNRLFLENISRDGQIAWESGPYGSIQTVRKEYAQ NHIAVTKRVVEVKGGLFKQMPLKKGHGEYPLKTNDPRFGNIAQYGGYTNVTG SYFVLVESMEKGKKRISLEYVPVYLHERLEDDPGHKLLKEYLVDHRKLNHPKIL LAKVRKNSLLKIDGFYYRLNGRSGNALILTNAVELIMDDWQTKTANKISGYMK RRAIDKKARVYQNEFHIQELEQLYDFYLDKLKNGVYKNRKNNQAELIHNEKEQ FMELKTEDQCVLLTEIKKLFVCSPMQADLTLIGGSKHTGMIAMSSNVTKADFA VIAEDPLGLRNKVIYSHKGEK (SEQ ID NO: 21) |
| KvCas9 *Kandleria vitulina* NCBI Reference Sequence: WP_0315899 69.1 Wild type | MSQNNNKIYNIGLDIGDASVGWAVVDEHYNLLKRHGKHMWGSRLFTQANTA VERRSSRSTRRRYNKRRERIRLLREIMEDMVLDVDPTFFIRLANVSFLDQEDKK DYLKENYHSNYNLFIDKDFNDKTYYDKYPTIYHLRKHLCESKEKEDPRLIYLAL HHIVKYRGNFLYEGQKFSMDVSNIEDKMIDVLRQFNEINLFEYVEDRKKIDEVL NVLKEPLSKKHKAEKAFALFDTTKDNKAAYKELCAALAGNKFNVTKMLKEAE LHDEDEKDISFKFSDATFDDAFVEKQPLLGDCVEFIDLLHDIYSWVELQNILGSA HTSEPSISAAMIQRYEDHKNDLKLLKDVIRKYLPKKYFEVFRDEKSKKNNYCNY INHPSKTPVDEFYKYIKKLIEKIDDPDVKTILNKIELESFMLKQNSRTNGAVPYQ MQLDELNKILENQSVYYSDLKDNEDKIRSILTFRIPYYFGPLNITKDRQFDWIIKK EGKENERILPWNANEIVDVDKTADEFIKRMRNFCTYFPDEPVMAKNSLTVSKY EVLNEINKLRINDHLIKRDMKDKMLHTLFMDHKSISANAMKKWLVKNQYFSN TDDIKIEGFQKENACSTSLTPWIDFTKIFGKINESNYDFIEKIIYDVTVFEDKKILR RRLKKEYDLDEEKIKKILKLKYSGWSRLSKKLLSGIKTKYKDSTRTPETVLEVM ERTNMNLMQVINDEKLGFKKTIDDANSTSVSGKFSYAEVQELAGSPAIKRGIWQ ALLIVDEIKKIMKHEPAHVYIEFARNEDEKERKDSFVNQMLKLYKDYDFEDETE KEANKHLKGEDAKSKIRSERLKLYYTQMGKCMYTGKSLDIDRLDTYQVDHIVP QSLLKDDSIDNKVLVLSSENQRKLDDLVIPSSIRNKMYGFWEKLFNNKIISPKKF YSLIKTEFNEKDQERFINRQIVETRQITKHVAQIIDNHYENTKVVTVRADLSHQF RERYHIYKNRDINDFHHAHDAYIATILGTYIGHRFESLDAKYIYGEYKRIFRNQK NKGKEMKKNNDGFILNSMRNIYADKDTGEIVWDPNYIDRIKKCFYYKDCFVTK KLEENNGTFFNVTVLPNDTNSDKDNTLATVPVNKYRSNVNKYGGFSGVNSFIV AIKGKKKKGKKVIEVNKLTGIPLMYKNADEEIKINYLKQAEDLEEVQIGKEILK NQLIEKDGGLYYIVAPTEIINAKQLILNESQTKLVCEIYKAMKYKNYDNLDSEKI IDLYRLLINKMELYYPEYRKQLVKKFEDRYEQLKVISIEEKCNIIKQILATLHCNS SIGKIMYSDFKISTTIGRLNGRTISLDDISFIAESPTGMYSKKYKL (SEQ ID NO: 22) |
| EfCas9 *Enterococcus faecalis* NCBI Reference Sequence: WP_0166310 44.1 Wild type | MRLFEEGHTAEDRRLKRTARRRISRRRNRLRYLQAFFEEAMTDLDENFFARLQE SFLVPEDKKWHRHPIFAKLEDEVAYHETYPTIYHLRKKLADSSEQADLRLIYLA LAHIVKYRGHFLIEGKLSTENTSVKDQFQQFMVIYNQTFVNGESRLVSAPLPESV LIEEELTEKASRTKKSEKVLQQFPQEKANGLFGQFLKLMVGNKADFKKVFGLE EEAKITYASESYEEDLEGILAKVGDEYSDVFLAAKNVYDAVELSTILADSDKKS HAKLSSSMIVRFTEHQEDLKKFKRFIRENCPDEYDNLFKNEQKDGYAGYIAHAG KVSQLKFYQYVKKIIQDIAGAEYFLEKIAQENFLRKQRTFDNGVIPHQIHLAELQ AIIHRQAAYYPFLKENQEKIEQLVTFRIPYYVGPLSKGDASTFAWLKRQSEEPIRP WNLQETVDLDQSATAFIERMTNFDTYLPSEKVLPKHSLLYEKFMVFNELTKISY TDDRGIKANFSGKEKEKIFDYLFKTRRKVKKKDIIQFYRNEYNTEIVTLSGLEED QFNASFSTYQDLLKCGLTRAELDHPDNAEKLEDIIKILTIFEDRQRIRTQLSTFKG QFSAEVLKKLERKHYTGWGRLSKKLINGIYDKESGKTILDYLVKDDGVSKHYN RNFMQLINDSQLSFKNAIQKAQSSEHEETLSETVNELAGSPAIKKGIYQSLKIVD ELVAIMGYAPKRIVVEMARENQTTSTGKRRSIQRLKIVEKAMAEIGSNLLKEQP TTNEQLRDTRLFLYYMQNGKDMYTGDELSLHRLSHYDIDHIIPQSFMKDDSLD NLVLVGSTENRGKSDDVPSKEVVKDMKAYWEKLYAAGLISQRKFQRLTKGEQ GGLTLEDKAHFIQRQLVETRQITKNVAGILDQRYNAKSKEKKVQIITLKASLTSQ FRSIFGLYKVREVNDYHHGQDAYLNCVVATTLLKVYPNLAPEFVYGEYPKFQT FKENKATAKAIIYTNLLRFFTEDEPRFTKDGEILWSNSYLKTIKKELNYHQMNIV KKVEVQKGGFSKESIKPKGPSNKLIPVKNGLDPQKYGGFDSPVVAYTVLFTHEK GKKPLIKQEILGITIMEKTRFEQNPILFLEEKGFLRPRVLMKLPKYTLYEFPEGRR RLLASAKEAQKGNQMVLPEHLLTLLYHAKQCLLPNQSESLAYVEQHQPEFQEI LERVVDFAEVHTLAKSKVQQIVKLFEANQTADVKEIAASFIQLMQFNAMGAPS TFKFFQKDIERARYTSIKEIFDATIIYQSPTGLYETRRKVVD (SEQ ID NO: 23) |
| *Staphylococc us aureus* Cas9 | KRNYILGLDIGITSVGYGIIDYETRDVIDAGVRLFKEANVENNEGRRSKRGARRL KRRRRHRIQRVKKLLFDYNLLTDHSELSGINPYEARVKGLSQKLSEEEFSAALL HLAKRRGVHNVNEVEEDTGNELSTKEQISRNSKALEEKYVAELQLERLKKDGE VRGSINRFKTSDYVKEAKQLLKVQKAYHQLDQSFIDTYIDLLETRRTYYEGPGE GSPFGWKDIKEWYEMLMGHCTYFPEELRSVKYAYNADLYNALNDLNNLVITR DENEKLEYYEKFQIIENVFKQKKKPTLKQIAKEILVNEEDIKGYRVTSTGKPEFT NLKVYHDIKDITARKEIIENAELLDQIAKILTIYQSSEDIQEELTNLNSELTQEEIE QISNLKGYTGTHNLSLKAINLILDELWHTNDNQIAIFNRLKLVPKKVDLSQQKEI PTTLVDDFILSPVVKRSFIQSIKVINAIIKKYGLPNDIIIELAREKNSKDAQKMINE MQKRNRQTNERIEEIIRTTGKENAKYLIEKIKLHDMQEGKCLYSLEAIPLEDLLN NPFNYEVDHIIPRSVSFDNSFNNKVLVKQEENSKKGNRTPFQYLSSSDSKISYETF KKHILNLAKGKGRISKTKKEYLLEERDINRFSVQKDFINRNLVDTRYATRGLMN LLRSYFRVNNLDVKVKSINGGFTSFLRRKWKFKKERNKGYKHHAEDALIIANA DFIFKEWKKLDKAKKVMENQMFEEKQAESMPEIETEQEYKEIFITPHQIKHIKDF KDYKYSHRVDKKPNRELINDTLYSTRKDDKGNTLIVNNLNGLYDKDNDKLKK LINKSPEKLLMYHHDPQTYQKLKLIMEQYGDEKNPLYKYYEETGNYLTKYSKK DNGPVIKKIKYYGNKLNAHLDITDDYPNSRNKVVKLSLKPYRFDVYLDNGVYK FVTVKNLDVIKKENYYEVNSKCYEEAKKLKKISNQAEFIASFYNNDLIKINGEL YRVIGVNNDLLNRIEVNMIDITYREYLENMNDKRPPRIIKTIASKTQSIKKYSTDI LGNLYEVKSKKHPQIIKKG (SEQ ID NO: 24) |
| *Geobacillus thermodenitri ficans* Cas9 | MKYKIGLDIGITSIGWAVINLDIPRIEDLGVRIFDRAENPKTGESLALPRRLARSA RRRLRRRKHRLERIRRLFVREGILTKEELNKLFEKKHEIDVWQLRVEALDRKLN NDELARILLHLAKRRGFRSNRKSERTNKENSTMLKHIEENQSILSSYRTVAEMV VKDPKFSLHKRNKEDNYTNTVARDDLEREIKLIFAKQREYGNIVCTEAFEHEYIS IWASQRPFASKDDIEKKVGFCTFEPKEKRAPKATYTFQSFTVWEHINKLRLVSP GGIRALTDDERRLIYKQAFHKNKITFHDVRTLLNLPDDTRFKGLLYDRNTTLKE NEKVRFLELGAYHKIRKAIDSVYGKGAAKSFRPIDFDTFGYALTMFKDDTDIRS YLRNEYEQNGKRMENLADKVYDEELIEELLNLSFSKFGHLSLKALRNILPYMEQ GEVYSTACERAGYTFTGPKKKQKTVLLPNIPPIANPVVMRALTQARKVVNAIIK KYGSPVSIHIELARELSQSFDERRKMQKEQEGNRKKNETAIRQLVEYGLTLNPT GLDIVKFKLWSEQNGKCAYSLQPIEIERLLEPGYTEVDHVIPYSRSLDDSYTNKV LVLTKENREKGNRTPAEYLGLGSERWQQFETFVLTNKQFSKKKRDRLLRLHYD ENEENEFKNRNLNDTRYISRFLANFIREHLKFADSDDKQKVYTVNGRITAHLRS RWNFNKNREESNLHHAVDAAIVACTTPSDIARVTAFYQRREQNKELSKKTDPQ FPQPWPHFADELQARLSKNPKESIKALNLGNYDNEKLESLQPVFVSRMPKRSIT GAAHQETLRRYIGIDERSGKIQTVVKKKLSEIQLDKTGHFPMYGKESDPRTYEAI RQRLLEHNNDPKKAFQEPLYKPKKNGELGPIIRTIKIIDTTNQVIPLNDGKTVAY NSNIVRVDVFEKDGKYYCVPIYTIDMMKGILPNKAIEPNKPYSEWKEMTEDYTF RFSLYPNDLIRIEFPREKTIKTAVGEEIKIKDLFAYYQTIDSSNGGLSLVSHDNNFS LRSIGSRTLKRFEKYQVDVLGNIYKVRGEKRVGVASSSHSKAGETIRPL (SEQ ID NO: 25) |
| ScCas9 | MEKKYSIGLDIGTNSVGWAVITDDYKVPSKKFKVLGNTNRKSIKKNLMGALLF DSGETAEATRLKRTARRRYTRRKNRIRYLQEIFANEMAKLDDSFFQRLEESFLV EEDKKNERHPIFGNLADEVAYHRNYPTIYHLRKKLADSPEKADLRLIYLALAHII KFRGHFLIEGKLNAENSDVAKLFYQLIQTYNQLFEESPLDEIEVDAKGILSARLS KSKRLEKLIAVFPNEKKNGLFGNIIALALGLTPNFKSNFDLTEDAKLQLSKDTYD DDLDELLGQIGDQYADLFSAAKNLSDAILLSDILRSNSEVTKAPLSASMVKRYD EHHQDLALLKTLVRQQFPEKYAEIFKDDTKNGYAGYVGIGIKHRKRTTKLATQ EEFYKFIKPILEKMDGAEELLAKLNRDDLLRKQRTFDNGSIPHQIHLKELHAILR RQEEFYPFLKENREKIEKILTFRIPYYVGPLARGNSRFAWLTRKSEEAITPWNFEE VVDKGASAQSFIERMTNFDEQLPNKKVLPKHSLLYEYFTVYNELTKVKYVTER MRKPEFLSGEQKKAIVDLLFKTNRKVTVKQLKEDYFKKIECFDSVEIIGVEDRFN ASLGTYHDLLKIIKDKDFLDNEENEDILEDIVLTLTLFEDREMIEERLKTYAHLFD DKVMKQLKRRHYTGWGRLSRKMINGIRDKQSGKTILDFLKSDGFSNRNFMQLI HDDSLTFKEEIEKAQVSGQGDSLHEQIADLAGSPAIKKGILQTVKIVDELVKVM GHKPENIVIEMARENQTTTKGLQQSRERKKRIEEGIKELESQILKENPVENTQLQ NEKLYLYYLQNGRDMYVDQELDINRLSDYDVDHIVPQSFIKDDSIDNKVLTRSV ENRGKSDNVPSEEVVKKMKNYWRQLLNAKLITQRKFDNLTKAERGGLSEADK AGFIKRQLVETRQITKHVARILDSRMNTKRDKNDKPIREVKVITLKSKLVSDFRK DFQLYKVRDINNYHHAHDAYLNAVVGTALIKKYPKLESEFVYGDYKVYDVRK MIAKSEQEIGKATAKRFFYSNIMNFFKTEVKLANGEIRKRPLIETNGETGEVVW NKEKDFATVRKVLAMPQVNIVKKTEVQTGGFSKESILSKRESAKLIPRKKGWD TRKYGGFGSPTVAYSILVVAKVEKGKAKKLKSVKVLVGITIMEKGSYEKDPIGF LEAKGYKDIKKELIFKLPKYSLFELENGRRRMLASATELQKANELVLPQHLVRL LYYTQNISATTGSNNLGYIEQHREEFKEIFEKIIDFSEKYILKNKVNSNLKSSFDE QFAVSDSILLSNSFVSLLKYTSFGASGGFTFLDLDVKQGRLRYQTVTEVLDATLI YQSITGLYETRTDLSQLGGD (SEQ ID NO: 26) |
| *S. canis* | |
| 1375 AA | |
| 159.2 kDa | |

The base editors described herein may include any of the above Cas9 ortholog sequences, or any variants thereof having at least 80%, at least 85%, at least 90%, at least 95%, or at least 99% sequence identity thereto.

The napDNAbp may include any suitable homologs and/or orthologs or naturally occurring enzymes, such as, Cas9. Cas9 homologs and/or orthologs have been described in various species, including, but not limited to, *S. pyogenes* and *S. thermophilus.* Preferably, the Cas moiety is configured (e.g, mutagenized, recombinantly engineered, or otherwise obtained from nature) as a nickase, i.e., capable of cleaving only a single strand of the target double-stranded DNA. Additional suitable Cas9 nucleases and sequences will be apparent to those of skill in the art based on this disclosure, and such Cas9 nucleases and sequences include Cas9 sequences from the organisms and loci disclosed in Chylinski, Rhun, and Charpentier, "The tracrRNA and Cas9 families of type II CRISPR-Cas immunity systems" (2013) RNA Biology 10:5, 726-737; the entire contents of which are incorporated herein by reference. In some embodiments, a Cas9 nuclease has an inactive (e.g., an inactivated) DNA cleavage domain; that is, the Cas9 is a nickase. In some embodiments, the Cas9 protein comprises an amino acid sequence that is at least 80% identical to the amino acid sequence of a Cas9 protein as provided by any one of the variants of Table 3. In some embodiments, the Cas9 protein comprises an amino acid sequence that is at least 85%, at least 90%, at least 92%, at least 95%, at least 96%, at least 97%, at least 98%, at least 99%, or at least 99.5% identical to the amino acid sequence of a Cas9 protein as provided by any one of the Cas9 orthologs in the above tables.

### (3) Dead Cas9 variant

In certain embodiments, the base editors described herein may include a dead Cas9, e.g., dead SpCas9, which has no nuclease activity due to one or more mutations that inactive both nuclease domains of Cas9, namely the RuvC domain (which cleaves the non-protospacer DNA strand) and HNH domain (which cleaves the protospacer DNA strand). The nuclease inactivation may be due to one or mutations that result in one or more substitutions and/or deletions in the amino acid sequence of the encoded protein, or any variants thereof having at least 80%, at least 85%, at least 90%, at least 95%, or at least 99% sequence identity thereto.

As used herein, the term "dCas9" refers to a nuclease-inactive Cas9 or nuclease-dead Cas9, or a functional fragment thereof, and embraces any naturally occurring dCas9 from any organism, any naturally-occurring dCas9 equivalent or functional fragment thereof, any dCas9 homolog, ortholog, or paralog from any organism, and any mutant or variant of a dCas9, naturally-occurring or engineered. The term dCas9 is not meant to be particularly limiting and may be referred to as a "dCas9 or equivalent." Exemplary dCas9 proteins and method for making dCas9 proteins are further described herein and/or are described in the art and are incorporated herein by reference.

In other embodiments, dCas9 corresponds to, or comprises in part or in whole, a Cas9 amino acid sequence having one or more mutations that inactivate the Cas9 nuclease activity. In other embodiments, Cas9 variants having mutations other than D10A and H840A are provided which may result in the full or partial inactivation of the endogenous Cas9 nuclease activity (e.g., nCas9 or dCas9, respectively). Such mutations, by way of example, include other amino acid substitutions at D10 and H840, or other substitutions within the nuclease domains of Cas9 (e.g., substitutions in the HNH nuclease subdomain and/or the RuvCl subdomain) with reference to a wild type sequence such as Cas9 from *Streptococcus pyogenes* (NCBI Reference Sequence: NC_017053.1). In some embodiments, variants or homologues of Cas9 (e.g., variants of Cas9 from *Streptococcus pyogenes* (NCBI Reference Sequence: NC_017053.1)) are provided which are at least about 70% identical, at least about 80% identical, at least about 90% identical, at least about 95% identical, at least about 98% identical, at least about 99% identical, at least about 99.5% identical, or at least about 99.9% identical to NCBI Reference Sequence: NC_017053.1. In some embodiments, variants of dCas9 (e.g., variants of NCBI Reference Sequence: NC_017053.1) are provided having amino acid sequences which are shorter, or longer than NC_017053.1 by about 5 amino acids, by about 10 amino acids, by about 15 amino acids, by about 20 amino acids, by about 25 amino acids, by about 30 amino acids, by about 40 amino acids, by about 50 amino acids, by about 75 amino acids, by about 100 amino acids or more.

In one embodiment, the dead Cas9 may be based on the canonical SpCas9 sequence of Q99ZW2 and may have the following sequence, which comprises a D10X and an H810X, wherein X may be any amino acid, substitutions (underlined and bolded), or a variant be variant of SEQ ID NO: 40 having at least 80%, at least 85%, at least 90%, at least 95%, or at least 99% sequence identity thereto.

In one embodiment, the dead Cas9 may be based on the canonical SpCas9 sequence of Q99ZW2 and may have the following sequence, which comprises a D10A and an H810A substitutions (underlined and bolded), or may be a variant of SEQ ID NO: 27 having at least 80%, at least 85%, at least 90%, at least 95%, or at least 99% sequence identity thereto:

| Description | Sequence | SEQ ID NO: |
|---|---|---|
| dead Cas9 or dCas9 | MDKKYSIGLXIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDXIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 27 |
| *Streptococcus pyogenes* Q99ZW2 Cas9 with D10**X** and H810**X** | | |
| Where "X" is any amino acid | | |
| dead Cas9 or dCas9 | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN | 28 |
| *Streptococcus pyogenes* | | |
| Q99ZW2 Cas9 with D10**A** and H810**A** | LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDAIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | |

### (4) Cas9 nickase variant

In one embodiment, the base editors described herein comprise a Cas9 nickase. The term "Cas9 nickase" of "nCas9" refers to a variant of Cas9 which is capable of introducing a single-strand break in a double strand DNA molecule target. In some embodiments, the Cas9 nickase comprises only a single functioning nuclease domain. The wild type Cas9 (e.g., the canonical SpCas9) comprises two separate nuclease domains, namely, the RuvC domain (which cleaves the non-protospacer DNA strand) and HNH domain (which cleaves the protospacer DNA strand). In one embodiment, the Cas9 nickase comprises a mutation in the RuvC domain which inactivates the RuvC nuclease activity. For example, mutations in aspartate (D) 10, histidine (H) 983, aspartate (D) 986, or glutamate (E) 762, have been reported as loss-of-function mutations of the RuvC nuclease domain and the creation of a functional Cas9 nickase (e.g., Nishimasu et al., "Crystal structure of Cas9 in complex with guide RNA and target DNA," Cell 156(5), 935-949, which is incorporated herein by reference). Thus, nickase mutations in the RuvC domain could include D10X, H983X, D986X, or E762X, wherein X is any amino acid other than the wild type amino acid. In certain embodiments, the nickase could be D10A, of H983A, or D986A, or E762A, or a combination thereof.

In various embodiments, the Cas9 nickase can have a mutation in the RuvC nuclease domain and have one of the following amino acid sequences, or a variant thereof having an amino acid sequence that has at least 80%, at least 85%, at least 90%, at least 95%, or at least 99% sequence identity thereto.

| Description | Sequence | SEQ ID NO: |
|---|---|---|
| Cas9 nickase | MDKKYSIGLXIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 29 |
| *Streptococcus pyogenes* Q99ZW2 Cas9 with D10**X,** wherein X is any alternate amino acid | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIX MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 30 |
| *Streptococcus pyogenes* Q99ZW2 Cas9 with E762X, wherein X is any alternate amino acid | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HXAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 31 |
| *Streptococcus pyogenes* Q99ZW2 Cas9 with H983X, wherein X is any alternate amino acid | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE | 32 |
| *Streptococcus pyogenes* Q99ZW2 Cas9 with D986X, wherein X is any alternate amino acid | MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHXAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | |
| Cas9 nickase | MDKKYSIGLAIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 33 |
| *Streptococcus pyogenes* Q99ZW2 Cas9 with D10**A** | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIA MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 34 |
| *Streptococcus pyogenes* Q99ZW2 Cas9 with E762A | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HAAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 35 |
| *Streptococcus pyogenes* Q99ZW2 Cas9 with H983A | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHAAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 36 |
| *Streptococcus pyogenes* Q99ZW2 Cas9 with D986A | | |

In another embodiment, the Cas9 nickase comprises a mutation in the HNH domain which inactivates the HNH nuclease activity. For example, mutations in histidine (H) 840 or asparagine (R) 863 have been reported as loss-of-function mutations of the HNH nuclease domain and the creation of a functional Cas9 nickase (e.g., Nishimasu et al., "Crystal structure of Cas9 in complex with guide RNA and target DNA," Cell 156(5), 935-949, which is incorporated herein by reference). Thus, nickase mutations in the HNH domain could include H840X and R863X, wherein X is any amino acid other than the wild type amino acid. In certain embodiments, the nickase could be H840A or R863A or a combination thereof.

In various embodiments, the Cas9 nickase can have a mutation in the HNH nuclease domain and have one of the following amino acid sequences, or a variant thereof having an amino acid sequence that has at least 80%, at least 85%, at least 90%, at least 95%, or at least 99% sequence identity thereto.

| Description | Sequence | SEQ ID NO: |
|---|---|---|
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDXIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 37 |
| *Streptococcus pyogenes* Q99ZW2 Cas9 with H840**X**, wherein X is any alternate amino acid | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDAIVPQSFLKD DSIDNKVLTRSDKNRGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 38 |
| *Streptococcus pyogenes* Q99ZW2 Cas9 with H840**A** | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNXGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 39 |
| *Streptococcus pyogenes* Q99ZW2 Cas9 with R863X, wherein X is any alternate amino acid | | |
| Cas9 nickase | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYPT IYHLRKKLVDSTDKADLRLIYLALAHMIKFRGHFLIEGDLNPDNS DVDKLFIQLVQTYNQLFEENPINASGVDAKAILSARLSKSRRLEN LIAQLPGEKKNGLFGNLIALSLGLTPNFKSNFDLAEDAKLQLSKD TYDDDLDNLLAQIGDQYADLFLAAKNLSDAILLSDILRVNTEITK APLSASMIKRYDEHHQDLTLLKALVRQQLPEKYKEIFFDQSKNG YAGYIDGGASQEEFYKFIKPILEKMDGTEELLVKLNREDLLRKQR TFDNGSIPHQIHLGELHAILRRQEDFYPFLKDNREKIEKILTFRIPY YVGPLARGNSRFAWMTRKSEETITPWNFEEVVDKGASAQSFIER MTNFDKNLPNEKVLPKHSLLYEYFTVYNELTKVKYVTEGMRKP AFLSGEQKKAIVDLLFKTNRKVTVKQLKEDYFKKIECFDSVEISG VEDRFNASLGTYHDLLKIIKDKDFLDNEENEDILEDIVLTLTLFED REMIEERLKTYAHLFDDKVMKQLKRRRYTGWGRLSRKLINGIRD KQSGKTILDFLKSDGFANRNFMQLIHDDSLTFKEDIQKAQVSGQG DSLHEHIANLAGSPAIKKGILQTVKVVDELVKVMGRHKPENIVIE MARENQTTQKGQKNSRERMKRIEEGIKELGSQILKEHPVENTQL QNEKLYLYYLQNGRDMYVDQELDINRLSDYDVDHIVPQSFLKD DSIDNKVLTRSDKNAGKSDNVPSEEVVKKMKNYWRQLLNAKLI TQRKFDNLTKAERGGLSELDKAGFIKRQLVETRQITKHVAQILDS RMNTKYDENDKLIREVKVITLKSKLVSDFRKDFQFYKVREINNY HHAHDAYLNAVVGTALIKKYPKLESEFVYGDYKVYDVRKMIAK SEQEIGKATAKYFFYSNIMNFFKTEITLANGEIRKRPLIETNGETGE IVWDKGRDFATVRKVLSMPQVNIVKKTEVQTGGFSKESILPKRNS DKLIARKKDWDPKKYGGFDSPTVAYSVLVVAKVEKGKSKKLKS VKELLGITIMERSSFEKNPIDFLEAKGYKEVKKDLIIKLPKYSLFEL ENGRKRMLASAGELQKGNELALPSKYVNFLYLASHYEKLKGSPE DNEQKQLFVEQHKHYLDEIIEQISEFSKRVILADANLDKVLSAYN KHRDKPIREQAENIIHLFTLTNLGAPAAFKYFDTTIDRKRYTSTKE VLDATLIHQSITGLYETRIDLSQLGGD | 40 |
| *Streptococcus pyogenes* Q99ZW2 Cas9 with R863**A** | | |

In some embodiments, the N-terminal methionine is removed from a Cas9 nickase, or from any Cas9 variant, ortholog, or equivalent disclosed or contemplated herein. For example, methionine-minus Cas9 nickases include the following sequences, or a variant thereof having an amino acid sequence that has at least 80%, at least 85%, at least 90%, at least 95%, or at least 99% sequence identity thereto.

| Description | Sequence |
|---|---|
| Cas9 nickase (Met minus) | DKKYSIGLDIGTNSVGWAVITDEYKVPSKKFKVLGNTDRHSIKKNLIGALLFD SGETAEATRLKRTARRRYTRRKNRICYLQEIFSNEMAKVDDSFFHRLEESFLV EEDKKHERHPIFGNIVDEVAYHEKYPTIYHLRKKLVDSTDKADLRLIYLALAH MIKFRGHFLIEGDLNPDNSDVDKLFIQLVQTYNQLFEENPINASGVDAKAILSA RLSKSRRLENLIAQLPGEKKNGLFGNLIALSLGLTPNFKSNFDLAEDAKLQLS KDTYDDDLDNLLAQIGDQYADLFLAAKNLSDAILLSDILRVNTEITKAPLSAS MIKRYDEHHQDLTLLKALVRQQLPEKYKEIFFDQSKNGYAGYIDGGASQEEF YKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEE VVDKGASAQSFIERMTNFDKNLPNEKVLPKHSLLYEYFTVYNELTKVKYVTE GMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTY AHLFDDKVMKQLKRRRYTGWGRLSRKLINGIRDKQSGKTILDFLKSDGFANR NFMQLIHDDSLTFKEDIQKAQVSGQGDSLHEHIANLAGSPAIKKGILQTVKVV DELVKVMGRHKPENIVIEMARENQTTQKGQKNSRERMKRIEEGIKELGSQIL KEHPVENTQLQNEKLYLYYLQNGRDMYVDQELDINRLSDYDVDXIVPQSFL KDDSIDNKVLTRSDKNRGKSDNVPSEEVVKKMKNYWRQLLNAKLITQRKFD NLTKAERGGLSELDKAGFIKRQLVETRQITKHVAQILDSRMNTKYDENDKLIR EVKVITLKSKLVSDFRKDFQFYKVREINNYHHAHDAYLNAVVGTALIKKYPK LESEFVYGDYKVYDVRKMIAKSEQEIGKATAKYFFYSNIMNFFKTEITLANGE IRKRPLIETNGETGEIVWDKGRDFATVRKVLSMPQVNIVKKTEVQTGGFSKES ILPKRNSDKLIARKKDWDPKKYGGFDSPTVAYSVLVVAKVEKGKSKKLKSV KELLGITIMERSSFEKNPIDFLEAKGYKEVKKDLIIKLPKYSLFELENGRKRML ASAGELQKGNELALPSKYVNFLYLASHYEKLKGSPEDNEQKQLFVEQHKHY LDEIIEQISEFSKRVILADANLDKVLSAYNKHRDKPIREQAENIIHLFTLTNLGA PAAFKYFDTTIDRKRYTSTKEVLDATLIHQSITGLYETRIDLSQLGGD (SEQ ID NO: 41) |
| *Streptococcus pyogenes* Q99ZW2 Cas9 with H840**X**, wherein X is any alternate amino acid | |
| Cas9 nickase (Met minus) | DKKYSIGLDIGTNSVGWAVITDEYKVPSKKFKVLGNTDRHSIKKNLIGALLFD SGETAEATRLKRTARRRYTRRKNRICYLQEIFSNEMAKVDDSFFHRLEESFLV EEDKKHERHPIFGNIVDEVAYHEKYPTIYHLRKKLVDSTDKADLRLIYLALAH MIKFRGHFLIEGDLNPDNSDVDKLFIQLVQTYNQLFEENPINASGVDAKAILSA RLSKSRRLENLIAQLPGEKKNGLFGNLIALSLGLTPNFKSNFDLAEDAKLQLS KDTYDDDLDNLLAQIGDQYADLFLAAKNLSDAILLSDILRVNTEITKAPLSAS MIKRYDEHHQDLTLLKALVRQQLPEKYKEIFFDQSKNGYAGYIDGGASQEEF YKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEE VVDKGASAQSFIERMTNFDKNLPNEKVLPKHSLLYEYFTVYNELTKVKYVTE GMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTY AHLFDDKVMKQLKRRRYTGWGRLSRKLINGIRDKQSGKTILDFLKSDGFANR NFMQLIHDDSLTFKEDIQKAQVSGQGDSLHEHIANLAGSPAIKKGILQTVKVV DELVKVMGRHKPENIVIEMARENQTTQKGQKNSRERMKRIEEGIKELGSQIL KEHPVENTQLQNEKLYLYYLQNGRDMYVDQELDINRLSDYDVDAIVPQSFL KDDSIDNKVLTRSDKNRGKSDNVPSEEVVKKMKNYWRQLLNAKLITQRKFD NLTKAERGGLSELDKAGFIKRQLVETRQITKHVAQILDSRMNTKYDENDKLIR EVKVITLKSKLVSDFRKDFQFYKVREINNYHHAHDAYLNAVVGTALIKKYPK LESEFVYGDYKVYDVRKMIAKSEQEIGKATAKYFFYSNIMNFFKTEITLANGE IRKRPLIETNGETGEIVWDKGRDFATVRKVLSMPQVNIVKKTEVQTGGFSKES ILPKRNSDKLIARKKDWDPKKYGGFDSPTVAYSVLVVAKVEKGKSKKLKSV KELLGITIMERSSFEKNPIDFLEAKGYKEVKKDLIIKLPKYSLFELENGRKRML ASAGELQKGNELALPSKYVNFLYLASHYEKLKGSPEDNEQKQLFVEQHKHY LDEIIEQISEFSKRVILADANLDKVLSAYNKHRDKPIREQAENIIHLFTLTNLGA PAAFKYFDTTIDRKRYTSTKEVLDATLIHQSITGLYETRIDLSQLGGD (SEQ ID NO: 42) |
| *Streptococcus pyogenes* Q99ZW2 Cas9 with H840**A** | |
| Cas9 nickase (Met minus) | DKKYSIGLDIGTNSVGWAVITDEYKVPSKKFKVLGNTDRHSIKKNLIGALLFD SGETAEATRLKRTARRRYTRRKNRICYLQEIFSNEMAKVDDSFFHRLEESFLV EEDKKHERHPIFGNIVDEVAYHEKYPTIYHLRKKLVDSTDKADLRLIYLALAH MIKFRGHFLIEGDLNPDNSDVDKLFIQLVQTYNQLFEENPINASGVDAKAILSA RLSKSRRLENLIAQLPGEKKNGLFGNLIALSLGLTPNFKSNFDLAEDAKLQLS KDTYDDDLDNLLAQIGDQYADLFLAAKNLSDAILLSDILRVNTEITKAPLSAS MIKRYDEHHQDLTLLKALVRQQLPEKYKEIFFDQSKNGYAGYIDGGASQEEF YKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEE |
| *Streptococcus pyogenes* Q99ZW2 Cas9 with R863X, wherein X is | |
| any alternate amino acid | VVDKGASAQSFIERMTNFDKNLPNEKVLPKHSLLYEYFTVYNELTKVKYVTE GMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTY AHLFDDKVMKQLKRRRYTGWGRLSRKLINGIRDKQSGKTILDFLKSDGFANR NFMQLIHDDSLTFKEDIQKAQVSGQGDSLHEHIANLAGSPAIKKGILQTVKVV DELVKVMGRHKPENIVIEMARENQTTQKGQKNSRERMKRIEEGIKELGSQIL KEHPVENTQLQNEKLYLYYLQNGRDMYVDQELDINRLSDYDVDHIVPQSFL KDDSIDNKVLTRSDKNXGKSDNVPSEEVVKKMKNYWRQLLNAKLITQRKFD NLTKAERGGLSELDKAGFIKRQLVETRQITKHVAQILDSRMNTKYDENDKLIR EVKVITLKSKLVSDFRKDFQFYKVREINNYHHAHDAYLNAVVGTALIKKYPK LESEFVYGDYKVYDVRKMIAKSEQEIGKATAKYFFYSNIMNFFKTEITLANGE IRKRPLIETNGETGEIVWDKGRDFATVRKVLSMPQVNIVKKTEVQTGGFSKES ILPKRNSDKLIARKKDWDPKKYGGFDSPTVAYSVLVVAKVEKGKSKKLKSV KELLGITIMERSSFEKNPIDFLEAKGYKEVKKDLIIKLPKYSLFELENGRKRML ASAGELQKGNELALPSKYVNFLYLASHYEKLKGSPEDNEQKQLFVEQHKHY LDEIIEQISEFSKRVILADANLDKVLSAYNKHRDKPIREQAENIIHLFTLTNLGA PAAFKYFDTTIDRKRYTSTKEVLDATLIHQSITGLYETRIDLSQLGGD(SEQ ID NO: 43) |
| Cas9 nickase (Met minus) | DKKYSIGLDIGTNSVGWAVITDEYKVPSKKFKVLGNTDRHSIKKNLIGALLFD SGETAEATRLKRTARRRYTRRKNRICYLQEIFSNEMAKVDDSFFHRLEESFLV EEDKKHERHPIFGNIVDEVAYHEKYPTIYHLRKKLVDSTDKADLRLIYLALAH MIKFRGHFLIEGDLNPDNSDVDKLFIQLVQTYNQLFEENPINASGVDAKAILSA RLSKSRRLENLIAQLPGEKKNGLFGNLIALSLGLTPNFKSNFDLAEDAKLQLS KDTYDDDLDNLLAQIGDQYADLFLAAKNLSDAILLSDILRVNTEITKAPLSAS MIKRYDEHHQDLTLLKALVRQQLPEKYKEIFFDQSKNGYAGYIDGGASQEEF YKFIKPILEKMDGTEELLVKLNREDLLRKQRTFDNGSIPHQIHLGELHAILRRQ EDFYPFLKDNREKIEKILTFRIPYYVGPLARGNSRFAWMTRKSEETITPWNFEE VVDKGASAQSFIERMTNFDKNLPNEKVLPKHSLLYEYFTVYNELTKVKYVTE GMRKPAFLSGEQKKAIVDLLFKTNRKVTVKQLKEDYFKKIECFDSVEISGVED RFNASLGTYHDLLKIIKDKDFLDNEENEDILEDIVLTLTLFEDREMIEERLKTY AHLFDDKVMKQLKRRRYTGWGRLSRKLINGIRDKQSGKTILDFLKSDGFANR NFMQLIHDDSLTFKEDIQKAQVSGQGDSLHEHIANLAGSPAIKKGILQTVKVV DELVKVMGRHKPENIVIEMARENQTTQKGQKNSRERMKRIEEGIKELGSQIL KEHPVENTQLQNEKLYLYYLQNGRDMYVDQELDINRLSDYDVDHIVPQSFL KDDSIDNKVLTRSDKNAGKSDNVPSEEVVKKMKNYWRQLLNAKLITQRKFD NLTKAERGGLSELDKAGFIKRQLVETRQITKHVAQILDSRMNTKYDENDKLIR EVKVITLKSKLVSDFRKDFQFYKVREINNYHHAHDAYLNAVVGTALIKKYPK LESEFVYGDYKVYDVRKMIAKSEQEIGKATAKYFFYSNIMNFFKTEITLANGE IRKRPLIETNGETGEIVWDKGRDFATVRKVLSMPQVNIVKKTEVQTGGFSKES ILPKRNSDKLIARKKDWDPKKYGGFDSPTVAYSVLVVAKVEKGKSKKLKSV KELLGITIMERSSFEKNPIDFLEAKGYKEVKKDLIIKLPKYSLFELENGRKRML ASAGELQKGNELALPSKYVNFLYLASHYEKLKGSPEDNEQKQLFVEQHKHY LDEIIEQISEFSKRVILADANLDKVLSAYNKHRDKPIREQAENIIHLFTLTNLGA PAAFKYFDTTIDRKRYTSTKEVLDATLIHQSITGLYETRIDLSQLGGD (SEQ ID NO: 44) |
| *Streptococcus pyogenes* Q99ZW2 Cas9 with R863A | |

### (5) Other Cas9 variants

Besides dead Cas9 and Cas9 nickase variants, the Cas9 proteins used herein may also include other "Cas9 variants" having at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to any reference Cas9 protein, including any wild type Cas9, or mutant Cas9 (e.g., a dead Cas9 or Cas9 nickase), or fragment Cas9, or circular permutant Cas9, or other variant of Cas9 disclosed herein or known in the art. In some embodiments, a Cas9 variant may have 1, 2, 3, 4, 5, 6, 7, 8, 9, 10, 11, 12, 13, 14, 15, 16, 17, 18, 19, 20, 21, 22, 21, 24, 25, 26, 27, 28, 29, 30, 31, 32, 33, 34, 35, 36, 37, 38, 39, 40, 41, 42, 43, 44, 45, 46, 47, 48, 49, 50 or more amino acid changes compared to a reference Cas9. In some embodiments, the Cas9 variant comprises a fragment of a reference Cas9 (*e.g.,* a gRNA binding domain or a DNA-cleavage domain), such that the fragment is at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to the corresponding fragment of wild type Cas9. In some embodiments, the fragment is is at least 30%, at least 35%, at least 40%, at least 45%, at least 50%, at least 55%, at least 60%, at least 65%, at least 70%, at least 75%, at least 80%, at least 85%, at least 90%, at least 95% identical, at least 96%, at least 97%, at least 98%, at least 99%, or at least 99.5% of the amino acid length of a corresponding wild type Cas9 (*e.g.,* SEQ ID NO: 5).

In some embodiments, the disclosure also may utilize Cas9 fragments which retain their functionality and which are fragments of any herein disclosed Cas9 protein. In some embodiments, the Cas9 fragment is at least 100 amino acids in length. In some embodiments, the fragment is at least 100, 150, 200, 250, 300, 350, 400, 450, 500, 550, 600, 650, 700, 750, 800, 850, 900, 950, 1000, 1050, 1100, 1150, 1200, 1250, or at least 1300 amino acids in length.

In various embodiments, the base editors disclosed herein may comprise one of the Cas9 variants described as follows, or a Cas9 variant thereof having at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to any reference Cas9 variants.

### (6) Small-sized Cas9 variants

In some embodiments, the base editors contemplated herein can include a Cas9 protein that is of smaller molecular weight than the canonical SpCas9 sequence. In some embodiments, the smaller-sized Cas9 variants may facilitate delivery to cells, e.g., by an expression vector, nanoparticle, or other means of delivery. In certain embodiments, the smaller-sized Cas9 variants can include enzymes categorized as type II enzymes of the Class 2 CRISPR-Cas systems. In some embodiments, the smaller-sized Cas9 variants can include enzymes categorized as type V enzymes of the Class 2 CRISPR-Cas systems. In other embodiments, the smaller-sized Cas9 variants can include enzymes categorized as type VI enzymes of the Class 2 CRISPR-Cas systems.

The canonical SpCas9 protein is 1368 amino acids in length and has a predicted molecular weight of 158 kilodaltons. The term "small-sized Cas9 variant", as used herein, refers to any Cas9 variant-naturally occurring, engineered, or otherwise-that is less than at least 1300 amino acids, or at least less than 1290 amino acids, or than less than 1280 amino acids, or less than 1270 amino acid, or less than 1260 amino acid, or less than 1250 amino acids, or less than 1240 amino acids, or less than 1230 amino acids, or less than 1220 amino acids, or less than 1210 amino acids, or less than 1200 amino acids, or less than 1190 amino acids, or less than 1180 amino acids, or less than 1170 amino acids, or less than 1160 amino acids, or less than 1150 amino acids, or less than 1140 amino acids, or less than 1130 amino acids, or less than 1120 amino acids, or less than 1110 amino acids, or less than 1100 amino acids, or less than 1050 amino acids, or less than 1000 amino acids, or less than 950 amino acids, or less than 900 amino acids, or less than 850 amino acids, or less than 800 amino acids, or less than 750 amino acids, or less than 700 amino acids, or less than 650 amino acids, or less than 600 amino acids, or less than 550 amino acids, or less than 500 amino acids, but at least larger than about 400 amino acids and retaining the required functions of the Cas9 protein. The Cas9 variants can include those categorized as type II, type V, or type VI enzymes of the Class 2 CRISPR-Cas system.

In various embodiments, the base editors disclosed herein may comprise one of the small-sized Cas9 variants described as follows, or a Cas9 variant thereof having at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to any reference small-sized Cas9 protein.

| Description | Sequence | SEQ ID NO: |
|---|---|---|
| SaCas9 | MGKRNYILGLDIGITSVGYGIIDYETRDVIDAGVRLFKEANVENNE GRRSKRGARRLKRRRRHRIQRVKKLLFDYNLLTDHSELSGINPYE ARVKGLSQKLSEEEFSAALLHLAKRRGVHNVNEVEEDTGNELSTK EQISRNSKALEEKYVAELQLERLKKDGEVRGSINRFKTSDYVKEA KQLLKVQKAYHQLDQSFIDTYIDLLETRRTYYEGPGEGSPFGWKD IKEWYEMLMGHCTYFPEELRSVKYAYNADLYNALNDLNNLVITR DENEKLEYYEKFQIIENVFKQKKKPTLKQIAKEILVNEEDIKGYRV TSTGKPEFTNLKVYHDIKDITARKEIIENAELLDQIAKILTIYQSSEDI QEELTNLNSELTQEEIEQISNLKGYTGTHNLSLKAINLILDELWHTN DNQIAIFNRLKLVPKKVDLSQQKEIPTTLVDDFILSPVVKRSFIQSIK VINAIIKKYGLPNDIIIELAREKNSKDAQKMINEMQKRNRQTNERIE EIIRTTGKENAKYLIEKIKLHDMQEGKCLYSLEAIPLEDLLNNPFNY EVDHIIPRSVSFDNSFNNKVLVKQEENSKKGNRTPFQYLSSSDSKIS YETFKKHILNLAKGKGRISKTKKEYLLEERDINRFSVQKDFINRNL VDTRYATRGLMNLLRSYFRVNNLDVKVKSINGGFTSFLRRKWKF KKERNKGYKHHAEDALIIANADFIFKEWKKLDKAKKVMENQMFE EKQAESMPEIETEQEYKEIFITPHQIKHIKDFKDYKYSHRVDKKPN RKLINDTLYSTRKDDKGNTLIVNNLNGLYDKDNDKLKKLINKSPE KLLMYHHDPQTYQKLKLIMEQYGDEKNPLYKYYEETGNYLTKYS KKDNGPVIKKIKYYGNKLNAHLDITDDYPNSRNKVVKLSLKPYRF DVYLDNGVYKFVTVKNLDVIKKENYYEVNSKCYEEAKKLKKISN QAEFIASFYKNDLIKINGELYRVIGVNNDLLNRIEVNMIDITYREYL ENMNDKRPPHIIKTIASKTQSIKKYSTDILGNLYEVKSKKHPQIIKK | 45 |
| *Staphylococcu s aureus* | | |
| 1053 AA | | |
| 123 kDa | | |
| NmeCas9 | MAAFKPNSINYILGLDIGIASVGWAMVEIDEEENPIRLIDLGVRVFE RAEVPKTGDSLAMARRLARSVRRLTRRRAHRLLRTRRLLKREGV | 46 |
| *N. meningitidis* | LQAANFDENGLIKSLPNTPWQLRAAALDRKLTPLEWSAVLLHLIK HRGYLSQRKNEGETADKELGALLKGVAGNAHALQTGDFRTPAEL ALNKFEKESGHIRNQRSDYSHTFSRKDLQAELILLFEKQKEFGNPH VSGGLKEGIETLLMTQRPALSGDAVQKMLGHCTFEPAEPKAAKN TYTAERFIWLTKLNNLRILEQGSERPLTDTERATLMDEPYRKSKLT YAQARKLLGLEDTAFFKGLRYGKDNAEASTLMEMKAYHAISRAL EKEGLKDKKSPLNLSPELQDEIGTAFSLFKTDEDITGRLKDRIQPEI LEALLKHISFDKFVQISLKALRRIVPLMEQGKRYDEACAEIYGDHY GKKNTEEKIYLPPIPADEIRNPVVLRALSQARKVINGVVRRYGSPA RIHIETAREVGKSFKDRKEIEKRQEENRKDREKAAAKFREYFPNFV GEPKSKDILKLRLYEQQHGKCLYSGKEINLGRLNEKGYVEIDAAL PFSRTWDDSFNNKVLVLGSENQNKGNQTPYEYFNGKDNSREWQE FKARVETSRFPRSKKQRILLQKFDEDGFKERNLNDTRYVNRFLCQ FVADRMRLTGKGKKRVFASNGQITNLLRGFWGLRKVRAENDRH HALDAVVVACSTVAMQQKITRFVRYKEMNAFDGKTIDKETGEVL HQKTHFPQPWEFFAQEVMIRVFGKPDGKPEFEEADTLEKLRTLLA EKLSSRPEAVHEYVTPLFVSRAPNRKMSGQGHMETVKSAKRLDE GVSVLRVPLTQLKLKDLEKMVNREREPKLYEALKARLEAHKDDP AKAFAEPFYKYDKAGNRTQQVKAVRVEQVQKTGVWVRNHNGIA DNATMVRVDVFEKGDKYYLVPIYSWQVAKGILPDRAVVQGKDE EDWQLIDDSFNFKFSLHPNDLVEVITKKARMFGYFASCHRGTGNI NIRIHDLDHKIGKNGILEGIGVKTALSFQKYQIDELGKEIRPCRLKK RPPVR | |
| 1083 AA | | |
| 124.5 kDa | | |
| CjCas9 | MARILAFDIGISSIGWAFSENDELKDCGVRIFTKVENPKTGESLALP RRLARSARKRLARRKARLNHLKHLIANEFKLNYEDYQSFDESLAK AYKGSLISPYELRFRALNELLSKQDFARVILHIAKRRGYDDIKNSD DKEKGAILKAIKQNEEKLANYQSVGEYLYKEYFQKFKENSKEFTN VRNKKESYERCIAQSFLKDELKLIFKKQREFGFSFSKKFEEEVLSV AFYKRALKDFSHLVGNCSFFTDEKRAPKNSPLAFMFVALTRIINLL NNLKNTEGILYTKDDLNALLNEVLKNGTLTYKQTKKLLGLSDDY EFKGEKGTYFIEFKKYKEFIKALGEHNLSQDDLNEIAKDITLIKDEI KLKKALAKYDLNQNQIDSLSKLEFKDHLNISFKALKLVTPLMLEG KKYDEACNELNLKVAINEDKKDFLPAFNETYYKDEVTNPVVLRAI KEYRKVLNALLKKYGKVHKINIELAREVGKNHSQRAKIEKEQNE NYKAKKDAELECEKLGLKINSKNILKLRLFKEQKEFCAYSGEKIKI SDLQDEKMLEIDHIYPYSRSFDDSYMNKVLVFTKQNQEKLNQTPF | 47 |
| *C. jejuni* | | |
| 984 AA | | |
| 114.9 kDa | | |
| | EAFGNDSAKWQKIEVLAKNLPTKKQKRILDKNYKDKEQKNFKDR NLNDTRYIARLVLNYTKDYLDFLPLSDDENTKLNDTQKGSKVHV EAKSGMLTSALRHTWGFSAKDRNNHLHHAIDAVIIAYANNSIVKA FSDFKKEQESNSAELYAKKISELDYKNKRKFFEPFSGFRQKVLDKI DEIFVSKPERKKPSGALHEETFRKEEEFYQSYGGKEGVLKALELGK IRKVNGKIVKNGDMFRVDIFKHKKTNKFYAVPIYTMDFALKVLPN KAVARSKKGEIKDWILMDENYEFCFSLYKDSLILIQTKDMQEPEFV YYNAFTSSTVSLIVSKHDNKFETLSKNQKILFKNANEKEVIAKSIGI QNLKVFEKYIVSALGEVTKAEFRQREDFKK | |
| GeoCas9 | MRYKIGLDIGITSVGWAVMNLDIPRIEDLGVRIFDRAENPQTGESL ALPRRLARSARRRLRRRKHRLERIRRLVIREGILTKEELDKLFEEKH EIDVWQLRVEALDRKLNNDELARVLLHLAKRRGFKSNRKSERSN KENSTMLKHIEENRAILSSYRTVGEMIVKDPKFALHKRNKGENYT NTIARDDLEREIRLIFSKQREFGNMSCTEEFENEYITIWASQRPVAS KDDIEKKVGFCTFEPKEKRAPKATYTFQSFIAWEHINKLRLISPSGA RGLTDEERRLLYEQAFQKNKITYHDIRTLLHLPDDTYFKGIVYDRG ESRKQNENIRFLELDAYHQIRKAVDKVYGKGKSSSFLPIDFDTFGY ALTLFKDDADIHSYLRNEYEQNGKRMPNLANKVYDNELIEELLNL SFTKFGHLSLKALRSILPYMEQGEVYSSACERAGYTFTGPKKKQK TMLLPNIPPIANPVVMRALTQARKVVNAIIKKYGSPVSIHIELARDL SQTFDERRKTKKEQDENRKKNETAIRQLMEYGLTLNPTGHDIVKF KLWSEQNGRCAYSLQPIEIERLLEPGYVEVDHVIPYSRSLDDSYTN KVLVLTRENREKGNRIPAEYLGVGTERWQQFETFVLTNKQFSKKK RDRLLRLHYDENEETEFKNRNLNDTRYISRFFANFIREHLKFAESD DKQKVYTVNGRVTAHLRSRWEFNKNREESDLHHAVDAVIVACTT PSDIAKVTAFYQRREQNKELAKKTEPHFPQPWPHFADELRARLSK HPKESIKALNLGNYDDQKLESLQPVFVSRMPKRSVTGAAHQETLR RYVGIDERSGKIQTVVKTKLSEIKLDASGHFPMYGKESDPRTYEAI RQRLLEHNNDPKKAFQEPLYKPKKNGEPGPVIRTVKIIDTKNQVIP LNDGKTVAYNSNIVRVDVFEKDGKYYCVPVYTMDIMKGILPNKA IEPNKPYSEWKEMTEDYTFRFSLYPNDLIRIELPREKTVKTAAGEEI NVKDVFVYYKTIDSANGGLELISHDHRFSLRGVGSRTLKRFEKYQ VDVLGNIYKVRGEKRVGLASSAHSKPGKTIRPLQSTRD | 48 |
| *G. stearothermop hilus* | | |
| 1087 AA | | |
| 127 kDa | | |
| LbaCas12a | MSKLEKFTNCYSLSKTLRFKAIPVGKTQENIDNKRLLVEDEKRAE DYKGVKKLLDRYYLSFINDVLHSIKLKNLNNYISLFRKKTRTEKEN KELENLEINLRKEIAKAFKGNEGYKSLFKKDIIETILPEFLDDKDEIA | 49 |
| *L. bacterium* | | |
| 1228 AA | LVNSFNGFTTAFTGFFDNRENMFSEEAKSTSIAFRCINENLTRYISN MDIFEKVDAIFDKHEVQEIKEKILNSDYDVEDFFEGEFFNFVLTQE GIDVYNAIIGGFVTESGEKIKGLNEYINLYNQKTKQKLPKFKPLYK QVLSDRESLSFYGEGYTSDEEVLEVFRNTLNKNSEIFSSIKKLEKLF KNFDEYSSAGIFVKNGPAISTISKDIFGEWNVIRDKWNAEYDDIHL KKKAVVTEKYEDDRRKSFKKIGSFSLEQLQEYADADLSVVEKLKE IIIQKVDEIYKVYGSSEKLFDADFVLEKSLKKNDAVVAIMKDLLDS VKSFENYIKAFFGEGKETNRDESFYGDFVLAYDILLKVDHIYDAIR NYVTQKPYSKDKFKLYFQNPQFMGGWDKDKETDYRATILRYGSK YYLAIMDKKYAKCLQKIDKDDVNGNYEKINYKLLPGPNKMLPKV FFSKKWMAYYNPSEDIQKIYKNGTFKKGDMFNLNDCHKLIDFFK DSISRYPKWSNAYDFNFSETEKYKDIAGFYREVEEQGYKVSFESAS KKEVDKLVEEGKLYMFQIYNKDFSDKSHGTPNLHTMYFKLLFDE NNHGQIRLSGGAELFMRRASLKKEELVVHPANSPIANKNPDNPKK TTTLSYDVYKDKRFSEDQYELHIPIAINKCPKNIFKINTEVRVLLKH DDNPYVIGIDRGERNLLYIVVVDGKGNIVEQYSLNEIINNFNGIRIK TDYHSLLDKKEKERFEARQNWTSIENIKELKAGYISQVVHKICELV EKYDAVIALEDLNSGFKNSRVKVEKQVYQKFEKMLIDKLNYMVD KKSNPCATGGALKGYQITNKFESFKSMSTQNGFIFYIPAWLTSKID PSTGFVNLLKTKYTSIADSKKFISSFDRIMYVPEEDLFEFALDYKNF SRTDADYIKKWKLYSYGNRIRIFRNPKKNNVFDWEEVCLTSAYKE LFNKYGINYQQGDIRALLCEQSDKAFYSSFMALMSLMLQMRNSIT GRTDVDFLISPVKNSDGIFYDSRNYEAQENAILPKNADANGAYNIA RKVLWAIGQFKKAEDEKLDKVKIAISNKEWLEYAQTSVKH | |
| 143.9 kDa | | |
| BhCas12b | MATRSFILKIEPNEEVKKGLWKTHEVLNHGIAYYMNILKLIRQEAI YEHHEQDPKNPKKVSKAEIQAELWDFVLKMQKCNSFTHEVDKDE VFNILRELYEELVPSSVEKKGEANQLSNKFLYPLVDPNSQSGKGTA SSGRKPRWYNLKIAGDPSWEEEKKKWEEDKKKDPLAKILGKLAE YGLIPLFIPYTDSNEPIVKEIKWMEKSRNQSVRRLDKDMFIQALERF LSWESWNLKVKEEYEKVEKEYKTLEERIKEDIQALKALEQYEKER QEQLLRDTLNTNEYRLSKRGLRGWREIIQKWLKMDENEPSEKYLE VFKDYQRKHPREAGDYSVYEFLSKKENHFIWRNHPEYPYLYATFC EIDKKKKDAKQQATFTLADPINHPLWVRFEERSGSNLNKYRILTE QLHTEKLKKKLTVQLDRLIYPTESGGWEEKGKVDIVLLPSRQFYN QIFLDIEEKGKHAFTYKDESIKFPLKGTLGGARVQFDRDHLRRYPH KVESGNVGRIYFNMTVNIEPTESPVSKSLKIHRDDFPKVVNFKPKE | 50 |
| *B. hisashii* | | |
| 1108 AA | | |
| 130.4 kDa | | |
| | LTEWIKDSKGKKLKSGIESLEIGLRVMSIDLGQRQAAAASIFEVVD QKPDIEGKLFFPIKGTELYAVHRASFNIKLPGETLVKSREVLRKAR EDNLKLMNQKLNFLRNVLHFQQFEDITEREKRVTKWISRQENSDV PLVYQDELIQIRELMYKPYKDWVAFLKQLHKRLEVEIGKEVKHW RKSLSDGRKGLYGISLKNIDEIDRTRKFLLRWSLRPTEPGEVRRLEP GQRFAIDQLNHLNALKEDRLKKMANTIIMHALGYCYDVRKKKW QAKNPACQIILFEDLSNYNPYEERSRFENSKLMKWSRREIPRQVAL QGEIYGLQVGEVGAQFSSRFHAKTGSPGIRCSVVTKEKLQDNRFF KNLQREGRLTLDKIAVLKEGDLYPDKGGEKFISLSKDRKCVTTHA DINAAQNLQKRFWTRTHGFYKVYCKAYQVDGQTVYIPESKDQKQ KIIEEFGEGYFILKDGVYEWVNAGKLKIKKGSSKQSSSELVDSDIL KDSFDLASELKGEKLMLYRDPSGNVFPSDKWMAAGVFFGKLERI LISKLTNQYSISTIEDDSSKQSM | |

### (7) Cas9 equivalents

In some embodiments, the base editors described herein can include any Cas9 equivalent. As used herein, the term "Cas9 equivalent" is a broad term that encompasses any napDNAbp protein that serves the same function as Cas9 in the present base editors despite that its amino acid primary sequence and/or its three-dimensional structure may be different and/or unrelated from an evolutionary standpoint. Thus, while Cas9 equivalents include any Cas9 ortholog, homolog, mutant, or variant described or embraced herein that are evolutionarily related, the Cas9 equivalents also embrace proteins that may have evolved through convergent evolution processes to have the same or similar function as Cas9, but which do not necessarily have any similarity with regard to amino acid sequence and/or three dimensional structure. The base editors described here embrace any Cas9 equivalent that would provide the same or similar function as Cas9 despite that the Cas9 equivalent may be based on a protein that arose through convergent evolution. For instance, if Cas9 refers to a type II enzyme of the CRISPR-Cas system, a Cas9 equivalent can refer to a type V or type VI enzyme of the CRISPR-Cas system.

For example, Cas12e (CasX) is a Cas9 equivalent that reportedly has the same function as Cas9 but which evolved through convergent evolution. Thus, the Cas12e (CasX) protein described in Liu et al., "CasX enzymes comprises a distinct family of RNA-guided genome editors," Nature, 2019, Vol.566: 218-223, is contemplated to be used with the base editors described herein. In addition, any variant or modification of Cas12e (CasX) is conceivable and within the scope of the present disclosure.

Cas9 is a bacterial enzyme that evolved in a wide variety of species. However, the Cas9 equivalents contemplated herein may also be obtained from archaea, which constitute a domain and kingdom of single-celled prokaryotic microbes different from bacteria.

In some embodiments, Cas9 equivalents may refer to Cas12e (CasX) or Cas12d (CasY), which have been described in, for example, Burstein et al., "New CRISPR-Cas systems from uncultivated microbes." Cell Res. 2017 Feb 21. doi: 10.1038/cr.2017.21, the entire contents of which is hereby incorporated by reference. Using genome-resolved metagenomics, a number of CRISPR-Cas systems were identified, including the first reported Cas9 in the archaeal domain of life. This divergent Cas9 protein was found in little-studied nanoarchaea as part of an active CRISPR-Cas system. In bacteria, two previously unknown systems were discovered, CRISPR-Cas12e and CRISPR-Cas12d, which are among the most compact systems yet discovered. In some embodiments, Cas9 refers to Cas12e, or a variant of Cas12e. In some embodiments, Cas9 refers to a Cas12d, or a variant of Cas12d. It should be appreciated that other RNA-guided DNA binding proteins may be used as a nucleic acid programmable DNA binding protein (napDNAbp), and are within the scope of this disclosure. Also see Liu et al., "CasX enzymes comprises a distinct family of RNA-guided genome editors," Nature, 2019, Vol.566: 218-223. Any of these Cas9 equivalents are contemplated.

In some embodiments, the Cas9 equivalent comprises an amino acid sequence that is at least 85%, at least 90%, at least 91%, at least 92%, at least 93%, at least 94%, at least 95%, at least 96%, at least 97%, at least 98%, at least 99%, or at least 99.5% identical to a naturally-occurring Cas12e (CasX) or Cas12d (CasY) protein. In some embodiments, the napDNAbp is a naturally-occurring Cas12e (CasX) or Cas12d (CasY) protein. In some embodiments, the napDNAbp comprises an amino acid sequence that is at least 85%, at least 90%, at least 91%, at least 92%, at least 93%, at least 94%, at least 95%, at least 96%, at least 97%, at least 98%, at least 99%, or at least 99.5% identical to a wild-type Cas moiety or any Cas moiety provided herein.

In various embodiments, the nucleic acid programmable DNA binding proteins include, without limitation, Cas9 (*e.g.,* dCas9 and nCas9), C2C3Cas12e (CasX), Cas12d (CasY), Cas12a (Cpfl), Cas12b1 (C2c1), Cas13a (C2c2), Cas12c (C2c3), Argonaute, . One example of a nucleic acid programmable DNA-binding protein that has different PAM specificity than Cas9 is Clustered Regularly Interspaced Short Palindromic Repeats from Prevotella and Francisella 1 (i.e., Cas12a (Cpfl)). Similar to Cas9, Cas12a (Cpfl) is also a Class 2 CRISPR effector, but it is a member of the type V subgroup of enyzmes, rather than the type II subgroup. It has been shown that Cas12a (Cpfl) mediates robust DNA interference with features distinct from Cas9. Cas12a (Cpfl) is a single RNA-guided endonuclease lacking tracrRNA, and it utilizes a T-rich protospacer-adjacent motif (TTN, TTTN, or YTN). Moreover, Cpfl cleaves DNA via a staggered DNA double-stranded break. Out of 16 Cpfl-family proteins, two enzymes from *Acidaminococcus* and *Lachnospiraceae* are shown to have efficient genome-editing activity in human cells. Cpfl proteins are known in the art and have been described previously, for example Yamano et al., "Crystal structure of Cpfl in complex with guide RNA and target DNA." Cell (165) 2016, p. 949-962; the entire contents of which is hereby incorporated by reference.

In still other embodiments, the Cas protein may include any CRISPR associated protein, including but not limited to, Cas12a, Cas12b1, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2. Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof, and preferably comprising a nickase mutation (e.g., a mutation corresponding to the D10A mutation of the wild type Cas9 polypeptide of SEQ ID NO: 5).

In various other embodiments, the napDNAbp can be any of the following proteins: a Cas9, a C2c3Cas12a (Cpfl), a Cas12e (CasX), a Cas12d (CasY), a Cas12b1 (C2c1), a Cas13a (C2c2), a Cas12c (C2c3), a GeoCas9, a CjCas9, a Cas12a, a Cas12b, a Cas12g, a Cas12h, a Cas12i, a Cas13b, a Cas13c, a Cas13d, a Cas14, a Csn2, an xCas9, an SpCas9-NG, a circularly permuted Cas9, or an Argonaute (Ago) domain, or a variant thereof.

Exemplary Cas9 equivalent protein sequences can include the following:

| Description | Sequence |
|---|---|
| AsCas12a (previously known as Cpfl) | MTQFEGFTNLYQVSKTLRFELIPQGKTLKHIQEQGFIEEDKARNDHYKELKPIID RIYKTYADQCLQLVQLDWENLSAAIDSYRKEKTEETRNALIEEQATYRNAIHD YFIGRTDNLTDAINKRHAEIYKGLFKAELFNGKVLKQLGTVTTTEHENALLRSF DKFTTYFSGFYENRKNVFSAEDISTAIPHRIVQDNFPKFKENCHIFTRLITAVPSL REHFENVKKAIGIFVSTSIEEVFSFPFYNQLLTQTQIDLYNQLLGGISREAGTEKI KGLNEVLNLAIQKNDETAHIIASLPHRFIPLFKQILSDRNTLSFILEEFKSDEEVIQ SFCKYKTLLRNENVLETAEALFNELNSIDLTHIFISHKKLETISSALCDHWDTLR NALYERRISELTGKITKSAKEKVQRSLKHEDINLQEIISAAGKELSEAFKQKTSEI LSHAHAALDQPLPTTLKKQEEKEILKSQLDSLLGLYHLLDWFAVDESNEVDPEF |
| *Acidaminoco ccus sp. (strain BV3L6)* | |
| UniProtKB U2UMQ6 | SARLTGIKLEMEPSLSFYNKARNYATKKPYSVEKFKLNFQMPTLASGWDVNKE KNNGAILFVKNGLYYLGIMPKQKGRYKALSFEPTEKTSEGFDKMYYDYFPDAA KMIPKCSTQLKAVTAHFQTHTTPILLSNNFIEPLEITKEIYDLNNPEKEPKKFQTA YAKKTGDQKGYREALCKWIDFTRDFLSKYTKTTSIDLSSLRPSSQYKDLGEYY AELNPLLYHISFQRIAEKEIMDAVETGKLYLFQIYNKDFAKGHHGKPNLHTLYW TGLFSPENLAKTSIKLNGQAELFYRPKSRMKRMAHRLGEKMLNKKLKDQKTPI PDTLYQELYDYVNHRLSHDLSDEARALLPNVITKEVSHEIIKDRRFTSDKFFFHV PITLNYQAANSPSKFNQRVNAYLKEHPETPIIGIDRGERNLIYITVIDSTGKILEQR SLNTIQQFDYQKKLDNREKERVAARQAWSVVGTIKDLKQGYLSQVIHEIVDLM IHYQAVVVLENLNFGFKSKRTGIAEKAVYQQFEKMLIDKLNCLVLKDYPAEKV GGVLNPYQLTDQFTSFAKMGTQSGFLFYVPAPYTSKIDPLTGFVDPFVWKTIKN HESRKHFLEGFDFLHYDVKTGDFILHFKMNRNLSFQRGLPGFMPAWDIVFEKN ETQFDAKGTPFIAGKRIVPVIENHRFTGRYRDLYPANELIALLEEKGIVFRDGSNI LPKLLENDDSHAIDTMVALIRSVLQMRNSNAATGEDYINSPVRDLNGVCFDSRF QNPEWPMDADANGAYHIALKGQLLLNHLKESKDLKLQNGISNQDWLAYIQEL RN (SEQ ID NO: 51) |
| AsCas12a nickase (e.g., R1226A) | MTQFEGFTNLYQVSKTLRFELIPQGKTLKHIQEQGFIEEDKARNDHYKELKPIID RIYKTYADQCLQLVQLDWENLSAAIDSYRKEKTEETRNALIEEQATYRNAIHD YFIGRTDNLTDAINKRHAEIYKGLFKAELFNGKVLKQLGTVTTTEHENALLRSF DKFTTYFSGFYENRKNVFSAEDISTAIPHRIVQDNFPKFKENCHIFTRLITAVPSL REHFENVKKAIGIFVSTSIEEVFSFPFYNQLLTQTQIDLYNQLLGGISREAGTEKI KGLNEVLNLAIQKNDETAHIIASLPHRFIPLFKQILSDRNTLSFILEEFKSDEEVIQ SFCKYKTLLRNENVLETAEALFNELNSIDLTHIFISHKKLETISSALCDHWDTLR NALYERRISELTGKITKSAKEKVQRSLKHEDINLQEIISAAGKELSEAFKQKTSEI LSHAHAALDQPLPTTLKKQEEKEILKSQLDSLLGLYHLLDWFAVDESNEVDPEF SARLTGIKLEMEPSLSFYNKARNYATKKPYSVEKFKLNFQMPTLASGWDVNKE KNNGAILFVKNGLYYLGIMPKQKGRYKALSFEPTEKTSEGFDKMYYDYFPDAA KMIPKCSTQLKAVTAHFQTHTTPILLSNNFIEPLEITKEIYDLNNPEKEPKKFQTA YAKKTGDQKGYREALCKWIDFTRDFLSKYTKTTSIDLSSLRPSSQYKDLGEYY AELNPLLYHISFQRIAEKEIMDAVETGKLYLFQIYNKDFAKGHHGKPNLHTLYW TGLFSPENLAKTSIKLNGQAELFYRPKSRMKRMAHRLGEKMLNKKLKDQKTPI PDTLYQELYDYVNHRLSHDLSDEARALLPNVITKEVSHEIIKDRRFTSDKFFFHV PITLNYQAANSPSKFNQRVNAYLKEHPETPIIGIDRGERNLIYITVIDSTGKILEQR SLNTIQQFDYQKKLDNREKERVAARQAWSVVGTIKDLKQGYLSQVIHEIVDLM IHYQAVVVLENLNFGFKSKRTGIAEKAVYQQFEKMLIDKLNCLVLKDYPAEKV GGVLNPYQLTDQFTSFAKMGTQSGFLFYVPAPYTSKIDPLTGFVDPFVWKTIKN |
| | HESRKHFLEGFDFLHYDVKTGDFILHFKMNRNLSFQRGLPGFMPAWDIVFEKN ETQFDAKGTPFIAGKRIVPVIENHRFTGRYRDLYPANELIALLEEKGIVFRDGSNI LPKLLENDDSHAIDTMVALIRSVLQMANSNAATGEDYINSPVRDLNGVCFDSRF QNPEWPMDADANGAYHIALKGQLLLNHLKESKDLKLQNGISNQDWLAYIQEL RN (SEQ ID NO: 52) |
| LbCas12a (previously known as Cpfl) | MNYKTGLEDFIGKESLSKTLRNALIPTESTKIHMEEMGVIRDDELRAEKQQELK EIMDDYYRTFIEEKLGQIQGIQWNSLFQKMEETMEDISVRKDLDKIQNEKRKEI CCYFTSDKRFKDLFNAKLITDILPNFIKDNKEYTEEEKAEKEQTRVLFQRFATAF TNYFNQRRNNFSEDNISTAISFRIVNENSEIHLQNMRAFQRIEQQYPEEVCGMEE EYKDMLQEWQMKHIYSVDFYDRELTQPGIEYYNGICGKINEHMNQFCQKNRIN KNDFRMKKLHKQILCKKSSYYEIPFRFESDQEVYDALNEFIKTMKKKEIIRRCV HLGQECDDYDLGKIYISSNKYEQISNALYGSWDTIRKCIKEEYMDALPGKGEKK EEKAEAAAKKEEYRSIADIDKIISLYGSEMDRTISAKKCITEICDMAGQISIDPLV CNSDIKLLQNKEKTTEIKTILDSFLHVYQWGQTFIVSDIIEKDSYFYSELEDVLED FEGITTLYNHVRSYVTQKPYSTVKFKLHFGSPTLANGWSQSKEYDNNAILLMR DQKFYLGIFNVRNKPDKQIIKGHEKEEKGDYKKMIYNLLPGPSKMLPKVFITSR SGQETYKPSKHILDGYNEKRHIKSSPKFDLGYCWDLIDYYKECIHKHPDWKNY DFHFSDTKDYEDISGFYREVEMQGYQIKWTYISADEIQKLDEKGQIFLFQIYNK DFSVHSTGKDNLHTMYLKNLFSEENLKDIVLKLNGEAELFFRKASIKTPIVHKK GSVLVNRSYTQTVGNKEIRVSIPEEYYTEIYNYLNHIGKGKLSSEAQRYLDEGKI KSFTATKDIVKNYRYCCDHYFLHLPITINFKAKSDVAVNERTLAYIAKKEDIHII GIDRGERNLLYISVVDVHGNIREQRSFNIVNGYDYQQKLKDREKSRDAARKNW EEIEKIKELKEGYLSMVIHYIAQLVVKYNAVVAMEDLNYGFKTGRFKVERQVY QKFETMLIEKLHYLVFKDREVCEEGGVLRGYQLTYIPESLKKVGKQCGFIFYVP AGYTSKIDPTTGFVNLFSFKNLTNRESRQDFVGKFDEIRYDRDKKMFEFSFDYN NYIKKGTILASTKWKVYTNGTRLKRIVVNGKYTSQSMEVELTDAMEKMLQRA GIEYHDGKDLKGQIVEKGIEAEIIDIFRLTVQMRNSRSESEDREYDRLISPVLNDK GEFFDTATADKTLPQDADANGAYCIALKGLYEVKQIKENWKENEQFPRNKLV QDNKTWFDFMQKKRYL (SEQ ID NO: 53) |
| *Lachnospira ceae bacterium GAM79* | |
| Ref Seq. WP_119623 382.1 | |
| PcCas12a - previously known at Cpfl | MAKNFEDFKRLYSLSKTLRFEAKPIGATLDNIVKSGLLDEDEHRAASYVKVKK LIDEYHKVFIDRVLDDGCLPLENKGNNNSLAEYYESYVSRAQDEDAKKKFKEI QQNLRSVIAKKLTEDKAYANLFGNKLIESYKDKEDKKKIIDSDLIQFINTAESTQ LDSMSQDEAKELVKEFWGFVTYFYGFFDNRKNMYTAEEKSTGIAYRLVNENL PKFIDNIEAFNRAITRPEIQENMGVLYSDFSEYLNVESIQEMFQLDYYNMLLTQK |
| *Prevotella copri* | QIDVYNAIIGGKTDDEHDVKIKGINEYINLYNQQHKDDKLPKLKALFKQILSDR NAISWLPEEFNSDQEVLNAIKDCYERLAENVLGDKVLKSLLGSLADYSLDGIFIR NDLQLTDISQKMFGNWGVIQNAIMQNIKRVAPARKHKESEEDYEKRIAGIFKK ADSFSISYINDCLNEADPNNAYFVENYFATFGAVNTPTMQRENLFALVQNAYT EVAALLHSDYPTVKHLAQDKANVSKIKALLDAIKSLQHFVKPLLGKGDESDKD ERFYGELASLWAELDTVTPLYNMIRNYMTRKPYSQKKIKLNFENPQLLGGWD ANKEKDYATIILRRNGLYYLAIMDKDSRKLLGKAMPSDGECYEKMVYKFFKD VTTMIPKCSTQLKDVQAYFKVNTDDYVLNSKAFNKPLTITKEVFDLNNVLYGK YKKFQKGYLTATGDNVGYTHAVNVWIKFCMDFLNSYDSTCIYDFSSLKPESYL SLDAFYQDANLLLYKLSFARASVSYINQLVEEGKMYLFQIYNKDFSEYSKGTPN MHTLYWKALFDERNLADVVYKLNGQAEMFYRKKSIENTHPTHPANHPILNKN KDNKKKESLFDYDLIKDRRYTVDKFMFHVPITMNFKSVGSENINQDVKAYLRH ADDMHIIGIDRGERHLLYLVVIDLQGNIKEQYSLNEIVNEYNGNTYHTNYHDLL DVREEERLKARQSWQTIENIKELKEGYLSQVIHKITQLMVRYHAIVVLEDLSKG FMRSRQKVEKQVYQKFEKMLIDKLNYLVDKKTDVSTPGGLLNAYQLTCKSDS SQKLGKQSGFLFYIPAWNTSKIDPVTGFVNLLDTHSLNSKEKIKAFFSKFDAIRY NKDKKWFEFNLDYDKFGKKAEDTRTKWTLCTRGMRIDTFRNKEKNSQWDNQ EVDLTTEMKSLLEHYYIDIHGNLKDAISAQTDKAFFTGLLHILKLTLQMRNSITG TETDYLVSPVADENGIFYDSRSCGNQLPENADANGAYNIARKGLMLIEQIKNAE DLNNVKFDISNKAWLNFAQQKPYKNG (SEQ ID NO: 54) |
| Ref Seq. WP_119227 726.1 | |
| ErCas12a - previously known at Cpfl | MFSAKLISDILPEFVIHNNNYSASEKEEKTQVIKLFSRFATSFKDYFKNRANCFS ANDISSSSCHRIVNDNAEIFFSNALVYRRIVKNLSNDDINKISGDMKDSLKEMSL EEIYSYEKYGEFITQEGISFYNDICGKVNLFMNLYCQKNKENKNLYKLRKLHKQ ILCIADTSYEVPYKFESDEEVYQSVNGFLDNISSKHIVERLRKIGENYNGYNLDK IYIVSKFYESVSQKTYRDWETINTALEIHYNNILPGNGKSKADKVKKAVKNDLQ KSITEINELVSNYKLCPDDNIKAETYIHEISHILNNFEAQELKYNPEIHLVESELKA SELKNVLDVIMNAFHWCSVFMTEELVDKDNNFYAELEEIYDEIYPVISLYNLVR NYVTQKPYSTKKIKLNFGIPTLADGWSKSKEYSNNAIILMRDNLYYLGIFNAKN KPDKKIIEGNTSENKGDYKKMIYNLLPGPNKMIPKVFLSSKTGVETYKPSAYILE GYKQNKHLKSSKDFDITFCHDLIDYFKNCIAIHPEWKNFGFDFSDTSTYEDISGF YREVELQGYKIDWTYISEKDIDLLQEKGQLYLFQIYNKDFSKKSSGNDNLHTM YLKNLFSEENLKDIVLKLNGEAEIFFRKSSIKNPIIHKKGSILVNRTYEAEEKDQF GNIQIVRKTIPENIYQELYKYFNDKSDKELSDEAAKLKNVVGHHEAATNIVKDY RYTYDKYFLHMPITINFKANKTSFINDRILQYIAKEKDLHVIGIDRGERNLIYVSV IDTCGNIVEQKSFNIVNGYDYQIKLKQQEGARQIARKEWKEIGKIKEIKEGYLSL |
| *Eubacterium rectale* | |
| Ref Seq. WP_119223 642.1 | |
| | VIHEISKMVIKYNAIIAMEDLSYGFKKGRFKVERQVYQKFETMLINKLNYLVFK DISITENGGLLKGYQLTYIPDKLKNVGHQCGCIFYVPAAYTSKIDPTTGFVNIFK FKDLTVDAKREFIKKFDSIRYDSDKNLFCFTFDYNNFITQNTVMSKSSWSVYTY GVRIKRRFVNGRFSNESDTIDITKDMEKTLEMTDINWRDGHDLRQDIIDYEIVQ HIFEIFKLTVQMRNSLSELEDRDYDRLISPVLNENNIFYDSAKAGDALPKDADA NGAYCIALKGLYEIKQITENWKEDGKFSRDKLKISNKDWFDFIQNKRYL(SEQ ID NO: 55) |
| CsCas12a - previously known at Cpfl | MNYKTGLEDFIGKESLSKTLRNALIPTESTKIHMEEMGVIRDDELRAEKQQELK EIMDDYYRAFIEEKLGQIQGIQWNSLFQKMEETMEDISVRKDLDKIQNEKRKEI CCYFTSDKRFKDLFNAKLITDILPNFIKDNKEYTEEEKAEKEQTRVLFQRFATAF TNYFNQRRNNFSEDNISTAISFRIVNENSEIHLQNMRAFQRIEQQYPEEVCGMEE EYKDMLQEWQMKHIYLVDFYDRVLTQPGIEYYNGICGKINEHMNQFCQKNRI NKNDFRMKKLHKQILCKKSSYYEIPFRFESDQEVYDALNEFIKTMKEKEIICRCV HLGQKCDDYDLGKIYISSNKYEQISNALYGSWDTIRKCIKEEYMDALPGKGEK KEEKAEAAAKKEEYRSIADIDKIISLYGSEMDRTISAKKCITEICDMAGQISTDPL VCNSDIKLLQNKEKTTEIKTILDSFLHVYQWGQTFIVSDIIEKDSYFYSELEDVLE DFEGITTLYNHVRSYVTQKPYSTVKFKLHFGSPTLANGWSQSKEYDNNAILLM RDQKFYLGIFNVRNKPDKQIIKGHEKEEKGDYKKMIYNLLPGPSKMLPKVFITS RSGQETYKPSKHILDGYNEKRHIKSSPKFDLGYCWDLIDYYKECIHKHPDWKN YDFHFSDTKDYEDISGFYREVEMQGYQIKWTYISADEIQKLDEKGQIFLFQIYN KDFSVHSTGKDNLHTMYLKNLFSEENLKDIVLKLNGEAELFFRKASIKTPVVHK KGSVLVNRSYTQTVGDKEIRVSIPEEYYTEIYNYLNHIGRGKLSTEAQRYLEER KIKSFTATKDIVKNYRYCCDHYFLHLPITINFKAKSDIAVNERTLAYIAKKEDIHI IGIDRGERNLLYISVVDVHGNIREQRSFNIVNGYDYQQKLKDREKSRDAARKN WEEIEKIKELKEGYLSMVIHYIAQLVVKYNAVVAMEDLNYGFKTGRFKVERQV YQKFETMLIEKLHYLVFKDREVCEEGGVLRGYQLTYIPESLKKVGKQCGFIFYV PAGYTSKIDPTTGFVNLFSFKNLTNRESRQDFVGKFDEIRYDRDKKMFEFSFDY NNYIKKGTMLASTKWKVYTNGTRLKRIVVNGKYTSQSMEVELTDAMEKMLQ RAGIEYHDGKDLKGQIVEKGIEAEIIDIFRLTVQMRNSRSESEDREYDRLISPVLN DKGEFFDTATADKTLPQDADANGAYCIALKGLYEVKQIKENWKENEQFPRNK LVQDNKTWFDFMQKKRYL (SEQ ID NO: 56) |
| *Clostridium sp. AF34-10BH* | |
| Ref Seq. WP_118538 418.1 | |
| BhCas12b | MATRSFILKIEPNEEVKKGLWKTHEVLNHGIAYYMNILKLIRQEAIYEHHEQDP KNPKKVSKAEIQAELWDFVLKMQKCNSFTHEVDKDEVFNILRELYEELVPSSV EKKGEANQLSNKFLYPLVDPNSQSGKGTASSGRKPRWYNLKIAGDPSWEEEKK KWEEDKKKDPLAKILGKLAEYGLIPLFIPYTDSNEPIVKEIKWMEKSRNQSVRR LDKDMFIQALERFLSWESWNLKVKEEYEKVEKEYKTLEERIKEDIQALKALEQ |
| *Bacillus hisashii* | |
| Ref Seq. WP_095142 515.1 | YEKERQEQLLRDTLNTNEYRLSKRGLRGWREIIQKWLKMDENEPSEKYLEVFK DYQRKHPREAGDYSVYEFLSKKENHFIWRNHPEYPYLYATFCEIDKKKKDAKQ QATFTLADPINHPLWVRFEERSGSNLNKYRILTEQLHTEKLKKKLTVQLDRLIY PTESGGWEEKGKVDIVLLPSRQFYNQIFLDIEEKGKHAFTYKDESIKFPLKGTLG GARVQFDRDHLRRYPHKVESGNVGRIYFNMTVNIEPTESPVSKSLKIHRDDFPK VVNFKPKELTEWIKDSKGKKLKSGIESLEIGLRVMSIDLGQRQAAAASIFEVVD QKPDIEGKLFFPIKGTELYAVHRASFNIKLPGETLVKSREVLRKAREDNLKLMN QKLNFLRNVLHFQQFEDITEREKRVTKWISRQENSDVPLVYQDELIQIRELMYK PYKDWVAFLKQLHKRLEVEIGKEVKHWRKSLSDGRKGLYGISLKNIDEIDRTR KFLLRWSLRPTEPGEVRRLEPGQRFAIDQLNHLNALKEDRLKKMANTIIMHAL GYCYDVRKKKWQAKNPACQIILFEDLSNYNPYEERSRFENSKLMKWSRREIPR QVALQGEIYGLQVGEVGAQFSSRFHAKTGSPGIRCSVVTKEKLQDNRFFKNLQ REGRLTLDKIAVLKEGDLYPDKGGEKFISLSKDRKCVTTHADINAAQNLQKRF WTRTHGFYKVYCKAYQVDGQTVYIPESKDQKQKIIEEFGEGYFILKDGVYEWV NAGKLKIKKGSSKQSSSELVDSDILKDSFDLASELKGEKLMLYRDPSGNVFPSD KWMAAGVFFGKLERILISKLTNQYSISTIEDDSSKQSM (SEQ ID NO: 57) |
| ThCas12b | MSEKTTQRAYTLRLNRASGECAVCQNNSCDCWHDALWATHKAVNRGAKAFG DWLLTLRGGLCHTLVEMEVPAKGNNPPQRPTDQERRDRRVLLALSWLSVEDE HGAPKEFIVATGRDSADDRAKKVEEKLREILEKRDFQEHEIDAWLQDCGPSLK AHIREDAVWVNRRALFDAAVERIKTLTWEEAWDFLEPFFGTQYFAGIGDGKDK DDAEGPARQGEKAKDLVQKAGQWLSARFGIGTGADFMSMAEAYEKIAKWAS QAQNGDNGKATIEKLACALRPSEPPTLDTVLKCISGPGHKSATREYLKTLDKKS TVTQEDLNQLRKLADEDARNCRKKVGKKGKKPWADEVLKDVENSCELTYLQ DNSPARHREFSVMLDHAARRVSMAHSWIKKAEQRRRQFESDAQKLKNLQERA PSAVEWLDRFCESRSMTTGANTGSGYRIRKRAIEGWSYVVQAWAEASCDTED KRIAAARKVQADPEIEKFGDIQLFEALAADEAICVWRDQEGTQNPSILIDYVTG KTAEHNQKRFKVPAYRHPDELRHPVFCDFGNSRWSIQFAIHKEIRDRDKGAKQ DTRQLQNRHGLKMRLWNGRSMTDVNLHWSSKRLTADLALDQNPNPNPTEVT RADRLGRAASSAFDHVKIKNVFNEKEWNGRLQAPRAELDRIAKLEEQGKTEQA EKLRKRLRWYVSFSPCLSPSGPFIVYAGQHNIQPKRSGQYAPHAQANKGRARL AQLILSRLPDLRILSVDLGHRFAAACAVWETLSSDAFRREIQGLNVLAGGSGEG DLFLHVEMTGDDGKRRTVVYRRIGPDQLLDNTPHPAPWARLDRQFLIKLQGED EGVREASNEELWTVHKLEVEVGRTVPLIDRMVRSGFGKTEKQKERLKKLRELG WISAMPNEPSAETDEKEGEIRSISRSVDELMSSALGTLRLALKRHGNRARIAFA MTADYKPMPGGQKYYFHEAKEASKNDDETKRRDNQIEFLQDALSLWHDLFSS PDWEDNEAKKLWQNHIATLPNYQTPEEISAELKRVERNKKRKENRDKLRTAA |
| *Thermomona* s *hydrotherma lis* | |
| Ref Seq. WP_072754 838 | |
| | KALAENDQLRQHLHDTWKERWESDDQQWKERLRSLKDWIFPRGKAEDNPSIR HVGGLSITRINTISGLYQILKAFKMRPEPDDLRKNIPQKGDDELENFNRRLLEAR DRLREQRVKQLASRIIEAALGVGRIKIPKNGKLPKRPRTTVDTPCHAVVIESLKT YRPDDLRTRRENRQLMQWSSAKVRKYLKEGCELYGLHFLEVPANYTSRQCSR TGLPGIRCDDVPTGDFLKAPWWRRAINTAREKNGGDAKDRFLVDLYDHLNNL QSKGEALPATVRVPRQGGNLFIAGAQLDDTNKERRAIQADLNAAANIGLRALL DPDWRGRWWYVPCKDGTSEPALDRIEGSTAFNDVRSLPTGDNSSRRAPREIEN LWRDPSGDSLESGTWSPTRAYWDTVQSRVIELLRRHAGLPTS (SEQ ID NO: 58) |
| LsCas 12b | MSIRSFKLKLKTKSGVNAEQLRRGLWRTHQLINDGIAYYMNWLVLLRQEDLFI RNKETNEIEKRSKEEIQAVLLERVHKQQQRNQWSGEVDEQTLLQALRQLYEEI VPSVIGKSGNASLKARFFLGPLVDPNNKTTKDVSKSGPTPKWKKMKDAGDPN WVQEYEKYMAERQTLVRLEEMGLIPLFPMYTDEVGDIHWLPQASGYTRTWDR DMFQQAIERLLSWESWNRRVRERRAQFEKKTHDFASRFSESDVQWMNKLREY EAQQEKSLEENAFAPNEPYALTKKALRGWERVYHSWMRLDSAASEEAYWQE VATCQTAMRGEFGDPAIYQFLAQKENHDIWRGYPERVIDFAELNHLQRELRRA KEDATFTLPDSVDHPLWVRYEAPGGTNIHGYDLVQDTKRNLTLILDKFILPDEN GSWHEVKKVPFSLAKSKQFHRQVWLQEEQKQKKREVVFYDYSTNLPHLGTLA GAKLQWDRNFLNKRTQQQIEETGEIGKVFFNISVDVRPAVEVKNGRLQNGLGK ALTVLTHPDGTKIVTGWKAEQLEKWVGESGRVSSLGLDSLSEGLRVMSIDLGQ RTSATVSVFEITKEAPDNPYKFFYQLEGTEMFAVHQRSFLLALPGENPPQKIKQ MREIRWKERNRIKQQVDQLSAILRLHKKVNEDERIQAIDKLLQKVASWQLNEEI ATAWNQALSQLYSKAKENDLQWNQAIKNAHHQLEPVVGKQISLWRKDLSTG RQGIAGLSLWSIEELEATKKLLTRWSKRSREPGVVKRIERFETFAKQIQHHINQV KENRLKQLANLIVMTALGYKYDQEQKKWIEVYPACQVVLFENLRSYRFSFERS RRENKKLMEWSHRSIPKLVQMQGELFGLQVADVYAAYSSRYHGRTGAPGIRC HALTEADLRNETNIIHELIEAGFIKEEHRPYLQQGDLVPWSGGELFATLQKPYD NPRILTLHADINAAQNIQKRFWHPSMWFRVNCESVMEGEIVTYVPKNKTVHKK QGKTFRFVKVEGSDVYEWAKWSKNRNKNTFSSITERKPPSSMILFRDPSGTFFK EQEWVEQKTFWGKVQSMIQAYMKKTIVQRMEE (SEQ ID NO: 59) |
| *Laceyella sacchari* | |
| WP_132221 894.1 | |
| DtCas12b | MVLGRKDDTAELRRALWTTHEHVNLAVAEVERVLLRCRGRSYWTLDRRGDP VHVPESQVAEDALAMAREAQRRNGWPVVGEDEEILLALRYLYEQIVPSCLLDD LGKPLKGDAQKIGTNYAGPLFDSDTCRRDEGKDVACCGPFHEVAGKYLGALPE WATPISKQEFDGKDASHLRFKATGGDDAFFRVSIEKANAWYEDPANQDALKN KAYNKDDWKKEKDKGISSWAVKYIQKQLQLGQDPRTEVRRKLWLELGLLPLF IPVFDKTMVGNLWNRLAVRLALAHLLSWESWNHRAVQDQALARAKRDELAA LFLGMEDGFAGLREYELRRNESIKQHAFEPVDRPYVVSGRALRSWTRVREEWL RHGDTQESRKNICNRLQDRLRGKFGDPDVFHWLAEDGQEALWKERDCVTSFS LLNDADGLLEKRKGYALMTFADARLHPRWAMYEAPGGSNLRTYQIRKTENGL WADVVLLSPRNESAAVEEKTFNVRLAPSGQLSNVSFDQIQKGSKMVGRCRYQS ANQQFEGLLGGAEILFDRKRIANEQHGATDLASKPGHVWFKLTLDVRPQAPQG WLDGKGRPALPPEAKHFKTALSNKSKFADQVRPGLRVLSVDLGVRSFAACSVF ELVRGGPDQGTYFPAADGRTVDDPEKLWAKHERSFKITLPGENPSRKEEIARRA AMEELRSLNGDIRRLKAILRLSVLQEDDPRTEHLRLFMEAIVDDPAKSALNAEL FKGFGDDRFRSTPDLWKQHCHFFHDKAEKVVAERFSRWRTETRPKSSSWQDW RERRGYAGGKSYWAVTYLEAVRGLILRWNMRGRTYGEVNRQDKKQFGTVAS ALLHHINQLKEDRIKTGADMIIQAARGFVPRKNGAGWVQVHEPCRLILFEDLAR YRFRTDRSRRENSRLMRWSHREIVNEVGMQGELYGLHVDTTEAGFSSRYLASS GAPGVRCRHLVEEDFHDGLPGMHLVGELDWLLPKDKDRTANEARRLLGGMV RPGMLVPWDGGELFATLNAASQLHVIHADINAAQNLQRRFWGRCGEAIRIVCN QLSVDGSTRYEMAKAPKARLLGALQQLKNGDAPFHLTSIPNSQKPENSYVMTP TNAGKKYRAGPGEKSSGEEDELALDIVEQAEELAQGRKTFFRDPSGVFFAPDR WLPSEIYWSRIRRRIWQVTLERNSSGRQERAEMDEMPY (SEQ ID NO: 60) |
| *Dsulfonatron um thiodismutan* s | |
| *WP_031386 437* | |

The base editors described herein may also comprise Cas12a (Cpfl) (dCpf1) variants that may be used as a guide nucleotide sequence-programmable DNA-binding protein domain. The Cas12a (Cpfl) protein has a RuvC-like endonuclease domain that is similar to the RuvC domain of Cas9 but does not have a HNH endonuclease domain, and the N-terminal of Cas12a (Cpfl) does not have the alfa-helical recognition lobe of Cas9. It was shown in Zetsche et al., Cell, 163, 759-771, 2015 (which is incorporated herein by reference) that, the RuvC-like domain of Cas12a (Cpfl) is responsible for cleaving both DNA strands and inactivation of the RuvC-like domain inactivates Cas12a (Cpfl) nuclease activity.

### (8) Cas9 equivalents with expanded PAM sequence

In some embodiments, the napDNAbp is a nucleic acid programmable DNA binding protein that does not require a canonical (NGG) PAM sequence. In some embodiments, the napDNAbp is an argonaute protein. One example of such a nucleic acid programmable DNA binding protein is an Argonaute protein from *Natronobacterium gregoryi* (NgAgo). NgAgo is a ssDNA-guided endonuclease. NgAgo binds 5' phosphorylated ssDNA of ~24 nucleotides (gDNA) to guide it to its target site and will make DNA double-strand breaks at the gDNA site. In contrast to Cas9, the NgAgo-gDNA system does not require a protospacer-adjacent motif (PAM). Using a nuclease inactive NgAgo (dNgAgo) can greatly expand the bases that may be targeted. The characterization and use of NgAgo have been described in Gao et al., Nat Biotechnol., 2016 Jul;34(7):768-73. PubMed PMID: 27136078; Swarts et al., Nature. 507(7491) (2014):258-61; and Swarts et al., Nucleic Acids Res. 43(10) (2015):5120-9, each of which is incorporated herein by reference.

In some embodiments, the napDNAbp is a prokaryotic homolog of an Argonaute protein. Prokaryotic homologs of Argonaute proteins are known and have been described, for example, in Makarova K., et al., "Prokaryotic homologs of Argonaute proteins are predicted to function as key components of a novel system of defense against mobile genetic elements", Biol Direct. 2009 Aug 25;4:29. doi: 10.1186/1745-6150-4-29, the entire contents of which is hereby incorporated by reference. In some embodiments, the napDNAbp is a Marinitoga piezophila Argunaute (MpAgo) protein. The CRISPR-associated Marinitoga piezophila Argunaute (MpAgo) protein cleaves single-stranded target sequences using 5'-phosphorylated guides. The 5' guides are used by all known Argonautes. The crystal structure of an MpAgo-RNA complex shows a guide strand binding site comprising residues that block 5' phosphate interactions. This data suggests the evolution of an Argonaute subclass with noncanonical specificity for a 5'-hydroxylated guide. See, e.g., Kaya et al., "A bacterial Argonaute with noncanonical guide RNA specificity", Proc Natl Acad Sci U S A. 2016 Apr 12;113(15):4057-62, the entire contents of which are hereby incorporated by reference). It should be appreciated that other argonaute proteins may be used, and are within the scope of this disclosure.

In some embodiments, the napDNAbp is a single effector of a microbial CRISPR-Cas system. Single effectors of microbial CRISPR-Cas systems include, without limitation, Cas9, Cas12a (Cpfl), Cas12b1 (C2c1), Cas13a (C2c2), and Cas12c (C2c3). Typically, microbial CRISPR-Cas systems are divided into Class 1 and Class 2 systems. Class 1 systems have multi-subunit effector complexes, while Class 2 systems have a single protein effector. For example, Cas9 and Cas12a (Cpfl) are Class 2 effectors. In addition to Cas9 and Cas12a (Cpfl), three distinct Class 2 CRISPR-Cas systems (Cas12b1, Cas13a, and Cas12c) have been described by Shmakov et al., "Discovery and Functional Characterization of Diverse Class 2 CRISPR Cas Systems", Mol. Cell, 2015 Nov 5; 60(3): 385-397, the entire contents of which is hereby incorporated by reference.

Effectors of two of the systems, Cas12b1 and Cas12c, contain RuvC-like endonuclease domains related to Cas12a. A third system, Cas13a contains an effector with two predicted HEPN RNase domains. Production of mature CRISPR RNA is tracrRNA-independent, unlike production of CRISPR RNA by Cas12b1. Cas12b1 depends on both CRISPR RNA and tracrRNA for DNA cleavage. Bacterial Cas13a has been shown to possess a unique RNase activity for CRISPR RNA maturation distinct from its RNA-activated single-stranded RNA degradation activity. These RNase functions are different from each other and from the CRISPR RNA-processing behavior of Cas12a. See, e.g., East-Seletsky, et al., "Two distinct RNase activities of CRISPR-C2c2 enable guide-RNA processing and RNA detection", Nature, 2016 Oct 13;538(7624):270-273, the entire contents of which are hereby incorporated by reference. *In vitro* biochemical analysis of Cas13a in *Leptotrichia shahii* has shown that Cas13a is guided by a single CRISPR RNA and can be programed to cleave ssRNA targets carrying complementary protospacers. Catalytic residues in the two conserved HEPN domains mediate cleavage. Mutations in the catalytic residues generate catalytically inactive RNA-binding proteins. See e.g., Abudayyeh et al., "C2c2 is a single-component programmable RNA-guided RNA-targeting CRISPR effector", Science, 2016 Aug 5; 353(6299), the entire contents of which are hereby incorporated by reference.

The crystal structure of *Alicyclobaccillus acidoterrastris* Cas12b1 (AacC2c1) has been reported in complex with a chimeric single-molecule guide RNA (sgRNA). See e.g., Liu et al., "C2c1-sgRNA Complex Structure Reveals RNA-Guided DNA Cleavage Mechanism", Mol. Cell, 2017 Jan 19;65(2):310-322, the entire contents of which are hereby incorporated by reference. The crystal structure has also been reported in *Alicyclobacillus acidoterrestris* C2c1 bound to target DNAs as ternary complexes. See e.g., Yang et al., "PAM-dependent Target DNA Recognition and Cleavage by C2C1 CRISPR-Cas endonuclease", Cell, 2016 Dec 15;167(7):1814-1828, the entire contents of which are hereby incorporated by reference. Catalytically competent conformations of AacC2c1, both with target and non-target DNA strands, have been captured independently positioned within a single RuvC catalytic pocket, with C2c1-mediated cleavage resulting in a staggered seven-nucleotide break of target DNA. Structural comparisons between C2c1 ternary complexes and previously identified Cas9 and Cpfl counterparts demonstrate the diversity of mechanisms used by CRISPR-Cas9 systems.

In some embodiments, the napDNAbp may be a Cas12b1, a Cas13a, or a Cas12c protein. In some embodiments, the napDNAbp is a Cas12b1 protein. In some embodiments, the napDNAbp is a Cas13a protein. In some embodiments, the napDNAbp is a Cas12c protein. In some embodiments, the napDNAbp comprises an amino acid sequence that is at least 85%, at least 90%, at least 91%, at least 92%, at least 93%, at least 94%, at least 95%, at least 96%, at least 97%, at least 98%, at least 99%, or at least 99.5% identical to a naturally-occurring Cas12b1 (C2c1), Cas13a (C2c2), or Cas12c (C2c3) protein. In some embodiments, the napDNAbp is a naturally-occurring Cas12b1 (C2c1), Cas13a (C2c2), or Cas12c (C2c3) protein.

Some aspects of the disclosure provide Cas9 domains that have different PAM specificities. Typically, Cas9 proteins, such as Cas9 from *S. pyogenes* (spCas9), require a canonical NGG PAM sequence to bind a particular nucleic acid region. This may limit the ability to edit desired bases within a genome. In some embodiments, the base editing fusion proteins provided herein may need to be placed at a precise location, for example where a target base is placed within a 4 base region (*e.g.,* a "editing window"), which is approximately 15 bases upstream of the PAM. See Komor, A.C., et al., "Programmable editing of a target base in genomic DNA without double-stranded DNA cleavage" Nature 533, 420-424 (2016), the entire contents of which are hereby incorporated by reference. Accordingly, in some embodiments, any of the fusion proteins provided herein may contain a Cas9 domain that is capable of binding a nucleotide sequence that does not contain a canonical (*e.g*., NGG) PAM sequence. Cas9 domains that bind to non-canonical PAM sequences have been described in the art and would be apparent to the skilled artisan. For example, Cas9 domains that bind non-canonical PAM sequences have been described in Kleinstiver, B. P., et al., "Engineered CRISPR-Cas9 nucleases with altered PAM specificities" Nature 523, 481-485 (2015); and Kleinstiver, B. P., et al., "Broadening the targeting range of Staphylococcus aureus CRISPR-Cas9 by modifying PAM recognition" Nature Biotechnology 33, 1293-1298 (2015); the entire contents of each are hereby incorporated by reference.

For example, a napDNAbp domain with altered PAM specificity, such as a domain with at least 80%, at least 85%, at least 90%, at least 95%, or at least 99% sequence identity with wild type *Francisella novicida* Cpfl (SEQ ID NO: 61) (D917, E1006, and D1255), which has the following amino acid sequence:

| |
|---|
| MSIYQEFVNKYSLSKTLRFELIPQGKTLENIKARGLILDDEKRAKDYKKAKQIIDKYHQFFIE EILSSVCISEDLLQNYSDVYFKLKKSDDDNLQKDFKSAKDTIKKQISEYIKDSEKFKNLFNQ NLIDAKKGQESDLILWLKQSKDNGIELFKANSDITDIDEALEIIKSFKGWTTYFKGFHENRK NVYSSNDIPTSIIYRIVDDNLPKFLENKAKYESLKDKAPEAINYEQIKKDLAEELTFDIDYKT SEVNQRVFSLDEVFEIANFNNYLNQSGITKFNTIIGGKFVNGENTKRKGINEYINLYSQQIND KTLKKYKMSVLFKQILSDTESKSFVIDKLEDDSDVVTTMQSFYEQIAAFKTVEEKSIKETLS LLFDDLKAQKLDLSKIYFKNDKSLTDLSQQVFDDYSVIGTAVLEYITQQIAPKNLDNPSKKE QELIAKKTEKAKYLSLETIKLALEEFNKHRDIDKQCRFEEILANFAAIPMIFDEIAQNKDNLA QISIKYQNQGKKDLLQASAEDDVKAIKDLLDQTNNLLHKLKIFHISQSEDKANILDKDEHF YLVFEECYFELANIVPLYNKIRNYITQKPYSDEKFKLNFENSTLANGWDKNKEPDNTAILFI KDDKYYLGVMNKKNNKIFDDKAIKENKGEGYKKIVYKLLPGANKMLPKVFFSAKSIKFY NPSEDILRIRNHSTHTKNGSPQKGYEKFEFNIEDCRKFIDFYKQSISKHPEWKDFGFRFSDTQ RYNSIDEFYREVENQGYKLTFENISESYIDSVVNQGKLYLFQIYNKDFSAYSKGRPNLHTLY WKALFDERNLQDVVYKLNGEAELFYRKQSIPKKITHPAKEAIANKNKDNPKKESVFEYDLI KDKRFTEDKFFFHCPITINFKSSGANKFNDEINLLLKEKANDVHILSIDRGERHLAYYTLVD GKGNIIKQDTFNIIGNDRMKTNYHDKLAAIEKDRDSARKDWKKINNIKEMKEGYLSQVVH EIAKLVIEYNAIVVFEDLNFGFKRGRFKVEKQVYQKLEKMLIEKLNYLVFKDNEFDKTGGV LRAYQLTAPFETFKKMGKQTGIIYYVPAGFTSKICPVTGFVNQLYPKYESVSKSQEFFSKFD KICYNLDKGYFEFSFDYKNFGDKAAKGKWTIASFGSRLINFRNSDKNHNWDTREVYPTKE LEKLLKDYSIEYGHGECIKAAICGESDKKFFAKLTSVLNTILQMRNSKTGTELDYLISPVAD VNGNFFDSRQAPKNMPQDADANGAYHIGLKGLMLLGRIKNNQEGKKLNLVIKNEEYFEFV QNRNN (SEQ ID NO: 61) |

An additional napDNAbp domain with altered PAM specificity, such as a domain having at least 80%, at least 85%, at least 90%, at least 95%, or at least 99% sequence identity with wild type *Geobacillus thermodenitrificans* Cas9 (SEQ ID NO: 62), which has the following amino acid sequence:

| |
|---|
| MKYKIGLDIGITSIGWAVINLDIPRIEDLGVRIFDRAENPKTGESLALPRRLARSARRRLRRR KHRLERIRRLFVREGILTKEELNKLFEKKHEIDVWQLRVEALDRKLNNDELARILLHLAKR RGFRSNRKSERTNKENSTMLKHIEENQSILSSYRTVAEMVVKDPKFSLHKRNKEDNYTNTV ARDDLEREIKLIFAKQREYGNIVCTEAFEHEYISIWASQRPFASKDDIEKKVGFCTFEPKEKR APKATYTFQSFTVWEHINKLRLVSPGGIRALTDDERRLIYKQAFHKNKITFHDVRTLLNLPD DTRFKGLLYDRNTTLKENEKVRFLELGAYHKIRKAIDSVYGKGAAKSFRPIDFDTFGYALT MFKDDTDIRSYLRNEYEQNGKRMENLADKVYDEELIEELLNLSFSKFGHLSLKALRNILPY MEQGEVYSTACERAGYTFTGPKKKQKTVLLPNIPPIANPVVMRALTQARKVVNAIIKKYGS PVSIHIELARELSQSFDERRKMQKEQEGNRKKNETAIRQLVEYGLTLNPTGLDIVKFKLWSE QNGKCAYSLQPIEIERLLEPGYTEVDHVIPYSRSLDDSYTNKVLVLTKENREKGNRTPAEYL |
| GLGSERWQQFETFVLTNKQFSKKKRDRLLRLHYDENEENEFKNRNLNDTRYISRFLANFIR EHLKFADSDDKQKVYTVNGRITAHLRSRWNFNKNREESNLHHAVDAAIVACTTPSDIARVT AFYQRREQNKELSKKTDPQFPQPWPHFADELQARLSKNPKESIKALNLGNYDNEKLESLQP VFVSRMPKRSITGAAHQETLRRYIGIDERSGKIQTVVKKKLSEIQLDKTGHFPMYGKESDPR TYEAIRQRLLEHNNDPKKAFQEPLYKPKKNGELGPIIRTIKIIDTTNQVIPLNDGKTVAYNSNI VRVDVFEKDGKYYCVPIYTIDMMKGILPNKAIEPNKPYSEWKEMTEDYTFRFSLYPNDLIRI EFPREKTIKTAVGEEIKIKDLFAYYQTIDSSNGGLSLVSHDNNFSLRSIGSRTLKRFEKYQVDV LGNIYKVRGEKRVGVASSSHSKAGETIRPL (SEQ ID NO: 62) |

In some embodiments, the nucleic acid programmable DNA binding protein (napDNAbp) is a nucleic acid programmable DNA binding protein that does not require a canonical (NGG) PAM sequence. In some embodiments, the napDNAbp is an argonaute protein. One example of such a nucleic acid programmable DNA binding protein is an Argonaute protein from *Natronobacterium gregoryi* (NgAgo). NgAgo is a ssDNA-guided endonuclease. NgAgo binds 5' phosphorylated ssDNA of ~24 nucleotides (gDNA) to guide it to its target site and will make DNA double-strand breaks at the gDNA site. In contrast to Cas9, the NgAgo-gDNA system does not require a protospacer-adjacent motif (PAM). Using a nuclease inactive NgAgo (dNgAgo) can greatly expand the bases that may be targeted. The characterization and use of NgAgo have been described in Gao et al., Nat Biotechnol., 34(7): 768-73 (2016), PubMed PMID: 27136078; Swarts et al., Nature, 507(7491): 258-61 (2014); and Swarts et al., Nucleic Acids Res. 43(10) (2015): 5120-9, each of which is incorporated herein by reference. The sequence of *Natronobacterium gregoryi* Argonaute is provided in SEQ ID NO: 63.

The disclosed fusion proteins may comprise a napDNAbp domain having at least 80%, at least 85%, at least 90%, at least 95%, or at least 99% sequence identity with wild type *Natronobacterium gregoryi* Argonaute (SEQ ID NO: 63), which has the following amino acid sequence:

| |
|---|
| MTVIDLDSTTTADELTSGHTYDISVTLTGVYDNTDEQHPRMSLAFEQDNGERRYITLWKNT TPKDVFTYDYATGSTYIFTNIDYEVKDGYENLTATYQTTVENATAQEVGTTDEDETFAGGEP LDHHLDDALNETPDDAETESDSGHVMTSFASRDQLPEWTLHTYTLTATDGAKTDTEYARR TLAYTVRQELYTDHDAAPVATDGLMLLTPEPLGETPLDLDCGVRVEADETRTLDYTTAKDR LLARELVEEGLKRSLWDDYLVRGIDEVLSKEPVLTCDEFDLHERYDLSVEVGHSGRAYLHI NFRHRFVPKLTLADIDDDNIYPGLRVKTTYRPRRGHIVWGLRDECATDSLNTLGNQSVVAY HRNNQTPINTDLLDAIEAADRRVVETRRQGHGDDAVSFPQELLAVEPNTHQIKQFASDGFH QQARSKTRLSASRCSEKAQAFAERLDPVRLNGSTVEFSSEFFTGNNEQQLRLLYENGESVLT |
| FRDGARGAHPDETFSKGIVNPPESFEVAVVLPEQQADTCKAQWDTMADLLNQAGAPPTRS ETVQYDAFSSPESISLNVAGAIDPSEVDAAFVVLPPDQEGFADLASPTETYDELKKALANM GIYSQMAYFDRFRDAKIFYTRNVALGLLAAAGGVAFTTEHAMPGDADMFIGIDVSRSYPED GASGQINIAATATAVYKDGTILGHSSTRPQLGEKLQSTDVRDIMKNAILGYQQVTGESPTHI VIHRDGFMNEDLDPATEFLNEQGVEYDIVEIRKQPQTRLLAVSDVQYDTPVKSIAAINQNEP RATVATFGAPEYLATRDGGGLPRPIQIERVAGETDIETLTRQVYLLSQSHIQVHNSTARLPITT AYADQASTHATKGYLVQTGAFESNVGFL (SEQ ID NO: 63) |

### (9) Cas9 circular permutants

In various embodiments, the base editors disclosed herein may comprise a circular permutant of Cas9.

The term "circularly permuted Cas9" or "circular permutant" of Cas9 or "CP-Cas9") refers to any Cas9 protein, or variant thereof, that occurs or has been modify to engineered as a circular permutant variant, which means the N-terminus and the C-terminus of a Cas9 protein (e.g., a wild type Cas9 protein) have been topically rearranged. Such circularly permuted Cas9 proteins, or variants thereof, retain the ability to bind DNA when complexed with a guide RNA (gRNA). See, Oakes et al., "Protein Engineering of Cas9 for enhanced function," Methods Enzymol, 2014, 546: 491-511 and Oakes et al., "CRISPR-Cas9 Circular Permutants as Programmable Scaffolds for Genome Modification," Cell, January 10, 2019, 176: 254-267, each of are incorporated herein by reference. The instant disclosure contemplates any previously known CP-Cas9 or use a new CP-Cas9 so long as the resulting circularly permuted protein retains the ability to bind DNA when complexed with a guide RNA (gRNA).

Any of the Cas9 proteins described herein, including any variant, ortholog, or naturally occurring Cas9 or equivalent thereof, may be reconfigured as a circular permutant variant.

In various embodiments, the circular permutants of Cas9 may have the following structure:

N-terminus-[original C-terminus] - [optional linker] - [original N-terminus]-C-terminus.

As an example, the present disclosure contemplates the following circular permutants of canonical *S. pyogenes* Cas9 (1368 amino acids of UniProtKB - Q99ZW2 (CAS9_STRP1) (numbering is based on the amino acid position in SEQ ID NO: 5)):
N-terminus-[1268-1368]-[optional linker]-[1-1267]-C-terminus;
N-terminus-[1168-1368]-[optionallinker]-[1-1167]-C-terminus;
N-terminus-[1068-1368]-[optional linker]-[1-1067]-C-terminus;
N-terminus-[968-1368]-[optional linker]-[1-967]-C-terminus;
N-terminus-[868-1368]-[optional linker]-[1-867]-C-terminus;
N-terminus-[768-1368]-[optional linker]-[1-767]-C-terminus;
N-terminus-[668-1368]-[optional linker]-[1-667]-C-terminus;
N-terminus-[568-1368]-[optional linker]-[1-567]-C-terminus;
N-terminus-[468-1368]-[optional linker]-[1-467]-C-terminus;
N-terminus-[368-1368]-[optional linker]-[1-367]-C-terminus;
N-terminus-[268-1368]-[optional linker]-[1-267]-C-terminus;
N-terminus-[168-1368]-[optional linker]-[1-167]-C-terminus;
N-terminus-[68-1368]-[optional linker]-[1-67]-C-terminus; or
N-terminus-[10-1368]-[optional linker]-[1-9]-C-terminus, or the corresponding circular permutants of other Cas9 proteins (including other Cas9 orthologs, variants, etc).

In particular embodiments, the circular permuant Cas9 has the following structure (based on *S. pyogenes* Cas9 (1368 amino acids of UniProtKB - Q99ZW2 (CAS9_STRP1) (numbering is based on the amino acid position in SEQ ID NO: 5):
N-terminus-[102-1368]-[optional linker]-[1-101]-C-terminus;
N-terminus-[1028-1368]-[optional linker]-[1-1027]-C-terminus;
N-terminus-[1041-1368]-[optional linker]-[1-1043]-C-terminus;
N-terminus-[1249-1368]-[optional linker]-[1-1248]-C-terminus; or
N-terminus-[1300-1368]-[optional linker]-[1-1299]-C-terminus, or the corresponding circular permutants of other Cas9 proteins (including other Cas9 orthologs, variants, etc).

In still other embodiments, the circular permuant Cas9 has the following structure (based on *S. pyogenes* Cas9 (1368 amino acids of UniProtKB - Q99ZW2 (CAS9_STRP1) (numbering is based on the amino acid position in SEQ ID NO: 5):
N-terminus-[103-1368]-[optional linker]-[1-102]-C-terminus;
N-terminus-[1029-1368]-[optional linker]-[1-1028]-C-terminus;
N-terminus-[1042-1368]-[optional linker]-[1-1041]-C-terminus;
N-terminus-[1250-1368]-[optional linker]-[1-1249]-C-terminus; or
N-terminus-[1301-1368]-[optional linker]-[1-1300]-C-terminus, or the corresponding circular permutants of other Cas9 proteins (including other Cas9 orthologs, variants, etc).

In some embodiments, the circular permutant can be formed by linking a C-terminal fragment of a Cas9 to an N-terminal fragment of a Cas9, either directly or by using a linker, such as an amino acid linker. In some embodiments, The C-terminal fragment may correspond to the C-terminal 95% or more of the amino acids of a Cas9 (e.g., amino acids about 1300-1368), or the C-terminal 90%, 85%, 80%, 75%, 70%, 65%, 60%, 55%, 50%, 45%, 40%, 35%, 30%, 25%, 20%, 15%, 10%, or 5% or more of a Cas9 (e.g., any one of SEQ ID NOs: 5, 8, 10, 12-26 ). The N-terminal portion may correspond to the N-terminal 95% or more of the amino acids of a Cas9 (e.g., amino acids about 1-1300), or the N-terminal 90%, 85%, 80%, 75%, 70%, 65%, 60%, 55%, 50%, 45%, 40%, 35%, 30%, 25%, 20%, 15%, 10%, or 5% or more of a Cas9 (e.g., of SEQ ID NO: 5, 8, 10, 12-26).

In some embodiments, the circular permutant can be formed by linking a C-terminal fragment of a Cas9 to an N-terminal fragment of a Cas9, either directly or by using a linker, such as an amino acid linker. In some embodiments, the C-terminal fragment that is rearranged to the N-terminus, includes or corresponds to the C-terminal 30% or less of the amino acids of a Cas9 (*e.g*., amino acids 1012-1368 of SEQ ID NO: 5). In some embodiments, the C-terminal fragment that is rearranged to the N-terminus, includes or corresponds to the C-terminal 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, or 1% of the amino acids of a Cas9 (*e.g.,* the Cas9 of SEQ ID NO: 5). In some embodiments, the C-terminal fragment that is rearranged to the N-terminus, includes or corresponds to the C-terminal 410 residues or less of a Cas9 (*e.g.,* the Cas9 of SEQ ID NO: 5). In some embodiments, the C-terminal portion that is rearranged to the N-terminus, includes or corresponds to the C-terminal 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, 100, 90, 80, 70, 60, 50, 40, 30, 20, or 10 residues of a Cas9 (*e.g.,* the Cas9 of SEQ ID NO: 5). In some embodiments, the C-terminal portion that is rearranged to the N-terminus, includes or corresponds to the C-terminal 357, 341, 328, 120, or 69 residues of a Cas9 (e.g., the Cas9 of SEQ ID NO: 5).

In other embodiments, circular permutant Cas9 variants may be defined as a topological rearrangement of a Cas9 primary structure based on the following method, which is based on *S. pyogenes* Cas9 of SEQ ID NO: 5: (a) selecting a circular permutant (CP) site corresponding to an internal amino acid residue of the Cas9 primary structure, which dissects the original protein into two halves: an N-terminal region and a C-terminal region; (b) modifying the Cas9 protein sequence (e.g., by genetic engineering techniques) by moving the original C-terminal region (comprising the CP site amino acid) to precede the original N-terminal region, thereby forming a new N-terminus of the Cas9 protein that now begins with the CP site amino acid residue. The CP site can be located in any domain of the Cas9 protein, including, for example, the helical-II domain, the RuvCIII domain, or the CTD domain. For example, the CP site may be located (relative the *S. pyogenes* Cas9 of SEQ ID NO: 5) at original amino acid residue 181, 199, 230, 270, 310, 1010, 1016, 1023, 1029, 1041, 1247, 1249, or 1282. Thus, once relocated to the N-terminus, original amino acid 181, 199, 230, 270, 310, 1010, 1016, 1023, 1029, 1041, 1247, 1249, or 1282 would become the new N-terminal amino acid. Nomenclature of these CP-Cas9 proteins may be referred to as Cas9-CP¹⁸¹, Cas9-CP¹⁹⁹, Cas9-CP²³⁰, Cas9-CP²⁷⁰, Cas9-CP³¹⁰, Cas9-CP¹⁰¹⁰, Cas9-CP¹⁰¹⁶ , Cas9-CP¹⁰²³, Cas9-CP¹⁰²⁹, Cas9-CP¹⁰⁴¹, Cas9-CP¹²⁴⁷, Cas9-CP¹²⁴⁹, and Cas9-CP¹²⁸², respectively. This description is not meant to be limited to making CP variants from SEQ ID NO: 5, but may be implemented to make CP variants in any Cas9 sequence, either at CP sites that correspond to these positions, or at other CP sites entirely. This description is not meant to limit the specific CP sites in any way. Virtually any CP site may be used to form a CP-Cas9 variant.

Exemplary CP-Cas9 amino acid sequences, based on the Cas9 of SEQ ID NO: 5, are provided below in which linker sequences are indicated by underlining and optional methionine (M) residues are indicated in bold. It should be appreciated that the disclosure provides CP-Cas9 sequences that do not include a linker sequence or that include different linker sequences. It should be appreciated that CP-Cas9 sequences may be based on Cas9 sequences other than that of SEQ ID NO: 5 and any examples provided herein are not meant to be limiting. Exemplary CP-Cas9 sequences are as follows:

| CP name | Sequence | SEQ ID NO: |
|---|---|---|
| CP1012 | DYKVYDVRKMIAKSEQEIGKATAKYFFYSNIMNFFKTEITLA NGEIRKRPLIETNGETGEIVWDKGRDFATVRKVLSMPQVNIV KKTEVQTGGFSKESILPKRNSDKLIARKKDWDPKKYGGFDS PTVAYSVLVVAKVEKGKSKKLKSVKELLGITIMERSSFEKNPI DFLEAKGYKEVKKDLIIKLPKYSLFELENGRKRMLASAGEL QKGNELALPSKYVNFLYLASHYEKLKGSPEDNEQKQLFVEQ HKHYLDEIIEQISEFSKRVILADANLDKVLSAYNKHRDKPIRE QAENIIHLFTLTNLGAPAAFKYFDTTIDRKRYTSTKEVLDATL IHQSITGLYETRIDLSQLGGDGGSGGSGGSGGSGGSGGSGGD KKYSIGLAIGTNSVGWAVITDEYKVPSKKFKVLGNTDRHSIK KNLIGALLFDSGETAEATRLKRTARRRYTRRKNRICYLQEIFS NEMAKVDDSFFHRLEESFLVEEDKKHERHPIFGNIVDEVAYH EKYPTIYHLRKKLVDSTDKADLRLIYLALAHMIKFRGHFLIE | SEQ ID NO: 64 |
| | GDLNPDNSDVDKLFIQLVQTYNQLFEENPINASGVDAKAILS ARLSKSRRLENLIAQLPGEKKNGLFGNLIALSLGLTPNFKSNF DLAEDAKLQLSKDTYDDDLDNLLAQIGDQYADLFLAAKNL SDAILLSDILRVNTEITKAPLSASMIKRYDEHHQDLTLLKALV RQQLPEKYKEIFFDQSKNGYAGYIDGGASQEEFYKFIKPILEK MDGTEELLVKLNREDLLRKQRTFDNGSIPHQIHLGELHAILR RQEDFYPFLKDNREKIEKILTFRIPYYVGPLARGNSRFAWMT RKSEETITPWNFEEVVDKGASAQSFIERMTNFDKNLPNEKV LPKHSLLYEYFTVYNELTKVKYVTEGMRKPAFLSGEQKKAI VDLLFKTNRKVTVKQLKEDYFKKIECFDSVEISGVEDRFNAS LGTYHDLLKIIKDKDFLDNEENEDILEDIVLTLTLFEDREMIE ERLKTYAHLFDDKVMKQLKRRRYTGWGRLSRKLINGIRDK QSGKTILDFLKSDGFANRNFMQLIHDDSLTFKEDIQKAQVSG QGDSLHEHIANLAGSPAIKKGILQTVKVVDELVKVMGRHKP ENIVIEMARENQTTQKGQKNSRERMKRIEEGIKELGSQILKE HPVENTQLQNEKLYLYYLQNGRDMYVDQELDINRLSDYDV DHIVPQSFLKDDSIDNKVLTRSDKNRGKSDNVPSEEVVKKM KNYWRQLLNAKLITQRKFDNLTKAERGGLSELDKAGFIKRQ LVETRQITKHVAQILDSRMNTKYDENDKLIREVKVITLKSKL VSDFRKDFQFYKVREINNYHHAHDAYLNAVVGTALIKKYPK LESEFVYG | |
| CP1028 | EIGKATAKYFFYSNIMNFFKTEITLANGEIRKRPLIETNGETG EIVWDKGRDFATVRKVLSMPQVNIVKKTEVQTGGFSKESIL PKRNSDKLIARKKDWDPKKYGGFDSPTVAYSVLVVAKVEK GKSKKLKSVKELLGITIMERSSFEKNPIDFLEAKGYKEVKKD LIIKLPKYSLFELENGRKRMLASAGELQKGNELALPSKYVNF LYLASHYEKLKGSPEDNEQKQLFVEQHKHYLDEIIEQISEFS KRVILADANLDKVLSAYNKHRDKPIREQAENIIHLFTLTNLG APAAFKYFDTTIDRKRYTSTKEVLDATLIHQSITGLYETRIDL SQLGGDGGSGGSGGSGGSGGSGGSGGMDKKYSIGLAIGTNS VGWAVITDEYKVPSKKFKVLGNTDRHSIKKNLIGALLFDSG ETAEATRLKRTARRRYTRRKNRICYLQEIFSNEMAKVDDSF FHRLEESFLVEEDKKHERHPIFGNIVDEVAYHEKYPTIYHLR KKLVDSTDKADLRLIYLALAHMIKFRGHFLIEGDLNPDNSD VDKLFIQLVQTYNQLFEENPINASGVDAKAILSARLSKSRRL | SEQ ID NO: 65 |
| | ENLIAQLPGEKKNGLFGNLIALSLGLTPNFKSNFDLAEDAKL QLSKDTYDDDLDNLLAQIGDQYADLFLAAKNLSDAILLSDI LRVNTEITKAPLSASMIKRYDEHHQDLTLLKALVRQQLPEK YKEIFFDQSKNGYAGYIDGGASQEEFYKFIKPILEKMDGTEE LLVKLNREDLLRKQRTFDNGSIPHQIHLGELHAILRRQEDFY PFLKDNREKIEKILTFRIPYYVGPLARGNSRFAWMTRKSEETI TPWNFEEVVDKGASAQSFIERMTNFDKNLPNEKVLPKHSLL YEYFTVYNELTKVKYVTEGMRKPAFLSGEQKKAIVDLLFKT NRKVTVKQLKEDYFKKIECFDSVEISGVEDRFNASLGTYHD LLKIIKDKDFLDNEENEDILEDIVLTLTLFEDREMIEERLKTY AHLFDDKVMKQLKRRRYTGWGRLSRKLINGIRDKQSGKTIL DFLKSDGFANRNFMQLIHDDSLTFKEDIQKAQVSGQGDSLH EHIANLAGSPAIKKGILQTVKVVDELVKVMGRHKPENIVIE MARENQTTQKGQKNSRERMKRIEEGIKELGSQILKEHPVEN TQLQNEKLYLYYLQNGRDMYVDQELDINRLSDYDVDHIVP QSFLKDDSIDNKVLTRSDKNRGKSDNVPSEEVVKKMKNYW RQLLNAKLITQRKFDNLTKAERGGLSELDKAGFIKRQLVET RQITKHVAQILDSRMNTKYDENDKLIREVKVITLKSKLVSDF RKDFQFYKVREINNYHHAHDAYLNAVVGTALIKKYPKLES EFVYGDYKVYDVRKMIAKSEQ | |
| CP1041 | NIMNFFKTEITLANGEIRKRPLIETNGETGEIVWDKGRDFAT VRKVLSMPQVNIVKKTEVQTGGFSKESILPKRNSDKLIARKK DWDPKKYGGFDSPTVAYSVLVVAKVEKGKSKKLKSVKELL GITIMERSSFEKNPIDFLEAKGYKEVKKDLIIKLPKYSLFELE NGRKRMLASAGELQKGNELALPSKYVNFLYLASHYEKLKG SPEDNEQKQLFVEQHKHYLDEIIEQISEFSKRVILADANLDK VLSAYNKHRDKPIREQAENIIHLFTLTNLGAPAAFKYFDTTI DRKRYTSTKEVLDATLIHQSITGLYETRIDLSQLGGDGGSGG SGGSGGSGGSGGSGGDKKYSIGLAIGTNSVGWAVITDEYKV PSKKFKVLGNTDRHSIKKNLIGALLFDSGETAEATRLKRTAR RRYTRRKNRICYLQEIFSNEMAKVDDSFFHRLEESFLVEEDK KHERHPIFGNIVDEVAYHEKYPTIYHLRKKLVDSTDKADLR LIYLALAHMIKFRGHFLIEGDLNPDNSDVDKLFIQLVQTYNQ LFEENPINASGVDAKAILSARLSKSRRLENLIAQLPGEKKNG LFGNLIALSLGLTPNFKSNFDLAEDAKLQLSKDTYDDDLDN | SEQ ID NO: 66 |
| | LLAQIGDQYADLFLAAKNLSDAILLSDILRVNTEITKAPLSAS MIKRYDEHHQDLTLLKALVRQQLPEKYKEIFFDQSKNGYAG YIDGGASQEEFYKFIKPILEKMDGTEELLVKLNREDLLRKQR TFDNGSIPHQIHLGELHAILRRQEDFYPFLKDNREKIEKILTFR IPYYVGPLARGNSRFAWMTRKSEETITPWNFEEVVDKGASA QSFIERMTNFDKNLPNEKVLPKHSLLYEYFTVYNELTKVKY VTEGMRKPAFLSGEQKKAIVDLLFKTNRKVTVKQLKEDYF KKIECFDSVEISGVEDRFNASLGTYHDLLKIIKDKDFLDNEE NEDILEDIVLTLTLFEDREMIEERLKTYAHLFDDKVMKQLKR RRYTGWGRLSRKLINGIRDKQSGKTILDFLKSDGFANRNFM QLIHDDSLTFKEDIQKAQVSGQGDSLHEHIANLAGSPAIKKG ILQTVKVVDELVKVMGRHKPENIVIEMARENQTTQKGQKN SRERMKRIEEGIKELGSQILKEHPVENTQLQNEKLYLYYLQN GRDMYVDQELDINRLSDYDVDHIVPQSFLKDDSIDNKVLTR SDKNRGKSDNVPSEEVVKKMKNYWRQLLNAKLITQRKFDN LTKAERGGLSELDKAGFIKRQLVETRQITKHVAQILDSRMN TKYDENDKLIREVKVITLKSKLVSDFRKDFQFYKVREINNY HHAHDAYLNAVVGTALIKKYPKLESEFVYGDYKVYDVRK MIAKSEQEIGKATAKYFFYS | |
| CP1249 | PEDNEQKQLFVEQHKHYLDEIIEQISEFSKRVILADANLDKV LSAYNKHRDKPIREQAENIIHLFTLTNLGAPAAFKYFDTTIDR KRYTSTKEVLDATLIHQSITGLYETRIDLSQLGGDGGSGGSG GSGGSGGSGGSGGMDKKYSIGLAIGTNSVGWAVITDEYKV PSKKFKVLGNTDRHSIKKNLIGALLFDSGETAEATRLKRTAR RRYTRRKNRICYLQEIFSNEMAKVDDSFFHRLEESFLVEEDK KHERHPIFGNIVDEVAYHEKYPTIYHLRKKLVDSTDKADLR LIYLALAHMIKFRGHFLIEGDLNPDNSDVDKLFIQLVQTYNQ LFEENPINASGVDAKAILSARLSKSRRLENLIAQLPGEKKNG LFGNLIALSLGLTPNFKSNFDLAEDAKLQLSKDTYDDDLDN LLAQIGDQYADLFLAAKNLSDAILLSDILRVNTEITKAPLSAS MIKRYDEHHQDLTLLKALVRQQLPEKYKEIFFDQSKNGYAG YIDGGASQEEFYKFIKPILEKMDGTEELLVKLNREDLLRKQR TFDNGSIPHQIHLGELHAILRRQEDFYPFLKDNREKIEKILTFR IPYYVGPLARGNSRFAWMTRKSEETITPWNFEEVVDKGASA QSFIERMTNFDKNLPNEKVLPKHSLLYEYFTVYNELTKVKY | SEQ ID NO: 67 |
| | VTEGMRKPAFLSGEQKKAIVDLLFKTNRKVTVKQLKEDYF KKIECFDSVEISGVEDRFNASLGTYHDLLKIIKDKDFLDNEE NEDILEDIVLTLTLFEDREMIEERLKTYAHLFDDKVMKQLKR RRYTGWGRLSRKLINGIRDKQSGKTILDFLKSDGFANRNFM QLIHDDSLTFKEDIQKAQVSGQGDSLHEHIANLAGSPAIKKG ILQTVKVVDELVKVMGRHKPENIVIEMARENQTTQKGQKN SRERMKRIEEGIKELGSQILKEHPVENTQLQNEKLYLYYLQN GRDMYVDQELDINRLSDYDVDHIVPQSFLKDDSIDNKVLTR SDKNRGKSDNVPSEEVVKKMKNYWRQLLNAKLITQRKFDN LTKAERGGLSELDKAGFIKRQLVETRQITKHVAQILDSRMN TKYDENDKLIREVKVITLKSKLVSDFRKDFQFYKVREINNY HHAHDAYLNAVVGTALIKKYPKLESEFVYGDYKVYDVRK MIAKSEQEIGKATAKYFFYSNIMNFFKTEITLANGEIRKRPLI ETNGETGEIVWDKGRDFATVRKVLSMPQVNIVKKTEVQTG GFSKESILPKRNSDKLIARKKDWDPKKYGGFDSPTVAYSVL VVAKVEKGKSKKLKSVKELLGITIMERSSFEKNPIDFLEAKG YKEVKKDLIIKLPKYSLFELENGRKRMLASAGELQKGNELA LPSKYVNFLYLASHYEKLKGS | |
| CP1300 | KPIREQAENIIHLFTLTNLGAPAAFKYFDTTIDRKRYTSTKEV LDATLIHQSITGLYETRIDLSQLGGDGGSGGSGGSGGSGGSG GSGGDKKYSIGLAIGTNSVGWAVITDEYKVPSKKFKVLGNT DRHSIKKNLIGALLFDSGETAEATRLKRTARRRYTRRKNRIC YLQEIFSNEMAKVDDSFFHRLEESFLVEEDKKHERHPIFGNI VDEVAYHEKYPTIYHLRKKLVDSTDKADLRLIYLALAHMIK FRGHFLIEGDLNPDNSDVDKLFIQLVQTYNQLFEENPINASG VDAKAILSARLSKSRRLENLIAQLPGEKKNGLFGNLIALSLG LTPNFKSNFDLAEDAKLQLSKDTYDDDLDNLLAQIGDQYA DLFLAAKNLSDAILLSDILRVNTEITKAPLSASMIKRYDEHH QDLTLLKALVRQQLPEKYKEIFFDQSKNGYAGYIDGGASQE EFYKFIKPILEKMDGTEELLVKLNREDLLRKQRTFDNGSIPH QIHLGELHAILRRQEDFYPFLKDNREKIEKILTFRIPYYVGPL ARGNSRFAWMTRKSEETITPWNFEEVVDKGASAQSFIERMT NFDKNLPNEKVLPKHSLLYEYFTVYNELTKVKYVTEGMRK PAFLSGEQKKAIVDLLFKTNRKVTVKQLKEDYFKKIECFDS VEISGVEDRFNASLGTYHDLLKIIKDKDFLDNEENEDILEDIV LTLTLFEDREMIEERLKTYAHLFDDKVMKQLKRRRYTGWG RLSRKLINGIRDKQSGKTILDFLKSDGFANRNFMQLIHDDSL TFKEDIQKAQVSGQGDSLHEHIANLAGSPAIKKGILQTVKVV DELVKVMGRHKPENIVIEMARENQTTQKGQKNSRERMKRI EEGIKELGSQILKEHPVENTQLQNEKLYLYYLQNGRDMYVD QELDINRLSDYDVDHIVPQSFLKDDSIDNKVLTRSDKNRGKS DNVPSEEVVKKMKNYWRQLLNAKLITQRKFDNLTKAERGG LSELDKAGFIKRQLVETRQITKHVAQILDSRMNTKYDENDK LIREVKVITLKSKLVSDFRKDFQFYKVREINNYHHAHDAYL NAVVGTALIKKYPKLESEFVYGDYKVYDVRKMIAKSEQEIG KATAKYFFYSNIMNFFKTEITLANGEIRKRPLIETNGETGEIV WDKGRDFATVRKVLSMPQVNIVKKTEVQTGGFSKESILPKR NSDKLIARKKDWDPKKYGGFDSPTVAYSVLVVAKVEKGKS KKLKSVKELLGITIMERSSFEKNPIDFLEAKGYKEVKKDLIIK LPKYSLFELENGRKRMLASAGELQKGNELALPSKYVNFLYL ASHYEKLKGSPEDNEQKQLFVEQHKHYLDEIIEQISEFSKRVI LADANLDKVLSAYNKHRD | SEQ ID NO: 68 |

The Cas9 circular permutants that may be useful in the base editing constructs described herein. Exemplary C-terminal fragments of Cas9, based on the Cas9 of SEQ ID NO: 5, which may be rearranged to an N-terminus of Cas9, are provided below. It should be appreciated that such C-terminal fragments of Cas9 are exemplary and are not meant to be limiting. These exemplary CP-Cas9 fragments have the following sequences:

| CP name | Sequence | SEQ ID NO: |
|---|---|---|
| CP1012 C-terminal fragment | DYKVYDVRKMIAKSEQEIGKATAKYFFYSNIMNFFKTEITL ANGEIRKRPLIETNGETGEIVWDKGRDFATVRKVLSMPQVN IVKKTEVQTGGFSKESILPKRNSDKLIARKKDWDPKKYGGF DSPTVAYSVLVVAKVEKGKSKKLKSVKELLGITIMERSSFEK NPIDFLEAKGYKEVKKDLIIKLPKYSLFELENGRKRMLASAG ELQKGNELALPSKYVNFLYLASHYEKLKGSPEDNEQKQLFV EQHKHYLDEIIEQISEFSKRVILADANLDKVLSAYNKHRDKP IREQAENIIHLFTLTNLGAPAAFKYFDTTIDRKRYTSTKEVLD ATLIHQSITGLYETRIDLSQLGGD | 69 |
| CP1028 C-terminal fragment | EIGKATAKYFFYSNIMNFFKTEITLANGEIRKRPLIETNGETG EIVWDKGRDFATVRKVLSMPQVNIVKKTEVQTGGFSKESIL PKRNSDKLIARKKDWDPKKYGGFDSPTVAYSVLVVAKVEK | 70 |
| | GKSKKLKSVKELLGITIMERSSFEKNPIDFLEAKGYKEVKKD LIIKLPKYSLFELENGRKRMLASAGELQKGNELALPSKYVNF LYLASHYEKLKGSPEDNEQKQLFVEQHKHYLDEIIEQISEFS KRVILADANLDKVLSAYNKHRDKPIREQAENIIHLFTLTNLG APAAFKYFDTTIDRKRYTSTKEVLDATLIHQSITGLYETRIDL SQLGGD | |
| CP1041 C-terminal fragment | NIMNFFKTEITLANGEIRKRPLIETNGETGEIVWDKGRDFAT VRKVLSMPQVNIVKKTEVQTGGFSKESILPKRNSDKLIARKK DWDPKKYGGFDSPTVAYSVLVVAKVEKGKSKKLKSVKELL GITIMERSSFEKNPIDFLEAKGYKEVKKDLIIKLPKYSLFELE NGRKRMLASAGELQKGNELALPSKYVNFLYLASHYEKLKG SPEDNEQKQLFVEQHKHYLDEIIEQISEFSKRVILADANLDK VLSAYNKHRDKPIREQAENIIHLFTLTNLGAPAAFKYFDTTI DRKRYTSTKEVLDATLIHQSITGLYETRIDLSQLGGD | 71 |
| CP1249 C-terminal fragment | PEDNEQKQLFVEQHKHYLDEIIEQISEFSKRVILADANLDKV LSAYNKHRDKPIREQAENIIHLFTLTNLGAPAAFKYFDTTIDR KRYTSTKEVLDATLIHQSITGLYETRIDLSQLGGD | 72 |
| CP1300 C-terminal fragment | KPIREQAENIIHLFTLTNLGAPAAFKYFDTTIDRKRYTSTKEV LDATLIHQSITGLYETRIDLSQLGGD | 73 |

### (10) Cas9 variants with modified PAM specificities

The base editors of the present disclosure may also comprise Cas9 variants with modified PAM specificities. For example, the base editors described herein may utilize any naturally occuring or engineered variant of SpCas9 having expanded and/or relaxed PAM specificities which are described in the literure, including in Nishimasu et al., "Engineered CRISPR-Cas9 nuclease with expanded targeting space," Science, 2018, 361: 1259-1262; Chatterjee et al., "Robust Genome Editing of Single-Base PAM Targets with Engineered ScCas9 Variants," BioRxiv, April 26, 2019. Some aspects of this disclosure provide Cas9 proteins that exhibit activity on a target sequence that does not comprise the canonical PAM (5'-NGG-3', where N is A, C, G, or T) at its 3'-end. In some embodiments, the Cas9 protein exhibits activity on a target sequence comprising a 5'-NGG-3' PAM sequence at its 3'-end. In some embodiments, the Cas9 protein exhibits activity on a target sequence comprising a 5'-NNG-3' PAM sequence at its 3'-end. In some embodiments, the Cas9 protein exhibits activity on a target sequence comprising a 5'-NNA-3' PAM sequence at its 3'-end. In some embodiments, the Cas9 protein exhibits activity on a target sequence comprising a 5'-NNC-3' PAM sequence at its 3'-end. In some embodiments, the Cas9 protein exhibits activity on a target sequence comprising a 5'-NNT-3' PAM sequence at its 3'-end. In some embodiments, the Cas9 protein exhibits activity on a target sequence comprising a 5'-NGT-3' PAM sequence at its 3'-end. In some embodiments, the Cas9 protein exhibits activity on a target sequence comprising a 5'-NGA-3' PAM sequence at its 3'-end. In some embodiments, the Cas9 protein exhibits activity on a target sequence comprising a 5'-NGC-3' PAM sequence at its 3'-end. In some embodiments, the Cas9 protein exhibits activity on a target sequence comprising a 5'-NAA-3' PAM sequence at its 3'-end. In some embodiments, the Cas9 protein exhibits activity on a target sequence comprising a 5'-NAC-3' PAM sequence at its 3'-end. In some embodiments, the Cas9 protein exhibits activity on a target sequence comprising a 5'-NAT-3' PAM sequence at its 3'-end. In still other embodiments, the Cas9 protein exhibits activity on a target sequence comprising a 5'-NAG-3' PAM sequence at its 3'-end.

It should be appreciated that any of the amino acid mutations described herein, (e.g., A262T) from a first amino acid residue (e.g., A) to a second amino acid residue (e.g., T) may also include mutations from the first amino acid residue to an amino acid residue that is similar to (e.g., conserved) the second amino acid residue. For example, mutation of an amino acid with a hydrophobic side chain (e.g., alanine, valine, isoleucine, leucine, methionine, phenylalanine, tyrosine, or tryptophan) may be a mutation to a second amino acid with a different hydrophobic side chain (e.g., alanine, valine, isoleucine, leucine, methionine, phenylalanine, tyrosine, or tryptophan). For example, a mutation of an alanine to a threonine (e.g., a A262T mutation) may also be a mutation from an alanine to an amino acid that is similar in size and chemical properties to a threonine, for example, serine. As another example, mutation of an amino acid with a positively charged side chain (e.g., arginine, histidine, or lysine) may be a mutation to a second amino acid with a different positively charged side chain (e.g., arginine, histidine, or lysine). As another example, mutation of an amino acid with a polar side chain (e.g., serine, threonine, asparagine, or glutamine) may be a mutation to a second amino acid with a different polar side chain (e.g., serine, threonine, asparagine, or glutamine). Additional similar amino acid pairs include, but are not limited to, the following: phenylalanine and tyrosine; asparagine and glutamine; methionine and cysteine; aspartic acid and glutamic acid; and arginine and lysine. The skilled artisan would recognize that such conservative amino acid substitutions will likely have minor effects on protein structure and are likely to be well tolerated without compromising function. In some embodiments, any amino of the amino acid mutations provided herein from one amino acid to a threonine may be an amino acid mutation to a serine. In some embodiments, any amino of the amino acid mutations provided herein from one amino acid to an arginine may be an amino acid mutation to a lysine. In some embodiments, any amino of the amino acid mutations provided herein from one amino acid to an isoleucine, may be an amino acid mutation to an alanine, valine, methionine, or leucine. In some embodiments, any amino of the amino acid mutations provided herein from one amino acid to a lysine may be an amino acid mutation to an arginine. In some embodiments, any amino of the amino acid mutations provided herein from one amino acid to an aspartic acid may be an amino acid mutation to a glutamic acid or asparagine. In some embodiments, any amino of the amino acid mutations provided herein from one amino acid to a valine may be an amino acid mutation to an alanine, isoleucine, methionine, or leucine. In some embodiments, any amino of the amino acid mutations provided herein from one amino acid to a glycine may be an amino acid mutation to an alanine. It should be appreciated, however, that additional conserved amino acid residues would be recognized by the skilled artisan and any of the amino acid mutations to other conserved amino acid residues are also within the scope of this disclosure.In some embodiments, the Cas9 protein comprises a combination of mutations that exhibit activity on a target sequence comprising a 5'-NAA-3' PAM sequence at its 3'-end. In some embodiments, the combination of mutations are present in any one of the clones listed in Table 1. In some embodiments, the combination of mutations are conservative mutations of the clones listed in Table 1. In some embodiments, the Cas9 protein comprises the combination of mutations of any one of the Cas9 clones listed in Table 1.

**Table 1: NAA PAM Clones**

| Mutations from wild-type SpCas9 (e.g., SEQ ID NO: 5) |
|---|
| D177N, K218R, D614N, D1135N, P1137S, E1219V, A1320V, A1323D, R1333K |
| D177N, K218R, D614N, D1135N, E1219V, Q1221H, H1264Y, A1320V, R1333K |
| A10T, I322V, S409I, E427G, G715C, D1135N, E1219V, Q1221H, H1264Y, A1320V, R1333K |
| A367T, K710E, R1114G, D1135N, P1137S, E1219V, Q1221H, H1264Y, A1320V, R1333K |
| A10T, I322V, S409I, E427G, R753G, D861N, D1135N, K1188R, E1219V, Q1221H, H1264H, A1320V, R1333K |
| A10T, I322V, S409I, E427G, R654L, V743I, R753G, M1021T, D1135N, D1180G, K1211R, E1219V, Q1221H, H1264Y, A1320V, R1333K |
| A10T, I322V, S409I, E427G, V743I, R753G, E762G, D1135N, D1180G, K1211R, E1219V, Q1221H, H1264Y, A1320V, R1333K |
| A10T, I322V, S409I, E427G, R753G, D1135N, D1180G, K1211R, E1219V, Q1221H, H1264Y, S1274R, A1320V, R1333K |
| A10T, I322V, S409I, E427G, A589S, R753G, D1135N, E1219V, Q1221H, H1264H, A1320V, R1333K |
| A10T, I322V, S409I, E427G, R753G, E757K, G865G, D1135N, E1219V, Q1221H, H1264Y, A1320V, R1333K |
| A10T, I322V, S409I, E427G, R654L, R753G, E757K, D1135N, E1219V, Q1221H, H1264Y, A1320V, R1333K |
| A10T, I322V, S409I, E427G, K599R, M631A, R654L, K673E, V743I, R753G, N758H, E762G, D1135N, D1180G, E1219V, Q1221H, Q1256R, H1264Y, A1320V, A1323D, R1333K |
| A10T, I322V, S409I, E427G, R654L, K673E, V743I, R753G, E762G, N869S, N1054D, R1114G, D1135N, D1180G, E1219V, Q1221H, H1264Y, A1320V, A1323D, R1333K |
| A10T, I322V, S409I, E427G, R654L, L727I, V743I, R753G, E762G, R859S, N946D, F1134L, D1135N, D1180G, E1219V, Q1221H, H1264Y, N1317T, A1320V, A1323D, R1333K |
| A10T, I322V, S409I, E427G, R654L, K673E, V743I, R753G, E762G, N803S, N869S, Y1016D, G1077D, R1114G, F1134L, D1135N, D1180G, E1219V, Q1221H, H1264Y, V1290G, L1318S, A1320V, A1323D, R1333K |
| A10T, I322V, S409I, E427G, R654L, K673E, V743I, R753G, E762G, N803S, N869S, Y1016D, G1077D, R1114G, F1134L, D1135N, K1151E, D1180G, E1219V, Q1221H, H1264Y, V1290G, L1318S, A1320V, R1333K |
| A10T, I322V, S409I, E427G, R654L, K673E, V743I, R753G, E762G, N803S, N869S, Y1016D, G1077D, R1114G, F1134L, D1135N, D1180G, E1219V, Q1221H, H1264Y, V1290G, L1318S, A1320V, A1323D, R1333K |
| A10T, I322V, S409I, E427G, R654L, K673E, F693L, V743I, R753G, E762G, N803S, N869S, L921P, Y1016D, G1077D, F1080S, R1114G, D1135N, D1180G, E1219V, Q1221H, H1264Y, L1318S, A1320V, A1323D, R1333K |
| A10T, I322V, S409I, E427G, E630K, R654L, K673E, V743I, R753G, E762G, Q768H, N803S, N869S, Y1016D, G1077D, R1114G, F1134L, D1135N, D1180G, E1219V, Q1221H, H1264Y, L1318S, A1320V, R1333K |
| A10T, I322V, S409I, E427G, R654L, K673E, F693L, V743I, R753G, E762G, Q768H, N803S, N869S, Y1016D, G1077D, R1114G, F1134L, D1135N, D1180G, E1219V, Q1221H, G1223S, H1264Y, L1318S, A1320V, R1333K |
| A10T, I322V, S409I, E427G, R654L, K673E, F693L, V743I, R753G, E762G, N803S, N869S, L921P, Y1016D, G1077D, F1801S, R1114G, D1135N, D1180G, E1219V, Q1221H, H1264Y, L1318S, A1320V, A1323D, R1333K |
| A10T, I322V, S409I, E427G, R654L, V743I, R753G, M1021T, D1135N, D1180G, K1211R, E1219V, Q1221H, H1264Y, A1320V, R1333K |
| A10T, I322V, S409I, E427G, R654L, K673E, V743I, R753G, E762G, M673I, N803S, N869S, G1077D, R1114G, D1135N, V1139A, D1180G, E1219V, Q1221H, A1320V, R1333K |
| A10T, I322V, S409I, E427G, R654L, K673E, V743I, R753G, E762G, N803S, N869S, R1114G, D1135N, E1219V, Q1221H, A1320V, R1333K |

In some embodiments, the Cas9 protein comprises an amino acid sequence that is at least 80% identical to the amino acid sequence of a Cas9 protein as provided by any one of the variants of Table 1. In some embodiments, the Cas9 protein comprises an amino acid sequence that is at least 85%, at least 90%, at least 92%, at least 95%, at least 96%, at least 97%, at least 98%, at least 99%, or at least 99.5% identical to the amino acid sequence of a Cas9 protein as provided by any one of the variants of Table 1.

In some embodiments, the Cas9 protein exhibits an increased activity on a target sequence that does not comprise the canonical PAM (5'-NGG-3') at its 3' end as compared to *Streptococcus pyogenes* Cas9 as provided by SEQ ID NO: 5. In some embodiments, the Cas9 protein exhibits an activity on a target sequence having a 3' end that is not directly adjacent to the canonical PAM sequence (5'-NGG-3') that is at least 5-fold increased as compared to the activity of *Streptococcus pyogenes* Cas9 as provided by SEQ ID NO: 5 on the same target sequence. In some embodiments, the Cas9 protein exhibits an activity on a target sequence that is not directly adjacent to the canonical PAM sequence (5'-NGG-3') that is at least 10-fold, at least 50-fold, at least 100-fold, at least 500-fold, at least 1,000-fold, at least 5,000-fold, at least 10,000-fold, at least 50,000-fold, at least 100,000-fold, at least 500,000-fold, or at least 1,000,000-fold increased as compared to the activity of *Streptococcus pyogenes* as provided by SEQ ID NO: 5 on the same target sequence. In some embodiments, the 3' end of the target sequence is directly adjacent to an AAA, GAA, CAA, or TAA sequence. In some embodiments, the Cas9 protein comprises a combination of mutations that exhibit activity on a target sequence comprising a 5'-NAC-3' PAM sequence at its 3'-end. In some embodiments, the combination of mutations are present in any one of the clones listed in Table 2. In some embodiments, the combination of mutations are conservative mutations of the clones listed in Table 2. In some embodiments, the Cas9 protein comprises the combination of mutations of any one of the Cas9 clones listed in Table 2.

**Table 2: NAC PAM Clones**

| |
|---|
| MUTATIONS FROM WILD-TYPE SPCAS9 (E.G., SEQ ID NO: 5) |
| T472I, R753G, K890E, D1332N, R1335Q, T1337N |
| I1057S, D1135N, P1301S, R1335Q, T1337N |
| T472I, R753G, D1332N, R1335Q, T1337N |
| D1135N, E1219V, D1332N, R1335Q, T1337N |
| T472I, R753G, K890E, D1332N, R1335Q, T1337N |
| I1057S, D1135N, P1301S, R1335Q, T1337N |
| T472I, R753G, D1332N, R1335Q, T1337N |
| T472I, R753G, Q771H, D1332N, R1335Q, T1337N |
| E627K, T638P, K652T, R753G, N803S, K959N, R1114G, D1135N, E1219V, D1332N, R1335Q, T1337N |
| E627K, T638P, K652T, R753G, N803S, K959N, R1114G, D1135N, K1156E, E1219V, D1332N, R1335Q, T1337N |
| E627K, T638P, V647I, R753G, N803S, K959N, G1030R, I1055E, R1114G, D1135N, E1219V, D1332N, R1335Q, T1337N |
| E627K, E630G, T638P, V647A, G687R, N767D, N803S, K959N, R1114G, D1135N, E1219V, D1332G, R1335Q, T1337N |
| E627K, T638P, R753G, N803S, K959N, R1114G, D1135N, E1219V, N1266H, D1332N, R1335Q, T1337N |
| E627K, T638P, R753G, N803S, K959N, I1057T, R1114G, D1135N, E1219V, D1332N, R1335Q, T1337N |
| E627K, T638P, R753G, N803S, K959N, R1114G, D1135N, E1219V, D1332N, R1335Q, T1337N |
| E627K, M631I, T638P, R753G, N803S, K959N, Y1036H, R1114G, D1135N, E1219V, D1251G, D1332G, R1335Q, T1337N |
| E627K, T638P, R753G, N803S, V875I, K959N, Y1016C, R1114G, D1135N, E1219V, D1251G, D1332G, R1335Q, T1337N, I1348V |
| K608R, E627K, T638P, V647I, R654L, R753G, N803S, T804A, K848N, V922A, K959N, R1114G, D1135N, E1219V, D1332N, R1335Q, T1337N |
| K608R, E627K, T638P, V647I, R753G, N803S, V922A, K959N, K1014N, V1015A, R1114G, D1135N, K1156N, E1219V, N1252D, D1332N, R1335Q, T1337N |
| K608R, E627K, R629G, T638P, V647I, A711T, R753G, K775R, K789E, N803S, K959N, V1015A, Y1036H, R1114G, D1135N, E1219V, N1286H, D1332N, R1335Q, T1337N |
| K608R, E627K, T638P, V647I, T740A, R753G, N803S, K948E, K959N, Y1016S, R1114G, D1135N, E1219V, N1286H, D1332N, R1335Q, T1337N |
| K608R, E627K, T638P, V647I, T740A, N803S, K948E, K959N, Y1016S, R1114G, D1135N, E1219V, N1286H, D1332N, R1335Q, T1337N |
| I670S, K608R, E627K, E630G, T638P, V647I, R653K, R753G, I795L, K797N, N803S, K866R, K890N, K959N, Y1016C, R1114G, D1135N, E1219V, D1332N, R1335Q, T1337N |
| K608R, E627K, T638P, V647I, T740A, G752R, R753G, K797N, N803S, K948E, K959N, V1015A, Y1016S, R1114G, D1135N, E1219V, N1266H, D1332N, R1335Q, T1337N |
| I570T, A589V, K608R, E627K, T638P, V647I, R654L, Q716R, R753G, N803S, K948E, K959N, Y1016S, R1114G, D1135N, E1207G, E1219V, N1234D, D1332N, R1335Q, T1337N |
| K608R, E627K, R629G, T638P, V647I, R654L, Q740R, R753G, N803S, K959N, N990S, T995S, V1015A, Y1036D, R1114G, D1135N, E1207G, E1219V, N1234D, N1266H, D1332N, R1335Q, T1337N |
| I562F, V565D, I570T, K608R, L625S, E627K, T638P, V647I, R654I, G752R, R753G, N803S, N808D, K959N, M1021L, R1114G, D1135N, N1177S, N1234D, D1332N, R1335Q, T1337N |
| I562F, I570T, K608R, E627K, T638P, V647I, R753G, E790A, N803S, K959N, V1015A, Y1036H, R1114G, D1135N, D1180E, A1184T, E1219V, D1332N, R1335Q, T1337N |
| I570T, K608R, E627K, T638P, V647I, R654H, R753G, E790A, N803S, K959N, V1015A, R1114G, D1127A, D1135N, E1219V, D1332N, R1335Q, T1337N |
| I570T, K608R, L625S, E627K, T638P, V647I, R654I, T703P, R753G, N803S, N808D, K959N, M1021L, R1114G, D1135N, E1219V, D1332N, R1335Q, T1337N |
| I570S, K608R, E627K, E630G, T638P, V647I, R653K, R753G, I795L, N803S, K866R, K890N, K959N, Y1016C, R1114G, D1135N, E1219V, D1332N, R1335Q, T1337N |
| I570T, K608R, E627K, T638P, V647I, R654H, R753G, E790A, N803S, K959N, V1016A, R1114G, D1135N, E1219V, K1246E, D1332N, R1335Q, T1337N |
| K608R, E627K, T638P, V647I, R654L, K673E, R753G, E790A, N803S, K948E, K959N, R1114G, D1127G, D1135N, D1180E, E1219V, N1286H, D1332N, R1335Q, T1337N |
| K608R, L625S, E627K, T638P, V647I, R654I, I670T, R753G, N803S, N808D, K959N, M1021L, R1114G, D1135N, E1219V, N1286H, D1332N, R1335Q, T1337N |
| E627K, M631V, T638P, V647I, K710E, R753G, N803S, N808D, K948E, M1021L, R1114G, D1135N, E1219V, D1332N, R1335Q, T1337N, S1338T, H1349R |

In some embodiments, the Cas9 protein comprises an amino acid sequence that is at least 80% identical to the amino acid sequence of a Cas9 protein as provided by any one of the variants of Table 2. In some embodiments, the Cas9 protein comprises an amino acid sequence that is at least 85%, at least 90%, at least 92%, at least 95%, at least 96%, at least 97%, at least 98%, at least 99%, or at least 99.5% identical to the amino acid sequence of a Cas9 protein as provided by any one of the variants of Table 2.

In some embodiments, the Cas9 protein exhibits an increased activity on a target sequence that does not comprise the canonical PAM (5'-NGG-3') at its 3' end as compared to *Streptococcus pyogenes* Cas9 as provided by SEQ ID NO: 5. In some embodiments, the Cas9 protein exhibits an activity on a target sequence having a 3' end that is not directly adjacent to the canonical PAM sequence (5'-NGG-3') that is at least 5-fold increased as compared to the activity of *Streptococcus pyogenes* Cas9 as provided by SEQ ID NO: 5 on the same target sequence. In some embodiments, the Cas9 protein exhibits an activity on a target sequence that is not directly adjacent to the canonical PAM sequence (5'-NGG-3') that is at least 10-fold, at least 50-fold, at least 100-fold, at least 500-fold, at least 1,000-fold, at least 5,000-fold, at least 10,000-fold, at least 50,000-fold, at least 100,000-fold, at least 500,000-fold, or at least 1,000,000-fold increased as compared to the activity of *Streptococcus pyogenes* as provided by SEQ ID NO: 5 on the same target sequence. In some embodiments, the 3' end of the target sequence is directly adjacent to an AAC, GAC, CAC, or TAC sequence.

In some embodiments, the Cas9 protein comprises a combination of mutations that exhibit activity on a target sequence comprising a 5'-NAT-3' PAM sequence at its 3'-end. In some embodiments, the combination of mutations are present in any one of the clones listed in Table 3. In some embodiments, the combination of mutations are conservative mutations of the clones listed in Table 3. In some embodiments, the Cas9 protein comprises the combination of mutations of any one of the Cas9 clones listed in Table 3.

**Table 3: NAT PAM Clones**

| MUTATIONS FROM WILD-TYPE SPCAS9 (E.G., SEQ ID NO: 5) |
|---|
| K961E, H985Y, D1135N, K1191N, E1219V, Q1221H, A1320A, P1321S, R1335L |
| D1135N, G1218S, E1219V, Q1221H, P1249S, P1321S, D1322G, R1335L |
| V743I, R753G, E790A, D1135N, G1218S, E1219V, Q1221H, A1227V, P1249S, N1286K, A1293T, P1321S, D1322G, R1335L, T1339I |
| F575S, M631L, R654L, V748I, V743I, R753G, D853E, V922A, R1114G D1135N, G1218S, E1219V, Q1221H, A1227V, P1249S, N1286K, A1293T, P1321S, D1322G, R1335L, T1339I |
| F575S, M631L, R654L, R664K, R753G, D853E, V922A, R1114G D1135N, D1180G, G1218S, E1219V, Q1221H, P1249S, N1286K, P1321S, D1322G, R1335L |
| M631L, R654L, R753G, K797E, D853E, V922A, D1012A, R1114G D1135N, G1218S, E1219V, Q1221H, P1249S, N1317K, P1321S, D1322G, R1335L |
| F575S, M631L, R654L, R664K, R753G, D853E, V922A, R1114G, Y1131C, D1135N, D1180G, G1218S, E1219V, Q1221H, P1249S, P1321S, D1322G, R1335L |
| F575S, M631L, R654L, R664K, R753G, D853E, V922A, R1114G, Y1131C, D1135N, D1180G, G1218S, E1219V, Q1221H, P1249S, P1321S, D1322G, R1335L |
| F575S, D596Y, M631L, R654L, R664K, R753G, D853E, V922A, R1114G, Y1131C, D1135N, D1180G, G1218S, E1219V, Q1221H, P1249S, Q1256R, P1321S, D1322G, R1335L |
| F575S, M631L, R654L, R664K, K710E, V750A, R753G, D853E, V922A, R1114G, Y1131C, D1135N, D1180G, G1218S, E1219V, Q1221H, P1249S, P1321S, D1322G, R1335L |
| F575S, M631L, K649R, R654L, R664K, R753G, D853E, V922A, R1114G, Y1131C, D1135N, K1156E, D1180G, G1218S, E1219V, Q1221H, P1249S, P1321S, D1322G, R1335L |
| F575S, M631L, R654L, R664K, R753G, D853E, V922A, R1114G, Y1131C, D1135N, D1180G, G1218S, E1219V, Q1221H, P1249S, P1321S, D1322G, R1335L |
| F575S, M631L, R654L, R664K, R753G, D853E, V922A, 11057G, R1114G, Y1131C, D1135N, D1180G, G1218S, E1219V, Q1221H, P1249S, N1308D, P1321S, D1322G, R1335L |
| M631L, R654L, R753G, D853E, V922A, R1114G, Y1131C, D1135N, E1150V, D1180G, G1218S, E1219V, Q1221H, P1249S, P1321S, D1332G, R1335L |
| M631L, R654L, R664K, R753G, D853E, 11057V, Y1131C, D1135N, D1180G, G1218S, E1219V, Q1221H, P1249S, P1321S, D1332G, R1335L |
| M631L, R654L, R664K, R753G, I1057V, R1114G, Y1131C, D1135N, D1180G, G1218S, E1219V, Q1221H, P1249S, P1321S, D1332G, R1335L |

| |
|---|
| (i) The above description of various napDNAbps which can be used in connection with the presently disclose base editors is not meant to be limiting in any way. The base editors may comprise the canonical SpCas9, or any ortholog Cas9 protein, or any variant Cas9 protein-including any naturally occurring variant, mutant, or otherwise engineered version of Cas9-that is known or which can be made or evolved through a directed evolutionary or otherwise mutagenic process. In various embodiments, the Cas9 or Cas9 varants have a nickase activity, i.e., only cleave of strand of the target DNA sequence. In other embodiments, the Cas9 or Cas9 variants have inactive nucleases, i.e., are "dead" Cas9 proteins. Other variant Cas9 proteins that may be used are those having a smaller molecular weight than the canonical SpCas9 (e.g., for easier delivery) or having modified or rearranged primary amino acid structure (e.g., the circular permutant formats). The base editors described herein may also comprise Cas9 equivalents, including Cas12a/Cpf1 and Cas12b proteins which are the result of convergent evolution. The napDNAbps used herein (e.g., SpCas9, Cas9 variant, or Cas9 equivalents) may also may also contain various modifications that alter/enhance their PAM specifities. Lastly, the application contemplates any Cas9, Cas9 variant, or Cas9 equivalent which has at least 70%, at least 75%, at least 80%, at least 85%, at least 90%, at least 91%, at least 92%, at least 93%, at least 94%, at least 95%, at least 96%, at |

least 97%, at least 98%, at least 99%, or at least 99.9% sequence identity to a reference Cas9 sequence, such as a references SpCas9 canonical sequences or a reference Cas9 equivalent (e.g., Cas12a/Cpf1).

In a particular embodiment, the Cas9 variant having expanded PAM capabilities is SpCas9 (H840A) VRQR, having the following amino acid sequence (with the V, R, Q, R substitutions relative to the SpCas9 (H840A) of SEQ ID NO: 42 show in bold underline. In addition, the methionine residue in SpCas9 (H840) was removed for SpCas9 (H840A) VRQR) ("SpCas9-VRQR"). This SpCas9 variant possesses an altered PAM-specificity which recognizes a PAM of 5'-NGA-3' instead of the canonical PAM of 5'-NGG-3':

| |
|---|
| **SpCas9-VRQR** |
| DKKYSIGLDIGTNSVGWAVITDEYKVPSKKFKVLGNTDRHSIKKNLIGALLFDSGETAEATR LKRTARRRYTRRKNRICYLQEIFSNEMAKVDDSFFHRLEESFLVEEDKKHERHPIFGNIVDE VAYHEKYPTIYHLRKKLVDSTDKADLRLIYLALAHMIKFRGHFLIEGDLNPDNSDVDKLFIQ LVQTYNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRVNTE ITKAPLSASMIKRYDEHHQDLTLLKALVRQQLPEKYKEIFFDQSKNGYAGYIDGGASQEEFY KFIKPILEKMDGTEELLVKLNREDLLRKQRTFDNGSIPHQIHLGELHAILRRQEDFYPFLKDN REKIEKILTFRIPYYVGPLARGNSRFAWMTRKSEETITPWNFEEVVDKGASAQSFIERMTNF DKNLPNEKVLPKHSLLYEYFTVYNELTKVKYVTEGMRKPAFLSGEQKKAIVDLLFKTNRK VTVKQLKEDYFKKIECFDSVEISGVEDRFNASLGTYHDLLKIIKDKDFLDNEENEDILEDIVL TLTLFEDREMIEERLKTYAHLFDDKVMKQLKRRRYTGWGRLSRKLINGIRDKQSGKTILDF LKSDGFANRNFMQLIHDDSLTFKEDIQKAQVSGQGDSLHEHIANLAGSPAIKKGILQTVKV VDELVKVMGRHKPENIVIEMARENQTTQKGQKNSRERMKRIEEGIKELGSQILKEHPVENT QLQNEKLYLYYLQNGRDMYVDQELDINRLSDYDVDAIVPQSFLKDDSIDNKVLTRSDKNR GKSDNVPSEEVVKKMKNYWRQLLNAKLITQRKFDNLTKAERGGLSELDKAGFIKRQLVET RQITKHVAQILDSRMNTKYDENDKLIREVKVITLKSKLVSDFRKDFQFYKVREINNYHHAH DAYLNAVVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQTGGFSKE SILPKRNSDKLIARKKDWDPKKYGGFVSPTVAYSVLVVAKVEKGKSKKLKSVKELLGITIM ERSSFEKNPIDFLEAKGYKEVKKDLIIKLPKYSLFELENGRKRMLASARELQKGNELALPSK YVNFLYLASHYEKLKGSPEDNEQKQLFVEQHKHYLDEIIEQISEFSKRVILADANLDKVLSA YNKHRDKPIREQAENIIHLFTLTNLGAPAAFKYFDTTIDRKQYRSTKEVLDATLIHQSITGLY ETRIDLSQLGGD (SEQ ID NO: 74) |

In another particular embodiment, the Cas9 variant having expanded PAM capabilities is SpCas9 (H840A) VQR, having the following amino acid sequence (with the V, Q, R substitutions relative to the SpCas9 (H840A) of SEQ ID NO: 42 show in bold underline. In addition, the methionine residue in SpCas9 (H840) was removed for SpCas9 (H840A) VRQR) ("SpCas9-VQR"). This SpCas9 variant possesses an altered PAM-specificity which recognizes a PAM of 5'-NGA-3' instead of the canonical PAM of 5'-NGG-3':

| |
|---|
| **SpCas9-VQR** |
| DKKYSIGLDIGTNSVGWAVITDEYKVPSKKFKVLGNTDRHSIKKNLIGALLFDSGETAEATR LKRTARRRYTRRKNRICYLQEIFSNEMAKVDDSFFHRLEESFLVEEDKKHERHPIFGNIVDE VAYHEKYPTIYHLRKKLVDSTDKADLRLIYLALAHMIKFRGHFLIEGDLNPDNSDVDKLFIQ LVQTYNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRVNTE ITKAPLSASMIKRYDEHHQDLTLLKALVRQQLPEKYKEIFFDQSKNGYAGYIDGGASQEEFY KFIKPILEKMDGTEELLVKLNREDLLRKQRTFDNGSIPHQIHLGELHAILRRQEDFYPFLKDN REKIEKILTFRIPYYVGPLARGNSRFAWMTRKSEETITPWNFEEVVDKGASAQSFIERMTNF DKNLPNEKVLPKHSLLYEYFTVYNELTKVKYVTEGMRKPAFLSGEQKKAIVDLLFKTNRK VTVKQLKEDYFKKIECFDSVEISGVEDRFNASLGTYHDLLKIIKDKDFLDNEENEDILEDIVL TLTLFEDREMIEERLKTYAHLFDDKVMKQLKRRRYTGWGRLSRKLINGIRDKQSGKTILDF LKSDGFANRNFMQLIHDDSLTFKEDIQKAQVSGQGDSLHEHIANLAGSPAIKKGILQTVKV VDELVKVMGRHKPENIVIEMARENQTTQKGQKNSRERMKRIEEGIKELGSQILKEHPVENT QLQNEKLYLYYLQNGRDMYVDQELDINRLSDYDVDAIVPQSFLKDDSIDNKVLTRSDKNR GKSDNVPSEEVVKKMKNYWRQLLNAKLITQRKFDNLTKAERGGLSELDKAGFIKRQLVET RQITKHVAQILDSRMNTKYDENDKLIREVKVITLKSKLVSDFRKDFQFYKVREINNYHHAH DAYLNAVVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQTGGFSKE SILPKRNSDKLIARKKDWDPKKYGGFVSPTVAYSVLVVAKVEKGKSKKLKSVKELLGITIM ERSSFEKNPIDFLEAKGYKEVKKDLIIKLPKYSLFELENGRKRMLASAGELQKGNELALPSK YVNFLYLASHYEKLKGSPEDNEQKQLFVEQHKHYLDEIIEQISEFSKRVILADANLDKVLSA YNKHRDKPIREQAENIIHLFTLTNLGAPAAFKYFDTTIDRKQYRSTKEVLDATLIHQSITGLY ETRIDLSQLGGD (SEQ ID NO: 75) |

In another particular embodiment, the Cas9 variant having expanded PAM capabilities is SpCas9 (H840A) VRER, having the following amino acid sequence (with the V, R, E, R substitutions relative to the SpCas9 (H840A) of SEQ ID NO: 42 are shown in bold underline. In addition, the methionine residue in SpCas9 (H840) was removed for SpCas9 (H840A) VRER) ("SpCas9-VRER"). This SpCas9 variant possesses an altered PAM-specificity which recognizes a PAM of 5'-NGCG-3' instead of the canonical PAM of 5'-NGG-3':

| |
|---|
| **SpCas9-VRER** |
| DKKYSIGLDIGTNSVGWAVITDEYKVPSKKFKVLGNTDRHSIKKNLIGALLFDSGETAEATR LKRTARRRYTRRKNRICYLQEIFSNEMAKVDDSFFHRLEESFLVEEDKKHERHPIFGNIVDE VAYHEKYPTIYHLRKKLVDSTDKADLRLIYLALAHMIKFRGHFLIEGDLNPDNSDVDKLFIQ LVQTYNQLFEENPINASGVDAKAILSARLSKSRRLENLIAQLPGEKKNGLFGNLIALSLGLTP NFKSNFDLAEDAKLQLSKDTYDDDLDNLLAQIGDQYADLFLAAKNLSDAILLSDILRVNTE ITKAPLSASMIKRYDEHHQDLTLLKALVRQQLPEKYKEIFFDQSKNGYAGYIDGGASQEEFY KFIKPILEKMDGTEELLVKLNREDLLRKQRTFDNGSIPHQIHLGELHAILRRQEDFYPFLKDN REKIEKILTFRIPYYVGPLARGNSRFAWMTRKSEETITPWNFEEVVDKGASAQSFIERMTNF DKNLPNEKVLPKHSLLYEYFTVYNELTKVKYVTEGMRKPAFLSGEQKKAIVDLLFKTNRK VTVKQLKEDYFKKIECFDSVEISGVEDRFNASLGTYHDLLKIIKDKDFLDNEENEDILEDIVL TLTLFEDREMIEERLKTYAHLFDDKVMKQLKRRRYTGWGRLSRKLINGIRDKQSGKTILDF LKSDGFANRNFMQLIHDDSLTFKEDIQKAQVSGQGDSLHEHIANLAGSPAIKKGILQTVKV VDELVKVMGRHKPENIVIEMARENQTTQKGQKNSRERMKRIEEGIKELGSQILKEHPVENT QLQNEKLYLYYLQNGRDMYVDQELDINRLSDYDVDAIVPQSFLKDDSIDNKVLTRSDKNR GKSDNVPSEEVVKKMKNYWRQLLNAKLITQRKFDNLTKAERGGLSELDKAGFIKRQLVET RQITKHVAQILDSRMNTKYDENDKLIREVKVITLKSKLVSDFRKDFQFYKVREINNYHHAH DAYLNAVVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQTGGFSKE SILPKRNSDKLIARKKDWDPKKYGGFVSPTVAYSVLVVAKVEKGKSKKLKSVKELLGITIM ERSSFEKNPIDFLEAKGYKEVKKDLIIKLPKYSLFELENGRKRMLASARELQKGNELALPSK YVNFLYLASHYEKLKGSPEDNEQKQLFVEQHKHYLDEIIEQISEFSKRVILADANLDKVLSA YNKHRDKPIREQAENIIHLFTLTNLGAPAAFKYFDTTIDRKEYRSTKEVLDATLIHQSITGLY ETRIDLSQLGGD (SEQ ID NO: 76) |

In yet particular embodiment, the Cas9 variant having expanded PAM capabilities is SpCas9-NG, as reported in Nishimasu et al., "Engineered CRISPR-Cas9 nuclease with expanded targeting space," Science, 2018, 361: 1259-1262, which is incorporated herein by reference. SpCas9-NG (VRVRFRR), having the following amino acid sequence substitutions: R1335V, L1111R, D1135V, G1218R, E1219F, A1322R, and T1337R relative to the canonical SpCas9 sequence (SEQ ID NO: 5. This SpCas9 has a relaxed PAM specificity, i.e., with activity on a PAM of NGH (wherein H = A, T, or C). See Nishimasu et al., "Engineered CRISPR-Cas9 nuclease with expanded targeting space," Science, 2018, 361: 1259-1262, which is incorporated herein by reference.

| |
|---|
| **SpCas9-NG** |
| MDKKYSIGLDIGTNSVGWAVITDEYKVPSKKFKVLGNTDRHSIKKNLIGALLFDSGETAEA TRLKRTARRRYTRRKNRICYLQEIFSNEMAKVDDSFFHRLEESFLVEEDKKHERHPIFGNIV DEVAYHEKYPTIYHLRKKLVDSTDKADLRLIYLALAHMIKFRGHFLIEGDLNPDNSDVDKL FIQLVQTYNQLFEENPINASGVDAKAILSARLSKSRRLENLIAQLPGEKKNGLFGNLIALSLG LTPNFKSNFDLAEDAKLQLSKDTYDDDLDNLLAQIGDQYADLFLAAKNLSDAILLSDILRV NTEITKAPLSASMIKRYDEHHQDLTLLKALVRQQLPEKYKEIFFDQSKNGYAGYIDGGASQ EEFYKFIKPILEKMDGTEELLVKLNREDLLRKQRTFDNGSIPHQIHLGELHAILRRQEDFYPF LKDNREKIEKILTFRIPYYVGPLARGNSRFAWMTRKSEETITPWNFEEVVDKGASAQSFIER MTNFDKNLPNEKVLPKHSLLYEYFTVYNELTKVKYVTEGMRKPAFLSGEQKKAIVDLLFK TNRKVTVKQLKEDYFKKIECFDSVEISGVEDRFNASLGTYHDLLKIIKDKDFLDNEENEDIL EDIVLTLTLFEDREMIEERLKTYAHLFDDKVMKQLKRRRYTGWGRLSRKLINGIRDKQSG KTILDFLKSDGFANRNFMQLIHDDSLTFKEDIQKAQVSGQGDSLHEHIANLAGSPAIKKGIL QTVKVVDELVKVMGRHKPENIVIEMARENQTTQKGQKNSRERMKRIEEGIKELGSQILKE HPVENTQLQNEKLYLYYLQNGRDMYVDQELDINRLSDYDVDHIVPQSFLKDDSIDNKVLT RSDKNRGKSDNVPSEEVVKKMKNYWRQLLNAKLITQRKFDNLTKAERGGLSELDKAGFI KRQLVETRQITKHVAQILDSRMNTKYDENDKLIREVKVITLKSKLVSDFRKDFQFYKVREI NNYHHAHDAYLNAVVGTALIKKYPKLESEFVYGDYKVYDVRKMIAKSEQEIGKATAKYF FYSNIMNFFKTEITLANGEIRKRPLIETNGETGEIVWDKGRDFATVRKVLSMPQVNIVKKTE VQTGGFSKESIRPKRNSDKLIARKKDWDPKKYGGFVSPTVAYSVLVVAKVEKGKSKKLKS VKELLGITIMERSSFEKNPIDFLEAKGYKEVKKDLIIKLPKYSLFELENGRKRMLASARFLQ KGNELALPSKYVNFLYLASHYEKLKGSPEDNEQKQLFVEQHKHYLDEIIEQISEFSKRVILA DANLDKVLSAYNKHRDKPIREQAENIIHLFTLTNLGAPRAFKYFDTTIDRKVYRSTKEVLD ATLIHQSITGLYETRIDLSQLGGD (SEQ ID NO: 77) |

In addition, any available methods may be utilized to obtain or construct a variant or mutant Cas9 protein. The term "mutation," as used herein, refers to a substitution of a residue within a sequence, e.g., a nucleic acid or amino acid sequence, with another residue, or a deletion or insertion of one or more residues within a sequence. Mutations are typically described herein by identifying the original residue followed by the position of the residue within the sequence and by the identity of the newly substituted residue. Various methods for making the amino acid substitutions (mutations) provided herein are well known in the art, and are provided by, for example, Green and Sambrook, Molecular Cloning: A Laboratory Manual (4th ed., Cold Spring Harbor Laboratory Press, Cold Spring Harbor, N.Y. (2012)). Mutations can include a variety of categories, such as single base polymorphisms, microduplication regions, indel, and inversions, and is not meant to be limiting in any way. Mutations can include "loss-of-function" mutations which is the normal result of a mutation that reduces or abolishes a protein activity. Most loss-of-function mutations are recessive, because in a heterozygote the second chromosome copy carries an unmutated version of the gene coding for a fully functional protein whose presence compensates for the effect of the mutation. Mutations also embrace "gain-of-function" mutations, which is one which confers an abnormal activity on a protein or cell that is otherwise not present in a normal condition. Many gain-of-function mutations are in regulatory sequences rather than in coding regions, and can therefore have a number of consequences. For example, a mutation might lead to one or more genes being expressed in the wrong tissues, these tissues gaining functions that they normally lack. Because of their nature, gain-of-function mutations are usually dominant.

Mutations can be introduced into a reference Cas9 protein using site-directed mutagenesis. Older methods of site-directed mutagenesis known in the art rely on sub-cloning of the sequence to be mutated into a vector, such as an M13 bacteriophage vector, that allows the isolation of single-stranded DNA template. In these methods, one anneals a mutagenic primer (i.e., a primer capable of annealing to the site to be mutated but bearing one or more mismatched nucleotides at the site to be mutated) to the single-stranded template and then polymerizes the complement of the template starting from the 3' end of the mutagenic primer. The resulting duplexes are then transformed into host bacteria and plaques are screened for the desired mutation. More recently, site-directed mutagenesis has employed PCR methodologies, which have the advantage of not requiring a single-stranded template. In addition, methods have been developed that do not require sub-cloning. Several issues must be considered when PCR-based site-directed mutagenesis is performed. First, in these methods it is desirable to reduce the number of PCR cycles to prevent expansion of undesired mutations introduced by the polymerase. Second, a selection must be employed in order to reduce the number of non-mutated parental molecules persisting in the reaction. Third, an extended-length PCR method is preferred in order to allow the use of a single PCR primer set. And fourth, because of the non-template-dependent terminal extension activity of some thermostable polymerases it is often necessary to incorporate an end-polishing step into the procedure prior to blunt-end ligation of the PCR-generated mutant product.

Mutations may also be introduced by directed evolution processes, such as phage-assisted continuous evolution (PACE) or phage-assisted noncontinuous evolution (PANCE). The term "phage-assisted continuous evolution (PACE)," as used herein, refers to continuous evolution that employs phage as viral vectors. The general concept of PACE technology has been described, for example, in International PCT Application, PCT/US2009/056194, filed September 8, 2009, published as WO 2010/028347 on March 11, 2010; International PCT Application, PCT/US2011/066747, filed December 22, 2011, published as WO 2012/088381 on June 28, 2012; U.S. Application, U.S. Patent No. 9,023,594, issued May 5, 2015, International PCT Application, PCT/US2015/012022, filed January 20, 2015, published as WO 2015/134121 on September 11, 2015, and International PCT Application, PCT/US2016/027795, filed April 15, 2016, published as WO 2016/168631 on October 20, 2016, the entire contents of each of which are incorporated herein by reference. Variant Cas9s may also be obtain by phage-assisted non-continuous evolution (PANCE)," which as used herein, refers to non-continuous evolution that employs phage as viral vectors. PANCE is a simplified technique for rapid in vivo directed evolution using serial flask transfers of evolving 'selection phage' (SP), which contain a gene of interest to be evolved, across fresh *E. coli* host cells, thereby allowing genes inside the host *E. coli* to be held constant while genes contained in the SP continuously evolve. Serial flask transfers have long served as a widely-accessible approach for laboratory evolution of microbes, and, more recently, analogous approaches have been developed for bacteriophage evolution. The PANCE system features lower stringency than the PACE system.

Any of the references noted above which relate to Cas9 or Cas9 equivalents are hereby incorporated by reference in their entireties, if not already stated so.

### III. Adenosine deaminases

In some embodiments, the disclosure provides base editors that comprise one or more adenosine deaminase domains. In some aspects, any of the disclosed base editors are capable of deaminating adenosine in a nucleic acid sequence (*e.g.,* DNA or RNA). As one example, any of the base editors provided herein may be base editors, (*e.g.,* adenine base editors). Without wishing to be bound by any particular theory, dimerization of adenosine deaminases (*e.g.,* in cis or in trans) may improve the ability (*e.g.,* efficiency) of the base editor to modify a nucleic acid base, for example to deaminate adenine.

Exemplary, non-limiting, embodiments of adenosine deaminases are provided herein. In some embodiments, the adenosine deaminase domain of any of the disclosed base editors comprises a single adenosine deaminase, or a monomer. In some embodiments, the adenosine deaminase domain comprises 2, 3, 4 or 5 adenosine deaminases. In some embodiments, the adenosine deaminase domain comprises two adenosine deaminases, or a dimer. In some embodiments, the deaminase domain comprises a dimer of an engineered (or evolved) deaminase and a wild-type deaminase, such as a wild-type *E. coli* deaminase. It should be appreciated that the mutations provided herein (e.g., mutations in ecTadA) may be applied to adenosine deaminases in other adenosine base editors, for example those provided in International Publication No. WO 2018/027078, published August 2, 2018; International Application No PCT/US2019/033848, filed May 23, 2019, which published as International Publication No. WO 2019/226593 on November 28, 2019; U.S. Patent Publication No. 2018/0073012, published March 15, 2018, which issued as U.S. Patent No. 10,113,163, on October 30, 2018; U.S. Patent Publication No. 2017/0121693, published May 4, 2017, which issued as U.S. Patent No. 10,167,457 on January 1, 2019; International Publication No. WO 2017/070633, published April 27, 2017; U.S. Patent Publication No. 2015/0166980, published June 18, 2015; U.S. Patent No. 9,840,699, issued December 12, 2017; and U.S. Patent No. 10,077,453, issued September 18, 2018, and U.S. Provisional Application No. 62/835,490, filed April 17, 2019; all of which are incorporated herein by reference in their entireties.

In some embodiments, any of the adenosine deaminases provided herein are capable of deaminating adenine, *e.g.,* deaminating adenine in a deoxyadenosine residue of DNA. The adenosine deaminase may be derived from any suitable organism (*e.g., E. coli*). In some embodiments, the adenosine deaminase is a naturally-occurring adenosine deaminase that includes one or more mutations corresponding to any of the mutations provided herein (*e.g.,* mutations in ecTadA). One of skill in the art will be able to identify the corresponding residue in any homologous protein and in the respective encoding nucleic acid by methods well known in the art, *e.g.,* by sequence alignment and determination of homologous residues. Accordingly, one of skill in the art would be able to generate mutations in any naturally-occurring adenosine deaminase (*e.g.,* having homology to ecTadA) that corresponds to any of the mutations described herein, *e.g.,* any of the mutations identified in ecTadA. In some embodiments, the adenosine deaminase is derived from a prokaryote. In some embodiments, the adenosine deaminase is from a bacterium. In some embodiments, the adenosine deaminase is from *Escherichia coli, Staphylococcus aureus, Salmonella typhi, Shewanella putrefaciens, Haemophilus influenzae, Caulobacter crescentus,* or *Bacillus subtilis.* In some embodiments, the adenosine deaminase is from *E. coli.*

In some embodiments, the adenosine deaminase may comprise one or more substitutions that include R26G, V69A, V88A, A109S, T111R, D119N, H122N, Y147D, F149Y, T166I, D167N relative to TadA7.10 (SEQ ID NO: 79), or a substitution at a corresponding amino acid in another adenosine deaminase. In some embodiments, the adenosine deaminase comprises T111R, D119N, and F149Y substitutions in TadA7.10 (SEQ ID NO: 79), or a corresponding mutation in another adenosine deaminase. In particular embodiments, the adenosine deaminase comprises T111R, D119N, and F149Y substitutions, and further comprises at least one substitution selected from R26C, V88A, A109S, H122N, T166I, and D167N, in TadA7.10 (SEQ ID NO: 79), or a corresponding mutation in another adenosine deaminase.

In some embodiments, the adenosine deaminase comprises A109S, T111R, D119N, H122N, F149Y, T166I, and D167N substitutions in TadA7.10 (SEQ ID NO: 79), or a corresponding mutation in another adenosine deaminase. In some embodiments, the adenosine deaminase comprises R26C, D108W, T111R, D119N, and F149Y substitutions in TadA7.10 (SEQ ID NO: 79), or a corresponding mutation in another adenosine deaminase. In some embodiments, the adenosine deaminase comprises V88A, D108W, T111R, D119N, and F149Y substitutions in TadA7.10 (SEQ ID NO: 79), or a corresponding mutation in another adenosine deaminase. In some embodiments, the adenosine deaminase further comprises a Y147D substitution in TadA7.10 (SEQ ID NO: 79), or a corresponding mutation in another adenosine deaminase.

In some embodiments, the adenosine deaminase comprises A109S, T111R, D119N, H122N, Y147D, F149Y, T166I and D167N substitutions in TadA7.10 (SEQ ID NO: 79), or a corresponding mutation in another adenosine deaminase. In some embodiments, the adenosine deaminase comprises TadA-8e. In some embodiments, the adenosine deaminase comprises A109S, T111R, D119N, H122N, Y147D, F149Y, T166I and D167N in TadA7.10 (SEQ ID NO: 79), or a corresponding mutation in another adenosine deaminase. In some embodiments, the adenosine deaminase further comprises at least one substitution selected from K20A, R21A, V82G, and V106W in TadA7.10 (SEQ ID NO: 79), or a corresponding mutation in another adenosine deaminase. In certain embodiments, the adenosine deaminase comprises V106W, A109S, T111R, D119N, H122N, Y147D, F149Y, T166I and D167N substitutions in TadA7.10 (SEQ ID NO: 79), or a corresponding mutation in another adenosine deaminase. In some embodiments, the adenosine deaminase comprises TadA-8e(V106W). It should be appreciated, however, that additional deaminases may similarly be aligned to identify homologous amino acid residues that may be mutated as provided herein.

It should be appreciated that any of the mutations provided herein (e.g., based on the ecTadA amino acid sequence of SEQ ID NO: 78) may be introduced into other adenosine deaminases, such as *S. aureus* TadA (saTadA), or other adenosine deaminases (*e.g.,* bacterial adenosine deaminases), such as those sequences provided below. It would be apparent to the skilled artisan how to identify amino acid residues from other adenosine deaminases that are homologous to the mutated residues in ecTadA. Thus, any of the mutations identified in ecTadA may be made in other adenosine deaminases that have homologous amino acid residues. It should also be appreciated that any of the mutations provided herein may be made individually or in any combination in ecTadA or another adenosine deaminase.

Exemplary adenosine deaminase variants of the disclosure are described below. In certain embodiments, the adenosine deaminase domain comprises an adenosine deaminase that has a sequence with at least 80%, at least 85%, at least 90%, at least 95%, at least 98%, at least 99%, or at least 99.5% sequence identity to one of the following:
***E. coli* TadA** 
MSEVEFSHEYWMRHALTLAKRAWDEREVPVGAVLVHNNRVIGEGWNRPIGRHDPT
AHAEIMALRQGGLVMQNYRLIDATLYVTLEPCVMCAGAMIHSRIGRVVFGARDAKT
GAAGSLMDVLHHPGMNHRVEITEGILADECAALLSDFFRMRRQEIKAQKKAQSSTD
(SEQ ID NO: 78)
***E. coli* TadA 7.10** 
MSEVEFSHEYWMRHALTLAKRARDEREVPVGAVLVLNNRVIGEGWNRAIGLHDPT
AHAEIMALRQGGLVMQNYRLIDATLYVTFEPCVMCAGAMIHSRIGRVVFGVRNAKT
GAAGSLMDVLHYPGMNHRVEITEGILADECAALLCYFFRMPRQVFNAQKKAQSSTD
(SEQ ID NO: 79)
***E. coli* TadA* 7.10** 
SEVEFSHEYWMRHALTLAKRARDEREVPVGAVLVLNNRVIGEGWNRAIGLHDPTA
HAEIMALRQGGLVMQNYRLIDATLYVTFEPCVMCAGAMIHSRIGRVVFGVRNAKTG
AAGSLMDVLHYPGMNHRVEITEGILADECAALLCYFFRMPRQVFNAQKKAQSSTD
(SEQ ID NO: 403)
**ABE7.10 TadA* monomer**
   DNA sequence 
TCTGAGGTGGAGTTTTCCCACGAGTACTGGATGAGACATGCCCTGACCCTGGCCA
AGAGGGCACGCGATGAGAGGGAGGTGCCTGTGGGAGCCGTGCTGGTGCTGAACA
ATAGAGTGATCGGCGAGGGCTGGAACAGAGCCATCGGCCTGCACGACCCAACAG
CCCATGCCGAAATTATGGCCCTGAGACAGGGCGGCCTGGTCATGCAGAACTACA
GACTGATTGACGCCACCCTGTACGTGACATTCGAGCCTTGCGTGATGTGCGCCGG
CGCCATGATCCACTCTAGGATCGGCCGCGTGGTGTTTGGCGTGAGGAACGCAAA
AACCGGCGCCGCAGGCTCCCTGATGGACGTGCTGCACTACCCCGGCATGAATCA
CCGCGTCGAAATTACCGAGGGAATCCTGGCAGATGAATGTGCCGCCCTGCTGTG
CTATTTCTTTCGGATGCCTAGACAGGTGTTCAATGCTCAGAAGAAGGCCCAGAGC
TCCACCGAC (SEQ ID NO: 404)
***E. coli* TadA 7.10 (V106W)** 
MSEVEFSHEYWMRHALTLAKRARDEREVPVGAVLVLNNRVIGEGWNRAIGLHDPT
AHAEIMALRQGGLVMQNYRLIDATLYVTFEPCVMCAGAMIHSRIGRVVFGWRNAK
TGAAGSLMDVLHYPGMNHRVEITEGILADECAALLCYFFRMPRQVFNAQKKAQSST
D (SEQ ID NO: 80)
***Staphylococcus aureus* TadA** 
MGSHMTNDIYFMTLAIEEAKKAAQLGEVPIGAIITKDDEVIARAHNLRETLQQ
PTAHAEHIAIERAAKVLGSWRLEGCTLYVTLEPCVMCAGTIVMSRIPRVVYGADDPK
GGCSGSLMNLLQQSNFNHRAIVDKGVLKEACSTLLTTFFKNLRANKKSTN (SEQ ID
NO: 81)
***Bacillus subtilis* TadA** 
MTQDELYMKEAIKEAKKAEEKGEVPIGAVLVINGEIIARAHNLRETEQRSIAHAEML
VIDEACKALGTWRLEGATLYVTLEPCPMCAGAVVLSRVEKVVFGAFDPKGGCSGTL
MNLLQEERFNHQAEVVSGVLEEECGGMLSAFFRELRKKKKAARKNLSE (SEQ ID
NO: 82)
***Salmonella typhimurium* TadA** 
MPPAFITGVTSLSDVELDHEYWMRHALTLAKRAWDEREVPVGAVLVHNHRVIGEG
WNRPIGRHDPTAHAEIMALRQGGLVLQNYRLLDTTLYVTLEPCVMCAGAMVHSRIG
RVVFGARDAKTGAAGSLIDVLHHPGMNHRVEIIEGVLRDECATLLSDFFRMRRQEIK
ALKKADRAEGAGPAV (SEQ ID NO: 83)
***Shewanella putrefaciens* TadA** 
MDEYWMQVAMQMAEKAEAAGEVPVGAVLVKDGQQIATGYNLSISQHDPTAHAEI
LCLRSAGKKLENYRLLDATLYITLEPCAMCAGAMVHSRIARVVYGARDEKTGAAGT
VVNLLQHPAFNHQVEVTSGVLAEACSAQLSRFFKRRRDEKKALKLAQRAQQGIE
(SEQ ID NO: 84)
***Haemophilus influenzae* F3031 TadA** 
MDAAKVRSEFDEKMMRYALELADKAEALGEIPVGAVLVDDARNIIGEGWNLSIVQS
DPTAHAEIIALRNGAKNIQNYRLLNSTLYVTLEPCTMCAGAILHSRIKRLVFGASDYK
TGAIGSRFHFFDDYKMNHTLEITSGVLAEECSQKLSTFFQKRREEKKIEKALLKSLSD
K (SEQ ID NO: 85)
***Caulobacter crescentus* TadA** 
MRTDESEDQDHRMMRLALDAARAAAEAGETPVGAVILDPSTGEVIATAGNGPIAAH
DPTAHAEIAAMRAAAAKLGNYRLTDLTLVVTLEPCAMCAGAISHARIGRVVFGADD
 
PKGGAVVHGPKFFAQPTCHWRPEVTGGVLADESADLLRGFFRARRKAKI (SEQ ID
NO: 86)
***Geobacter sulfurreducens* TadA**

MSSLKKTPIRDDAYWMGKAIREAAKAAARDEVPIGAVIVRDGAVIGRGHNLREGSN
DPSAHAEMIAIRQAARRSANWRLTGATLYVTLEPCLMCMGAIILARLERVVFGCYDP
KGAAGSLYDLSADPRLNHQVRLSPGVCQEECGTMLSDFFRDLRRRKKAKATPALFI
DERKVPPEP (SEQ ID NO: 87)

In some embodiments, the adenosine deaminase domain comprises an N-terminal truncated *E. coli* TadA. In certain embodiments, the adenosine deaminase comprises the amino acid sequence:

MSEVEFSHEYWMRHALTLAKRAWDEREVPVGAVLVHNNRVIGEGWNRPIGRHDPT
AHAEIMALRQGGLVMQNYRLIDATLYVTLEPCVMCAGAMIHSRIGRVVFGARDAKT
GAAGSLMDVLHHPGMNHRVEITEGILADECAALLSDFFRMRRQEIKAQKKAQSSTD
(SEQ ID NO: 88).

In some embodiments, the TadA deaminase is a full-length *E. coli* TadA deaminase (ecTadA). For example, in certain embodiments, the adenosine deaminase domain comprises a deaminase that comprises the amino acid sequence: 
MRRAFITGVFFLSEVEFSHEYWMRHALTLAKRAWDEREVPVGAVLVHNNRVIGEG
WNRPIGRHDPTAHAEIMALRQGGLVMQNYRLIDATLYVTLEPCVMCAGAMIHSRIG
RVVFGARDAKTGAAGSLMDVLHHPGMNHRVEITEGILADECAALLSDFFRMRRQEI
KAQKKAQSSTD (SEQ ID NO: 89)
**ABE8 TadA*** monomer
   DNA sequence 
TCTGAGGTGGAGTTTTCCCACGAGTACTGGATGAGACATGCCCTGACCCTGGCCA
AGAGGGCACGGGATGAGAGGGAGGTGCCTGTGGGAGCCGTGCTGGTGCTGAAC
AATAGAGTGATCGGCGAGGGCTGGAACAGAGCCATCGGCCTGCACGACCCAACA
GCCCATGCCGAAATTATGGCCCTGAGACAGGGCGGCCTGGTCATGCAGAACTAC
AGACTGATTGACGCCACCCTGTACGTGACATTCGAGCCTTGCGTGATGTGCGCCG
GCGCCATGATCCACTCTAGGATCGGCCGCGTGGTGTTTGGCGTGAGGAACTCAA
AAAGAGGCGCCGCAGGCTCCCTGATGAACGTGCTGAACTACCCCGGCATGAATC
ACCGCGTCGAAATTACCGAGGGAATCCTGGCAGATGAATGTGCCGCCCTGCTGT
GCGATTTCTATCGGATGCCTAGACAGGTGTTCAATGCTCAGAAGAAGGCCCAGA
GCTCCATCAAC (SEQ ID NO: 90)
**ABE8 TadA* monomer**
   Amino Acid Sequence

SEVEFSHEYWMRHALTLAKRARDEREVPVGAVLVLNNRVIGEGWNRAIGLHDPTA
HAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSIN
(SEQ ID NO: 91)

In other aspects, the disclosure provides adenine base editors with broadened target sequence compatibility. In general, native ecTadA deaminates the adenine in the sequence UAC (e.g., the target sequence) of the anticodon loop of tRNA^{Arg}. Without wishing to be bound by any particular theory, in order to expand the utility of ABEs comprising one or more ecTadA deaminases, such as any of the adenosine deaminases provided herein, the adenosine deaminase proteins were optimized to recognize a wide variety of target sequences within the protospacer sequence without compromising the editing efficiency of the adenosine nucleobase editor complex. In some embodiments, the target sequence is an A in the center of a 5'-NAN-3' sequence, wherein N is T, C, G, or A. In some embodiments, the target sequence comprises 5'-TAC-3'. In some embodiments, the target sequence comprises 5'-GAA-3'.

Any two or more of the adenosine deaminases described herein may be connected to one another (e.g., by a linker) within an adenosine deaminase domain of the base editors provided herein. For instance, the base editors provided herein may contain only two adenosine deaminases. In some embodiments, the adenosine deaminases are the same. In some embodiments, the adenosine deaminases are any of the adenosine deaminases provided herein. In some embodiments, the adenosine deaminases are different. In some embodiments, the first adenosine deaminase is any of the adenosine deaminases provided herein, and the second adenosine is any of the adenosine deaminases provided herein, but is not identical to the first adenosine deaminase. In some embodiments, the base editor comprises two adenosine deaminases (*e.g*., a first adenosine deaminase and a second adenosine deaminase). In some embodiments, the base editor comprises a first adenosine deaminase and a second adenosine deaminase. In some embodiments, the first adenosine deaminase is N-terminal to the second adenosine deaminase in the base editor. In some embodiments, the first adenosine deaminase is C-terminal to the second adenosine deaminase in the base editor. In some embodiments, the first adenosine deaminase and the second deaminase are fused directly or via a linker.

In some embodiments, the adenosine deaminase domain comprises an adenosine deaminase that comprises an amino acid sequence that is at least 60%, at least 65%, at least 70%, at least 75%, at least 80%, at least 85%, at least 90%, at least 95%, at least 96%, at least 97%, at least 98%, at least 99%, or at least 99.5% identical to any one of the amino acid sequences set forth in any one of SEQ ID NOs: 78-91, or to any of the adenosine deaminases provided herein. In certain embodiments, the adenosine deaminase comprises an amino acid sequence that is at least 80%, at least 85%, at least 90%, at least 95%, at least 96%, at least 97%, at least 98%, at least 99%, or at least 99.5% identical to the amino acid sequence of TadA7.10 (SEQ ID NO: 403). It should be appreciated that adenosine deaminases provided herein may include one or more mutations (*e.g*., any of the mutations provided herein). The disclosure provides adenosine deaminases with a certain percent identiy plus any of the mutations or combinations thereof described herein. In some embodiments, the adenosine deaminase comprises an amino acid sequence that has 1, 2, 3, 4, 5, 6, 7, 8, 9, 10, 11, 12, 13, 14, 15, 16, 17, 18, 19, 20, 21, 22, 21, 24, 25, 26, 27, 28, 29, 30, 31, 32, 33, 34, 35, 36, 37, 38, 39, 40, 41, 42, 43, 44, 45, 46, 47, 48, 49, 50, or more mutations compared to any one of the amino acid sequences set forth in SEQ ID NOs: 78-91, and 403-404 *(e.g.,* TadA7.10), or any of the adenosine deaminases provided herein. In some embodiments, the adenosine deaminase comprises an amino acid sequence that has at least 5, at least 10, at least 15, at least 20, at least 25, at least 30, at least 35, at least 40, at least 45, at least 50, at least 60, at least 70, at least 80, at least 90, at least 100, at least 110, at least 120, at least 130, at least 140, at least 150, at least 160, or at least 170 identical contiguous amino acid residues as compared to any one of the amino acid sequences set forth in SEQ ID NOs: 78-91, and 403-*404 (e.g.,* TadA7.10), or any of the adenosine deaminases provided herein.

In some embodiments, the adenosine deaminase comprises TadA 7.10, whose sequence is set forth as SEQ ID NO: 79, or a variant thereof. TadA7.10 comprises the following mutations in wild-type ecTadA: W23R, H36L, P48A, R51L, L84F, A106V, D108N, H123Y, S146C, D147Y, R152P, E155V, I156F, and K157N.

In some embodiments, the adenosine deaminase is at least 50%, at least 55%, at least 60%, at least 65%, at least 70%, at least 75% at least 80%, at least 85%, at least 90%, at least 95%, at least 96%, at least 97%, at least 98%, at least 99%, or at least 99.5% identical to a naturally-occurring adenosine deaminase, e.g., *E. coli* TadA 7.10 of SEQ ID NO: 79. In some embodiments, the adenosine deaminase is from a bacterium, such as, *E.coli, S. aureus, S. typhi, S. putrefaciens, H. influenzae,* or *C*. *crescentus.* In some embodiments, the adenosine deaminase is a TadA deaminase. In some embodiments, the TadA deaminase is an *E. coli* TadA deaminase (ecTadA). In some embodiments, the TadA deaminase is a truncated *E. coli* TadA deaminase. For example, the truncated ecTadA may be missing one or more N-terminal or C-terminal amino acids relative to a full-length ecTadA. In some embodiments, the truncated ecTadA may be missing 1, 2, 3, 4, 5 ,6, 7, 8, 9, 10, 11, 12, 13, 14, 15, 6, 17, 18, 19, or 20 N-terminal amino acid residues relative to the full length ecTadA. In some embodiments, the truncated ecTadA may be missing 1, 2, 3, 4, 5 ,6, 7, 8, 9, 10, 11, 12, 13, 14, 15, 6, 17, 18, 19, or 20 C-terminal amino acid residues relative to the full length ecTadA. In some embodiments, the ecTadA deaminase does not comprise an N-terminal methionine.

In some embodiments, the TadA 7.10 of SEQ ID NO: 79 comprises an N-terminal methionine. It should be appreciated that the amino acid numbering scheme relating to the mutations in TadA 7.10 may be based on the TadA sequence of SEQ ID NO: 78, which contains an N-terminal methionine.

In some embodiments, the adenosine deaminase comprises a D108X mutation in ecTadA SEQ ID NO: 89, or a corresponding mutation in another adenosine deaminase, where X indicates any amino acid other than the corresponding amino acid in the wild-type adenosine deaminase. In some embodiments, the adenosine deaminase comprises a D108G, D108N, D108V, D108A, or D108Y mutation in ecTadA SEQ ID NO: 89, or a corresponding mutation in another adenosine deaminase. In some embodiments, the adenosine deaminase comprises a D108N mutation in ecTadA SEQ ID NO: 89, or a corresponding mutation in another adenosine deaminase. It should be appreciated, however, that additional deaminases may similarly be aligned to identify homologous amino acid residues that can be mutated as provided herein.

In some embodiments, the adenosine deaminase comprises an A106X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an A106V mutation in ecTadA SEQ ID NO: 78, or a corresponding mutation in another adenosine deaminase.

In some embodiments, the adenosine deaminase comprises a E155X mutation in ecTadA SEQ ID NO: 78, or a corresponding mutation in another adenosine deaminase, where the presence of X indicates any amino acid other than the corresponding amino acid in the wild-type adenosine deaminase. In some embodiments, the adenosine deaminase comprises a E155D, E155G, or E155V mutation in ecTadA SEQ ID NO: 78, or a corresponding mutation in another adenosine deaminase. In some embodiments, the adenosine deaminase comprises a E155V mutation in ecTadA SEQ ID NO: 78, or a corresponding mutation in another adenosine deaminase).

In some embodiments, the adenosine deaminase comprises a D147X mutation in ecTadA SEQ ID NO: 78, or a corresponding mutation in another adenosine deaminase, where the presence of X indicates any amino acid other than the corresponding amino acid in the wild-type adenosine deaminase. In some embodiments, the adenosine deaminase comprises a D147Y mutation in ecTadA SEQ ID NO: 78, or a corresponding mutation in another adenosine deaminase.

In some embodiments, an adenosine deaminase comprises the following group of mutations (groups of mutations are separated by a ";") in ecTadA SEQ ID NO: 78, or corresponding mutations in another adenosine deaminase: D108N and A106V; D108N and E155V; D108N and D147Y; A106V and E155V; A106V and D147Y; E155V and D147Y; D108N, A106V, and E55V; D108N, A106V, and D147Y; D108N, E55V, and D147Y; A106V, E55V, and D147Y; and D108N, A106V, E55V, and D147Y. It should be appreciated, however, that any combination of corresponding mutations provided herein may be made in an adenosine deaminase (*e.g*., ecTadA). In some embodiments, an adenosine deaminase comprises one or more of the mutations provided herein, which identifies individual mutations and combinations of mutations made in ecTadA. In some embodiments, an adenosine deaminase comprises any mutation or combination of mutations provided herein.

In some embodiments, the adenosine deaminase comprises an L84X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an L84F mutation in ecTadA SEQ ID NO: 78, or a corresponding mutation in another adenosine deaminase.

In some embodiments, the adenosine deaminase comprises an H123X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an H123Y mutation in ecTadA SEQ ID NO: 78, or a corresponding mutation in another adenosine deaminase.

In some embodiments, the adenosine deaminase comprises an I156X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an I156F mutation in ecTadA SEQ ID NO: 78, or a corresponding mutation in another adenosine deaminase.

In some embodiments, the adenosine deaminase comprises one, two, three, four, five, six, or seven mutations selected from the group consisting of L84X, A106X, D108X, H123X, D147X, E155X, and I156X in ecTadA SEQ ID NO: 78, or a corresponding mutation or mutations in another adenosine deaminase, where X indicates the presence of any amino acid other than the corresponding amino acid in the wild-type adenosine deaminase.

In some embodiments, the adenosine deaminase comprises one, two, three, four, five, six, or seven mutations selected from the group consisting of L84F, A106V, D108N, H123Y, D147Y, E155V, and I156F in ecTadA SEQ ID NO: 78, or a corresponding mutation or mutations in another adenosine deaminase. In some embodiments, the adenosine deaminase comprises one, two, three, four, five, or six mutations selected from the group consisting of S2A, I49F, A106V, D108N, D147Y, and E155V in ecTadA SEQ ID NO: 78, or a corresponding mutation or mutations in another adenosine deaminase. In some embodiments, the adenosine deaminase comprises one, two, three, four, or five, mutations selected from the group consisting of H8Y, A106T, D108N, N127S, and K160S in ecTadA SEQ ID NO: 78, or a corresponding mutation or mutations in another adenosine deaminase.

In some embodiments, the adenosine deaminase comprises an A142X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an A142N, A142D, A142G, mutation in ecTadA SEQ ID NO: 78, or a corresponding mutation in another adenosine deaminase. In some embodiments, the adenosine deaminase comprises an A142N mutation in ecTadA SEQ ID NO: 78, or a corresponding mutation in another adenosine deaminase.

In some embodiments, the adenosine deaminase comprises an H36X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an H36L mutation in ecTadA SEQ ID NO: 78, or a corresponding mutation in another adenosine deaminase.

In some embodiments, the adenosine deaminase comprises an N37X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an N37T, or N37S mutation in ecTadA SEQ ID NO: 78, or a corresponding mutation in another adenosine deaminase. In some embodiments, the adenosine deaminase comprises a N37S mutation in ecTadA SEQ ID NO: 78, or a corresponding mutation in another adenosine deaminase.

In some embodiments, the adenosine deaminase comprises an P48X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an P48T, P48S, P48A, or P48L mutation in ecTadA SEQ ID NO: 78, or a corresponding mutation in another adenosine deaminase. In some embodiments, the adenosine deaminase comprises a P48T mutation in ecTadA SEQ ID NO: 78, or a corresponding mutation in another adenosine deaminase. In some embodiments, the adenosine deaminase comprises a P48S mutation in ecTadA SEQ ID NO: 78, or a corresponding mutation in another adenosine deaminase. In some embodiments, the adenosine deaminase comprises a P48A mutation in ecTadA SEQ ID NO: 78, or a corresponding mutation in another adenosine deaminase.

In some embodiments, the adenosine deaminase comprises an R51X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an R51H, or R51L mutation in ecTadA SEQ ID NO: 78, or a corresponding mutation in another adenosine deaminase. In some embodiments, the adenosine deaminase comprises a R51L mutation in ecTadA SEQ ID NO: 78, or a corresponding mutation in another adenosine deaminase.

In some embodiments, the adenosine deaminase comprises an S146X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises an S146R, or S146C mutation in ecTadA SEQ ID NO: 78, or a corresponding mutation in another adenosine deaminase. In some embodiments, the adenosine deaminase comprises a S146C mutation in ecTadA SEQ ID NO: 78, or a corresponding mutation in another adenosine deaminase.

In some embodiments, the adenosine deaminase comprises an K157X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a K157N mutation in ecTadA SEQ ID NO: 78, or a corresponding mutation in another adenosine deaminase.

In some embodiments, the adenosine deaminase comprises an W23X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a W23R, or W23L mutation in ecTadA SEQ ID NO: 78, or a corresponding mutation in another adenosine deaminase. In some embodiments, the adenosine deaminase comprises a W23R mutation in ecTadA SEQ ID NO: 78, or a corresponding mutation in another adenosine deaminase. In some embodiments, the adenosine deaminase comprises a W23L mutation in ecTadA SEQ ID NO: 78, or a corresponding mutation in another adenosine deaminase.

In some embodiments, the adenosine deaminase comprises an R152X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a R152P, or R52H mutation in ecTadA SEQ ID NO: 78, or a corresponding mutation in another adenosine deaminase. In some embodiments, the adenosine deaminase comprises a R152P mutation in ecTadA SEQ ID NO: 78, or a corresponding mutation in another adenosine deaminase. In some embodiments, the adenosine deaminase comprises a R152H mutation in ecTadA SEQ ID NO: 78, or a corresponding mutation in another adenosine deaminase.

In some embodiments, the adenosine deaminase comprises an R26X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a R26G mutation in ecTadA SEQ ID NO: 78, or a corresponding mutation in another adenosine deaminase.

In some embodiments, the adenosine deaminase comprises an I49X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a I49V mutation in ecTadA SEQ ID NO: 78, or a corresponding mutation in another adenosine deaminase.

In some embodiments, the adenosine deaminase comprises an N72X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a N72D mutation in ecTadA SEQ ID NO: 78, or a corresponding mutation in another adenosine deaminase.

In some embodiments, the adenosine deaminase comprises an S97X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a S97C mutation in ecTadA SEQ ID NO: 78, or a corresponding mutation in another adenosine deaminase.

In some embodiments, the adenosine deaminase comprises an G125X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a G125A mutation in ecTadA SEQ ID NO: 78, or a corresponding mutation in another adenosine deaminase.

In some embodiments, the adenosine deaminase comprises an K161X mutation in ecTadA SEQ ID NO: 78, or a corresponding mutation in another adenosine deaminase, where X indicates any amino acid other than the corresponding amino acid in the wild-type adenosine deaminase. In some embodiments, the adenosine deaminase comprises a K161T mutation in ecTadA SEQ ID NO: 78, or a corresponding mutation in another adenosine deaminase.

In some embodiments, the adenosine deaminase comprises one or more of a W23X, H36X, N37X, P48X, I49X, R51X, N72X, L84X, S97X, A106X, D108X, H123X, G125X, A142X, S146X, D147X, R152X, E155X, I156X, K157X, and/or K161X mutation in ecTadA SEQ ID NO: 78, or one or more corresponding mutations in another adenosine deaminase, where the presence of X indicates any amino acid other than the corresponding amino acid in the wild-type adenosine deaminase. In some embodiments, the adenosine deaminase comprises one or more of W23L, W23R, H36L, P48S, P48A, R51L, L84F, A106V, D108N, H123Y, A142N, S146C, D147Y, R152P, E155V, I156F, and/or K157N mutation in ecTadA SEQ ID NO: 78, or one or more corresponding mutations in another adenosine deaminase. In some embodiments, the adenosine deaminase comprises one or more of the mutations provided herein corresponding to ecTadA SEQ ID NO: 78, or one or more corresponding mutations in another adenosine deaminase.

In some embodiments, the adenosine deaminase comprises or consists of one or two mutations selected from A106X and D108X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one or two mutations selected from A106V and D108N in ecTadA SEQ ID NO: 78, or a corresponding mutation or mutations in another adenosine deaminase.

In some embodiments, the adenosine deaminase comprises or consists of one, two, three, or four mutations selected from A106X, D108X, D147X, and E155X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, or four mutations selected from A106V, D108N, D147Y, and E155V in ecTadA SEQ ID NO: 78, or a corresponding mutation or mutations in another adenosine deaminase. In some embodiments, the adenosine deaminase comprises or consists of a A106V, D108N, D147Y, and E155V mutation in ecTadA SEQ ID NO: 78, or corresponding mutations in another adenosine deaminase.

In some embodiments, the adenosine deaminase comprises or consists of one, two, three, four, five, six, or seven mutations selected from L84X, A106X, D108X, H123X, D147X, E155X, and I156X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, or seven mutations selected from L84F, A106V, D108N, H123Y, D147Y, E155V, and I156F in ecTadA SEQ ID NO: 78, or a corresponding mutation or mutations in another adenosine deaminase. In some embodiments, the adenosine deaminase comprises or consists of a L84F, A106V, D108N, H123Y, D147Y, E155V, and I156F mutation in ecTadA SEQ ID NO: 78, or corresponding mutations in another adenosine deaminase.

In some embodiments, the adenosine deaminase comprises or consists of one, two, three, four, five, six, seven, eight, nine, ten, or eleven mutations selected from H36X, R51X, L84X, A106X, D108X, H123X, S146X, D147X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, or eleven mutations selected from H36L, R51L, L84F, A106V, D108N, H123Y, S146C, D147Y, E155V, I156F, and K157N in ecTadA SEQ ID NO: 78, or a corresponding mutation or mutations in another adenosine deaminase. In some embodiments, the adenosine deaminase comprises or consists of a H36L, R51L, L84F, A106V, D108N, H123Y, S146C, D147Y, E155V, I156F, and K157N mutation in ecTadA SEQ ID NO: 78, or corresponding mutations in another adenosine deaminase.

**In** some embodiments, the adenosine deaminase comprises or consists of one, two, three, four, five, six, seven, eight, nine, ten, eleven, or twelve mutations selected from H36X, P48X, R51X, L84X, A106X, D108X, H123X, S146X, D147X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, eleven, or twelve mutations selected from H36L, P48S, R51L, L84F, A106V, D108N, H123Y, S146C, D147Y, E155V, I156F, and K157N in ecTadA SEQ ID NO: 78, or a corresponding mutation or mutations in another adenosine deaminase. In some embodiments, the adenosine deaminase comprises or consists of a H36L, P48S, R51L, L84F, A106V, D108N, H123Y, S146C, D147Y, E155V, I156F, and K157N mutation in ecTadA SEQ ID NO: 78, or corresponding mutations in another adenosine deaminase.

**In** some embodiments, the adenosine deaminase comprises or consists of one, two, three, four, five, six, seven, eight, nine, ten, eleven, twelve, or thirteen mutations selected from H36X, P48X, R51X, L84X, A106X, D108X, H123X, A142X, S146X, D147X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, eleven, twelve, or thirteen mutations selected from H36L, P48S, R51L, L84F, A106V, D108N, H123Y, A142N, S146C, D147Y, E155V, I156F, and K157N in ecTadA SEQ ID NO: 78, or a corresponding mutation or mutations in another adenosine deaminase. In some embodiments, the adenosine deaminase comprises or consists of a H36L, P48S, R51L, L84F, A106V, D108N, H123Y, A142N, S146C, D147Y, E155V, I156F, and K157N mutation in ecTadA SEQ ID NO: 78, or corresponding mutations in another adenosine deaminase.

**In** some embodiments, the adenosine deaminase comprises or consists of one, two, three, four, five, six, seven, eight, nine, ten, eleven, twelve, thirteen, or fourteen mutations selected from W23X, H36X, P48X, R51X, L84X, A106X, D108X, H123X, A142X, S146X, D147X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, eleven, twelve, thirteen, or fourteen mutations selected from W23L, H36L, P48A, R51L, L84F, A106V, D108N, H123Y, A142N, S146C, D147Y, E155V, I156F, and K157N in ecTadA SEQ ID NO: 78 or a corresponding mutation or mutations in another adenosine deaminase. In some embodiments, the adenosine deaminase comprises or consists of a W23L, H36L, P48A, R51L, L84F, A106V, D108N, H123Y, A142N, S146C, D147Y, E155V, I156F, and K157N mutation in ecTadA SEQ ID NO: 78, or corresponding mutations in another adenosine deaminase.

In some embodiments, the adenosine deaminase comprises or consists of one, two, three, four, five, six, seven, eight, nine, ten, eleven, twelve, thirteen, or fourteen mutations selected from W23X, H36X, P48X, R51X, L84X, A106X, D108X, H123X, S146X, D147X, R152X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, eleven, twelve, thirteen, or fourteen mutations selected from W23R, H36L, P48A, R51L, L84F, A106V, D108N, H123Y, S146C, D147Y, R152P, E155V, I156F, and K157N in ecTadA SEQ ID NO: 78, or a corresponding mutation or mutations in another adenosine deaminase. In some embodiments, the adenosine deaminase comprises or consists of a W23R, H36L, P48A, R51L, L84F, A106V, D108N, H123Y, S146C, D147Y, R152P, E155V, I156F, and K157N mutation in ecTadA SEQ ID NO: 78, or corresponding mutations in another adenosine deaminase.

In some embodiments, the adenosine deaminase comprises or consists of one, two, three, four, five, six, seven, eight, nine, ten, eleven, twelve, thirteen, fourteen, or fifteen mutations selected from W23X, H36X, P48X, R51X, L84X, A106X, D108X, H123X, A142X, S146X, D147X, R152X, E155X, I156X, and K157X in ecTadA SEQ ID NO: 78, or a corresponding mutation or mutations in another adenosine deaminase, where X indicates the presence of any amino acid other than the corresponding amino acid in the wild-type adenosine deaminase. In some embodiments, the adenosine deaminase comprises or consists of one, two, three, four, five, six, seven, eight, nine, ten, eleven, twelve, thirteen, fourteen, or fifteen mutations selected from W23L, H36L, P48A, R51L, L84F, A106V, D108N, H123Y, A142N, S146C, D147Y, R152P, E155V, I156F, and K157N in ecTadA SEQ ID NO: 78, or a corresponding mutation or mutations in another adenosine deaminase. In some embodiments, the adenosine deaminase comprises or consists of a W23L, H36L, P48A, R51L, L84F, A106V, D108N, H123Y, A142N, S146C, D147Y, R152P, E155V, I156F, and K157N mutation in ecTadA SEQ ID NO: 78, or corresponding mutations in another adenosine deaminase.

In some embodiments, the adenosine deaminase comprises one or more of the mutations provided herein corresponding to ecTadA SEQ ID NO: 78, or one or more of the corresponding mutations in another deaminase. In some embodiments, the adenosine deaminase comprises or consists of a variant of ecTadA SEQ ID NO: 78 provided herein, or the corresponding variant in another adenosine deaminase.

It should be appreciated that the adenosine deaminase (*e.g*., a first or second adenosine deaminase) may comprise one or more of the mutations provided in any of the adenosine deaminases (*e.g*., ecTadA adenosine deaminases) provided herein. In some embodiments, the adenosine deaminase comprises the combination of mutations of any of the adenosine deaminases (*e.g*., ecTadA adenosine deaminases) provided herein. For example, the adenosine deaminase may comprise the mutations W23R, H36L, P48A, R51L, L84F, A106V, D108N, H123Y, S146C, D147Y, R152P, E155V, I156F, and K157N (relative to ecTadA SEQ ID NO: 78), which corresponds to ABE7.10 provided herein. In some embodiments, the adenosine deaminase may comprise the mutations H36L, R51L, L84F, A106V, D108N, H123Y, S146C, D147Y, E155V, I156F, and K157N (relative to ecTadA SEQ ID NO: 78).

In some embodiments, the adenosine deaminase comprises any of the following combination of mutations relative to ecTadA SEQ ID NO: 78, where each mutation of a combination is separated by a "_" and each combination of mutations is between parentheses:
(A106V_D108N), (R107C_D108N), (H8Y_D108N_S127S_D147Y_Q154H),
(H8Y_R24W_D108N_N127S_D147Y_E155V), (D108N_D147Y_E155V), (H8Y_D108N_S127S),
(H8Y_D108N_N127S_D147Y_Q154H), (A106V_D108N_D147Y_E155V), (D108Q_D147Y_E155V), (D108M_D147Y_E155V), (D108L_D147Y_E155V), (D108K_D147Y_E155V), (D108I_D147Y_E155V),
(D108F_D147Y_E155V), (A106V_D108N_D147Y), (A106V_D108M_D147Y_E155V),
(E59A_A106V_D108N _D147Y_E155V), (E59A cat dead _A106V_D108N_D147Y _E155V),
(L84F_A106V_D108N_H123Y_D147Y_E155V_I156Y),
(L84F_A106V_D108N_H123Y_D147Y_E155V_I156F), (D103A_D014N), (G22P_D103A_D104N),
(G22P_D103A_D104N_S138A), (D103A_D104N_S138A),
(R26G_L84F_A106V_R107H_D108N_H123Y_A142N_A143D_D147Y_E155V_I156F),
(E25G_R26G_L84F_A106V_R107H_D108N_H123Y_A142N_A143D_D147Y_E155V_
I156F),(E25D_R26G_L84F_A106V_R107K_D108N_H123Y_A142N_A143G_D147Y_E155V_I156F),
(R26Q_L84F_A106V_DI10SN_H123Y_A142N_D147Y_E155V_I156F),
(E25M_R26G_L84F_A106V_R107P_D108N_H123Y_A142N_A143D_D147Y_E155V_I156F),
(R26C_L84F_A106V_R107H_D108N_H123Y_A142N _D147Y_E155V_I156F),
(L84F_A106V_D108N_H123Y_A142N_A143L_D147Y_E155V_I156F),
(R26G_L84F_A106V_D108N_H123Y_A142N_D147Y_E155V_I156F),
(E25A_R26G_L84F_A106V_R107N_D108N_H123Y_A142N_A143E_D147Y_E155V_I156F),
(R26G_L84F_A106V_R107H_D108N_H123Y_A142N_A143D_D147Y_E155V_I156F),
(A106V_D108N_A142N_D147Y_E155V), (R26G_A106V_D108N_A142N_D147Y_E155V),
(E25D_R26G_A106V_R107K_D108N_A142N_A143G_D147Y_E155V),
(R26G_A106V_D108N_R107H_A142N_A143D_D147Y_E155V),
(E25D_R26G_A106V_D108N_A142N_D147Y_E155V), (A106V_R107K_D108N_A142N_D147Y_E155V),
(A106V_D108N_A142N_A143G_D147Y_E155V), (A106V_D108N_A142N_A143L_D147Y_E155V),
(H36L_RS1L_L84F_A106V_D108N_H123Y_S146C_D147Y_E155V_I156F_K157N),
(H36L_P48S_R51L_L84F_A106V_D108N_H123Y_S146C_D147Y_E155V_I156F_K157N),
(H36L_P48S_R51L_L84F_A106V_D108N_H123Y_A142N_S146C_D147Y_E155V_I156F_K157N),(W23L_ H36L_P48A_R51L_L84F_A106V_D108N_H123Y_A142N_S146C_D147Y_E155V_I156F_K157N),(W23L_ H36L_P48A_R51L_L84F_A106V_D108N_H123Y_A142N_S146C_D147Y_R152P_E155V_I156F_K157N),( N37T_P48T_M70L_L84F_A106V_D108N_H123Y_D147Y_I49V_E155V _I156F),(N37S_L84F_A106V_D10 8N_H123Y_D147Y_E155V_I156F_K161T),
(H36L_L84F_A106V_D108N_H123Y_D147Y_Q154H_E155V_I156F),
(N72S_L84F_A106V_D108N_H123Y_S146R_D147Y_E155V_I156F),
(H36L_P48L_L84F_A106V_D108N_H123Y_E134G_D147Y_E155V_I156F),
(H36L_L84F_A106V_D108N_H123Y_D147Y_E155V_I156F_K157N),
(H36L_L84F_A106V_D108N_H123Y_S146C_D147Y_E155V_I156F),
(L84F_A106V_D108N_H123Y_S146R_D147Y_E155V_I156F_K161T),
(N37S_R51H_D77G_L84F_A106V_D108N_H123Y_D147Y_E155V_I156F),
(R51L_L84F_A106V_D108N_H123Y_D147Y_E155V_I156F_K157N),
(D24G_Q71R_L84F H96L_A106V_D108N_H123Y_D147Y_E155V_I156F_K160E),
(H36L_G67V_L84F_A106V_D108N_H123Y_S146T_D147Y_E155V_I156F),
(Q71L_L84F_A106V_D108N_H123Y_L137M_A143E_D147Y_E155V_I156F),
(E25G_L84F_A106V_D108N_H123Y_D147Y_E155V_I156F_Q159L),
(L84F_A91T_F104I_A106V_D108N_H123Y_D147Y_E155V_I156F),
(N72D_L84F_A106V_D108N_H123Y_G125A_D147Y_E155V_I156F),
(P48S_L84F_S97C_A106V_D108N_H123Y_D147Y_E155V_I156F),
(W23G_L84F_A106V_D108N_H123Y_D147Y_E155V_I156F),
(D24G_P48L_Q71R_L84F_A106V_D108N_H123Y_D147Y_E155V_I156F_Q159L),
(L84F_A106V_D108N_H123Y_A142N_D147Y_E155V_I156F),
(H36L_R51L_L84F_A106V_D108N_H123Y_A142N_S146C_D147Y_E155V_I156F_K157N),
(N37S_L84F_A106V_D108N_H123Y_A142N_D147Y_E155V_I156F_K161T),
(L84F_A106V_D108N_D147Y_E155V_I156F),
(R51L_L84F_A106V_D108N_H123Y_S146C_D147Y_E155V_I156F_K157N_K161T),
(L84F_A106V_D108N_H123Y_S146C_D147Y_E155V_I156F_K161T),
(L84F_A106V_D108N_H123Y_S146C_D147Y_E155V_I156F_K157N_K160E_K161T),
(L84F_A106V_D108N_H123Y_S146C_D147Y_E155V_I156F_K157N_K160E), (R74Q
L84F_A106V_D108N_H123Y_D147Y_E155V_I156F),
(R74A_L84F_A106V_D108N_H123Y_D147Y_E155V_I156F),
(L84F_A106V_D108N_H123Y_D147Y_E155V_I156F),
(R74Q_L84F_A106V_D108N_H123Y_D147Y_E155V_I156F),
(L84F_R98Q_A106V_D108N_H123Y_D147Y_E155V_I156F),
(L84F_A106V_D108N_H123Y_R129Q_D147Y_E155V_I156F),
(P48S_L84F_A106V_D108N_H123Y_A142N_D147Y_E155V_I156F), (P48S_A142N),
(P48T_149V_L84F_A106V_D108N_H123Y_A142N_D147Y_E155V_I156F_L157N), (P48T_I49V_A142N),
(H36L_P48S_R51L_L84F_A106V_D108N_H123Y_S146C_D147Y_E155V_I156F_K157N),
(H36L_P48S_RS51L_L84F_A106V_D108N_H123Y_S146C_A142N_D147Y_E155V_I156F
_K157N),(H36L_P48T_I49V_R51L_L84F_A106V_D108N_H123Y_S146C_D147Y_E155V_I156F_K157N),
(H36L_P48T_I49V_R51L_L84F_A106V_D108N_H123Y_A142N_S146C_D147Y_E155V_I156F_K157N),
(H36L_P48A_R51L_L84F_A106V_D108N_H123Y_S146C_D147Y_E155V_I156F_K157N),
(H36L _P48A_R51L_L84F_A106V_D108N_H123Y_A142N_S146C_D147Y_E155V_I156F_K157N),
(H36L_P48A_R51L_L84F_A106V_D108N_H123Y_S146C_A142N_D147Y_E155V_I156F_K157N),
(W23L_H36L_P48A_R51L_L84F_A106V_D108N_H123Y_S146C_D147Y_E155V_I156F_K157N),
(W23R_H36L_P48A_R51L_L84F_A106V_D108N_H123Y_S146C_D147Y_E155V_I156F_K157N),
(W23L_H36L_P48A R5IL_L84F A106V_DI108N _HI23Y S146R_D147Y E155V_I156F K161T),
(H36L_P48A_R51L_L84F_A106V_D108N_H123Y_S146C_D147Y_R152H_E155V_I156F_K157N),
(H36L_P48A_R51L_L84F_A106V_D108N_H123Y_S146C_D147Y_R152P_E155V_I156F_K157N),
(W23L_H36L_P48A_R51L_L84F_A106V_D108N_H123Y_S146C_D147Y_R152P_E155V_I156F _K157N),
(W23L_H36L_P48A_R51L_L84F_A106V_D108N_H123Y_A142A_S146C_D147Y_E155V_I156F_K157N),
(W23L_H36L_P48A_R51L_L84F_A106V_D108N_H123Y_A142A_S146C_D147Y_R152P_E155V_I156F _K157N), (W23L_H36L_P48A_R51L_L84F_A106V_D108N_H123Y_S146R_D147Y_E155V_I156F _K161T), (W23R_H36L_P48A_R51L_L84F_A106V_D108N_H123Y_S146C_D147Y_R152P_E155V_I156F _K157N), (H36L_P48A_R51L_L84F_A106V_D108N_H123Y_A142N_S146C_D147Y_R152P_E155V_I156F _K157N).

### IV. Cytidine deaminases

In some embodiments, the disclosure provides base editors that comprise one or more cytidine deaminase domains. In some aspects, any of the disclosed base editors are capable of deaminating cytidine in a nucleic acid sequence (*e.g*., genomic DNA). As one example, any of the base editors provided herein may be base editors, (*e.g*., cytidine base editors).

In some embodiments, the cytidine deaminase is an apolipoprotein B mRNA-editing complex (APOBEC) family deaminase. In some embodiments, the cytidine deaminase is an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or an APOBEC4 deaminase. In some embodiments, the cytidine deaminase is an activation-induced deaminase (AID). In some embodiments, the deaminase is a Lamprey CDA1 (pmCDA1) deaminase. In some embodiments, the cytidine deaminase is from a human, chimpanzee, gorilla, monkey, cow, dog, rat, or mouse. In some embodiments, the deaminase is from a human. In some embodiments the deaminase is from a rat. In some embodiments, the cytidine deaminase is a human APOBEC1 deaminase. In some embodiments, the cytidine deaminase is pmCDA1. In some embodiments, the deaminase is human APOBEC3G . In some embodiments, the deaminase is a human APOBEC3G variant. In some embodiments, the deaminase is at least 80%, at least 85%, at least 90%, at least 92%, at least 95%, at least 96%, at least 97%, at least 98%, at least 99%, or at least 99.5% identical to any one of the APOBEC amino acid sequences set forth herein.

Some exemplary suitable cytidine deaminases domains that can be fused to Cas9 domains according to aspects of this disclosure are provided below. It should be understood that the disclosure also embraces other cytidine deaminases comprising an amino acid sequence having at least 80%, at least 85%, at least 90%, at least 95%, at least 98%, at least 99%, or at least 99.5% sequence identity to one of the following exemplary cytidine deaminases:
Human AID: 



 (SEQ ID NO: 92)
Mouse AID: 
MDSLLMKQKKFLYHFKNVRWAKGRHETYLCYVVKRRDSATSCSLDFGHLRNKSGC
HVELLFLRYISDWDLDPGRCYRVTWFTSWSPCYDCARHVAEFLRWNPNLSLRIFTAR
 
LYFCEDRKAEPEGLRRLHRAGVQIGIMTFKDYFYCWNTFVENRERTFKAWEGLHEN
SVRLTRQLRRILLPLYEVDDLRDAFRMLGF (SEQ ID NO: 93)
Dog AID: 
MDSLLMKQRKFLYHFKNVRWAKGRHETYLCYVVKRRDSATSFSLDFGHLRNKSGC
HVELLFLRYISDWDLDPGRCYRVTWFTSWSPCYDCARHVADFLRGYPNLSLRIFAAR
LYFCEDRKAEPEGLRRLHRAGVQIAIMTFKDYFYCWNTFVENREKTFKAWEGLHEN
SVRLSRQLRRILLPLYEVDDLRDAFRTLGL (SEQ ID NO: 94)
Bovine AID: 
MDSLLKKQRQFLYQFKNVRWAKGRHETYLCYVVKRRDSPTSFSLDFGHLRNKAGC
HVELLFLRYISDWDLDPGRCYRVTWFTSWSPCYDCARHVADFLRGYPNLSLRIFTAR
LYFCDKERKAEPEGLRRLHRAGVQIAIMTFKDYFYCWNTFVENHERTFKAWEGLHE
NSVRLSRQLRRILLPLYEVDDLRDAFRTLGL (SEQ ID NO: 95)
Rat:AID: 
MAVGSKPKAALVGPHWERERIWCFLCSTGLGTQQTGQTSRWLRPAATQDPVSPPRS
LLMKQRKFLYHFKNVRWAKGRHETYLCYVVKRRDSATSFSLDFGYLRNKSGCHVE
LLFLRYISDWDLDPGRCYRVTWFTSWSPCYDCARHVADFLRGNPNLSLRIFTARLTG
WGALPAGLMSPARPSDYFYCWNTFVENHERTFKAWEGLHENSVRLSRRLRRILLPL
YEVDDLRDAFRTLGL (SEQ ID NO: 96)
Mouse APOBEC-3: 
MGPFCLGCSHRKCYSPIRNLISQETFKFHFKNLGYAKGRKDTFLCYEVTRKDCDSPV
SLHHGVFKNKDNIHAEICFLYWFHDKVLKVLSPREEFKITWYMSWSPCFECAEQIVR
FLATHHNLSLDIFSSRLYNVQDPETQQNLCRLVQEGAQVAAMDLYEFKKCWKKFVD
NGGRRFRPWKRLLTNFRYQDSKLQEILRPCYIPVPSSSSTLSNICLTKGLPETRFCVE
GRRMDPLSEEEFYSQFYNQRVKHLCYYHRMKPYLCYQLEQFNGQAPLKGCLLSEKG
KQHAEILFLDKIRSMELSQVTITCYLTWSPCPNCAWQLAAFKRDRPDLILHIYTSRLY
FHWKRPFQKGLCSLWQSGILVDVMDLPQFTDCWTNFVNPKRPFWPWKGLEIISRRT
QRRLRRIKESWGLQDLVNDFGNLQLGPPMS (SEQ ID NO: 97)
Rat APOBEC-3: 
MGPFCLGCSHRKCYSPIRNLISQETFKFHFKNLRYAIDRKDTFLCYEVTRKDCDSPVS
LHHGVFKNKDNIHAEICFLYWFHDKVLKVLSPREEFKITWYMSWSPCFECAEQVLRF
LATHHNLSLDIFSSRLYNIRDPENQQNLCRLVQEGAQVAAMDLYEFKKCWKKFVDN
GGRRFRPWKKLLTNFRYQDSKLQEILRPCYIPVPSSSSSTLSNICLTKGLPETRFCVER
RRVHLLSEEEFYSQFYNQRVKHLCYYHGVKPYLCYQLEQFNGQAPLKGCLLSEKGK
QHAEILFLDKIRSMELSQVIITCYLTWSPCPNCAWQLAAFKRDRPDLILHIYTSRLYFH
 
WKRPFQKGLCSLWQSGILVDVMDLPQFTDCWTNFVNPKRPFWPWKGLEIISRRTQR
RLHRIKESWGLQDLVNDFGNLQLGPPMS (SEQ ID NO: 98)
Rhesus macaque APOBEC-3G: 
MVEPMDPRTFVSNFNNRPILSGLNTVWLCCEVKTKDPSGPPLDAKIFQGKVYSKAKY
HPEMRFLRWFHKWRQLHHDQEYKVTWYVSWSPCTRCANSVATFLAKDPKVTLTIF
VARLYYFWKPDYQQALRILCQKRGGPHATMKIMNYNEFQDCWNKFVDGRGKPFKP
RNNLPKHYTLLQATLGELLRHLMDPGTFTSNFNNKPWVSGQHETYLCYKVERLHND
TWVPLNQHRGFLRNQAPNIHGFPKGRHAELCFLDLIPFWKLDGQQYRVTCFTSWSPC
FSCAQEMAKFISNNEHVSLCIFAARIYDDQGRYQEGLRALHRDGAKIAMMNYSEFEY
CWDTFVDRQGRPFQPWDGLDEHSQALSGRLRAI (SEQ ID NO: 99)
Chimpanzee APOBEC-3G: 
MKPHFRNPVERMYQDTFSDNFYNRPILSHRNTVWLCYEVKTKGPSRPPLDAKIFRGQ
VYSKLKYHPEMRFFHWFSKWRKLHRDQEYEVTWYISWSPCTKCTRDVATFLAEDP
KVTLTIFVARLYYFWDPDYQEALRSLCQKRDGPRATMKIMNYDEFQHCWSKFVYS
QRELFEPWNNLPKYYILLHIMLGEILRHSMDPPTFTSNFNNELWVRGRHETYLCYEV
ERLHNDTWVLLNQRRGFLCNQAPHKHGFLEGRHAELCFLDVIPFWKLDLHQDYRVT
CFTSWSPCFSCAQEMAKFISNNKHVSLCIFAARIYDDQGRCQEGLRTLAKAGAKISIM
TYSEFKHCWDTFVDHQGCPFQPWDGLEEHSQALSGRLRAILQNQGN (SEQ ID NO:
100)
Green monkey APOBEC-3G: 
MNPQIRNMVEQMEPDIFVYYFNNRPILSGRNTVWLCYEVKTKDPSGPPLDANIFQGK
LYPEAKDHPEMKFLHWFRKWRQLHRDQEYEVTWYVSWSPCTRCANSVATFLAEDP
KVTLTIFVARLYYFWKPDYQQALRILCQERGGPHATMKIMNYNEFQHCWNEFVDG
QGKPFKPRKNLPKHYTLLHATLGELLRHVMDPGTFTSNFNNKPWVSGQRETYLCYK
VERSHNDTWVLLNQHRGFLRNQAPDRHGFPKGRHAELCFLDLIPFWKLDDQQYRVT
CFTSWSPCFSCAQKMAKFISNNKHVSLCIFAARIYDDQGRCQEGLRTLHRDGAKIAV
MNYSEFEYCWDTFVDRQGRPFQPWDGLDEHSQALSGRLRAI (SEQ ID NO: 101)
Human APOBEC-3G: 
MKPHFRNTVERMYRDTFSYNFYNRPILSRRNTVWLCYEVKTKGPSRPPLDAKIFRGQ
VYSELKYHPEMRFFHWFSKWRKLHRDQEYEVTWYISWSPCTKCTRDMATFLAEDP
KVTLTIFVARLYYFWDPDYQEALRSLCQKRDGPRATMKIMNYDEFQHCWSKFVYS
QRELFEPWNNLPKYYILLHIMLGEILRHSMDPPTFTFNFNNEPWVRGRHETYLCYEV
ERMHNDTWVLLNQRRGFLCNQAPHKHGFLEGRHAELCFLDVIPFWKLDLDQDYRV
TCFTSWSPCFSCAQEMAKFISKNKHVSLCIFTARIYDDQGRCQEGLRTLAEAGAKISI
 
MTYSEFKHCWDTFVDHQGCPFQPWDGLDEHSQDLSGRLRAILQNQEN (SEQ ID NO:
102)
Human APOBEC-3F: 
MKPHFRNTVERMYRDTFSYNFYNRPILSRRNTVWLCYEVKTKGPSRPRLDAKIFRGQ
VYSQPEHHAEMCFLSWFCGNQLPAYKCFQITWFVSWTPCPDCVAKLAEFLAEHPNV
TLTISAARLYYYWERDYRRALCRLSQAGARVKIMDDEEFAYCWENFVYSEGQPFMP
WYKFDDNYAFLHRTLKEILRNPMEAMYPHIFYFHFKNLRKAYGRNESWLCFTMEV
VKHHSPVSWKRGVFRNQVDPETHCHAERCFLSWFCDDILSPNTNYEVTWYTSWSPC
PECAGEVAEFLARHSNVNLTIFTARLYYFWDTDYQEGLRSLSQEGASVEIMGYKDFK
YCWENFVYNDDEPFKPWKGLKYNFLFLDSKLQEILE (SEQ ID NO: 103)
Human APOBEC-3B: 
MNPQIRNPMERMYRDTFYDNFENEPILYGRSYTWLCYEVKIKRGRSNLLWDTGVFR
GQVYFKPQYHAEMCFLSWFCGNQLPAYKCFQITWFVSWTPCPDCVAKLAEFLSEHP
NVTLTISAARLYYYWERDYRRALCRLSQAGARVTIMDYEEFAYCWENFVYNEGQQ
FMPWYKFDENYAFLHRTLKEILRYLMDPDTFTFNFNNDPLVLRRRQTYLCYEVERL
DNGTWVLMDQHMGFLCNEAKNLLCGFYGRHAELRFLDLVPSLQLDPAQIYRVTWFI
SWSPCFSWGCAGEVRAFLQENTHVRLRIFAARIYDYDPLYKEALQMLRDAGAQVSI
MTYDEFEYCWDTFVYRQGCPFQPWDGLEEHSQALSGRLRAILQNQGN (SEQ ID
NO: 104)
Rat APOBEC-3B: 
MQPQGLGPNAGMGPVCLGCSHRRPYSPIRNPLKKLYQQTFYFHFKNVRYAWGRKN
NFLCYEVNGMDCALPVPLRQGVFRKQGHIHAELCFIYWFHDKVLRVLSPMEEFKVT
WYMSWSPCSKCAEQVARFLAAHRNLSLAIFSSRLYYYLRNPNYQQKLCRLIQEGVH
VAAMDLPEFKKCWNKFVDNDGQPFRPWMRLRINFSFYDCKLQEIFSRMNLLREDVF
YLQFNNSHRVKPVQNRYYRRKSYLCYQLERANGQEPLKGYLLYKKGEQHVEILFLE
KMRSMELSQVRITCYLTWSPCPNCARQLAAFKKDHPDLILRIYTSRLYFYWRKKFQK
GLCTLWRSGIHVDVMDLPQFADCWTNFVNPQRPFRPWNELEKNSWRIQRRLRRIKE
SWGL (SEQ ID NO: 105)
Bovine APOBEC-3B: 
DGWEVAFRSGTVLKAGVLGVSMTEGWAGSGHPGQGACVWTPGTRNTMNLLREVL
FKQQFGNQPRVPAPYYRRKTYLCYQLKQRNDLTLDRGCFRNKKQRHAEIRFIDKINS
LDLNPSQSYKIICYITWSPCPNCANELVNFITRNNHLKLEIFASRLYFHWIKSFKMGLQ
DLQNAGISVAVMTHTEFEDCWEQFVDNQSRPFQPWDKLEQYSASIRRRLQRILTAPI
(SEQ ID NO: 106)
Chimpanzee APOBEC-3B: 
MNPQIRNPMEWMYQRTFYYNFENEPILYGRSYTWLCYEVKIRRGHSNLLWDTGVFR
GQMYSQPEHHAEMCFLSWFCGNQLSAYKCFQITWFVSWTPCPDCVAKLAKFLAEH
PNVTLTISAARLYYYWERDYRRALCRLSQAGARVKIMDDEEFAYCWENFVYNEGQ
PFMPWYKFDDNYAFLHRTLKEIIRHLMDPDTFTFNFNNDPLVLRRHQTYLCYEVERL
DNGTWVLMDQHMGFLCNEAKNLLCGFYGRHAELRFLDLVPSLQLDPAQIYRVTWFI
SWSPCFSWGCAGQVRAFLQENTHVRLRIFAARIYDYDPLYKEALQMLRDAGAQVSI
MTYDEFEYCWDTFVYRQGCPFQPWDGLEEHSQALSGRLRAILQVRASSLCMVPHRP
PPPPQSPGPCLPLCSEPPLGSLLPTGRPAPSLPFLLTASFSFPPPASLPPLPSLSLSPGHLP
VPSFHSLTSCSIQPPCSSRIRETEGWASVSKEGRDLG (SEQ ID NO: 107)
Human APOBEC-3C: 
MNPQIRNPMKAMYPGTFYFQFKNLWEANDRNETWLCFTVEGIKRRSVVSWKTGVF
RNQVDSETHCHAERCFLSWFCDDILSPNTKYQVTWYTSWSPCPDCAGEVAEFLARH
SNVNLTIFTARLYYFQYPCYQEGLRSLSQEGVAVEIMDYEDFKYCWENFVYNDNEPF
KPWKGLKTNFRLLKRRLRESLQ (SEQ ID NO: 108)
Gorilla APOBEC3C: 
MNPQIRNPMKAMYPGTFYFQFKNLWEANDRNETWLCFTVEGIKRRSVVSWKTGVF
RNQVDSETHCHAERCFLSWFCDDILSPNTNYQVTWYTSWSPCPECAGEVAEFLARH
SNVNLTIFTARLYYFQDTDYQEGLRSLSQEGVAVKIMDYKDFKYCWENFVYNDDEP
FKPWKGLKYNFRFLKRRLQEILE (SEQ ID NO: 109)
Human APOBEC-3A: 
MEASPASGPRHLMDPHIFTSNFNNGIGRHKTYLCYEVERLDNGTSVKMDQHRGFLH
NQAKNLLCGFYGRHAELRFLDLVPSLQLDPAQIYRVTWFISWSPCFSWGCAGEVRAF
LQENTHVRLRIFAARIYDYDPLYKEALQMLRDAGAQVSIMTYDEFKHCWDTFVDHQ
GCPFQPWDGLDEHSQALSGRLRAILQNQGN (SEQ ID NO: 110)
Rhesus macaque APOBEC-3A: 
MDGSPASRPRHLMDPNTFTFNFNNDLSVRGRHQTYLCYEVERLDNGTWVPMDERR
GFLCNKAKNVPCGDYGCHVELRFLCEVPSWQLDPAQTYRVTWFISWSPCFRRGCAG
QVRVFLQENKHVRLRIFAARIYDYDPLYQEALRTLRDAGAQVSIMTYEEFKHCWDT
FVDRQGRPFQPWDGLDEHSQALSGRLRAILQNQGN (SEQ ID NO: 111)
Bovine APOBEC-3A: 
MDEYTFTENFNNQGWPSKTYLCYEMERLDGDATIPLDEYKGFVRNKGLDQPEKPCH
AELYFLGKIHSWNLDRNQHYRLTCFISWSPCYDCAQKLTTFLKENHHISLHILASRIY
 
THNRFGCHQSGLCELQAAGARITIMTFEDFKHCWETFVDHKGKPFQPWEGLNVKSQ
ALCTELQAILKTQQN (SEQ ID NO: 112)
Human APOBEC-3H: 
MALLTAETFRLQFNNKRRLRRPYYPRKALLCYQLTPQNGSTPTRGYFENKKKCHAEI
CFINEIKSMGLDETQCYQVTCYLTWSPCSSCAWELVDFIKAHDHLNLGIFASRLYYH
WCKPQQKGLRLLCGSQVPVEVMGFPKFADCWENFVDHEKPLSFNPYKMLEELDKN
SRAIKRRLERIKIPGVRAQGRYMDILCDAEV (SEQ ID NO: 113)
Rhesus macaque APOBEC-3H: 
MALLTAKTFSLQFNNKRRVNKPYYPRKALLCYQLTPQNGSTPTRGHLKNKKKDHAE
IRFINKIKSMGLDETQCYQVTCYLTWSPCPSCAGELVDFIKAHRHLNLRIFASRLYYH
WRPNYQEGLLLLCGSQVPVEVMGLPEFTDCWENFVDHKEPPSFNPSEKLEELDKNS
QAIKRRLERIKSRSVDVLENGLRSLQLGPVTPSSSIRNSR (SEQ ID NO: 114)
Human APOBEC-3D: 
MNPQIRNPMERMYRDTFYDNFENEPILYGRSYTWLCYEVKIKRGRSNLLWDTGVFR
GPVLPKRQSNHRQEVYFRFENHAEMCFLSWFCGNRLPANRRFQITWFVSWNPCLPC
VVKVTKFLAEHPNVTLTISAARLYYYRDRDWRWVLLRLHKAGARVKIMDYEDFAY
CWENFVCNEGQPFMPWYKFDDNYASLHRTLKEILRNPMEAMYPHIFYFHFKNLLKA
CGRNESWLCFTMEVTKHHSAVFRKRGVFRNQVDPETHCHAERCFLSWFCDDILSPN
TNYEVTWYTSWSPCPECAGEVAEFLARHSNVNLTIFTARLCYFWDTDYQEGLCSLS
QEGASVKIMGYKDFVSCWKNFVYSDDEPFKPWKGLQTNFRLLKRRLREILQ (SEQ
ID NO: 115)
Human APOBEC-1: 
MTSEKGPSTGDPTLRRRIEPWEFDVFYDPRELRKEACLLYEIKWGMSRKIWRSSGKN
TTNHVEVNFIKKFTSERDFHPSMSCSITWFLSWSPCWECSQAIREFLSRHPGVTLVIYV
ARLFWHMDQQNRQGLRDLVNSGVTIQIMRASEYYHCWRNFVNYPPGDEAHWPQY
PPLWMMLYALELHCIILSLPPCLKISRRWQNHLTFFRLHLQNCHYQTIPPHILLATGLI
HPSVAWR (SEQ ID NO: 116)
Mouse APOBEC-1: 
MSSETGPVAVDPTLRRRIEPHEFEVFFDPRELRKETCLLYEINWGGRHSVWRHTSQN
TSNHVEVNFLEKFTTERYFRPNTRCSITWFLSWSPCGECSRAITEFLSRHPYVTLFIYIA
RLYHHTDQRNRQGLRDLISSGVTIQIMTEQEYCYCWRNFVNYPPSNEAYWPRYPHL
WVKLYVLELYCIILGLPPCLKILRRKQPQLTFFTITLQTCHYQRIPPHLLWATGLK
(SEQ ID NO: 117)
Rat APOBEC-1: 



 (SEQ
ID NO: 118)
Human APOBEC-2: 
MAQKEEAAVATEAASQNGEDLENLDDPEKLKELIELPPFEIVTGERLPANFFKFQFRN
VEYSSGRNKTFLCYVVEAQGKGGQVQASRGYLEDEHAAAHAEEAFFNTILPAFDPA
LRYNVTWYVSSSPCAACADRIIKTLSKTKNLRLLILVGRLFMWEEPEIQAALKKLKE
AGCKLRIMKPQDFEYVWQNFVEQEEGESKAFQPWEDIQENFLYYEEKLADILK (SEQ
ID NO: 119)
Mouse APOBEC-2: 
MAQKEEAAEAAAPASQNGDDLENLEDPEKLKELIDLPPFEIVTGVRLPVNFFKFQFR
NVEYSSGRNKTFLCYVVEVQSKGGQAQATQGYLEDEHAGAHAEEAFFNTILPAFDP
ALKYNVTWYVSSSPCAACADRILKTLSKTKNLRLLILVSRLFMWEEPEVQAALKKL
KEAGCKLRIMKPQDFEYIWQNFVEQEEGESKAFEPWEDIQENFLYYEEKLADILK
(SEQ ID NO: 120)
Rat APOBEC-2: 
MAQKEEAAEAAAPASQNGDDLENLEDPEKLKELIDLPPFEIVTGVRLPVNFFKFQFR
NVEYSSGRNKTFLCYVVEAQSKGGQVQATQGYLEDEHAGAHAEEAFFNTILPAFDP
ALKYNVTWYVSSSPCAACADRILKTLSKTKNLRLLILVSRLFMWEEPEVQAALKKL
KEAGCKLRIMKPQDFEYLWQNFVEQEEGESKAFEPWEDIQENFLYYEEKLADILK
(SEQ ID NO: 121)
Bovine APOBEC-2: 
MAQKEEAAAAEPASQNGEEVENLEDPEKLKELIELPPFEIVTGERLPAHYFKFQFRN
VEYSSGRNKTFLCYVVEAQSKGGQVQASRGYLEDEHATNHAEEAFFNSIMPTFDPA
LRYMVTWYVSSSPCAACADRIVKTLNKTKNLRLLILVGRLFMWEEPEIQAALRKLK
EAGCRLRIMKPQDFEYIWQNFVEQEEGESKAFEPWEDIQENFLYYEEKLADILK
(SEQ ID NO: 122)
Petromyzon marinus CDA1 (pmCDA1): 
MTDAEYVRIHEKLDIYTFKKQFFNNKKSVSHRCYVLFELKRRGERRACFWGYAVNK
PQSGTERGIHAEIFSIRKVEEYLRDNPGQFTINWYSSWSPCADCAEKILEWYNQELRG
NGHTLKIWACKLYYEKNARNQIGLWNLRDNGVGLNVMVSEHYQCCRKIFIQSSHN
QLNENRWLEKTLKRAEKRRSELSIMIQVKILHTTKSPAV (SEQ ID NO: 123)
Human APOBEC3G D316R_D317R: 
MKPHFRNTVERMYRDTFSYNFYNRPILSRRNTVWLCYEVKTKGPSRPPLDAKIFRGQ
VYSELKYHPEMRFFHWFSKWRKLHRDQEYEVTWYISWSPCTKCTRDMATFLAEDP
KVTLTIFVARLYYFWDPDYQEALRSLCQKRDGPRATMKIMNYDEFQHCWSKFVYS
QRELFEPWNNLPKYYILLHIMLGEILRHSMDPPTFTFNFNNEPWVRGRHETYLCYEV
ERMHNDTWVLLNQRRGFLCNQAPHKHGFLEGRHAELCFLDVIPFWKLDLDQDYRV
TCFTSWSPCFSCAQEMAKFISKNKHVSLCIFTARIYRRQGRCQEGLRTLAEAGAKISI
MTYSEFKHCWDTFVDHQGCPFQPWDGLDEHSQDLSGRLRAILQNQEN (SEQ ID NO:
124)
Human APOBEC3G chain A: 
MDPPTFTFNFNNEPWVRGRHETYLCYEVERMHNDTWVLLNQRRGFLCNQAPHKHG
FLEGRHAELCFLDVIPFWKLDLDQDYRVTCFTSWSPCFSCAQEMAKFISKNKHVSLCI
FTARIYDDQGRCQEGLRTLAEAGAKISIMTYSEFKHCWDTFVDHQGCPFQPWDGLD
EHSQDLSGRLRAILQ (SEQ ID NO: 125)
Human APOBEC3G chain A D120R_D121R:

MDPPTFTFNFNNEPWVRGRHETYLCYEVERMHNDTWVLLNQRRGFLCNQAPHKHG
FLEGRHAELCFLDVIPFWKLDLDQDYRVTCFTSWSPCFSCAQEMAKFISKNKHVSLCI
FTARIYRRQGRCQEGLRTLAEAGAKISIMTYSEFKHCWDTFVDHQGCPFQPWDGLD
EHSQDLSGRLRAILQ (SEQ ID NO: 126)

Any of the aforementioned DNA effector domains may be subjected to a continuous evolution process (e.g., PACE) or may be otherwise further evolved using a mutagenesis methodology known in the art.

In some embodiments, the cytidine deaminase is an apolipoprotein B mRNA-editing complex (APOBEC) family deaminase. In some embodiments, the deaminase is an APOBEC1 deaminase. In some embodiments, the deaminase is an APOBEC2 deaminase. In some embodiments, the deaminase is an APOBEC3 deaminase. In some embodiments, the deaminase is an APOBEC3A deaminase. In some embodiments, the deaminase is an APOBEC3B deaminase. In some embodiments, the deaminase is an APOBEC3C deaminase. In some embodiments, the deaminase is an APOBEC3D deaminase. In some embodiments, the deaminase is an APOBEC3E deaminase. In some embodiments, the deaminase is an APOBEC3F deaminase. In some embodiments, the deaminase is an APOBEC3G deaminase. In some embodiments, the deaminase is an APOBEC3H deaminase. In some embodiments, the deaminase is an APOBEC4 deaminase. In some embodiments, the deaminase is an activation-induced deaminase (AID). In some embodiments, the deaminase is a vertebrate deaminase. In some embodiments, the deaminase is an invertebrate deaminase. In some embodiments, the deaminase is a human, chimpanzee, gorilla, monkey, cow, dog, rat, or mouse deaminase. In some embodiments, the deaminase is a human deaminase. In some embodiments, the deaminase is a rat deaminase, e.g., rAPOBEC1.

Some aspects of the disclosure are based on the recognition that modulating the deaminase domain catalytic activity of any of the fusion proteins provided herein, for example by making point mutations in the deaminase domain, affect the processivity of the fusion proteins (e.g., base editors). For example, mutations that reduce, but do not eliminate, the catalytic activity of a deaminase domain within a base editing fusion protein can make it less likely that the deaminase domain will catalyze the deamination of a residue adjacent to a target residue, thereby narrowing the deamination window. The ability to narrow the deamination window may prevent unwanted deamination of residues adjacent of specific target residues, which may decrease or prevent off-target effects.

In some embodiments, any of the fusion proteins provided herein comprise a deaminase domain (e.g., a cytidine deaminase domain) that has reduced catalytic deaminase activity. In some embodiments, any of the fusion proteins provided herein comprise a deaminase domain (e.g., a cytidine deaminase domain) that has a reduced catalytic deaminase activity as compared to an appropriate control. For example, the appropriate control may be the deaminase activity of the deaminase prior to introducing one or more mutations into the deaminase. In other embodiments, the appropriate control may be a wild-type deaminase. In some embodiments, the appropriate control is a wild-type apolipoprotein B mRNA-editing complex (APOBEC) family deaminase. In some embodiments, the appropriate control is an APOBEC 1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, or an APOBEC3H deaminase. In some embodiments, the appropriate control is an activation induced deaminase (AID). In some embodiments, the appropriate control is a cytidine deaminase 1 from Petromyzon marinus (pmCDA1). In some embodiments, the deaminase domain may be a deaminase domain that has at least 1%, at least 5%, at least 15%, at least 20%, at least 25%, at least 30%, at least 40%, at least 50%, at least 60%, at least 70%, at least 80%, at least 90%, or at least 95% less catalytic deaminase activity as compared to an appropriate control.

The apolipoprotein B mRNA-editing complex (APOBEC) family of cytidine deaminase enzymes encompasses eleven proteins that serve to initiate mutagenesis in a controlled and beneficial manner. One family member, activation-induced cytidine deaminase (AID), is responsible for the maturation of antibodies by converting cytosines in ssDNA to uracils in a transcription-dependent, strand-biased fashion. The apolipoprotein B editing complex 3 (APOBEC3) enzyme provides protection to human cells against a certain HIV-1 strain via the deamination of cytosines in reverse-transcribed viral ssDNA. These proteins all require a Zn2+-coordinating motif (His-X-Glu-X23-26-Pro-Cys-X2-4-Cys; (SEQ ID NO: 402) and bound water molecule for catalytic activity. The Glu residue acts to activate the water molecule to a zinc hydroxide for nucleophilic attack in the deamination reaction. Each family member preferentially deaminates at its own particular "hotspot", ranging from WRC (W is A or T, R is A or G) for hAID, to TTC for hAPOBEC3F. A recent crystal structure of the catalytic domain of APOBEC3G revealed a secondary structure comprised of a five-stranded β-sheet core flanked by six α-helices, which is believed to be conserved across the entire family. The active center loops have been shown to be responsible for both ssDNA binding and in determining "hotspot" identity. Overexpression of these enzymes has been linked to genomic instability and cancer, thus highlighting the importance of sequencespecific targeting.

Some aspects of this disclosure relate to the recognition that the activity of cytidine deaminase enzymes such as APOBEC enzymes can be directed to a specific site in genomic DNA. Without wishing to be bound by any particular theory, advantages of using Cas9 as a recognition agent include (1) the sequence specificity of Cas9 can be easily altered by simply changing the sgRNA sequence; and (2) Cas9 binds to its target sequence by denaturing the dsDNA, resulting in a stretch of DNA that is single-stranded and therefore a viable substrate for the deaminase. It should be understood that other catalytic domains, or catalytic domains from other deaminases, can also be used to generate fusion proteins with Cas9, and that the disclosure is not limited in this regard.

Some aspects of this disclosure are based on the recognition that Cas9:deaminase fusion proteins can efficiently deaminate nucleotides. In view of the results provided herein regarding the nucleotides that can be targeted by Cas9:deaminase fusion proteins, a person of skill in the art will be able to design suitable guide RNAs to target the fusion proteins to a target sequence that comprises a nucleotide to be deaminated.

In certain embodiments, the reference cytidine deaminase domain comprises a "FERNY" polypeptide having an amino acid sequence according to SEQ ID NO: 127 or an amino acid sequence that is at least 60%, 65%, 70%, 75%, 80%, 85%, 90%, 95, 98%, 99%, or 99.5% identical to SEQ ID NO: 127, as follows:

MFERNYDPRELRKETYLLYEIKWGKSGKLWRHWCQNNRTQHAEVYFLENIFNARR
FNPSTHCSITWYLSWSPCAECSQKIVDFLKEHPNVNLEIYVARLYYHEDERNRQGLR
DLVNSGVTIRIMDLPDYNYCWKTFVSDQGGDEDYWPGHFAPWIKQYSLKL (SEQ ID
NO: 127)

In certain other embodiment, the evolved cytidine deaminase domain (i.e., as a result of the continuous evolution process described herein) comprises a "evoFERNY" polypeptide having an amino acid sequence according to SEQ ID NO: 128 or an amino acid sequence that is at least 60%, 65%, 70%, 75%, 80%, 85%, 90%, 95, 98%, 99%, or 99.5% identical to SEQ ID NO: 128, comprising an H102P and D104N substitutions, as follows:

MFERNYDPRELRKETYLLYEIKWGKSGKLWRHWCQNNRTQHAEVYFLENIFNARR
FNPSTHCSITWYLSWSPCAECSQKIVDFLKEHPNVNLEIYVARLYYPENERNRQGLR
DLVNSGVTIRIMDLPDYNYCWKTFVSDQGGDEDYWPGHFAPWIKQYSLKL (SEQ ID
NO: 128)

In other embodiments, the reference cytidine deaminase domain comprises a "Rat APOBEC-1" polypeptide having an amino acid sequence according to SEQ ID NO: 129 or an amino acid sequence that is at least 60%, 65%, 70%, 75%, 80%, 85%, 90%, 95, 98%, 99%, or 99.5% identical to SEQ ID NO: 129, as follows:




 (SEQ
ID NO: 129)

In certain other embodiment, the evolved cytidine deaminase domain (i.e., as a result of the continuous evolution process described herein) comprises a "evoAPOBEC" polypeptide having an amino acid sequence according to SEQ ID NO: 130 or an amino acid sequence that is at least 60%, 65%, 70%, 75%, 80%, 85%, 90%, 95, 98%, 99%, or 99.5% identical to SEQ ID NO: 130, and comprising substitutions E4K; H109N; H122L; D124N; R154H; A165S; P201S; F205S, as follows:

MSSKTGPVAVDPTLRRRIEPHEFEVFFDPRELRKETCLLYEINWGGRHSIWRHTSQNT
NKHVEVNFIEKFTTERYFCPNTRCSITWFLSWSPCGECSRAITEFLSRYPNVTLFIYIAR
LYHLANPRNRQGLRDLISSGVTIQIMTEQESGYCWHNFVNYSPSNESHWPRYPHLW
VRLYVLELYCIILGLPPCLNILRRKQSQLTSFTIALQSCHYQRLPPHILWATGLK (SEQ
ID NO: 130)

In still other embodiments, the reference cytidine deaminase domain comprises a "Petromyzon marinus CDA1 (pmCDA1)" polypeptide having an amino acid sequence according to SEQ ID NO: 131 or an amino acid sequence that is at least 60%, 65%, 70%, 75%, 80%, 85%, 90%, 95, 98%, 99%, or 99.5% identical to SEQ ID NO: 131, as follows:

MTDAEYVRIHEKLDIYTFKKQFFNNKKSVSHRCYVLFELKRRGERRACFWGYAVNK
PQSGTERGIHAEIFSIRKVEEYLRDNPGQFTINWYSSWSPCADCAEKILEWYNQELRG
NGHTLKIWACKLYYEKNARNQIGLWNLRDNGVGLNVMVSEHYQCCRKIFIQSSHN
QLNENRWLEKTLKRAEKRRSELSIMIQVKILHTTKSPAV (SEQ ID NO: 131)

In other embodiment, the evolved cytidine deaminase domain (i.e., as a result of the continuous evolution process described herein) comprises a "evoCDA" polypeptide having an amino acid sequence according to SEQ ID NO: 132 or an amino acid sequence that is at least 60%, 65%, 70%, 75%, 80%, 85%, 90%, 95, 98%, 99%, or 99.5% identical to SEQ ID NO: 132 and comprising substitutions F23S; A123V; I195F, as follows:

MTDAEYVRIHEKLDIYTFKKQFSNNKKSVSHRCYVLFELKRRGERRACFWGYAVNK
PQSGTERGIHAEIFSIRKVEEYLRDNPGQFTINWYSSWSPCADCAEKILEWYNQELRG
NGHTLKIWVCKLYYEKNARNQIGLWNLRDNGVGLNVMVSEHYQCCRKIFIQSSHN
QLNENRWLEKTLKRAEKRRSELSIMFQVKILHTTKSPAV (SEQ ID NO: 132)

In yet other embodiments, the reference cytidine deaminase domain comprises a "Anc689 APOBEC" polypeptide having an amino acid sequence according to SEQ ID NO: 133 or an amino acid sequence that is at least 60%, 65%, 70%, 75%, 80%, 85%, 90%, 95, 98%, 99%, or 99.5% identical to SEQ ID NO: 133, as follows:

MSSETGPVAVDPTLRRRIEPHEFEVFFDPRELRKETCLLYEIKWGTSHKIWRHSSKNT
TKHVEVNFIEKFTSERHFCPSTSCSITWFLSWSPCGECSKAITEFLSQHPNVTLVIYVA
RLYHHMDQQNRQGLRDLVNSGVTIQIMTAPEYDYCWRNFVNYPPGKEAHWPRYPP
LWMKLYALELHAGILGLPPCLNILRRKQPQLTFFTIALQSCHYQRLPPHILWATGLK
(SEQ ID NO: 133)

In other embodiments, the evolved cytidine deaminase domain (i.e., as a result of the continuous evolution process described herein) comprises a "evoAnc689 APOBEC" polypeptide having an amino acid sequence according to SEQ ID NO: 134 or an amino acid sequence that is at least 60%, 65%, 70%, 75%, 80%, 85%, 90%, 95, 98%, 99%, or 99.5% identical to SEQ ID NO: 134 and comprising substitutions E4K; H122L; D124N; R154H; A165S; P201S; F205S, as follows:

MSSKTGPVAVDPTLRRRIEPHEFEVFFDPRELRKETCLLYEIKWGTSHKIWRHSSKNT
TKHVEVNFIEKFTSERHFCPSTSCSITWFLSWSPCGECSKAITEFLSQHPNVTLVIYVA

RLYHLMNQQNRQGLRDLVNSGVTIQIMTAPEYDYCWHNFVNYPPGKESHWPRYPP
LWMKLYALELHAGILGLPPCLNILRRKQSQLTSFTIALQSCHYQRLPPHILWATGLK
(SEQ ID NO: 134)

**In** some aspects, the specification provides evolved cytidine deaminases which are used to construct base editors that have improved properties. For example, evolved cytidine deaminases, such as those provided herein, are capable of improving base editing efficiency and/or improving the ability of base editors to more efficiently edit bases regardless of the surrounding sequence. For example, in some aspects the disclosure provides evolved APOBEC deaminases (e.g., evolved rAPOBEC1) with improved base editing efficiency in the context of a 5'-G-3' when it is 5' to a target base (e.g., C). In some embodiments, the disclosure provides base editors comprising any of the evolved cytidine deaminases provided herein. It should be appreciated that any of the evolved cytidine deaminases provided herein may be used as a deaminase in a base editor protein, such as any of the base editors provided herein. It should also be appreciated that the disclosure contemplates cytidine deaminases having any of the mutations provided herein, for example any of the mutations described in the Examples section.

### V. Other functional domains

**In** various embodiments, the base editors and their various components may comprise additional functional moieties, such as, but not limited to, linkers, uracil glycosylase inhibitors, nuclear localization signals, split-intein sequences (to join split proteins, such as split napDNAbps, split adenine deaminases, split cytidine deaminases, split CBEs, or split ABEs), and RNA-protein recruitment domains (such as, MS2 tagging system).

### (1) Linkers

**In** certain embodiments, linkers may be used to link any of the protein or protein domains described herein (e.g., a deaminase domain and a Cas9 domain). The linker may be as simple as a covalent bond, or it may be a polymeric linker many atoms in length. In certain embodiments, the linker is a polypeptide or based on amino acids. In other embodiments, the linker is not peptide-like. In certain embodiments, the linker is a covalent bond *(e.g.,* a carbon-carbon bond, disulfide bond, carbon-heteroatom bond, *etc*.). In certain embodiments, the linker is a carbon-nitrogen bond of an amide linkage. In certain embodiments, the linker is a cyclic or acyclic, substituted or unsubstituted, branched or unbranched aliphatic or heteroaliphatic linker. In certain embodiments, the linker is polymeric *(e.g.,* polyethylene, polyethylene glycol, polyamide, polyester, *etc*.). In certain embodiments, the linker comprises a monomer, dimer, or polymer of aminoalkanoic acid. In certain embodiments, the linker comprises an aminoalkanoic acid (*e.g*., glycine, ethanoic acid, alanine, beta-alanine, 3-aminopropanoic acid, 4-aminobutanoic acid, 5-pentanoic acid, *etc*.). In certain embodiments, the linker comprises a monomer, dimer, or polymer of aminohexanoic acid (Ahx). In certain embodiments, the linker is based on a carbocyclic moiety (*e.g*., cyclopentane, cyclohexane). In other embodiments, the linker comprises a polyethylene glycol moiety (PEG). In other embodiments, the linker comprises amino acids. In certain embodiments, the linker comprises a peptide. In certain embodiments, the linker comprises an aryl or heteroaryl moiety. In certain embodiments, the linker is based on a phenyl ring. The linker may include functionalized moieties to facilitate attachment of a nucleophile (*e.g*., thiol, amino) from the peptide to the linker. Any electrophile may be used as part of the linker. Exemplary electrophiles include, but are not limited to, activated esters, activated amides, Michael acceptors, alkyl halides, aryl halides, acyl halides, and isothiocyanates.

In some embodiments, the linker is an amino acid or a plurality of amino acids *(e.g.,* a peptide or protein). In some embodiments, the linker is a bond *e.g.,* a covalent bond), an organic molecule, group, polymer, or chemical moiety. In some embodiments, the linker is 5-100 amino acids in length, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35-40, 40-45, 45-50, 50-60, 60-70, 70-80, 80-90, 90-100, 100-110, 110-120, 120-130, 130-140, 140-150, or 150-200 amino acids in length. Longer or shorter linkers are also contemplated. In some embodiments, a linker comprises the amino acid sequence SGSETPGTSESATPES (SEQ ID NO: 143), which may also be referred to as the XTEN linker. In some embodiments, the linker is 32 amino acids in length. In some embodiments, the linker comprises the amino acid sequence (SGGS)₂-SGSETPGTSESATPES-(SGGS)₂ (SEQ ID NO: 144), which may also be referred to as (SGGS)₂-XTEN-(SGGS)₂ (SEQ ID NO: 144). In some embodiments, the linker comprises the amino acid sequence, wherein n is 0, 1, 2, 3, 4, 5, 6, 7, 8, 9, or 10. In some embodiments, a linker comprises the amino acid sequence SGGS (SEQ ID NO: 138). In some embodiments, a linker comprises (SGGS)ₙ (SEQ ID NO: 139), (GGGS)ₙ (SEQ ID NO: 140), (GGGGS)ₙ (SEQ ID NO: 141), (G)ₙ (SEQ ID NO: 135), (EAAAK)ₙ (SEQ ID NO: 142), (SGGS)ₙ-SGSETPGTSESATPES-(SGGS)ₙ (SEQ ID NO: 145), (GGS)n (SEQ ID NO: 137), SGSETPGTSESATPES (SEQ ID NO: 143), or (XP)ₙ (SEQ ID NO: 136) motif, or a combination of any of these, wherein n is independently an integer between 1 and 30, and wherein X is any amino acid. In some embodiments, n is 1, 2, 3, 4, 5, 6, 7, 8, 9, 10, 11, 12, 13, 14, or 15. In some embodiments, a linker comprises SGSETPGTSESATPES (SEQ ID NO: 143), and SGGS (SEQ ID NO: 138). In some embodiments, a linker comprises SGGSSGSETPGTSESATPESSGGS (SEQ ID NO: 145). In some embodiments, a linker comprises SGGSSGGSSGSETPGTSESATPESSGGSSGGS (SEQ ID NO: 144). In some embodiments, a linker comprises GGSGGSPGSPAGSPTSTEEGTSESATPESGPGTSTEPSEGSAPGSPAGSPTSTEEGTSTE PSEGSAPGTSTEPSEGSAPGTSESATPESGPGSEPATSGGSGGS (SEQ ID NO: 151). In some embodiments, the linker is 24 amino acids in length. In some embodiments, the linker comprises the amino acid sequence SGGSSGGSSGSETPGTSESATPES (SEQ ID NO: 146). In some embodiments, the linker is 40 amino acids in length. In some embodiments, the linker comprises the amino acid sequence SGGSSGGSSGSETPGTSESATPESSGGSSGGSSGGSSGGS (SEQ ID NO: 148). In some embodiments, the linker is 64 amino acids in length. In some embodiments, the linker comprises the amino acid sequence SGGSSGGSSGSETPGTSESATPESSGGSSGGSSGGSSGGSSGSETPGTSESATPESSGGS SGGS (SEQ ID NO: 149). In some embodiments, the linker is 92 amino acids in length. In some embodiments, the linker comprises the amino acid sequence PGSPAGSPTSTEEGTSESATPESGPGTSTEPSEGSAPGSPAGSPTSTEEGTSTEPSEGSAP GTSTEPSEGSAPGTSESATPESGPGSEPATS (SEQ ID NO: 150). It should be appreciated that any of the linkers provided herein may be used to link a first adenosine deaminase and a second adenosine deaminase; an adenosine deaminase (*e.g.*, a first or a second adenosine deaminase) and a napDNAbp; a napDNAbp and an NLS; or an adenosine deaminase (*e.g*., a first or a second adenosine deaminase) and an NLS.

In some embodiments, any of the fusion proteins provided herein, comprise an adenosine or a cytidine deaminase and a napDNAbp that are fused to each other via a linker. In some embodiments, any of the fusion proteins provided herein, comprise a first adenosine deaminase and a second adenosine deaminase that are fused to each other via a linker. In some embodiments, any of the fusion proteins provided herein, comprise an NLS, which may be fused to an adenosine deaminase (*e.g*., a first and/or a second adenosine deaminase), a nucleic acid programmable DNA binding protein (napDNAbp). Various linker lengths and flexibilities between an adenosine deaminase (*e.g*., an engineered ecTadA) and a napDNAbp *(e.g.,* a Cas9 domain), and/or between a first adenosine deaminase and a second adenosine deaminase can be employed (*e.g.*, ranging from very flexible linkers of the form (GGGGS)ₙ (SEQ ID NO: 141), (GGGGS)ₙ (SEQ ID NO: 141), and (G)ₙ (SEQ ID NO: 135) to more rigid linkers of the form (EAAAK)ₙ (SEQ ID NO: 142), (SGGS)ₙ (SEQ ID NO: 139), SGSETPGTSESATPES (SEQ ID NO: 143) (see, *e.g.,* Guilinger JP, Thompson DB, Liu DR. Fusion of catalytically inactive Cas9 to FokI nuclease improves the specificity of genome modification. Nat. Biotechnol. 2014; 32(6): 577-82; the entire contents are incorporated herein by reference) and (XP)ₙ (SEQ ID NO: 136)) in order to achieve the optimal length for deaminase activity for the specific application. In some embodiments, n is 1, 2, 3, 4, 5, 6, 7, 8, 9, 10, 11, 12, 13, 14, or 15. In some embodiments, the linker comprises a (GGS)ₙ (SEQ ID NO: 137) motif, wherein n is 1, 3, or 7. In some embodiments, the adenosine deaminase and the napDNAbp, and/or the first adenosine deaminase and the second adenosine deaminase of any of the fusion proteins provided herein are fused via a linker comprising the amino acid sequence SGSETPGTSESATPES (SEQ ID NO: 143), SGGS (SEQ ID NO: 138), SGGSSGSETPGTSESATPESSGGS (SEQ ID NO: 145), SGGSSGGSSGSETPGTSESATPESSGGSSGGS (SEQ ID NO: 144), or GGSGGSPGSPAGSPTSTEEGTSESATPESGPGTSTEPSEGSAPGSPAGSPTSTEEGTSTE PSEGSAPGTSTEPSEGSAPGTSESATPESGPGSEPATSGGSGGS (SEQ ID NO: 151). In some embodiments, the linker is 24 amino acids in length. In some embodiments, the linker comprises the amino acid sequence SGGSSGGSSGSETPGTSESATPES (SEQ ID NO: 146). In some embodiments, the linker is 32 amino acids in length. In some embodiments, the linker is 32 amino acids in length. In some embodiments, the linker comprises the amino acid sequence (SGGS)₂-SGSETPGTSESATPES-(SGGS)₂ (SEQ ID NO: 144), which may also be referred to as (SGGS)₂-XTEN-(SGGS)₂ (SEQ ID NO: 144). In some embodiments, the linker comprises the amino acid sequence, wherein n is 0, 1, 2, 3, 4, 5, 6, 7, 8, 9, or 10. In some embodiments, the linker is 40 amino acids in length. In some embodiments, the linker comprises the amino acid sequence SGGSSGGSSGSETPGTSESATPESSGGSSGGSSGGSSGGS (SEQ ID NO: 148). In some embodiments, the linker is 64 amino acids in length. In some embodiments, the linker comprises the amino acid sequence SGGSSGGSSGSETPGTSESATPESSGGSSGGSSGGSSGGSSGSETPGTSESATPESSGGS SGGS (SEQ ID NO: 149). In some embodiments, the linker is 92 amino acids in length. In some embodiments, the linker comprises the amino acid sequence

PGSPAGSPTSTEEGTSESATPESGPGTSTEPSEGSAPGSPAGSPTSTEEGTSTEPSEGSAP
GTSTEPSEGSAPGTSESATPESGPGSEPATS (SEQ ID NO: 150).

### (2) UGI domain

In other embodiments, the base editors described herein may comprise one or more uracil glycosylase inhibitors. The term "uracil glycosylase inhibitor" or "UGI," as used herein, refers to a protein that is capable of inhibiting a uracil-DNA glycosylase base-excision repair enzyme. In some embodiments, a UGI domain comprises a wild-type UGI or a UGI as set forth in SEQ ID NO: 163. In some embodiments, the UGI proteins provided herein include fragments of UGI and proteins homologous to a UGI or a UGI fragment. For example, in some embodiments, a UGI domain comprises a fragment of the amino acid sequence set forth in SEQ ID NO: 163. In some embodiments, a UGI fragment comprises an amino acid sequence that comprises at least 60%, at least 65%, at least 70%, at least 75%, at least 80%, at least 85%, at least 90%, at least 95%, at least 96%, at least 97%, at least 98%, at least 99%, or at least 99.5% of the amino acid sequence as set forth in SEQ ID NO: 163. In some embodiments, a UGI comprises an amino acid sequence homologous to the amino acid sequence set forth in SEQ ID NO: 163, or an amino acid sequence homologous to a fragment of the amino acid sequence set forth in SEQ ID NO: 163. In some embodiments, proteins comprising UGI or fragments of UGI or homologs of UGI or UGI fragments are referred to as "UGI variants." A UGI variant shares homology to UGI, or a fragment thereof. For example a UGI variant is at least 70% identical, at least 75% identical, at least 80% identical, at least 85% identical, at least 90% identical, at least 95% identical, at least 96% identical, at least 97% identical, at least 98% identical, at least 99% identical, at least 99.5% identical, or at least 99.9% identical to a wild type UGI or a UGI as set forth in SEQ ID NO: 163. In some embodiments, the UGI variant comprises a fragment of UGI, such that the fragment is at least 70% identical, at least 80% identical, at least 90% identical, at least 95% identical, at least 96% identical, at least 97% identical, at least 98% identical, at least 99% identical, at least 99.5% identical, or at least 99.9% to the corresponding fragment of wild-type UGI or a UGI as set forth in SEQ ID NO: 163. In some embodiments, the UGI comprises the following amino acid sequence:
Uracil-DNA glycosylase inhibitor:
>sp|P14739|UNGI_BPPB2

MTNLSDIIEKETGKQLVIQESILMLPEEVEEVIGNKPESDILVHTAYDESTDENVMLLT
SDAPEYKPWALVIQDSNGENKIKML (SEQ ID NO: 163).

The base editors described herein may comprise more than one UGI domain, which may be separated by one or more linkers as described herein. It will also be understood that in the context of the herein disclosed base editors, the UGI domain may be linked to a deaminase domain or

### (3) NLS domains

In various embodiments, the base editor proteins may comprise one or more nuclear localization sequences (NLS), which help promote translocation of a protein into the cell nucleus. Such sequences are well-known in the art and can include the following examples:

| DESCRIPTI ON | SEQUENCE | SEQUENCE IDENTIFIER |
|---|---|---|
| NLS OF SV40 LARGE TAG | PKKKRKV | 152 |
| NLS OF POLYOMA LARGE TAG | VSRKRPRP | 153 |
| NLS OF C-MYC | PAAKRVKLD | 155 |
| NLS OF TUS-PROTEIN | KLKIKRPVK | 154 |
| NLS OF HEPATITIS D VIRUS ANTIGEN | EGAPPAKRAR | 156 |
| NLS OF MURINE P53 | PPQPKKKPLDGE | 157 |
| NLS | MKRTADGSEFESPKKKRKV | 158 |
| NLS OF NUCLEOPL ASMIN | AVKRPAATKKAGQAKKKKLD | 159 |
| NLS OF PE1 AND PE2 | SGGSKRTADGSEFEPKKKRKV | 160 |
| NLS OF EGL-13 | MSRRRKANPTKLSENAKKLAKEVEN | 161 |
| NLS | MDSLLMNRRKFLYQFKNVRWAKGRRETYLC | 162 |

The NLS examples above are non-limiting. The base editor proteins may comprise any known NLS sequence, including any of those described in Cokol et al., "Finding nuclear localization signals," EMBO Rep., 2000, 1(5): 411-415 and Freitas et al., "Mechanisms and Signals for the Nuclear Import of Proteins," Current Genomics, 2009, 10(8): 550-7, each of which are incorporated herein by reference.

In various embodiments, the base editors and constructs encoding the base editors disclosed herein further comprise one or more, preferably, at least two nuclear localization signals. In certain embodiments, the base editors comprise at least two NLSs. In embodiments with at least two NLSs, the NLSs can be the same NLSs or they can be different NLSs. In addition, the NLSs may be expressed as part of a fusion protein with the remaining portions of the base editors. In some embodiments, one or more of the NLSs are bipartite NLSs ("bpNLS"). In certain embodiments, the disclosed fusion proteins comprise two bipartite NLSs. In some embodiments, the disclosed fusion proteins comprise more than two bipartite NLSs.

The location of the NLS fusion can be at the N-terminus, the C-terminus, or within a sequence of a base editor (e.g., inserted between the encoded napDNAbp component (e.g., Cas9) and a deaminase (e.g., a cytidine of adenine deaminase).

The NLSs may be any known NLS sequence in the art. The NLSs may also be any future-discovered NLSs for nuclear localization. The NLSs also may be any naturally-occurring NLS, or any non-naturally occurring NLS (e.g., an NLS with one or more desired mutations).
The term "nuclear localization sequence" or "NLS" refers to an amino acid sequence that promotes import of a protein into the cell nucleus, for example, by nuclear transport. Nuclear localization sequences are known in the art and would be apparent to the skilled artisan. For example, NLS sequences are described in Plank et al., International PCT application PCT/EP2000/011690, filed November 23, 2000, published as WO/2001/038547 on May 31, 2001, the contents of which are incorporated herein by reference. In some embodiments, an NLS comprises the amino acid sequence PKKKRKV (SEQ ID NO: 152), MDSLLMNRRKFLYQFKNVRWAKGRRETYLC (SEQ ID NO: 162), KRTADGSEFESPKKKRKV (SEQ ID NO: 467), or KRTADGSEFEPKKKRKV (SEQ ID NO: 468). In other embodiments, NLS comprises the amino acid sequences NLSKRPAAIKKAGQAKKKK (SEQ ID NO: 469), PAAKRVKLD (SEQ ID NO: 155), RQRRNELKRSF (SEQ ID NO: 470), NQSSNFGPMKGGNFGGRSSGPYGGGGQYFAKPRNQGGY (SEQ ID NO: 471).

In one aspect of the disclosure, a base editor may be modified with one or more nuclear localization signals (NLS), preferably at least two NLSs. In certain embodiments, the base editors are modified with two or more NLSs. The disclosure contemplates the use of any nuclear localization signal known in the art at the time of the disclosure, or any nuclear localization signal that is identified or otherwise made available in the state of the art after the time of the instant filing. A representative nuclear localization signal is a peptide sequence that directs the protein to the nucleus of the cell in which the sequence is expressed. A nuclear localization signal is predominantly basic, can be positioned almost anywhere in a protein's amino acid sequence, generally comprises a short sequence of four amino acids (Autieri & Agrawal, (1998) J. Biol. Chem. 273: 14731-37, incorporated herein by reference) to eight amino acids, and is typically rich in lysine and arginine residues (Magin et al., (2000) Virology 274: 11-16, incorporated herein by reference). Nuclear localization signals often comprise proline residues. A variety of nuclear localization signals have been identified and have been used to effect transport of biological molecules from the cytoplasm to the nucleus of a cell. See, e.g., Tinland et al., (1992) Proc. Natl. Acad. Sci. U.S.A. 89:7442-46; Moede et al., (1999) FEBS Lett. 461:229-34, which is incorporated by reference. Translocation is currently thought to involve nuclear pore proteins.

Most NLSs can be classified in three general groups: (i) a monopartite NLS exemplified by the SV40 large T antigen NLS (PKKKRKV (SEQ ID NO: 152)); (ii) a bipartite motif consisting of two basic domains separated by a variable number of spacer amino acids and exemplified by the *Xenopus nucleoplasmin* NLS (KRXXXXXXXXXXKKKL (SEQ ID NO: 472)); and (iii) noncanonical sequences such as M9 of the hnRNP Al protein, the influenza virus nucleoprotein NLS, and the yeast Gal4 protein NLS (Dingwall and Laskey 1991).
Nuclear localization signals appear at various points in the amino acid sequences of proteins. NLS's have been identified at the N-terminus, the C-terminus and in the central region of proteins. Thus, the disclosure provides base editors that may be modified with one or more NLSs at the C-terminus, the N-terminus, as well as at in internal region of the base editor. The residues of a longer sequence that do not function as component NLS residues should be selected so as not to interfere, for example tonically or sterically, with the nuclear localization signal itself. Therefore, although there are no strict limits on the composition of an NLS-comprising sequence, in practice, such a sequence can be functionally limited in length and composition.

The present disclosure contemplates any suitable means by which to modify a base editor to include one or more NLSs. In one aspect, the base editors may be engineered to express a base editor protein that is translationally fused at its N-terminus or its C-terminus (or both) to one or more NLSs, i.e., to form a base editor-NLS fusion construct. In other embodiments, the base editor-encoding nucleotide sequence may be genetically modified to incorporate a reading frame that encodes one or more NLSs in an internal region of the encoded base editor. In addition, the NLSs may include various amino acid linkers or spacer regions encoded between the base editor and the N-terminally, C-terminally, or internallyattached NLS amino acid sequence, e.g, and in the central region of proteins. Thus, the present disclosure also provides for nucleotide constructs, vectors, and host cells for expressing fusion proteins that comprise a base editor and one or more NLSs.

The base editors described herein may also comprise nuclear localization signals which are linked to a base editor through one or more linkers, e.g., and polymeric, amino acid, nucleic acid, polysaccharide, chemical, or nucleic acid linker element. The linkers within the contemplated scope of the disclosure are not intended to have any limitations and can be any suitable type of molecule (e.g., polymer, amino acid, polysaccharide, nucleic acid, lipid, or any synthetic chemical linker domain) and be joined to the base editor by any suitable strategy that effectuates forming a bond (e.g., covalent linkage, hydrogen bonding) between the base editor and the one or more NLSs.

### (4) Split-intein domains

It will be understood that in some embodiments (e.g., delivery of a base editor *in vivo* using AAV particles), it may be advantageous to split a polypeptide (e.g., a deaminase or a napDNAbp) or a fusion protein (e.g., a base editor) into an N-terminal half and a C-terminal half, delivery them separately, and then allow their colocalization to reform the complete protein (or fusion protein as the case may be) within the cell. Separate halves of a protein or a fusion protein may each comprise a split-intein tag to facilitate the reformation of the complete protein or fusion protein by the mechanism of protein trans splicing.

Protein trans-splicing, catalyzed by split inteins, provides an entirely enzymatic method for protein ligation. A split-intein is essentially a contiguous intein (e.g. a mini-intein) split into two pieces named N-intein and C-intein, respectively. The N-intein and C-intein of a split intein can associate non-covalently to form an active intein and catalyze the splicing reaction essentially in same way as a contiguous intein does. Split inteins have been found in nature and also engineered in laboratories. As used herein, the term "split intein" refers to any intein in which one or more peptide bond breaks exists between the N-terminal and C-terminal amino acid sequences such that the N-terminal and C-terminal sequences become separate molecules that can non-covalently reassociate, or reconstitute, into an intein that is functional for trans-splicing reactions. Any catalytically active intein, or fragment thereof, may be used to derive a split intein for use in the methods of the invention. For example, in one aspect the split intein may be derived from a eukaryotic intein. In another aspect, the split intein may be derived from a bacterial intein. In another aspect, the split intein may be derived from an archaeal intein. Preferably, the split intein so-derived will possess only the amino acid sequences essential for catalyzing trans-splicing reactions.

As used herein, the "N-terminal split intein (In)" refers to any intein sequence that comprises an N- terminal amino acid sequence that is functional for trans-splicing reactions. An In thus also comprises a sequence that is spliced out when trans-splicing occurs. An In can comprise a sequence that is a modification of the N-terminal portion of a naturally occurring intein sequence. For example, an In can comprise additional amino acid residues and/or mutated residues so long as the inclusion of such additional and/or mutated residues does not render the In non-functional in trans-splicing. Preferably, the inclusion of the additional and/or mutated residues improves or enhances the trans-splicing activity of the In.

As used herein, the "C-terminal split intein (Ic)" refers to any intein sequence that comprises a C- terminal amino acid sequence that is functional for trans-splicing reactions. In one aspect, the Ic comprises 4 to 7 contiguous amino acid residues, at least 4 amino acids of which are from the last β-strand of the intein from which it was derived. An Ic thus also comprises a sequence that is spliced out when trans-splicing occurs. An Ic can comprise a sequence that is a modification of the C-terminal portion of a naturally occurring intein sequence. For example, an Ic can comprise additional amino acid residues and/or mutated residues so long as the inclusion of such additional and/or mutated residues does not render the In non-functional in trans-splicing. Preferably, the inclusion of the additional and/or mutated residues improves or enhances the trans-splicing activity of the Ic.

In some embodiments of the invention, a peptide linked to an Ic or an In can comprise an additional chemical moiety including, among others, fluorescence groups, biotin, polyethylene glycol (PEG), amino acid analogs, unnatural amino acids, phosphate groups, glycosyl groups, radioisotope labels, and pharmaceutical molecules. In other embodiments, a peptide linked to an Ic can comprise one or more chemically reactive groups including, among others, ketone, aldehyde, Cys residues and Lys residues. The N-intein and C-intein of a split intein can associate non-covalently to form an active intein and catalyze the splicing reaction when an "intein-splicing polypeptide (ISP)" is present. As used herein, "intein-splicing polypeptide (ISP)" refers to the portion of the amino acid sequence of a split intein that remains when the Ic, In, or both, are removed from the split intein. In certain embodiments, the In comprises the ISP. In another embodiment, the Ic comprises the ISP. In yet another embodiment, the ISP is a separate peptide that is not covalently linked to In nor to Ic.

Split inteins may be created from contiguous inteins by engineering one or more split sites in the unstructured loop or intervening amino acid sequence between the -12 conserved beta-strands found in the structure of mini-inteins. Some flexibility in the position of the split site within regions between the beta-strands may exist, provided that creation of the split will not disrupt the structure of the intein, the structured beta-strands in particular, to a sufficient degree that protein splicing activity is lost.

In protein trans-splicing, one precursor protein consists of an N-extein part followed by the N-intein, another precursor protein consists of the C-intein followed by a C-extein part, and a trans-splicing reaction (catalyzed by the N- and C-inteins together) excises the two intein sequences and links the two extein sequences with a peptide bond. Protein trans-splicing, being an enzymatic reaction, can work with very low (e.g. micromolar) concentrations of proteins and can be carried out under physiological conditions.

Exemplary sequences are as follows:

| NAME | SEQUENCE OF LIGAND-DEPENDENT INTEIN |
|---|---|
| 2-4 INTEIN: | CLAEGTRIFDPVTGTTHRIEDVVDGRKPIHVVAAAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 164) |
| 3-2 INTEIN | CLAEGTRIFDPVTGTTHRIEDVVDGRKPIHVVAVAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYTNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 165) |
| 30R3-1 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPIPYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC (SEQ ID NO: 166) |
| 30R3-2 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 167) |
| 30R3-3 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPIPYSEYDPTSPFSEASMMGLLTNLADRE LVHMINWAKRVPGFVDLTLHDQAHLLECAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 168) |
| 37R3-1 INTEIN | CLAEGTRIFDPVTGTTHRIEDVVDGRKPIHVVAAAKDGTLLARPVVSWFD QGTRDVIGLRIAGGATVWATPDHKVLTEYGWRAAGELRKGDRVAGPGGS GNSLALSLTADQMVSALLDAEPPILYSEYNPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC ((SEQ ID NO: 169) |
| 37R3-2 INTEIN | CLAEGTRIFDPVTGTTHRIEDVVDGRKPIHVVAAAKDGTLLARPVVSWFD QGTRDVIGLRIAGGAIVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEGLRYSVIREVLPTRRARTFDLEVEE LHTLVAEGVVVHNC (SEQ ID NO: 170) |
| 37R3-3 INTEIN | CLAEGTRIFDPVTGTTHRIEDVVDGRKPIHVVAVAKDGTLLARPVVSWFD QGTRDVIGLRIAGGATVWATPDHKVLTEYGWRAAGELRKGDRVAGPGGS GNSLALSLTADQMVSALLDAEPPILYSEYDPTSPFSEASMMGLLTNLADRE LVHMINWAKRVPGFVDLTLHDQAHLLERAWLEILMIGLVWRSMEHPGKL LFAPNLLLDRNQGKCVEGMVEIFDMLLATSSRFRMMNLQGEEFVCLKSII LLNSGVYTFLSSTLKSLEEKDHIHRALDKITDTLIHLMAKAGLTLQQQHQR LAQLLLILSHIRHMSNKGMEHLYSMKYKNVVPLYDLLLEMLDAHRLHAG GSGASRVQAFADALDDKFLHDMLAEELRYSVIREVLPTRRARTFDLEVEE LHTLVAEGVVVHNC (SEQ ID NO: 171) |

Although inteins are most frequently found as a contiguous domain, some exist in a naturally split form. In this case, the two fragments are expressed as separate polypeptides and must associate before splicing takes place, so-called protein trans-splicing.

An exemplary split intein is the *Ssp* DnaE intein, which comprises two subunits, namely, DnaE-N and DnaE-C. The two different subunits are encoded by separate genes, namely *dnaE-n* and *dnaE-c,* which encode the DnaE-N and DnaE-C subunits, respectively. DnaE is a naturally occurring split intein in *Synechocytis sp.* PCC6803 and is capable of directing trans-splicing of two separate proteins, each comprising a fusion with either DnaE-N or DnaE-C.

Additional naturally occurring or engineered split-intein sequences are known in the art or can be made from whole-intein sequences described herein or those available in the art. Examples of split-intein sequences can be found in Stevens et al., "A promiscuous split intein with expanded protein engineering applications," PNAS, 2017, Vol.114: 8538-8543; Iwai et al., "Highly efficient protein trans-splicing by a naturally split DnaE intein from Nostc punctiforme, FEBS Lett, 580: 1853-1858, each of which are incorporated herein by reference. Additional split intein sequences can be found, for example, in WO 2013/045632, WO 2014/055782, WO 2016/069774, and EP2877490, the contents each of which are incorporated herein by reference.
In addition, protein splicing in trans has been described *in vivo and in vitro* (Shingledecker, et al., Gene 207:187 (1998), Southworth, et al., EMBO J. 17:918 (1998); Mills, et al., Proc. Natl. Acad. Sci. USA, 95:3543-3548 (1998); Lew, et al., J. Biol. Chem., 273:15887-15890 (1998); Wu, et al., Biochim. Biophys. Acta 35732:1 (1998b), Yamazaki, et al., J. Am. Chem. Soc. 120:5591 (1998), Evans, et al., J. Biol. Chem. 275:9091 (2000); Otomo, et al., Biochemistry 38:16040-16044 (1999); Otomo, et al., J. Biolmol. NMR 14:105-114 (1999); Scott, et al., Proc. Natl. Acad. Sci. USA 96:13638-13643 (1999)) and provides the opportunity to express a protein as to two inactive fragments that subsequently undergo ligation to form a functional product.

### (5) RNA-protein recruitment system

**In** various embodiments, two separate protein domains (e.g., a Cas9 domain and a cytidine deaminase domain) may be colocalized to one another to form a functional complex (akin to the function of a fusion protein comprising the two separate protein domains) by using an "RNA-protein recruitment system," such as the "MS2 tagging technique." Such systems generally tag one protein domain with an "RNA-protein interaction domain" (aka "RNA-protein recruitment domain") and the other with an "RNA-binding protein" that specifically recognizes and binds to the RNA-protein interaction domain, e.g., a specific hairpin structure. These types of systems can be leveraged to colocalize the domains of a base editor, as well as to recruitment additional functionalities to a base editor, such as a UGI domain. In one example, the MS2 tagging technique is based on the natural interaction of the MS2 bacteriophage coat protein ("MCP" or "MS2cp") with a stem-loop or hairpin structure present in the genome of the phage, i.e., the "MS2 hairpin." In the case of the MS2 hairpin, it is recognized and bound by the MS2 bacteriophage coat protein (MCP). Thus, in one exemplarly scenario a deaminase-MS2 fusion can recruit a Cas9-MCP fusion.

A review of other modular RNA-protein interaction domains are described in the art, for example, in Johansson et al., "RNA recognition by the MS2 phage coat protein," Sem Virol., 1997, Vol. 8(3): 176-185; Delebecque et al., "Organization of intracellular reactions with rationally designed RNA assemblies," Science, 2011, Vol. 333: 470-474; Mali et al., "Cas9 transcriptional activators for target specificity screening and paired nickases for cooperative genome engineering," Nat. Biotechnol., 2013, Vol.31: 833-838; and Zalatan et al., "Engineering complex synthetic transcriptional programs with CRISPR RNA scaffolds," Cell, 2015, Vol.160: 339-350, each of which are incorporated herein by reference in their entireties. Other systems include the PP7 hairpin, which specifically recruits the PCP protein, and the "com" hairpin, which specifically recruits the Com protein. See Zalatan et al.

The nucleotide sequence of the MS2 hairpin (or equivalently referred to as the "MS2 aptamer") is: GCCAACATGAGGATCACCCATGTCTGCAGGGCC (SEQ ID NO: 172).

The amino acid sequence of the MCP or MS2cp is:

GSASNFTQFVLVDNGGTGDVTVAPSNFANGVAEWISSNSRSQAYKVTCSVRQSSAQ
NRKYTIKVEVPKVATQTVGGEELPVAGWRSYLNMELTIPIFATNSDCELIVKAMQGL
LKDGNPIPSAIAANSGIY (SEQ ID NO: 173).

### VI. Base editors

**In** various aspects, the instant specification provides base editors and methods of using the same, along with a suitable guide RNA, to treat Spinal Muscular Atrophy (SMA) by installing precise nucleobase changes in the *SMN2* gene such the resulting product has increased stability and/or activity. In one embodiment, the inventors surprisingly found that adenosine base editors could be used to efficiently edit C840T in the *SMN2* gene both *in vitro* and *in vivo,* which is useful for the treatment of SMA.

The state of the art has described numerous base editors as of this filing. It will be understood that the methods and approaches herein described for editing the *SMN2* gene locus may be applied to any previously known base editor, or to base editors that may be developed in the future.

Exemplary base editors that may be used in accordance with the present disclosure include those described in the following references and/or patent publications, each of which are incorporated by reference in their entireties: (a) PCT/US2014/070038 (published as WO2015/089406, June 18, 2015) and its equivalents in the US or around the world; (b) PCT/US2016/058344 (published as WO2017/070632, April 27, 2017) and its equivalents in the US or around the world; (c) PCT/US2016/058345 (published as WO2017/070633, April 27. 2017) and its equivalent in the US or around the world; (d) PCT/US2017/045381 (published as WO2018/027078, February 8, 2018) and its equivalents in the US or around the world; (e) PCT/US2017/056671 (published as WO2018/071868, April 19, 2018) and its equivalents in the US or around the world; PCT/2017/048390 (WO2017/048390, March 23, 2017) and its equivalents in the US or around the world; (f) PCT/US2017/068114 (not published) and its equivalents in the US or around the world; (g) PCT/US2017/068105 (not published)and its equivalents in the US or around the world; (h) PCT/US2017/046144 (WO2018/031683, February 15, 2018) and its equivalents in the US or around the world; (i) PCT/US2018/024208 (not published) and its equivalents in the US or around the world; (j) PCT/2018/021878 (WO2018/021878, February 1, 2018) and its equivalents in the US and around the world; (k) Komor, A.C., Kim, Y.B., Packer, M.S., Zuris, J.A. & Liu, D.R. Programmable editing of a target base in genomic DNA without double-stranded DNA cleavage. Nature 533, 420- (2016); (1) Gaudelli, N.M. et al. Programmable base editing of A.T to G.C in genomic DNA without DNA cleavage. Nature 551, 464- (2017); (m) Gehrke, et al. An APOBEC3A-Cas9 base editor with minimized bystander and off-target activities. Nat. Biotechnol. 36(10), 977- (2018); (n) Lee, S. et al. Single C-to-T substitution using engineered APOBEC3G-nCas9 base editors with minimum genome- and transcriptome-wide off-target effects. Science Advances 6, eaba1773 (2020); (o) Gaudelli, N. M. et al. "Directed Evolution of Adenine Base Editors with Increased Activity and Therapeutic Application." Nat. Biotechnol. 38, 892-900 (2020); (p) Yu, Y. et al. "Next-generation cytosine base editors with minimized unguided DNA and RNA off-target events and high on-target activity." Nat. Commun. 11 (2020); and (q) any of the references listed in this specification entitled "References" and which reports or describes a base editor known in the art.

In various aspects, the improved or modified base editors described herein have the following generalized structures:

[A] - [B] or [B] - [A],

wherein [A] is a napDNAbp and [B] is nucleic acid effector domain (e.g., an adenosine deaminase, or cytidine deaminase), and "]-[" represents an optional a linker that joins the [A] and [B] domains together, either covalently or non-covalently.

Such base editors may also comprise one or more additional functional moieties, [C], such as UGI domains or NLS domains, joined optionally through a linker to [A] and/or [B].

In some embodiments, the base editors provided herein can be made as a recombinant fusion protein comprising one or more protein domains, thereby generating a base editor. In certain embodiments, the base editors provided herein comprise one or more features that improve the base editing activity (e.g., efficiency, selectivity, and/or specificity) of the base editor proteins. For example, the base editor proteins provided herein may comprise a Cas9 domain that has reduced nuclease activity. In some embodiments, the base editor proteins provided herein may have a Cas9 domain that does not have nuclease activity (dCas9), or a Cas9 domain that cuts one strand of a duplexed DNA molecule, referred to as a Cas9 nickase (nCas9). Without wishing to be bound by any particular theory, the presence of the catalytic residue *(e.g.,* H840) maintains the activity of the Cas9 to cleave the non-edited *(e.g.,* nondeaminated) strand containing a T opposite the targeted A. Mutation of the catalytic residue *(e.g.,* D10 to A10) of Cas9 prevents cleavage of the edited strand containing the targeted A residue. Such Cas9 variants are able to generate a single-strand DNA break (nick) at a specific location based on the gRNA-defined target sequence, leading to repair of the non-edited strand, ultimately resulting in a T to C change on the non-edited strand.

In particular, the disclosure provides adenosine base editors that can be used to edit C840T in an *SMN2* gene to treat SMA. Exemplary domains used in base editing fusion proteins, including adenosine deaminases, napDNA/RNAbp (*e.g*., Cas9), and nuclear localization sequences (NLSs) are described in further detail below.

Some aspects of the disclosure provide fusion proteins comprising a nucleic acid programmable DNA binding protein (napDNAbp) and an adenosine deaminase. In some embodiments, any of the fusion proteins provided herein is a base editor. In some embodiments, the napDNAbp is a Cas9 domain, a Cpf1 domain, a CasX domain, a CasY domain, a C2c1 domain, a C2c2 domain, aC2c3 domain, or an Argonaute domain. In some embodiments, the napDNAbp is any napDNAbp provided herein. Some aspects of the disclosure provide fusion proteins comprising a Cas9 domain and an adenosine deaminase. The Cas9 domain may be any of the Cas9 domains or Cas9 proteins (*e.g*., dCas9 or nCas9) provided herein. In some embodiments, any of the Cas9 domains or Cas9 proteins (*e.g*., dCas9 or nCas9) provided herein may be fused with any of the deaminases provided herein. In some embodiments, the fusion protein comprises the structure:

NH₂-[deaminase]-[napDNAbp]-COOH;

or

NH₂-[napDNAbp]-[deaminase]-COOH

In some embodiments, the fusion proteins comprising a deaminase and a napDNAbp *(e.g.,* Cas9 domain) do not include a linker sequence. In some embodiments, a linker is present between the deaminase domain and the napDNAbp . In some embodiments, the "]-[" used in the general architecture above indicates the presence of an optional linker. In some embodiments, the deaminase and the napDNAbp are fused via any of the linkers provided herein. For example, in some embodiments the deaminase and the napDNAbp are fused via any of the linkers provided below in the section entitled "Linkers". In some embodiments, the deaminase and the napDNAbp are fused via a linker that comprises between 1 and and 200 amino acids. In some embodiments, the adenosine deaminase and the napDNAbp are fused via a linker that comprises from 1 to 5, 1 to 10, 1 to 20, 1 to 30, 1 to 40, 1 to 50, 1 to 60, 1 to 80, 1 to 100, 1 to 150, 1 to 200, 5 to 10, 5 to 20, 5 to 30, 5 to 40, 5 to 60, 5 to 80, 5 to 100, 5 to 150, 5 to 200, 10 to 20, 10 to 30, 10 to 40, 10 to 50, 10 to 60, 10 to 80, 10 to 100, 10 to 150, 10 to 200, 20 to 30, 20 to 40, 20 to 50, 20 to 60, 20 to 80, 20 to 100, 20 to 150, 20 to 200, 30 to 40, 30 to 50, 30 to 60, 30 to 80, 30 to 100, 30 to 150, 30 to 200, 40 to 50, 40 to 60, 40 to 80, 40 to 100, 40 to 150, 40 to 200, 50 to 60 50 to 80, 50 to 100, 50 to 150, 50 to 200, 60 to 80, 60 to 100, 60 to 150, 60 to 200, 80 to 100, 80 to 150, 80 to 200, 100 to 150, 100 to 200, or 150 to 200 amino acids in length. In some embodiments, the adenosine deaminase and the napDNAbp are fused via a linker that comprises 3, 4, 16, 24, 32, 64, 100, or 104 amino acids in length.

In some embodiments, the based editors provided herein further comprise one or more nuclear targeting sequences, for example, a nuclear localization sequence (NLS). In some embodiments, a NLS comprises an amino acid sequence that facilitates the importation of a protein, that comprises an NLS, into the cell nucleus *(e.g.,* by nuclear transport). In some embodiments, any of the fusion proteins provided herein further comprise a nuclear localization sequence (NLS). In some embodiments, the NLS is fused to the N-terminus of the fusion protein. In some embodiments, the NLS is fused to the C-terminus of the fusion protein. In some embodiments, the NLS is fused to the N-terminus of the napDNAbp. In some embodiments, the NLS is fused to the C-terminus of the napDNAbp. In some embodiments, the NLS is fused to the N-terminus of the adenosine deaminase. In some embodiments, the NLS is fused to the C-terminus of the adenosine deaminase. In some embodiments, the NLS is fused to the fusion protein via one or more linkers. In some embodiments, the NLS is fused to the fusion protein without a linker. In some embodiments, the NLS comprises an amino acid sequence of any one of the NLS sequences provided or referenced herein. In some embodiments, the NLS comprises an amino acid sequence as set forth in any one of SEQ ID NOs: 152-162. Additional nuclear localization sequences are known in the art and would be apparent to the skilled artisan. For example, NLS sequences are described in Plank *et al.,* PCT/EP2000/011690, the contents of which are incorporated herein by reference for their disclosure of exemplary nuclear localization sequences.

In some embodiments, the general architecture of exemplary fusion proteins with a deaminase and a napDNAbp comprises any one of the following structures, where NLS is a nuclear localization sequence (*e.g*., any NLS provided herein), NH₂ is the N-terminus of the fusion protein, and COOH is the C-terminus of the fusion protein. Fusion proteins comprising an adenosine deaminase, a napDNAbp, and a NLS:

NH₂-[NLS]-[deaminase]-[napDNAbp]-COOH;

NH₂-[deaminase]-[NLS]-[napDNAbp]-COOH;

NH₂-[ deaminase]-[napDNAbp]-[NLS]-COOH;

NH₂-[NLS]-[napDNAbp]-[deaminase]-COOH;

NH₂-[napDNAbp]-[NLS]-[deaminase]-COOH;

and

NH₂-[napDNAbp]-[deaminase]-[NLS]-COOH.

Some aspects of the disclosure provide ABEs (adenine base editors) that comprise a nucleic acid programmable DNA binding protein (napDNAbp) and at least two adenosine deaminase domains. Without wishing to be bound by any particular theory, dimerization of adenosine deaminases *(e.g.,* in cis or in trans) may improve the ability *(e.g.,* efficiency) of the fusion protein to modify a nucleic acid base, for example to deaminate adenine. In some embodiments, any of the fusion proteins may comprise 2, 3, 4 or 5 adenosine deaminase domains. In some embodiments, any of the fusion proteins provided herein comprise two adenosine deaminases. In some embodiments, any of the fusion proteins provided herein contain only two adenosine deaminases. In some embodiments, the adenosine deaminases are the same. In some embodiments, the adenosine deaminases are any of the adenosine deaminases provided herein. In some embodiments, the adenosine deaminases are different. In some embodiments, the first adenosine deaminase is any of the adenosine deaminases provided herein, and the second adenosine is any of the adenosine deaminases provided herein, but is not identical to the first adenosine deaminase. As one example, the fusion protein may comprise a first adenosine deaminase and a second adenosine deaminase that both comprise the amino acid sequence of SEQ ID NO: 91, which contains a W23R; H36L; P48A; R51L; L84F; A106V; D108N; H123Y; S146C; D147Y; R152P; E155V; I156F; and K157N mutation from ecTadA (SEQ ID NO: 89). In some embodiments, the fusion protein may comprise a first adenosine deaminase that comprises the amino acid sequence, e.g., of SEQ ID NO: 89, and a second adenosine deaminase domain that comprises the amino amino acid sequence of TadA7.10 of SEQ ID NO: 79. Additional fusion protein constructs comprising two adenosine deaminase domains are illustrated herein and are provided in the art.

In some embodiments, the fusion protein comprises two adenosine deaminases (*e.g*., a first adenosine deaminase and a second adenosine deaminase). In some embodiments, the fusion protein comprises a first adenosine deaminase and a second adenosine deaminase. In some embodiments, the first adenosine deaminase is N-terminal to the second adenosine deaminase in the fusion protein. In some embodiments, the first adenosine deaminase is C-terminal to the second adenosine deaminase in the fusion protein. In some embodiments, the first adenosine deaminase and the second deaminase are fused directly or via a linker. In some embodiments, the linker is any of the linkers provided herein, for example, any of the linkers described in the "Linkers" section.

In some embodiments, the first adenosine deaminase is the same as the second adenosine deaminase. In some embodiments, the first adenosine deaminase and the second adenosine deaminase are any of the adenosine deaminases described herein. In some embodiments, the first adenosine deaminase and the second adenosine deaminase are different. In some embodiments, the first adenosine deaminase is any of the adenosine deaminases provided herein. In some embodiments, the second adenosine deaminase is any of the adenosine deaminases provided herein but is not identical to the first adenosine deaminase. In some embodiments, the first adenosine deaminase is an ecTadA adenosine deaminase. In some embodiments, the first adenosine deaminase comprises an amino acid sequence that is at least least 60%, at least 65%, at least 70%, at least 75%, at least 80%, at least 85%, at least 90%, at least 95%, at least 96%, at least 97%, at least 98%, at least 99%, or at least 99.5% identical to any one of the amino acid sequences set forth in any one of SEQ ID NOs: 78-91, and 403-404, or to any of the adenosine deaminases provided herein. In some embodiments, the first adenosine deaminase comprises an amino acid sequence, e.g.,of SEQ ID NO: 78-91, and 403-404. In some embodiments, the second adenosine deaminase comprises an amino acid sequence that is at least least 60%, at least 65%, at least 70%, at least 75%, at least 80%, at least 85%, at least 90%, at least 95%, at least 96%, at least 97%, at least 98%, at least 99%, or at least 99.5% identical to any one of the amino acid sequences set forth in any one of SEQ ID NOs: 78-91, and 403-404, or to any of the deaminases provided herein. The amino acid sequences can be the same or different. In some embodiments, the second adenosine deaminase comprises an amino acid sequence of any one of SEQ ID NOs: 78-91, and 403-404

In some embodiments, the general architecture of exemplary fusion proteins with a first adenosine deaminase, a second adenosine deaminase, and a napDNAbp comprises any one of the following structures, where NLS is a nuclear localization sequence (*e.g*., any NLS provided herein), NH₂ is the N-terminus of the fusion protein, and COOH is the C-terminus of the fusion protein.

Thus, in some embodiments, the disclosure provides based editors comprising a first adenosine deaminase, a second adenosine deaminase, and a napDNAbp, such as:

NH₂-[first adenosine deaminase]-[second adenosine deaminase]-[napDNAbp]-COOH;

NH₂-[first adenosine deaminase]-[napDNAbp]-[second adenosine deaminase]-COOH;

NH₂-[napDNAbp]-[first adenosine deaminase]-[second adenosine deaminase]-COOH;

NH₂-[second adenosine deaminase]-[first adenosine deaminase]-[napDNAbp]-COOH;

NH₂-[second adenosine deaminase]-[napDNAbp]-[first adenosine deaminase]-COOH;

NH₂-[napDNAbp]-[second adenosine deaminase]-[first adenosine deaminase]-COOH;

In some embodiments, the fusion proteins provided herein do not comprise a linker. In some embodiments, a linker is present between one or more of the domains or proteins *(e.g.,* first adenosine deaminase, second adenosine deaminase, and/or napDNAbp). In some embodiments, the "-" used in the general architecture above indicates the presence of an optional linker.

In other embodiments, the disclosure provides based editors comprising a first adenosine deaminase, a second adenosine deaminase, a napDNAbp, and an NLS, such as: NH₂-[NLS]-[first adenosine deaminase]-[second adenosine deaminase]-[napDNAbp]-COOH; NH₂-[first adenosine deaminase]-[NLS]-[second adenosine deaminase]-[napDNAbp]-COOH; NH₂-[first adenosine deaminase]-[second adenosine deaminase]-[NLS]-[napDNAbp]-COOH; NH₂-[first adenosine deaminase]-[second adenosine deaminase]-[napDNAbp]-[NLS]-COOH; NH₂-[NLS]-[first adenosine deaminase]-[napDNAbp]-[second adenosine deaminase]-COOH; NH₂-[first adenosine deaminase]-[NLS]-[napDNAbp]-[second adenosine deaminase]-COOH; NH₂-[first adenosine deaminase]-[napDNAbp]-[NLS]-[second adenosine deaminase]-COOH; NH₂-[first adenosine deaminase]-[napDNAbp]-[second adenosine deaminase]-[NLS]-COOH; NH₂-[NLS]-[napDNAbp]-[first adenosine deaminase]-[second adenosine deaminase]-COOH; NH₂-[napDNAbp]-[NLS]-[first adenosine deaminase]-[second adenosine deaminase]-COOH; NH₂-[napDNAbp]-[first adenosine deaminase]-[NLS]-[second adenosine deaminase]-COOH; NH₂-[napDNAbp]-[first adenosine deaminase]-[second adenosine deaminase]-[NLS]-COOH; NH₂-[NLS]-[second adenosine deaminase]-[first adenosine deaminase]-[napDNAbp]-COOH; NH₂-[second adenosine deaminase]-[NLS]-[first adenosine deaminase]-[napDNAbp]-COOH; NH₂-[second adenosine deaminase]-[first adenosine deaminase]-[NLS]-[napDNAbp]-COOH; NH₂-[second adenosine deaminase]-[first adenosine deaminase]-[napDNAbp]-[NLS]-COOH; NH₂-[NLS]-[second adenosine deaminase]-[napDNAbp]-[first adenosine deaminase]-COOH; NH₂-[second adenosine deaminase]-[NLS]-[napDNAbp]-[first adenosine deaminase]-COOH; NH₂-[second adenosine deaminase]-[napDNAbp]-[NLS]-[first adenosine deaminase]-COOH; NH₂-[second adenosine deaminase]-[napDNAbp]-[first adenosine deaminase]-[NLS]-COOH; NH₂-[NLS]-[napDNAbp]-[second adenosine deaminase]-[first adenosine deaminase]-COOH; NH₂-[napDNAbp]-[NLS]-[second adenosine deaminase]-[first adenosine deaminase]-COOH; NH₂-[napDNAbp]-[second adenosine deaminase]-[NLS]-[first adenosine deaminase]-COOH; NH₂-[napDNAbp]-[second adenosine deaminase]-[first adenosine deaminase]-[NLS]-COOH;

In some embodiments, the fusion proteins provided herein do not comprise a linker. In some embodiments, a linker is present between one or more of the domains or proteins *(e.g.,* first adenosine deaminase, second adenosine deaminase, napDNAbp, and/or NLS). In some embodiments, the "-" used in the general architecture above indicates the presence of an optional linker.

It should be appreciated that the fusion proteins of the present disclosure may comprise one or more additional features. For example, in some embodiments, the fusion protein may comprise cytoplasmic localization sequences, export sequences, such as nuclear export sequences, or other localization sequences, as well as sequence tags that are useful for solubilization, purification, or detection of the fusion proteins. Suitable protein tags provided herein include, but are not limited to, biotin carboxylase carrier protein (BCCP) tags, myctags, calmodulin-tags, FLAG-tags, hemagglutinin (HA)-tags, polyhistidine tags, also referred to as histidine tags or His-tags, maltose binding protein (MBP)-tags, nus-tags, glutathione-S-transferase (GST)-tags, green fluorescent protein (GFP)-tags, thioredoxin-tags, S-tags, Softags *(e.g.,* Softag 1, Softag 3), strep-tags , biotin ligase tags, FlAsH tags, V5 tags, and SBP-tags. Additional suitable sequences will be apparent to those of skill in the art. In some embodiments, the fusion protein comprises one or more His tags.

### (1) Exemplary ABEs

Some aspects of the disclosure provide base editors comprising a napDNAbp domain (e.g., an nCas9 domain) and one or more adenosine deaminase domains (e.g., a heterodimer of adenosine deaminases). Such fusion proteins can be referred to as adenosine base editors (ABEs). In some embodiments, the ABEs have reduced off-target effects. In some embodiments, the base editors comprise adenine base editors for multiplexing applications. In still other embodiments, the base editors comprise ancestrally reconstructed adenine base editors.

The present disclosure provides motifs of newly discovered mutations to TadA 7.10 (SEQ ID NO: 79) (the TadA* used in ABEmax) that yield adenosine deaminase variants and confer broader Cas compatibility to the deaminase. These motifs also confer reduced off-target effects, such as reduced RNA editing activity and off-target DNA editing activity, on the base editor. The base editors of the present disclosure comprise one or more of the disclosed adenosine deaminase variants. In other embodiments, the base editors may comprise one or more adenosine deaminases having two or more such substitutions in combination. In some embodiments, the base editors comprise adenosine deaminases comprising comprises a sequence with at least 80%, 85%, 90%, 95%, 98%, 99%, or 99.5% sequence identity to SEQ ID NO: 91 (TadA-8e).

Exemplary ABEs of this disclosure comprise the monomer and dimer versions of the following editors: ABE8e, SaABE8e, SaKKH-ABE8e, NG-ABE8e, ABE-xCas9, ABE8e-NRTH, ABE8e-NRRH, ABE8e-NRCH, ABE8e-NG-CP1041, ABE8e-VRQR-CP1041, ABE8e-CP1041, ABE8e-CP1028, ABE8e-VRQR, ABE8e-LbCas12a (LbABE8e), ABE8e-AsCas12a (enAsABE8e), ABE8e-SpyMac, ABE8e (TadA-8e V106W), ABE8e (K20A,R21A), and ABE8e(TadA-8e V82G). The monomer version refers to an editor having an adenosine deaminase domain that comprises a TadA8e and does not comprise a second adenosine deaminase enzyme. The dimer version refers refers to an editor having an adenosine deaminase domain that comprises a first and second adenosine deaminase, *i.e., a* wild-type TadA enzyme and a TadA8e enzyme.

Exemplary ABEs include, without limitation, the following fusion proteins (for the purposes of clarity, and wherein shown, the adenosine deaminase domain is shown in **bold;** mutations of the ecTadA deaminase domain are shown in **bold underlining;** the XTEN linker is shown in *italics*; the UGI/AAG/EndoV domains are shown in ***bold italics*;** and NLS is shown in *underlined italics),* and any base editors comprise sequences that are at least least 85%, at least 90%, at least 95%, at least 98%, at least 99%, or at least 99.5% identical to any of the following amino acid sequences:
**ecTadA(wt)-XTEN-nCas9-NLS** 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
 
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGDSGGSPKKKRKV (SEQ ID NO: 174)
**ecTadA(D108N)-XTEN-nCas9-NLS**
   (mammalian construct, active on DNA, A to G editing): 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARNAKTGAAGSLMDVLHHPGMNHRVEITEGILADECAALLSDFFRMRRQEIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
 
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGDSGGSPKKKRKV (SEQ ID NO: 175)
**ecTadA(D108G)-XTEN-nCas9-NLS**
   (mammalian construct, active on DNA, A to G editing): 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARGAKTGAAGSLMDVLHHPGMNHRVEITEGILADECAALLSDFFRMRRQEIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
 
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGDSGGSPKKKRKV (SEQ ID NO: 176)
**ecTadA(D108V)-XTEN-nCas9-NLS**
   (mammalian construct, active on DNA, A to G editing): 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARVAKTGAAGSLMDVLHHPGMNHRVEITEGILADECAALLSDFFRMRRQEIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGDSGGSPKKKRKV (SEQ ID NO: 177)
**ecTadA(H8Y D108N N127S)-XTEN-dCas9**
   (variant resulting from first round of evolution in bacteria): 
MSEVEFSYEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARNAKTGAAGSLMDVLHHPGMSHRVEITEGILADECAALLSDFFRMRRQEIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DAIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGD (SEQ ID NO: 178)
(H8Y D108N N127S E155X)-XTEN-dCas9; X=D, G or V
   (Enriched variants from second round of evolution (in bacteria) ecTadA): 
MSEVEFSYEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
 
ARNAKTGAAGSLMDVLHHPGMSHRVEITEGILADECAALLSDFFRMRRQXIKA
QKKAQSSTDSGSETPGTSESATPESDKKYSIGLAIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQ
LSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILE
KMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIA
NLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDV
DAIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETN
GETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPID
FLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITG
LYETRIDLSQLGGD (SEQ ID NO: 179)
**ABE7.7**
   ecTadA_{(wild type)}-(SGGS)2-*XTEN*-(SGGS)₂-ecTadA_{(W23L_H36L_P48A_R51L_L84F_A106V_D108N_ H123Y_S146C_D147Y_R152P_E155VI156F_K157N)}-(SGGS)₂-*XTEN*-(SGGS)₂_nCas9_SGGS_NLS 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRALDEREVPVGAVLVLNNRVIGEGWNRAIGLHDPTAHAEIMALRQGGLV
 
MQNYRLIDATLYVTFEPCVMCAGAMIHSRIGRVVFGVRNAKTGAAGSLMDVLH
YPGMNHRVEITEGILADECAALLCYFFRMPRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 180)
**pNMG-624**
   ecTadA_{(wild type)}-*32 a.a. linker*-ecTadA_{(W23R_H36L_P48A_R51L_L84F_A106V_D108N_H123Y_ S146C_D147Y_R152P_E155V_I156F_K157N)}-*24 a.a. linker_*nCas9_*SGGS_NLS* 
MSEVEFSHEYWMRHALTLAKRAWDEREVPVGAVLVHNNRVIGEGWNR
PIGRHDPTAHAEIMALRQGGLVMQNYRLIDATLYVTLEPCVMCAGAMIHSRIG
RVVFGARDAKTGAAGSLMDVLHHPGMNHRVEITEGILADECAALLSDFFRMRR
QEIKAQKKAQSSTDSGGSSGGSSGSETPGTSESATPESSGGSSGGSSEVEFSHEYWMR
 
HALTLAKRARDEREVPVGAVLVLNNRVIGEGWNRAIGLHDPTAHAEIMALRQG
GLVMQNYRLIDATLYVTFEPCVMCAGAMIHSRIGRVVFGVRNAKTGAAGSLMD
VLHYPGMNHRVEITEGILADECAALLCYFFRMPRQVFNAQKKAQSSTDSGGSSG
GSSGSETPGTSESATPESDKKYSIGLAIGTNSVGWAVITDEYKVPSKKFKVLGNTDRHS
IKKNLIGALLFDSGETAEATRLKRTARRRYTRRKNRICYLQEIFSNEMAKVDDSFFHR
LEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARL
SKSRRLENLIAQLPGEKKNGLFGNLIALSLGLTPNFKSNFDLAEDAKLQLSKDTYDDD
LDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELL
VKLNREDLLRKQRTFDNGSIPHQIHLGELHAILRRQEDFYPFLKDNREKIEKILTFRIP
YYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTV
KQLKEDYFKKIECFDSVEISGVEDRFNASLGTYHDLLKIIKDKDFLDNEENEDILEDIV
LTLTLFEDREMIEERLKTYAHLFDDKVMKQLKRRRYTGWGRLSRKLINGIRDKQSG
KTILDFLKSDGFANRNFMQLIHDDSLTFKEDIQKAQVSGQGDSLHEHIANLAGSPAIK
KGILQTVKVVDELVKVMGRHKPENIVIEMARENQTTQKGQKNSRERMKRIEEGIKEL
GSQILKEHPVENTQLQNEKLYLYYLQNGRDMYVDQELDINRLSDYDVDHIVPQSFL
KDDSIDNKVLTRSDKNRGKSDNVPSEEVVKKMKNYWRQLLNAKLITQRKFDNLTK
AERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEIVWD
KGRDFATVRKVLSMPQVNIVKKTEVQTGGFSKESILPKRNSDKLIARKKDWDPKKY
GGFDSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLK
GSPEDNEQKQLFVEQHKHYLDEIIEQISEFSKRVILADANLDKVLSAYNKHRDKPIRE
QAENIIHLFTLTNLGAPAAFKYFDTTIDRKRYTSTKEVLDATLIHQSITGLYETRIDLSQ
LGGDSGGSPKKKRKV (SEQ ID NO: 181)
**ABE3.2**
   ecTadA_{(wild-type)}-(SGGS)₂-*XTEN*-(SGGS)₂-ecTadA_{(L84F_A106V_D108N_H123Y_D147Y_E155V_I156F)}-(SGGS)₂-*XTEN*-(SGGS)₂_nCas9_SGGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
 
QKKAQSSTDSGGSSGGSSGSETPGTSESATPESSGGSSGGSSEVEFSHEYWMRHALT
LAKRAWDEREVPVGAVLVHNNRVIGEGWNRPIGRHDPTAHAEIMALRQGGLV
MQNYRLIDATLYVTFEPCVMCAGAMIHSRIGRVVFGVRNAKTGAAGSLMDVLH
YPGMNHRVEITEGILADECAALLSYFFRMRRQVFKAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 182)
**ABE5.3**
   ecTadA_{(wild-type)}-(SGGS)₂-*XTEN*-(SGGS)₂-ecTadA_{(H36L_R51L_L84F_A106V_D108N_H123Y_S146C_ D147Y_E155V_I156F_K157N)}-(SGGS)2-*XTEN*-(SGGS)₂_nCas9_SGGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
 
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRPIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 183)
**pNMG-558**
   ecTadA_{(wild-type)}- *32 a.a. linker*-ecTadA_{(H36L_R51L_L84F_A106V_D108N_}H123Y _{_S146C_}D147Y_{_}E155V_{_I156F _K157N)}- *24 a.a.* linker nCas9_SGGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALTL
AKRAWDEREVPVGAVLVLNNRVIGEGWNRPIGLHDPTAHAEIMALRQGGLVM
QNYRLIDATLYVTFEPCVMCAGAMIHSRIGRVVFGVRNAKTGAAGSLMDVLHY
PGMNHRVEITEGILADECAALLCYFFRMRRQVFNAQKKAQSSTDSGGSSGGSSGS
ETPGTSESATPESDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMI
KFRGHFLIEGDLNPDNSDVDKLFIQLVQTYNQLFEENPINASGVDAKAILSARLSKSR
RLENLIAQLPGEKKNGLFGNLIALSLGLTPNFKSNFDLAEDAKLQLSKDTYDDDLDN
LLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLK
EDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDF
LKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILK
EHPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERGGL
SELDKAGFIKRQLVETRQITKHVAQILDSRMNTKYDENDKLIREVKVITLKSKLVSDF
RKDFQFYKVREINNYHHAHDAYLNAVVGTALIKKYPKLESEFVYGDYKVYDVRKM
IAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSP
TVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDSG
GSPKKKRKV (SEQ ID NO: 184)
**pNMG-576**
   ecTadA_{(wild-type)}-(SGGS)₂-*XTEN*-(SGGS)₂-ecTadA_{(H36L_P48S_R51L_L84F_A106V_D108N_H123Y_S146C_ D147Y_E155V_I156F_K157N)}-(SGGS)₂-*XTEN*-(SGGS)₂_nCas9_GGS_NLS 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRSIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 185)
**pNMG-577**
   ecTadA_{(wild-type)}-(SGGS)₂-*XTEN*-(SGGS)₂-ecTadA_{(H36L_P48S_R51L_L84F_A106V_D108N_H123Y_ A142N_S146C_D147Y_E155V_I156F_K157N)}-(SGGS)₂-*XTEN*-(SGGS)₂_nCas9_GGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRSIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
 
KPIREQAENIIHLFTLTNLGAPAAFKYFDTTIDRKRYTSTKEVLDATLIHQSITGLYETR
IDLSQLGGDSGGSPKKKRKV (SEQ ID NO: 186)
**pNMG-586**
   ecTadA_{(wild-type)}-(SGGS)2-*XTEN*-(SGGS)2-ecTadA_{(H36L_P48A_R51L_L84F_A106V}__{D108N_ H123Y_S146C_D147Y_E155V_I156F_K157N)}-(SGGS)2-*XTEN*-(SGGS)2nCas9 GGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
 
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 187)
**ABE7.2**
   ecTadA_{(wild-type)}-(SGGS)₂-*XTEN*-(SGGS)₂-ecTadA_{(H36L_P48A_R51L_L84F_A106V_D108N_ H123Y_A142N_S146C_D147Y_E155V_I156F_K157N)}-(SGGS)₂-*XTEN*-(SGGS)₂_nCas9_GGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
 
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 188)
**pNMG-620**
   ecTadA_{(wild-type)}-(SGGS)₂-*XTEN*-(SGGS)₂-ecTadA_{(W23R_H36L_P48A_R51L_L84F_A106V}__{D108N_ H123Y_S146C_D147Y_R152P_E155V_I156F_K157N)}-(SGGS)₂-*XTEN*-(SGGS)₂_nCas9_GGS_*NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRARDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMPRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
 
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 189)
**pNMG-617**
   ecTadA*(wild-type)*-(SGGS)₂-*XTEN*-(SGGS)₂-ecTadA_{(W23L_H36L_P48A_R51L_L84F_A106V_D108N_ H123Y_A142A_S146C_D147Y_E155V_I156F_K157N)}-(SGGS)₂-*XTEN*-(SGGS)₂_nCas9_GGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRALDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
 
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 190)
**pNMG-618**
   ecTadA_{(wild-type)}-(SGGS)2-*XTEN*-(SGGS)2-ecTadA_{(W23L_H36L_P48A_R51L_L84F_A106V_D108N_ H123Y_A142A_S146C_D147Y_R152P_E155V_I156F_K157N)}-(SGGS)2-*XTEN*-(SGGS)2_nCas9_GGS_*NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRALDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMPRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
 
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 191)
**pNMG-620**
   ecTadA_{(wild-type)}-(SGGS)₂-*XTEN*-(SGGS)₂-ecTadA_{(W23R_H36L_P48A_R51L_L84F_A106V_D108N_ H123Y_S146C_D147Y_R152P-E155V_I156F_K157N)}-(SGGS)2-*XTEN*-(SGGS)₂_nCas9_*GGS_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRARDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMPRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
 
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSPKKKRKV (SEQ ID NO: 192)
**pNMG-621**
   ecTadA_{(wild-type)}- *32 a.a. linker*-ecTadA_{(H36L_P48A_R51L_L84F_A106V_D108N_H123Y_ S146C_D147Y_R152P_E155V_I156F_K157N)}- *24 a.a. linker*_nCas9_GGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALTL
AKRAWDEREVPVGAVLVLNNRVIGEGWNRAIGLHDPTAHAEIMALRQGGLVM
QNYRLIDATLYVTFEPCVMCAGAMIHSRIGRVVFGVRNAKTGAAGSLMDVLHY
PGMNHRVEITEGILADECAALLCYFFRMPRQVFNAQKKAQSSTDSGGSSGGSSGS
ETPGTSESATPESDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMI
KFRGHFLIEGDLNPDNSDVDKLFIQLVQTYNQLFEENPINASGVDAKAILSARLSKSR
RLENLIAQLPGEKKNGLFGNLIALSLGLTPNFKSNFDLAEDAKLQLSKDTYDDDLDN
LLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLK
EDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDF
LKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILK
EHPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERGGL
 
SELDKAGFIKRQLVETRQITKHVAQILDSRMNTKYDENDKLIREVKVITLKSKLVSDF
RKDFQFYKVREINNYHHAHDAYLNAVVGTALIKKYPKLESEFVYGDYKVYDVRKM
IAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSP
TVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDSG
GSPKKKRKV (SEQ ID NO: 193)
**pNMG-622**
   ecTadA_{(wild-type)}- *32 a.a. linker*-ecTadA_{(H36L_P48A_R51L_L84F_A106V_D108N_H123Y_A142N_ S146C_D147Y_R152P_E155V_I156F_K157N)}- *24 a.a. linker*_nCas9_GGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALTL
AKRAWDEREVPVGAVLVLNNRVIGEGWNRAIGLHDPTAHAEIMALRQGGLVM
QNYRLIDATLYVTFEPCVMCAGAMIHSRIGRVVFGVRNAKTGAAGSLMDVLHY
PGMNHRVEITEGILADECNALLCYFFRMPRQVFNAQKKAQSSTDSGGSSGGSSGS
ETPGTSESATPESDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMI
KFRGHFLIEGDLNPDNSDVDKLFIQLVQTYNQLFEENPINASGVDAKAILSARLSKSR
RLENLIAQLPGEKKNGLFGNLIALSLGLTPNFKSNFDLAEDAKLQLSKDTYDDDLDN
LLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLK
EDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDF
LKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILK
EHPVENTQLQNEKLYLYYLQNGRDMYVDQELDINRLSDYDVDHIVPQSFLKDDSID
 
NKVLTRSDKNRGKSDNVPSEEVVKKMKNYWRQLLNAKLITQRKFDNLTKAERGGL
SELDKAGFIKRQLVETRQITKHVAQILDSRMNTKYDENDKLIREVKVITLKSKLVSDF
RKDFQFYKVREINNYHHAHDAYLNAVVGTALIKKYPKLESEFVYGDYKVYDVRKM
IAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSP
TVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDSG
GSPKKKRKV (SEQ ID NO: 194)
**pNMG-623**
   ecTadA_{(wild-type)}- *32 a.a. linker*-ecTadA_{(W23L_H36L_P48A_R51L_L84F_A106V_D108N_H123Y_S146C_ D147Y_R152P_E155V_I156F_K157N)}- *24 a.a. linker*_nCas9_*GGS_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALTL
AKRALDEREVPVGAVLVLNNRVIGEGWNRAIGLHDPTAHAEIMALRQGGLVM
QNYRLIDATLYVTFEPCVMCAGAMIHSRIGRVVFGVRNAKTGAAGSLMDVLHY
PGMNHRVEITEGILADECAALLCYFFRMPRQVFNAQKKAQSSTDSGGSSGGSSGS
ETPGTSESATPESDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEES
FLVEEDKKHERHPIFGNIVDEVAYHEKYPTIYHLRKKLVDSTDKADLRLIYLALAHMI
KFRGHFLIEGDLNPDNSDVDKLFIQLVQTYNQLFEENPINASGVDAKAILSARLSKSR
RLENLIAQLPGEKKNGLFGNLIALSLGLTPNFKSNFDLAEDAKLQLSKDTYDDDLDN
LLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKL
NREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLK
EDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDF
LKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILK
 
EHPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERGGL
SELDKAGFIKRQLVETRQITKHVAQILDSRMNTKYDENDKLIREVKVITLKSKLVSDF
RKDFQFYKVREINNYHHAHDAYLNAVVGTALIKKYPKLESEFVYGDYKVYDVRKM
IAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSP
TVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDSG
GSPKKKRKV (SEQ ID NO: 195)
**ABE6.3**
   ecTadA_{(wild-type})-(SGGS)₂-*XTEN*-(SGGS)₂-ecTadA_{(H36L_P48S_R51L_L84F__A106V_D108N_H123Y S146C_D147Y_E155V_I156F_K157N)}-(SGGS)₂-*XTEN*-(SGGS)₂_nCas9_SGGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRSIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
 
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYE
TRIDLSQLGGDSGGSPKKKRKV (SEQ ID NO: 196)
**ABE6.4**
   ecTadA_{(wild-type)}-(SGGS)₂-*XTEN*-(SGGS)₂-ecTadA_{(H36L_P48S_R51L_L84F__A106V_D108N_H123Y_ A142N_S146C_D147Y_E155V_I156F_K157N)}-(SGGS)₂-*XTEN-*(SGGS)₂_nCas9_SGGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRAWDEREVPVGAVLVLNNRVIGEGWNRSIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
 
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYE
TRIDLSQLGGDSGGSPKKKRKV (SEQ ID NO: 197)
**ABE7.8**
   ecTadA_{(wild-type)}-(SGGS)₂-*XTEN*-(SGGS)₂-ecTadA_{(W23L_H36L_P48A_R51L_L84F_A106V}__{D108N_ H123Y_A142N_S146C_D147Y_E155V_I156F_K157N})-(SGGS)₂-*XTEN*-(SGGS)₂_nCas9_SGGS*_NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRALDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECNALLCYFFRMRRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
 
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYE
TRIDLSQLGGDSGGSPKKKRKVc(SEQ ID NO: 198)
**ABE7.9**
   ecTadA_{(wild-type})-(SGGS)₂-*XTEN*-(SGGS)₂-ecTadA_{(W23L_H36L_P48A_R51L_L84F_A106V_ D108N_H123Y_A142N_S146C_D147Y_R152P_E155V_I156F_K157N)}*-*(SGGS)₂*-XTEN-*(SGGS)₂nCas9 SGGS_*NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRALDEREVPVGAVLVLNNRGEGWNRAIGLHDPTAHAEIMALRQGGLVMQ
NYRLIDATLYVTFEPCVMCAGAMIHSRIGRVVFGVRNAKTGAAGSLMDVLHYP
GMNHRVEITEGILADECNALLCYFFRMPRQVFNAQKKAQSSTDSGGSSGGSSGSE
TPGTSESATPESSGGSSGGSDKKYSIGLAIGTNSVGWAVITDEYKVPSKKFKVLGNTD
RHSIKKNLIGALLFDSGETAEATRLKRTARRRYTRRKNRICYLQEIFSNEMAKVDDSF
FHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTY
DDDLDNLLAQIGDQYADLFLAAKNLSDAILLSDILRVNTEITKAPLSASMIKRYDEHH
QDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLP
 
NEKVLPKHSLLYEYFTVYNELTKVKYVTEGMRKPAFLSGEQKKAIVDLLFKTNRKV
TVKQLKEDYFKKIECFDSVEISGVEDRFNASLGTYHDLLKIIKDKDFLDNEENEDILE
DIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPA
IKKGILQTVKVVDELVKVMGRHKPENIVIEMARENQTTQKGQKNSRERMKRIEEGIK
ELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITL
KSKLVSDFRKDFQFYKVREINNYHHAHDAYLNAVVGTALIKKYPKLESEFVYGDYK
VYDVRKMIAKSEQEIGKATAKYFFYSNIMNFFKTEITLANGEIRKRPLIETNGETGEIV
WDKGRDFATVRKVLSMPQVNIVKKTEVQTGGFSKESILPKRNSDKLIARKKDWDPK
KYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYE
KLKGSPEDNEQKQLFVEQHKHYLDEIIEQISEFSKRVILADANLDKVLSAYNKHRDKP
IREQAENIIHLFTLTNLGAPAAFKYFDTTIDRKRYTSTKEVLDATLIHQSITGLYETR
IDLSQLGGDSGGSPKKKRKV (SEQ ID NO: 199)
**ABE7.10**
   ecTadA_{(wild-type)}-(SGGS)₂-*XTEN*-(SGGS)₂-ecTadA_{(W23R_H36L_P48A_R51L_L84F_A106V_ D108N_H123Y_S146C_D147Y_R152P_E155V_I156F_K157N)}-(SGGS)₂-*XTEN*-(SGGS)₂_nCas9_SGGS *NLS* 
MSEVEFSHEYWMRHALTLAKRAWDEREVPVGAVLVHNNRVIGEGWNRPIGRH
DPTAHAEIMALRQGGLVMQNYRLIDATLYVTLEPCVMCAGAMIHSRIGRVVFG
ARDAKTGAAGSLMDVLHHPGMNHRVEITEGILADECAALLSDFFRMRRQEIKA
QKKAQSSTDSGGSSGGSSGSETPGTSESATPESSGGSSGGSSEVEFSHEYWMRHALT
LAKRARDEREVPVGAVLVLNNRVIGEGWNRAIGLHDPTAHAEIMALRQGGLV
MQNYRLIDATLYVTFEPCVMCAGAMIHSRIGRVVFGVRNAKTGAAGSLMDVLH
YPGMNHRVEITEGILADECAALLCYFFRMPRQVFNAQKKAQSSTDSGGSSGGSS
GSETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
 
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYE
TRIDLSQLGGDSGGSPKKKRKV (SEQ ID NO: 200)
**ABEmax (7.10)**
   *NLS* ecTadA_{(wild-type)}-(SGGS)₂-*XTEN*-(SGGS)₂-ecTadA7.10_{(W23R_H36LP48A_R51L_ L84F_A106V_D108N_H123Y_S146C_D147Y _R152P_E155V_I156F_K157N)}-(SGGS)₂-*XTEN*-(SGGS)₂_nCas9 VRQR_SGGS_*NLS*

MKRTADGSEFESPKKKRKVSEVEFSHEYWMRHALTLAKRAWDEREVPVGAVLVH
NNRVIGEGWNRPIGRHDPTAHAEIMALRQGGLVMQNYRLIDATLYVTLEPCVM
CAGAMIHSRIGRVVFGARDAKTGAAGSLMDVLHHPGMNHRVEITEGILADECA
ALLSDFFRMRRQEIKAQKKAQSSTDSGGSSGGSSGSETPGTSESATPESSGGSSGGSS
EVEFSHEYWMRHALTLAKRARDEREVPVGAVLVLNNRVIGEGWNRAIGLHDP
TAHAEIMALRQGGLVMQNYRLIDATLYVTFEPCVMCAGAMIHSRIGRVVFGVR
NAKTGAAGSLMDVLHYPGMNHRVEITEGILADECAALLCYFFRMPRQVFNAQK
KAQSSTDSGGSSGGSSGSETPGTSESATPESSGGSSGGSDKKYSIGLAIGTNSVGWAV
ITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNF
KSNFDLAEDAKLQLSKDTYDDDLDNLLAQIGDQYADLFLAAKNLSDAILLSDILRVN

TEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRR
QEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAF
LSGEQKKAIVDLLFKTNRKVTVKQLKEDYFKKIECFDSVEISGVEDRFNASLGTYHD
LLKIIKDKDFLDNEENEDILEDIVLTLTLFEDREMIEERLKTYAHLFDDKVMKQLKRR
RYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQ
TTQKGQKNSRERMKRIEEGIKELGSQILKEHPVENTQLQNEKLYLYYLQNGRDMYV
DQELDINRLSDYDVDHIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDS
RMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKE
SILPKRNSDKLIARKKDWDPKKYGGFVSPTVAYSVLVVAKVEKGKSKKLKSVKELL
GITIMERSSFEKNPIDFLEAKGYKEVKKDLIIKLPKYSLFELENGRKRMLASARELQK
GNELALPSKYVNFLYLASHYEKLKGSPEDNEQKQLFVEQHKHYLDEIIEQISEFSKRV
ILADANLDKVLSAYNKHRDKPIREQAENIIHLFTLTNLGAPAAFKYFDTTIDRKQYRS
TKEVLDATLIHQSITGLYETRIDLSQLGGDSGGSKRTADGSEFEPKKKRKV (SEQ ID
NO: 201)

Exemplary base editors comprise sequences that are at least least 85%, at least 90%, at least 95%, at least 98%, at least 99%, or at least 99.5% identical to any of the following amino acid sequences:
**ABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQS
 
KNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDE
IIEQISEFSKRVILADANLDKVLSAYNKHRDKPIREQAENIIHLFTLTNLGAPAAFKYF
DTTIDRKRYTSTKEVLDATLIHQSITGLYETRIDLSQLGGDSGGSKRTADGSEFEPKK
KRKV (SEQ ID NO: 202)
**ABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
 
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDP
KKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETR
IDLSQLGGDSGGSKRTADGSEFEPKKKRKV(SEQ ID NO: 203)
**SaABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSGKRNYILG
LAIGITSVGYGIIDYETRDVIDAGVRLFKEANVENNEGRRSKRGARRLKRRRRHRIQR
VKKLLFDYNLLTDHSELSGINPYEARVKGLSQKLSEEEFSAALLHLAKRRGVHNVNE
VEEDTGNELSTKEQISRNSKALEEKYVAELQLERLKKDGEVRGSINRFKTSDYVKEA
KQLLKVQKAYHQLDQSFIDTYIDLLETRRTYYEGPGEGSPFGWKDIKEWYEMLMGH
CTYFPEELRSVKYAYNADLYNALNDLNNLVITRDENEKLEYYEKFQIIENVFKQKKK
PTLKQIAKEILVNEEDIKGYRVTSTGKPEFTNLKVYHDIKDITARKEIIENAELLDQIAK
ILTIYQSSEDIQEELTNLNSELTQEEIEQISNLKGYTGTHNLSLKAINLILDELWHTNDN
QIAIFNRLKLVPKKVDLSQQKEIPTTLVDDFILSPVVKRSFIQSIKVINAIIKKYGLPNDII
IELAREKNSKDAQKMINEMQKRNRQTNERIEEIIRTTGKENAKYLIEKIKLHDMQEGK
CLYSLEAIPLEDLLNNPFNYEVDHIIPRSVSFDNSFNNKVLVKQEENSKKGNRTPFQY
LSSSDSKISYETFKKHILNLAKGKGRISKTKKEYLLEERDINRFSVQKDFINRNLVDTR
 
YATRGLMNLLRSYFRVNNLDVKVKSINGGFTSFLRRKWKFKKERNKGYKHHAEDA
LIIANADFIFKEWKKLDKAKKVMENQMFEEKQAESMPEIETEQEYKEIFITPHQIKHIK
DFKDYKYSHRVDKKPNRELINDTLYSTRKDDKGNTLIVNNLNGLYDKDNDKLKKLI
NKSPEKLLMYHHDPQTYQKLKLIMEQYGDEKNPLYKYYEETGNYLTKYSKKDNGP
VIKKIKYYGNKLNAHLDITDDYPNSRNKVVKLSLKPYRFDVYLDNGVYKFVTVKNL
DVIKKENYYEVNSKCYEEAKKLKKISNQAEFIASFYNNDLIKINGELYRVIGVNNDLL
NRIEVNMIDITYREYLENMNDKRPPRIIKTIASKTQSIKKYSTDILGNLYEVKSKKHPQI
IKKGSGGSKRTADGSEFEPKKKRKV (SEQ ID NO: 204)
**SaABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSGKRNYILGLAIGITSVGYGIIDYETRDVIDAGVRLFKE
ANVENNEGRRSKRGARRLKRRRRHRIQRVKKLLFDYNLLTDHSELSGINPYEARVK
GLSQKLSEEEFSAALLHLAKRRGVHNVNEVEEDTGNELSTKEQISRNSKALEEKYVA
ELQLERLKKDGEVRGSINRFKTSDYVKEAKQLLKVQKAYHQLDQSFIDTYIDLLETR
RTYYEGPGEGSPFGWKDIKEWYEMLMGHCTYFPEELRSVKYAYNADLYNALNDLN
NLVITRDENEKLEYYEKFQIIENVFKQKKKPTLKQIAKEILVNEEDIKGYRVTSTGKPE
FTNLKVYHDIKDITARKEIIENAELLDQIAKILTIYQSSEDIQEELTNLNSELTQEEIEQIS
NLKGYTGTHNLSLKAINLILDELWHTNDNQIAIFNRLKLVPKKVDLSQQKEIPTTLVD
DFILSPVVKRSFIQSIKVINAIIKKYGLPNDIIIELAREKNSKDAQKMINEMQKRNRQTN
ERIEEIIRTTGKENAKYLIEKIKLHDMQEGKCLYSLEAIPLEDLLNNPFNYEVDHIIPRS
VSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSIN
GGFTSFLRRKWKFKKERNKGYKHHAEDALIIANADFIFKEWKKLDKAKKVMENQM
FEEKQAESMPEIETEQEYKEIFITPHQIKHIKDFKDYKYSHRVDKKPNRELINDTLYST
RKDDKGNTLIVNNLNGLYDKDNDKLKKLINKSPEKLLMYHHDPQTYQKLKLIMEQY
GDEKNPLYKYYEETGNYLTKYSKKDNGPVIKKIKYYGNKLNAHLDITDDYPNSRNK
VVKLSLKPYRFDVYLDNGVYKFVTVKNLDVIKKENYYEVNSKCYEEAKKLKKISNQ
AEFIASFYNNDLIKINGELYRVIGVNNDLLNRIEVNMIDITYREYLENMNDKRPPRIIK
 
TIASKTQSIKKYSTDILGNLYEVKSKKHPQIIKKGSGGSKRTADGSEFEPKKKRKV
(SEQ ID NO: 205)
**LbABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSSKLEKFTN
CYSLSKTLRFKAIPVGKTQENIDNKRLLVEDEKRAEDYKGVKKLLDRYYLSFINDVL
HSIKLKNLNNYISLFRKKTRTEKENKELENLEINLRKEIAKAFKGNEGYKSLFKKDIIE
TILPEFLDDKDEIALVNSFNGFTTAFTGFFDNRENMFSEEAKSTSIAFRCINENLTRYIS
NMDIFEKVDAIFDKHEVQEIKEKILNSDYDVEDFFEGEFFNFVLTQEGIDVYNAIIGGF
VTESGEKIKGLNEYINLYNQKTKQKLPKFKPLYKQVLSDRESLSFYGEGYTSDEEVLE
VFRNTLNKNSEIFSSIKKLEKLFKNFDEYSSAGIFVKNGPAISTISKDIFGEWNVIRDK
WNAEYDDIHLKKKAVVTEKYEDDRRKSFKKIGSFSLEQLQEYADADLSVVEKLKEIII
QKVDEIYKVYGSSEKLFDADFVLEKSLKKNDAVVAIMKDLLDSVKSFENYIKAFFGE
GKETNRDESFYGDFVLAYDILLKVDHIYDAIRNYVTQKPYSKDKFKLYFQNPQFMG
GWDKDKETDYRATILRYGSKYYLAIMDKKYAKCLQKIDKDDVNGNYEKINYKLLP
GPNKMLPKVFFSKKWMAYYNPSEDIQKIYKNGTFKKGDMFNLNDCHKLIDFFKDSIS
RYPKWSNAYDFNFSETEKYKDIAGFYREVEEQGYKVSFESASKKEVDKLVEEGKLY
MFQIYNKDFSDKSHGTPNLHTMYFKLLFDENNHGQIRLSGGAELFMRRASLKKEEL
VVHPANSPIANKNPDNPKKTTTLSYDVYKDKRFSEDQYELHIPIAINKCPKNIFKINTE
VRVLLKHDDNPYVIGIARGERNLLYIVVVDGKGNIVEQYSLNEIINNFNGIRIKTDYHS
LLDKKEKERFEARQNWTSIENIKELKAGYISQVVHKICELVEKYDAVIALEDLNSGFK
NSRVKVEKQVYQKFEKMLIDKLNYMVDKKSNPCATGGALKGYQITNKFESFKSMST
QNGFIFYIPAWLTSKIDPSTGFVNLLKTKYTSIADSKKFISSFDRIMYVPEEDLFEFALD
YKNFSRTDADYIKKWKLYSYGNRIRIFRNPKKNNVFDWEEVCLTSAYKELFNKYGIN
YQQGDIRALLCEQSDKAFYSSFMALMSLMLQMRNSITGRTDVDFLISPVKNSDGIFY
DSRNYEAQENAILPKNADANGAYNIARKVLWAIGQFKKAEDEKLDKVKIAISNKEW
LEYAQTSVKSGGSKRTADGSEFEPKKKRKV(SEQ ID NO: 206)
**LbABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
 
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSSKLEKFTNCYSLSKTLRFKAIPVGKTQENIDNKRLLV
EDEKRAEDYKGVKKLLDRYYLSFINDVLHSIKLKNLNNYISLFRKKTRTEKENKELE
NLEINLRKEIAKAFKGNEGYKSLFKKDIIETILPEFLDDKDEIALVNSFNGFTTAFTGFF
DNRENMFSEEAKSTSIAFRCINENLTRYISNMDIFEKVDAIFDKHEVQEIKEKILNSDY
DVEDFFEGEFFNFVLTQEGIDVYNAIIGGFVTESGEKIKGLNEYINLYNQKTKQKLPK
FKPLYKQVLSDRESLSFYGEGYTSDEEVLEVFRNTLNKNSEIFSSIKKLEKLFKNFDEY
SSAGIFVKNGPAISTISKDIFGEWNVIRDKWNAEYDDIHLKKKAVVTEKYEDDRRKSF
KKIGSFSLEQLQEYADADLSVVEKLKEIIIQKVDEIYKVYGSSEKLFDADFVLEKSLK
KNDAVVAIMKDLLDSVKSFENYIKAFFGEGKETNRDESFYGDFVLAYDILLKVDHIY
DAIRNYVTQKPYSKDKFKLYFQNPQFMGGWDKDKETDYRATILRYGSKYYLAIMD
KKYAKCLQKIDKDDVNGNYEKINYKLLPGPNKMLPKVFFSKKWMAYYNPSEDIQKI
YKNGTFKKGDMFNLNDCHKLIDFFKDSISRYPKWSNAYDFNFSETEKYKDIAGFYRE
VEEQGYKVSFESASKKEVDKLVEEGKLYMFQIYNKDFSDKSHGTPNLHTMYFKLLF
DENNHGQIRLSGGAELFMRRASLKKEELVVHPANSPIANKNPDNPKKTTTLSYDVYK
DKRFSEDQYELHIPIAINKCPKNIFKINTEVRVLLKHDDNPYVIGIARGERNLLYIVVV
DGKGNIVEQYSLNEIINNFNGIRIKTDYHSLLDKKEKERFEARQNWTSIENIKELKAG
YISQVVHKICELVEKYDAVIALEDLNSGFKNSRVKVEKQVYQKFEKMLIDKLNYMV
DKKSNPCATGGALKGYQITNKFESFKSMSTQNGFIFYIPAWLTSKIDPSTGFVNLLKT
KYTSIADSKKFISSFDRIMYVPEEDLFEFALDYKNFSRTDADYIKKWKLYSYGNRIRIF
RNPKKNNVFDWEEVCLTSAYKELFNKYGINYQQGDIRALLCEQSDKAFYSSFMALM
SLMLQMRNSITGRTDVDFLISPVKNSDGIFYDSRNYEAQENAILPKNADANGAYNIA
RKVLWAIGQFKKAEDEKLDKVKIAISNKEWLEYAQTSVKSGGSKRTADGSEFEPKK
KRKV (SEQ ID NO: 207)
**LbABE7.10** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNAKTGAAGSLMDV
LHYPGMNHRVEITEGILADECAALLCYFFRMPRQVFNAQKKAQSSTDSGGSSGGSSG
 
SETPGTSESATPESSGGSSGGSSKLEKFTNCYSLSKTLRFKAIPVGKTQENIDNKRLLV
EDEKRAEDYKGVKKLLDRYYLSFINDVLHSIKLKNLNNYISLFRKKTRTEKENKELE
NLEINLRKEIAKAFKGNEGYKSLFKKDIIETILPEFLDDKDEIALVNSFNGFTTAFTGFF
DNRENMFSEEAKSTSIAFRCINENLTRYISNMDIFEKVDAIFDKHEVQEIKEKILNSDY
DVEDFFEGEFFNFVLTQEGIDVYNAIIGGFVTESGEKIKGLNEYINLYNQKTKQKLPK
FKPLYKQVLSDRESLSFYGEGYTSDEEVLEVFRNTLNKNSEIFSSIKKLEKLFKNFDEY
SSAGIFVKNGPAISTISKDIFGEWNVIRDKWNAEYDDIHLKKKAVVTEKYEDDRRKSF
KKIGSFSLEQLQEYADADLSVVEKLKEIIIQKVDEIYKVYGSSEKLFDADFVLEKSLK
KNDAVVAIMKDLLDSVKSFENYIKAFFGEGKETNRDESFYGDFVLAYDILLKVDHIY
DAIRNYVTQKPYSKDKFKLYFQNPQFMGGWDKDKETDYRATILRYGSKYYLAIMD
KKYAKCLQKIDKDDVNGNYEKINYKLLPGPNKMLPKVFFSKKWMAYYNPSEDIQKI
YKNGTFKKGDMFNLNDCHKLIDFFKDSISRYPKWSNAYDFNFSETEKYKDIAGFYRE
VEEQGYKVSFESASKKEVDKLVEEGKLYMFQIYNKDFSDKSHGTPNLHTMYFKLLF
DENNHGQIRLSGGAELFMRRASLKKEELVVHPANSPIANKNPDNPKKTTTLSYDVYK
DKRFSEDQYELHIPIAINKCPKNIFKINTEVRVLLKHDDNPYVIGIARGERNLLYIVVV
DGKGNIVEQYSLNEIINNFNGIRIKTDYHSLLDKKEKERFEARQNWTSIENIKELKAG
YISQVVHKICELVEKYDAVIALEDLNSGFKNSRVKVEKQVYQKFEKMLIDKLNYMV
DKKSNPCATGGALKGYQITNKFESFKSMSTQNGFIFYIPAWLTSKIDPSTGFVNLLKT
KYTSIADSKKFISSFDRIMYVPEEDLFEFALDYKNFSRTDADYIKKWKLYSYGNRIRIF
RNPKKNNVFDWEEVCLTSAYKELFNKYGINYQQGDIRALLCEQSDKAFYSSFMALM
SLMLQMRNSITGRTDVDFLISPVKNSDGIFYDSRNYEAQENAILPKNADANGAYNIA
RKVLWAIGQFKKAEDEKLDKVKIAISNKEWLEYAQTSVKSGGSKRTADGSEFEPKK
KRKV (SEQ ID NO: 208)
**enAsABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSMTQFEGFT
NLYQVSKTLRFELIPQGKTLKHIQEQGFIEEDKARNDHYKELKPIIDRIYKTYADQCL
QLVQLDWENLSAAIDSYRKEKTEETRNALIEEQATYRNAIHDYFIGRTDNLTDAINKR
HAEIYKGLFKAELFNGKVLKQLGTVTTTEHENALLRSFDKFTTYFSGFYRNRKNVFS
AEDISTAIPHRIVQDNFPKFKENCHIFTRLITAVPSLREHFENVKKAIGIFVSTSIEEVFS
FPFYNQLLTQTQIDLYNQLLGGISREAGTEKIKGLNEVLNLAIQKNDETAHIIASLPHR
FIPLFKQILSDRNTLSFILEEFKSDEEVIQSFCKYKTLLRNENVLETAEALFNELNSIDLT
 
HIFISHKKLETISSALCDHWDTLRNALYERRISELTGKITKSAKEKVQRSLKHEDINLQ
EIISAAGKELSEAFKQKTSEILSHAHAALDQPLPTTLKKQEEKEILKSQLDSLLGLYHL
LDWFAVDESNEVDPEFSARLTGIKLEMEPSLSFYNKARNYATKKPYSVEKFKLNFQ
MPTLARGWDVNREKNNGAILFVKNGLYYLGIMPKQKGRYKALSFEPTEKTSEGFDK
MYYDYFPDAAKMIPKCSTQLKAVTAHFQTHTTPILLSNNFIEPLEITKEIYDLNNPEKE
PKKFQTAYAKKTGDQKGYREALCKWIDFTRDFLSKYTKTTSIDLSSLRPSSQYKDLG
EYYAELNPLLYHISFQRIAEKEIMDAVETGKLYLFQIYNKDFAKGHHGKPNLHTLYW
TGLFSPENLAKTSIKLNGQAELFYRPKSRMKRMAHRLGEKMLNKKLKDQKTPIPDTL
YQELYDYVNHRLSHDLSDEARALLPNVITKEVSHEIIKDRRFTSDKFFFHVPITLNYQ
AANSPSKFNQRVNAYLKEHPETPIIGIARGERNLIYITVIDSTGKILEQRSLNTIQQFDY
QKKLDNREKERVAARQAWSVVGTIKDLKQGYLSQVIHEIVDLMIHYQAVVVLENLN
FGFKSKRTGIAEKAVYQQFEKMLIDKLNCLVLKDYPAEKVGGVLNPYQLTDQFTSF
AKMGTQSGFLFYVPAPYTSKIDPLTGFVDPFVWKTIKNHESRKHFLEGFDFLHYDVK
TGDFILHFKMNRNLSFQRGLPGFMPAWDIVFEKNETQFDAKGTPFIAGKRIVPVIENH
RFTGRYRDLYPANELIALLEEKGIVFRDGSNILPKLLENDDSHAIDTMVALIRSVLQM
RNSNAATGEDYINSPVRDLNGVCFDSRFQNPEWPMDADANGAYHIALKGQLLLNHL
KESKDLKLQNGISNQDWLAYIQELRNSGGSKRTADGSEFEPKKKRKV (SEQ ID NO:
209)
**enAsABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSMTQFEGFTNLYQVSKTLRFELIPQGKTLKHIQEQGFI
EEDKARNDHYKELKPIIDRIYKTYADQCLQLVQLDWENLSAAIDSYRKEKTEETRNA
LIEEQATYRNAIHDYFIGRTDNLTDAINKRHAEIYKGLFKAELFNGKVLKQLGTVTTT
EHENALLRSFDKFTTYFSGFYRNRKNVFSAEDISTAIPHRIVQDNFPKFKENCHIFTRLI
TAVPSLREHFENVKKAIGIFVSTSIEEVFSFPFYNQLLTQTQIDLYNQLLGGISREAGTE
KIKGLNEVLNLAIQKNDETAHIIASLPHRFIPLFKQILSDRNTLSFILEEFKSDEEVIQSF
CKYKTLLRNENVLETAEALFNELNSIDLTHIFISHKKLETISSALCDHWDTLRNALYE
RRISELTGKITKSAKEKVQRSLKHEDINLQEIISAAGKELSEAFKQKTSEILSHAHAAL
 
DQPLPTTLKKQEEKEILKSQLDSLLGLYHLLDWFAVDESNEVDPEFSARLTGIKLEME
PSLSFYNKARNYATKKPYSVEKFKLNFQMPTLARGWDVNREKNNGAILFVKNGLY
YLGIMPKQKGRYKALSFEPTEKTSEGFDKMYYDYFPDAAKMIPKCSTQLKAVTAHF
QTHTTPILLSNNFIEPLEITKEIYDLNNPEKEPKKFQTAYAKKTGDQKGYREALCKWI
DFTRDFLSKYTKTTSIDLSSLRPSSQYKDLGEYYAELNPLLYHISFQRIAEKEIMDAVE
TGKLYLFQIYNKDFAKGHHGKPNLHTLYWTGLFSPENLAKTSIKLNGQAELFYRPKS
RMKRMAHRLGEKMLNKKLKDQKTPIPDTLYQELYDYVNHRLSHDLSDEARALLPN
VITKEVSHEIIKDRRFTSDKFFFHVPITLNYQAANSPSKFNQRVNAYLKEHPETPIIGIA
RGERNLIYITVIDSTGKILEQRSLNTIQQFDYQKKLDNREKERVAARQAWSVVGTIKD
LKQGYLSQVIHEIVDLMIHYQAVVVLENLNFGFKSKRTGIAEKAVYQQFEKMLIDKL
NCLVLKDYPAEKVGGVLNPYQLTDQFTSFAKMGTQSGFLFYVPAPYTSKIDPLTGFV
DPFVWKTIKNHESRKHFLEGFDFLHYDVKTGDFILHFKMNRNLSFQRGLPGFMPAW
DIVFEKNETQFDAKGTPFIAGKRIVPVIENHRFTGRYRDLYPANELIALLEEKGIVFRD
GSNILPKLLENDDSHAIDTMVALIRSVLQMRNSNAATGEDYINSPVRDLNGVCFDSR
FQNPEWPMDADANGAYHIALKGQLLLNHLKESKDLKLQNGISNQDWLAYIQELRNS
GGSKRTADGSEFEPKKKRKV (SEQ ID NO: 210)
**enAsABE7.10** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNAKTGAAGSLMDV
LHYPGMNHRVEITEGILADECAALLCYFFRMPRQVFNAQKKAQSSTDSGGSSGGSSG
SETPGTSESATPESSGGSSGGSMTQFEGFTNLYQVSKTLRFELIPQGKTLKHIQEQGFI
EEDKARNDHYKELKPIIDRIYKTYADQCLQLVQLDWENLSAAIDSYRKEKTEETRNA
LIEEQATYRNAIHDYFIGRTDNLTDAINKRHAEIYKGLFKAELFNGKVLKQLGTVTTT
EHENALLRSFDKFTTYFSGFYRNRKNVFSAEDISTAIPHRIVQDNFPKFKENCHIFTRLI
TAVPSLREHFENVKKAIGIFVSTSIEEVFSFPFYNQLLTQTQIDLYNQLLGGISREAGTE
KIKGLNEVLNLAIQKNDETAHIIASLPHRFIPLFKQILSDRNTLSFILEEFKSDEEVIQSF
CKYKTLLRNENVLETAEALFNELNSIDLTHIFISHKKLETISSALCDHWDTLRNALYE
RRISELTGKITKSAKEKVQRSLKHEDINLQEIISAAGKELSEAFKQKTSEILSHAHAAL
DQPLPTTLKKQEEKEILKSQLDSLLGLYHLLDWFAVDESNEVDPEFSARLTGIKLEME
PSLSFYNKARNYATKKPYSVEKFKLNFQMPTLARGWDVNREKNNGAILFVKNGLY
 
YLGIMPKQKGRYKALSFEPTEKTSEGFDKMYYDYFPDAAKMIPKCSTQLKAVTAHF
QTHTTPILLSNNFIEPLEITKEIYDLNNPEKEPKKFQTAYAKKTGDQKGYREALCKWI
DFTRDFLSKYTKTTSIDLSSLRPSSQYKDLGEYYAELNPLLYHISFQRIAEKEIMDAVE
TGKLYLFQIYNKDFAKGHHGKPNLHTLYWTGLFSPENLAKTSIKLNGQAELFYRPKS
RMKRMAHRLGEKMLNKKLKDQKTPIPDTLYQELYDYVNHRLSHDLSDEARALLPN
VITKEVSHEIIKDRRFTSDKFFFHVPITLNYQAANSPSKFNQRVNAYLKEHPETPIIGIA
RGERNLIYITVIDSTGKILEQRSLNTIQQFDYQKKLDNREKERVAARQAWSVVGTIKD
LKQGYLSQVIHEIVDLMIHYQAVVVLENLNFGFKSKRTGIAEKAVYQQFEKMLIDKL
NCLVLKDYPAEKVGGVLNPYQLTDQFTSFAKMGTQSGFLFYVPAPYTSKIDPLTGFV
DPFVWKTIKNHESRKHFLEGFDFLHYDVKTGDFILHFKMNRNLSFQRGLPGFMPAW
DIVFEKNETQFDAKGTPFIAGKRIVPVIENHRFTGRYRDLYPANELIALLEEKGIVFRD
GSNILPKLLENDDSHAIDTMVALIRSVLQMRNSNAATGEDYINSPVRDLNGVCFDSR
FQNPEWPMDADANGAYHIALKGQLLLNHLKESKDLKLQNGISNQDWLAYIQELRNS
GGSKRTADGSEFEPKKKRKV (SEQ ID NO: 211)
**SpCas9NG-ABE8e ("NG-ABE8e")** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
 
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESIRPKRNSDKLIARKKDWDPKKYGGFVSPTVAYSVLVVAKVEKGK
SKKLKSVKELLGITIMERSSFEKNPIDFLEAKGYKEVKKDLIIKLPKYSLFELENGRKR
MLASARFLQKGNELALPSKYVNFLYLASHYEKLKGSPEDNEQKQLFVEQHKHYLDE
IIEQISEFSKRVILADANLDKVLSAYNKHRDKPIREQAENIIHLFTLTNLGAPRAFKYFD
TTIDRKVYRSTKEVLDATLIHQSITGLYETRIDLSQLGGDSGGSKRTADGSEFEPKKK
RKV (SEQ ID NO: 212)
**NG-ABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGN
TDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAI
LSARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMD
GTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDK
NLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDN
 
LTKAERGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEI
VWDKGRDFATVRKVLSMPQVNIVKKTEVQTGGFSKESIRPKRNSDKLIARKKDWDP
KKYGGFVSPTVAYSVLVVAKVEKGKSKKLKSVKELLGITIMERSSFEKNPIDFLEAK
GYKEVKKDLIIKLPKYSLFELENGRKRMLASARFLQKGNELALPSKYVNFLYLASHY
EKLKGSPEDNEQKQLFVEQHKHYLDEIIEQISEFSKRVILADANLDKVLSAYNKHRD
KPIREQAENIIHLFTLTNLGAPRAFKYFDTTIDRKVYRSTKEVLDATLIHQSITGLYETR
IDLSQLGGDSGGSKRTADGSEFEPKKKRKV (SEQ ID NO: 213)
**SaKKH-ABE8e ("KKH-ABE8e")** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSGKRNYILG
LAIGITSVGYGIIDYETRDVIDAGVRLFKEANVENNEGRRSKRGARRLKRRRRHRIQR
VKKLLFDYNLLTDHSELSGINPYEARVKGLSQKLSEEEFSAALLHLAKRRGVHNVNE
VEEDTGNELSTKEQISRNSKALEEKYVAELQLERLKKDGEVRGSINRFKTSDYVKEA
KQLLKVQKAYHQLDQSFIDTYIDLLETRRTYYEGPGEGSPFGWKDIKEWYEMLMGH
CTYFPEELRSVKYAYNADLYNALNDLNNLVITRDENEKLEYYEKFQIIENVFKQKKK
PTLKQIAKEILVNEEDIKGYRVTSTGKPEFTNLKVYHDIKDITARKEIIENAELLDQIAK
ILTIYQSSEDIQEELTNLNSELTQEEIEQISNLKGYTGTHNLSLKAINLILDELWHTNDN
QIAIFNRLKLVPKKVDLSQQKEIPTTLVDDFILSPVVKRSFIQSIKVINAIIKKYGLPNDII
IELAREKNSKDAQKMINEMQKRNRQTNERIEEIIRTTGKENAKYLIEKIKLHDMQEGK
CLYSLEAIPLEDLLNNPFNYEVDHIIPRSVSFDNSFNNKVLVKQEENSKKGNRTPFQY
LSSSDSKISYETFKKHILNLAKGKGRISKTKKEYLLEERDINRFSVQKDFINRNLVDTR
YATRGLMNLLRSYFRVNNLDVKVKSINGGFTSFLRRKWKFKKERNKGYKHHAEDA
LIIANADFIFKEWKKLDKAKKVMENQMFEEKQAESMPEIETEQEYKEIFITPHQIKHIK
DFKDYKYSHRVDKKPNRELINDTLYSTRKDDKGNTLIVNNLNGLYDKDNDKLKKLI
NKSPEKLLMYHHDPQTYQKLKLIMEQYGDEKNPLYKYYEETGNYLTKYSKKDNGP
VIKKIKYYGNKLNAHLDITDDYPNSRNKVVKLSLKPYRFDVYLDNGVYKFVTVKNL
DVIKKENYYEVNSKCYEEAKKLKKISNQAEFIASFYNNDLIKINGELYRVIGVNNDLL
NRIEVNMIDITYREYLENMNDKRPPRIIKTIASKTQSIKKYSTDILGNLYEVKSKKHPQI
IKKGSGGSKRTADGSEFEPKKKRKV (SEQ ID NO: 214)
SaKKH-ABE8e-dimer
**SaKKH-ABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSGKRNYILGLAIGITSVGYGIIDYETRDVIDAGVRLFKE
ANVENNEGRRSKRGARRLKRRRRHRIQRVKKLLFDYNLLTDHSELSGINPYEARVK
GLSQKLSEEEFSAALLHLAKRRGVHNVNEVEEDTGNELSTKEQISRNSKALEEKYVA
ELQLERLKKDGEVRGSINRFKTSDYVKEAKQLLKVQKAYHQLDQSFIDTYIDLLETR
RTYYEGPGEGSPFGWKDIKEWYEMLMGHCTYFPEELRSVKYAYNADLYNALNDLN
NLVITRDENEKLEYYEKFQIIENVFKQKKKPTLKQIAKEILVNEEDIKGYRVTSTGKPE
FTNLKVYHDIKDITARKEIIENAELLDQIAKILTIYQSSEDIQEELTNLNSELTQEEIEQIS
NLKGYTGTHNLSLKAINLILDELWHTNDNQIAIFNRLKLVPKKVDLSQQKEIPTTLVD
DFILSPVVKRSFIQSIKVINAIIKKYGLPNDIIIELAREKNSKDAQKMINEMQKRNRQTN
ERIEEIIRTTGKENAKYLIEKIKLHDMQEGKCLYSLEAIPLEDLLNNPFNYEVDHIIPRS
VSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSIN
GGFTSFLRRKWKFKKERNKGYKHHAEDALIIANADFIFKEWKKLDKAKKVMENQM
FEEKQAESMPEIETEQEYKEIFITPHQIKHIKDFKDYKYSHRVDKKPNRELINDTLYST
RKDDKGNTLIVNNLNGLYDKDNDKLKKLINKSPEKLLMYHHDPQTYQKLKLIMEQY
GDEKNPLYKYYEETGNYLTKYSKKDNGPVIKKIKYYGNKLNAHLDITDDYPNSRNK
VVKLSLKPYRFDVYLDNGVYKFVTVKNLDVIKKENYYEVNSKCYEEAKKLKKISNQ
AEFIASFYNNDLIKINGELYRVIGVNNDLLNRIEVNMIDITYREYLENMNDKRPPRIIK
TIASKTQSIKKYSTDILGNLYEVKSKKHPQIIKKGSGGSKRTADGSEFEPKKKRKV
(SEQ ID NO: 215)
**CP1028-ABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSEIGKATAK
YFFYSNIMNFFKTEITLANGEIRKRPLIETNGETGEIVWDKGRDFATVRKVLSMPQVN
IVKKTEVQTGGFSKESILPKRNSDKLIARKKDWDPKKYGGFDSPTVAYSVLVVAKVE
 
KGKSKKLKSVKELLGITIMERSSFEKNPIDFLEAKGYKEVKKDLIIKLPKYSLFELENG
RKRMLASAGELQKGNELALPSKYVNFLYLASHYEKLKGSPEDNEQKQLFVEQHKH
YLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGDGGSGGSGGSGGSGG
SGGSGGMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLEN
LIAQLPGEKKNGLFGNLIALSLGLTPNFKSNFDLAEDAKLQLSKDTYDDDLDNLLAQI
GDQYADLFLAAKNLSDAILLSDILRVNTEITKAPLSASMIKRYDEHHQDLTLLKALVR
QQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLAR
GNSRFAWMTRKSEETITPWNFEEVVDKGASAQSFIERMTNFDKNLPNEKVLPKHSLL
YEYFTVYNELTKVKYVTEGMRKPAFLSGEQKKAIVDLLFKTNRKVTVKQLKEDYFK
KIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKV
VDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRK
DFQFYKVREINNYHHAHDAYLNAVVGTALIKKYPKLESEFVYGDYKVYDVRKMIA
KSEQSGGSKRTADGSEFEPKKKRKV (SEQ ID NO: 216)
**CP1028-ABE8e-dimer** 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEY
WMRHALTLAKRARDEREVPVGAVLVLNNRVIGEGWNRAIGLHDPTAHAEIMALRQ
GGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSINSGGSSGGSSG
SETPGTSESATPESSGGSSGGSEIGKATAKYFFYSNIMNFFKTEITLANGEIRKRPLIET
NGETGEIVWDKGRDFATVRKVLSMPQVNIVKKTEVQTGGFSKESILPKRNSDKLIAR
KKDWDPKKYGGFDSPTVAYSVLVVAKVEKGKSKKLKSVKELLGITIMERSSFEKNPI
 
DFLEAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLSAY
NKHRDKPIREQAENIIHLFTLTNLGAPAAFKYFDTTIDRKRYTSTKEVLDATLIHQSIT
GLYETRIDLSQLGGDGGSGGSGGSGGSGGSGGSGGMDKKYSIGLAIGTNSVGWAVIT
DEYKVPSKKFKVLGNTDRHSIKKNLIGALLFDSGETAEATRLKRTARRRYTRRKNRI
CYLQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTYNQ
LFEENPINASGVDAKAILSARLSKSRRLENLIAQLPGEKKNGLFGNLIALSLGLTPNFK
SNFDLAEDAKLQLSKDTYDDDLDNLLAQIGDQYADLFLAAKNLSDAILLSDILRVNT
EITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQ
EDFYPFLKDNREKIEKILTFRIPYYVGPLARGNSRFAWMTRKSEETITPWNFEEVVDK
GASAQSFIERMTNFDKNLPNEKVLPKHSLLYEYFTVYNELTKVKYVTEGMRKPAFLS
GEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRY
TGWGRLSRKLINGIRDKQSGKTILDFLKSDGFANRNFMQLIHDDSLTFKEDIQKAQVS
GQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQE
LDINRLSDYDVDHIVPQSFLKDDSIDNKVLTRSDKNRGKSDNVPSEEVVKKMKNYW
RQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTA
LIKKYPKLESEFVYGDYKVYDVRKMIAKSEQSGGSKRTADGSEFEPKKKRKV (SEQ
ID NO: 217)
**CP1041-ABE8e** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSNIMNFFKT
EITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKVEKGKSKKLKSVKE
LLGITIMERSSFEKNPIDFLEAKGYKEVKKDLIIKLPKYSLFELENGRKRMLASAGELQ
KGNELALPSKYVNFLYLASHYEKLKGSPEDNEQKQLFVEQHKHYLDEIIEQISEFSKR
VILADANLDKVLSAYNKHRDKPIREQAENIIHLFTLTNLGAPAAFKYFDTTIDRKRYT
STKEVLDATLIHQSITGLYETRIDLSQLGGDGGSGGSGGSGGSGGSGGDKKYSIG
 
LAIGTNSVGWAVITDEYKVPSKKFKVLGNTDRHSIKKNLIGALLFDSGETAEATRLK
RTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLF
GNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQ
SKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SSGGSKRTADGSEFEPKKKRKV (SEQ ID NO: 218)
**ABE8e(TadA-8e V82G)** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
 
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDE
IIEQISEFSKRVILADANLDKVLSAYNKHRDKPIREQAENIIHLFTLTNLGAPAAFKYF
DTTIDRKRYTSTKEVLDATLIHQSITGLYETRIDLSQLGGDSGGSKRTADGSEFEPKK
KRKV (SEQ ID NO: 219)
**ABE8e(TadA-8e K20AR21A)** 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
 
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDE
IIEQISEFSKRVILADANLDKVLSAYNKHRDKPIREQAENIIHLFTLTNLGAPAAFKYF
DTTIDRKRYTSTKEVLDATLIHQSITGLYETRIDLSQLGGDSGGSKRTADGSEFEPKK
KRKV (SEQ ID NO: 220)
ABE8e(TadA-8e V106W) 
MKRTADGSEFESPKKKRKVSEVEFSHEYWMRHALTLAKRARDEREVPVGAVLVLNNRVIGE
GWNRAIGLHDPTAHAEIMALRQGGLVMQNYRLIDATLYVTFEPCVMCAGAMIHSRIGRVVFG
WRNSKRGAAGSLMNVLNYPGMNHRVEITEGILADECAALLCDFYRMPRQVFNAQKKAQSSI
NSGGSSGGSSGSETPGTSESATPESSGGSSGGSDKKYSIGLAIGTNSVGWAVITDEYKVPSKKF
KVLGNTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSKSRRLE
NLIAQLPGEKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPEKYKEI
FFDQSKNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETITPWNF
EEVVDKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGRLSRK
LINGIRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEEGIKEL
GSQILKEHPVENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAERGGLSELDK
AGFIKRQLVETRQITKHVAQILDSRMNTKYDENDKLIREVKVITLKSKLVSDFRKDFQFYKVR
EINNYHHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVKKTEVQ
TGGFSKESILPKRNSDKLIARKKDWDPKKYGGFDSPTVAYSVLVVAKVEKGKSKKLKSVKEL
LGITIMERSSFEKNPIDFLEAKGYKEVKKDLIIKLPKYSLFELENGRKRMLASAGELQKGNELA
LPSKYVNFLYLASHYEKLKGSPEDNEQKQLFVEQHKHYLDEIIEQISEFSKRVILADANLDKVL
 
SAYNKHRDKPIREQAENIIHLFTLTNLGAPAAFKYFDTTIDRKRYTSTKEVLDATLIHQSITGLY
ETRIDLSQLGGDSGGSKRTADGSEFEPKKKRKV (SEQ ID NO: 473)
ABE8e-NRTH dimer editor: NLS, **wtTadA,** linker, TadA*, SpCas9-NRTH 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSDKKYSIGLAIGTNSVGWAVITD
EYKVPSKKFKVLGNTDRHSIKKNLIGALLFDSGETAEATRLKRTARRRYTRRKNRICYLQEIFSNEMA
KVDDSFFHRLEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQYADLFLAA
KNLSDAILLSDILRVNTEITKAPLSASMVKRYDEHHQDLTLLKALVRQQLPEKYKEIFFDQSKNGYA
GYIDGGASQEEFYKFIKPILEKMDGTEELLVKLNREDLLRKQRTFDNGIIPHQIHLGELHAILRRQG
DFYPFLKDNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFED
REMIEERLKTYAHLFDDKVMKQLKRLRYTGWGRLSRKLINGIRDKQSGKTILDFLKSDGFANRNFM
QLIHDDSLTFKEDIQKAQVSCQGDSLHEHIANLAGSPAIKKGILQTVKVVDELIKVMGGHKPENIVI
EMARENQTTQKGQKNSRERMKRIEEGIKELGSQILKEHPVENTQLQNEKLYLYYLQNGRDMYVDQ
ELDINRLSDYDVDHIVPQSFLKDDSIENKVLTRSDKNRGKSDNVPSEEVVKKMKNYWRQLLNAKLI
TQRKFDNLTKAERGGLSELDKAGFIKRQLAETRQITKHVAQILDSRMNTKYDENDKLIREVKVITLK
SKLVSDFRKDFQFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVRKVLSMPQVNI
VKKTEVQTGGFSKESILPKGNSDKLIARKKDWDPKKYGGFNSPTVAYSVLVVAKVEKGKSKKLKSV
KELLGITIMERSSFEKNPIGFLEAKGYKEVKKDLIIKLPKYSLFELENGRKRMLASASVLHKGNELAL
PSKYVNFLYLASHYEKLKGSSEDNKQKQLFVEQHKHYLDEIIEQISEFSKRVILADANLDKVLSAYNK
HRDKPIREQAENIIHLFTLTNLGASAAFKYFDTTIGRKLYTSTKEVLDATLIHQSITGLYETRIDLSQLG
GDSGGSKRTADGSEFEPKKKRKV (SEQ ID NO: 474)
ABE8e-NRTH monomer editor: NLS, linker, TadA*, SpCas9-NRTH 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGNTDRHSIKKN
 
LIGALLFDSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILRVNTEITKAP
LSASMVKRYDEHHQDLTLLKALVRQQLPEKYKEIFFDQSKNGYAGYIDGGASQEEFYKFIKPILEK
MDGTEELLVKLNREDLLRKQRTFDNGIIPHQIHLGELHAILRRQGDFYPFLKDNREKIEKILTFRIP
YYVGPLARGNSRFAWMTRKSEETITPWNFEEVVDKGASAQSFIERMTNFDKNLPNEKVLPKHSLLY
EYFTVYNELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHLFDDKVMK
QLKRLRYTGWGRLSRKLINGIRDKQSGKTILDFLKSDGFANRNFMQLIHDDSLTFKEDIQKAQVSC
QGDSLHEHIANLAGSPAIKKGILQTVKVVDELIKVMGGHKPENIVIEMARENQTTQKGQKNSRERM
KRIEEGIKELGSQILKEHPVENTQLQNEKLYLYYLQNGRDMYVDQELDINRLSDYDVDHIVPQSFLK
DDSIENKVLTRSDKNRGKSDNVPSEEVVKKMKNYWRQLLNAKLITQRKFDNLTKAERGGLSELDK
AGFIKRQLAETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFFYSNIMNFFKT
EITLANGEIRKRPLIETNGETGEIVWDKGRDFATVRKVLSMPQVNIVKKTEVQTGGFSKESILPKGNS
DKLIARKKDWDPKKYGGFNSPTVAYSVLVVAKVEKGKSKKLKSVKELLGITIMERSSFEKNPIGFLE
AKGYKEVKKDLIIKLPKYSLFELENGRKRMLASASVLHKGNELALPSKYVNFLYLASHYEKLKGSSE
DNKQKQLFVEQHKHYLDEIIEQISEFSKRVILADANLDKVLSAYNKHRDKPIREQAENIIHLFTLTNL
GASAAFKYFDTTIGRKLYTSTKEVLDATLIHQSITGLYETRIDLSQLGGDSGGSKRTADGSEFEPKK
KRKV (SEQ ID NO: 475)
ABE8e-SpyMac dimer editor: NLS, **wtTadA,** linker, TadA*, SpCas9-SpyMac 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLIIDPTAIIAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSDKKYSIGLAIGTNSVGWAVITD
EYKVPSKKFKVLGNTDRHSIKKNLIGALLFDSGETAEATRLKRTARRRYTRRKNRICYLQEIFSNEMA
KVDDSFFHRLEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQYADLFLAA
KNLSDAILLSDILRVNTEITKAPLSASMIKRYDEHHQDLTLLKALVRQQLPEKYKEIFFDQSKNGYAG
YIDGGASQEEFYKFIKPILEKMDGTEELLVKLNREDLLRKQRTFDNGSIPHQIHLGELHAILRRQED
FYPFLKDNREKIEKILTFRIPYYVGPLARGNSRFAWMTRKSEETITPWNFEEVVDKGASAQSFIERMT
 
NFDKNLPNEKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQL
IHDDSLTFKEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDMYVDQEL
DINRLSDYDVDHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVYDVRKMIAKSEQ
EIGKATAKYFFYSNIMNFFKTEITLANGEIRKRPLIETNGETGEIVWDKGRDFATVRKVLSMPQVNIV
KKTEIQTVGQNGGLFDDNPKSPLEVTPSKLVPLKKELNPKKYGGYQKPTTAYPVLLITDTKQLIPISV
MNKKQFEQNPVKFLRDRGYQQVGKNDFIKLPKYTLVDIGDGIKRLWASSKEIHKGNQLVVSKKSQI
LLYHAHHLDSDLSNDYLQNHNQQFDVLFNEIISFSKKCKLGKEHIQKIENVYSNKKNSASIEELAES
FIKLLGFTQLGATSPFNFLGVKLNQKQYKGKKDYILPCTEGTLIRQSITGLYETRVDLSKIGEDSGGS
KRTADGSEFEPKKKRKV (SEQ ID NO: 476)
ABE8e-SpyMac monomer editor: NLS, **wtTadA,** linker, TadA*, SpCas9-SpyMac 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKNSRERMKR
IEEGIKELGSQILKEHPVENTQLQNEKLYLYYLQNGRDMYVDQELDINRLSDYDVDHIVPQSFLKD
DSIDNKVLTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYKVREINNYH
HAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTEIQTVGQNGGLFDDNPKS
PLEVTPSKLVPLKKELNPKKYGGYQKPTTAYPVLLITDTKQLIPISVMNKKQFEQNPVKFLRDRGYQ
 
QVGKNDFIKLPKYTLVDIGDGIKRLWASSKEIHKGNQLVVSKKSQILLYHAHHLDSDLSNDYLQNH
NQQFDVLFNEIISFSKKCKLGKEHIQKIENVYSNKKNSASIEELAESFIKLLGFTQLGATSPFNFLGV
KLNQKQYKGKKDYILPCTEGTLIRQSITGLYETRVDLSKIGEDSGGSKRTADGSEFEPKKKRKV
(SEQ ID NO: 477)
ABE8e-VRQR-CP1041 dimer: NLS, **wtTadA,** linker, TadA*, SpCas9-VROR-CP1041 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSNIMNFFKTEITLANGEIRKRPL
IETNGETGEIVWDKGRDFATVRKVLSMPQVNIVKKTEVQTGGFSKESIRPKRNSDKLIARKKDWDP
KKYGGFVSPTVAYSVLVVAKVEKGKSKKLKSVKELLGITIMERSSFEKNPIDFLEAKGYKEVKKDLII
KLPKYSLFELENGRKRMLASARFLQKGNELALPSKYVNFLYLASHYEKLKGSPEDNEQKQLFVEQH
KHYLDEIIEQISEFSKRVILADANLDKVLSAYNKHRDKPIREQAENIIHLFTLTNLGAPRAFKYFDTTI
DRKVYRSTKEVLDATLIHQSITGLYETRIDLSQLGGDGGSGGSGGSGGSGGSGGSGGDKKYSIGLAI
GTNSVGWAVITDEYKVPSKKFKVLGNTDRHSIKKNLIGALLFDSGETAEATRLKRTARRRYTRRKNRI
CYLQEIFSNEMAKVDDSFFHRLEESFLVEEDKKHERHPIFGNIVDEVAYHEKYPTIYHLRKKLVDST
DKADLRLIYLALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTYDDDLDNLLA
QIGDQYADLFLAAKNLSDAILLSDILRVNTEITKAPLSASMIKRYDEHHQDLTLLKALVRQQLPEKYK
EIFFDQSKNGYAGYIDGGASQEEFYKFIKPILEKMDGTEELLVKLNREDLLRKQRTFDNGSIPHQIH
LGELHAILRRQEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAFLSGEQKKAIV
DLLFKTNRKVTVKQLKEDYFKKIECFDSVEISGVEDRFNASLGTYHDLLKIIKDKDFLDNEENEDIL
EDIVLTLTLFEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVKVVDELVK
VMGRHKPENIVIEMARENQTTQKGQKNSRERMKRIEEGIKELGSQILKEHPVENTQLQNEKLYLYY
LQNGRDMYVDQELDINRLSDYDVDHIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLESEFVYGDY
KVYDVRKMIAKSEQEIGKATAKYFFYSSGGSKRTADGSEFEPKKKRKV (SEQ ID NO: 478)
ABE8e-VRQR-CP1041 monomer: NLS, linker, TadA*, SpCas9-VRQR-CP1041 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSNIMNFFKTEITLANGEIRKRPLIETNGETGEIVWDKGRDFATVRK
VLSMPQVNIVKKTEVQTGGFSKESIRPKRNSDKLIARKKDWDPKKYGGFVSPTVAYSVLVVAKVEK
GKSKKLKSVKELLGITIMERSSFEKNPIDFLEAKGYKEVKKDLIIKLPKYSLFELENGRKRMLASARF
LQKGNELALPSKYVNFLYLASHYEKLKGSPEDNEQKQLFVEQHKHYLDEIIEQISEFSKRVILADAN
LDKVLSAYNKHRDKPIREQAENIIHLFTLTNLGAPRAFKYFDTTIDRKVYRSTKEVLDATLIHQSITGL
YETRIDLSQLGGDGGSGGSGGSGGSGGSGGSGGDKKYSIGLAIGTNSVGWAVITDEYKVPSKKFKV
LGNTDRHSIKKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHMIKFRGHFLI
EGDLNPDNSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNLSDAILLSD
ILRVNTEITKAPLSASMIKRYDEHHQDLTLLKALVRQQLPEKYKEIFFDQSKNGYAGYIDGGASQEE
FYKFIKPILEKMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKEDYFKKI
ECFDSVEISGVEDRFNASLGTYHDLLKIIKDKDFLDNEENEDILEDIVLTLTLFEDREMIEERLKTYA
HLFDDKVMKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDINRLSDYD
VDHIVPQSFLKDDSIDNKVLTRSDKNRGKSDNVPSEEVVKKMKNYWRQLLNAKLITQRKFDNLTKA
ERGGLSELDKAGFIKRQLVETRQITKHVAQILDSRMNTKYDENDKLIREVKVITLKSKLVSDFRKDF
QFYKVREINNYHHAHDAYLNAVVGTALIKKYPKLESEFVYGDYKVYDVRKMIAKSEQEIGKATAKYF
FYSSGGSKRTADGSEFEPKKKRKV (SEQ ID NO: 479)
ABE8e-SaCas9 dimer editor: NLS, **wtTadA,** linker, TadA*, SaCas9 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSGKRNYILGLAIGITSVGYGIIDY
ETRDVIDAGVRLFKEANVENNEGRRSKRGARRLKRRRRHRIQRVKKLLFDYNLLTDHSELSGINPYE
ARVKGLSQKLSEEEFSAALLHLAKRRGVHNVNEVEEDTGNELSTKEQISRNSKALEEKYVAELQLER
LKKDGEVRGSINRFKTSDYVKEAKQLLKVQKAYHQLDQSFIDTYIDLLETRRTYYEGPGEGSPFGW
 
KDIKEWYEMLMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQIAKILTIYQS
SEDIQEELTNLNSELTQEEIEQISNLKGYTGTHNLSLKAINLILDELWHTNDNQIAIFNRLKLVPKKVD
LSQQKEIPTTLVDDFILSPVVKRSFIQSIKVINAIIKKYGLPNDIIIELAREKNSKDAQKMINEMQKRNR
QTNERIEEIIRTTGKENAKYLIEKIKLHDMQEGKCLYSLEAIPLEDLLNNPFNYEVDHIIPRSVSFDNS
FNNKVLVKQEENSKKGNRTPFQYLSSSDSKISYETFKKHILNLAKGKGRISKTKKEYLLEERDINRFS
VQKDFINRNLVDTRYATRGLMNLLRSYFRVNNLDVKVKSINGGFTSFLRRKWKFKKERNKGYKHH
AEDALIIANADFIFKEWKKLDKAKKVMENQMFEEKQAESMPEIETEQEYKEIFITPHQIKHIKDFK
DYKYSHRVDKKPNRELINDTLYSTRKDDKGNTLIVNNLNGLYDKDNDKLKKLINKSPEKLLMYHHD
PQTYQKLKLIMEQYGDEKNPLYKYYEETGNYLTKYSKKDNGPVIKKIKYYGNKLNAHLDITDDYPN
SRNKVVKLSLKPYRFDVYLDNGVYKFVTVKNLDVIKKENYYEVNSKCYEEAKKLKKISNQAEFIASF
YNNDLIKINGELYRVIGVNNDLLNRIEVNMIDITYREYLENMNDKRPPRIIKTIASKTQSIKKYSTDILG
NLYEVKSKKHPQIIKKGSGGSKRTADGSEFEPKKKRKV (SEQ ID NO: 205)
ABE8e-SaCas9 monomer editor: NLS, linker, TadA*, SaCas9 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSGKRNYILGLAIGITSVGYGIIDYETRDVIDAGVRLFKEANVENNEG
RRSKRGARRLKRRRRHRIQRVKKLLFDYNLLTDHSELSGINPYEARVKGLSQKLSEEEFSAALLHLA
KRRGVHNVNEVEEDTGNELSTKEQISRNSKALEEKYVAELQLERLKKDGEVRGSINRFKTSDYVKE
AKQLLKVQKAYHQLDQSFIDTYIDLLETRRTYYEGPGEGSPFGWKDIKEWYEMLMGHCTYFPEEL
RSVKYAYNADLYNALNDLNNLVITRDENEKLEYYEKFQIIENVFKQKKKPTLKQIAKEILVNEEDIKG
YRVTSTGKPEFTNLKVYHDIKDITARKEIIENAELLDQIAKILTIYQSSEDIQEELTNLNSELTQEEIEQI
SNLKGYTGTHNLSLKAINLILDELWHTNDNQIAIFNRLKLVPKKVDLSQQKEIPTTLVDDFILSPVVK
RSFIQSIKVINAIIKKYGLPNDIIIELAREKNSKDAQKMINEMQKRNRQTNERIEEIIRTTGKENAKYLI
EKIKLHDMQEGKCLYSLEAIPLEDLLNNPFNYEVDHIIPRSVSFDNSFNNKVLVKQEENSKKGNRTP
FQYLSSSDSKISYETFKKHILNLAKGKGRISKTKKEYLLEERDINRFSVQKDFINRNLVDTRYATRGL
MNLLRSYFRVNNLDVKVKSINGGFTSFLRRKWKFKKERNKGYKHHAEDALIIANADFIFKEWKKLD
KAKKVMENQMFEEKQAESMPEIETEQEYKEIFITPHQIKHIKDFKDYKYSHRVDKKPNRELINDTL
YSTRKDDKGNTLIVNNLNGLYDKDNDKLKKLINKSPEKLLMYHHDPQTYQKLKLIMEQYGDEKNP
LYKYYEETGNYLTKYSKKDNGPVIKKIKYYGNKLNAHLDITDDYPNSRNKVVKLSLKPYRFDVYLDN
GVYKFVTVKNLDVIKKENYYEVNSKCYEEAKKLKKISNQAEFIASFYNNDLIKINGELYRVIGVNNDL
LNRIEVNMIDITYREYLENMNDKRPPRIIKTIASKTQSIKKYSTDILGNLYEVKSKKHPQIIKKGSGGS
KRTADGSEFEPKKKRKV (SEQ ID NO: 204)
ABE8e-NRCH dimer editor: NLS, **wtTadA,** linker, TadA*, SpCas9-NRCH 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSDKKYSIGLAIGTNSVGWAVITD
EYKVPSKKFKVLGNTDRHSIKKNLIGALLFDSGETAEATRLKRTARRRYTRRKNRICYLQEIFSNEMA
KVDDSFFHRLEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQYADLFLAA
KNLSDAILLSDILRVNTEITKAPLSASMVKRYDEHHQDLTLLKALVRQQLPEKYKEIFFDQSKNGYA
GYIDGGASQEEFYKFIKPILEKMDGTEELLVKLNREDLLRKQRTFDNGIIPHQIHLGELHAILRRQG
DFYPFLKDNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFED
REMIEERLKTYAHLFDDKVMKQLKRLRYTGWGRLSRKLINGIRDKQSGKTILDFLKSDGFANRNFM
QLIHDDSLTFKEDIQKAQVSCQGDSLHEHIANLAGSPAIKKGILQTVKVVDELIKVMGGHKPENIVI
EMARENQTTQKGQKNSRERMKRIEEGIKELGSQILKEHPVENTQLQNEKLYLYYLQNGRDMYVDQ
ELDINRLSDYDVDHIVPQSFLKDDSIENKVLTRSDKNRGKSDNVPSEEVVKKMKNYWRQLLNAKLI
TQRKFDNLTKAERGGLSELDKAGFIKRQLAETRQITKHVAQILDSRMNTKYDENDKLIREVKVITLK
SKLVSDFRKDFQFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVRKVLSMPQVNI
VKKTEVQTGGFSKESILPKGNSDKLIARKKDWDPKKYGGFNSPTVAYSVLVVAKVEKGKSKKLKSV
KELLGITIMERSSFEKNPIDFLEAKGYKEVKKDLIIKLPKYSLFELENGRKRMLASAGVLQKGNELAL
PSKYVNFLYLASHYEKLKGSPEDNEQKQLFVEQHKHYLDEIIEQISEFSKRVILADANLDKVLSAYNK
HRDKPIREQAENIIHLFTLTNLGAPAAFKYFDTTINRKQYNTTKEVLDATLIRQSITGLYETRIDLSQL
GGDSGGSKRTADGSEFEPKKKRKV (SEQ ID NO: 480)
ABE8e-NRCH monomer editor: NLS, linker, TadA*, SpCas9-NRCH 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFGNLIALSLGLT
 
PNFKSNFDLAEDAKLQLSKDTYDDDLDNLLAQIGDQYADLFLAAKNLSDAILLSDILRVNTEITKAP
LSASMVKRYDEHHQDLTLLKALVRQQLPEKYKEIFFDQSKNGYAGYIDGGASQEEFYKFIKPILEK
MDGTEELLVKLNREDLLRKQRTFDNGIIPHQIHLGELHAILRRQGDFYPFLKDNREKIEKILTFRIP
YYVGPLARGNSRFAWMTRKSEETITPWNFEEVVDKGASAQSFIERMTNFDKNLPNEKVLPKHSLLY
EYFTVYNELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHLFDDKVMK
QLKRLRYTGWGRLSRKLINGIRDKQSGKTILDFLKSDGFANRNFMQLIHDDSLTFKEDIQKAQVSC
QGDSLHEHIANLAGSPAIKKGILQTVKVVDELIKVMGGHKPENIVIEMARENQTTQKGQKNSRERM
KRIEEGIKELGSQILKEHPVENTQLQNEKLYLYYLQNGRDMYVDQELDINRLSDYDVDHIVPQSFLK
DDSIENKVLTRSDKNRGKSDNVPSEEVVKKMKNYWRQLLNAKLITQRKFDNLTKAERGGLSELDK
AGFIKRQLAETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFFYSNIMNFFKT
EITLANGEIRKRPLIETNGETGEIVWDKGRDFATVRKVLSMPQVNIVKKTEVQTGGFSKESILPKGNS
DKLIARKKDWDPKKYGGFNSPTVAYSVLVVAKVEKGKSKKLKSVKELLGITIMERSSFEKNPIDFLE
AKGYKEVKKDLIIKLPKYSLFELENGRKRMLASAGVLQKGNELALPSKYVNFLYLASHYEKLKGSPE
DNEQKQLFVEQHKHYLDEIIEQISEFSKRVILADANLDKVLSAYNKHRDKPIREQAENIIHLFTLTNL
GAPAAFKYFDTTINRKQYNTTKEVLDATLIRQSITGLYETRIDLSQLGGDSGGSKRTADGSEFEPKK
KRKV (SEQ ID NO: 481)
*ABE8e-NRRH dimer editor:* NLS, wtTadA, linker, *TadA*, SpCas9-NRRH* 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEYW
MRHALTLAKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLNYPGMNHRV
EITEGILADECAALLCDFYRMPRQVFNAQKKAQSSINSGGSSGGSSGSETPGTSESATPES
SGGSSGGSDKKYSIGLAIGTNSVGWAVITDEYKVPSKKFKVLGNTDRHSIKKNLIGALLFDS
GETAEATRLKRTARRRYTRRKNRICYLQEIFSNEMAKVDDSFFHRLEESFLVEEDKKHERH
PIFGNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGLFGN
LIALSLGLTPNFKSNFDLAEDAKLQLSKDTYDDDLDNLLAQIGDQYADLFLAAKNLSDAIL
LSDILRVNTEITKAPLSASMVKRYDEHHQDLTLLKALVRQQLPEKYKEIFFDQSKNGYAGYI
DGGASQEEFYKFIKPILEKMDGTEELLVKLNREDLLRKQRTFDNGIIPHQIHLGELHAILR
RQGDFYPFLKDNREKIEKILTFRIPYYVGPLARGNSRFAWMTRKSEETITPWNFEEVVDKG
ASAQSFIERMTNFDKNLPNEKVLPKHSLLYEYFTVYNELTKVKYVTEGMRKPAFLSGEQKK
 
AIVDLLFKTNRKVTVKQLKEDYFKKIECFDSVEISGVEDRFNASLGTYHDLLKIIKDKDFLD
NEENEDILEDIVLTLTLFEDREMIEERLKTYAHLFDDKVMKQLKRLRYTGWGRLSRKLING
IRDKQSGKTILDFLKSDGFANRNFMQLIHDDSLTFKEDIQKAQVSCQGDSLHEHIANLAG
SPAIKKGILQTVKVVDELIKVMGGHKPENIVIEMARENQTTQKGQKNSRERMKRIEEGIKE
LGSQILKEHPVENTQLQNEKLYLYYLQNGRDMYVDQELDINRLSDYDVDHIVPQSFLKDD
SIENKVLTRSDKNRGKSDNVPSEEVVKKMKNYWRQLLNAKLITQRKFDNLTKAERGGLSE
LDKAGFIKRQLAETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEIGKA
TAKYFFYSNIMNFFKTEITLANGEIRKRPLIETNGETGEIVWDKGRDFATVRKVLSMPQVNI
VKKTEVQTGGFSKESILPKGNSDKLIARKKDWDPKKYGGFNSPTAAYSVLVVAKVEKGKS
KKLKSVKELLGITIMERSSFEKNPIGFLEAKGYKEVKKDLIIKLPKYSLFELENGRKRMLAS
AGVLHKGNELALPSKYVNFLYLASHYEKLKGSPEDNEQKQLFVEQHKHYLDEIIEQISEFS
KRVILADANLDKVLSAYNKHRDKPIREQAENIIHLFTLTNLGVPAAFKYFDTTIDKKRYTST
KEVLDATLIHQSITGLYETRIDLSQLGGDSGGSKRTADGSEFEPKKKRKV (SEQ ID NO:
482)
ABE8e-NRRH monomer editor: NLS, linker, TadA*, SpCas9-NRRH 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMVKRYDEHHQDLTLLKALVRQQLPEKYKEIFFDQ
SKNGYAGYIDGGASQEEFYKFIKPILEKMDGTEELLVKLNREDLLRKQRTFDNGIIPH
QIHLGELHAILRRQGDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRLRYTGWGRLSRKLINGIRDKQSGKTILDFLKSDGFANRNFMQLIHD
DSLTFKEDIQKAQVSCQGDSLHEHIANLAGSPAIKKGILQTVKVVDELIKVMGGHKP
ENIVIEMARENQTTQKGQKNSRERMKRIEEGIKELGSQILKEHPVENTQLQNEKLYLY
 
YLQNGRDMYVDQELDINRLSDYDVDHIVPQSFLKDDSIENKVLTRSDKNRGKSDNV
PSEEVVKKMKNYWRQLLNAKLITQRKFDNLTKAERGGLSELDKAGFIKRQLAETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESILPKGNSDKLIARKKDWDPKKYGGFNSPTAAYSVLVVAKVEKGK
SKKLKSVKELLGITIMERSSFEKNPIGFLEAKGYKEVKKDLIIKLPKYSLFELENGRKR
MLASAGVLHKGNELALPSKYVNFLYLASHYEKLKGSPEDNEQKQLFVEQHKHYLD
EIIEQISEFSKRVILADANLDKVLSAYNKHRDKPIREQAENIIHLFTLTNLGVPAAFKYF
DTTIDKKRYTSTKEVLDATLIHQSITGLYETRIDLSQLGGDSGGSKRTADGSEFEPKK
KRKV (SEQ ID NO: 483)
*SaKKH-ABE8e dimer editor:* NLS, wtTadA, linker, *TadA*, SaKKH* 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEYW
MRHALTLAKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLNYPGMNHRV
EITEGILADECAALLCDFYRMPRQVFNAQKKAQSSINSGGSSGGSSGSETPGTSESATPES
SGGSSGGSGKRNYILGLAIGITSVGYGIIDYETRDVIDAGVRLFKEANVENNEGRRSKRGAR
RLKRRRRHRIQRVKKLLFDYNLLTDHSELSGINPYEARVKGLSQKLSEEEFSAALLHLAKRR
GVHNVNEVEEDTGNELSTKEQISRNSKALEEKYVAELQLERLKKDGEVRGSINRFKTSDYV
KEAKQLLKVQKAYHQLDQSFIDTYIDLLETRRTYYEGPGEGSPFGWKDIKEWYEMLMGH
CTYFPEELRSVKYAYNADLYNALNDLNNLVITRDENEKLEYYEKFQIIENVFKQKKKPTLK
QIAKEILVNEEDIKGYRVTSTGKPEFTNLKVYHDIKDITARKEIIENAELLDQIAKILTIYQSSE
DIQEELTNLNSELTQEEIEQISNLKGYTGTHNLSLKAINLILDELWHTNDNQIAIFNRLKLVP
KKVDLSQQKEIPTTLVDDFILSPVVKRSFIQSIKVINAIIKKYGLPNDIIIELAREKNSKDAQK
MINEMQKRNRQTNERIEEIIRTTGKENAKYLIEKIKLHDMQEGKCLYSLEAIPLEDLLNNPF
NYEVDHIIPRSVSFDNSFNNKVLVKQEENSKKGNRTPFQYLSSSDSKISYETFKKHILNLAK
GKGRISKTKKEYLLEERDINRFSVQKDFINRNLVDTRYATRGLMNLLRSYFRVNNLDVKVK
SINGGFTSFLRRKWKFKKERNKGYKHHAEDALIIANADFIFKEWKKLDKAKKVMENQMF
EEKQAESMPEIETEQEYKEIFITPHQIKHIKDFKDYKYSHRVDKKPNRKLINDTLYSTRKDD
KGNTLIVNNLNGLYDKDNDKLKKLINKSPEKLLMYHHDPQTYQKLKLIMEQYGDEKNPL
YKYYEETGNYLTKYSKKDNGPVIKKIKYYGNKLNAHLDITDDYPNSRNKVVKLSLKPYRFD
 
VYLDNGVYKFVTVKNLDVIKKENYYEVNSKCYEEAKKLKKISNQAEFIASFYKNDLIKINGE
LYRVIGVNNDLLNRIEVNMIDITYREYLENMNDKRPPHIIKTIASKTQSIKKYSTDILGNLYEV
KSKKHPQIIKKGSGGSKRTADGSEFEPKKKRKV (SEQ ID NO: 484)
SaKKH-ABE8e monomer editor: NLS, linker, TadA*, SaKKH 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSGKRNYILG
LAIGITSVGYGIIDYETRDVIDAGVRLFKEANVENNEGRRSKRGARRLKRRRRHRIQR
VKKLLFDYNLLTDHSELSGINPYEARVKGLSQKLSEEEFSAALLHLAKRRGVHNVNE
VEEDTGNELSTKEQISRNSKALEEKYVAELQLERLKKDGEVRGSINRFKTSDYVKEA
KQLLKVQKAYHQLDQSFIDTYIDLLETRRTYYEGPGEGSPFGWKDIKEWYEMLMGH
CTYFPEELRSVKYAYNADLYNALNDLNNLVITRDENEKLEYYEKFQIIENVFKQKKK
PTLKQIAKEILVNEEDIKGYRVTSTGKPEFTNLKVYHDIKDITARKEIIENAELLDQIAK
ILTIYQSSEDIQEELTNLNSELTQEEIEQISNLKGYTGTHNLSLKAINLILDELWHTNDN
QIAIFNRLKLVPKKVDLSQQKEIPTTLVDDFILSPVVKRSFIQSIKVINAIIKKYGLPNDII
IELAREKNSKDAQKMINEMQKRNRQTNERIEEIIRTTGKENAKYLIEKIKLHDMQEGK
CLYSLEAIPLEDLLNNPFNYEVDHIIPRSVSFDNSFNNKVLVKQEENSKKGNRTPFQY
LSSSDSKISYETFKKHILNLAKGKGRISKTKKEYLLEERDINRFSVQKDFINRNLVDTR
YATRGLMNLLRSYFRVNNLDVKVKSINGGFTSFLRRKWKFKKERNKGYKHHAEDA
LIIANADFIFKEWKKLDKAKKVMENQMFEEKQAESMPEIETEQEYKEIFITPHQIKHIK
DFKDYKYSHRVDKKPNRKLINDTLYSTRKDDKGNTLIVNNLNGLYDKDNDKLKKLI
NKSPEKLLMYHHDPQTYQKLKLIMEQYGDEKNPLYKYYEETGNYLTKYSKKDNGP
VIKKIKYYGNKLNAHLDITDDYPNSRNKVVKLSLKPYRFDVYLDNGVYKFVTVKNL
DVIKKENYYEVNSKCYEEAKKLKKISNQAEFIASFYKNDLIKINGELYRVIGVNNDLL
NRIEVNMIDITYREYLENMNDKRPPHIIKTIASKTQSIKKYSTDILGNLYEVKSKKHPQ
IIKKGSGGSKRTADGSEFEPKKKRKV (SEQ ID NO: 485)
ABE8e-NG dimer editor: NLS, **wtTadA,** linker, TadA*, SpCas9-NG 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
 
VFNAQKKAQSSINSGGSSGGSSGSETPGTSESATPESSGGSSGGSDKKYSIGLAIGTNSVGWAVITD
EYKVPSKKFKVLGNTDRHSIKKNLIGALLFDSGETAEATRLKRTARRRYTRRKNRICYLQEIFSNEMA
KVDDSFFHRLEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSKSRRLENLIA
QLPGEKKNGLFGNLIALSLGLTPNFKSNFDLAEDAKLQLSKDTYDDDLDNLLAQIGDQYADLFLAA
KNLSDAILLSDILRVNTEITKAPLSASMIKRYDEHHQDLTLLKALVRQQLPEKYKEIFFDQSKNGYAG
YIDGGASQEEFYKFIKPILEKMDGTEELLVKLNREDLLRKQRTFDNGSIPHQIHLGELHAILRRQED
FYPFLKDNREKIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQL
IHDDSLTFKEDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRDMYVDQEL
DINRLSDYDVDHIVPQSFLKDDSIDNKVLTRSDKNRGKSDNVPSEEVVKKMKNYWRQLLNAKLITQ
RKFDNLTKAERGGLSELDKAGFIKRQLVETRQITKHVAQILDSRMNTKYDENDKLIREVKVITLKSK
LVSDFRKDFQFYKVREINNYHHAHDAYLNAVVGTALIKKYPKLESEFVYGDYKVYDVRKMIAKSEQ
EIGKATAKYFFYSNIMNFFKTEITLANGEIRKRPLIETNGETGEIVWDKGRDFATVRKVLSMPQVNIV
KKTEVQTGGFSKESIRPKRNSDKLIARKKDWDPKKYGGFVSPTVAYSVLVVAKVEKGKSKKLKSVK
ELLGITIMERSSFEKNPIDFLEAKGYKEVKKDLIIKLPKYSLFELENGRKRMLASARFLQKGNELALP
SKYVNFLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRIDLSQL
GGDSGGSKRTADGSEFEPKKKRKV (SEQ ID NO: 213)
ABE8e-NG monomer editor: NLS, linker, TadA*, SpCas9-NG ("NG-ABE8e") 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSDKKYSIGLAIGTNSVGWAVITDEYKVPSKKFKVLGNTDRHSIKKN
LIGALLFDSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLFDDKVMKQL
 
KRRRYTGWGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKNSRERMKR
IEEGIKELGSQILKEHPVENTQLQNEKLYLYYLQNGRDMYVDQELDINRLSDYDVDHIVPQSFLKD
DSIDNKVLTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYKVREINNYH
HAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTEVQTGGFSKESIRPKRNSD
KLIARKKDWDPKKYGGFVSPTVAYSVLVVAKVEKGKSKKLKSVKELLGITIMERSSFEKNPIDFLEA
KGYKEVKKDLIIKLPKYSLFELENGRKRMLASARFLQKGNELALPSKYVNFLYLASHYEKLKGSPED
NEQKQLFVEQHKHYLDEIIEQISEFSKRVILADANLDKVLSAYNKHRDKPIREQAENIIHLFTLTNLG
APRAFKYFDTTIDRKVYRSTKEVLDATLIHQSITGLYETRIDLSQLGGDSGGSKRTADGSEFEPKKK
RKV (SEQ ID NO: 212)
*ABE8e-CP1041 dimer editor:* NLS, wtTadA, linker, *TadA*, CP1041* 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEYW
MRHALTLAKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLNYPGMNHRV
EITEGILADECAALLCDFYRMPRQVFNAQKKAQSSINSGGSSGGSSGSETPGTSESATPES
SGGSSGGSNIMNFFKTEITLANGEIRKRPLIETNGETGEIVWDKGRDFATVRKVLSMPQVN
IVKKTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRMLAS
AGELQKGNELALPSKYVNFLYLASHYEKLKGSPEDNEQKQLFVEQHKHYLDEIIEQISEFS
KRVILADANLDKVLSAYNKHRDKPIREQAENIIHLFTLTNLGAPAAFKYFDTTIDRKRYTST
KEVLDATLIHQSITGLYETRIDLSQLGGDGGSGGSGGSGGSGGSGGSGGDKKYSIGLAIGT
NSVGWAVITDEYKVPSKKFKVLGNTDRHSIKKNLIGALLFDSGETAEATRLKRTARRRYTRR
KNRICYLQEIFSNEMAKVDDSFFHRLEESFLVEEDKKHERHPIFGNIVDEVAYHEKYPTIY
HLRKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFDLA
EDAKLQLSKDTYDDDLDNLLAQIGDQYADLFLAAKNLSDAILLSDILRVNTEITKAPLSASM
IKRYDEHHQDLTLLKALVRQQLPEKYKEIFFDQSKNGYAGYIDGGASQEEFYKFIKPILEK
MDGTEELLVKLNREDLLRKQRTFDNGSIPHQIHLGELHAILRRQEDFYPFLKDNREKIEKI
LTFRIPYYVGPLARGNSRFAWMTRKSEETITPWNFEEVVDKGASAQSFIERMTNFDKNLPN
 
EKVLPKHSLLYEYFTVYNELTKVKYVTEGMRKPAFLSGEQKKAIVDLLFKTNRKVTVKQLK
EDYFKKIECFDSVEISGVEDRFNASLGTYHDLLKIIKDKDFLDNEENEDILEDIVLTLTLFE
DREMIEERLKTYAHLFDDKVMKQLKRRRYTGWGRLSRKLINGIRDKQSGKTILDFLKSDG
FANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSSGGSKRTADG
SEFEPKKKRKV (SEQ ID NO: 486)
ABE8e-CP1041 monomer editor: NLS, linker, TadA*, CP1041 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSNIMNFFKT
EITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKVEKGKSKKLKSVKE
LLGITIMERSSFEKNPIDFLEAKGYKEVKKDLIIKLPKYSLFELENGRKRMLASAGELQ
KGNELALPSKYVNFLYLASHYEKLKGSPEDNEQKQLFVEQHKHYLDEIIEQISEFSKR
VILADANLDKVLSAYNKHRDKPIREQAENIIHLFTLTNLGAPAAFKYFDTTIDRKRYT
STKEVLDATLIHQSITGLYETRIDLSQLGGDGGSGGSGGSGGSGGSGGSGGDKKYSIG
LAIGTNSVGWAVITDEYKVPSKKFKVLGNTDRHSIKKNLIGALLFDSGETAEATRLK
RTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLF
GNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQ
SKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
 
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SSGGSKRTADGSEFEPKKKRKV (SEQ ID NO: 218)
ABE8e-CP 1028 dimer editor: NLS, **wtTadA,** linker, TadA*, CP1028 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSEIGKATAKYFFYSNIMNFFKTE
ITLANGEIRKRPLIETNGETGEIVWDKGRDFATVRKVLSMPQVNIVKKTEVQTGGFSKESILPKRNSD
KLIARKKDWDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHYEKLKGSPED
NEQKQLFVEQHKHYLDEIIEQISEFSKRVILADANLDKVLSAYNKHRDKPIREQAENIIHLFTLTNLG
APAAFKYFDTTIDRKRYTSTKEVLDATLIHQSITGLYETRIDLSQLGGDGGSGGSGGSGGSGGSGGS
GGMDKKYSIGLAIGTNSVGWAVITDEYKVPSKKFKVLGNTDRHSIKKNLIGALLFDSGETAEATRLK
RTARRRYTRRKNRICYLQEIFSNEMAKVDDSFFHRLEESFLVEEDKKHERHPIFGNIVDEVAYHEKY
PTIYHLRKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYDEHHQDLTLL
KALVRQQLPEKYKEIFFDQSKNGYAGYIDGGASQEEFYKFIKPILEKMDGTEELLVKLNREDLLRK
QRTFDNGSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYVTEGMR
KPAFLSGEQKKAIVDLLFKTNRKVTVKQLKEDYFKKIECFDSVEISGVEDRFNASLGTYHDLLKIIK
DKDFLDNEENEDILEDIVLTLTLFEDREMIEERLKTYAHLFDDKVMKQLKRRRYTGWGRLSRKLIN
GIRDKQSGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQILKEHPV
ENTQLQNEKLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVETRQITKHVAQ
ILDSRMNTKYDENDKLIREVKVITLKSKLVSDFRKDFQFYKVREINNYHHAHDAYLNAVVGTALIKK
YPKLESEFVYGDYKVYDVRKMIAKSEQSGGSKRTADGSEFEPKKKRKV (SEQ ID NO: 217)
ABE8e-CP1028 monomer editor: NLS, linker, TadA*, CP1028 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSEIGKATAKYFFYSNIMNFFKTEITLANGEIRKRPLIETNGETGEIV
WDKGRDFATVRKVLSMPQVNIVKKTEVQTGGFSKESILPKRNSDKLIARKKDWDPKKYGGFDSPT
VAYSVLVVAKVEKGKSKKLKSVKELLGITIMERSSFEKNPIDFLEAKGYKEVKKDLIIKLPKYSLFELE
NGRKRMLASAGELQKGNELALPSKYVNFLYLASHYEKLKGSPEDNEQKQLFVEQHKHYLDEIIEQI
SEFSKRVILADANLDKVLSAYNKHRDKPIREQAENIIHLFTLTNLGAPAAFKYFDTTIDRKRYTSTKEV
LDATLIHQSITGLYETRIDLSQLGGDGGSGGSGGSGGSGGSGGSGGMDKKYSIGLAIGTNSVGWAVI
TDEYKVPSKKFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSARLSKSRRLE
NLIAQLPGEKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHLGELHAILR
RQEDFYPFLKDNREKIEKILTFRIPYYVGPLARGNSRFAWMTRKSEETITPWNFEEVVDKGASAQSFI
ERMTNFDKNLPNEKVLPKHSLLYEYFTVYNELTKVKYVTEGMRKPAFLSGEQKKAIVDLLFKTNRK
VTVKQLKEDYFKKIECFDSVEISGVEDRFNASLGTYHDLLKIIKDKDFLDNEENEDILEDIVLTLTLF
EDREMIEERLKTYAHLFDDKVMKQLKRRRYTGWGRLSRKLINGIRDKQSGKTILDFLKSDGFANRN
FMQLIHDDSLTFKEDIQKAQVSGQGDSLHEHIANLAGSPAIKKGILQTVKVVDELVKVMGRHKPEN
IVIEMARENQTTQKGQKNSRERMKRIEEGIKELGSQILKEHPVENTQLQNEKLYLYYLQNGRDMYV
DQELDINRLSDYDVDHIVPQSFLKDDSIDNKVLTRSDKNRGKSDNVPSEEVVKKMKNYWRQLLNA
KLITQRKFDNLTKAERGGLSELDKAGFIKRQLVETRQITKHVAQILDSRMNTKYDENDKLIREVKVI
TLKSKLVSDFRKDFQFYKVREINNYHHAHDAYLNAVVGTALIKKYPKLESEFVYGDYKVYDVRKMIA
KSEQSGGSKRTADGSEFEPKKKRKV (SEQ ID NO: 216)
*ABE8e-VRQR dimer editor:* NLS, wtTadA, linker, *TadA*, SpCas9-VRQR* 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEYW
MRHALTLAKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLNYPGMNHRV
EITEGILADECAALLCDFYRMPRQVFNAQKKAQSSINSGGSSGGSSGSETPGTSESATPES
SGGSSGGSDKKYSIGLAIGTNSVGWAVITDEYKVPSKKFKVLGNTDRHSIKKNLIGALLFDS
GETAEATRLKRTARRRYTRRKNRICYLQEIFSNEMAKVDDSFFHRLEESFLVEEDKKHERH
PIFGNIVDEVAYHEKYPTIYHLRKKLVDSTDKADLRLIYLALAHMIKFRGHFLIEGDLNPDN
 
SDVDKLFIQLVQTYNQLFEENPINASGVDAKAILSARLSKSRRLENLIAQLPGEKKNGLFGN
LIALSLGLTPNFKSNFDLAEDAKLQLSKDTYDDDLDNLLAQIGDQYADLFLAAKNLSDAIL
LSDILRVNTEITKAPLSASMIKRYDEHHQDLTLLKALVRQQLPEKYKEIFFDQSKNGYAGYI
DGGASQEEFYKFIKPILEKMDGTEELLVKLNREDLLRKQRTFDNGSIPHQIHLGELHAILR
RQEDFYPFLKDNREKIEKILTFRIPYYVGPLARGNSRFAWMTRKSEETITPWNFEEVVDKG
ASAQSFIERMTNFDKNLPNEKVLPKHSLLYEYFTVYNELTKVKYVTEGMRKPAFLSGEQKK
AIVDLLFKTNRKVTVKQLKEDYFKKIECFDSVEISGVEDRFNASLGTYHDLLKIIKDKDFLD
NEENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLAG
SPAIKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDHIVPQSFLKDD
SIDNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEIGKA
TAKYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFVSPTVAYSVLVVAKVEKGKS
KKLKSVKELLGITIMERSSFEKNPIDFLEAKGYKEVKKDLIIKLPKYSLFELENGRKRMLAS
ARELQKGNELALPSKYVNFLYLASHYEKLKGSPEDNEQKQLFVEQHKHYLDEIIEQISEFS
KRVILADANLDKVLSAYNKHRDKPIREQAENIIHLFTLTNLGAPAAFKYFDTTIDRKQYRST
KEVLDATLIHQSITGLYETRIDLSQLGGDSGGSKRTADGSEFEPKKKRKV (SEQ ID NO:
487)
ABE8e-VRQR monomer editor: NLS, linker, TadA*, SpCas9-VROR 
MKRTADGSEFESPKKKRKVSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSINSGGSSGGSSGSETPGTSESATPESSGGSSGGSDKKYSIGL
AIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVD
EVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNL
SDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSE
 
ETITPWNFEEVVDKGASAQSFIERMTNFDKNLPNEKVLPKHSLLYEYFTVYNELTKV
KYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLY
YLQNGRDMYVDQELDINRLSDYDVDHIVPQSFLKDDSIDNKVLTRSDKNRGKSDNV
PSEEVVKKMKNYWRQLLNAKLITQRKFDNLTKAERGGLSELDKAGFIKRQLVETRQ
ITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFY
SNIMNFFKTEITLANGEIRKRPLIETNGETGEIVWDKGRDFATVRKVLSMPQVNIVKK
TEVQTGGFSKESILPKRNSDKLIARKKDWDPKKYGGFVSPTVAYSVLVVAKVEKGK
SKKLKSVKELLGITIMERSSFEKNPIDFLEAKGYKEVKKDLIIKLPKYSLFELENGRKR
MLASARELQKGNELALPSKYVNFLYLASHYEKLKGSPEDNEQKQLFVEQHKHYLDE
IIEQISEFSKRVILADANLDKVLSAYNKHRDKPIREQAENIIHLFTLTNLGAPAAFKYF
DTTIDRKQYRSTKEVLDATLIHQSITGLYETRIDLSQLGGDSGGSKRTADGSEFEPKK
KRKV (SEQ ID NO: 488)
ABE8e-NG-CP1041 dimer editor: NLS, **wtTadA,** linker, TadA*, SpCas9-NG-CP1041 
MKRTADGSEFESPKKKRKVSEVEFSHEYWMRHALTLAKRAWDEREVPVGAVLVHNNRVI
GEGWNRPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFRMRRQEIK
AQKKAQSSTDSGGSSGGSSGSETPGTSESATPESSGGSSGGSSEVEFSHEYWMRHALTLAKRAR
DEREVPVGAVLVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALLCDFYRMPRQ
VFNAQKKAQSSINSGGSSGGSSGSETPGTSESATPESSGGSSGGSNIMNFFKTEITLANGEIRKRPL
IETNGETGEIVWDKGRDFATVRKVLSMPQVNIVKKTEVQTGGFSKESIRPKRNSDKLIARKKDWDP
KKYGGFVSPTVAYSVLVVAKVEKGKSKKLKSVKELLGITIMERSSFEKNPIDFLEAKGYKEVKKDLII
KLPKYSLFELENGRKRMLASARFLQKGNELALPSKYVNFLYLASHYEKLKGSPEDNEQKQLFVEQH
KHYLDEIIEQISEFSKRVILADANLDKVLSAYNKHRDKPIREQAENIIHLFTLTNLGAPRAFKYFDTTI
DRKVYRSTKEVLDATLIHQSITGLYETRIDLSQLGGDGGSGGSGGSGGSGGSGGSGGDKKYSIGLAI
GTNSVGWAVITDEYKVPSKKFKVLGNTDRHSIKKNLIGALLFDSGETAEATRLKRTARRRYTRRKNRI
CYLQEIFSNEMAKVDDSFFHRLEESFLVEEDKKHERHPIFGNIVDEVAYHEKYPTIYHLRKKLVDST
DKADLRLIYLALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTYDDDLDNLLA
 
QIGDQYADLFLAAKNLSDAILLSDILRVNTEITKAPLSASMIKRYDEHHQDLTLLKALVRQQLPEKYK
EIFFDQSKNGYAGYIDGGASQEEFYKFIKPILEKMDGTEELLVKLNREDLLRKQRTFDNGSIPHQIH
LGELHAILRRQEDFYPFLKDNREKIEKILTFRIPYYVGPLARGNSRFAWMTRKSEETITPWNFEEVVD
KGASAQSFIERMTNFDKNLPNEKVLPKHSLLYEYFTVYNELTKVKYVTEGMRKPAFLSGEQKKAIV
DLLFKTNRKVTVKQLKEDYFKKIECFDSVEISGVEDRFNASLGTYHDLLKIIKDKDFLDNEENEDIL
EDIVLTLTLFEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVKVVDELVK
VMGRHKPENIVIEMARENQTTQKGQKNSRERMKRIEEGIKELGSQILKEHPVENTQLQNEKLYLYY
LQNGRDMYVDQELDINRLSDYDVDHIVPQSFLKDDSIDNKVLTRSDKNRGKSDNVPSEEVVKKMK
NYWRQLLNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLESEFVYGDY
KVYDVRKMIAKSEQEIGKATAKYFFYSSGGSKRTADGSEFEPKKKRKV (SEQ ID NO: 478)
ABE8e-NG-CP1041 monomer editor: NLS, linker, TadA*, SpCas9-NG-CP1041 
MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSNIMNFFKTEITLANGEIRKRPLIETNGETGEIVWDKGRDFATVRK
VLSMPQVNIVKKTEVQTGGFSKESIRPKRNSDKLIARKKDWDPKKYGGFVSPTVAYSVLVVAKVEK
GKSKKLKSVKELLGITIMERSSFEKNPIDFLEAKGYKEVKKDLIIKLPKYSLFELENGRKRMLASARF
LQKGNELALPSKYVNFLYLASHYEKLKGSPEDNEQKQLFVEQHKHYLDEIIEQISEFSKRVILADAN
LDKVLSAYNKHRDKPIREQAENIIHLFTLTNLGAPRAFKYFDTTIDRKVYRSTKEVLDATLIHQSITGL
YETRIDLSQLGGDGGSGGSGGSGGSGGSGGSGGDKKYSIGLAIGTNSVGWAVITDEYKVPSKKFKV
LGNTDRHSIKKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHMIKFRGHFLI
EGDLNPDNSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNLSDAILLSD
ILRVNTEITKAPLSASMIKRYDEHHQDLTLLKALVRQQLPEKYKEIFFDQSKNGYAGYIDGGASQEE
FYKFIKPILEKMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKEDYFKKI
ECFDSVEISGVEDRFNASLGTYHDLLKIIKDKDFLDNEENEDILEDIVLTLTLFEDREMIEERLKTYA
HLFDDKVMKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDINRLSDYD
VDHIVPQSFLKDDSIDNKVLTRSDKNRGKSDNVPSEEVVKKMKNYWRQLLNAKLITQRKFDNLTKA
ERGGLSELDKAGFIKRQLVETRQITKHVAQILDSRMNTKYDENDKLIREVKVITLKSKLVSDFRKDF
 
QFYKVREINNYHHAHDAYLNAVVGTALIKKYPKLESEFVYGDYKVYDVRKMIAKSEQEIGKATAKYF
FYSSGGSKRTADGSEFEPKKKRKV (SEQ ID NO: 479)
*ABE8e-iSpyMac dimer editor:* NLS, wtTadA, linker, *TadA*, SpCas9-iSpyMac* 
MKRTADGSEFESPKKKRKVSEVEFSHEYWMRHALTLAKRAWDEREVPVGAVLVHN
NRVIGEGWNRPIGRHDPTAHAEIMALRQGGLVMQNYRLIDATLYVTLEPCVMCAGA
MIHSRIGRVVFGARDAKTGAAGSLMDVLHHPGMNHRVEITEGILADECAALLSDFFR
MRRQEIKAQKKAQSSTDSGGSSGGSSGSETPGTSESATPESSGGSSGGSSEVEFSHEYW
MRHALTLAKRARDEREVPVGAVLVLNNRVIGEGWNRAIGLHDPTAHAEIMALRQGGLVM
QNYRLIDATLYVTFEPCVMCAGAMIHSRIGRVVFGVRNSKRGAAGSLMNVLNYPGMNHRV
EITEGILADECAALLCDFYRMPRQVFNAQKKAQSSINSGGSSGGSSGSETPGTSESATPES
SGGSSGGSDKKYSIGLDIGTNSVGWAVITDEYKVPSKKFKVLGNTDRHSIKKNLIGALLFD
SGETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLNPD
NSDVDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLSDAI
LLSDILRVNTEITKAPLSASMIKRYDEHHQDLTLLKALVRQQLPEKYKEIFFDQSKNGYAGY
IDGGASQEEFYKFIKPILEKMDGTEELLVKLNREDLLRKQRTFDNGSIPHQIHLGELHAIL
RRQEDFYPFLKDNREKIEKILTFRIPYYVGPLARGNSRFAWMTRKSEETITPWNFEEVVDK
GASAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKDKDF
LDNEENEDILEDIVLTLTLFEDREMIEERLKTYAHLFDDKVMKQLKRRRYTGWGRLSRKLI
NGIRDKQSGKTILDFLKSDGFANRNFMQLIHDDSLTFKEDIQKAQVSGQGDSLHEHIANL
AGSPAIKKGILQTVKVVDELVKVMGRHKPENIVIEMARENQTTQKGQKNSRERMKRIEEGI
KELGSQILKEHPVENTQLQNEKLYLYYLQNGRDMYVDQELDINRLSDYDVDHIVPQSFLK
DDSIDNKVLTRSDKNRGKSDNVPSEEVVKKMKNYWRQLLNAKLITQRKFDNLTKAERGG
LSELDKAGFIKRQLVETRQITKHVAQILDSRMNTKYDENDKLIREVKVITLKSKLVSDFRKD
FQFYKVREINNYHHAHDAYLNAVVGTALIKKYPKLESEFVYGDYKVYDVRKMIAKSEQEIG
KATAKYFFYSNIMNFFKTEITLANGEIRKRPLIETNGETGEIVWDKGRDFATVRKVLSMPQ
VNIVKKTESGGSKRTADGSEFEPKKKRKV (SEQ ID NO: 489)
*ABE8e-iSpyMac monomer editor:* NLS, linker, *TadA*, SpCas9-iSpyMac*

MKRTADGSEFESPKKKRKVSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSINSGGSSGGSSG
SETPGTSESATPESSGGSSGGSDKKYSIGLDIGTNSVGWAVITDEYKVPSKKFKVLGNTDRHSIKKN

LIGALLFDSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNPDNSDV
DKLFIQLVQTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECFDSVEISGVE
DRFNASLGTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKNSRERMKR
IEEGIKELGSQILKEHPVENTQLQNEKLYLYYLQNGRDMYVDQELDINRLSDYDVDHIVPQSFLKD
DSIDNKVLTRSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFYKVREINNYH
HAHDAYLNAVVGTALIKKYPKLESEFVYGDYKVYDVRKMIAKSEQEIGKATAKYFFYSNIMNFFKTEI
TLANGEIRKRPLIETNGETGEIVWDKGRDFATVRKVLSMPQVNIVKKTESGGSKRTADGSEFEPKK
KRKV (SEQ ID NO: 490)

### Exemplary CBEs

In various embodiments, the present disclosure provides novel cytidine base editors (CBEs) comprising a napDNAbp domain and a cytidine deaminase domain that enzymatically deaminates a cytosine nucleobase of a C:G nucleobase pair to a uracil. The uracil may be subsequently converted to a thymine (T) by the cell's DNA repair and replication machinery. The mismatched guanine (G) on the opposite strand may subsequently be converted to an adenine (A) by the cell's DNA repair and replication machinery. In this manner, a target C:G nucleobase pair is ultimately converted to a T:A nucleobase pair.

The disclosed novel cytidine base editors exhibit increased on-target editing scope while maintaining minimized off-target DNA editing relative to existing CBEs. The CBEs described herein provide ~10- to ~100-fold lower average Cas9-independent off-target DNA editing, while maintaining efficient on-target editing at most positions targetable by existing CBEs. The disclosed CBEs comprise combinations of mutant cytidine deaminases, such as the YE1, YE2, YEE, and R33A deaminases, and Cas9 domains, and/or novel combinations of mutant cytidine deaminases, Cas9 domains, uracil glycosylase inhibitor (UGI) domains and nuclear localizations sequence (NLS) domains, relative to existing base editors. Existing base editors include BE3, which comprises the structure NH₂-[NLS]-[rAPOBEC1 deaminase]-[Cas9 nickase (D10A)]-[UGI domain]-[NLS]-COOH; BE4, which comprises the structure NH₂-[NLS]-[rAPOBEC1 deaminase]-[Cas9 nickase (D10A)]-[UGI domain]-[UGI domain]-[NLS]-COOH; and BE4max, which is a version of BE4 for which the codons of the base editor-encoding construct has been codon-optimized for expression in human cells.

Zuo *et al.* recently reported that, when overexpressed in mouse embryos and rice, BE3, the original CBE, induces an average random C:G-to-T:A mutation frequency of 5x10⁻⁸ per bp and 1.7x10⁻⁷ per bp, respectively. See "Cytidine base editor generates substantial off-target single-nucleotide variants in mouse embryos." Science 364, 289-292 (2019), herein incorporated by reference. Editing was observed in sequences that had little to no similarity to the target sequences. These off-target edits may have arisen from the intrinsic DNA affinity of BE3's deaminase domain, independent of the guide RNA-programmed DNA binding of Cas9. See also Jin et al., Cytosine, but not adenine, base editors induce genome-wide off-target mutations in rice. Science 364, (2019), herein incorporated by reference.

Zuo *et al.* also found that Cas9-independent off-target editing events were enriched in transcribed regions of the genome, particularly in highly-expressed genes. Some of these were tumor suppressor genes. Accordingly, there is a need in the art to develop base editors that possess low off-target editing frequencies that may avoid undesired activation or inactivation of genes associated with diseases or disorders, such as cancer, and assays that rapidly measure the off-target editing frequencies of these base editors.

Exemplary CBEs may provide an off-target editing frequency of less than 2.0% after being contacted with a nucleic acid molecule comprising a target sequence, e.g., a target nucleobase pair. Further exemplary CBEs provide an off-target editing frequency of less than 1.5% after being contacted with a nucleic acid molecule comprising a target sequence comprising a target nucleobase pair. Further exemplary CBEs may provide an off-target editing frequency of less than 1.25%, less than 1.1%, less than 1%, less than 0.75%, less than 0.5%, less than 0.4%, less than 0.25%, less than 0.2%, less than 0.15%, less than 0.1%, less than 0.05%, or less than 0.025%, after being contacted with a nucleic acid molecule comprising a target sequence.

For instance, the cytidine base editors YE1-BE4, YE1-CP1028, YE1-SpCas9-NG (also referred to herein as YE1-NG), R33A-BE4, and R33A+K34A-BE4-CP1028, which are described below, may exhibit off-target editing frequencies of less than 0.75% (e.g., about 0.4% or less) while maintaining on-target editing efficiencies of about 60% or more, in target sequences in mammalian cells. Each of these base editors comprises modified cytidine deaminases (e.g., YE1, R33A, or R33A+K34A) and may further comprise a modified napDNAbp domain such as a circularly permuted Cas9 domain (e.g., CP1028) or a Cas9 domain with an expanded PAM window (e.g., SpCas9-NG). These five base editors may be the most preferred for applications in which off-target editing, and in particular Cas9-independent off-target editing, must be minimized. In particular, base editors comprising a YE1 deaminase domain provide efficient on-target editing with greatly decreased Cas9-independent editing, as confirmed by whole-genome sequencing.

Exemplary CBEs may further possess an on-target editing efficiency of more than 50% after being contacted with a nucleic acid molecule comprising a target sequence. Further exemplary CBEs possess an on-target editing efficiency of more than 60% after being contacted with a nucleic acid molecule comprising a target sequence. Further exemplary CBEs possess an on-target editing efficiency of more than 65%, more than 70%, more than 75%, more than 80%, more than 82.5%, or more than 85% after being contacted with a nucleic acid molecule comprising a target sequence.

The disclosed CBEs may exhibit indel frequencies of less than 0.75%, less than 0.6%, less than 0.5%, less than 0.4%, less than 0.3%, or less than 0.2% after being contacted with a nucleic acid molecule containing a target sequence. The disclosed CBEs may further exhibit reduced RNA off-target editing relative to existing CBEs. The disclosed CBEs may further result in increased product purity after being contacted with a nucleic acid molecule containing a target sequence relative to existing CBEs.

The disclosed CBEs may further comprise one or more nuclear localization signals (NLSs) and/or two or more uracil glycosylase inhibitor (UGI) domains. Thus, the base editors may comprise the structure: NH₂-[first nuclear localization sequence]-[cytidine deaminase domain]-[napDNAbp domain]-[first UGI domain]-[second UGI domain]-[second nuclear localization sequence]-COOH, wherein each instance of "]-[" indicates the presence of an optional linker sequence. Exemplary CBEs may have a structure that comprises the "BE4max" architecture, with an NH₂-[NLS]-[cytidine deaminase]-[Cas9 nickase]-[UGI domain]-[UGI domain]-[NLS]-COOH structure, having optimized nuclear localization signals and wherein the napDNAbp domain comprises a Cas9 nickase. This BE4max structure was reported to have optimized codon usage for expression in human cells, as reported in Koblan et al., Nat Biotechnol. 2018;36(9):843-846, herein incorporated by reference.

In other embodiments, exemplary CBEs may have a structure that comprises a modified BE4max architecture that contains a napDNAbp domain comprising a Cas9 variant other than Cas9 nickase, such as SpCas9-NG, xCas9, or circular permutant CP1028. Accordingly, exemplary CBEs may comprise the structure: NH₂-[NLS]-[cytidine deaminase]-[CP1028]-[UGI domain]-[UGI domain]-[NLS]-COOH; NH₂-[NLS]-[cytidine deaminase]-[xCas9]-[UGI domain]-[UGI domain]-[NLS]-COOH; or NH₂-[NLS]-[cytidine deaminase]-[SpCas9-NG]-[UGI domain]-[UGI domain]-[NLS]-COOH, , wherein each instance of "]-[" indicates the presence of an optional linker sequence.

The disclosed CBEs may comprise modified (or evolved) cytidine deaminase domains, such as deaminase domains that recognize an expanded PAM sequence, have improved efficiency of deaminating 5'-GC targets, and/or make edits in a narrower target window, In some embodiments, the disclosed cytidine base editors comprise evolved nucleic acid programmable DNA binding proteins (napDNAbp), such as an evolved Cas9.

Exemplary cytidine base editors comprise sequences that are at least least 85%, at least 90%, at least 95%, at least 98%, at least 99%, or at least 99.5% identical to the following amino acid sequences, SEQ ID NOs: 223-248.

Where indicated, "-BE4" refers to the BE4max architecture, or NH₂-[first nuclear localization sequence]-[cytidine deaminase domain]-[32aa linker]-[SpCas9 nickase (nCas9, or nSpCas9) domain]-[9aa linker]-[first UGI domain]-[9aa-linker]-[second UGI domain]-[second nuclear localization sequence]-COOH. Where indicated, "BE4max, modified with SpCas9-NG" and "-SpCas9-NG" refer to a modified BE4max architecture in which the SpCas9 nickase domain has been replaced with an SpCas9-NG, i.e., NH₂-[first nuclear localization sequence]-[cytidine deaminase domain]-[32aa linker]-[SpCas9-NG]-[9aa linker]-[first UGI domain]-[9aa-linker]-[second UGI domain]-[second nuclear localization sequence]-COOH. And where indicated, "BE4-CP1028" refers to a modified BE4max architecture in which the Cas9 nickase domain has been replaced with a *S. pyogenes* CP1028, i.e., NH₂-[first nuclear localization sequence]-[cytidine deaminase domain]-[32aa linker]-[CP1028]-[9aa linker]-[first UGI domain]-[9aa-linker]-[second UGI domain]-[second nuclear localization sequence]-COOH.

As discussed above, preferred base editors comprise modified cytidine deaminases (e.g., YE1, R33A, or R33A+K34A) and may further comprise a modified napDNAbp domain such as a circularly permuted Cas9 domain (e.g., CP1028) or a Cas9 domain with an expanded PAM window (e.g., SpCas9-NG). The napDNAbp domains in the following amino acid sequences are indicated in *italics.*
**BE4max** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSR
 
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 223)
**YE1-BE4** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLRDLISSGVTIQIMTEQESGYCWRNFV
NYSPSNEAHWPRYPHLWVRLYVLELYCIILGLPPCLNILRRKQPQLTFFTIALQSCHY
 
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 224)
**YE2-BE4** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPRNRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
 
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 225)
**YEE-BE4** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
 
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 226)
**EE-BE4** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
 
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 227)
**R33A-BE4** 
MKRTADGSEFESPKKKRKVSSETGPVAVDPTLRRRIEPHEFEVFFDPRELAKETCLLY
EINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
 
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 228)
**R33A+K34A-BE4** 
MKRTADGSEFESPKKKRKVSSETGPVAVDPTLRRRIEPHEFEVFFDPRELAAETCLLY
EINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
 
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 229)
**APOBEC3A (A3A)-BE4** 
MKRTADGSEFESPKKKRKVSEASPASGPRHLMDPHIFTSNFNNGIGRHKTYLCYEVE
RLDNGTSVKMDQHRGFLHNQAKNLLCGFYGRHAELRFLDLVPSLQLDPAQIYRVT
WFISWSPCFSWGCAGEVRAFLQENTHVRLRIFAARIYDYDPLYKEALQMLRDAGAQ
VSIMTYDEFKHCWDTFVDHQGCPFQPWDGLDEHSQALSGRLRAILQNQGNSGGSSG
GSSGSETPGTSESATPESSGGSSGGSDKKYSIGLAIGTNSVGWAVITDEYKVPSKKFKVLG
NTDRHSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYLALAH
MIKFRGHFLIEGDLNPDNSDVDKLFIQLVQTYNQLFEENPINASGVDAKAILSARLSKSRRL
ENLIAQLPGEKKNGLFGNLIALSLGLTPNFKSNFDLAEDAKLQLSKDTYDDDLDNLLAQIG
DQYADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRTFD
NGSIPHQIHLGELHAILRRQEDFYPFLKDNREKIEKILTFRIPYYVGPLARGNSRFAWMTRK
SEETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKA
 
QVSGQGDSLHEHIANLAGSPAIKKGILQTVKVVDELVKVMGRHKPENIVIEMARENQTTQ
KGQKNSRERMKRIEEGIKELGSQILKEHPVENTQLQNEKLYLYYLQNGRDMYVDQELDIN
RLSDYDVDHIVPQSFLKDDSIDNKVLTRSDKNRGKSDNVPSEEVVKKMKNYWRQLLNAKL
ITQRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEIVWD
KGRDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLIIKL
PKYSLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDSGGSGGSGGSTNLS
DIIEKETGKQLVIQESILMLPEEVEEVIGNKPESDILVHTAYDESTDENVMLLTSDAPE
YKPWALVIQDSNGENKIKMLSGGSGGSGGSTNLSDIIEKETGKQLVIQESILMLPEEV
EEVIGNKPESDILVHTAYDESTDENVMLLTSDAPEYKPWALVIQDSNGENKIKMLSG
GSKRTADGSEFEPKKKRKV (SEQ ID NO: 230)
**APOBEC3B (A3B)-BE4** 
MKRTADGSEFESPKKKRKVNPQIRNPMERMYRDTFYDNFENEPILYGRSYTWLCYE
VKIKRGRSNLLWDTGVFRGQVYFKPQYHAEMCFLSWFCGNQLPAYKCFQITWFVS
WTPCPDCVAKLAEFLSEHPNVTLTISAARLYYYWERDYRRALCRLSQAGARVTIMD
YEEFAYCWENFVYNEGQQFMPWYKFDENYAFLHRTLKEILRYLMDPDTFTFNFNND
PLVLRRRQTYLCYEVERLDNGTWVLMDQHMGFLCNEAKNLLCGFYGRHAELRFLD
LVPSLQLDPAQIYRVTWFISWSPCFSWGCAGEVRAFLQENTHVRLRIFAARIYDYDPL
YKEALQMLRDAGAQVSIMTYDEFEYCWDTFVYRQGCPFQPWDGLEEHSQALSGRL
RAILQNQGNSGGSSGGSSGSETPGTSESATPESSGGSSGGSDKKYSIGLAIGTNSVGWAV
ITDEYKVPSKKFKVLGNTDRHSIKKNLIGALLFDSGETAEATRLKRTARRRYTRRKNRICYL
QEIFSNEMAKVDDSFFHRLEESFLVEEDKKHERHPIFGNIVDEVAYHEKYPTIYHLRKKLV
DSTDKADLRLIYLALAHMIKFRGHFLIEGDLNPDNSDVDKLFIQLVQTYNQLFEENPINAS
GVDAKAILSARLSKSRRLENLIAQLPGEKKNGLFGNLIALSLGLTPNFKSNFDLAEDAKLQL
SKDTYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTEEL
LVKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLPKH
SLLYEYFTVYNELTKVKYVTEGMRKPAFLSGEQKKAIVDLLFKTNRKVTVKQLKEDYFKKI
ECFDSVEISGVEDRFNASLGTYHDLLKIIKDKDFLDNEENEDILEDIVLTLTLFEDREMIEE
 
RLKTYAHLFDDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELVKVMGRH
KPENIVIEMARENQTTQKGQKNSRERMKRIEEGIKELGSQILKEHPVENTQLQNEKLYLYY
LQNGRDMYVDQELDINRLSDYDVDHIVPQSFLKDDSIDNKVLTRSDKNRGKSDNVPSEEV
VKKMKNYWRQLLNAKLITQRKFDNLTKAERGGLSELDKAGFIKRQLVETRQITKHVAQIL
DSRMNTKYDENDKLIREVKVITLKSKLVSDFRKDFQFYKVREINNYHHAHDAYLNAVVGT
ALIKKYPKLESEFVYGDYKVYDVRKMIAKSEQEIGKATAKYFFYSNIMNFFKTEITLANGEI
RKRPLIETNGETGEIVWDKGRDFATVRKVLSMPQVNIVKKTEVQTGGFSKESILPKRNSDK
LIARKKDWDPKKYGGFDSPTVAYSVLVVAKVEKGKSKKLKSVKELLGITIMERSSFEKNPI
DFLEAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRDKP
IREQAENIIHLFTLTNLGAPAAFKYFDTTIDRKRYTSTKEVLDATLIHQSITGLYETRIDLSQL
GGDSGGSGGSGGSTNLSDIIEKETGKQLVIQESILMLPEEVEEVIGNKPESDILVHTAY
DESTDENVMLLTSDAPEYKPWALVIQDSNGENKIKMLSGGSGGSGGSTNLSDIIEKET
GKQLVIQESILMLPEEVEEVIGNKPESDILVHTAYDESTDENVMLLTSDAPEYKPWAL
VIQDSNGENKIKMLSGGSKRTADGSEFEPKKKRKV (SEQ ID NO: 231)
**APOBEC3G (A3G)-BE4** 
MKRTADGSEFESPKKKRKVKPHFRNTVERMYRDTFSYNFYNRPILSRRNTVWLCYE
VKTKGPSRPPLDAKIFRGQVYSELKYHPEMRFFHWFSKWRKLHRDQEYEVTWYISW
SPCTKCTRDMATFLAEDPKVTLTIFVARLYYFWDPDYQEALRSLCQKRDGPRATMKI
MNYDEFQHCWSKFVYSQRELFEPWNNLPKYYILLHIMLGEILRHSMDPPTFTFNFNN
EPWVRGRHETYLCYEVERMHNDTWVLLNQRRGFLCNQAPHKHGFLEGRHAELCFL
DVIPFWKLDLDQDYRVTCFTSWSPCFSCAQEMAKFISKNKHVSLCIFTARIYDDQGR
CQEGLRTLAEAGAKISIMTYSEFKHCWDTFVDHQGCPFQPWDGLDEHSQDLSGRLR
AILQNQENSGGSSGGSSGSETPGTSESATPESSGGSSGGSDKKYSIGLAIGTNSVGWAVIT
DEYKVPSKKFKVLGNTDRHSIKKNLIGALLFDSGETAEATRLKRTARRRYTRRKNRICYLQE
IFSNEMAKVDDSFFHRLEESFLVEEDKKHERHPIFGNIVDEVAYHEKYPTIYHLRKKLVDS
TDKADLRLIYLALAHMIKFRGHFLIEGDLNPDNSDVDKLFIQLVQTYNQLFEENPINASGV
DAKAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDEHHQ
DLTLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYVG
PLARGNSRFAWMTRKSEETITPWNFEEVVDKGASAQSFIERMTNFDKNLPNEKVLPKHSL
LYEYFTVYNELTKVKYVTEGMRKPAFLSGEQKKAIVDLLFKTNRKVTVKQLKEDYFKKIEC
 
FDSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNFMQ
LIHDDSLTFKEDIQKAQVSGQGDSLHEHIANLAGSPAIKKGILQTVKVVDELVKVMGRHK
PENIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEEVV
KKMKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVVGTAL
IKKYPKLESEFVYGDYKVYDVRKMIAKSEQEIGKATAKYFFYSNIMNFFKTEITLANGEIRK
RPLIETNGETGEIVWDKGRDFATVRKVLSMPQVNIVKKTEVQTGGFSKESILPKRNSDKLI
ARKKDWDPKKYGGFDSPTVAYSVLVVAKVEKGKSKKLKSVKELLGITIMERSSFEKNPIDF
LEAKGYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQLG
GDSGGSGGSGGSTNLSDIIEKETGKQLVIQESILMLPEEVEEVIGNKPESDILVHTAYD
ESTDENVMLLTSDAPEYKPWALVIQDSNGENKIKMLSGGSGGSGGSTNLSDIIEKETG
KQLVIQESILMLPEEVEEVIGNKPESDILVHTAYDESTDENVMLLTSDAPEYKPWALV
IQDSNGENKIKMLSGGSKRTADGSEFEPKKKRKV (SEQ ID NO: 232)
**AID-BE4** 
MKRTADGSEFESPKKKRKVDSLLMNRRKFLYQFKNVRWAKGRRETYLCYVVKRRD
SATSFSLDFGYLRNKNGCHVELLFLRYISDWDLDPGRCYRVTWFTSWSPCYDCARH
VADFLRGNPNLSLRIFTARLYFCEDRKAEPEGLRRLHRAGVQIAIMTFKDYFYCWNT
FVENHERTFKAWEGLHENSVRLSRQLRRILLPLYEVDDLRDAFRTLGLSGGSSGGSS
GSETPGTSESATPESSGGSSGGSDKKYSIGLAIGTNSVGWAVITDEYKVPSKKFKVLGNTD
RHSIKKNLIGALLFDSGETAEATRLKRTARRRYTRRKNRICYLQEIFSNEMAKVDDSFFHRL
EESFLVEEDKKHERHPIFGNIVDEVAYHEKYPTIYHLRKKLVDSTDKADLRLIYLALAHMIK
FRGHFLIEGDLNPDNSDVDKLFIQLVQTYNQLFEENPINASGVDAKAILSARLSKSRRLENL
IAQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYV
TEGMRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQLK
RRRYTGWGRLSRKLINGIRDKQSGKTILDFLKSDGFANRNFMQLIHDDSLTFKEDIQKAQ
 
VSGQGDSLHEHIANLAGSPAIKKGILQTVKVVDELVKVMGRHKPENIVIEMARENQTTQK
GQKNSRERMKRIEEGIKELGSQILKEHPVENTQLQNEKLYLYYLQNGRDMYVDQELDINR
LSDYDVDHIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIREV
KVITLKSKLVSDFRKDFQFYKVREINNYHHAHDAYLNAVVGTALIKKYPKLESEFVYGDYK
VYDVRKMIAKSEQEIGKATAKYFFYSNIMNFFKTEITLANGEIRKRPLIETNGETGEIVWDK
GRDFATVRKVLSMPQVNIVKKTEVQTGGFSKESILPKRNSDKLIARKKDWDPKKYGGFDS
PTVAYSVLVVAKVEKGKSKKLKSVKELLGITIMERSSFEKNPIDFLEAKGYKEVKKDLIIKLP
KYSLFELENGRKRMLASAGELQKGNELALPSKYVNFLYLASHYEKLKGSPEDNEQKQLFV
EQHKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSGGSGGSTNLSDIIEKETGKQLVIQESILMLPEEVEEV
IGNKPESDILVHTAYDESTDENVMLLTSDAPEYKPWALVIQDSNGENKIKMLSGGSK
RTADGSEFEPKKKRKV (SEQ ID NO: 233)
**CDA-BE4** 
MKRTADGSEFESPKKKRKVTDAEYVRIHEKLDIYTFKKQFFNNKKSVSHRCYVLFEL
KRRGERRACFWGYAVNKPQSGTERGIHAEIFSIRKVEEYLRDNPGQFTINWYSSWSP
CADCAEKILEWYNQELRGNGHTLKIWACKLYYEKNARNQIGLWNLRDNGVGLNV
MVSEHYQCCRKIFIQSSHNQLNENRWLEKTLKRAEKRRSELSIMIQVKILHTTKSPAV
SGGSSGGSSGSETPGTSESATPESSGGSSGGSDKKYSIGLAIGTNSVGWAVITDEYKVPSK
KFKVLGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKADLRL
IYLALAHMIKFRGHFLIEGDLNPDNSDVDKLFIQLVQTYNQLFEENPINASGVDAKAILSAR
LSKSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLKAL
VRQQLPEKYKEIFFDQSKNGYAGYIDGGASQEEFYKFIKPILEKMDGTEELLVKLNREDLL
RKQRTFDNGSIPHQIHLGELHAILRRQEDFYPFLKDNREKIEKILTFRIPYYVGPLARGNSR
FAWMTRKSEETITPWNFEEVVDKGASAQSFIERMTNFDKNLPNEKVLPKHSLLYEYFTVY
NELTKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHLFD
DKVMKQLKRRRYTGWGRLSRKLINGIRDKQSGKTILDFLKSDGFANRNFMQLIHDDSLTF
KEDIQKAQVSGQGDSLHEHIANLAGSPAIKKGILQTVKVVDELVKVMGRHKPENIVIEMAR
ENQTTQKGQKNSRERMKRIEEGIKELGSQILKEHPVENTQLQNEKLYLYYLQNGRDMYVD
 
QELDINRLSDYDVDHIVPQSFLKDDSIDNKVLTRSDKNRGKSDNVPSEEVVKKMKNYWRQ
LLNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLESEF
VYGDYKVYDVRKMIAKSEQEIGKATAKYFFYSNIMNFFKTEITLANGEIRKRPLIETNGETG
EIVWDKGRDFATVRKVLSMPQVNIVKKTEVQTGGFSKESILPKRNSDKLIARKKDWDPKK
YGGFDSPTVAYSVLVVAKVEKGKSKKLKSVKELLGITIMERSSFEKNPIDFLEAKGYKEVKK
DLIIKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLFTLT
NLGAPAAFKYFDTTIDRKRYTSTKEVLDATLIHQSITGLYETRIDLSQLGGDSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 234)
**FERNY-BE4** 
MKRTADGSEFESPKKKRKVFERNYDPRELRKETYLLYEIKWGKSGKLWRHWCQNN
RTQHAEVYFLENIFNARRFNPSTHCSITWYLSWSPCAECSQKIVDFLKEHPNVNLEIY
VARLYYHEDERNRQGLRDLVNSGVTIRIMDLPDYNYCWKTFVSDQGGDEDYWPGH
FAPWIKQYSLKLSGGSSGGSSGSETPGTSESATPESSGGSSGGSDKKYSIGLAIGTNSVG
WAVITDEYKVPSKKFKVLGNTDRHSIKKNLIGALLFDSGETAEATRLKRTARRRYTRRKNRI
CYLQEIFSNEMAKVDDSFFHRLEESFLVEEDKKHERHPIFGNIVDEVAYHEKYPTIYHLRK
KLVDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAEDAK
LQLSKDTYDDDLDNLLAQIGDQYADLFLAAKNLSDAILLSDILRVNTEITKAPLSASMIKRY
DEHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNEKVL
PKHSLLYEYFTVYNELTKVKYVTEGMRKPAFLSGEQKKAIVDLLFKTNRKVTVKQLKEDYF
KKIECFDSVEISGVEDRFNASLGTYHDLLKIIKDKDFLDNEENEDILEDIVLTLTLFEDREM
IEERLKTYAHLFDDKVMKQLKRRRYTGWGRLSRKLINGIRDKQSGKTILDFLKSDGFANR
NFMQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNEKL
YLYYLQNGRDMYVDQELDINRLSDYDVDHIVPQSFLKDDSIDNKVLTRSDKNRGKSDNVP
SEEVVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYLNAV
 
VGTALIKKYPKLESEFVYGDYKVYDVRKMIAKSEQEIGKATAKYFFYSNIMNFFKTEITLAN
GEIRKRPLIETNGETGEIVWDKGRDFATVRKVLSMPQVNIVKKTEVQTGGFSKESILPKRN
SDKLIARKKDWDPKKYGGFDSPTVAYSVLVVAKVEKGKSKKLKSVKELLGITIMERSSFEK
NPIDFLEAKGYKEVKKDLIIKLPKYSLFELENGRKRMLASAGELQKGNELALPSKYVNFLY
LASHYEKLKGSPEDNEQKQLFVEQHKHYLDEIIEQISEFSKRVILADANLDKVLSAYNKHR
DKPIREQAENIIHLFTLTNLGAPAAFKYFDTTIDRKRYTSTKEVLDATLIHQSITGLYETRIDL
SQLGGDSGGSGGSGGSTNLSDIIEKETGKQLVIQESILMLPEEVEEVIGNKPESDILVHT
AYDESTDENVMLLTSDAPEYKPWALVIQDSNGENKIKMLSGGSGGSGGSTNLSDIIE
KETGKQLVIQESILMLPEEVEEVIGNKPESDILVHTAYDESTDENVMLLTSDAPEYKP
WALVIQDSNGENKIKMLSGGSKRTADGSEFEPKKKRKV (SEQ ID NO: 235)
**Evolved APOBEC3A (eA3A)-BE4** 
MKRTADGSEFESPKKKRKVEASPASGPRHLMDPHIFTSNFNNGIGRHKTYLCYEVER
LDNGTSVKMDQHRGFLHGQAKNLLCGFYGRHAELRFLDLVPSLQLDPAQIYRVTWF
ISWSPCFSWGCAGEVRAFLQENTHVRLRIFAARIYDYDPLYKEALQMLRDAGAQVSI
MTYDEFKHCWDTFVDHQGCPFQPWDGLDEHSQALSGRLRAILQNQGNSGGSSGGSS
GSETPGTSESATPESSGGSSGGSDKKYSIGLAIGTNSVGWAVITDEYKVPSKKFKVLGNTD
RHSIKKNLIGALLFDSGETAEATRLKRTARRRYTRRKNRICYLQEIFSNEMAKVDDSFFHRL
EESFLVEEDKKHERHPIFGNIVDEVAYHEKYPTIYHLRKKLVDSTDKADLRLIYLALAHMIK
FRGHFLIEGDLNPDNSDVDKLFIQLVQTYNQLFEENPINASGVDAKAILSARLSKSRRLENL
IAQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEKY
KEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKYV
TEGMRKPAFLSGEQKKAIVDLLFKTNRKVTVKQLKEDYFKKIECFDSVEISGVEDRFNASL
GTYIIDLLKIIKDKDFLDNEENEDILEDIVLTLTLFEDREMIEERLKTYAIILFDDKVMKQLK
RRRYTGWGRLSRKLINGIRDKQSGKTILDFLKSDGFANRNFMQLIHDDSLTFKEDIQKAQ
VSGQGDSLHEHIANLAGSPAIKKGILQTVKVVDELVKVMGRHKPENIVIEMARENQTTQK
GQKNSRERMKRIEEGIKELGSQILKEHPVENTQLQNEKLYLYYLQNGRDMYVDQELDINR
LSDYDVDHIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIREV
KVITLKSKLVSDFRKDFQFYKVREINNYHHAHDAYLNAVVGTALIKKYPKLESEFVYGDYK
VYDVRKMIAKSEQEIGKATAKYFFYSNIMNFFKTEITLANGEIRKRPLIETNGETGEIVWDK
GRDFATVRKVLSMPQVNIVKKTEVQTGGFSKESILPKRNSDKLIARKKDWDPKKYGGFDS
 
PTVAYSVLVVAKVEKGKSKKLKSVKELLGITIMERSSFEKNPIDFLEAKGYKEVKKDLIIKLP
KYSLFELENGRKRMLASAGELQKGNELALPSKYVNFLYLASHYEKLKGSPEDNEQKQLFV
EQHKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSGGSGGSTNLSDIIEKETGKQLVIQESILMLPEEVEEV
IGNKPESDILVHTAYDESTDENVMLLTSDAPEYKPWALVIQDSNGENKIKMLSGGSK
RTADGSEFEPKKKRKV (SEQ ID NO: 236)
**AALN-BE4** 
MKRTADGSEFESPKKKRKVSSETGPVAVDPTLRRRIEPHEFEVFFDPRELAAETCLLY
EINWGGRHSIWRHTSQNTNKHVEVNFIEKFTTERYFCPNTRCSITWFLSWSPCGECSR
AITEFLSRYPHVTLFIYIARLYHLANPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESILP
KRNSDKLIARKKDWDPKKYGGFDSPTVAYSVLVVAKVEKGKSKKLKSVKELLGITIMERSS
FEKNPIDFLEAKGYKEVKKDLIIKLPKYSLFELENGRKRMLASAGELQKGNELALPSKYVN
 
FLYLASHYEKLKGSPEDNEQKQLFVEQHKHYLDEIIEQISEFSKRVILADANLDKVLSAYNK
HRDKPIREQAENIIHLFTLTNLGAPAAFKYFDTTIDRKRYTSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 237)
**BE4max, modified with SpCas9-NG** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
 
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 238)
**YE1-SpCas9-NG base editor (YE1-NG)** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
 
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 239)
**YE2-SpCas9-NG base editor** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPRNRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 240)
**YEE-SpCas9-NG base editor** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 241)
**EE-SpCas9-NG base editor** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 242)
**R33A+K34A-SpCas9-NG base editor** 
MKRTADGSEFESPKKKRKVSSETGPVAVDPTLRRRIEPHEFEVFFDPRELAAETCLLY
EINWGGRHSIWRHTSQNTNKHVEVNFIEKFTTERYFCPNTRCSITWFLSWSPCGECSR
 
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSDKKYSIGLAIGTNS
VGWAVITDEYKVPSKKFKVLGNTDRHSIKKNLIGALLFDSGETAEATRLKRTARRRYTRRK
NRICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFEEN
PINASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSASMIK
RYDEHHQDLTLLKALVRQQLPEKYKEIFFDQSKNGYAGYIDGGASQEEFYKFIKPILEKM
DGTEELLVKLNREDLLRKQRTFDNGSIPHQIHLGELHAILRRQEDFYPFLKDNREKIEKIL
TFRIPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSD
NVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIRP
KRNSDKLIARKKDWDPKKYGGFVSPTVAYSVLVVAKVEKGKSKKLKSVKELLGITIMERSS
FEKNPIDFLEAKGYKEVKKDLIIKLPKYSLFELENGRKRMLASARFLQKGNELALPSKYVN
FLYLASHYEKLKGSPEDNEQKQLFVEQHKHYLDEIIEQISEFSKRVILADANLDKVLSAYNK
HRDKPIREQAENIIHLFTLTNLGAPRAFKYFDTTIDRKVYRSTKEVLDATLIHQSITGLYETRI
DLSQLGGDSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDI
IEKETGKQLVIQESILMLPEEVEEVIGNKPESDILVHTAYDESTDENVMLLTSDAPEYK
PWALVIQDSNGENKIKMLSGGSKRTADGSEFEPKKKRKV (SEQ ID NO: 243)
**YE1-CP1028 base editor (YE1-BE4-CP1028, or YE1-CP)** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLRDLISSGVTIQIMTEQESGYCWRNFV
NYSPSNEAHWPRYPHLWVRLYVLELYCIILGLPPCLNILRRKQPQLTFFTIALQSCHY
 
QRLPPHILWATGLKSGGSSGGSSGSETPGTSESATPESSGGSSGGSEIGKATAKYFFYS
NIMNFFKTEITLANGEIRKRPLIETNGETGEIVWDKGRDFATVRKVLSMPQVNIVKKT
EVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRM
LASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDT
TIDRKRYTSTKEVLDATLIHQSITGLYETRIDLSQLGGDGGSGGSGGSGGSGGSGGSG
GMDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALSL
GLTPNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 244)
**YE2-CP1028 base editor (YE2-BE4-CP1028)** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPRNRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSEIGKATAKYFFYS
 
NIMNFFKTEITLANGEIRKRPLIETNGETGEIVWDKGRDFATVRKVLSMPQVNIVKKT
EVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRM
LASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDT
TIDRKRYTSTKEVLDATLIHQSITGLYETRIDLSQLGGDGGSGGSGGSGGSGGSGGSG
GMDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALSL
GLTPNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 245)
**YEE-CP1028 base editor (YEE-BE4-CP1028)** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSYSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSEIGKATAKYFFYS
NIMNFFKTEITLANGEIRKRPLIETNGETGEIVWDKGRDFATVRKVLSMPQVNIVKKT
 
EVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRM
LASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDT
TIDRKRYTSTKEVLDATLIHQSITGLYETRIDLSQLGGDGGSGGSGGSGGSGGSGGSG
GMDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALSL
GLTPNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 246)
**EE-CP1028 base editor (EE-BE4-CP1028)** 
MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSR
AITEFLSRYPHVTLFIYIARLYHHADPENRQGLEDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSEIGKATAKYFFYS
NIMNFFKTEITLANGEIRKRPLIETNGETGEIVWDKGRDFATVRKVLSMPQVNIVKKT
EVQTGGFSKESILPKRNSDKLIARKKDWDPKKYGGFDSPTVAYSVLVVAKVEKGKS
 
KKLKSVKELLGITIMERSSFEKNPIDFLEAKGYKEVKKDLIIKLPKYSLFELENGRKRM
LASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDT
TIDRKRYTSTKEVLDATLIHQSITGLYETRIDLSQLGGDGGSGGSGGSGGSGGSGGSG
GMDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALSL
GLTPNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 247)
**R33A+K34A-CP1028 base editor (R33A+K34A-BE4-CP1028)**

MKRTADGSEFESPKKKRKVSSETGPVAVDPTLRRRIEPHEFEVFFDPRELAAETCLLY
EINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFV
NYSPSNEAHWPRYPHLWVRLYVLELYCIILGLPPCLNILRRKQPQLTFFTIALQSCHY
QRLPPHILWATGLKSGGSSGGSSGSETPGTSESATPESSGGSSGGSEIGKATAKYFFYS
NIMNFFKTEITLANGEIRKRPLIETNGETGEIVWDKGRDFATVRKVLSMPQVNIVKKT
EVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRM

LASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDT
TIDRKRYTSTKEVLDATLIHQSITGLYETRIDLSQLGGDGGSGGSGGSGGSGGSGGSG
GMDKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALSL
GLTPNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGGAS
QEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQSF
IERMTNFDKNLPNEKVLPKHSLLYEYFTVYNELTKVKYVTEGMRKPAFLSGEQKKAIVDLL
FKTNRKVTVKQLKEDYFKKIECFDSVEISGVEDRFNASLGTYHDLLKIIKDKDFLDNEENE
DILEDIVLTLTLFEDREMIEERLKTYAHLFDDKVMKQLKRRRYTGWGRLSRKLINGIRDKQ
SGKTILDFLKSDGFANRNFMQLIHDDSLTFKEDIQKAQVSGQGDSLHEHIANLAGSPAIKK
GILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKV
LTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQSGGSGGSGGS
TNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILML
PEEVEEVIGNKPESDILVHTAYDESTDENVMLLTSDAPEYKPWALVIQDSNGENKIK
MLSGGSKRTADGSEFEPKKKRKV (SEQ ID NO: 248)

These disclosed CBEs exhibit low off-target editing frequencies, and in particular low Cas9-independent off-target editing frequencies, while exhibiting high on-target editing efficiencies. For example, the YEl-BE4, YE1-CP1028, YEl-SpCas9-NG, R33A-BE4, and R33A+K34A-BE4-CP1028 base editors may exhibit off-target editing frequencies of less than 0.75% (e.g., about 0.4% or less) while maintaining on-target editing efficiencies of about 60% or more, in target sequences in mammalian cells. (See, e.g., FIGs. 11, 15A, 15B and 17.) The Examples of the present disclosure suggest that CBEs with cytidine deaminases that have a low instrinsic catalytic efficiency (*k_{cat}*/Kₘ) for cytosine-containing ssDNA substrates exhibit reduced Cas9-independent off-target deamination.

In some embodiments, the fusion protein comprises an amino acid sequence that is at least 60%, at least 65%, at least 70%, at least 75%, at least 80%, at least 85%, at least 90%, at least 95%, at least 96%, at least 97%, at least 98%, at least 99%, or at least 99.5% identical to any one of the amino acid sequences set forth in any one of SEQ ID NOs: 223-248, or to any of the fusion proteins provided herein. In some embodiments, the fusion protein comprises an amino acid sequence that has 1, 2, 3, 4, 5, 6, 7, 8, 9, 10, 11, 12, 13, 14, 15, 16, 17, 18, 19, 20, 21, 22, 21, 24, 25, 26, 27, 28, 29, 30, 31, 32, 33, 34, 35, 36, 37, 38, 39, 40, 41, 42, 43, 44, 45, 46, 47, 48, 49, 50, or more mutations compared to any one of the amino acid sequences set forth in SEQ ID NOs: 223-248, or any of the fusion proteins provided herein. In some embodiments, the fusion protein comprises an amino acid sequence that has at least 5, at least 10, at least 15, at least 20, at least 25, at least 30, at least 35, at least 40, at least 45, at least 50, at least 60, at least 70, at least 80, at least 90, at least 100, at least 110, at least 120, at least 130, at least 140, at least 150, at least 160, at least 170, at least 200, at least 300, at least 400, at least 500, at least 600, at least 700, at least 800, at least 900, at least 1000, at least 1100, at least 1200, at least 1300, at least 1400, at least 1500, at least 1600, at least 1700, at least 1750, or at least 1800 identical contiguous amino acid residues as compared to any one of the amino acid sequences set forth in SEQ ID NOs: 223-248, or any of the fusion proteins provided herein. In some embodiments, the fusion protein (base editor) comprises the amino acid sequence of SEQ ID NO: 223, or a variant thereof that is at lest 60%, at least 65%, at least 70%, at least 75%, at least 80%, at least 85%, at least 90%, at least 95%, at least 96%, at least 97%, at least 98%, at least 99%, or at least 99.5% identical.

In some embodiments, the base editor fusion proteins provided herein are capable of modifying a specific nucleotide base without generating a significant proportion of indels. An "indel", as used herein, refers to the insertion or deletion of a nucleotide base within a nucleic acid. Such insertions or deletions can lead to frame shift mutations within a coding region of a gene. In some embodiments, it is desirable to generate base editors that efficiently modify (*e.g*. mutate or deaminate) a specific nucleotide within a nucleic acid, without generating a large number of insertions or deletions (*i.e.,* indels) in the nucleic acid. In certain embodiments, any of the base editors provided herein are capable of generating a greater proportion of intended modifications (*e.g*., point mutations or deaminations) versus indels. In some embodiments, the base editors provided herein are capable of generating a ratio of intended point mutations to indels that is greater than 1:1. In some embodiments, the base editors provided herein are capable of generating a ratio of intended point mutations to indels that is at least 1.5:1, at least 2:1, at least 2.5:1, at least 3:1, at least 3.5:1, at least 4:1, at least 4.5:1, at least 5:1, at least 5.5:1, at least 6:1, at least 6.5:1, at least 7:1, at least 7.5:1, at least 8:1, at least 10:1, at least 12:1, at least 15:1, at least 20:1, at least 25:1, at least 30:1, at least 40:1, at least 50:1, at least 100: 1, at least 200:1, at least 300: 1, at least 400: 1, at least 500:1, at least 600: 1, at least 700:1, at least 800: 1, at least 900: 1, or at least 1000: 1, or more. The number of intended mutations and indels may be determined using any suitable method. In some embodiments, to calculate indel frequencies, sequencing reads are scanned for exact matches to two 10-bp sequences that flank both sides of a window in which indels might occur. If no exact matches are located, the read is excluded from analysis. If the length of this indel window exactly matches the reference sequence the read is classified as not containing an indel. If the indel window is two or more bases longer or shorter than the reference sequence, then the sequencing read is classified as an insertion or deletion, respectively.

In some embodiments, the base editors provided herein are capable of limiting formation of indels in a region of a nucleic acid. In some embodiments, the region is at a nucleotide targeted by a base editor or a region within 2, 3, 4, 5, 6, 7, 8, 9, or 10 nucleotides of a nucleotide targeted by a base editor. In some embodiments, any of the base editors provided herein are capable of limiting the formation of indels at a region of a nucleic acid to less than 1%, less than 1.5%, less than 2%, less than 2.5%, less than 3%, less than 3.5%, less than 4%, less than 4.5%, less than 5%, less than 6%, less than 7%, less than 8%, less than 9%, less than 10%, less than 12%, less than 15%, or less than 20%. The number of indels formed at a nucleic acid region may depend on the amount of time a nucleic acid (*e.g.,* a nucleic acid within the genome of a cell) is exposed to a base editor. In some embodiments, an number or proportion of indels is determined after at least 1 hour, at least 2 hours, at least 6 hours, at least 12 hours, at least 24 hours, at least 36 hours, at least 48 hours, at least 3 days, at least 4 days, at least 5 days, at least 7 days, at least 10 days, or at least 14 days of exposing a nucleic acid (*e.g.,* a nucleic acid within the genome of a cell) to a base editor.

Some aspects of the disclosure are based on the recognition that any of the base editors provided herein are capable of efficiently generating an intended mutation, such as a point mutation, in a nucleic acid (*e.g.* a nucleic acid within a genome of a subject) without generating a significant number of unintended mutations, such as unintended point mutations. In some embodiments, an intended mutation is a mutation that is generated by a specific base editor bound to a gRNA, specifically designed to generate the intended mutation. In some embodiments, the intended mutation is a mutation associated with a disease or disorder. In some embodiments, the intended mutation is an adenine (A) to guanine (G) point mutation associated with a disease or disorder. In some embodiments, the intended mutation is a thymine (T) to cytosine (C) point mutation associated with a disease or disorder. In some embodiments, the intended mutation is an adenine (A) to guanine (G) point mutation within the coding region of a gene. In some embodiments, the intended mutation is a thymine (T) to cytosine (C) point mutation within the coding region of a gene. In some embodiments, the intended mutation is a point mutation that generates a stop codon, for example, a premature stop codon within the coding region of a gene. In some embodiments, the intended mutation is a mutation that eliminates a stop codon. In some embodiments, the intended mutation is a mutation that alters the splicing of a gene. In some embodiments, the intended mutation is a mutation that alters the regulatory sequence of a gene (*e.g.,* a gene promotor or gene repressor). In some embodiments, any of the base editors provided herein are capable of generating a ratio of intended mutations to unintended mutations (*e.g*., intended point mutations:unintended point mutations) that is greater than 1:1. In some embodiments, any of the base editors provided herein are capable of generating a ratio of intended mutations to unintended mutations (*e.g*., intended point mutations:unintended point mutations) that is at least 1.5:1, at least 2:1, at least 2.5:1, at least 3:1, at least 3.5:1, at least 4:1, at least 4.5:1, at least 5:1, at least 5.5:1, at least 6:1, at least 6.5:1, at least 7:1, at least 7.5:1, at least 8:1, at least 10:1, at least 12:1, at least 15:1, at least 20:1, at least 25:1, at least 30:1, at least 40:1, at least 50:1, at least 100:1, at least 150:1, at least 200:1, at least 250:1, at least 500:1, or at least 1000:1, or more.

### VII. gRNAs

Some aspects of the invention relate to guide sequences ("guide RNA" or "gRNA") that are capable of guiding a napDNAbp or a base editor comprising a napDNAbp to a target site in *SMN2* (*e.g.,* C840T in *SMN2*)*.* In various embodiments base editors (*e.g.,* base editors provided herein) can be complexed, bound, or otherwise associated with (*e.g*., via any type of covalent or non-covalent bond) one or more guide sequences, i.e., the sequence which becomes associated or bound to the base editor and directs its localization to a specific target sequence having complementarity to the guide sequence or a portion thereof. The particular design aspects of a guide sequence will depend upon the nucleotide sequence of a genomic target site of interest (*e.g*., the mutant T840 residue of human SMN2) and the type of napDNA/RNAbp (*e.g*., type of Cas protein) present in the base editor, among other factors, such as PAM sequence locations, percent G/C content in the target sequence, the degree of microhomology regions, secondary structures, etc.

In general, a guide sequence is any polynucleotide sequence having sufficient complementarity with a target polynucleotide sequence to hybridize with the target sequence and direct sequence-specific binding of a napDNAbp (*e.g*., a Cas9, Cas9 homolog, or Cas9 variant) to the target sequence, such as a sequence within an SMN2 gene that comprises C840T. In some embodiments, the degree of complementarity between a guide sequence and its corresponding target sequence (*e.g*., SMN2), when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (*e.g*. the Burrows Wheeler Aligner), ClustalW, Clustal X, BLAT, Novoalign (Novocraft Technologies, ELAND (Illumina, San Diego, Calif.), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 31, 32, 33, 34, 35, 40, 45, 50, 75, or more nucleotides in length.

In some embodiments, a guide sequence is less than about 75, 50, 45, 40, 35, 30, 25, 20, 15, 12, or fewer nucleotides in length. The ability of a guide sequence to direct sequence-specific binding of a base editor to a target sequence may be assessed by any suitable assay. For example, the components of a base editor, including the guide sequence to be tested, may be provided to a host cell having the corresponding target sequence (*e.g*., a HGADFN 167 or HGADFN 188 cell line), such as by transfection with vectors encoding the components of a base editor disclosed herein, followed by an assessment of preferential cleavage within the target sequence, such as by Surveyor assay as described herein. Similarly, cleavage of a target polynucleotide sequence may be evaluated in a test tube by providing the target sequence, components of a base editor, including the guide sequence to be tested and a control guide sequence different from the test guide sequence, and comparing binding or rate of cleavage at the target sequence between the test and control guide sequence reactions. Other assays are possible, and will occur to those skilled in the art.

In some embodiments, a guide sequence designed to target C840T in SMN2. In some embodiments, the target sequence is a SMN2 sequence within a genome of a cell. An exemplary sequence within the human SMN2 gene that contains a wild-type C840T residue is provided below. It should be appreciated, however that additional exemplary SMN2 gene sequences are within the scope of this disclosure and guide RNAs can be designed to accommodate any differences between SMN2 sequences provided herein and any SMN2 sequences, or variants thereof (*e.g*., mutants), found in nature.

Portion of *homo sapiens* SMN2 exon 7 genomic sequence (SEQ ID NO: 249), including the wild-type C840 residue that, when mutated, leads to the development of spinal muscular atrophy (SMA). The wild-type C840 residue is indicated in bold underlined.
*5'-GGTTT**C**AGACAAAATCAAAAAGAAGGAAGGTGCTCACATTCCTTAAATTAA-*3*'* (SEQ ID NO: 249)

An exemplary portion of *homo sapiens* SMN2 exon 7 gene (SEQ ID NO: 249), where the C840 residue has been mutated to a T, is provided below. The mutant T840 is indicated in bold. The underlined portion indicates the nucleic acid residues of mutant LMNA that is complementary to the nucleic residues of the guide sequence
5'- AUUUUGUCU**A**AAACCCUGUA-3' (SEQ ID NO: 250)

5′- GGTTTTAGACAAAATCAAAAAGAAGGAAGGTGCTCACATTCCTTAAATTAA -
3′ (SEQ ID NO: 251)

Additional exemplary portions of the *SMN2* gene include the following:
5'- ATTTTCCTTACAGGGTTT**T**A -3' (SEQ ID NO: 252)
5'- TTTTCCTTACAGGGTTT**T**AG -3' (SEQ ID NO: 398)
5'- TTTCCTTACAGGGTTT**T**AGA -3' (SEQ ID NO: 253)
5'- TTCCTTACAGGGTTT**T**AGAC -3' (SEQ ID NO: 254)
5'- TCCTTACAGGGTTT**T**AGACA -3' (SEQ ID NO: 255)
5'- CCTTACAGGGTTT**T**AGACAA -3' (SEQ ID NO: 256)
5'- CTTACAGGGTTT**T**AGACAAA -3' (SEQ ID NO: 257)
5'- TTACAGGGTTT**T**AGACAAAA -3' (SEQ ID NO: 258)
5'- TACAGGGTTT**T**AGACAAAAT -3' (SEQ ID NO: 259)
5'- ACAGGGTTT**T**AGACAAAATC -3' (SEQ ID NO: 260)
5'- GTTT**T**AGACAAAATC-3' (SEQ ID NO: 261)
5'- GGTTT**T**AGACAAAATCA -3' (SEQ ID NO: 262)
5'- GGGTTT**T**AGACAAAATCAA -3' (SEQ ID NO: 263)
5'- AGGGTTT**T**AGACAAAATCAAA -3' (SEQ ID NO: 264)
5'- CAGGGTTT**T**AGACAAAATCAAAA -3' (SEQ ID NO: 265)
5'- ACAGGGTTT**T**AGACAAAATCAAAA -3' (SEQ ID NO: 266)
5'- CATAGAGCAGCACTAAA**T**G -3' (SEQ ID NO: 267)
5'- ATAGAGCAGCACTAAA**T**GA -3' (SEQ ID NO: 268)
5'- TAGAGCAGCACTAAA**T**GAC -3' (SEQ ID NO: 269)
5'- AGAGCAGCACTAAA**T**GACA -3' (SEQ ID NO: 270)
5'- GAGCAGCACTAAA**T**GACAC -3' (SEQ ID NO: 271)
5'- AGCAGCACTAAA**T**GACACC -3' (SEQ ID NO: 272)
5'- GCAGCACTAAA**T**GACACCA -3' (SEQ ID NO: 273)
5'- CAGCACTAAA**T**GACACCAT -3' (SEQ ID NO: 274)
5'- AGCACTAAA**T**GACACCATA -3' (SEQ ID NO: 275)
5'- GCACTAAA**T**GACACCATAA -3' (SEQ ID NO: 276)
5'- TAAA**T**GACACCATAA -3' (SEQ ID NO: 277)
5'- CTAAA**T**GACACCATAAA -3' (SEQ ID NO: 278)
5'- ACTAAA**T**GACACCATAAAG -3' (SEQ ID NO: 279)
5'- CACTAAA**T**GACACCATAAAGA -3' (SEQ ID NO: 280)
5'- GCACTAAA**T**GACACCATAAAGAA -3' (SEQ ID NO: 281)
5'- AGCACTAAA**T**GACACCATAAAGAAA -3' (SEQ ID NO: 282)
5'- AATTTCATGGTACATGAG**T**G -3' (SEQ ID NO: 283)
5'- ATTTCATGGTACATGAG**T**GG -3' (SEQ ID NO: 399)
5'- TTTCATGGTACATGAG**T**GGC -3' (SEQ ID NO: 284)
5'- TTCATGGTACATGAG**T**GGCT -3' (SEQ ID NO: 285)
5'- TCATGGTACATGAG**T**GGCTA -3' (SEQ ID NO: 286)
5'- CATGGTACATGAG**T**GGCTAT -3' (SEQ ID NO: 287)
5'- ATGGTACATGAG**T**GGCTATC -3' (SEQ ID NO: 288)
5'- TGGTACATGAG**T**GGCTATCA -3' (SEQ ID NO: 289)
5'- GGTACATGAG**T**GGCTATCAT -3' (SEQ ID NO: 290)
5'- GTACATGAG**T**GGCTATCATA -3' (SEQ ID NO: 291)
5'- TGAG**T**GGCTATCATA -3' (SEQ ID NO: 292)
5'- ATGAG**T**GGCTATCATAC -3' (SEQ ID NO: 293)
5'- CATGAG**T**GGCTATCATACT -3' (SEQ ID NO: 294)
5'- ACATGAG**T**GGCTATCATACTG -3' (SEQ ID NO: 295)
5'- TACATGAG**T**GGCTATCATACTGG -3' (SEQ ID NO: 296)

The disclosure also contemplates exemplary portions of the *SMN2* gene that are shorter or longer than any one of the exemplary portions of the *SMN2* gene provided in any one of SEQ ID NOs: 252-298, 398, and 399. It should be appreciated that guide sequences may be engineered that are complementary (*e.g*., 100% complementary) to any of the exemplary portions of the *SMN2* gene provided herein (*e.g.,* SEQ ID NOs: 252-298, 398, and 399). In some embodiments, a guide sequence is complementary (*e.g*., 100% complementary) to any one of SEQ ID NOs: 250-298, 398, and 399. In some embodiments, a guide sequence is complementary (*e.g*., 100% complementary) to a sequence of any one of SEQ ID NOs: 250-298,398, and 399 absent the first 1, 2, 3, 4, 5, 7, 8, 9, 10, 11, or 12 nucleic acid residues at the 5' end. In some embodiments, a guide sequence is complementary (e.g., 100% complementary) to a sequence of any one of SEQ ID NOs: 250-298, 398, and 399 absent the first 1, 2, 3, 4, 5, 7, 8, 9, 10, 11, or 12 nucleic acid residues at the 3' end.

In some embodiments, a guide sequence is selected to reduce the degree of secondary structure within the guide sequence. Secondary structure may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see *e.g.* A. R. Gruber et al., 2008, Cell 106(1): 23-24; and PA Carr and GM Church, 2009, Nature Biotechnology 27(12): 1151-62). Further algorithms may be found in U.S. application Ser. No. 61/836,080; Broad Reference BI-2013/004A); incorporated herein by reference.

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a complex at a target sequence, wherein the complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. Preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In some embodiments, the single transcript further includes a transcription termination sequence; preferably this is a polyT sequence, for example six T nucleotides. Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator:
(1) 
NNNNNNNNgtttttgtactctcaagatttaGAAAtaaatcttgcagaagctacaaagataaggctt
catgccgaaatcaacaccctgtcattttatggcagggtgttttcgttatttaaTTTTTT (SEQ ID NO: 297);
(2) 
NNNNNNNNNNNNNNNNNNNNgtttttgtactctcaGAAAtgcagaagctacaaagataaggcttcatgccgaaatca
acaccctgtcattttatggcagggtgttttcgttatttaaTTTTTT (SEQ ID NO: 298);
(3) 
NNNNNNNNNNNNNNNNNNNNgtttttgtactctcaGAAAtgcagaagctacaaagataaggcttcatgccgaa
atca acaccctgtcattttatggcagggtgtTTTTT (SEQ ID NO: 299);
(4) 
NNNNNNNNNNNNNNNNNNNNgttttagagctaGAAAtagcaagttaaaataaggctagtccgttatcaacttga
aaa agtggcaccgagtcggtgcTTTTTT (SEQ ID NO: 300);
(5) 
NNNNNNNNNNNNNNNNNNNNgttttagagctaGAAATAGcaagttaaaataaggctagtccgttatcaactt
gaa aaagtgTTTTTTT (SEQ ID NO: 301); and
(6)

NNNNNNNNNNNNNNNNNNNNgttttagagctagAAATAGcaagttaaaataaggctagtccgttatcaTTT
TT TTT (SEQ ID NO: 302).

The disclosure also relates to guide RNA sequences that are variants of any of the herein disclosed guide RNA sequences or target sequences (including SEQ ID NOs.: 250-302), wherein the variants include guide RNA sequences or target sequences having a deletion of 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides from any of the guide RNA or target sequence disclosed herein (e.g., SEQ ID NOs.: 250-302). In other embodiments, the variants also include guide RNA sequences or target sequences having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or up to 99.9% sequence identity with a guide RNA or target sequence disclosed herein (e.g., SEQ ID NOs.: 250-302).

In some embodiments, sequences (1) to (3) are used in combination with Cas9 from S. *thermophilus* CRISPR. In some embodiments, sequences (4) to (6) are used in combination with Cas9 from *S. pyogenes.* In some embodiments, the tracr sequence is a separate transcript from a transcript comprising the tracr mate sequence.

It will be apparent to those of skill in the art that in order to target any of the fusion proteins comprising a Cas9 domain and an adenosine deaminase, as disclosed herein, to a target site, *e.g.,* a site comprising C840T in *SMN2* to be edited, it is typically necessary to co-express the fusion protein together with a guide RNA, *e.g.,* an sgRNA. As explained in more detail elsewhere herein, a guide RNA typically comprises a tracrRNA framework allowing for Cas9 binding, and a guide sequence, which confers sequence specificity to the Cas9:nucleic acid editing enzyme/domain fusion protein.

In some embodiments, the guide RNA comprises a structure 5'-[guide sequence]-[Cas9-binding sequence]-3', where the Cas9 binding sequence comprises a nucleic acid sequence that is at least 70%, 75%, 80%, 85%, 90%, 95%, 96%, 97%, 98%, 99%, or 99.5% identical to SEQ ID NO: 306-323, and which are effective to targeting C840T in SMN2. In other embodiments, the guide RNA comprises a structure 5'-[guide sequence]-[Cas9-binding sequence]-3', where the Cas9 binding sequence comprises a nucleic acid sequence that is at least 70%, 75%, 80%, 85%, 90%, 95%, 96%, 97%, 98%, 99%, or 99.5% identical to SEQ ID NO: 324-329, and which are effective to targeting a stop codon in exon 8 of SMN2. In yet other embodiments, the guide RNA comprises a structure 5'-[guide sequence]-[Cas9-binding sequence]-3', where the Cas9 binding sequence comprises a nucleic acid sequence that is at least 70%, 75%, 80%, 85%, 90%, 95%, 96%, 97%, 98%, 99%, or 99.5% identical to SEQ ID NO: 330, and which are effective to targeting the S270 amino acid in exon 6 of SMN2. In some embodiments, the guide RNA comprises a structure 5'-[guide sequence]-[Cas9-binding sequence]-3', where the Cas9 binding sequence comprises a nucleic acid sequence SEQ ID NO: 303, SEQ ID NO: 304, or SEQ ID NO: 303 or 304 absent the poly-U terminator sequence at the 3' end.

5′GUUUUAGAGCUAGAAAUAGCAAGUUAAAAUAAGGCUAGUCCGUUAU
CAACUUGAAAAAGUGGCACCGAGUCGGUGCUUUUU-3′ (SEQ ID NO: 303)

5′GUUUUAGAGCUAGAAAUAGCAAGUUAAAAUAAGGCUAGUCCGUUAUC
AACUUGAAAAAGUGGCACCGAGUCGGUGCUUUUUUU-3′ (SEQ ID NO: 304)

In some embodiments, the guide RNA comprises a nucleic acid sequence that is at least 70%, 75%, 80%, 85%, 90%, 95%, 96%, 97%, 98%, 99%, or 99.5% identical to SEQ ID NO: 305, or SEQ ID NO: 305 absent the poly-U terminator sequence at the 3' end. In some embodiments, the guide RNA comprises the nucleic acid sequence SEQ ID NO: 305, or SEQ ID NO: 305 absent the poly-U terminator sequence at the 3' end.

In some embodiments, the guide RNA comprises the nucleic acid sequence

5′GGUCCACCCACCUGGGCUCCGUUUUAGAGCUAGAAAUAGCAAGUUAAAAUA
AGGCUAGUCCGUUAUCAACUUGAAAAAGUGGCACCGAGUCGGUGCUUUUUU-
3′ (SEQ ID NO: 305).

The guide sequence is typically approximately 20 nucleotides long. Exemplary guide sequences for targeting a base editor *(e.g.,* ABE7.10) to a site comprising C840T in *SMN2* are provided below. It should be appreciated, however, that changes to such guide sequences can be made based on the specific *SMN2* sequence found within a cell, for example the cell of a patient having spinal muscular atrophy (SMA). Such suitable guide RNA sequences typically comprise guide sequences that are complementary to a nucleic sequence within 50 nucleotides upstream or downstream of the target nucleotide to be edited.

Exemplary guide sequences to target C840T in *SMN2:*
5'- UUUUUGAUUUUGUCUAAAACCCUGUA -3' (SEQ ID NO: 306)
5'- UUUUGAUUUUGUCUAAAACCCUGUA -3' (SEQ ID NO: 307)
5'- UUUGAUUUUGUCUAAAACCCUGUA -3' (SEQ ID NO: 308)
5'- UUGAUUUUGUCUAAAACCCUGUA -3' (SEQ ID NO: 309)
5'- UGAUUUUGUCUAAAACCCUGUA -3' (SEQ ID NO: 310)
5'- GAUUUUGUCUAAAACCCUGUA -3' (SEQ ID NO: 311)
5'- AUUUUGUCUAAAACCCUGUA -3' (SEQ ID NO: 312)
5'- UUUUGUCUAAAACCCUGUA -3' (SEQ ID NO: 313)
5'- UUUGUCUAAAACCCUGUA -3' (SEQ ID NO: 314)
5'- UUGUCUAAAACCCUGUA -3' (SEQ ID NO: 315)
5'- UGUCUAAAACCCUGUA -3' (SEQ ID NO: 316)
5'- UUUGUCUAAAACCCUGUAAG -3' (SEQ ID NO: 317)
5'- UUUUGUCUAAAACCCUGUAA -3' (SEQ ID NO: 318)
5'- UGAUUUUGUCUAAAACCC -3' (SEQ ID NO: 319)
5'- GAUUUUGUCUAAAACCCU -3' (SEQ ID NO: 320)
5'- AUUUUGUCUAAAACCCUG -3' (SEQ ID NO: 321)
5'- GUCUAAAACCCUGUAAGG -3' (SEQ ID NO: 322)
5'- UCUAAAACCCUGUAAGGA -3' (SEQ ID NO: 323)
5'- GAUUUUGUCUAAAACCCUGUAAG -3' (SEQ ID NO: 451)
5'- UUGUCUAAAACCCUGUAAGG -3' (SEQ ID NO: 452)
5'- UGUCUAAAACCCUGUAAGGA -3' (SEQ ID NO: 453)
5'- GUCUAAAACCCUGUAAGGAA -3' (SEQ ID NO: 454)

Exemplary guide sequences to target a stop codon in exon 8 of *SMN2:*
5'- UUUGCAGGAAAUGCUGGCAU - 3' (SEQ ID NO: 324)
5' - UUCUCAUUUGCAGGAAAUGC - 3' (SEQ ID NO: 325)
5' - CAUUUAGUGCUGCUCUAUGC - 3' (SEQ ID NO: 326)
5' - CAGGAAAUGCUGGCAUAGAG - 3' (SEQ ID NO: 327)
5' - UUGCAGGAAAUGCUGGCAUA - 3' (SEQ ID NO: 328)
5' - AUUUGCAGGAAAUGCUGGCA - 3' (SEQ ID NO: 329)

Exemplary guide sequences to target the S270 amino acid in exon 6 of *SMN2:*
5' - UACAUGAGUGGCUAUCAUAC - 3' (SEQ ID NO: 330)

Exemplary guide sequences to target *SMN2* ISS-N1:
5'- AAAAGUAAGAUUCACUUUCA -3' (SEQ ID NO: 455)
5'- CAAAAGUAAGAUUCACUUUC -3' (SEQ ID NO: 456)
5'- ACAAAAGUAAGAUUCACUUU -3' (SEQ ID NO: 457)
5'- UACAAAAGUAAGAUUCACUU -3' (SEQ ID NO: 458)

For the exemplary guide sequences provided above (e.g., SEQ ID NOs: 306-323), the A that is complementary to the mutant T of T840 in *SMN2* is shown in bold. For the exemplary guide sequences provided above (e.g., SEQ ID NOs: 324-329), the A that is complementary to a stop codon comprising T of exon 8 in *SMN2* is shown in bold. For the exemplary guide sequences provided above (e.g., SEQ ID NO: 330), the A that is complementary to the S270 codon T of in exon 6 of *SMN2* is shown in bold.

The disclosure also provides guide sequences that are truncated variants of any of the guide sequences provided herein (e.g., SEQ ID NOs: 306-330). In some embodiments, the guide sequence comprises the amino acid sequence of any one of SEQ ID NOs: 306-330, absent the first 1, 2, 3, 4, 5, 6, 7, 8, 9, or 10 nucleic acid residues from the 5' end. It should be appreciated that any of the 5' truncated guide sequences provided herein may further comprise a G residue at the 5' end. In some embodiments, the guide sequence comprises the amino acid sequence of any one of SEQ ID NOs: 306-330, absent the first 1, 2, 3, 4, 5, 6, 7, 8, 9, 10, 11, or 12 nucleic acid residues from the 3' end.

The disclosure also provides guide sequences that are longer variants of any of the guide sequences provided herein (e.g., SEQ ID NOs: 306-330). In some embodiments, the guide sequence comprises one additional residue that is 5'-U-3' at the 3' end of any one of SEQ ID NOs: 306-330. In some embodiments, the guide sequence comprises two additional residues that are 5'-UG-3' at the 3' end of any one of SEQ ID NOs: 306-330. In some embodiments, the guide sequence comprises three additional residues that are 5'-UGA-3' at the 3' end of any one of SEQ ID NOs: 306-330. In some embodiments, the guide sequence comprises four additional residues that are 5'-UGAG-3' at the 3' end of any one of SEQ ID NOs: 306-330. In some embodiments, the guide sequence comprises five additional residues that are 5'-UGAGC-3' at the 3' end of any one of SEQ ID NOs: 306-330. In some embodiments, the guide sequence comprises six additional residues that are 5'-UGAGCC-3' at the 3' end of any one of SEQ ID NOs: 306-330. In some embodiments, the guide sequence comprises seven additional residues that are 5'-UGAGCCG-3' at the 3' end of any one of SEQ ID NOs: 306-330. In some embodiments, the guide sequence comprises eight additional residues that are 5'-UGAGCCGC-3' at the 3' end of any one of SEQ ID NOs: 306-330. In some embodiments, the guide sequence comprises nine additional residues that are 5'-UGAGCCGCU-3' at the 3' end of any one of SEQ ID NOs: 306-330. In some embodiments, the guide sequence comprises ten additional residues that are 5'-UGAGCCGCUG-3' (SEQ ID NO: 400) at the 3' end of any one of SEQ ID NOs: 306-330. In some embodiments, the guide sequence comprises eleven additional residues that are 5'-UGAGCCGCUGG-3' (SEQ ID NO: 401) at the 3' end of any one of SEQ ID NOs: 306-330.

### VIII. Fusion Protein/gRNA Complexes

Some aspects of this disclosure provide complexes comprising any of the fusion proteins (e.g., base editor) provided herein, for example any of the adenosine base editors provided herein, and a guide nucleic acid bound to napDNAbp of the fusion protein. In some embodiments, the guide nucleic acid is any one of the guide RNAs provided herein. In some embodiments, the disclosure provides any of the fusion proteins (e.g., adenosine base editors) provided herein bound to any of the guide RNAs provided herein. In some embodiments, the napDNAbp of the fusion protein *(e.g.,* adenosine base editor) is a Cas9 domain *(e.g.,* a dCas9, a nuclease active Cas9, or a Cas9 nickase), which is bound to a guide RNA. In some embodiments, the complexes provided herein are configured to generate a mutation in a nucleic acid, for example to correct a point mutation in a gene (*e.g., SMN2*) to modulate expression of one or more proteins (e.g., SMN).

In some embodiments, the guide RNA comprises a guide sequence that comprises at least 8, 9, 10, 11, 12, 13, 14, 15, 16, 17, 18, 19, 20, 21, 22, 23, 24, 25, 26, 27, 28, 29, or 30 contiguous nucleic acids that are 100% complementary to a target sequence, for example a target DNA sequence (e.g., a target DNA sequence of any one of SEQ ID NOs: 253-296 and 398-399). In some embodiments, the guide RNA comprises a guide sequence that comprises at least 8, 9, 10, 11, 12, 13, 14, 15, 16, 17, 18, 19, 20, 21, 22, 23, 24, 25, 26, 27, 28, 29, or 30 contiguous nucleic acids that are 100% complementary to a DNA sequence in a *SMN2* gene *(e.g.,* a target DNA sequence of any one of SEQ ID NOs: 253-296 and 398-399), for example a region of a human *SMN2* gene. In some embodiments, the *SMN2* is the *SMN2* of Gene ID: 6607.

In some embodiments, any of the complexes provided herein comprise a gRNA having a guide sequence that comprises at least 8, 9, 10, 11, 12, 13, 14, 15, 16, 17, 18, 19, 20, 21, 22, 23, 24, 25, 26, 27, 28, 29, or 30 contiguous nucleic acids that are 100% complementary to any one of the nucleic acid sequences provided herein. It should be appreciated that the guide sequence of the gRNA may comprise one or more nucleotides that are not complementary to a target sequence. In some embodiments, the guide sequence of the gRNA is at the 5' end of the gRNA. In some embodiments, the guide sequence of the gRNA further comprises a G at the 5' end of the gRNA. In some embodiments, the G at the 5' end of the gRNA is not complementary with the target sequence. In some embodiments, the guide sequence of the gRNA comprises 1, 2, 3, 4, 5, 6, 7, or 8 nucleotides that are not complementary to a target sequence *(e.g.,* any of the target sequences provided herein (*e.g.,* SEQ ID NOs: 297-305, 306-362, 400-401, and 405-406)). In some embodiments, the gRNA comprises the sequence of SEQ ID NO: 297, or the sequence of any one of SEQ ID NOs: 297-305, 306-362, 400-401, and 405-406, where the nucleotide target is indicated in bold. It should be appreciated that the T's indicated in any of the gRNA sequences of SEQ ID NOs: 297-305, 306-362, 400-401, and 405-406are uricils (Us) in the RNA sequence. Accordingly, in some embodiments, the gRNA comprises the sequence 5'-AUUUUGUCUAAAACCCUGUA -3' (SEQ ID NO: 312).

In some embodiments, the guide RNA comprises a guide sequence that comprises at least 8, 9, 10, 11, 12, 13, 14, 15, 16, 17, 18, 19, 20, 21, 22, 23, 24, 25, 26, 27, 28, 29, or 30 contiguous nucleic acids that are 100% complementary to a target sequence, for example a target DNA sequence in a *SMN2* gene. In some embodiments, the guide RNA comprises a guide sequence that comprises at least 8, 9, 10, 11, 12, 13, 14, 15, 16, 17, 18, 19, 20, 21, 22, 23, 24, 25, 26, 27, 28, 29, or 30 contiguous nucleic acids that are 100% complementary to a DNA sequence in a human *SMN2* gene. In some embodiments, the *SMN2* gene is a human, chimpanzee, ape, monkey, dog, mouse, or rat *SMN2* gene. In some embodiments, the *SMN2* gene is a human *SMN2* gene. In some embodiments, the *SMN2* gene is the *SMN2* gene of Gene ID: 6607, which has also been referred to as SMNC, BCD541, GEMIN1, TDRD16B, or C-BCD541.

### IX. Editing Methods

Some aspects of this disclosure provide methods of using the fusion proteins, or complexes comprising a guide nucleic acid *(e.g.,* gRNA) and a nucleobase editor provided herein to edit DNA, e.g., to edit *SMN2.* For example, some aspects of this disclosure provide methods comprising contacting a DNA, or RNA molecule with any of the fusion proteins provided herein, and with at least one guide nucleic acid *(e.g.,* guide RNA), wherein the guide nucleic acid, *(e.g.,* guide RNA) is comprises a sequence *(e.g.,* a guide sequence that binds to a DNA target sequence) of at least 10 *(e.g.,* at least 10, 15, 20, 25, or 30) contiguous nucleotides that is 100% complementary to a target sequence *(e.g.,* any of the target *SMN2* sequences provided herein). In some embodiments, the 3' end of the target sequence is immediately adjacent to a canonical PAM sequence (NGG). In some embodiments, the 3' end of the target sequence is not immediately adjacent to a canonical PAM sequence (NGG). In some embodiments, the 3' end of the target sequence is immediately adjacent to an AGC, GAG, TTT, GTG, or CAA sequence.

Some aspects of the disclosure provide methods of using base editors (e.g., any of the fusion proteins provided herein) and gRNAs to correct a point mutation in an *SMN2* gene. In some embodiments, the disclosure provides methods of using base editors (e.g., any of the fusion proteins provided herein) and gRNAs to generate an A to G and/or T to C mutation in an *SMN2* gene. In some embodiments, the disclosure provides method for deaminating an adenosine nucleobase (A) in an *SMN2* gene, the method comprising contacting the *SMN2* gene with a base editor and a guide RNA bound to the base editor, where the guide RNA comprises a guide sequence that is complementary to a target nucleic acid sequence in the *SMN2* gene. In some embodiments, the *SMN2* gene comprises a C to T mutation. In some embodiments, the C to T mutation in the *SMN2* gene masks an acceptor splice site, resulting in a truncated *SMN* protein encoded by the *SMN2* gene (*i.e.,* exon 7 is not transcribed). While the resulting protein functions as a full-length SMN protein, it is prone to rapid degradation due to the presence of an EMLA (SEQ ID NO: 466) tail from exon 8 and the exposed exon 6 C-terminal amino acid chain. In some embodiments, the C to T mutation in the *SMN2* gene results in the degradation of at least 1%, 2%, 5%, 10%, 15%, 20%, 25%, 30%, 35%, 40%, 45%, 50%, 55%, 60%, 65%, 70%, 75%, 80%, 85%, 90%, 95%, 98%, or at least 99% of the resulting SMN protein.

In some embodiments, deaminating an adenosine (A) nucleobase complementary to the T corrects the C to T mutation in the *SMN2* gene. In some embodiments, the C to T or G to A mutation in the *SMN2* gene leads to a Cys (C) to Tyr (Y) mutation in the SMN2 protein encoded by the *SMN2* gene. In some embodiments, deaminating the adenosine nucleobase complementary to the T corrects the Cys to Tyr mutation in the SMN2 protein.

In some embodiments, the guide sequence of the gRNA comprises at least 8, 9, 10, 11, 12, 13, 14, 15, 16, 17, 18, 19, 20, 21, 22, 23, 24, 25, 26, 27, 28, 29, 30, 31, 32, 33, 34, or 35 contiguous nucleic acids that are 100% complementary to a target nucleic acid sequence of the *SMN2* gene. In some embodiments, the base editor nicks the target sequence that is complementary to the guide sequence.

In some embodiments, the target DNA sequence comprises a sequence associated with a disease or disorder, e.g., SMA. In some embodiments, the target DNA sequence comprises a point mutation associated with a disease or disorder *(e.g.,* exon 7 of *SMN2*)*.* In some embodiments, the activity of the fusion protein (e.g., comprising an adenosine deaminase and a Cas9 domain), or the complex, results in a correction of the point mutation. In some embodiments, the target DNA sequence comprises a C→T point mutation associated with a disease or disorder, and wherein the deamination of the mutant base results in a sequence that is not associated with a disease or disorder. In some embodiments, the target DNA sequence encodes a protein, and the point mutation is in a codon and results in a change in the splice site of an exon, resulting in production of a full-length, fully functional protein *(e.g.,* SMN protein). In some embodiments, the deamination of the mutant base results in the wild-type amino acid.

In some embodiments, the target DNA sequence comprises a sequence associated with a stop codon in an exon 8 of a *SMN2* gene. In some embodiments, the activity of the fusion protein (e.g., comprising an adenosine deaminase and a Cas9 domain), or the complex, results in destruction of the stop codon and/or a frameshift mutation. Without wishing to be bound by theory, it is thought that destroying a stop codon *(e.g.,* the 5^{th} codon stop sequence) and/or inducing at least one frameshift mutation result in a more stable SMN protein product, regardless of whether amino acids encoded by exon 7 are included in the protein. For example, in one embodiment, activity of the fusion protein (e.g., comprising an adenosine deaminase and a Cas9 domain), or the complex, results in adenine deamination of the 5^{th} codon stop sequence of a *SMN2's* exon 8, facilitating the addition of five amino acids at the C-terminal end of the translated SMN protein.

In some embodiments, the target DNA sequence comprises a sequence associated with an amino acid present in exon 6 of an *SMN2* gene. Modification of one amino acid (*e.g.,* S270) using the methods described herein, can be used to slow the rate of SMN protein degradation.

In some embodiments, the contacting is *in vivo* in a subject. In some embodiments, the subject has or has been diagnosed with a disease or disorder (e.g., SMA).

Some embodiments provide methods for using the DNA editing fusion proteins provided herein. In some embodiments, the fusion protein is used to introduce a point mutation into a nucleic acid by deaminating a target nucleobase. In some embodiments, the deamination of the target nucleobase results in the correction of a genetic defect, *e.g.,* in the correction of a point mutation that leads to degradation of the resulting SMN protein. In some embodiments, the genetic defect is associated with a disease or disorder, e.g., SMA.

In some embodiments, the purposes of the methods provided herein are to restore the full-length gene or to stabilize the resulting protein product via genome editing. The nucleobase editing proteins provided herein can be validated for gene editing-based human therapeutics *in vitro, e.g.,* by correcting a disease-associated mutation in human cell culture. It will be understood by the skilled artisan that the nucleobase editing proteins provided herein, *e.g.,* the fusion proteins comprising a nucleic acid programmable DNA binding protein (e.g., Cas9) and an adenosine deaminase domain can be used to correct any single point G to A or C to T mutation. In the first case, deamination of the mutant A to I corrects the mutation, and in the latter case, deamination of the A that is base-paired with the mutant T, followed by a round of replication or followed by base editing repair activity, corrects the mutation.

The instant disclosure provides methods for the treatment of a subject diagnosed with a disease associated with or caused by a point mutation that can be corrected by a DNA editing fusion protein provided herein. For example, in some embodiments, a method is provided that comprises administering to a subject having such a disease, e.g., SMA.

In some embodiments, a fusion protein recognizes canonical PAMs and therefore can correct the pathogenic G to A or C to T mutations with canonical PAMs, *e.g.,* NGG, respectively, in the flanking sequences. For example, Cas9 proteins that recognize canonical PAMs comprise an amino acid sequence that is at least 80%, 85%, 90%, 95%, 97%, 98%, or 99% identical to the amino acid sequence of *Streptococcus pyogenes* Cas9 as provided by any one of SEQ ID NOs: 5, 8, 10, 12, and 407 or to a fragment thereof comprising the RuvC and HNH domains of any one of SEQ ID NOs: 5, 8, 10, 12, and 407.

It will be apparent to those of skill in the art that in order to target any of the fusion proteins comprising a Cas9 domain and an adenosine deaminase, as disclosed herein, to a target site, *e.g.,* a site comprising a point mutation to be edited, it is typically necessary to co-express the fusion protein together with a guide RNA, *e.g.,* an sgRNA. As explained in more detail elsewhere herein, a guide RNA typically comprises a tracrRNA framework allowing for Cas9 binding, and a guide sequence, which confers sequence specificity to the Cas9:nucleic acid editing enzyme/domain fusion protein. In some embodiments, the guide RNA sequence 5'-ATTTTGTCTAAAACCCTGTA-3' (SEQ ID NO: 331), where the nucleotide target is indicated in bold. It should be appreciated that the Ts indicated in the gRNA sequence are uracil (Us) in the RNA sequence. Accordingly, in some embodiments, the gRNA comprises the sequence 5'-AUUUUGUCUAAAACCCUGUA-3' (SEQ ID NO: 332).

In some embodiments, the guide sequence of the gRNA comprises a nucleic acid sequence selected from the group consisting of 5'-TTTGTCTAAAACCCTGTAAG-3' (SEQ ID NO: 333), 5'-TTTTGTCTAAAACCCTGTAA-3' (SEQ ID NO: 334), 5'-TGATTTTGTCTAAAACCC-3' (SEQ ID NO: 335), 5'-GATTTTGTCTAAAACCCT-3' (SEQ ID NO: 336), 5'-ATTTTGTCTAAAACCCTG-3' (SEQ ID NO: 337), 5'-GTCTAAAACCCTGTAAGG-3' (SEQ ID NO: 338), and 5'-TCTAAAACCCTGTAAGGA-3' (SEQ ID NO: 339). As noted previously, the gRNA sequence may comprise uracil (U) instead of thymine (T). Therefore, in some embodiments, the guide sequence of the gRNA comprises a nucleic acid sequence selected from the group consisting of 5'-UUUGUCUAAAACCCUGUAAG-3' (SEQ ID NO: 340), 5'-UUUUGUCUAAAACCCUGUAA-3' (SEQ ID NO: 341), 5'-UGAUUUUGUCUAAAACCC-3' (SEQ ID NO: 342), 5'-GAUUUUGUCUAAAACCCU-3' (SEQ ID NO: 343), 5'-AUUUUGUCUAAAACCCUG-3' (SEQ ID NO: 344), 5'-GUCUAAAACCCUGUAAGG-3' (SEQ ID NO: 345), and 5'-UCUAAAACCCUGUAAGGA-3' (SEQ ID NO: 346).

In some embodiments, the gRNA comprises a nucleic acid sequence selected from the group consisting of: 5'-TTTGCAGGAAATGCTGGCAT-3' (SEQ ID NO: 347), 5'-TTCTCATTTGCAGGAAATGC-3' (SEQ ID NO: 348), 5'-CATTTAGTGCTGCTCTATGC-3' (SEQ ID NO: 349), 5'-CAGGAAATGCTGGCATAGAG-3' (SEQ ID NO: 350), 5'-TTGCAGGAAATGCTGGCATA-3' (SEQ ID NO: 351), 5'-ATTTGCAGGAAATGCTGGCA-3' (SEQ ID NO: 352), and 5'-TGGCATAGAGCAGCACTAAA-3' (SEQ ID NO: 353), where the nucleotide target is indicated in bold. It should be appreciated that the Ts indicated in the gRNA sequence are uracil (Us) in the RNA sequence. Accordingly, in some embodiments, the gRNA comprises a sequence selected from the group consisting of: 5'-UUUGCAGGAAAUGCUGGCAU-3' (SEQ ID NO: 354), 5'-UUCUCAUUUGCAGGAAAUGC-3' (SEQ ID NO: 355), 5'-CAUUUAGUGCUGCUCUAUGC-3' (SEQ ID NO: 356), 5'-CAGGAAAUGCUGGCAUAGAG-3' (SEQ ID NO: 357), 5'-UUGCAGGAAAUGCUGGCAUA-3' (SEQ ID NO: 358), 5'-AUUUGCAGGAAAUGCUGGCA-3' (SEQ ID NO: 359), and 5'-UGGCAUAGAGCAGCACUAAA-3' (SEQ ID NO: 360).

In some embodiments, the gRNA comprises the nucleic acid sequence 5'-TGGCATAGAGCAGCACTAAA-3' (SEQ ID NO: 361), where the nucleotide target is indicated in bold. It should be appreciated that the Ts indicated in the gRNA sequence are uracil (Us) in the RNA sequence. Accordingly, in some embodiments, the gRNA comprises the sequence: 5'-UGGCAUAGAGCAGCACUAAA-3' (SEQ ID NO: 362).

Some aspects of the disclosure provide methods for editing a nucleic acid. In some embodiments, the method is a method for editing a nucleobase of a nucleic acid *(e.g.,* a base pair of a double-stranded DNA sequence). In some embodiments, the method comprises the steps of: a) contacting a target region of a nucleic acid *(e.g.,* a double-stranded DNA sequence) with a complex comprising a base editor *(e.g.,* a Cas9 domain fused to an adenosine deaminase) and a guide nucleic acid (e.g., gRNA), wherein the target region comprises a targeted nucleobase pair, b) inducing strand separation of said target region, c) converting a first nucleobase of said target nucleobase pair in a single strand of the target region to a second nucleobase, and d) cutting no more than one strand of said target region, where a third nucleobase complementary to the first nucleobase base is replaced by a fourth nucleobase complementary to the second nucleobase. In some embodiments, the method results in less than 20% indel formation in the nucleic acid. It should be appreciated that in some embodiments, step b is omitted. In some embodiments, the first nucleobase is an adenine. In some embodiments, the second nucleobase is a deaminated adenine, or inosine. In some embodiments, the third nucleobase is a thymine. In some embodiments, the fourth nucleobase is a cytosine. In some embodiments, the method results in less than 19%, 18%, 16%, 14%, 12%, 10%, 8%, 6%, 4%, 2%, 1%, 0.5%, 0.2%, or less than 0.1% indel formation. In some embodiments, the method further comprises replacing the second nucleobase with a fifth nucleobase that is complementary to the fourth nucleobase, thereby generating an intended edited base pair *(e.g.,* A:T to G:C). In some embodiments, the fifth nucleobase is a guanine. In some embodiments, at least 5% of the intended base pairs are edited. In some embodiments, at least 10%, 15%, 20%, 25%, 30%, 35%, 40%, 45%, or 50% of the intended base pairs are edited.

In some embodiments, the ratio of intended products to unintended products in the target nucleotide is at least 2:1, 5:1, 10:1, 20:1, 30:1, 40:1, 50:1, 60:1, 70:1, 80:1, 90:1, 100:1, or 200:1, or more. In some embodiments, the ratio of intended point mutation to indel formation is greater than 1:1, 10:1, 50:1, 100:1, 500:1, or 1000:1, or more. In some embodiments, the cut single strand (nicked strand) is hybridized to the guide nucleic acid. In some embodiments, the cut single strand is opposite to the strand comprising the first nucleobase. In some embodiments, the base editor comprises a Cas9 domain. In some embodiments, the first base is adenine, and the second base is not a G, C, A, or T. In some embodiments, the second base is inosine. In some embodiments, the first base is adenine. In some embodiments, the second base is not a G, C, A, or T. In some embodiments, the second base is inosine. In some embodiments, the base editor inhibits base excision repair of the edited strand. In some embodiments, the base editor protects or binds the non-edited strand. In some embodiments, the base editor comprises UGI activity. In some embodiments, the base editor comprises a catalytically inactive inosine-specific nuclease. In some embodiments, the base editor comprises nickase activity. In some embodiments, the intended edited base pair is upstream of a PAM site. In some embodiments, the intended edited base pair is 1, 2, 3, 4, 5, 6, 7, 8, 9, 10, 11, 12, 13, 14, 15, 16, 17, 18, 19, or 20 nucleotides upstream of the PAM site. In some embodiments, the intended edited basepair is downstream of a PAM site. In some embodiments, the intended edited base pair is 1, 2, 3, 4, 5, 6, 7, 8, 9, 10, 11, 12, 13, 14, 15, 16, 17, 18, 19, or 20 nucleotides downstream stream of the PAM site. In some embodiments, the method does not require a canonical (e.g., NGG) PAM site. In some embodiments, the nucleobase editor comprises a linker. In some embodiments, the linker is 1-25 amino acids in length. In some embodiments, the linker is 5-20 amino acids in length. In some embodiments, linker is 10, 11, 12, 13, 14, 15, 16, 17, 18, 19, or 20 amino acids in length. In some embodiments, the target region comprises a target window, wherein the target window comprises the target nucleobase pair. In some embodiments, the target window comprises 1-10 nucleotides. In some embodiments, the target window is 1-9, 1-8, 1-7, 1-6, 1-5, 1-4, 1-3, 1-2, or 1 nucleotides in length. In some embodiments, the target window is 1, 2, 3, 4, 5, 6, 7, 8, 9, 10, 11, 12, 13, 14, 15, 16, 17, 18, 19, or 20 nucleotides in length. In some embodiments, the intended edited base pair is within the target window. In some embodiments, the target window comprises the intended edited base pair. In some embodiments, the method is performed using any of the base editors provided herein. In some embodiments, a target window is a deamination window.

In some embodiments, the disclosure provides methods for editing a nucleotide. In some embodiments, the disclosure provides a method for editing a nucleobase pair of a double-stranded DNA sequence. In some embodiments, the method comprises a) contacting a target region of the double-stranded DNA sequence with a complex comprising a base editor and a guide nucleic acid (e.g., gRNA), where the target region comprises a target nucleobase pair, b) inducing strand separation of said target region, c) converting a first nucleobase of said target nucleobase pair in a single strand of the target region to a second nucleobase, d) cutting no more than one strand of said target region, wherein a third nucleobase complementary to the first nucleobase base is replaced by a fourth nucleobase complementary to the second nucleobase, and the second nucleobase is replaced with a fifth nucleobase that is complementary to the fourth nucleobase, thereby generating an intended edited base pair, wherein the efficiency of generating the intended edited base pair is at least 5%. It should be appreciated that in some embodiments, step b is omitted. In some embodiments, at least 5% of the intended base pairs are edited. In some embodiments, at least 10%, 15%, 20%, 25%, 30%, 35%, 40%, 45%, or 50% of the intended base pairs are edited. In some embodiments, the method causes less than 19%, 18%, 16%, 14%, 12%, 10%, 8%, 6%, 4%, 2%, 1%, 0.5%, 0.2%, or less than 0.1% indel formation. In some embodiments, the ratio of intended product to unintended products at the target nucleotide is at least 2:1, 5:1, 10:1, 20:1, 30:1, 40:1, 50:1, 60:1, 70:1, 80:1, 90:1, 100:1, or 200:1, or more. In some embodiments, the ratio of intended point mutation to indel formation is greater than 1:1, 10:1, 50:1, 100:1, 500:1, or 1000:1, or more. In some embodiments, the cut single strand is hybridized to the guide nucleic acid. In some embodiments, the cut single strand is opposite to the strand comprising the first nucleobase. In some embodiments, the first base is adenine. In some embodiments, the second nucleobase is not G, C, A, or T. In some embodiments, the second base is inosine. In some embodiments, the base editor inhibits base excision repair of the edited strand. In some embodiments, the base editor protects (e.g., form base excision repair) or binds the non-edited strand. In some embodiments, the nucleobase editor comprises UGI activity. In some embodiments, the base editor comprises a catalytically inactive inosine-specific nuclease. In some embodiments, the nucleobase editor comprises nickase activity. In some embodiments, the intended edited base pair is upstream of a PAM site. In some embodiments, the intended edited base pair is 1, 2, 3, 4, 5, 6, 7, 8, 9, 10, 11, 12, 13, 14, 15, 16, 17, 18, 19, or 20 nucleotides upstream of the PAM site. In some embodiments, the intended edited basepair is downstream of a PAM site. In some embodiments, the intended edited base pair is 1, 2, 3, 4, 5, 6, 7, 8, 9, 10, 11, 12, 13, 14, 15, 16, 17, 18, 19, or 20 nucleotides downstream stream of the PAM site. In some embodiments, the method does not require a canonical (e.g., NGG) PAM site. In some embodiments, the nucleobase editor comprises a linker. In some embodiments, the linker is 1-25 amino acids in length. In some embodiments, the linker is 5-20 amino acids in length. In some embodiments, the linker is 10, 11, 12, 13, 14, 15, 16, 17, 18, 19, or 20 amino acids in length. In some embodiments, the target region comprises a target window, wherein the target window comprises the target nucleobase pair. In some embodiments, the target window comprises 1-10 nucleotides. In some embodiments, the target window is 1-9, 1-8, 1-7, 1-6, 1-5, 1-4, 1-3, 1-2, or 1 nucleotides in length. In some embodiments, the target window is 1, 2, 3, 4, 5, 6, 7, 8, 9, 10, 11, 12, 13, 14, 15, 16, 17, 18, 19, or 20 nucleotides in length. In some embodiments, the intended edited base pair occurs within the target window. In some embodiments, the target window comprises the intended edited base pair. In some embodiments, the nucleobase editor is any one of the base editors provided herein.
The instant disclosure provides methods for the treatment of a subject diagnosed with a disease associated with or caused by a point mutation that can be corrected by the editing system provided herein, e.g., spinal muscular atrophy (SMA). For example, in some embodiments, a method is provided that comprises administering to a subject having such a disease, e.g., SMA, a an effective amount of the adenosine base editor and guide RNA described herein that corrects the exon 7 point mutation of *SMN2* (*e.g.,* C840T) or modifies a flanking exon (e.g., exon 6 or exon 8) so that the resulting SMN protein product more stable *(e.g.,* is less prone to degradation). In some embodiments, a method for treating a subject is provided which further comprises administering a therapeutic agent to the subject with the adenosine base editor and guide RNA. In some embodiments, the therapeutic agent is an antisense oligonucleotide (e.g., nusinersen). In certain embodiments, the therapeutic agent is risdiplam.

### X. Base Editor Delivery

In another aspect, the present disclosure provides for the delivery of base editors *in vitro* and *in vivo* using various strategies, including on separate vectors using split inteins and as well as direct delivery strategies of the ribonucleoprotein complex (i.e., the base editor complexed to the gRNA and/or the second-site gRNA) using techniques such as electroporation, use of cationic lipid-mediated formulations, and induced endocytosis methods using receptor ligands fused to to the ribonucleoprotein complexes. Any such methods are contemplated herein.

In some aspects, the invention provides methods comprising delivering one or more base editor-encoding polynucleotides, such as or one or more vectors as described herein encoding one or more components of the base editing system described herein, one or more transcripts thereof, and/or one or proteins transcribed therefrom, to a host cell. In some aspects, the invention further provides cells produced by such methods, and organisms (such as animals, plants, or fungi) comprising or produced from such cells. In some embodiments, a base editor as described herein in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a base editor to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Bihm (eds) (1995); and Yu et al., Gene Therapy 1:13-26 (1994).

Methods of non-viral delivery of nucleic acids include lipofection, nucleofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam^{™} and Lipofectin^{™}). Cationic and neutral lipids that are suitable for efficient receptor-recognition lipofection of polynucleotides include those of Feigner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (*in vivo*) or they can be used to treat cells in vitro, and the modified cells may optionally be administered to patients (*ex vivo*)*.* Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a viruses can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700). In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producing a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also be infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Additional methods for the delivery of nucleic acids to cells are known to those skilled in the art. See, for example, US20030087817, incorporated herein by reference.

In various embodiments, the base editor constructs (including, the split-constructs) may be engineered for delivery in one or more rAAV vectors. An rAAV as related to any of the methods and compositions provided herein may be of any serotype including any derivative or pseudotype (e.g., 1, 2, 3, 4, 5, 6, 7, 8, 9, 10, 11, 12, 13, 2/1, 2/5, 2/8, 2/9, 3/1, 3/5, 3/8, or 3/9). An rAAV may comprise a genetic load (i.e., a recombinant nucleic acid vector that expresses a gene of interest, such as a whole or split base editor fusion protein that is carried by the rAAV into a cell) that is to be delivered to a cell. An rAAV may be chimeric.

As used herein, the serotype of an rAAV refers to the serotype of the capsid proteins of the recombinant virus. Non-limiting examples of derivatives and pseudotypes include rAAV2/1, rAAV2/5, rAAV2/8, rAAV2/9, AAV2-AAV3 hybrid, AAVrh.10, AAVhu.14, AAV3a/3b, AAVrh32.33, AAV-HSC15, AAV-HSC17, AAVhu.37, AAVrh.8, CHt-P6, AAV2.5, AAV6.2, AAV2i8, AAV-HSC15/17, AAVM41, AAV9.45, AAV6(Y445F/Y731F), AAV2.5T, AAV-HAE1/2, AAV clone 32/83, AAVShH10, AAV2 (Y->F), AAV8 (Y733F), AAV2.15, AAV2.4, AAVM41, and AAVr3.45. A non-limiting example of derivatives and pseudotypes that have chimeric VP1 proteins is rAAV2/5-1VP1u, which has the genome of AAV2, capsid backbone of AAV5 and VP1u of AAV1. Other non-limiting example of derivatives and pseudotypes that have chimeric VP1 proteins are rAAV2/5-8VP1u, rAAV2/9-1VP1u, and rAAV2/9-8VP1u.

AAV derivatives/pseudotypes, and methods of producing such derivatives/pseudotypes are known in the art (see, e.g., Mol Ther. 2012 Apr;20(4):699-708. doi: 10.1038/mt.2011.287. Epub 2012 Jan 24. The AAV vector toolkit: poised at the clinical crossroads. Asokan A1, Schaffer DV, Samulski RJ.). Methods for producing and using pseudotyped rAAV vectors are known in the art (see, e.g., Duan et al., J. Virol., 75:7662-7671, 2001; Halbert et al., J. Virol., 74:1524-1532, 2000; Zolotukhin et al., Methods, 28:158-167, 2002; and Auricchio et al., Hum. Molec. Genet., 10:3075-3081, 2001).

Methods of making or packaging rAAV particles are known in the art and reagents are commercially available (see, e.g., Zolotukhin et al. Production and purification of serotype 1, 2, and 5 recombinant adeno-associated viral vectors. Methods 28 (2002) 158-167; and U.S. Patent Publication Numbers US20070015238 and US20120322861, which are incorporated herein by reference; and plasmids and kits available from ATCC and Cell Biolabs, Inc.). For example, a plasmid comprising a gene of interest may be combined with one or more helper plasmids, e.g., that contain a rep gene (e.g., encoding Rep78, Rep68, Rep52 and Rep40) and a cap gene (encoding VP1, VP2, and VP3, including a modified VP2 region as described herein), and transfected into a recombinant cells such that the rAAV particle can be packaged and subsequently purified.

Recombinant AAV may comprise a nucleic acid vector, which may comprise at a minimum: (a) one or more heterologous nucleic acid regions comprising a sequence encoding a protein or polypeptide of interest or an RNA of interest (e.g., a siRNA or microRNA), and (b) one or more regions comprising inverted terminal repeat (ITR) sequences (e.g., wild-type ITR sequences or engineered ITR sequences) flanking the one or more nucleic acid regions (e.g., heterologous nucleic acid regions). Herein, heterologous nucleic acid regions comprising a sequence encoding a protein of interest or RNA of interest are referred to as genes of interest.

Any one of the rAAV particles provided herein may have capsid proteins that have amino acids of different serotypes outside of the VPlu region. In some embodiments, the serotype of the backbone of the VP1 protein is different from the serotype of the ITRs and/or the Rep gene. In some embodiments, the serotype of the backbone of the VP1 capsid protein of a particle is the same as the serotype of the ITRs. In some embodiments, the serotype of the backbone of the VP1 capsid protein of a particle is the same as the serotype of the Rep gene. In some embodiments, capsid proteins of rAAV particles comprise amino acid mutations that result in improved transduction efficiency.

In some embodiments, the nucleic acid vector comprises one or more regions comprising a sequence that facilitates expression of the nucleic acid (e.g., the heterologous nucleic acid), e.g., expression control sequences operatively linked to the nucleic acid. Numerous such sequences are known in the art. Non-limiting examples of expression control sequences include promoters, insulators, silencers, response elements, introns, enhancers, initiation sites, termination signals, and poly(A) tails. Any combination of such control sequences is contemplated herein (e.g., a promoter and an enhancer).

Final AAV constructs may incorporate a sequence encoding the gRNA. In other embodiments, the AAV constructs may incorporate a sequence encoding the second-site nicking guide RNA. In still other embodiments, the AAV constructs may incorporate a sequence encoding the second-site nicking guide RNA and a sequence encoding the gRNA.

In various embodiments, the gRNAs and the second-site nicking guide RNAs can be expressed from an appropriate promoter, such as a human U6 (hU6) promoter, a mouse U6 (mU6) promoter, or other appropriate promoter. The gRNAs and the second-site nicking guide RNAs can be driven by the same promoters or different promoters.

In some embodiments, a rAAV constructs or the herein compositions are administered to a subject enterally. In some embodiments, a rAAV constructs or the herein compositions are administered to the subject parenterally. In some embodiments, a rAAV particle or the herein compositions are administered to a subject subcutaneously, intraocularly, intravitreally, subretinally, intravenously (IV), intracerebro-ventricularly, intramuscularly, intrathecally (IT), intracisternally, intraperitoneally, via inhalation, topically, or by direct injection to one or more cells, tissues, or organs. In some embodiments, a rAAV particle or the herein compositions are administered to the subject by injection into the hepatic artery or portal vein.

In other aspects, the base editors can be divided at a split site and provided as two halves of a whole/complete base editor. The two halves can be delivered to cells (e.g., as expressed proteins or on separate expression vectors) and once in contact inside the cell, the two halves form the complete base editor through the self-splicing action of the inteins on each base editor half. Split intein sequences can be engineered into each of the halves of the encoded base editor to facilitate their transplicing inside the cell and the concomitant restoration of the complete, functioning base editor.

These split intein-based methods overcome several barriers to in vivo delivery. For example, the DNA encoding base editors is larger than the rAAV packaging limit, and so requires special solutions. One such solution is formulating the editor fused to split intein pairs that are packaged into two separate rAAV particles that, when co-delivered to a cell, reconstitute the functional editor protein. Several other special considerations to account for the unique features of base editing are described, including the optimization of second-site nicking targets and properly packaging base editors into virus vectors, including lentiviruses and rAAV.

In this aspect, the base editors can be divided at a split site and provided as two halves of a whole/complete base editor. The two halves can be delivered to cells (e.g., as expressed proteins or on separate expression vectors) and once in contact inside the cell, the two halves form the complete base editor through the self-splicing action of the inteins on each base editor half. Split intein sequences can be engineered into each of the halves of the encoded base editor to facilitate their transplicing inside the cell and the concomitant restoration of the complete, functioning base editor.

In various embodiments, the base editors may be engineered as two half proteins (i.e., a BE N-terminal half and a BE C-terminal half) by "splitting" the whole base editor as a "split site." The "split site" refers to the location of insertion of split intein sequences (i.e., the N intein and the C intein) between two adjacent amino acid residues in the base editor. More specifically, the "split site" refers to the location of dividing the whole base editor into two separate halves, wherein in each halve is fused at the split site to either the N intein or the C intein motifs. The split site can be at any suitable location in the base editor fusion protein, but preferably the split site is located at a position that allows for the formation of two half proteins which are appropriately sized for delivery (e.g., by expression vector) and wherein the inteins, which are fused to each half protein at the split site termini, are available to sufficiently interact with one another when one half protein contacts the other half protein inside the cell.

In some embodiments, the split site is located in the napDNAbp domain. In other embodiments, the split site is located in the RT domain. In other embodiments, the split site is located in a linker that joins the napDNAbp domain and the RT domain.

In various embodiments, split site design requires finding sites to split and insert an N- and C- terminal intein that are both structurally permissive for purposes of packaging the two half base editor domains into two different AAV genomes. Additionally, intein residues necessary for trans splicing can be incorporated by mutating residues at the N terminus of the C terminal extein or inserting residues that will leave an intein "scar."

In various embodiments, using SpCas9 nickase (SEQ ID NO: 29, 1368 amino acids) as an example, the split can between between any two amino acids between 1 and 1368. Preferred splits, however, will be located between the central region of the protein, e.g., from amino acids 50-1250, or from 100-1200, or from 150-1150, or from 200-1100, or from 250-1050, or from 300-1000, or from 350-950, or from 400-900, or from 450-850, or from 500-800, or from 550-750, or from 600-700 of SEQ ID NO: 29. In specific exemplary embodiments, the split site may be between 740/741, or 801/802, or 1010/1011, or 1041/1042. In other embodiments the split site may be between 1/2, 2/3, 3/4, 4/5, 5/6, 6/7, 7/8, 8/9, 9/10, 10/11, 12/13, 14/15, 15/16, 17/18, 19/20...50151...100/101...200/201...300/301... 400/401...500/501...600/601...700/701...800/801...900/901...1000/1001...1100/1101...12 00/1201...1300/1301...and 1367/1368, including all adjacent pairs of amino acid residues.

In various embodiments, the split intein sequences can be engineered by from the following intein sequences.
2-4 INTEIN: 
CLAEGTRIFDPVTGTTHRIEDVVDGRKPIHVVAAAKDGTLLARPVVSWFDQGTRDVI
GLRIAGGAIVWATPDHKVLTEYGWRAAGELRKGDRVAGPGGSGNSLALSLTADQM
VSALLDAEPPILYSEYDPTSPFSEASMMGLLTNLADRELVHMINWAKRVPGFVDLTL
HDQAHLLECAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEELRYSVIREVLPTRRARTFDL
EVEELHTLVAEGVVVHNC (SEQ ID NO: 164)
3-2 INTEIN 
CLAEGTRIFDPVTGTTHRIEDVVDGRKPIHVVAVAKDGTLLARPVVSWFDQGTRDVI
GLRIAGGAIVWATPDHKVLTEYGWRAAGELRKGDRVAGPGGSGNSLALSLTADQM
VSALLDAEPPILYSEYDPTSPFSEASMMGLLTNLADRELVHMINWAKRVPGFVDLTL
HDQAHLLECAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYTNVVPLYDLLLE
MLDAHRLHAGGSGASRVQAFADALDDKFLHDMLAEELRYSVIREVLPTRRARTFDL
EVEELHTLVAEGVVVHNC (SEQ ID NO: 165)
30R3-1 INTEIN 
CLAEGTRIFDPVTGTTHRIEDVVDGRKPIHVVAAAKDGTLLARPVVSWFDQGTRDVI
GLRIAGGATVWATPDHKVLTEYGWRAAGELRKGDRVAGPGGSGNSLALSLTADQM
VSALLDAEPPIPYSEYDPTSPFSEASMMGLLTNLADRELVHMINWAKRVPGFVDLTL
HDQAHLLECAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEGLRYSVIREVLPTRRARTFDL
EVEELHTLVAEGVVVHNC (SEQ ID NO: 166)
30R3-2 INTEIN 
CLAEGTRIFDPVTGTTHRIEDVVDGRKPIHVVAAAKDGTLLARPVVSWFDQGTRDVI
GLRIAGGATVWATPDHKVLTEYGWRAAGELRKGDRVAGPGGSGNSLALSLTADQM
VSALLDAEPPILYSEYDPTSPFSEASMMGLLTNLADRELVHMINWAKRVPGFVDLTL
HDQAHLLECAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEELRYSVIREVLPTRRARTFDL
EVEELHTLVAEGVVVHNC (SEQ ID NO: 167)
30R3-3 INTEIN 
CLAEGTRIFDPVTGTTHRIEDVVDGRKPIHVVAAAKDGTLLARPVVSWFDQGTRDVI
GLRIAGGATVWATPDHKVLTEYGWRAAGELRKGDRVAGPGGSGNSLALSLTADQM
VSALLDAEPPIPYSEYDPTSPFSEASMMGLLTNLADRELVHMINWAKRVPGFVDLTL
HDQAHLLECAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
 
MLDAHRLHAGGSGASRVQAFADALDDKFLHDMLAEELRYSVIREVLPTRRARTFDL
EVEELHTLVAEGVVVHNC (SEQ ID NO: 168)
37R3-1 INTEIN 
CLAEGTRIFDPVTGTTHRIEDVVDGRKPIHVVAAAKDGTLLARPVVSWFDQGTRDVI
GLRIAGGATVWATPDHKVLTEYGWRAAGELRKGDRVAGPGGSGNSLALSLTADQM
VSALLDAEPPILYSEYNPTSPFSEASMMGLLTNLADRELVHMINWAKRVPGFVDLTL
HDQAHLLERAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEGLRYSVIREVLPTRRARTFDL
EVEELHTLVAEGVVVHNC ((SEQ ID NO: 169)
37R3-2 INTEIN 
CLAEGTRIFDPVTGTTHRIEDVVDGRKPIHVVAAAKDGTLLARPVVSWFDQGTRDVI
GLRIAGGAIVWATPDHKVLTEYGWRAAGELRKGDRVAGPGGSGNSLALSLTADQM
VSALLDAEPPILYSEYDPTSPFSEASMMGLLTNLADRELVHMINWAKRVPGFVDLTL
HDQAHLLERAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEGLRYSVIREVLPTRRARTFDL
EVEELHTLVAEGVVVHNC (SEQ ID NO: 170)
37R3-3 INTEIN

CLAEGTRIFDPVTGTTHRIEDVVDGRKPIHVVAVAKDGTLLARPVVSWFDQGTRDVI
GLRIAGGATVWATPDHKVLTEYGWRAAGELRKGDRVAGPGGSGNSLALSLTADQM
VSALLDAEPPILYSEYDPTSPFSEASMMGLLTNLADRELVHMINWAKRVPGFVDLTL
HDQAHLLERAWLEILMIGLVWRSMEHPGKLLFAPNLLLDRNQGKCVEGMVEIFDML
LATSSRFRMMNLQGEEFVCLKSIILLNSGVYTFLSSTLKSLEEKDHIHRALDKITDTLI
HLMAKAGLTLQQQHQRLAQLLLILSHIRHMSNKGMEHLYSMKYKNVVPLYDLLLE
MLDAHRLHAGGSGASRVQAFADALDDKFLHDMLAEELRYSVIREVLPTRRARTFDL
EVEELHTLVAEGVVVHNC (SEQ ID NO: 171)

In various embodiments, the split inteins can be used to separately deliver separate portions of a complete Base editor fusion protein to a cell, which upon expression in a cell, become reconstituted as a complete Base editor fusion protein through the trans splicing.

In some embodiments, the disclosure provides a method of delivering a Base editor fusion protein to a cell, comprising: constructing a first expression vector encoding an N-terminal fragment of the Base editor fusion protein fused to a first split intein sequence; constructing a second expression vector encoding a C-terminal fragment of the Base editor fusion protein fused to a second split intein sequence; delivering the first and second expression vectors to a cell, wherein the N-terminal and C-terminal fragment are reconstituted as the Base editor fusion protein in the cell as a result of trans splicing activity causing self-excision of the first and second split intein sequences.

In other embodiments, the split site is in the napDNAbp domain.

In still other embodiments, the split site is in the adenosine deaminase domain.

In yet other embodiments, the split site is in the linker.

In other embodiments, the base editors may be delivered by ribonucleoprotein complexes.

In this aspect, the base editors may be delivered by non-viral delivery strategies involving delivery of a base editor complexed with a gRNA (i.e., a BE ribonucleoprotein complex) by various methods, including electroporation and lipid nanoparticles. Methods of non-viral delivery of nucleic acids include lipofection, nucleofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam^{™} and Lipofectin^{™}). Cationic and neutral lipids that are suitable for efficient receptor-recognition lipofection of polynucleotides include those of Feigner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

### XI. Pharmaceutical Compositions

Other aspects of the present disclosure relate to pharmaceutical compositions comprising any of the adenosine deaminases, fusion proteins, or the fusion protein-gRNA complexes described herein. The term "pharmaceutical composition", as used herein, refers to a composition formulated for pharmaceutical use. In some embodiments, the pharmaceutical composition further comprises a pharmaceutically acceptable carrier. In some embodiments, the pharmaceutical composition comprises additional agents (*e.g.* for specific delivery, increasing half-life, or other therapeutic compounds). In some embodiments, the pharmaceutical composition further comprises an additional therapeutic agent. In some embodiments, the therapeutic agent is an antisense oligonucleotide (e.g., nusinersen). In certain embodiments, the therapeutic agent is risdiplam.

As used here, the term "pharmaceutically-acceptable carrier" means a pharmaceutically-acceptable material, composition or vehicle, such as a liquid or solid filler, diluent, excipient, manufacturing aid *(e.g.,* lubricant, talc magnesium, calcium or zinc stearate, or steric acid), or solvent encapsulating material, involved in carrying or transporting the compound from one site *(e.g.,* the delivery site) of the body, to another site *(e.g.,* organ, tissue or portion of the body). A pharmaceutically acceptable carrier is "acceptable" in the sense of being compatible with the other ingredients of the formulation and not injurious to the tissue of the subject *(e.g.,* physiologically compatible, sterile, physiologic pH, *etc.*)*.* Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C2-C12 alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

In some embodiments, the pharmaceutical composition is formulated for delivery to a subject, e.g., for gene editing. Suitable routes of administrating the pharmaceutical composition described herein include, without limitation: topical, subcutaneous, transdermal, intradermal, intralesional, intraarticular, intraperitoneal, intravesical, transmucosal, gingival, intradental, intracochlear, transtympanic, intraorgan, epidural, intrathecal, intramuscular, intravenous, intravascular, intraosseus, periocular, intratumoral, intracerebral, and intracerebroventricular administration.

In some embodiments, the pharmaceutical composition described herein is administered locally to a diseased site *(e.g.,* tumor site). In some embodiments, the pharmaceutical composition described herein is administered to a subject by injection, by means of a catheter, by means of a suppository, or by means of an implant, the implant being of a porous, non-porous, or gelatinous material, including a membrane, such as a sialastic membrane, or a fiber.

In other embodiments, the pharmaceutical composition described herein is delivered in a controlled release system. In one embodiment, a pump may be used (see, *e.g.,* Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, *e.g.,* Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.). Other controlled release systems are discussed, for example, in Langer, supra.

In some embodiments, the pharmaceutical composition is formulated in accordance with routine procedures as a composition adapted for intravenous or subcutaneous administration to a subject, e.g., a human. In some embodiments, pharmaceutical compositions for administration by injection are solutions in sterile isotonic aqueous buffer. Where necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the pharmaceutical is to be administered by infusion, it can be dispensed with an infusion bottle containing sterile pharmaceutical grade water or saline. Where the pharmaceutical composition is administered by injection, an ampoule of sterile water for injection or saline can be provided so that the ingredients can be mixed prior to administration.

A pharmaceutical composition for systemic administration may be a liquid, e.g., sterile saline, lactated Ringer's or Hank's solution. In addition, the pharmaceutical composition can be in solid forms and re-dissolved or suspended immediately prior to use. Lyophilized forms are also contemplated.

A pharmaceutical composition for systemic administration may be a liquid, e.g., sterile saline, lactated Ringer's or Hank's solution. In addition, the pharmaceutical composition can be in solid forms and re-dissolved or suspended immediately prior to use. Lyophilized forms are also contemplated.

The pharmaceutical composition can be contained within a lipid particle or vesicle, such as a liposome or microcrystal, which is also suitable for parenteral administration. The particles can be of any suitable structure, such as unilamellar or plurilamellar, so long as compositions are contained therein. Compounds can be entrapped in "stabilized plasmid-lipid particles" (SPLP) containing the fusogenic lipid dioleoylphosphatidylethanolamine (DOPE), low levels (5-10 mol%) of cationic lipid, and stabilized by a polyethyleneglycol (PEG) coating (Zhang Y. P. et al., Gene Ther. 1999, 6:1438-47). Positively charged lipids such as N-[1-(2,3-dioleoyloxi)propyl]-N,N,N-trimethyl-amoniummethylsulfate, or "DOTAP," are particularly preferred for such particles and vesicles. The preparation of such lipid particles is well known. See, *e.g.,* U.S. Patent Nos. 4,880,635; 4,906,477; 4,911,928; 4,917,951; 4,920,016; and 4,921,757; each of which is incorporated herein by reference.

The pharmaceutical composition described herein may be administered or packaged as a unit dose, for example. The term "unit dose" when used in reference to a pharmaceutical composition of the present disclosure refers to physically discrete units suitable as unitary dosage for the subject, each unit containing a predetermined quantity of active material calculated to produce the desired therapeutic effect in association with the required diluent; *i.e.,* carrier, or vehicle.

Further, the pharmaceutical composition can be provided as a pharmaceutical kit comprising (a) a container containing a compound of the invention in lyophilized form and (b) a second container containing a pharmaceutically acceptable diluent (e.g., sterile water) for injection. The pharmaceutically acceptable diluent can be used for reconstitution or dilution of the lyophilized compound of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

**In** another aspect, an article of manufacture containing materials useful for the treatment of the diseases described above is included. In some embodiments, the article of manufacture comprises a container and a label. Suitable containers include, for example, bottles, vials, syringes, and test tubes. The containers may be formed from a variety of materials such as glass or plastic. In some embodiments, the container holds a composition that is effective for treating a disease described herein and may have a sterile access port. For example, the container may be an intravenous solution bag or a vial having a stopper pierceable by a hypodermic injection needle. The active agent in the composition is a compound of the invention. In some embodiments, the label on or associated with the container indicates that the composition is used for treating the disease of choice. The article of manufacture may further comprise a second container comprising a pharmaceutically-acceptable buffer, such as phosphate-buffered saline, Ringer's solution, or dextrose solution. It may further include other materials desirable from a commercial and user standpoint, including other buffers, diluents, filters, needles, syringes, and package inserts with instructions for use.

### XII. Kits, Vectors, Cells

Some aspects of this disclosure provide kits comprising a nucleic acid construct comprising a nucleotide sequence encoding a base editor, or a component thereof, including a cytidine deaminase, adenosine deaminase, or an napDNAbp, and/or a guide RNA, for editing a target DNA in a cell. In some embodiments, the nucleotide sequence encodes any of the napDNAbps, cytidine deaminases, and/or adenosine deaminases, and/or guide RNAs provided herein. In some embodiments, the nucleotide sequence comprises a heterologous promoter that drives expression of the napDNAbps, cytidine deaminases, and/or adenosine deaminases, and/or guide RNAs described herein. The nucleotide sequence may further comprise one or more heterologous promoters that drive expression of the napDNAbps, cytidine deaminases, and/or adenosine deaminases, and/or guide RNAs, either from the same nucleotide sequence or separate nucleotide sequences.

In some embodiments, the kit further comprises an expression construct encoding a guide nucleic acid backbone, e.g., a guide RNA backbone, wherein the construct comprises a cloning site positioned to allow the cloning of a nucleic acid sequence identical or complementary to a target sequence into the guide nucleic acid, e.g., guide RNA backbone.

The disclosure further provides kits comprising a nucleic acid construct, comprising (a) a nucleotide sequence encoding a napDNAbp (e.g., a Cas9 domain) fused to a deaminase, or a base editor comprising a napDNAbp (e.g., Cas9 domain) and a deaminase as provided herein; and (b) a heterologous promoter that drives expression of the sequence of (a). In some embodiments, the kit further comprises an expression construct encoding a guide nucleic acid backbone, (e.g., a guide RNA backbone), wherein the construct comprises a cloning site positioned to allow the cloning of a nucleic acid sequence identical or complementary to a target sequence into the guide nucleic acid (e.g., guide RNA backbone).

Some embodiments of this disclosure provide cells comprising any of the base editors or complexes provided herein. In some embodiments, the cells comprise nucleotide constructs that encode any of the base editors provided herein. In some embodiments, the cells comprise any of the nucleotides or vectors provided herein. In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calul, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A 172, A20, A253, A431, A-549, ALC, B16, B35, BCP-1 cells, BEAS-2B, bEnd.3, BHK-21, BR 293. BxPC3. C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK 11, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN/OPCT cell lines, Peer, PNT-1A/PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some aspects, the present disclosure provides uses of any one of the base editors described herein and a guide RNA targeting this base editor to a target A:T base pair in a nucleic acid molecule in the manufacture of a kit for nucleic acid editing, wherein the nucleic acid editing comprises contacting the nucleic acid molecule with the base editor and guide RNA under conditions suitable for the substitution of the adenine (A) of the A:T nucleobase pair with an guanine (G). In some embodiments of these uses, the nucleic acid molecule is a double-stranded DNA molecule. In some embodiments, the step of contacting of induces separation of the double-stranded DNA at a target region. In some embodiments, the step of contacting further comprises nicking one strand of the double-stranded DNA, wherein the one strand comprises an unmutated strand that comprises the T of the target A:T nucleobase pair.

In some aspects, the present disclosure provides uses of any one of the base editors described herein and a guide RNA targeting this base editor to a target A:T base pair in a nucleic acid molecule in the manufacture of a kit for evaluating the off-target effects of a base editor, wherein the step of evaluating the off-target effects comprises contacting the base editor with the nucleic acid molecule and determining off-target effects in accordance with any one of the disclosed methods. In some embodiments of these uses, the nucleic acid molecule is a double-stranded DNA molecule. In some embodiments, the step of contacting of induces separation of the double-stranded DNA at a target region. In some embodiments, the step of contacting further comprises nicking one strand of the double-stranded DNA, wherein the one strand comprises an unmutated strand that comprises the T of the target A:T nucleobase pair.

In some embodiments of the described uses, the step of contacting is performed *in vitro.* In other embodiments, the step of contacting is performed *in vivo.* In some embodiments, the step of contacting is performed in a subject (e.g., a human subject or a non-human animal subject). In some embodiments, the step of contacting is performed in a cell, such as a human or non-human animal cell.

The present disclosure also provides uses of any one of the base editors described herein as a medicament. The present disclosure also provides uses of any one of the complexes of base editors and guide RNAs described herein as a medicament.

Some aspects of this disclosure provide kits comprising a nucleic acid construct comprising a nucleotide sequence encoding an adenosine deaminase capable of deaminating an adenosine in a deoxyribonucleic acid (DNA) molecule. In some embodiments, the nucleotide sequence encodes any of the adenosine deaminases provided herein. In some embodiments, the nucleotide sequence comprises a heterologous promoter that drives expression of the adenosine deaminase.

Some aspects of this disclosure provide kits comprising a nucleic acid construct, comprising (a) a nucleotide sequence encoding a napDNAbp (e.g., a Cas9 domain) fused to an adenosine deaminase, or a fusion protein comprising a napDNAbp (e.g., Cas9 domain) and an adenosine deaminase as provided herein; and (b) a heterologous promoter that drives expression of the sequence of (a). In some embodiments, the kit further comprises an expression construct encoding a guide nucleic acid backbone, (e.g., a guide RNA backbone), wherein the construct comprises a cloning site positioned to allow the cloning of a nucleic acid sequence identical or complementary to a target sequence into the guide nucleic acid *(e.g.,* guide RNA backbone).

Some aspects of this disclosure provide cells comprising any of the adenosine deaminases, fusion proteins, or complexes provided herein. In some embodiments, the cells comprise a nucleotide that encodes any of the adenosine deaminases or fusion proteins provided herein. In some embodiments, the cells comprise any of the nucleotides or vectors provided herein.

The description of exemplary embodiments of the systems described above is provided for illustration purposes only and not meant to be limiting. Additional systems, e.g., variations of the exemplary systems described in detail above, are also embraced by this disclosure.

It should be appreciated however, that additional fusion proteins would be apparent to the skilled artisan based on the present disclosure and knowledge in the art.

The function and advantage of these and other embodiments of the present invention will be more fully understood from the Examples below. The following Examples are intended to illustrate the benefits of the present invention and to describe particular embodiments, but are not intended to exemplify the full scope of the invention. Accordingly, it will be understood that the Examples are not meant to limit the scope of the invention.

### EXAMPLES

Spinal Muscular Atrophy (SMA) is a progressive motor neuron degeneration disorder that occurs as a result of insufficient survival motor neuron (SMN) protein in spinal motor neurons which leads to atrophy of skeletal muscle and paralysis of the patient. The disease typically involves the status of two SMN-encoding genes, namely, the telomeric *SMN1* gene and the almost identical centromeric copy, the *SMN2* gene. In SMA, the *SMN1* gene is not present due to homozygous deletion (see Cho et al., "A degron created by SMN2 exon 7 skipping is a principle contributor to spinal muscular atrophy severity," Genes & Development, vol. 24: pp. 438-442, which is incorporated herein by reference). Thus, SMA patients do not typically express SMN1 protein. The centromeric *SMN2* gene partially rescues the deleted *SMN1* gene but the level of rescue is insufficient because of the presence of a single nucleotide mutation in the splice acceptor site at position 6 within exon 7 that results in the frequent skipping (i.e., ~ 80%) of exon 7 during *SMN2* post-transcriptional processing (a C-to-T substitution at position 6 of exon 7). Thus, while a small amount of full-size SMN protein is produced from the *SMN2* gene, the majority of the *SMN2* gene product is truncated in the region corresponding to exon 7. In addition, the defective *SMN2* gene product also acquires four amino acids, EMLA (SEQ ID NO: 466) , encoded by exon 8 as a new C-terminus of the protein ("the EMLA (SEQ ID NO: 466) tail"). This defective gene product is often referred to as the SMNδ7 product.

While the SMNδ7 product bears the same function as SMN1, although somewhat diminished, it is rapidly degraded due to the appearance of at least two degron signals formed at the C-terminus of the SMN protein as a result of the exon-7 skipping event. Specifically, it has been reported that the truncated SMNδ7 product signals its own cellular degradation by the proteasome complex due to the presence of (1) the C-terminal portion of the region encoded by exon 6 and (2) the 4-amino acid region encoded by exon 8 (i.e., the EMLA (SEQ ID NO: 466) -tail), both of which function as degron signals in the absence of the exon 7-encoded region^{8, 9}.

The Examples below describe the design and use of various exemplary base editors (BEs) that are capable of installing precise nucleobase changes in the *SMN2* genomic locus which lead to the production of a modified SMN2 protein that avoids or limits proteasome-dependent degradation and retains SMN1-compensatory function. For example, the base editors described herein may be used to eliminate and/or modify one or more degrons in the natually occurring truncated SMNδ7 product to produce a modified SMN2 product having greater stability. For example, such BE-induced modifications can include, but are not limited to, (1) the removal and/or modification of the C-terminal portion of the region encoded by exon 6 and (2) removal and/or modification of the 4-amino acid region encoded by exon 8 (i.e., the EMLA (SEQ ID NO: 466) -tail) so as to remove or limit their degron activity.

The modified SMN2 polypeptides are less susceptible to cellular degradation, and thus, show an improved ability to rescue the loss of SMN1 function, which is the hallmark of SMA.

The disclosure and Examples envision the installation of any suitable nucleobase change that can give rise to a modified SMN2 protein that is able to rescue the homozygous loss of the *SMN1* gene using base editors described herein.

The base editors embrace any type of base editor, and in particular, are exemplified herein using cytidine deaminase base editors (i.e., capable of installing a C-to-T edit) and adenosine base editors (i.e., capable of installing an A-to-G edit) to account for a variety of genetic strategies that result in the production of a modified SMN2 protein that is both stable and functional, and which is capable of rescuing the loss of SMN1 function. Such genetic changes are permanent since they are at the level of genomic change, as opposed to a more transient effect of the use of antisense oligonucleotides (ASO) (e.g., nusinersen, approved in the U.S. as SPINRAZA^{®} in 2016), which are only capable of transiently repairing exon 7 splicing in an *SMN2* mRNA transcript.

### EXAMPLE 1: Base editing to address Spinal Muscular Atrophy (SMA)

Spinal Muscular Atrophy (SMA) is a progressive motor neuron degeneration disorder that SMA occurs as a result of insufficient survival motor neuron (SMN) protein in spinal motor neurons that leads to atrophy of skeletal muscle, and paralysis of the patient.

The decrease in SMN protein is often a direct result of homozygous loss by deletion of the telomeric *SMN1* gene that is responsible for encoding stable full-length SMN protein (FL-SMN) transcripts.¹ As a result, cells rely on the nearly identical centromeric *SMN2* gene that contains a single SNP at position 6 of the final exon 7 from a C to T, that results in masking of the acceptor splice site.⁴ The SMN2 pre-mRNA transcript undergoes frequent skipping of exon 7, producing a truncated SMNδ7 mRNA transcript where exon 6 is directly followed by the exon 8 3'-UTR that encodes four amino acids, EMLA (SEQ ID NO: 466) (the EMLA (SEQ ID NO: 466) -tail). Though the amino acids encoded by exon 7 have been lost, the functionality of SMN1 is preserved in the SMNδ7 truncated gene product of *SMN2,* thereby allowing the *SMN2* gene to partially compensate for the loss of *SMN1.* As such, *SMN2* copy numbers are a strong determinate in patients' symptomatic outcome.⁵⁻⁷ Nevertheless, both the EMLA (SEQ ID NO: 466) -tail and the now more exposed exon 6 C-terminal amino acid chain independently signal for rapid protein degradation by the proteasome complex,^{8, 9} and together they prompt reduction of SMN protein to levels insufficient for cell survival.

Here, base editing strategies to target the *SMN2* genomic locus for point mutations that affect SMN protein stability, e.g., by correcting the C-to-T transition mutation in position 6 of exon 7 (i.e., the SMA classic disease mutation) or installing another one or more nucleobase edits which have the effect of removing or inactivating a degron, such as the C-terminal portion of the region encoded by exon 6 or the 4-amino acid region encoded by exon 8 (i.e., the EMLA (SEQ ID NO: 466) -tail) so as to remove or limit their degron activity of the disease SNP or otherwise are using adenine base editor (ABE) constructs, are described.

### Repair of C840T in Exon 7

As noted above, *SMN2* comprises C840T at position 6 of exon 7 from a C to T that masks the acceptor splice site. The genomic sequence of the *SMN2* exon 7 is presented below (the C→T mutation is bolded and underlined; capitalization represents the exon):

Gtgaaacaaaatgctttttaacatccatataaagctatctatatatagctatctatatctatatagctattttttttaacttcctttattttccttaca
gGGTTTTAGACAAAATCAAAAAGAAGGAAGGTGCTCACATTCCTTAAATTAAggag
taagtctgccagcattatgaaagtgaatcttacttttgtaaaactttatggtttgtggaaaacaaatgtttttgaacatttaaaaagttcagatgt
(SEQ ID NO: 363)

To repair C840T, different base editing strategies were developed. The SNP was successfully corrected using a circular permutant (CP) adenine base editor.²¹ Canonical ABE-editing occurs between positions 4-8. Transfection experiments in mouse embryonic stem cells (mESCs) derived from the SMA mouse model (Jackson Laboratories 005025) at the human *SMN2* transgene were performed. Pairing of the adenine-deaminase with the circular permutant 1040 (CP₁₀₄₁) ABE allowed for higher-position editing at greater efficiency, as shown in Figure 1. This strategy can alternatively be paired with wildtype Cas9 or other CP variants. In addition, a split-intein version of the CP-ABE was created to allow for packaging and delivery by AAV. As shown in Figure 1, the split-intein version also showed Exon 7 editing. Exemplary sgRNA sequences are provided below:
CP₁₀₄₁: 5' - ATTTTGTCTAAAACCctgta - 3' (SEQ ID NO: 364)(5' - AUUUUGUCUAAAACCcUgUa - 3' (SEQ ID NO: 365))
   (PAM: agg; SpCas9)
5' - TTTGTCTAAAACCctgtaag- 3' (SEQ ID NO: 366) (5' - UUUGUCUAAAACCcUgUaag- 3' (SEQ ID NO: 367))
   (PAM: gaaaat; saCas9-KKH)
5' - TTTTGTCTAAAACCctgtaa- 3' (SEQ ID NO: 368) (5' - UUUUGUCUAAAACCcUgUaa- 3' (SEQ ID NO: 369))
   (PAM: gga; Cas9-VQR, Cas9-VRQR, Cas9-VRER, Cas9-NG)
5' - TGATTTTGTCTAAAACCC - 3' (SEQ ID NO: 370) (5' - UGAUUUUGUCUAAAACCC - 3' (SEQ ID NO: 371))
   (PAM: TTTT; CPF1)
5' - GATTTTGTCTAAAACCCT - 3' (SEQ ID NO: 372) (5' - GAUUUUGUCUAAAACCCU - 3' (SEQ ID NO: 373))
   (PAM: TTTT; CPF1)
5' - ATTTTGTCTAAAACCCTG - 3' (SEQ ID NO: 374) (5' - AUUUUGUCUAAAACCCUG - 3' (SEQ ID NO: 375))
   (PAM: GTTT; CPF1)
5' - GTCTAAAACCCTGTAAGG- 3' (SEQ ID NO: 376) (5' - GUCUAAAACCCUGUAAGG- 3' (SEQ ID NO: 377))
   (PAM: TTTT; CPF1)
5' - TCTAAAACCCTGTAAGGA- 3' (SEQ ID NO: 378) (5' - UCUAAAACCCUGUAAGGA- 3' (SEQ ID NO: 379))
   (PAM: GTTT; CPF1)

### Improved Stability of SMN Protein Products (Exon 8)

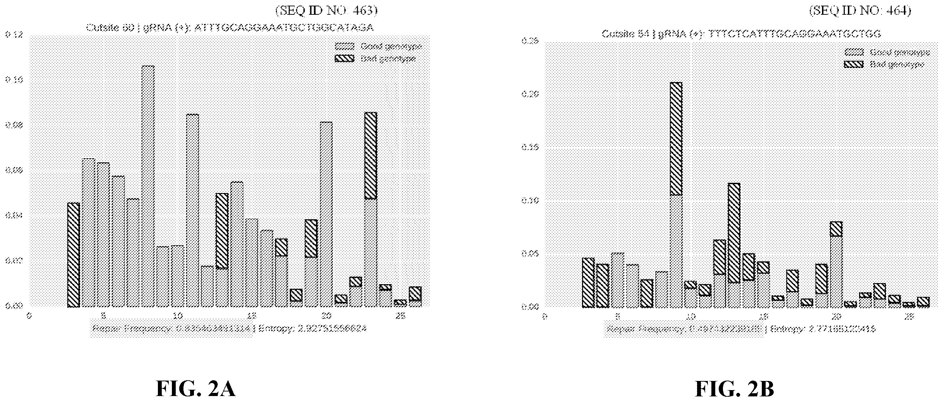

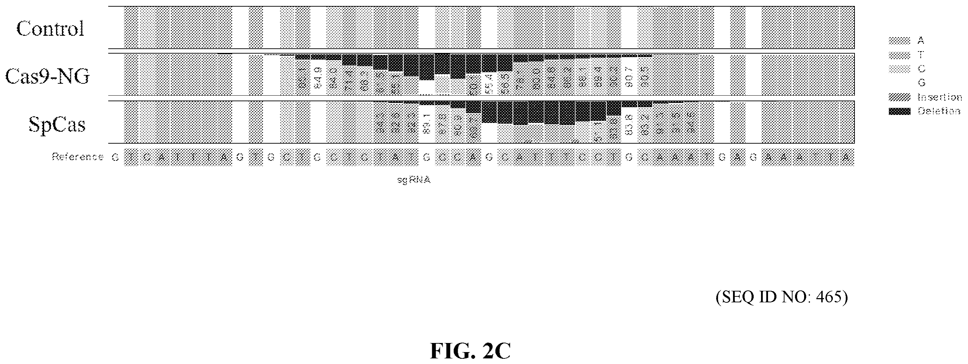

Using the inDelphi Cas9 outcome predictive algorithm²² sgRNAs that were predicted facilitate SpCas9 cutting of the start of exon 8, were found. These sgRNAs were expected to generate allelic outcomes that would result in more stable SMN protein products (Figures 2A, 2B). Transfection experiments in mouse embryonic stem cells (mESCs) derived from the SMA mouse model (Jackson Laboratories 005025) at the human *SMN2* transgene were performed (Figure 2C). The designed sgRNAs fit the criteria that the majority of products do not disturb the splice junction and allow for 5< codons before a STOP codon is reached,⁹ either destroying the 5th codon STOP sequence, inducing frameshifts, or combinations thereof. sgRNA targeting with Cas9-NG enables high-efficiency editing; however, this strategy can alternatively be paired with other Cas9 variants that can recognize "NGA"-PAM sequences. The exon 8 *SNM2* genomic sequence is provided below. The sequence of the exon is capitalized; the stop codes are bolded.

Ctctggttctaatttctcatttgcag GAAATGCTGGCATAGAGCAGCACTAAATGACACCACTAA
AGAAACGATCA (SEQ ID NO: 380)

Exemplary sgRNA sequences that modify exon 8 nuclease are provided below:
5' - TTTGCAGGAAATGCTGGCAT- 3' (SEQ ID NO: 381) (5' - UUUGCAGGAAAUGCUGGCAU- 3' (SEQ ID NO: 382))
   (PAM: aga; Cas9-VQR, Cas9-VRQR, Cas9-VRER, Cas9-NG)
5' - TTCTCATTTGCAGGAAATGC- 3' (SEQ ID NO: 383) (5' - UUCUCAUUUGCAGGAAAUGC- 3' (SEQ ID NO: 384))
   (PAM: tgg; SpCas9)
5' - CATTTAGTGCTGCTCTATGC- 3' (SEQ ID NO: 385) (5' - CAUUUAGUGCUGCUCUAUGC- 3' (SEQ ID NO: 386))
   (PAM: CAG; SpCas9)
5' - CAGGAAATGCTGGCATAGAG- 3' (SEQ ID NO: 387) (5' - CAGGAAAUGCUGGCAUAGAG- 3' (SEQ ID NO: 388))
   (PAM: CAG; SpCas9)
5' - TTGCAGGAAATGCTGGCATA- 3' (SEQ ID NO: 389) (5' - UUGCAGGAAAUGCUGGCAUA- 3' (SEQ ID NO: 390))
   (PAM: GAG; SpCas9)
5' - ATTTGCAGGAAATGCTGGCA - 3' (SEQ ID NO: 391) (5' - AUUUGCAGGAAAUGCUGGCA - 3' (SEQ ID NO: 392))
   (PAM: **TAG;** SpCas9)

An exemplary sequence for exon 8 base editing modification is given below. In this approach, adenine deamination of the 5^{th} codon stop sequence facilitates the addition of 5 amino acids at the C-terminal of the translated protein (e.g., stabilizes the translated protein).

5' - TGGCA**TAG**AGCAGCACTAAA - 3' (SEQ ID NO: 393) (5' - UGGCA**UAG**AGCAGCACUAAA - 3' (SEQ ID NO: 394))
(PAM: TGA; Cas9-VQR, Cas9-VRQR, Cas9-VRER, Cas9-NG)

### Improved Stability of SMN Protein Products (Exon 6)

Modification of the S270 amino acid of SMN2 derived proteins has previously been shown to slow the rate of protein degradation.⁹ An ABE was designed to target the S270 amino acid codon of exon 6, decreasing the rate of SMA protein degradation.

The *SMN2* exon 6 genomic sequence is provided below. The sequence of the exon is capitalized and the S270 codon is bolded.

CTTTGGGAAGTATGTTAATTTCATGGTACATGAGTGGCTATCATACTGGCTATTA
TATGgtaagtaatcactcagcatcttttcctgacaatttttttgtagttatgtgactttgttttgtaaattt (SEQ ID NO: 395)

An exemplary sequence for ABE-mediated codon-switching in order to increase the stability of SMN2-derived proteins is provided below.

5' - TACATGAGTGGCTATCATAC - 3' (SEQ ID NO: 396) (5' - UACAUGAGUGGCUAUCAUAC - 3' (SEQ ID NO: 397))
(PAM: TGG; SpCas9)

### EXAMPLE 2: Base editing to address Spinal Muscular Atrophy (SMA)

Spinal Muscular Atrophy (SMA) is a progressive motor neuron degeneration disorder that arises in infants, and it is the leading hereditary childhood disease to result in fatality [1,2]. SMA occurs as a result of insufficient survival motor neuron (SMN) protein in spinal motor neurons that leads to atrophy of skeletal muscle, and paralysis of the patient. The SMN-complex is responsible for the generation of snRNPs, and as such SMN plays an important role in pre-mRNA processing [3] however, the comprehensive molecular cascade leading to cellular degeneration initiated by SMN loss has not yet been elucidated.

The decrease in SMN protein is often a direct result of homozygous loss by deletion of the telomeric SMN1 gene that is responsible for encoding stable full-length SMN protein (FL-SMN) transcripts [1]. As a result, cells rely on the nearly identical centromeric SMN2 gene that contains a single SNP at position 6 of the final exon 7 from a C to T, that results in masking of the acceptor splice site [4]. The SMN2 pre-mRNA transcript undergoes frequent skipping of exon 7, producing a truncated SMNδ7 mRNA transcript where exon 6 is directly followed by the exon 8 3'-UTR that encodes four amino acids, EMLA (SEQ ID NO: 466) . Though the 27 amino acids encoded by exon 7 have been lost, the functionality of FL-SMN is preserved in SMNδ7, thereby allowing SMN2 to partially compensate for the loss of SMN1. As such, SMN2 copy numbers are a strong determinate in patients' symptomatic outcome [5-7]. Nevertheless, both the EMLA (SEQ ID NO: 466) -tail and the now more exposed exon 6 C-terminal amino acid- chain independently signal for rapid protein degradation by the proteasome complex [8,9], and together they prompt reduction of SMN protein to levels insufficient for cell survival.

The genetics of SMA are well understood and lend well to genetic therapy strategies. Antisense oligonucleotides (ASO) encoding short sequences that target SMN2 exon 7 splicing regulatory sites aid in the recruitment of the splicing machinery to the acceptor splice site to create corrected SMN mRNA transcripts that no longer translate into unstable proteins [10,11]. While this method has proved effective in upregulating SMN even in vivo in mouse models of SMA, the post-transcriptional modification of ASOs is only transient. Genetic modification that corrects SMN protein stability would lead to a lasting cure for SMA once accomplished.

The recent dawning of targeted CRISPR/Cas9 mediated gene editing technologies has accelerated the course of clinical gene therapy research, and a great deal of focus has gone towards the development of genomic modification strategies as medical treatments for genetic disorders [12-16]. Most commonly, SMN2 transcript correction relies on the reversal of the early exon 7 SNP to that of SMN1. Permanent correction of SMN2 can be achieved through the inefficient process of homology directed repair (HDR) after induction of a DNA double stranded break at the genomic site of the exon 7 SNP [17], however, while CRISPR/Cas9 mediated HR can be performed at high efficiency in cell culture of rapidly dividing cells [18] it is not known if post-mitotic neurons are capable of HDR to any degree [19]. Base editing enzymes coupled to CRISPR/Cas9 allow for the specific conversion of one nucleotide to another in a site directed manner and are therefore uniquely suited to treat SNP based monogenic disorders [20].

To this end, we are devising base editing strategies to target the SMN2 genomic locus for point mutations that affect SMN protein stability. Below, we list sgRNAs targeting CRISPR/Cas9 base editing protein complexes to SMN2 genomic sites of interest.

### References for Example 2:

1. Lefebvre et al. Cell 80, 155-65 (1995)
2. Pearn et al. Lancet 1, 919-922 (1980)
3. Schrank et al. PNAS 94, 9920-5 (1997)
4. Cartegni et al. Am J Hum Genet 78, 63-77 (2006)
5. Wirth et al. Hum Genet 119, 422-8 (2006
6. Talbot et al. Semin Neurol 21 (189-197 (2001)
7. Murray et al. Hum Mol Genet 17, 949-62 (2008)
8. Chang et al. Neurochem Int 45, 1107-12 (2004)
9. Cho et al. Genes Dev 24, 438-42 (2010)
10. Sing et al. Mol Cell Biol 26, 1333-46 (2006)
11. Porensky et al. Hum Mol Genet 21, 1625-38 (2012)
12. Cong et al. Science 339, 819-23 (2013)
13. Ran et al. Nature 520, 186-90 (2015)
14. Long et al. Science 5725 (2015)
15. Tabebordbar et al. Science 5177, 1-9 (2015)
16. Nelson et al. Science 5143m 1-8 (2015)
17. Corti et al. Sci Transl Med 4, 165ra162 (2012)
18. Arbab et al. Stem Cell Reports 5, 1-10 (2015)
19. Iyama et al. SNA Repair (Amst). 12, 620-626 (2013)
20. Komor et al. Science 533, 420-424 (2016)

### Results

Design of sgRNA's to target base editing enzymes to the genomic region of intron 6 to exon 8 of the human SMN2 gene, as a means of upregulating the level of SMN protein.

### A. Genomic region surrounding exon 7:

| | | | Exon in CAPS | | C->T mutation in **red** | | | Regions affecting Exon 7 splicing are underlined | | | |
|---|---|---|---|---|---|---|---|---|---|---|---|
| gtgaaacaaaatgctttttaacatccatataaagctatctatatatagctatctatatctatatagctattttttttaacttcctttattttccttacag GGTTTTAGACAAAATCAAAAAGAAGGAAGGTGCTCACATTCCTTAAATTAAggagt aagtctgccagcattatgaaagtgaatcttacttttgtaaaactttatggtttgtggaaaacaaatgtttttgaacatttaaaaagttcagatgt (SEQ ID NO: 363) | | | | | | | | | | | |
| **1.** PAM: | | **agg** | (NGG) | | Species: | | spCas^{WT} | | | | |
| sgRNA: | | 5' - G AAACCctgtaaggaaaataa-3' (SEQ ID NO: 414) (Rv) | | | | | | | | | |
| Site 1 | Editor: | C to T | | Base: | ⁻¹G | Position : | | 5 | Notes: | | |
| Site 2 | Editor: | C to T | | Base: | ¹G | Position : | | 6 | Notes: | | |
| **2.** PAM: | | ***ga**aaat* (NNNRRT) | | | | Species: | | saCas^{mut} | | | |
| sgRNA: | | 5' - G TTTGTCTAAAACCctgtaag-3' (SEQ ID NO: 415) (Rv) | | | | | | | | | |
| Site 1 | Editor: | C to T | | Base: | ⁸G | | Position: | | 6 | Notes: | SAME As SG3, BELOW. |
| **3.** PAM: | | **gga** | (NGA) | | Species: | | spCas^{mut1} | | | | |
| sgRNA: | | 5' - GTTTTGTCTAAAACCctgtaa-3' (SEQ ID NO: 416) (Rv) | | | | | | | | | |
| Site 1 | Editor: | C to T | | | Base: | ⁸G | Position: | | 7 | Notes: | SAME AS SG2, ABOVE. |
| **4.** PAM: | | **TGA** | (NGA) | | Species: | | spCas^{mut1} | | | | |
| sgRNA: | | 5' - GTGAGCACCTTCCTTCTTTT-3' (SEQ ID NO: 417) (Rv) | | | | | | | | | |
| Site 1 | Editor: | C to T | | Base: | ³⁰G | Position: | | 8 | Notes: | | |
| Site 2 | Editor: | C to T | | Base: | ³²G | Position: | | 6 | Notes: | | |
| Site 3 | Editor: | A to G | | Base: | ³⁴T | Position: | | 4 | Notes: | | |
| **5.** PAM: | | *TT**TGA**T* | | (NNNRRT) | | | Species : | | | | |
| sgRNA: | | 5' - GATGTGAGCACCTTCCTTCTT-3'(SEQ ID NO: 418) (Rv) | | | | | | | | | |
| Site 1 | Editor: | C to T | | Base: | ³²G | Position: | | 8 | Notes: | | |
| Site 2 | Editor: | A to G | | Base: | ³⁴T | Position: | | 6 | Notes: | | |
| **6.** PAM: | | **TGA** | | | | | spCas^{mut1} | | | | |
| sgRNA: | | 5' - GactccTTAATTTAAGGAATG -3'(SEQ ID NO: 419) (Rv) | | | | | | | | | |
| Site 1 | Editor: | C to T | | | Base: | ⁵⁰T | Position : | | 7 Notes: | | |
| **7.** PAM: | | ***AGG**AAT* | | (NNNRRT) | | | Species : | | saCas^{mut} | | |
| sgRNA: | | 5' - G cagacttactccTTAATTTA-3' (SEQ ID NO: 420) (Rv) | | | | | | | | | |
| Site 1 | Editor: | C to T | | Base: | ⁶⁰G | Position: | | 5 | Notes: | | |
| Site 2 | Editor: | A to G | | Base: | ⁶¹T | Position: | | 4 | Notes: SAME AS SG8, BELOW | | |
| **8.** PAM: | | ***AGG*** | (NGG) | | Species: | | spCas^{WT} | | | | |
| sgRNA: | | 5' - G cagacttactccTTAATTTA-3' (SEQ ID NO: 421) (Rv) | | | | | | | | | |
| Site 1 | Editor: | C to T | | Base: | ⁱ⁵G | Position: | | 5 | Notes: | | |
| Site 2 | Editor: | A to G | | Base: | ⁱ⁶T | Position: | | 4 | Notes: SAME AS SG7, ABOVE | | |
| **9.** PAM: | | *CTTAAT* | | (NNNRRT) | | | Species : | | saCas^{mut} | | |
| sgRNA: | | 5' - G taatgctggcagacttactc-3' (SEQ ID NO: 422) (Rv) | | | | | | | | | |
| Site 1 | Editor: | C to T | | Base: | ⁱ¹³G | Position: | | 6 | Notes: | | |
| **10.** PAM: | | **aga** | (NGA) | | Species: | | spCas | | | | |
| sgRNA: | | 5' - G ttcactttcataatgctggc-3' (SEQ ID NO: 423) (Rv) | | | | | | | | | |
| Site 1 | Editor: | C to T | | Base: | ⁱ²⁴G | Position: | | 5 | Notes: | | |
| Site 2 | Editor: | A to G | | Base: | ⁱ²⁵T | Position: | | 4 | Notes: | | |
| **11.** PAM: | | **tgg** | (NGG) | | Species: | | spCas^{WT} | | | | |
| sgRNA: | | 5' - G (SEQ ID NO: 424) (Rv) | | | | | | | | | |
| Site 1 | Editor: | C to T | | Base: | ⁱ²⁶G | Position: | | 7 | Notes: | | |
| Site 2 | Editor: | A to G | | Base: | ⁱ²⁹T | Position: | | 4 | Notes: | | |
| **12.** PAM: | | *cataat* | (NNNRRT) | | | Secies: | | saCas^{mut} | | | |
| sgRNA: | | 5' - G acaaaagtaagattcacttt-3' (SEQ ID NO: 425) (Rv) | | | | | | | | | |
| Site 1 | Editor: | A to G | | | Base: | ⁱ³⁶T | Position: | | 5 Notes: | | |
| Site 2 | Editor: | A to G | | Base: | ⁱ³⁷T | Position: | | 4 | Notes: | | |
| **13.** PAM: | | **agG** | (NGG) | | Species: | | spCas^{WT} | | | | |
| sgRNA: | | 5' - G acttcctttattttccttac-3' (SEQ ID NO: 426) (Fw) | | | | | | | | | |
| Site 1 | Editor: | C to T | | Base: | ⁻¹⁸C | Position: | | 5 | Notes: | | |
| Site 2 | Editor: | C to T | | Base: | ⁻¹⁷C | Position: | | 6 | Notes: | | |
| **14.** PAM: | | *c**agG**GT* | | (NNNRRT) | | | Species | | saCas^{mut} | | |
| sgRNA: | | 5' - G aacttcctttattttcctta-3' (SEQ ID NO: 427) (Fw) | | | | | | | | | |
| Site 1 | Editor: | C to T | | Base: | ⁻¹⁸C | Position: | | 6 | Notes: | | |
| Site 2 | Editor: | C to T | | Base: | ⁻¹⁷C | Position: | | 7 | Notes: | | |
| **15.** PAM: | | *CAAAAT* | | (NNNRRT) | | | Species: | | saCas^{mut} | | |
| sgRNA: | | 5' - G tttccttacagGGTTTTAGA-3' (SEQ ID NO: 428) (Fw) | | | | | | | | | |
| Site 1 | Editor: | C to T | | Base: | ⁻⁷C | Position: | | 5 | Notes: | | |
| **16.** PAM: | | **AGG** | (NGG) | | Species: | | spCas^{WT} | | | | |
| sgRNA: | | 5' - G TT**T**AGACAAAATCAAAAAGA-3' (SEQ ID NO: 429) | | | | | | | | | |
| | (Fw) | | | | | | | | | | |
| Site 1 | Editor: | A to G | | Base: | ⁷A | Position: | | 4 | Notes: | | |
| Site 2 | Editor: | A to G | | Base: | ⁹A | Position: | | 6 | Notes: | | |
| Site 3 | Editor: | C to T | | Base: | ¹⁰C | Position: | | 7 | Notes: | | |
| **17.** PAM: | | **GGA** | (NGA) | | Species | | spCas^{WT} | | | | |
| sgRNA: | | 5' - G T**T**AGACAAAATCAAAAAGAA-3' (SEQ ID NO: 430) | | | | | | | | | |
| | (Fw) | | | | | | | | | | |
| Site 1 | Editor: | A to G | | Base: | ⁹A | Position: | | 5 | Notes: | | |
| Site 2 | Editor: | C to T | | Base: | ¹⁰C | Position: | | 6 | Notes: | | |
| **18.** PAM: | | *GA**AGG**T* | | (NNNRRT) | | | Species: | | saCas^{mut} | | |
| sgRNA: | | 5' - G **T**AGACAAAATCAAAAAGAAG-3' (SEQ ID NO: 431) | | | | | | | | | |
| | (Fw) | | | | | | | | | | |
| Site 1 | Editor: | A to G | | Base: | ⁹A | Position: | | 4 | Notes: | | |
| Site 2 | Editor: | C to T | | Base: | ¹⁰C | Position: | | 5 | Notes: | | |
| Site 3 | Editor: | A to G | | Base: | ¹¹A | Position: | | 6 | Notes: | | |
| **19.** PAM: | | **AGG** | (NGG) | | Species: | | spCas^{WT} | | | | |
| sgRNA: | | 5' - GACAAAATCAAAAAGAAGGA-3' (SEQ ID NO: 432) (Fw) | | | | | | | | | |
| Site 1 | Editor: | A to G | | Base: | ¹¹A | Position: | | 4 | Notes: | | |
| Site 2 | Editor: | A to G | | Base: | ¹²A | Position: | | 5 | Notes: | | |
| Site 3 | Editor: | A to G | | Base: | ¹³A | Position: | | 6 | Notes: | | |
| **20.** PAM: | | *TTAAAT* | | (NNNRRT) | | | Species: | | saCas^{mut} | | |
| sgRNA: | | 5' - G AAGGAAGGTGCTCACATTCC-3' (SEQ ID NO: 433) | | | | | | | | | |
| | (Fw) | | | | | | | | | | |
| Site 1 | Editor: | A to G | | Base: | ²⁷A | Position: | | 4 | Notes: | | |
| Site 2 | Editor: | A to G | | Base: | ²⁸A | Position: | | 5 | Notes: | | |
| **21.** PAM: | | **Agg** | (NGG) | | Species: | | spCas^{WT} | | | | |
| sgRNA: | | 5' - G TGCT**CAC**ATTCCTTAAA TTA-3' (SEQ ID NO: 434) (Fw) | | | | | | | | | |
| Site 1 | Editor: | C to T | | Base: | ³⁵C | Position: | | 5 | Notes: | | |
| Site 2 | Editor: | A to G | | Base: | ³⁶A | Position: | | 6 | Notes: | | |
| Site 3 | Editor: | C to T | | Base: | ³⁷C | Position: | | 7 | Notes: | SAME AS SG23, | |
| BELOW. OVERLAPWITH SG22 | | | | | | | | | | | |
| **22.** PAM: | | **gga** | (NGA) | | Species | | spCas^{mut} | | | | |
| sgRNA: | | 5' - *GT*GCTCACATTCC*TTAAAT*T-3-3' (SEQ ID NO: 435) (Fw) | | | | | | | | | |
| Site 1 | Editor: | C to T | | Base: | ³⁵C | Position: | | 6 | Notes: | | |
| Site 2 | Editor: | C to T | | Base: | ³⁷C | Position: | | 8 | Notes: | OVERLAP WITH 21 AND 23 | |
| **23.** PAM: | | *Aggagt* (NNGRRT) | | | | Species: | | spCas^{WT} | | | |
| sgRNA: | | 5' - G TGCTCACATTCCTTAAATTA-3' (SEQ ID NO: 436) (Fw) | | | | | | | | | |
| Site 1 | Editor: | C to T | | Base: | ³⁵C | Position: | | 5 | Notes: | | |
| Site 2 | Editor: | A to G | | Base: | ³⁶A | Position: | | 6 | Notes: | | |
| Site 3 | Editor: | C to T WITH SG22 | | Base: | ³⁷C | Position: | | 7 | Notes: | SAME AS SG21, ABOVE. OVERLAP | |
| **24.** PAM: | | *gtaagt* (NNNRRT) | | | | Species: | | saCas^{mut} | | | |
| sgRNA: | | 5' - G CACATTC*CTTAAATTAAgga*-3' (SEQ ID NO: 437) (Fw) | | | | | | | | | |
| Site 1 | Editor: | A to G | | Base: | ³⁸A | Position: | | 4 | Notes: | | |
| Site 2 | Editor: | C to T | | Base: | ⁴¹C | Position: | | 7 | Notes: | | |
| **25.** PAM: | | **tga** | (NGA) | | Species: | | spCas^{mut} | | | | |
| sgRNA: | | 5' - gagtaagtctgccagcatta-3' (SEQ ID NO: 438) (Fw) | | | | | | | | | |
| Site 1 | Editor: | A to G | | Base: | ⁱ³A | Position: | | 5 | Notes: | | |
| Site 2 | Editor: | A to G | | Base: | ⁱ⁴A | Position: | | 6 | Notes: | | |
| **26.** PAM: | | *gaaagt* | (NNNRRT) | | | Species: | | saCas^{mut} | | | |
| sgRNA: | | 5' - agtaagtctgccagcattat-3' (SEQ ID NO: 439) (Fw) | | | | | | | | | |
| Site 1 | Editor: | A to G | | Base: | ⁱ³A | Position: | | 4 | Notes: | | |
| Site 2 | Editor: | A to G | | Base: | ⁱ⁴A | Position: | | 5 | Notes: | | |
| Site 3 | Editor: | C to T | | Base: | ⁱ⁷C | Position: | | 8 | Notes: | | |
| **27.** PAM: | | **tga** | (NGA) | | Species: | | spCas^{mut} | | | | |
| sgRNA: | | 5' - gtctgccagcattatgaaag-3' (SEQ ID NO: 440) (Fw) | | | | | | | | | |
| Site 1 | Editor: | C to T | | Base: | ⁱ¹⁰C | Position: | | 6 | Notes: | | |
| Site 2 | Editor: | C to T | | Base: | ⁱ¹¹C | Position: | | 7 | Notes: | SAME AS SG28 | |
| **28.** PAM: | | *gtgaat* | (NNNRRT) | | Species: | | saCas^{mut} | | | | |
| sgRNA: | | 5' - G agtctgccagcattatgaaa-3' (SEQ ID NO: 441) (Fw) | | | | | | | | | |
| Site 1 | Editor: | C to T | | Base: | ⁱ¹⁰C | Position: | | 7 | Notes: | | |
| Site 2 | Editor: | C to T | | Base: | ⁱ¹¹C | Position: | | 8 | Notes: | SAME AS SG27 | |
| **29.** PAM: | | **tgg** | (NGG) | | Species: | | spCas^{WT} | | | | |
| sgRNA: | | 5' - G cttacttttgtaaaacttta-3' (SEQ ID NO: 442) (Fw) | | | | | | | | | |
| Site 1 | Editor: | A to G | | Base: | ⁱ³³A | Position: | | 4 | Notes: | | |
| Site 2 | Editor: | C to T | | Base: | ⁱ³⁴C | Position: | | 5 | Notes: | | |
| **30.** PAM: | | **tgg** | (NGG) | | Species | | spCas^{WT} | | | | |
| sgRNA: | | 5' - G ttgtaaaactttatggtttg-3' (SEQ ID NO: 443) (Fw) | | | | | | | | | |
| Site 1 | Editor: | A to G | | Base: | ⁱ⁴¹A | Position: | | 5 | Notes: | | |
| Site 1 | Editor: | A to G | | Base: | ⁱ⁴²A | Position: | | 6 | Notes: | | |

| **B. Genomic region surrounding exon 8:** | | | | | | | | | | | |
|---|---|---|---|---|---|---|---|---|---|---|---|
| | | | | | | | | Exon in CAPS | | STOP codons in **red** | |
| aagcctctggttctaatttctcatttgcagGAAATGCTGGCATAGAGCAGCACTAAATGACACCACT AAAGAAACGATCAG (SEQ ID NO: 444) | | | | | | | | | | | |
| **31.** PAM: | | **aga** | (NGA) | | Species | | spCas^{mut} | | | | |
| sgRNA: | | 5' - G TTTCctgcaaatgagaaatt-3' (SEQ ID NO: 445) (Rv) | | | | | | | | | |
| Site 1 | Editor: | C to T | | Base: | ^{E8-1}G | Position: | | 8 | Notes: | | |
| Site 2 | Editor: | C to T | | Base: | ^{E-4}T | Position: | | 5 | Notes: | | |
| **32.** PAM: | | agaaat | (NNNRRT) | | | Species: | | saCas^{mut} | | | |
| sgRNA: | | 5' - GCCAGCATTTCctgcaaatg-3' (SEQ ID NO: 446) (Rv) | | | | | | | | | |
| Site 1 | Editor: | C to T | | Base: | ⁶G | Position: | | 6 | Notes: | | |
| Site 2 | Editor: | A to G | | Base: | ⁸T | Position: | | 4 | Notes: | OVERLAP WITH 33 | |
| **33.** PAM: | | **tga** | (NGA) | | Species: | | spCas^{WT} | | | | |
| sgRNA: | | 5' - G **A**TGCCAGCATTTCctgcaaa-3' (SEQ ID NO: 447) (Rv) | | | | | | | | | |
| Site 1 | Editor: | C to T | | Base: | ⁶G | Position: | | 8 | Notes: | | |
| Site 2 | Editor: | A to G | | Base: | ⁸T | Position: | | 6 | Notes: | | |
| Site 3 | Editor: | C to T | | Base: | ⁹G | Position: | | 5 | Notes: | OVERLAP WITH 32 | |
| **34.** PAM: | | *gcaaat* | (NNNRRT) | | | Species: | | saCas^{mut} | | | |
| sgRNA: | | 5' - GCT**CTA**TGCCAGCATTTCct-3' (SEQ ID NO: 448) (Rv) | | | | | | | | | |
| Site 1 | Editor: | A to G | | Base: | ¹³T | Position: | | 6 | Notes: | | |
| **35.** PAM: | | *CTAAAT* | | (NNNRRT) | | | Species | | saCas^{mut} | | |
| sgRNA: | | 5' - G AATGCTGGCA**TAG**AGCAGCA-3' (SEQ ID NO: 449) | | | | | | | | | |
| | (Fw) | | | | | | | | | | |
| Site 1 | Editor: | C to T | | Base: | ⁷C | Position: | | 5 | Notes: | | |
| **36.** PAM: | | **TGA** | (NGA) | | Species: | | spCas^{WT} | | | | |
| sgRNA: | | 5' - G TGGCA**TAG**AGCAGCAC*TAAA*-3' (SEQ ID NO: 450)(Fw) | | | | | | | | | |
| Site 1 | Editor: | A to G | | Base: | ¹²A | Position: | | 5 | Notes: | | |

### EXAMPLE 3: Use of ABE8 editor to edit C:G→T:A in exon 7 Spinal Muscular Atrophy (SMA)

This Example tested a series of ABE8-base editors to repair C:G->T:A in position 6 of exon 7 that is causal to spinal muscular atrophy (SMA). Eleven different editors were tested, including ABE8 fusions to SpCas9, SaKKH, Cas9-NG, CP1028, CP1041, CP1041-NG, VRQR, Cpf1, SpyMac, and the evolved NRRH and NRCH editors developed by our lab in combination with a series of sgRNAs that placed the target nucleotide at positions ranging 5 through 12 in exon 7.

### Fusion constructs tested:

| **BASE EDITOR FUSION** | **AMINO ACID SEQUENCE** |
|---|---|
| ABE8-NRTH editor | (SEQ ID NO: 463) |
| ABE8-SpyMac editor | (SEQ ID NO: 464) |
| ABE8-VRQR-CP1041 editor | (SEQ ID NO: 465) |
| ABE8-SaCas9 editor | (SEQ ID NO: 466) |
| ABE8-NRCH editor | (SEQ ID NO: 467) |
| ABE8-NRRH editor | (SEQ ID NO: 468) |
| ABE8-SaKKH editor | (SEQ ID NO: 469) |
| ABE8-Cas9-NG editor | (SEQ ID NO: 470) |
| ABE8-CP1041 editor | (SEQ ID NO: 471) |
| ABE8-CP1028 editor | (SEQ ID NO: 472) |
| ABE8-CPF1 editor | (SEQ ID NO: 473) |
| ABE8-VRQR editor | (SEQ ID NO: 474) |
| ABE8-Cas9-NG-CP1041 editor | (SEQ ID NO: 475) |
| ABE8-iSpyMac editor | (SEQ ID NO: 476) |

### sgRNAs tested for each contruct:

| **sgRNA name** | **PAM** | **SNV position** | **sequence** |
|---|---|---|---|
| 3 | NGA | 9 | TTTTGTCTAAAACCctgtaaSEQ ID NO: 368) |
| 37 | NGG | 10 | ATTTTGTCTAAAACCctgta(SEQ ID NO: 364) |
| 139 | NNNRRT | 8 | TTTGTCTAAAACCctgtaag(SEQ ID NO: 366) |
| 52 | NAA | 8 | TTTGTCTAAAACCctgtaag(SEQ ID NO: 366) |
| 177 | NAT | 5 | GTCTAAAACCCTGTAAGGAA(SEQ ID NO: 408) |
| 178 | NAA | 6 | TGTCTAAAACCCTGTAAGGA(SEQ ID NO: 409) |
| 179 | NAA | 7 | TTGTCTAAAACCCTGTAAGG(SEQ ID NO: 410) |
| 54 | TTTV | 10 | ATTTTGTCTAAAACCCTGTAAGG(SEQ ID NO: 411) |
| 55 | TTTV | 11 | GATTTTGTCTAAAACCCTGTAAG(SEQ ID NO: 412) |
| 56 | TTTV | 12 | TGATTTTGTCTAAAACCCTGTAA(SEQ ID NO: 413) |

FIG. 4 shows the results of adenine base editing of the SMN2 disease causing C840T in SMA mESCs. Editors are denoted below the x-axis with PAM sequence in parentheses, and protospacer position of the target nucleotide assuming a 20nt protospacer where the PAM is at position 21-23. The results show that the iSpyMac and the CP constructs edited C840T with high efficiency.

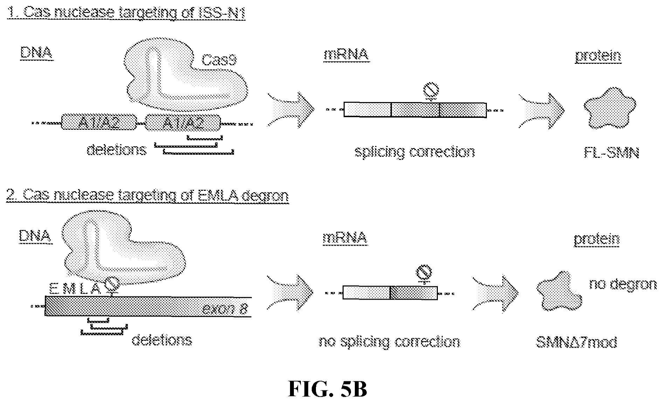

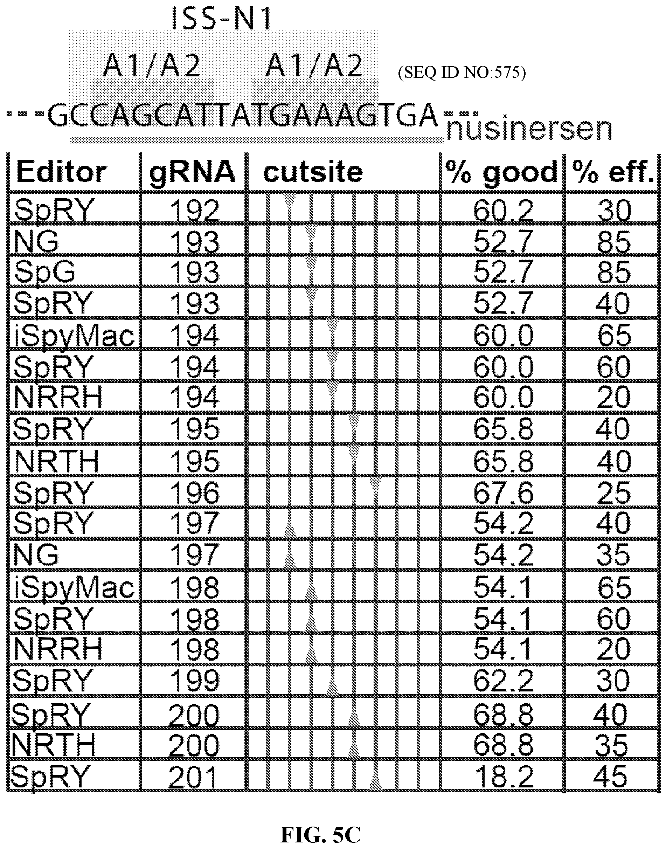

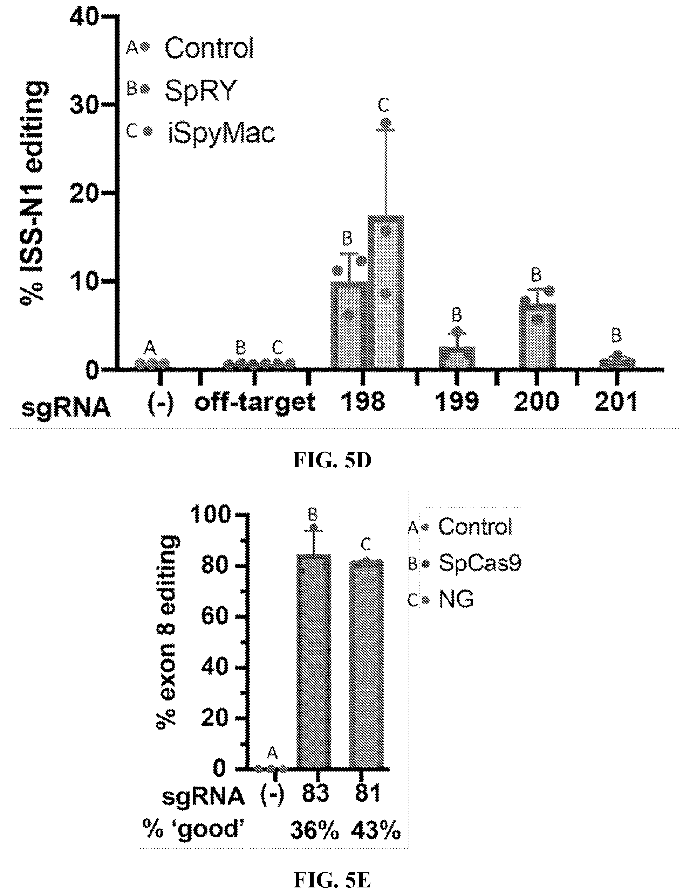

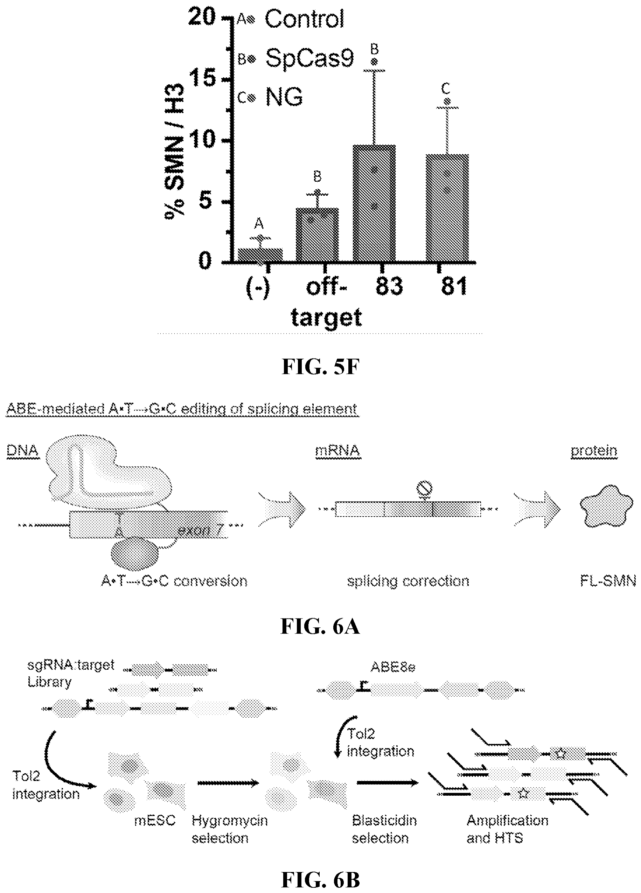

Proof of repair of the exon 7 splicing error is shown in FIG. 5, which shows a gel electrophoresis image of SMN cDNA PCR amplification spanning exon 6 to exon 8, depicting bands that include or that have skipped exon 7 in pre-mRNA splicing in SMA mESCs treated with the indicated ABE8-fusion base editors.

### EXAMPLE 4: Genome editing therapeutics development for Spinal Muscular Atrophy

SMA is a progressive motor neuron disease (MND) that is the leading genetic cause of infant death across all ethnic groups¹⁻³. SMA is caused by homozygous loss of the survival motor neuron 1 *(SMN1)* gene with retention of one or more copies of the highly similar *SMN2,* that harbors a C-to-T transition at position 6 of exon 7 (C6T) which causes exon skipping (FIG. 5A)^{4,5}. The resulting truncated SMNΔ7 protein partially compensates for the loss of cell essential SMN, but is rapidly degraded in cells resulting in SMN insufficiency that leads to paralysis and fatality of patients⁶⁻⁹.

Correcting SMN in patient tissues can rescue motor function and substantially improve the prognosis of SMA patients¹⁰⁻¹⁴. The therapeutic antisense oligonucleotide (ASO) nusinersen (Spinraza) and the small molecule splicing modifier risdiplam (Evrysdi) stimulate splicing inclusion of exon 7 in *SMN2* transcripts, however, their effect is transient and thus continued treatment is required throughout patients' lifetimes to maintain a therapeutic benefit¹⁵⁻¹⁸. AAV-mediated gene complementation with Zolgensma provides constitutive high-level *SMN1* cDNA expression in targeted cells¹⁹⁻²¹, yet it is currently unknown whether episomal AAV will permanently persist in motor neurons to provide a life-long therapeutic benefit to patients. Moreover, endogenous SMN expression is tightly regulated and restricted to relatively low levels across all tissues²²⁻²⁵, and high-level SMN expression may induce tissue-specific pathologies over time²⁶⁻²⁹. A therapeutic modality that provides permanent correction of SMN expression subject to native gene regulation could address limitations of the currently approved therapies.

Presented herein are adenine base editing and Cas9-nuclease genome editing strategies to stably correct *SMN* mRNA and protein phenotypes of SMA. It has been demonstrated that high-throughput characterization of editing outcomes coupled with machine learning can enable accurate *in silico* prediction of base editing and Cas9-nuclease genome editing outcomes at a target locus of interest³⁰⁻³⁹, which can facilitate the design of precision genome editing therapeutics. The sequence determinants of base editing were characterized by the recently reported adenine base editor 8e (ABE8e) and BE-Hive and inDelphi predictive models of genome editing outcomes were used to identify strategies that improve expression of SMN. An ABE8e editing strategy was identified that uses the engineered Cas9 protein SpyMac⁴⁰ to enable correction of the pathogenic A•T base pair in *SMN2* with 99% efficiency and >80% single nucleotide editing precision in mutant Δ7 SMA (*SMN2*+/+*; SMNΔ7+*/*+; Smn-*/*-*) mouse embryonic stem cells, and the subsequent upregulation of *SMN2* exon 7 splicing inclusion and SMN protein expression was observed. 90% C6T editing *in vivo* in targeted cells of the central nervous system (CNS) following AAV9-mediated delivery by intracerebroventricular injection in SMA mice⁴¹ was achieved, resulting in a significant increase in survival and improvement of motor function.

A dual-therapy strategy combining ABE8e-SpyMac treatment with nusinersen was explored to transiently ameliorate disease phenotypes of SMA Δ7 mice and extend the narrow temporal window for phenotypic rescue. It was demonstrated that nusinersen did not perturb base editing efficiency, and that co-delivery of a single low dose of nusinersen on P0 heightened survival of AAV9-ABE8e-SpyMac treated animals up to 317 days (the longest living animal is alive at the time of this filing). These findings establish the therapeutic potential of adenine base editing for stable genetic modification of the *SMN2* gene as a novel treatment modality for SMA.

### Precise SMN2 nuclease editing increases SMN protein levels

SMN levels are subject to genetic, transcriptomic, and post-translational regulation. Cas-nuclease disruption of key regulatory sequences was explored to determine whether it could enhance SMN levels in Δ7 SMA mutant mouse embryonic stem cells that are knockout for endogenous *Smn1* and homozygous for human *SMN2* and *SMNΔ7-*cDNA transgenes (SMA mESCs)⁴⁵. Splicing of *SMN2* exon 7 was strongly influenced by the downstream intronic splicing silencer ISS-N1 that harbors two heterogeneous nuclear ribonucleoprotein (hnRNPs) A1/A2 binding sites (FIG. 5A)⁴⁶. Sequence deletions in the ISS-N1 region, in particular within and downstream of the 3'-hnRNP A1/A2 domain, significantly restored inclusion of exon 7 in *SMN2* mRNA transcripts similar to blocking of ISS-N1 by the nusinersen ASO^{46,47}.

As a means to correct *SMN2* exon 7 missplicing, wildtype or variant SpCas9 nuclease strategies were investigated at the ISS-N1 region (FIG. 5B). sgRNAs that enabled 'good' outcomes, defined as the deletion of four or more nucleotides in ISS-N1 including at least 1 nt of the 3'-hnRNP A1/A2 domain, were predicted by inDelphi models of editing outcomes based on wildtype SpCas9-cutting data (FIG. 5C)³⁰. The compatibility of Cas nucleases was assessed based on their reported activity at the respective sgRNA PAM sequences^{40,48-51} focusing on strategies expected to result in ≥30% good editing.

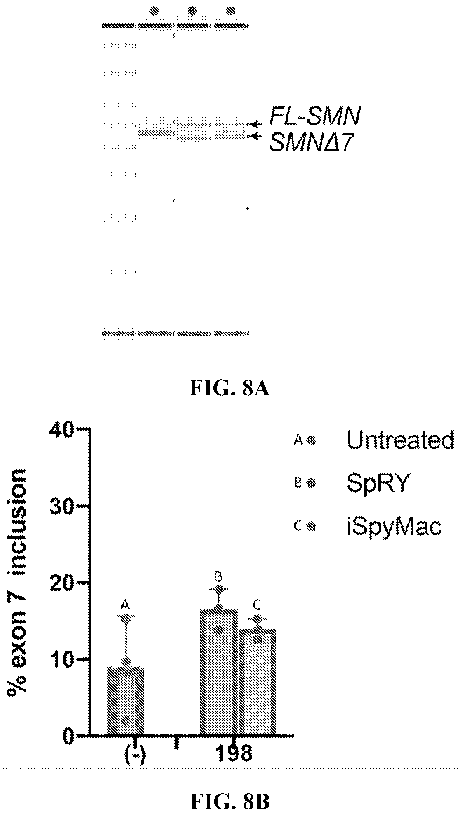

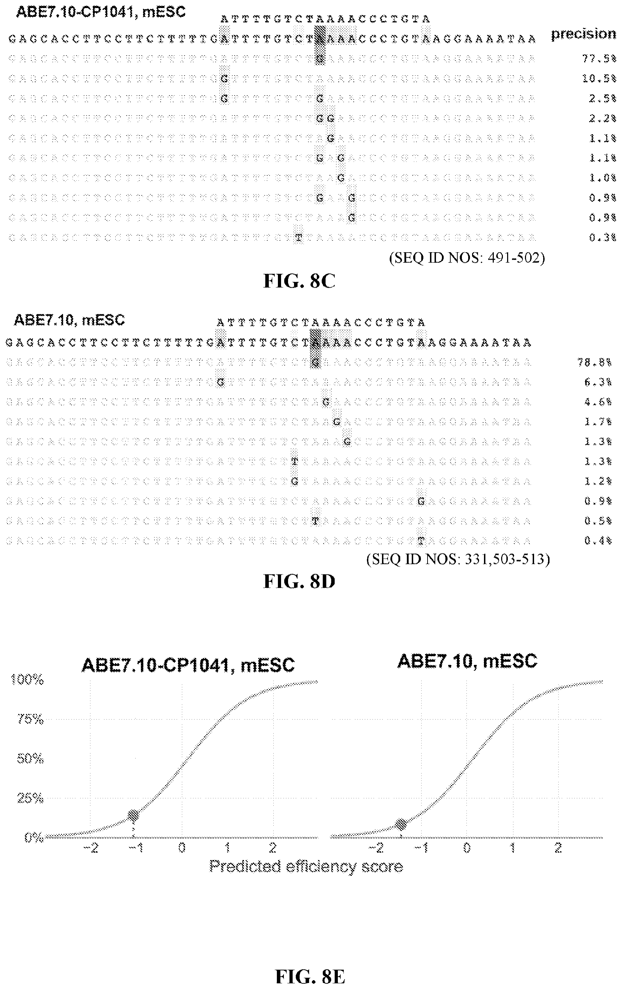

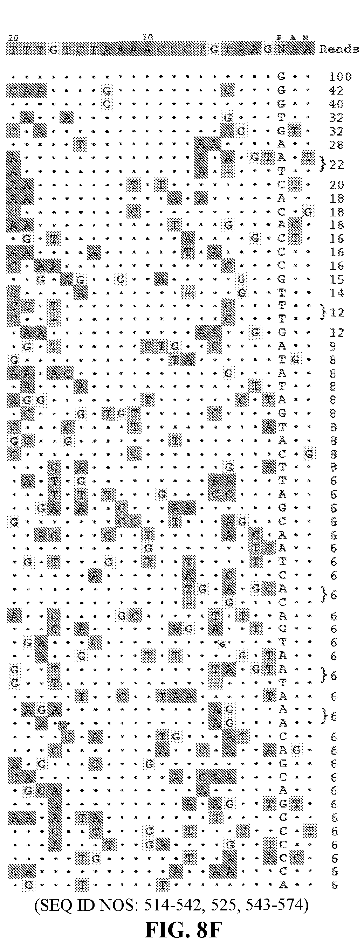

SMA mESCs were co-transfected with a nuclease plasmid carrying a Blasticidin-resistance cassette and an sgRNA expression plasmid carrying a Hygromycin-resistance cassette, both of which were flanked by Tol2 transposase sequences, alongside a Tol2 transposon plasmid to allow for stable antibiotic selection and stable integration of all genome editing components. Genome editing efficiency at the ISS-N1 locus was limited and no significant increase of exon 7 inclusion was observed in *SMN2* mRNA (FIG. 5D, FIGs. 8A-8B), suggesting that at present commonly used Cas-nucleases are not well suited to improve *SMN2* splicing by modification of ISS-N1.

The critical distinction between functional full-length SMN (FL-SMN) and SMNΔ7 is the substitution of the C-terminal 16 amino acids encoded by exon 7 with four amino acids (EMLA (SEQ ID NO: 466) ) encoded by the 3'-UTR in exon 8 that causes protein instability (FIG. 5B)⁶. The EMLA (SEQ ID NO: 466) peptide chain stimulates degradation of SMN; however, appendage of five or more heterologous peptides to the C-terminus of SMN exons 1-6 or SMNΔ7 has previously been shown to restore protein stability and quantitatively increase cellular SMN levels⁵². DNA modification of the 5' of exon 8 to induce a frame-shift or to ablate the early stop-codon was investigated to determine whether it could yield C-terminally modified SMNΔ7 (SMNΔ7mod) protein products that were not subject to the rapid degradation suffered by SMNΔ7. Nuclease editing strategies were evaluated using SpCas9-variants targeting the first 5 codons of exon 8 using inDelphi³⁰, defining 'good' editing outcomes as those that did not disrupt the exon 8 acceptor splice site and resulted in the addition of 5 or more alternative amino acids to the C-terminus of SMNΔ7. Two strategies compatible with SpCas9 and Cas9-NG that induced good genotypes in over 50% of outcomes were identified. Efficient editing was observed following transfection in SMA mESCs, resulting in a 2-fold upregulation in SMNΔ7mod protein levels relative to mock-edited cells (FIGs. 5E-5F). Thus, Cas-nuclease editing of *SMN2* guided by predictive modeling of editing outcomes improved a cellular hallmark of SMA pathology. *Systematic characterization of ABE8e base editing activity*

Double-stranded DNA breaks, such as those created by Cas9-nuclease, may result in large deletions and complex rearrangements^{53,54} especially when more than one genomic locus is targeted simultaneously⁵⁵. Copy number of *SMN2* varies from 0 to 8, and SMA patients generally have 2 or more copies⁵⁶; thus, Cas9-targeting of *SMN2* may result in unintended restructuring of chromosome 5q13 that harbors the *SMN* genes, in particular as multiple identical gene copies are present in cis. Base editing rarely induces DSBs³¹, and may thus present a safer alternative to nuclease editing as a potential genome editing therapeutic.

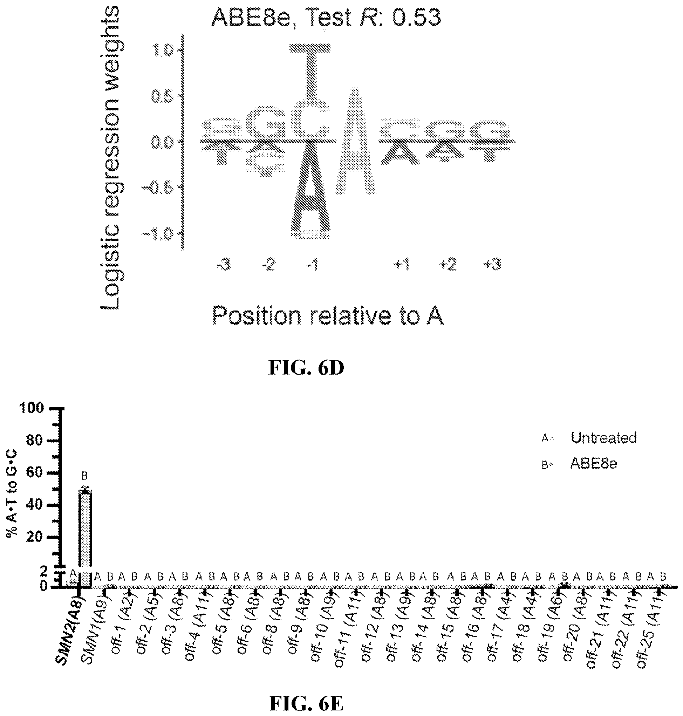

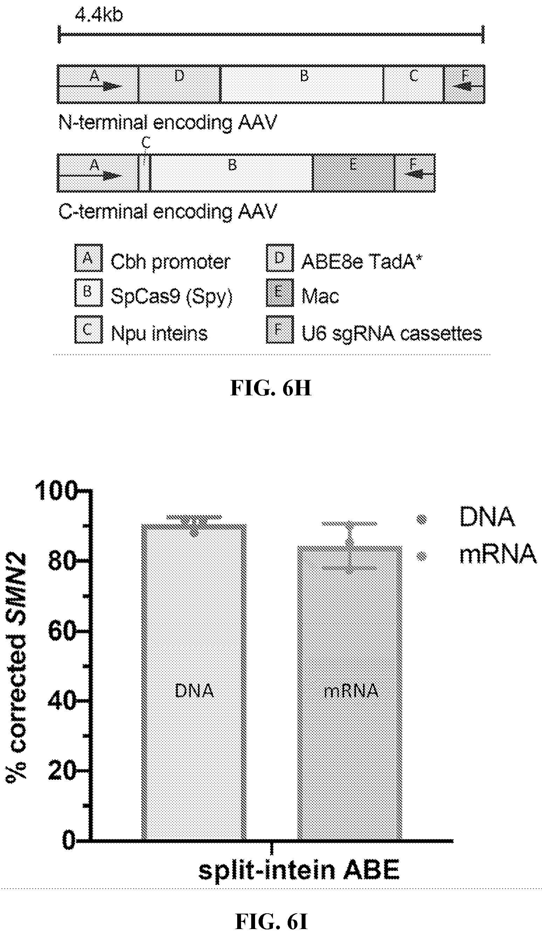

Adenine base editing of the C-to-T transition in exon 7 (C6T) was investigated as a means to correct *SMN2* missplicing (FIG. 6A). It was found that the sequence flanking C6T was deplete of canonical SpCas9 'NGG' PAMs to position the target nucleotide within a typical base editor window^{31,32}. Base editing outcome predictions by BE-Hive machine learning models for canonical ABE7.10 utilizing SpCas9 suggested that the A•T target in *SMN2* was largely intractable to base editing (FIGs. 8C-8E)³¹, and the ABE7.10 deaminase enzyme often performed poorly with Cas9-protein alternatives^{58,59}.

A newly evolved ABE8e that demonstrates increased deaminase activity relative to ABE7.10 and has greater compatibility with a wide range of Cas-protein variants was recently reported^{59,60}. Since local sequence features, Cas-proteins, and base editor positioning all affect deamination efficiency and single-nucleotide precision of base editors³¹, the sequence features that govern ABE8e editing outcomes to aid the selection of an optimal ABE8e editing strategy for *SMN2* C6T correction were investigated.

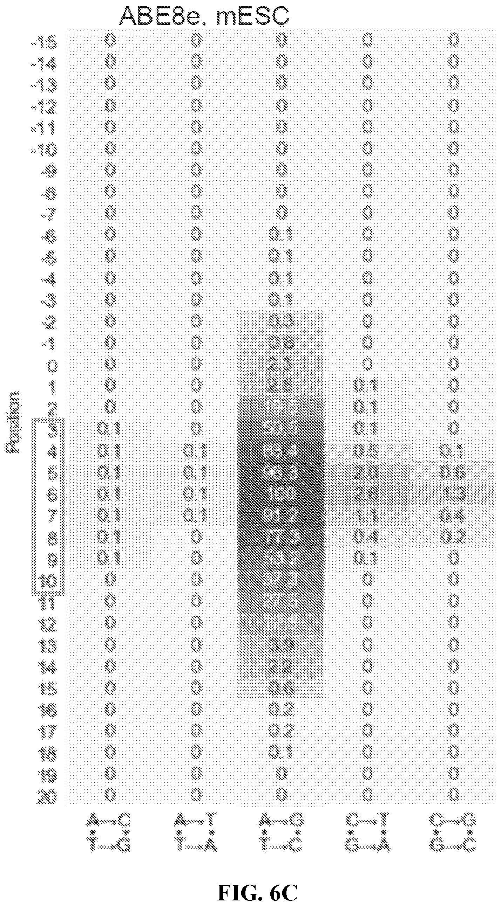

Sequence determinants of ABE8e (bpNLS-TadA*-SpCas9 D10A-bpNLS) editing outcomes were characterized using the previously reported stably integrated library of 10,638 matched sgRNA and target pairs in mouse embryonic stem cells (mESCs) named the "comprehensive context library"³¹. This library included 8,142 base editor target sequences with all possible 6-mers surrounding a substrate A or C nucleotide at protospacer position 6, and 2,496 sgRNA-target pairs that collectively contained all possible 5-mers across positions -1 to 13 (FIG. 6B). A high correlation of ABE8e base editing outcomes was observed across two biological replicates of the comprehensive context library in mESCs, and an increase in average base editing activity (frequency of target-modified outcomes among total sequenced reads) relative to ABE7.10 and ABE7.10-CP1041 was observed across all sites (66% ABE8e, compared to 18% and 12% for ABE7.10 and ABE7.10-CP1041 respectively³¹). The resulting data was used to train BE-Hive machine learning models to predict bystander editing patterns and base editing efficiency of ABE8e³¹.
The phage-assisted evolution of ABE7.10 towards ABE8e significantly altered base editing sequence-activity characteristics^{31,32,59}. A positional preference for ABE8e editing activity centered on position 6 with an editing window spanning protospacer positions 3-10 was observed (window defined as ≥30% of maximum editing, FIG. 6C). Similar to previous-generation ABEs, ABE8e maintained a dislike for bystander adenines surrounding a target A (FIG. 6D)^{31,32,59}, though this effect was outweighed by an overall substantial increase in enzymatic activity⁶⁰. These characterizations refine and expand upon previous characterizations of ABE8e base editing activity⁵⁹

### Adenine base editing correction of the SMN2 pathogenic A•T base pair

For a canonical ABE8e editing approach using SpCas9, the nearest downstream 'NGG' PAM placed the target A•T pair of *SMN2* at position 10, which falls at the edge of the ABE8e base editing window. Use of alternative Cas-proteins with alternative PAM preferences could allow for better placement of the target nucleotide. BE-Hive was used to predict correction efficiency and precision of the *SMN2* A•T target pair with other sgRNAs that were compatible with alternative Cas-proteins, by assuming similar base editing characteristics for ABEs using engineered and evolved SpCas9-variants^{40,48,49,61}, and accounting for a window shift of ABEs using SaCas9 (+1nt) and LbCas12a (+4nt) as previously described³¹.

SMA mESCs were transfected with ABE7.10 and ABE8e base editor variants, and editing outcomes consistent with BE-Hive prediction were observed, demonstrating that computationally learned characteristics of ABEs using SpCas9 can be extended to ABE Cas-variants. Notably, base editing efficiency varied dependent on Cas-protein as previously observed^{58,59}. The highest efficiency A•T-to-G•C conversion overall was acheived using SpyMac and iSpyMac-variant ABE8e paired with sgRNA52 (98.9±0.71% SpyMac and 98.8±0.42% iSpyMac) or sgRNA179 (94±3.35% SpyMac and 98±0.59% iSpyMac), and with ABE8e-CP1028 paired with sgRNA37 (99.5±0.06%), that each placed the pathogenic SNP at positions 5, 8, and 10 respectively. Analysis of the resulting base edited genotypes revealed that ABE8e-SpyMac paired with sgRNA52 induced the highest purity single nucleotide A•T-to-G•C edits devoid of bystander editing outcomes or indels that resulted from base editing (84.2±2.4%).

### Off-target analysis of an ABE8e strategy to correct SMN2 in the human genome

To assess the genome specificity of the optimized ABE8e-SpyMac strategy, off-target editing was characterized by the CIRCLE-seq method⁶². Purified ribonucleoprotein (RNP) complexes comprised of SpyMac nuclease and sgRNA52 were generated, and a short list of SpyMac-dependent DNA off-target loci were identified *in vitro* using human genomic DNA extracted from HEK293T cells (FIG. 8F). Top targets included the *SMN2* on-target locus and the equivalent locus in the *SMN1* gene that had the single A•T-to-G•C mismatch at sgRNA position 8, which is present in wild type human genomes but absent in those of SMA patients.

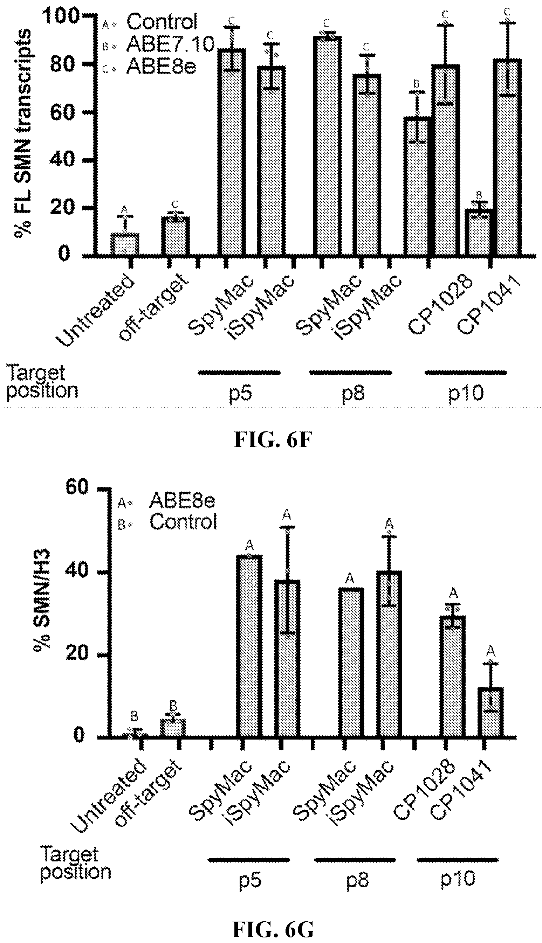

Off-target base editing activity was assessed at the top 25 CIRCLE-seq hits, which had ≥8% editing efficiency relative to the on-target site, alongside *SMN2* and *SMN1* loci by transfection of ABE8e-SpyMac + sgRNA52 in wild type HEK293T cells followed by targeted amplicon sequencing. 48.5±1.8% C6T editing at on-target *SMN2* loci was acheived, and minimal ABE8e-SpyMac editing at *SMN1* loci (0.15±0.07%) and at off-target site rank 19 (0.4±0.14%) which falls in an intergenic region of chromosome 15 was observed (FIG. 6F). No base editing was detected (>0.03% relative to untreated control) at the remaining off-target loci.

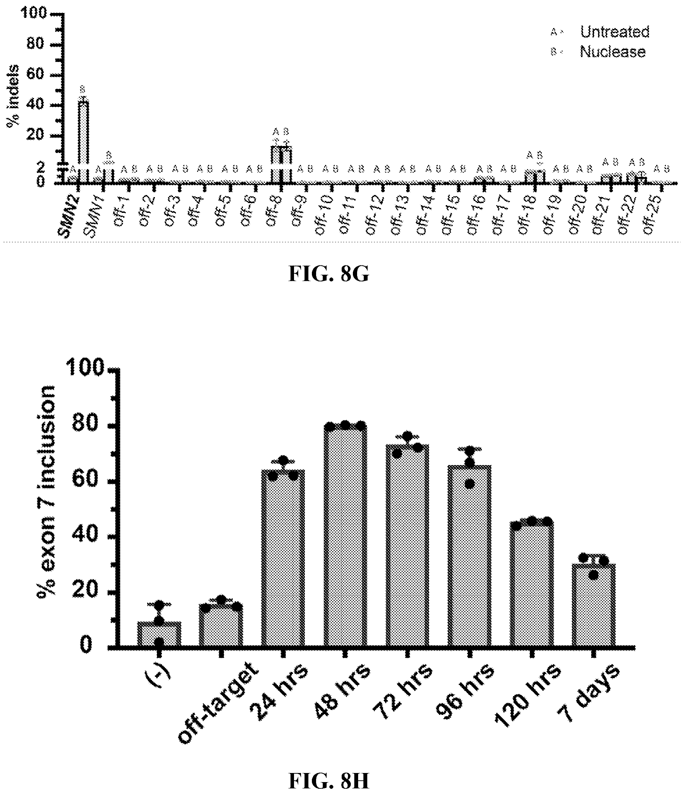

Off-target analysis of SpyMac nuclease + sgRNA52 in HEK293T cells yielded highly similar results. Indels were observed at 41.9±3.0% of *SMN2* alleles and 2.1±0.33% of *SMN1* alleles, and no indels were detected at the remaining off-target loci (FIG. 8G). Together, these data indicated that the SpyMac Cas-component, and the ABE8e-SpyMac base editor overall paired with sgRNA52 were highly specific to the *SMN2* on-target locus. The optimized ABE8e-SpyMac + sgRNA52 strategy is referred to as 'ABE' editing, hereafter.

### ABE-mediated rescue of SMA pathology in mice

The C6T substitution in *SMN2* exon 7 affected a multitude of splicing regulatory interactions that resulted in majority *SMNΔ7* processed mRNA transcripts at the expense of full length-SMN (*FL-SMN*), leading to the production of unstable SMNΔ7 protein products⁶³. A substantial increase in *FL-SMN* mRNA transcripts and SMN protein levels was observed following ABE editing of SMA mESCs by the ABE editing strategy presented herein (FIGs. 6G-6H). Reverse-transcription was performed and followed by PCR (RT-PCR) of *SMN2* transcripts, and *SMNΔ7* and *FL-SMN* products were quantified by automated electrophoresis. A >5.6-fold increase in the subset of *FL-SMN* products among all *SMN* transcripts in ABE treated cells (87.1±1.5%) was observed relative to mock-treated cells (15.5±1.6%) that was comparable to the *SMN1* exon 7 inclusion ratio observed in wild type cells^{46,47}, equivalent to peak exon 7 inclusion following SMA mESC transfection with 20nM nusinersen (56.4±1.3%, FIG. 8H). SMN protein expression was quantified by Western Blot and normalized to endogenous Histone H3 levels, and a >8.2-fold increase was detected in SMN protein in ABE treated SMA mESCs (36.3±8.9%) relative to mock-treated cells (4.4±1.2%, two-tailed t-test*p* = *0.02*). Thus, correction of *SMN2* C6T by ABE editing rescues cellular hallmarks of SMA pathology.

Next, an adeno associated virus (AAV) strategy for *in vivo* delivery of the ABE8e-SpyMac base editor and sgRNA52 was developed. Together, base editor and sgRNA expression cassettes exceeded the packaging capacity of a single AAV vector; however, it has been previously demonstrated that co-delivery of split-base editors packaged as single-stranded (ss) viral genomes into AAV-particles can overcome this size limitation⁶⁴⁻⁶⁷. Fusion of trans-splicing inteins to split-base editors severed within their Cas9 component enabled efficient self-assembly of full-length base editors when each half was co-expressed in cells. A split-intein ABE8e-SpyMac dual-AAV strategy driven by a Cbh-promoter was designed, using each split DnaE intein half from Nostoc punctiforme (Npu) to divide ABE8e-SpyMac within the SpCas9 domain immediately before Cys 574 (FIG. 6I)^{40,68}, a design previously demonstrated to enable efficient editing by ABEmax^{66,67}.

A first sgRNA52 expression cassette was included on the ssAAV genome encoding the C-terminal portion of split-ABE8e-SpyMac, as before with v5 AAV-ABEmax⁶⁶. ABE8e functions with a single N-terminal TadA* monomer and is therefore smaller (4.8kb) than ABE7.10 (5.4kb) which uses an N-terminally encoded wtTadA-TadA* heterodimer^{32,59}. This 600bp reduction at the N-terminal split-ABE8e-SpyMac component afforded the space to include an additional sgRNA52 expression cassette into this ssAAV genome. Co-transfection of split-intein ABE8e-SpyMac plasmids in SMA mESCs induced A•T-to-G•C conversion of *SMN2* C6T with similar efficiency to full-length ABE8e-SpyMac (FIG. 6J). The ssAAV-encoded split-intein ABE8e-SpyMac with dual-sgRNA52 is referred to as 'ssAAV-ABE' hereafter.

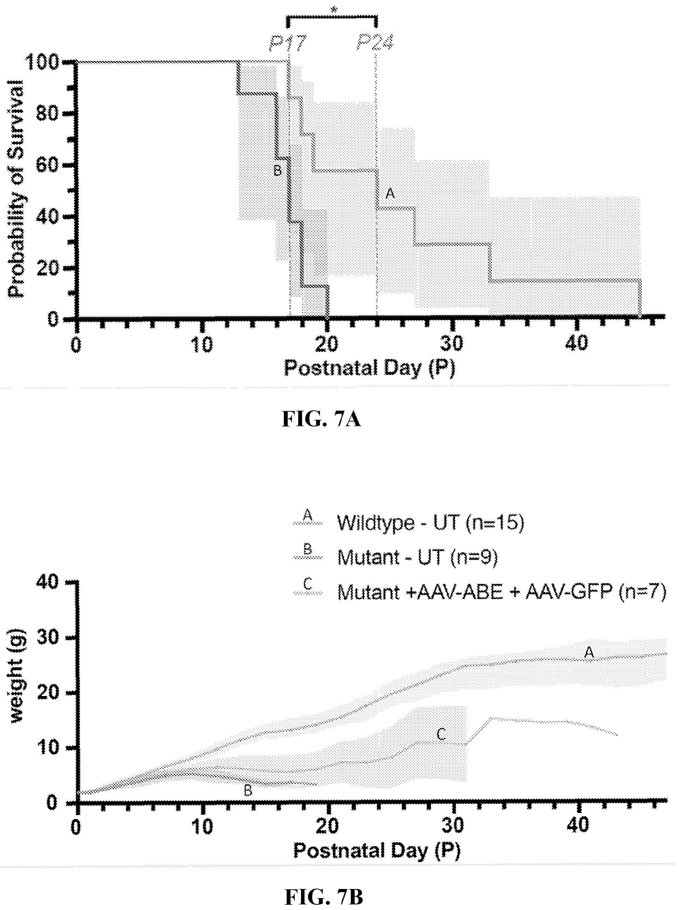

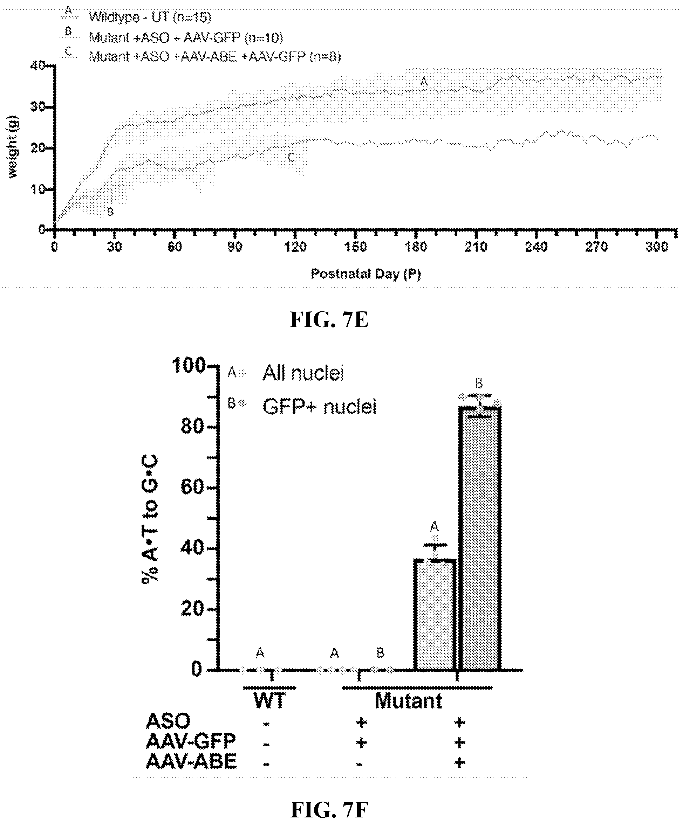

1.3E13 vg/kg of each half of ssAAV-ABE was co-delivered on postnatal day (P) 0 and P1 along with 2.7E12 vg/kg AAV9-Cbh-eGFP-KASH (Klarsicht/ANC-1/Syne-1 homology domain, hereafter AAV9-nucGFP)⁶⁶ as a transduction control, by intracerebroventricular (ICV) injection into homozygous mutant Δ7 SMA neonates. Nuclei from the central nervous system (CNS) of deceased treated animals was isolated and 87.0±3.5% conversion of the A•T target pair in *SMN2* exon 7 in GFP-positive nuclei was observed (FIG. 7F), indicating that the optimized ABE strategy edited at high efficiency in transduced tissues *in vivo,* similar to SMA mESCs in culture.

A marked improvement in the bodyweight of ssAAV-ABE treated SMA mutant animals, and a significant increase in survival relative to untreated controls from median 17 to 24 days was observed (Mantel-Cox test *p = 0.008,* FIG. 7A). The bodyweight and survival of SMA animals was comparable to that following late neonatal (> P6) ICV treatment with self-complementary (sc) AAV9-SMN1, and injection of low doses (~ 1E13 vg/kg) scAAV9-SMN 1 in previous reports^{19,69}.

Treatment with ssAAV-ABE partially rescued SMA motor phenotypes. At P7 the ability of all mutant animals to right themselves was similar, however, by P 14 the righting reflex of ssAAV-ABE treated animals improved relative to untreated controls. At P25 ssAAV-ABE treated SMA mutant animals showed difficulty in the inverted screen test relative to control animals. Notably, age-matched untreated animals were not available as controls for this assay due to their short lifespan. Collectively these data show that postnatal ssAAV-ABE treatment can correct the *SMN2* C6T with high efficiency *in vivo,* leading to partial rescue of motor function and lethality in Δ7 SMA mice.

### Combining ABE and nusinersen treatment enhances rescue of SMA mice

Upregulation of SMN can greatly rescue motor function and improve survival of SMA patients and animal models, so long as sufficient levels of SMN are achieved prior to onset of neuromuscular pathology^{19,69,70}. Even high levels of SMN protein cannot correct neuromuscular junction (NMJ) pathology and fatality once SMA has progressed to an advanced stage. The clinically approved scAAV9 therapy for SMA confers only partial rescue in Δ7 SMA mice when administered at low doses or after P6, and earlier postnatal SMN rescue is correlated with improved survival.

For ssAAVs, second-strand synthesis is a rate-limiting step in the expression of virally-transduced expression cassettes⁷¹⁻⁷⁴. Sufficient accumulation SMN protein following ssAAV9-ABE8e co-transduction requires a series of processes to be completed first, including (1) second-strand synthesis of the split ssAAV-ABE genomes, (2) transcription and translation of the split-intein ABE8e-SpyMac protein segments, (3) self-assembly of full-length ABE8e-SpyMac and sgRNA52 RNP, (4) targeted base editing and DNA repair, (5) transcription of corrected human *SMN2* genes from the low-expression endogenous promoter, (6) mRNA processing, and (7) translation of corrected *SMN2* transcripts. The ability of endogenous transcriptional and post-transcriptional regulatory mechanisms to restrict the expression of rescued *SMN* alleles to the typically low levels of wild type tissues is a key desirable feature of a potential base editing therapeutic, which may be of particular importance for long-term rescue of SMA patients. However, the steps (1-7) described above can dampen the phenotypic rescue that follows from postnatal ssAAV-ABE treatment of Δ7 mice in spite of efficient *in vivo* base editing correction. It was hypothesized that the timeframe for sufficient SMN protein to accumulate in cells after ssAAV-ABE co-transduction exceeds the temporal window for complete rescue of Δ7 mice, but that transient amelioration of SMA in spinal motor neurons by other means could allow more opportunity for ssAAV9-ABE8e mediated rescue to take hold.

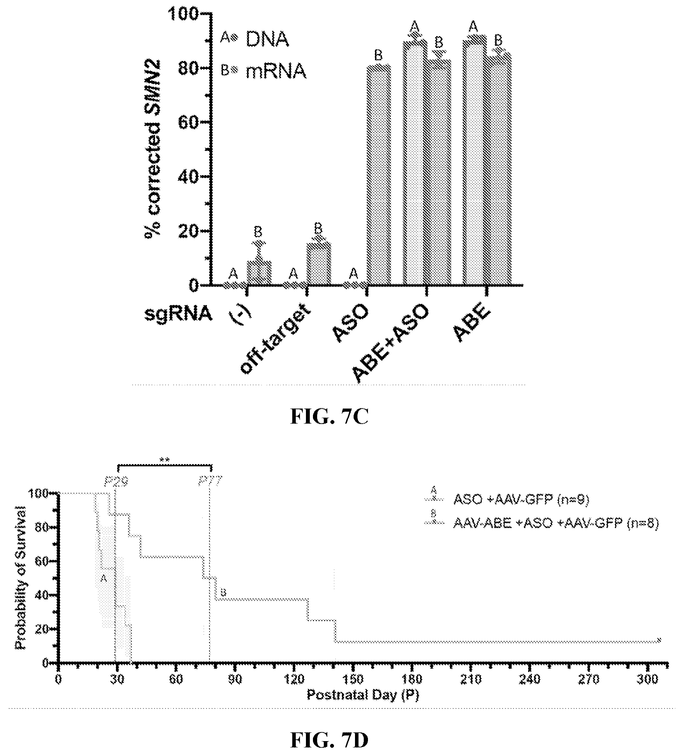

Single dose ICV injection of nusinersen on P0 resulted in transient rescue of molecular and physiological motor neuron degeneration phenotypes in Δ7 mice and an improvement in survival of several weeks⁷⁵. The mechanism of ABE-mediated editing of *SMN2* exon 7 DNA is entirely orthogonal to nusinersen binding of *SMN2* intron 7 pre-mRNA, and no change was observed in base editing outcomes following co-transfection of 20nM nusinersen with ABE8e-SpyMac and sgRNA52 in SMA mESCs (FIG. 7C). Thus, a single dose of nusinersen may be used to transiently extend the temporal window for rescue of Δ7 mice to provide a greater opportunity for stable genotypic correction of SMA by ssAAV9-ABE.

Mutant Δ7 mice were ICV injected with 1 ug nusinersen on P0 and either 1.3E13 vg/kg of each ssAAV9-ABE with 2.7E12 vg/kg AAV9-nucGFP as before, or with 2.7E13 vg/kg AAV9-nucGFP alone on P0 and P1. Co-injection of nusinersen and AAV9-nucGFP improved survival relative to untreated controls (median 29 days, Mantel-Cox test *p* = *0.0001),* but the increase was not significant compared to ssAAV9-ABE treatment without nusinersen (FIG. 7D). Promisingly, combined ssAAV9-ABE treatment with nusinersen further improved survival of SMA mice relative to individual nusinersen or ssAAV9-ABE treatment conditions (median 77 days, Mantel-Cox test *p = 0.002*) with a maximum survival of over 317 days (animal is alive at the time of this filing). Notably, no significant difference in bodyweight was observed between any of the treatment cohorts (FIG. 7E). Rather, body weight for all cohorts was improved relative to untreated mutants, yet lower than wild type controls as previously observed following nusinersen or scAAV9-SMN1 treatment of Δ7 mice^{19,69,76}.

Animals treated with ssAAV-ABE had improved motor skills relative to animals treated only with nusinersen. Both cohorts showed a similar improvement in their ability to right themselves at P7 and P14, suggesting that ssAAV-ABE mediated genome editing provides little additional benefit in the presence of nusinersen. However, as the transient effect of nusinersen dissipated, a significant improvement in motor function was observed. ssAAV-ABE and nusinersen treated animals had a longer latency in the inverted screen test at P25 than nusinersen control animals, at which point a significant fraction of the control cohort was deceased. At P40, voluntary movement of ssAAV-ABE and nusinersen treated animals was measured by open-field testing and though they were active, their mobility was reduced relative to wild type animals. Notably, all age-matched nusinersen control animals were deceased and therefore not available as controls for this assay. Collectively these data show that postnatal ssAAV-ABE treatment can correct the SMN2 C6T with high efficiency *in vivo,* leading to partial rescue of motor function and fatality of Δ7 SMA mice.

### Discussion

Genome editing strategies were investigated as a novel therapeutic paradigm for the treatment of SMA by permanent modification of the *SMN2* gene. Adenine base editing and Cas9-nuclease genome editing strategies that modify well-characterized transcriptional and post-transcriptional regulatory sequences of *SMN2* were thoroughly investigated. It was demonstrated that the BE-Hive and inDelphi predictive models trained on SpCas9 base editors and nucleases facilitated the design of precision base editing strategies, and nonconventional nuclease editing strategies using SpCas9 and Cas-variant editors that restore expression of SMN. A novel base editor variant was developed that utilizes the ABE8e deaminase and SpyMac Cas-component that enables highly efficient and precise single nucleotide correction of the pathogenic *SMN2* C6T target with minimal off-target specificity in the genome.

To investigate the therapeutic potential of an *SMN2* base editing treatment, ssAAV9-ABE was delivered by ICV injection to Δ7 SMA mice and up to 90% C6T correction in transduced cells *in vivo* was acheived. It was shown that the FDA-approved ASO drug nusinersen did not interfere with base editing efficiency in cells, and a combination therapy approach was developed that improved survival of ssAAV9-ABE treated SMA mice by co-delivery of nusinersen to extend the temporal window in which ABE-mediated rescue could occur. Notably, the temporal window for treatment of SMA patients was far greater than in Δ7 SMA mice; thus an ssAAV9-ABE therapy developed for clinical use may not require transient rescue of motor neurons for base editing rescue to take hold.

While *SMN2* and *SMN1* expression are similar in SMA patients, the *SMN2* transgene in SMA mice produces significantly fewer transcripts than the endogenous murine *Smn1* owing to an unfavorable integration locus. While it was expected that SMN expression would be significantly delayed and reduced following modification of the genomic *SMN2* gene relative to scAAV9-SMN1 transduction, significant rescue of survival in SMA mice that mimics 1E13 vg/kg ICV delivery of scAAV9-SMN1¹⁹ was observed. The requisite co-delivery of the dual ssAAV9-ABE vectors lowered the deliverable therapeutic dose by half, yet 90% C6T editing correction in GFP-sorted nuclei was observed, indicating that even efficient triple-transduction (ssAAV9-ABE + AAV9-nucGFP) and genome editing do take place in the CNS of treated animals. Collectively, these data show the therapeutic potential of a base editing approach to permanently correct the endogenous *SMN2* C6T for the treatment of SMA.

### References for Example 4

1. Lefebvre, S. et al. Identification and characterization of a spinal muscular atrophy-determining gene. Cell 80, 155-65 (1995).
2. Pearn, J. Classification of spinal muscular atrophies. Lancet 1, 919-922 (1980).
3. Sugarman, E. A. et al. Pan-ethnic carrier screening and prenatal diagnosis for spinal muscular atrophy: clinical laboratory analysis of > 72 400 specimens. Eur. J. Hum. Genet. 20, 27-32 (2012).
4. Lorson, C. L., Hahnen, E., Androphy, E. J. & Wirth, B. A single nucleotide in the SMN gene regulates splicing and is responsible for spinal muscular atrophy. Proc. Natl. Acad. Sci. 96, 6307-6311 (1999).
5. Monani, U. R. et al. A single nucleotide difference that alters splicing patterns distinguishes the SMA gene SMN1 from the copy gene SMN2. Hum. Mol. Genet. 8, 1177-1183 (1999).
6. Cho, S. & Dreyfuss, G. A degron created by SMN2 exon 7 skipping is a principal contributor to spinal muscular atrophy severity. Genes Dev. 24, 438-42 (2010).
7. Lorson, C. L. & Androphy, E. J. An exonic enhancer is required for inclusion of an essential exon in the SMA-determining gene SMN. Hum. Mol. Genet. 9, 259-266 (2000).
8. Vitte, J. et al. Refined characterization of the expression and stability of the SMN gene products. Am. J. Pathol. 171, 1269-1280 (2007).
9. Burnett, B. G. et al. Regulation of SMN Protein Stability. Mol. Cell. Biol. 29, 1107-1115 (2009).
10. Mendell, J. R. et al. Single-dose gene-replacement therapy for spinal muscular atrophy. N. Engl. J. Med. 377, 1713-1722 (2017).
11. Ottesen, E. W. ISS-N1 makes the first FDA-approved drug for spinal muscular atrophy. Translational Neuroscience 8, 1-6 (2017).
12. Hoy, S. M. Onasemnogene Abeparvovec: First Global Approval. 79, 1255-1262 (2019).
13. Sumner, C. J. & Crawford, T. O. Two breakthrough gene-targeted treatments for spinal muscular atrophy: challenges remain. J Clin Invest 128, 3219 (2018).
14. Parente, V. & Corti, S. Advances in spinal muscular atrophy therapeutics. Therapeutic Advances in Neurological Disorders 11, (2018).
15. Singh, N. N., Howell, M. D., Androphy, E. J. & Singh, R. N. How the discovery of ISS-N1 led to the first medical therapy for spinal muscular atrophy. Gene Therapy 24, 520-526 (2017).
16. Ratni, H. et al. Discovery of Risdiplam, a Selective Survival of Motor Neuron-2 (SMN2) Gene Splicing Modifier for the Treatment of Spinal Muscular Atrophy (SMA). J. Med. Chem. 61, 6501-6517 (2018).
17. Poirier, A. et al. Risdiplam distributes and increases SMN protein in both the central nervous system and peripheral organs. Pharmacol. Res. Perspect. 6, (2018).
18. Wurster, C. D. & Ludolph, A. C. Nusinersen for spinal muscular atrophy. Therapeutic Advances in Neurological Disorders 11, (2018).
19. Meyer, K. et al. Improving single injection CSF delivery of AAV9-mediated gene therapy for SMA: A dose-response study in mice and nonhuman primates. Mol. Ther. 23, 477-487 (2015).
20. Armbruster, N. et al. Efficacy and biodistribution analysis of intracerebroventricular administration of an optimized scAAV9-SMN1 vector in a mouse model of spinal muscular atrophy. Mol. Ther. - Methods Clin. Dev. 3, 16060 (2016).
21. Bevan, A. K. et al. Systemic gene delivery in large species for targeting spinal cord, brain, and peripheral tissues for pediatric disorders. Mol. Ther. 19, 1971-1980 (2011).
22. Kernochan, L. E. et al. The role of histone acetylation in SMN gene expression. doi:10.1093/hmg/ddi130
23. d'Ydewalle, C. et al. The Antisense Transcript SMN-AS1 Regulates SMN Expression and Is a Novel Therapeutic Target for Spinal Muscular Atrophy. Neuron 93, 66-79 (2017).
24. Woo, C. J. et al. Gene activation of SMN by selective disruption of lncRNA-mediated recruitment of PRC2 for the treatment of spinal muscular atrophy. Proc. Natl. Acad. Sci. U. S. A. 114, E1509-E1518 (2017).
25. Ramos, D. M. et al. Age-dependent SMN expression in disease-relevant tissue and implications for SMA treatment. J. Clin. Invest. 129, 4817-4831 (2019).
26. Cucchiarini, M., Madry, H. & Terwilliger, E. F. Enhanced expression of the central survival of motor neuron (SMN) protein during the pathogenesis of osteoarthritis. J. Cell. Mol. Med. 18, 115-124 (2014).
27. Gama-Carvalho, M. et al. Linking amyotrophic lateral sclerosis and spinal muscular atrophy through RNA-transcriptome homeostasis: a genomics perspective. J. Neurochem. 141, 12-30 (2017).
28. Blauw, H. M. et al. SMN1 gene duplications are associated with sporadic ALS. Neurology 78, 776-780 (2012).
29. Corcia, P. et al. The importance of the SMN genes in the genetics of sporadic ALS. Amyotroph. Lateral Scler. 10, 436-40 (2009).
30. Shen, M. et al. Predictable and precise template-free CRISPR editing of pathogenic variants. Nature 563, 646-651 (2018).
31. Arbab, M. et al. Determinants of Base Editing Outcomes from Target Library Analysis and Machine Learning. Cell 182, 463-480.e30 (2020).
32. Gaudelli, N. M. et al. Programmable base editing of A•T to G•C in genomic DNA without DNA cleavage. Nature 551, 464-471 (2017).
33. Komor, A. C., Kim, Y. B., Packer, M. S., Zuris, J. A. & Liu, D. R. Programmable editing of a target base in genomic DNA without double-stranded DNA cleavage. Nature 533, 420-424 (2016).
34. Nishida, K. et al. Targeted nucleotide editing using hybrid prokaryotic and vertebrate adaptive immune systems. Science (80-. ). 353, aaf8729 (2016).
35. Thuronyi, B. W. et al. Continuous evolution of base editors with expanded target compatibility and improved activity. Nat. Biotechnol. (2019). doi:10.1038/s41587-019-0193-0
36. Gehrke, J. M. et al. An APOBeC3A-Cas9 base editor with minimized bystander and off-target activities. Nat. Biotechnol. 36, 977 (2018).
37. Cong, L. et al. Multiplex genome engineering using CRISPR/Cas systems. Science (80-. ). 339, 819-823 (2013).
38. Mali, P. et al. RNA-guided human genome engineering via Cas9. Science (80-. ). 339, 823-826 (2013).
39. Jinek, M. et al. A programmable dual-RNA-guided DNA endonuclease in adaptive bacterial immunity. Science (80-. ). 337, 816-21 (2012).
40. Chatterjee, P. et al. A Cas9 with PAM recognition for adenine dinucleotides. Nat. Commun. 11, 1-6 (2020).
41. Le, T. T. et al. SMNΔ7, the major product of the centromeric survival motor neuron (SMN2) gene, extends survival in mice with spinal muscular atrophy and associates with full-length SMN. Hum. Mol. Genet. 14, 845-857 (2005).
42. Harada, Y. et al. Combination molecular therapies for type 1 spinal muscular atrophy. Muscle and Nerve 62, 550-554 (2020).
43. Lee, B. H. et al. Combination therapy with nusinersen and AVXS-101 in SMA type 1. (2019). doi:10.1212/WNL.0000000000008207
44. CURE SMA. Scientific considerations for drug combinations. (2019).
45. Rodriguez-Muela, N. et al. Single-Cell Analysis of SMN Reveals Its Broader Role in Neuromuscular Disease. Cell Rep. 18, 1484-1498 (2017).
46. Singh, N. K., Singh, N. N., Androphy, E. J. & Singh, R. N. Splicing of a critical exon of human Survival Motor Neuron is regulated by a unique silencer element located in the last intron. Mol. Cell. Biol. 26, 1333-46 (2006).
47. Hua, Y., Vickers, T. A., Okunola, H. L., Bennett, C. F. & Krainer, A. R. Antisense Masking of an hnRNP A1/A2 Intronic Splicing Silencer Corrects SMN2 Splicing in Transgenic Mice. Am. J. Hum. Genet. 82, 834-848 (2008).
48. Walton, R. T., Christie, K. A., Whittaker, M. N. & Kleinstiver, B. P. Unconstrained genome targeting with near-PAMless engineered CRISPR-Cas9 variants. Science (80-. ). 368, 290-296 (2020).
49. Miller, S. et al. Continuous evolution of SpCas9 variants compatible with non-G PAMs. Nat. Biotechnol. 38, 471-481 (2020).
50. Hu, J. H. et al. Evolved Cas9 variants with broad PAM compatibility and high DNA specificity. Nature 556, 57 (2018).
51. Nishimasu, H. et al. Engineered CRISPR-Cas9 nuclease with expanded targeting space. Science (80-. ). 361, 1259 (2018).
52. Wolstencroft, E. C., Mattis, V., Bajer, A. A., Young, P. J. & Lorson, C. L. A non-sequence-specific requirement for SMN protein activity: the role of aminoglycosides in inducing elevated SMN protein levels. doi:10.1093/hmg/ddi131
53. Kosicki, M., Tomberg, K. & Bradley, A. Repair of double-strand breaks induced by CRISPR-Cas9 leads to large deletions and complex rearrangements. Nat. Biotechnol. 36, (2018).
54. Zhang, L. et al. Large genomic fragment deletions and insertions in mouse using CRISPR/Cas9. PLoS One 10, (2015).
55. Shin, H. Y. et al. CRISPR/Cas9 targeting events cause complex deletions and insertions at 17 sites in the mouse genome. Nat. Commun. 8, 1-10 (2017).
56. Butchbach, M. E. R. Copy number variations in the survival motor neuron genes: Implications for spinal muscular atrophy and other neurodegenerative diseases. Frontiers in Molecular Biosciences 3, (2016).
57. Lin, X. et al. Base editing-mediated splicing correction therapy for spinal muscular atrophy. Cell Research 30, 548-550 (2020).
58. Huang, T. P. et al. Circularly permuted and PAM-modified Cas9 variants broaden the targeting scope of base editors. Nat. Biotechnol. (2019).
59. Richter, M. F. et al. Phage-assisted evolution of an adenine base editor with improved Cas domain compatibility and activity. Nat. Biotechnol. (2020). doi:10.1038/s41587-020-0453-z
60. Lapinaite, A. et al. DNA capture by a CRISPR-Cas9-guided adenine base editor. Science (80-. ). 369, 566-571 (2020).
61. Rees, H. A. & Liu, D. R. Base editing: precision chemistry on the genome and transcriptome of living cells. Nat Rev Genet 19, 770-788 (2018).
62. Tsai, S. Q. et al. CIRCLE-seq: a highly sensitive in vitro screen for genome-wide CRISPR-Cas9 nuclease off-targets. Nat Methods 14, 607-614 (2017).
63. Singh, R. N. & Singh, N. N. Mechanism of splicing regulation of spinal muscular atrophy genes. in Advances in Neurobiology 20, 31-61 (2018).
64. Villiger, L. et al. Treatment of a metabolic liver disease by in vivo genome base editing in adult mice. Nat Med 24, 1519-1525 (2018).
65. Ryu, S. M. et al. Adenine base editing in mouse embryos and an adult mouse model of Duchenne muscular dystrophy. Nat. Biotechnol. 36, 536-539 (2018).
66. Levy, J. M. et al. Cytosine and adenine base editing of the brain, liver, retina, heart and skeletal muscle of mice via adeno-associated viruses. Nat. Biomed. Eng. 4, 97-110 (2020).
67. Koblan, L. W. et al. In vivo base editing rescues Hutchinson-Gilford progeria syndrome in mice. Nature 1-7 (2021). doi:10.1038/s41586-020-03086-7
68. Zettler, J., Schütz, V. & Mootz, H. D. The naturally split Npu DnaE intein exhibits an extraordinarily high rate in the protein trans-splicing reaction. FEBS Lett. 583, 909-914 (2009).
69. Robbins, K. L., Glascock, J. J., Osman, E. Y., Miller, M. R. & Lorson, C. L. Defining the therapeutic window in a severe animal model of spinal muscular atrophy. Hum. Mol. Genet. 23, 4559-4568 (2014).
70. Lutz, C. M. et al. Postsymptomatic restoration of SMN rescues the disease phenotype in a mouse model of severe spinal muscular atrophy. J. Clin. Invest. 121, 3029-3041 (2011).
71. Hauck, B., Zhao, W., High, K. & Xiao, W. Intracellular Viral Processing, Not Single-Stranded DNA Accumulation, Is Crucial for Recombinant Adeno-Associated Virus Transduction. J. Virol. 78, 13678-13686 (2004).
72. Zhou, X. et al. Adeno-associated virus of a single-polarity DNA genome is capable of transduction in vivo. Mol. Ther. 16, 494-499 (2008).
73. McCarty, D. M., Monahan, P. E. & Samulski, R. J. Self-complementary recombinant adeno-associated virus (scAAV) vectors promote efficient transduction independently of DNA synthesis. Gene Ther. 8, 1248-1254 (2001).
74. Wang, Z. et al. Rapid and highly efficient transduction by double-stranded adeno-associated virus vectors in vitro and in vivo. Gene Ther. 10, 2105-2111 (2003).
75. Passini, M. A. et al. Antisense Oligonucleotides Delivered to the Mouse CNS Ameliorate Symptoms of Severe Spinal Muscular Atrophy. Sci. Transl. Med. 3, 72ra18-72ra18 (2011).
76. Passini, M. A. et al. Translational fidelity of intrathecal delivery of self-complementary AAV9-survival motor neuron 1 for spinal muscular atrophy. Hum. Gene Ther. 25, 619-630 (2014).

### REFERENCES

The following references are each incorporated herein by reference in their entireties.
1. Jinek, Lefebvre et al. Cell 80, 155-65 (1995).
2. Pearn et al. Lancet 1, 919-922 (1980)
3. Schrank et al. PNAS 94, 9920-5 (1997)
4. Cartegni et al. Am J Hum Genet 78, 63-77 (2006)
5. Wirth et al. Hum Genet 119, 422-8 (2006)
6. Talbot et al. Semin Neurol 21 (189-197 (2001)
7. Murray et al. Hum Mol Genet 17, 949-62 (2008)
8. Chang et al. Neurochem Int 45, 1107-12 (2004)
9. Cho et al. Genes Dev 24, 438-42 (2010)
10. Sing et al. Mol Cell Biol 26, 1333-46 (2006)
11. Porensky et al. Hum Mol Genet 21, 1625-38 (2012)
12. Cong et al. Science 339, 819-23 (2013)
13. Ran et al. Nature 520, 186-90 (2015)
14. Long et al. Science 5725 (2015)
15. Tabebordbar et al. Science 5177, 1-9 (2015)
16. Nelson et al. Science 5143m 1-8 (2015)
17. Corti et al. Sci Transl Med 4, 165ra162 (2012)
18. Arbab et al. Stem Cell Reports 5, 1-10 (2015)
19. Iyama et al. SNA Repair (Amst). 12, 620-626 (2013)
20. Komor et al. Science 533, 420-424 (2016)
21. Huang et al. Nature Biotech (2019)
22. Shen et al. Nature 2018

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims are introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

Aspects of the invention are disclosed as the following clauses:
1. A method for deaminating an adenosine nucleobase (A) in an *SMN2* gene, the method comprising contacting the *SMN2* gene with a base editor in association with a guide RNA (gRNA), wherein the gRNA comprises a guide sequence that is complementary to a target nucleic acid sequence in the *SNM2* gene.
2. The method of clause 1, wherein the guide sequence comprises at least 10, 11, 12, 13, 14, 15, 16, 17, 18, 19, 20, 21, 22, 23, 24, or 25 contiguous nucleobases that are at least 80%, 85%, 90%, 95%, 98%, 99%, or 100% complementary to the target nucleic acid sequence of the *SMN2* gene.
3. The method of clause 1 or 2, wherein the target nucleic acid sequence in the *SMN2* gene comprises or consists of the nucleic acid sequence of any one of SEQ ID NOs: 249-399, or a naturally-occurring variant thereof.
4. The method of any one of clauses 1-3, wherein the target nucleic acid sequence in the *SMN2* gene comprises or consists of the nucleic acid sequence SEQ ID NO: 1.
5. The method of any one of clauses 1-4, wherein the base editor comprises a nucleic acid programmable DNA binding protein (napDNAbp) and an adenosine deaminase.
6. The method of clause 5, wherein the napDNAbp comprises a nuclease or a nickase.
7. The method of clause 6, wherein the base editor nicks the target sequence that is complementary to the guide sequence.
8. The method of any one of clauses 5-7, wherein the napDNAbp comprises a circularly permuted napDNAbp.
9. The method of clause 8, wherein the circularly permuted napDNAbp is a circularly permuted Cas9 or variant thereof.
10. The method of clause 9, wherein the circularly permuted napDNAbp is a circularly permuted Cas9 that is circularly permuted at any one of amino residue number 1000 to 1340 of a Cas9.
11. The method of clause 10, wherein the circularly permuted napDNAbp is a circularly permuted Cas9 that is circularly permuted at amino acid residue 1040 of a Cas9.
12. The method of any one of clauses 1-11, wherein the base editor comprises a split-intein base editor.
13. The method of any one of clauses 1-7, wherein the base editor comprises wild-type Cas9.
14. The method of any one of clauses 1-7, wherein the base editor comprises saCas9-KKH, Cas9-VQR, Cas9-VRQR, Cas9-VRER, Cas9-NG, ABE7.7, pNMG-624, ABE3.2, ABE5.3, pNMG-558, pNMG-576, pNMG-577, pNMG-586, ABE7.2, pNMG-620, pNMG-617, pNMG-618, pNMG-620, pNMG-621, pNGM-622, pNMG-623, ABE6.3, ABE6.4, ABE7.8, ABE7.9, ABE7.10, ABEMax, ABE8e, CP1028-ABE8e, ABE7.10-CP1041, or CP1041-ABE8e.
15. The method of any one of clauses 1-14, wherein the guide sequence of the gRNA comprises the nucleic acid sequence 5'-AUUUUGUCUAAAACCCUGUA-3' (SEQ ID NO: 250).
16. The method of any one of clauses 1-14, wherein the guide sequence of the gRNA comprises a nucleic acid sequence selected from the group consisting of:
   5'-UUUGUCU**A**AAACCCUGUAAG-3' (SEQ ID NO: 367),
   5'-UUUUGUCU**A**AAACCCUGUAA-3' (SEQ ID NO: 369),
   5'-UGAUUUUGUCU**A**AAACCC-3' (SEQ ID NO: 371),
   5'-GAUUUUGUCU**A**AAACCCU-3' (SEQ ID NO: 373),
   5'-AUUUUGUCU**A**AAACCCUG-3' (SEQ ID NO: 375),
   5'-GUCU**A**AAACCCUGUAAGG-3' (SEQ ID NO: 377), and
   5'-UCU**A**AAACCCUGUAAGGA-3' (SEQ ID NO: 379).
17. The method of any one of clauses 1-14, wherein the guide sequence of the gRNA comprises a nucleic acid sequence selected from the group consisting of:
   5'-UUUGCAGGAAAUGCUGGCAU-3' (SEQ ID NO: 382),
   5'-UUCUCAUUUGCAGGAAAUGC-3' (SEQ ID NO: 384),
   5'-CAUUUAGUGCUGCUCU**A**UGC-3' (SEQ ID NO: 386),
   5'-CAGGAAAUGCUGGCAU**A**GAG-3' (SEQ ID NO: 388),
   5'-UUGCAGGAAAUGCUGGCAU**A**-3' (SEQ ID NO: 390),
   5'-AUUUGCAGGAAAUGCUGGCA-3' (SEQ ID NO: 392), and
   5'-TGGCATAGAGCAGCACTAAA-3' (SEQ ID NO: 393).
18. The method of any one of clauses 1-12, wherein the guide sequence of the gRNA comprises the nucleic acid sequence 5'-UACAUGAGUGGCUAUCAUAC-3'(SEQ ID NO: 397).
19. The method of any one of clauses 1-18, wherein the gRNA comprises the structure
   5'-[guide sequence]-[Cas9 binding sequence]-3', and wherein the Cas9 binding sequence is at least 70%, 75%, 80%, 85%, 90%, 95%, 96%, 97%, 98%, 99%, or 99.5% identical to 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304).
20. The method of any one of clauses 1-19, wherein the gRNA comprises the structure
   5'-[guide sequence]-[Cas9 binding sequence]-3', and wherein the Cas9 binding sequence comprises 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304).
21. The method of any one of clauses 1-19, wherein the gRNA comprises the nucleic acid sequence 
5′-
AUUUUGUCUAAAACCCUGUAGUUUUAGAGCUAGAAAUAGCAAGUUAAAAUAA
 
AGGCUAGUCCGUUAUCAACUUGAAAAAGUGGCACCGAGUCGGUGCUUUUU-3'
(SEQ ID NO: 405);
 or 
5′-
AUUUUGUCUAAAACCCUGUAGUUUUAGAGCUAGAAAUAGCAAGUUAAAAUAA
GGCUAGUCCGUUAUCAACUUGAAAAAGUGGCACCGAGUCGGUGCUUUUUUU-3′
(SEQ ID NO: 406).
22. The method of any one of clauses 1-21, wherein deaminating the adenosine nucleobase in the *SMN2* gene results in a T-A base pair in the *SMN2* gene being mutated to a C-G base pair in the *SMN2* gene.
23. The method of any one of clauses 1-22, wherein the *SMN2* gene comprises C840T.
24. The method of any one of clauses 1-23, wherein deaminating an adenosine nucleobase in the *SMN2* gene results in a sequence that is not associated with spinal muscular atrophy (SMA).
25. The method of any one of clauses 1-24, wherein deaminating an adenosine nucleobase in the *SMN2* gene leads to an increase in full-length SMA protein.
26. The method of any one of clauses 1-25, wherein deaminating an adenosine nucleobase in the *SMN2* gene leads to an increase in SMA protein stability.
27. The method of any one of clauses 1-26, wherein the method is performed *in vitro.*
28. The method of any one of clauses 1-26, wherein the method is performed *in vivo.*
29. The method of any one of clauses 1-26, wherein the method is performed *ex vivo.*
30. The method of any one of clauses 1-29, wherein the method is performed in a subject.
31. The method of clause 30, wherein the subject has or is suspected of having spinal muscular atrophy (SMA).
32. The method of clause 30 or 31, wherein an *SMN2* gene of the subject comprises C840T.
33. The method of any one of clauses 30-32, wherein the subject is human.
34. The method of any one of clauses 30-33, wherein the subject is *in utero.*
35. The method of any of clauses 30-34, wherein the subject is a zygote.
36. The method of any one of clauses 30-35, wherein the subject is a fetus.
37. The method of any one of clauses 30-33, wherein the subject is a baby that is less than 1, 2, 3, 4, or 5 days old.
38. The method of any one of clauses 30-33, wherein the subject is a baby that is less than 1, 2, 3, or 4, weeks old.
39. The method of any one of clauses 30-33, wherin the subject is a baby that is less than 1, 2, 3, 4, 5, or 6 months old.
40. The method of any one of clauses 30-33, wherein the subject is a baby that is less than 1, 2, 3, 4, 5, 6, 7, 8, 9, or 10 years old.
41. The method of any one of clauses 1-40, wherein the base editor is an adenine base editor.
42. The method of any one of clauses 1-41, wherein the base editor comprises the structure: NH2-[first nuclear localization sequence]-[first adenosine deaminase]-[second adenosine deaminase]-[Cas9 domain]-[second nuclear localization sequence]-COOH, and each instance of "-" comprises an optional linker.
43. The method of clause 42, wherein the first nuclear localization sequence comprises the amino acid sequence MKRTADGSEFESPKKKRKV (SEQ ID NO: 158), or a variant thereof that is at least 70%, 75%, 80%, 85%, 90%, 95%, 96%, 97%, 98%, 99%, or 99.5% identical thereto, and the second nuclear localization sequence comprises the amino acid sequence MKRTADGSEFEPKKKRKV (SEQ ID NO: 158), or a variant thereof that is at least 70%, 75%, 80%, 85%, 90%, 95%, 96%, 97%, 98%, 99%, or 99.5% identical thereto.
44. The method of clause 42 or 43, wherein the first adenosine deaminase comprises the amino acid sequence MSEVEFSHEYWMRHALTLAKRAWDEREVPVGAVLVHNNRVIGEGWNRPIGRHDPT AHAEIMALRQGGLVMQNYRLIDATLYVTLEPCVMCAGAMIHSRIGRVVFGARDAKT GAAGSLMDVLHHPGMNHRVEITEGILADECAALLSDFFRMRRQEIKAQKKAQSSTD (SEQ ID NO: 88), or a variant thereof that is at least 70%, 75%, 80%, 85%, 90%, 95%, 96%, 97%, 98%, 99%, or 99.5% identical thereto.
45. The method of any one of clauses 42-44, wherein the second adenosine deaminase comprises the amino acid sequence MSEVEFSHEYWMRHALTLAKRARDEREVPVGAVLVLNNRVIGEGWNRAIGLHDPT AHAEIMALRQGGLVMQNYRLIDATLYVTFEPCVMCAGAMIHSRIGRVVFGVRNAKT GAAGSLMDVLHYPGMNHRVEITEGILADECAALLCYFFRMPRQVFNAQKKAQSSTD (SEQ ID NO: 79), or a variant thereof that is at least 70%, 75%, 80%, 85%, 90%, 95%, 96%, 97%, 98%, 99%, or 99.5% identical thereto.
46. The method of any one of clauses 42-45, wherein the Cas9 domain comprises the amino acid sequence DKKYSIGLAIGTNSVGWAVITDEYKVPSKKFKVLGNTDRHSIKKNLIGALLFDSGETA EATRLKRTARRRYTRRKNRICYLQEIFSNEMAKVDDSFFHRLEESFLVEEDKKHERH PIFGNIVDEVAYHEKYPTIYHLRKKLVDSTDKADLRLIYLALAHMIKFRGHFLIEGDL NPDNSDVDKLFIQLVQTYNQLFEENPINASGVDAKAILSARLSKSRRLENLIAQLPGE KKNGLFGNLIALSLGLTPNFKSNFDLAFDAKLQLSKDTYDDDLDNLLAQIGDQYADL FLAAKNLSDAILLSDILRVNTEITKAPLSASMIKRYDEHHQDLTLLKALVRQQLPEKY KEIFFDQSKNGYAGYIDGGASQEEFYKFIKPILEKMDGTEELLVKLNREDLLRKQRTF DNGSIPHQIHLGELHAILRRQEDFYPFLKDNREKIEKILTFRIPYYVGPLARGNSRFAW MTRKSEETITPWNFEEVVDKGASAQSFIERMTNFDKNLPNEKVLPKHSLLYEYFTVY NELTKVKYVTEGMRKPAFLSGEQKKAIVDLLFKTNRKVTVKQLKEDYFKKIECFDS VEISGVEDRFNASLGTYHDLLKIIKDKDFLDNEENEDILEDIVLTLTLFEDREMIEERL KTYAHLFDDKVMKQLKRRRYTGWGRLSRKLINGIRDKQSGKTILDFLKSDGFANRN FMQLIHDDSLTFKEDIQKAQVSGQGDSLHEHIANLAGSPAIKKGILQTVKVVDELVK VMGRHKPENIVIEMARENQTTQKGQKNSRERMKRIEEGIKELGSQILKEHPVENTQL QNEKLYLYYLQNGRDMYVDQELDINRLSDYDVDHIVPQSFLKDDSIDNKVLTRSDK NRGKSDNVPSEEVVKKMKNYWRQLLNAKLITQRKFDNLTKAERGGLSELDKAGFIK RQLVETRQITKHVAQILDSRMNTKYDENDKLIREVKVITLKSKLVSDFRKDFQFYKV REINNYHHAHDAYLNAVVGTALIKKYPKLESEFVYGDYKVYDVRKMIAKSEQEIGK ATAKYFFYSNIMNFFKTEITLANGEIRKRPLIETNGETGEIVWDKGRDFATVRKVLSM PQVNIVKKTEVQTGGFSKESILPKRNSDKLIARKKDWDPKKYGGFVSPTVAYSVLVV AKVEKGKSKKLKSVKELLGITIMERSSFEKNPIDFLEAKGYKEVKKDLIIKLPKYSLFE LENGRKRMLASARELQKGNELALPSKYVNFLYLASHYEKLKGSPEDNEQKQLFVEQ HKHYLDEIIEQISEFSKRVILADANLDKVLSAYNKHRDKPIREQAENIIHLFTLTNLGA PAAFKYFDTTIDRKQYRSTKEVLDATLIHQSITGLYETRIDLSQLGGD (SEQ ID NO: 407), or a variant thereof that is at least 70%, 75%, 80%, 85%, 90%, 95%, 96%, 97%, 98%, 99%, or 99.5% identical thereto.
47. The method of any one of clauses 42-46, wherein the base editor comprises the amino acid sequence MKRTADGSEFESPKKKRKVSEVEFSHEYWMRHALTLAKRAWDEREVPVGAVLVHN NRVIGEGWNRPIGRHDPTAHAEIMALRQGGLVMQNYRLIDATLYVTLEPCVMCAGA MIHSRIGRVVFGARDAKTGAAGSLMDVLHHPGMNHRVEITEGILADECAALLSDFFR MRRQEIKAQKKAQSSTDSGGSSGGSSGSETPGTSESATPESSGGSSGGSSEVEFSHEY WMRHALTLAKRARDEREVPVGAVLVLNNRVIGEGWNRAIGLHDPTAHAEIMALRQ GGLVMQNYRLIDATLYVTFEPCVMCAGAMIHSRIGRVVFGVRNAKTGAAGSLMDV LHYPGMNHRVEITEGILADECAALLCYFFRMPRQVFNAQKKAQSSTDSGGSSGGSSG SETPGTSESATPESSGGSSGGSDKKYSIGLAIGTNSVGWAVITDEYKVPSKKFKVLGN TDRHSIKKNLIGALLFDSGETAEATRLKRTARRRYTRRKNRICYLQEIFSNEMAKVDD SFFHRLEESFLVEEDKKHERHPIFGNIVDEVAYHEKYPTIYHLRKKLVDSTDKADLRL IYLALAHMIKFRGHFLIEGDLNPDNSDVDKLFIQLVQTYNQLFEENPINASGVDAKAI LSARLSKSRRLENLIAQLPGEKKNGLFGNLIALSLGLTPNFKSNFDLAEDAKLQLSKD TYDDDLDNLLAQIGDQYADLFLAAKNLSDAILLSDILRVNTEITKAPLSASMIKRYDE HHQDL TLLKAL VRQQLPEKYKEIFFDQSKNGY AGYIDGGASQEEFYKFIKPILEKMD GTEELLVKLNREDLLRKQRTFDNGSIPHQIHLGELHAILRRQEDFYPFLKDNREKIEKI LTFRIPYYVGPLARGNSRFAWMTRKSEETITPWNFEEVVDKGASAQSFIERMTNFDK NLPNEKVLPKHSLLYEYFTVYNELTKVKYVTEGMRKPAFLSGEQKKAIVDLLFKTN RKVTVKQLKEDYFKKIECFDSVEISGVEDRFNASLGTYHDLLKIIKDKDFLDNEENED ILEDIVLTLTLFEDREMIEERLKTYAHLFDDKVMKQLKRRRYTGWGRLSRKLINGIRD KQSGKTILDFLKSDGFANRNFMQLIHDDSLTFKEDIQKAQVSGQGDSLHEHIANLAG SPAIKKGILQTVKVVDELVKVMGRHKPENIVIEMARENQTTQKGQKNSRERMKRIEE GIKELGSQILKEHPVENTQLQNEKLYLYYLQNGRDMYVDQELDINRLSDYDVDHIVP QSFLKDDSIDNKVLTRSDKNRGKSDNVPSEEVVKKMKNYWRQLLNAKLITQRKFDN LTKAERGGLSELDKAGFIKRQLVETRQITKHVAQILDSRMNTKYDENDKLIREVKVIT LKSKLVSDFRKDFQFYKVREINNYHHAHDAYLNAVVGTALIKKYPKLESEFVYGDY KVYDVRKMIAKSEQEIGKATAKYFFYSNIMNFFKTEITLANGEIRKRPLIETNGETGEI VWDKGRDFATVRKVLSMPQVNIVKKTEVQTGGFSKESILPKRNSDKLIARKKDWDP KKYGGFVSPTVAYSVLVVAKVEKGKSKKLKSVKELLGITIMERSSFEKNPIDFLEAK GYKEVKKDLIIKLPKYSLFELENGRKRMLASARELQKGNELALPSKYVNFLYLASHY EKLKGSPEDNEQKQLFVEQHKHYLDEIIEQISEFSKRVILADANLDKVLSAYNKHRD KPIREQAENIIHLFTLTNLGAPAAFKYFDTTIDRKQYRSTKEVLDATLIHQSITGLYET RIDLSQLGGDSGGSKRTADGSEFEPKKKRKV (SEQ ID NO: 201), or a variant thereof that is at least 70%, 75%, 80%, 85%, 90%, 95%, 96%, 97%, 98%, 99%, or 99.5% identical thereto.
48. The method of any one of clauses 1-47, wherein the method is a method for treating a subject having or suspected of having spinal muscular atrophy (SMA).
49. The method of clause 48, further comprising administering a therapeutic agent to the subject.
50. The method of clause 49, wherein the therapeutic agent is an antisense oligonucleotide therapy.
51. The method of clause 49 or 50, wherein the therapeutic agent is nusinersen.
52. The method of clause 49, wherein the therapeutic agent is risdiplam.
53. A guide RNA comprising a guide sequence, wherein the guide sequence of the guide RNA comprises or consists of
   5'-UUUGUCU**A**AAACCCUGUAAG-3' (SEQ ID NO: 367),
   5'-UUUUGUCU**A**AAACCCUGUAA-3' (SEQ ID NO: 369),
   5'-UGAUUUUGUCU**A**AAACCC-3' (SEQ ID NO: 371),
   5'-GAUUUUGUCU**A**AAACCCU-3' (SEQ ID NO: 373),
   5'-AUUUUGUCU**A**AAACCCUG-3' (SEQ ID NO: 375),
   5'-GUCU**A**AAACCCUGUAAGG-3' (SEQ ID NO: 377),
   5'-UCU**A**AAACCCUGUAAGGA-3' (SEQ ID NO: 379),
   5'-UUUGCAGGAAAUGCUGGCAU-3' (SEQ ID NO: 382),
   5'-UUCUCAUUUGCAGGAAAUGC-3' (SEQ ID NO: 384),
   5'-CAUUUAGUGCUGCUCU**A**UGC-3' (SEQ ID NO: 386),
   5'-CAGGAAAUGCUGGCAU**A**GAG-3' (SEQ ID NO: 388),
   5'-UUGCAGGAAAUGCUGGCAU**A**-3' (SEQ ID NO: 390),
   5'-AUUUGCAGGAAAUGCUGGCA-3' (SEQ ID NO: 392),
   5'-UGGCAUAGAGCAGCACUAAA-3' (SEQ ID NO: 394),
   5'-GAUUUUGUCU**A**AAACCCUGUAAG-3' (SEQ ID NO: 451),
   5'-UUGUCU**A**AAACCCUGUAAGG-3' (SEQ ID NO: 452),
   5'-UGUCU**A**AAACCCUGUAAGGA-3' (SEQ ID NO: 453),
   5'-GUCU**A**AAACCCUGUAAGGAA-3' (SEQ ID NO: 454),
   5'-AAAAGUAAGAUUCACUUUCA-3' (SEQ ID NO: 455),
   5'-CAAAAGUAAGAUUCACUUUC-3' (SEQ ID NO: 456),
   5'-ACAAAAGUAAGAUUCACUUU-3' (SEQ ID NO: 457), or
   5'-UACAAAAGUAAGAUUCACUU-3' (SEQ ID NO: 458).
54. The guide RNA of clause 53, wherein the guide sequence of the gRNA comprises or consists of the nucleic acid sequence 5'-AUUUUGUCUAAAACCCUGUA-3' (SEQ ID NO: 365) or 5'-UACAUGAGUGGCUAUCAUAC-3'(SEQ ID NO: 397)
55. The guide RNA of clause 53 or 54, wherein the gRNA comprises the structure
   5'-[guide sequence]-[Cas9 binding sequence]-3', and wherein the Cas9 binding sequence is at least 70%, 75%, 80%, 85%, 90%, 95%, 96%, 97%, 98%, 99%, or 99.5% identical to 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304).
56. The guide RNA of any one of clauses 53-55, wherein the gRNA comprises the structure
   5'-[guide sequence]-[Cas9 binding sequence]-3', and wherein the Cas9 binding sequence comprises 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304).
57. The guide RNA of any one of clauses 53-56, wherein the gRNA comprises the nucleic acid sequence 
5′-
AUUUUGUCUAAAACCCUGUAGUUUUAGAGCUAGAAAUAGCAAGUUAAAAUAA
AGGCUAGUCCGUUAUCAACUUGAAAAAGUGGCACCGAGUCGGUGCUUUUU-3′
(SEQ ID NO: 405);
 or 
5′-
AUUUGUCUAAAACCCUGUAGUUUUAGAGCUAGAAAUAGCAAGUUAAAAUAA
GGCUAGUCCGUUAUCAACUUGAAAAAGUGGCACCGAGUCGGUGCUUUUUUU-3′
(SEQ ID NO: 406).
58. A nucleic acid encoding the guide RNA of any one of clauses 53-57.
59. A complex comprising (i) a base editor, and (ii) the guide RNA (gRNA) of any one of clauses 53-58.
60. The complex of clause 59, wherein the base editor is an adenine base editor.
61. The complex of any one of clauses 59-60, wherein the base editor is any of the base editors provided herein.
62. A pharmaceutical composition comprising the complex of any one of clauses 59-61.
63. A pharmaceutical composition comprising the guide RNA of any one of clauses 53-57.
64. A pharmaceutical composition comprising the nucleic acid of clause 58.
65. The pharmaceutical composition of any one of clauses 62-64, further comprising a pharmaceutically acceptable excipient.
66. The pharmaceutical composition of any one of clauses 62-65 further comprising a cationic lipid or cationic polymer.
67. The pharmaceutical composition of any one of clauses 62-66, further comprising a therapeutic agent.
68. The pharmaceutical composition of clause 67, wherein the therapeutic agent is an antisense oligonucleotide therapy.
69. The pharmaceutical composition of clause 67 or 68, wherein the therapeutic agent is nusinersen.
70. The pharmaceutical composition of clause 67, wherein the therapeutic agent is risdiplam.
71. A virus comprising a nucleic acid encoding the guide RNA of any one of clauses 53-57.
72. A virus comprising a nucleic acid encoding
   (i) a base editor, and (ii) the guide RNA of any one of clauses 53-57.
73. The virus of clause 72, wherein the base editor is split between two different nucleic acid molecules.
74. A first nucleic acid molecule encoding an N-terminal portion of an adenine base editor fused at its C-terminus to a first intein sequence.
75. The first nucleic acid molecule of clause 74, wherein the N-terminal portion of the adenine base editor is an N-terminal portion of any of the adenine base editors provided herein.
76. The first nucleic acid molecule of clause 74 or 75, wherein the first nucleic acid molecule is operably linked to a first promoter.
77. The first nucleic acid molecule of any one of clauses 74-76, wherein the N-terminal portion of the adenine base editor includes a portion of the Cas9 domain, or an amino acid sequence up to residue E572 of SEQ ID NO: 73.
78. A second nucleic acid molecule encoding a C-terminal portion of a nucleobase editor fused at its N-terminus to a second intein sequence.
79. The second nucleic acid molecule of clause 78, wherein the C-terminal portion of the adenine base editor is a C-terminal portion of any of the adenine base editors provided herein.
80. The second nucleic acid molecule of clause 78 or 79, wherein the second nucleic acid molecule is operably linked to a second promoter.
81. The second nucleic acid molecule of any one of clauses 78-80, wherein the C-terminal portion of the adenine base editor includes a portion of the Cas9 domain, or an amino acid sequence from the C573 residue of SEQ ID NO: 73.
82. The first nucleic acid molecule of any one of clauses 74-77, further comprising a nucleic acid segment encoding the guide RNA of any one of clauses 53-57 operably linked to a third prompter, wherein the direction of the transcription of the nucleic acid segment is reversed relative to the direction of transcription of the first nucleic acid molecule.
83. The second nucleic acid molecule of any one of clauses 78-81, further comprising a nucleic acid segment encoding the guide RNA of any one of clauses 53-57 operably linked to a third prompter, wherein the direction of the transcription of the nucleic acid segment is reversed relative to the direction of transcription of the second nucleic acid molecule.
84. A composition comprising the first nucleic acid molecule of any one of clauses 74-77 or 82.
85. A composition comprising the second nucleic acid molecule of any one of clauses 78-81, or 83.
86. A virus comprising the nucleic acid molecule of any one of clauses 74-77 or 82.
87. A virus comprising the nucleic acid molecule of any one of clauses 78-81, or 83.
88. The virus of clause 86 or 87, wherein the virus is an adenovirus.
89. The virus of any one of clauses 86-88, wherein the virus is a recombinant adeno-associated virus (rAAV).
90. A composition comprising:
   a) the first nucleic acid molecule of any one of clauses 74-77 or 82, and
   b) the second nucleic acid molecule of any one of clauses 78-81, or 83.
91. A virus comprising:
   a) the first nucleic acid molecule of any one of clauses 74-77 or 82, and
   b) the second nucleic acid molecule of any one of clauses 78-81, or 83.
92. The virus of clause 91, wherein the virus is an adenovirus.
93. The virus of clause 91 or 92, wherein the virus is a recombinant adeno-associated virus (rAAV).
94. The method of any one of clauses 1-52, comprising the use of the composition of any one of clauses 84, 85, or 90, or the virus of any one of clauses 86-89 or 91-93.
95. The method of any one of clauses 30-52, comprising contacting the subject with the composition of any one of clauses 84, 85, or 90, or the virus of any one of clauses 86-89 or 91-93.
96. A pharmaceutical composition comprising the composition of any one of clauses 84, 85, or 90, or the virus of any one of clauses 86-89 or 91-93.
97. The pharmaceutical composition of clause 96, further comprising a pharmaceutically acceptable excipient.
98. The pharmaceutical composition of clause 96 or 97, further comprising a cationic lipid or cationic polymer.
99. A vector comprising the nucleic acid of clause 58.
100. The vector of clause 99, wherein the nucleic acid encoding the gRNA is under the control of a heterologous promoter.
101. The vector of clause 99 or 100, further comprising a nucleic acid encoding a base editor.
102. The vector of clause 101, wherein the base editor comprises a fusion protein comprising a nucleic acid programmable DNA binding protein (napDNAbp) and an adenosine deaminase.
103. The vector of clause 101 or 102, wherein the base editor is under the control of a heterologous promoter.
104. A kit comprising
   (i) a base editor comprising a nucleic acid programmable DNA binding protein (napDNAbp) and an adenosine deaminase; and
   (ii) a guide RNA (gRNA), wherein the gRNA comprises any one of the guide sequences of clause 53.

## Claims

1. A method for deaminating an adenosine nucleobase (A) in an *SMN2* gene, the method comprising contacting the *SMN2* gene with a base editor in association with a guide RNA (gRNA), wherein the gRNA comprises a guide sequence that is complementary to a target nucleic acid sequence in the *SMN2* gene.

2. The method of claim 1, wherein:
(i) the guide sequence comprises at least 10, 11, 12, 13, 14, 15, 16, 17, 18, 19, 20, 21, 22, 23, 24, or 25 contiguous nucleobases that are at least 80%, 85%, 90%, 95%, 98%, 99%, or 100% complementary to the target nucleic acid sequence of the *SMN2* gene; and/or
(ii) the target nucleic acid sequence in the *SMN2* gene comprises or consists of the nucleic acid sequence of any one of SEQ ID NOs: 249-399, or a naturally-occurring variant thereof; and/or
(iii) the target nucleic acid sequence in the *SMN2* gene comprises or consists of the nucleic acid sequence SEQ ID NO: 1; and/or
(iv) the base editor comprises a nucleic acid programmable DNA binding protein (napDNAbp) and an adenosine deaminase, optionally wherein:
(a) the napDNAbp comprises a nuclease or a nickase; and/or
(b) the base editor nicks the target sequence that is complementary to the guide sequence; and/or
(c) the napDNAbp comprises a circularly permuted napDNAbp, optionally wherein the circularly permuted napDNAbp is a circularly permuted Cas9 or variant thereof, optionally wherein the circularly permuted napDNAbp is a circularly permuted Cas9 that is circularly permuted at any one of amino residue numbers 1000 to 1340 of a Cas9, and optionally wherein the circularly permuted napDNAbp is a circularly permuted Cas9 that is circularly permuted at amino acid residue 1040 of a Cas9; and/or
(v) the base editor comprises a split-intein base editor; and/or
(vi) the base editor comprises wild-type Cas9, saCas9-KKH, Cas9-VQR, Cas9-VRQR, Cas9-VRER, Cas9-NG, ABE7.7, pNMG-624, ABE3.2, ABE5.3, pNMG-558, pNMG-576, pNMG-577, pNMG-586, ABE7.2, pNMG-620, pNMG-617, pNMG-618, pNMG-620, pNMG-621, pNGM-622, pNMG-623, ABE6.3, ABE6.4, ABE7.8, ABE7.9, ABE7.10, ABEMax, ABE8e, CP1028-ABE8e, ABE7.10-CP1041, or CP1041-ABE8e; and/or
(vii) the guide sequence of the gRNA comprises:
(a) the nucleic acid sequence 5'-AUUUUGUCU**A**AAACCCUGUA-3' (SEQ ID NO: 250); or
(b) a nucleic acid sequence selected from the group consisting of:
5'-UUUGUCU**A**AAACCCUGUAAG-3' (SEQ ID NO: 367),
5'-UUUUGUCU**A**AAACCCUGUAA-3' (SEQ ID NO: 369),
5'-UGAUUUUGUCU**A**AAACCC-3' (SEQ ID NO: 371),
5'-GAUUUUGUCU**A**AAACCCU-3' (SEQ ID NO: 373),
5'-AUUUUGUCU**A**AAACCCUG-3' (SEQ ID NO: 375),
5'-GUCU**A**AAACCCUGUAAGG-3' (SEQ ID NO: 377), and
5'-UCU**A**AAACCCUGUAAGGA-3' (SEQ ID NO: 379); or
(c) a nucleic acid sequence selected from the group consisting of:
5'-UUUGCAGGAAAUGCUGGCAU-3' (SEQ ID NO: 382),
5'-UUCUCAUUUGCAGGAAAUGC-3' (SEQ ID NO: 384),
5'-CAUUUAGUGCUGCUCU**A**UGC-3' (SEQ ID NO: 386),
5'-CAGGAAAUGCUGGCAU**A**GAG-3' (SEQ ID NO: 388),
5'-UUGCAGGAAAUGCUGGCAU**A**-3' (SEQ ID NO: 390),
5'-AUUUGCAGGAAAUGCUGGCA-3' (SEQ ID NO: 392), and
5'-TGGCATAGAGCAGCACTAAA-3' (SEQ ID NO: 393); and/or
(viii) the gRNA comprises the structure
5'-[guide sequence]-[Cas9 binding sequence]-3', and wherein the Cas9 binding sequence is at least 70%, 75%, 80%, 85%, 90%, 95%, 96%, 97%, 98%, 99%, or 99.5% identical to 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304);
 and/or
(vix) the gRNA comprises the structure
5'-[guide sequence]-[Cas9 binding sequence]-3', and wherein the Cas9 binding sequence comprises 5′- 
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304);
 and/or
(x) the gRNA comprises the nucleic acid sequence 
5′-
AUUUGUCUAAAACCCUGUAGUUUUAGAGCUAGAAAUAGCAAGUUAAAAUAA
AGGCUAGUCCGUUAUCAACUUGAAAAAGUGGCACCGAGUCGGUGCUUUUU-3′
(SEQ ID NO: 405);
or

5′-
AUUUGUCUAAAACCCUGUAGUUUUAGAGCUAGAAAUAGCAAGUUAAAAUAA
GGCUAGUCCGUUAUCAACUUGAAAAAGUGGCACCGAGUCGGUGCUUUUUUU-3′
(SEQ ID NO: 406).

3. A guide RNA comprising a guide sequence, wherein the guide sequence of the guide RNA comprises or consists of
5'-UUUGUCU**A**AAACCCUGUAAG-3' (SEQ ID NO: 367),
5'-UUUUGUCU**A**AAACCCUGUAA-3' (SEQ ID NO: 369),
5'-UGAUUUUGUCU**A**AAACCC-3' (SEQ ID NO: 371),
5'-GAUUUUGUCU**A**AAACCCU-3' (SEQ ID NO: 373),
5'-AUUUUGUCU**A**AAACCCUG-3' (SEQ ID NO: 375),
5'-GUCU**A**AAACCCUGUAAGG-3' (SEQ ID NO: 377),
5'-UCU**A**AAACCCUGUAAGGA-3' (SEQ ID NO: 379),
5'-UUUGCAGGAAAUGCUGGCAU-3' (SEQ ID NO: 382),
5'-UUCUCAUUUGCAGGAAAUGC-3' (SEQ ID NO: 384),
5'-CAUUUAGUGCUGCUCU**A**UGC-3' (SEQ ID NO: 386),
5'-CAGGAAAUGCUGGCAU**A**GAG-3' (SEQ ID NO: 388),
5'-UUGCAGGAAAUGCUGGCAU**A**-3' (SEQ ID NO: 390),
5'-AUUUGCAGGAAAUGCUGGCA-3' (SEQ ID NO: 392),
5'-UGGCAUAGAGCAGCACUAAA-3' (SEQ ID NO: 394),
5'-GAUUUUGUCU**A**AAACCCUGUAAG-3' (SEQ ID NO: 451),
5'-UUGUCU**A**AAACCCUGUAAGG-3' (SEQ ID NO: 452),
5'-UGUCU**A**AAACCCUGUAAGGA-3' (SEQ ID NO: 453),
5'-GUCU**A**AAACCCUGUAAGGAA-3' (SEQ ID NO: 454),
5'-AAAAGUAAGAUUCACUUUCA-3' (SEQ ID NO: 455),
5'-CAAAAGUAAGAUUCACUUUC-3' (SEQ ID NO: 456),
5'-ACAAAAGUAAGAUUCACUUU-3' (SEQ ID NO: 457), or
5'-UACAAAAGUAAGAUUCACUU-3' (SEQ ID NO: 458); optionally wherein:
(i) the guide sequence of the gRNA comprises or consists of the nucleic acid sequence 5'-AUUUUGUCU**A**AAACCCUGUA-3' (SEQ ID NO: 365) or 5'-UACAUG**A**GUGGCUAUCAUAC-3'(SEQ ID NO: 397); and/or
(ii) the gRNA comprises the structure
5'-[guide sequence]-[Cas9 binding sequence]-3', and wherein the Cas9 binding sequence is at least 70%, 75%, 80%, 85%, 90%, 95%, 96%, 97%, 98%, 99%, or 99.5% identical to 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304);
 and/or
(iii) the gRNA comprises the structure
5'-[guide sequence]-[Cas9 binding sequence]-3', and wherein the Cas9 binding sequence comprises 
5′-
GUUUUAGAGCUAGAAAUAGCAAGUUAAAAUAAGGCUAGUCCGUUAUCAACU
UGAAAAAGUGGCACCGAGUCGGUGCUUUUU-3′ (SEQ ID NO: 303);
 or 
5′-
GUUUUAGAGCUAGAAAUAGCAAGUUAAAAUAAGGCUAGUCCGUUAUCAACUU
GAAAAAGUGGCACCGAGUCGGUGCUUUUUUU-3′ (SEQ ID NO: 304);
 and/or
(iv) the gRNA comprises the nucleic acid sequence 
5′-
AUUUUGUCUAAAACCCUGUAGUUUUAGAGCUAGAAAUAGCAAGUUAAAAUAA
AGGCUAGUCCGUUAUCAACUUGAAAAAGUGGCACCGAGUCGGUGCUUUUU-3′
(SEQ ID NO: 405);
 or

5′-
AUUUUGUCUAAAACCCUGUAGUUUUAGAGCUAGAAAUAGCAAGUUAAAAUAA

GGCUAGUCCGUUAUCAACUUGAAAAAGUGGCACCGAGUCGGUGCUUUUUU-3'
(SEQ ID NO: 406).

4. A nucleic acid encoding a guide RNA of claim 3.

5. A vector comprising the nucleic acid of claim 4, optionally wherein the nucleic acid encoding the gRNA is under the control of a heterologous promoter, and/or wherein the vector further comprises a nucleic acid encoding a base editor, and optionally wherein:
(i) the base editor comprises a fusion protein comprising a nucleic acid programmable DNA binding protein (napDNAbp) and an adenosine deaminase; and/or
(ii) the base editor is under the control of a heterologous promoter.

6. A complex comprising (i) a base editor, and (ii) a guide RNA (gRNA) of claim 3, optionally wherein the base editor is an adenine base editor, and/or the base editor is any of the base editors provided herein.

7. A virus comprising a nucleic acid molecule encoding:
(a) a guide RNA of claim 3; or
(b) a base editor and a guide RNA of claim 3, optionally wherein the base editor is split between two different nucleic acid molecules.

8. A first nucleic acid molecule encoding an N-terminal portion of an adenine base editor fused at its C-terminus to a first intein sequence, wherein:
(i) the N-terminal portion of the adenine base editor is an N-terminal portion of any of the adenine base editors provided herein; and/or
(ii) the first nucleic acid molecule is operably linked to a first promoter; and/or
(iii) the N-terminal portion of the adenine base editor includes a portion of the Cas9 domain, or an amino acid sequence up to residue E572 of SEQ ID NO: 73; and/or
(iv) the first nucleic acid molecule further comprises a nucleic acid segment encoding a guide RNA of claim 3 operably linked to a third promoter, wherein the direction of the transcription of the nucleic acid segment is reversed relative to the direction of transcription of the first nucleic acid molecule.

9. A second nucleic acid molecule encoding a C-terminal portion of a nucleobase editor fused at its N-terminus to a second intein sequence, wherein:
(i) the C-terminal portion of the adenine base editor is a C-terminal portion of any of the adenine base editors provided herein; and/or
(ii) the second nucleic acid molecule is operably linked to a second promoter; and/or
(iii) the C-terminal portion of the adenine base editor includes a portion of the Cas9 domain, or an amino acid sequence from the C573 residue of SEQ ID NO: 73; and/or
(iv) the second nucleic acid molecule further comprises a nucleic acid segment encoding a guide RNA of claim 3 operably linked to a third prompter, wherein the direction of the transcription of the nucleic acid segment is reversed relative to the direction of transcription of the second nucleic acid molecule.

10. A composition comprising the first nucleic acid molecule of claim 8 and/or the second nucleic acid molecule of claim 9.

11. A virus comprising the first nucleic acid molecule of claim 8 and/or the second nucleic acid molecule of claim 9, optionally wherein:
(i) the virus is an adenovirus; and/or
(ii) the virus is a recombinant adeno-associated virus (rAAV).

12. A pharmaceutical composition comprising the guide RNA of claim 3, the nucleic acid of claim 4, the complex of claim 6, the composition of claim 10, or the virus of claim 11, optionally wherein the pharmaceutical composition further comprises:
(i) a pharmaceutically acceptable excipient; and/or
(ii) a cationic lipid or cationic polymer; and/or
(iii) a therapeutic agent, optionally wherein the therapeutic agent is:
(a) an antisense oligonucleotide therapy and/or nusinersen; or
(b) risdiplam.

13. The method of claim 1 or 2, wherein deaminating the adenosine nucleobase in the *SMN2* gene results in a T-A base pair in the *SMN2* gene being mutated to a C-G base pair in the *SMN2* gene; optionally wherein:
(i) the *SMN2* gene comprises C840T; and/or
(ii) deaminating an adenosine nucleobase in the *SMN2* gene results in a sequence that is not associated with spinal muscular atrophy (SMA); and/or
(iii) deaminating an adenosine nucleobase in the *SMN2* gene leads to an increase in full-length SMA protein; and/or
(iv) deaminating an adenosine nucleobase in the *SMN2* gene leads to an increase in SMA protein stability; and/or
(v) the method comprises the use of the composition of claim 10, the virus of claim 11, or the pharmaceutical composition of claim 12;
and wherein the method is not a method for the treatment of the human or animal body by surgery or therapy.

14. The guide RNA of claim 3, the nucleic acid of claim 4, the complex of claim 6, the composition of claim 10, the virus of claim 11, or the pharmaceutical composition of claim 12 for use in treating SMA in a subject, wherein the subject has or is suspected of having SMA; optionally wherein:
(i) an *SMN2* gene of the subject comprises C840T; and/or
(ii) the subject is human; and/or
(iii) the subject is *in utero;* and/or
(iv) the subject is a zygote; and/or
(v) the subject is a fetus; and/or
(vi) the subject is a baby that is less than 10 years old; and/or
(vii) the use further comprises administering a therapeutic agent to the subject, optionally wherein the therapeutic agent is:
(a) an antisense oligonucleotide therapy and/or nusinersen; or
(b) risdiplam.

15. The method of any one of claims 1, 2, or 13, or the guide RNA of claim 3, the nucleic acid of claim 4, the complex of claim 6, the composition of claim 10, the virus of claim 11, or the pharmaceutical composition of claim 12 for use according to claim 14; wherein:
(i) the method is performed *in vitro;* and/or
(ii) the method is performed *in vivo;* and/or
(iii) the method is performed *ex vivo;* and/or
(iv) the base editor is an adenine base editor; and/or
(v) the base editor comprises the structure: NH2-[first nuclear localization sequence ]-[ first adenosine deaminase]-[second adenosine deaminase]-[Cas9 domain]-[second nuclear localization sequence]-COOH, and each instance of "-" comprises an optional linker, optionally wherein:
(a) the first nuclear localization sequence comprises the amino acid sequence of SEQ ID NO: 158, or a variant thereof that is at least 70%, at least 75%, at least 80%, at least 85%, at least 90%, at least 95%, at least 96%, at least 97%, at least 98%, at least 99%, or at least 99.5% identical thereto, and the second nuclear localization sequence comprises the amino acid sequence of SEQ ID NO: 158, or a variant thereof that is at least 70%, at least 75%, at least 80%, at least 85%, at least 90%, at least 95%, at least 96%, at least 97%, at least 98%, at least 99%, or at least 99.5% identical thereto; and/or
(b) the first adenosine deaminase comprises the amino acid sequence of SEQ ID NO: 88, or a variant thereof that is at least 70%, at least 75%, at least 80%, at least 85%, at least 90%, at least 95%, at least 96%, at least 97%, at least 98%, at least 99%, or at least 99.5% identical therto; and/or
(c) the second adenosine deaminase comprises the amino acid sequence of SEQ ID NO: 79, or a variant thereof that is at least 70%, at least 75%, at least 80%, at least 85%, at least 90%, at least 95%, at least 96%, at least 97%, at least 98%, at least 99%, or at least 99.5% identical thereto; and/or
(d) the Cas9 domain comprises the amino acid sequence of SEQ ID NO: 407, or a variant thereof that is at least 70%, at least 75%, at least 80%, at least 85%, at least 90%, at least 95%, at least 96%, at least 97%, at least 98%, at least 99%, or at least 99.5% identical thereto; and/or
(e) the base editor comprises the amino acid sequence of SEQ ID NO: 201, or a variant thereof that is at least 70%, at least 75%, at least 80%, at least 85%, at least 90%, at least 95%, at least 96%, at least 97%, at least 98%, at least 99%, or at least 99.5% identical thereto.

16. A kit comprising
(i) a base editor comprising a nucleic acid programmable DNA binding protein (napDNAbp) and an adenosine deaminase; and
(ii) a guide RNA (gRNA), wherein the gRNA comprises any one of the guide sequences of claim 3.
